(19) Europäisches Patentamt
European Patent Office
Office européen des brevets

(11) EP 4 631 940 A1

(12) # EUROPEAN PATENT APPLICATION
published in accordance with Art. 153(4) EPC

(43) Date of publication:
**15.10.2025 Bulletin 2025/42**

(21) Application number: **23899998.1**

(22) Date of filing: **06.12.2023**

(51) International Patent Classification (IPC):
***C07D 401/14*** (2006.01) ***C07D 487/08*** (2006.01)
***C07D 471/08*** (2006.01) ***C07D 403/12*** (2006.01)
***A61K 31/517*** (2006.01) ***A61P 35/00*** (2006.01)

(52) Cooperative Patent Classification (CPC):
**A61K 31/517; A61P 35/00; C07D 401/14; C07D 403/12; C07D 471/08; C07D 487/08**

(86) International application number:
**PCT/CN2023/136686**

(87) International publication number:
**WO 2024/120424 (13.06.2024 Gazette 2024/24)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **07.12.2022 CN 202211564213**
**18.08.2023 CN 202311043105**
**28.09.2023 CN 202311266772**

(71) Applicant: **Betta Pharmaceuticals Co., Ltd.**
**Hangzhou, Zhejiang 311100 (CN)**

(72) Inventors:
- **WU, Hao**
  **Hangzhou, Zhejiang 311100 (CN)**
- **XU, Renqi**
  **Hangzhou, Zhejiang 311100 (CN)**
- **YANG, Xiaofeng**
  **Hangzhou, Zhejiang 311100 (CN)**
- **LU, Yuan**
  **Hangzhou, Zhejiang 311100 (CN)**
- **ZOU, Zhengyao**
  **Hangzhou, Zhejiang 311100 (CN)**
- **WANG, Dong**
  **Hangzhou, Zhejiang 311100 (CN)**
- **ZHANG, Hongbo**
  **Hangzhou, Zhejiang 311100 (CN)**
- **WANG, Ze**
  **Hangzhou, Zhejiang 311100 (CN)**
- **ZHENG, Hang**
  **Hangzhou, Zhejiang 311100 (CN)**
- **TAN, Yaoming**
  **Hangzhou, Zhejiang 311100 (CN)**
- **HE, Jiangqi**
  **Hangzhou, Zhejiang 311100 (CN)**
- **XIA, Hongfeng**
  **Hangzhou, Zhejiang 311100 (CN)**
- **SHI, Zhaotao**
  **Hangzhou, Zhejiang 311100 (CN)**
- **LIN, Yuanwang**
  **Hangzhou, Zhejiang 311100 (CN)**
- **LI, Tengfei**
  **Hangzhou, Zhejiang 311100 (CN)**
- **FANG, Longcheng**
  **Hangzhou, Zhejiang 311100 (CN)**
- **YUAN, Ding**
  **Hangzhou, Zhejiang 311100 (CN)**
- **XU, Yanjie**
  **Hangzhou, Zhejiang 311100 (CN)**
- **LU, Lu**
  **Hangzhou, Zhejiang 311100 (CN)**
- **LI, Shusen**
  **Hangzhou, Zhejiang 311100 (CN)**
- **ZHAO, Zhichang**
  **Hangzhou, Zhejiang 311100 (CN)**
- **ZHAN, Bo**
  **Hangzhou, Zhejiang 311100 (CN)**
- **WANG, Wei**
  **Hangzhou, Zhejiang 311100 (CN)**
- **ZHOU, Quan**
  **Hangzhou, Zhejiang 311100 (CN)**
- **LAN, Hong**
  **Beijing 100176 (CN)**
- **WANG, Jiabing**
  **Beijing 100176 (CN)**
- **DING, Lieming**
  **Hangzhou, Zhejiang 311100 (CN)**

(74) Representative: **v. Bezold & Partner Patentanwälte - PartG mbB**
**Ridlerstraße 57**
**80339 München (DE)**

EP 4 631 940 A1

(54) **COMPOUND TARGETING PAN-KRAS PROTEIN DEGRADATION AGENT AND USE THEREOF**

(57) A compound targeting a pan-KRAS protein degradation agent and a use thereof, relating to the technical field of medicines. Provided are a compound having an F-L-M structure, or a tautomer, racemate, enantiomer, or diastereomer thereof, or a mixture thereof, or a pharmaceutically acceptable salt thereof, wherein the F is a KRAS protein binding fragment; the L is a linking unit for linking the F and the M; and the M is a VHL binding fragment. The compound can be used for treating diseases caused by KRAS mutations or amplifications.

## Description

### Technical Field

The present application relates to the technical field of medicines, and specifically relates to a compound targeting a KRAS protein degradation agent and a use thereof.

### Background

PROTAC is a heterobifunctional molecule. One end of the molecule is a ligand that binds to a target protein, and the other end is a ligand of the E3 ligase, with the two ends connected by an appropriate chain. Degradation of the target protein by PROTAC is achieved through a ubiquitin-proteasome system (UPS). The general process is that the PROTAC molecule binds to the protein of interest (POI) and E3 ligase to form a ternary complex, so that the protein of interest is marked with a ubiquitin molecule, and then the ubiquitinated protein is recognized and degraded by the intracellular proteasome 26S.

The conventional small molecules and antibodies all inhibit the function of target protein through an action mode of "occupancy-driven" to exert therapeutic effects on diseases. This action mode requires a relatively high concentration of inhibitor or monoclonal antibody in order to occupy the active site of the target and block the transduction of downstream signaling pathways. In contrast, PROTAC is "event-driven", which does not affect the function of protein but mediates the degradation of a pathogenic target protein. As long as PROTAC mediates the formation of the ternary complex and marked the target protein with ubiquitination, PROTAC can theoretically be recycled and reused, and thus a catalytic dose can exert its effect. It has characteristics such as a wider range of action, a higher activity, ability to target "undruggable" targets, improved selectivity, activity and safety, and ability to overcome drug resistance.

The activity of KRAS is altered due to conversion of its binding with GDP or GTP. KRAS is in an inactive state when binding to GDP and is activated when binding to GTP. When KRAS protein is mutated, GAP is restricted from entering GTP, thereby preventing hydrolysis and forming a continuously activated GTP-bound state. Due to the high affinity of mutant KRAS for guanosine triphosphate (GTP), as well as factors such as small catalytic sites and smooth protein surfaces making it difficult to target, the development of small molecule inhibitors has always been a challenge, resulting in "undruggable" characteristic of KRAS. In one aspect, KRAS binds to GDP and GTP with an affinity at picomolar concentration, which seriously hinders the development of a nucleotide-competitive inhibitor. In another aspect, KRAS protein lacks ideal small molecule binding pocket, making it difficult to design a high-affinity allosteric inhibitor.

Cancers caused by RAS mutations account for 25% to 30% of all human cancers. Among them, KRAS mutations account for 86% of the three RAS mutations. Among all the KRAS mutations, there are multiple mutations such as G12D (35%), G12V (29%), G12C (21%), G13D (6.7%), G12R (4.3%), and G12A (4.1%). If these different KRAS mutations can be targeted simultaneously, it will greatly satisfied clinical needs and exhibits great commercial value.

However, available KRAS inhibitors are facing a predicament of limited efficacy and high susceptibility to drug resistance in clinical practice. If the characteristics including anti-resistance, high efficacy, and the like of PROTAC can be combined to develop novel pan-KRAS PROTAC degradation agent drugs for the treatment of diseases caused by KRAS mutation or amplification, it will have great social significance and commercial value.

### Summary

The present application provides a novel pan-KRAS PROTAC degradation agent, which can be used for treating a disease caused by KRAS mutations or amplifications.

In one aspect, the present application provides a compound having an F-L-M structure, or a tautomer, racemate, enantiomer, diastereomer thereof, or a mixture thereof, or a pharmaceutically acceptable salt thereof, wherein:

F is a KRAS protein binding fragment represented by general formula (A);

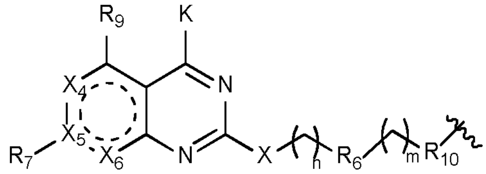

(A)

X is selected from the group consisting of -O- and

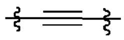;

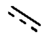

represents a bond independently selected from the group consisting of a single bond and a double bond;

$X_4$ is $CR_8$, $C(R_8)_2$, O, N or $NR_8$; $R_8$ is each independently selected from the group consisting of H, hydroxyl, oxo, halogen, cyano, $C_{1-6}$ alkyl, $C_{1-6}$ haloalkyl, -S- $C_{1-6}$ alkyl, -$C_{0-3}$ alkylene-$C_{2-4}$ alkenyl, $C_{1-6}$ alkoxy, $C_{1-6}$ haloalkoxy and $C_{3-14}$ cycloalkyl, wherein $C_{3-14}$ cycloalkyl, or $C_{1-6}$ alkyl is optionally further substituted by one or more $R_a$;

$X_3$ is selected from the group consisting of C, CH and N;

$X_6$ is selected from the group consisting of $CR_{14}$, $C(R_{14})_2$, O, N and $NR_{14}$; $R_{14}$ is each independently selected from the group consisting of H, hydroxyl, oxo, halogen, cyano, $C_{1-6}$ alkyl, $C_{1-6}$ haloalkyl, -S-$C_{1-6}$ alkyl, -$C_{0-3}$ alkylene-$C_{2-4}$ alkenyl, $C_{1-6}$ alkoxy, $C_{1-6}$ haloalkoxy and $C_{3-14}$ cycloalkyl, wherein $C_{3-14}$ cycloalkyl, or $C_{1-6}$ alkyl is optionally further substituted by one or more $R_a$;

$R_6$ is absent or is selected from the group consisting of $C_{3-14}$ cycloalkyl and 3- to 14-membered heterocyclyl, wherein $C_{3-14}$ cycloalkyl or 3- to 14-membered heterocyclyl is optionally further substituted by one or more $R_a$;

$R_9$ is selected from the group consisting of H, amino, substituted amino, cyano, $C_{1-6}$ alkyl, $C_{1-6}$ alkoxy, halogen, $C_{2-6}$ alkenyl and $C_{3-6}$ cycloalkyl, wherein $C_{1-6}$ alkyl, $C_{2-6}$ alkenyl or $C_{3-6}$ cycloalkyl is optionally further substituted by one or more $R_a$;

$R_{10}$ is absent or is selected from the group consisting of -O- and -$NR_{11}$-; $R_{11}$ is selected from the group consisting of H, $C_{1-6}$ alkyl, $C_{1-6}$ haloalkyl, $C_{1-6}$ hydroxylalkyl and $C_{1-6}$ aminoalkyl;

$R_7$ is selected from the group consisting of $C_{3-14}$ cycloalkyl, 3- to 14-membered heterocyclyl, $C_{6-18}$ aryl and 5- to 18-membered heteroaryl, wherein $C_{3-14}$ cycloalkyl, 3- to 14-membered heterocyclyl, $C_{6-18}$ aryl or 5- to 18-membered heteroaryl is optionally further substituted by one or more $R_a$;

K is selected from the group consisting of $C_{3-14}$ cycloalkyl and 3- to 14-membered heterocyclyl, wherein $C_{3-14}$ cycloalkyl or 3- to 14-membered heterocyclyl is a monocyclic ring, a fused ring, a spiro ring or a bridged ring, optionally further substituted by one or more $R_a$, wherein 3- to 14-membered heterocyclyl is not

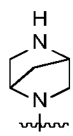, 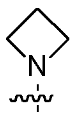, 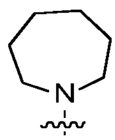, 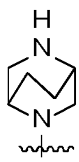, 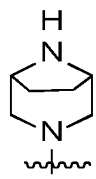,

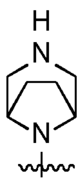 or 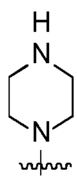;

m and n are each independently selected from the group consisting of 0, 1, 2, 3, 4 and 5;

L is a connection unit connecting F and M;

L is selected from the group consisting of

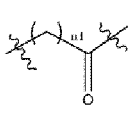, 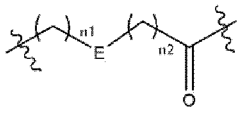, 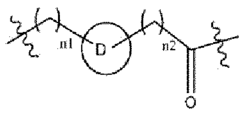, 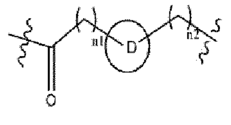,

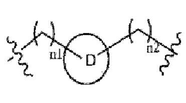, 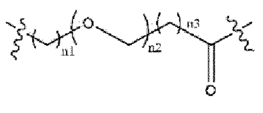, 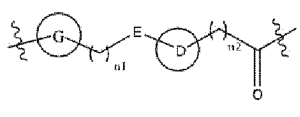, 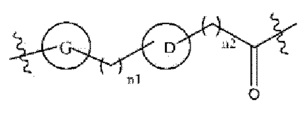,

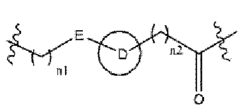, 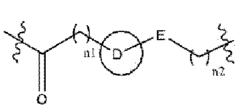, 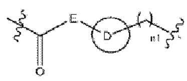, 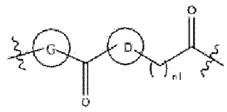, 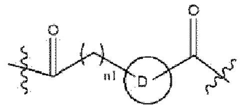,

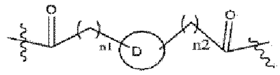, 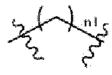, 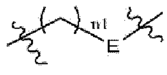, 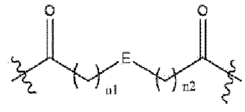, 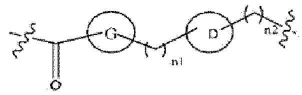,

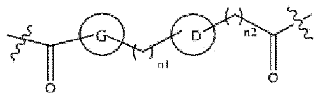, 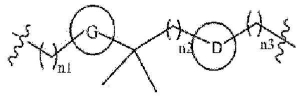, 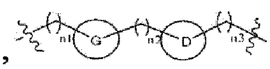, 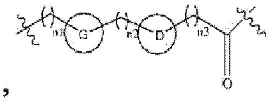,

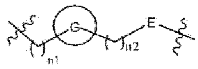, 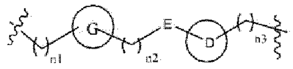, 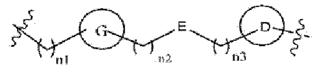, 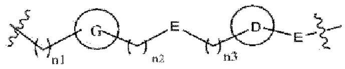,

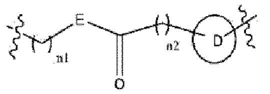, 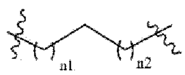, 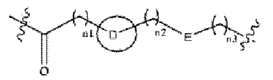, 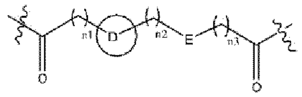,

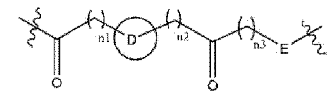, 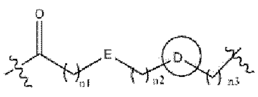, 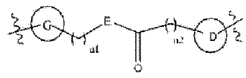 and 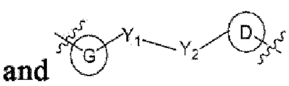;

wherein,

ring G or ring D is each independently selected from the group consisting of $C_{6-14}$ aryl, 5- to 14-membered heteroaryl, $C_{3-14}$ cycloalkyl and 3- to 14-membered heterocyclyl, wherein $C_{6-14}$ aryl, 5- to 14-membered heteroaryl or 3- to 14-membered heterocyclyl is optionally further substituted by one or more $R_a$;

E is each independently selected from the group consisting of -O-, -NH- and $-NCH_3-$;

$Y_1$ and $Y_2$ are each independently absent or selected from the group consisting of -O-, -NH-, 3- to 6-membered N-containing heterocyclyl, $C_{1-6}$ alkylene, carbonyl, acetylenyl and $C_{3-6}$ cycloalkyl;

n1, n2 and n3 are each independently selected from integers ranging from 0 to 10, and preferably n1, n2 and n3 are each independently 0, 1, 2, 3 or 4;

M is a VHL binding fragment represented by general formula (III) or (IV);

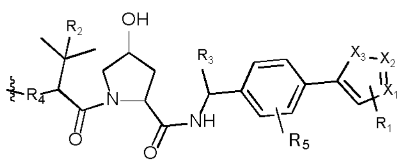

(III)

or

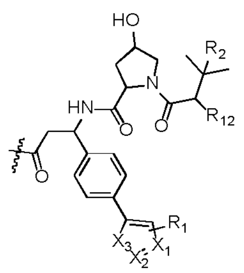

(IV)

$X_1$ is selected from the group consisting of C, CH and N;

$X_2$ is selected from the group consisting of C, CH and N;

$X_3$ is selected from the group consisting of N, NH, O and S;

$R_1$ is selected from the group consisting of H, $C_{1-6}$ alkyl, halogen and cyano, and is preferably methyl, ethyl, F or cyano;

$R_2$ is selected from the group consisting of H and $C_{1-6}$ alkyl, and is preferably H or methyl;

$R_3$ is selected from the group consisting of H, $C_{1-6}$ alkyl, $C_{1-6}$ hydroxylalkyl and $-(CH_2)_n-CO-NH-(CH_2)_n-CH_3$, wherein n is selected from the group consisting of 0, 1, 2 and 3, and $R_3$ is preferably H, methyl, hydroxylmethyl,

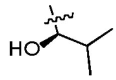

or $-CH_2-CO-NH-CH_3$;

$R_4$ is selected from the group consisting of

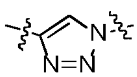, 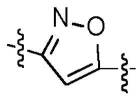

and -NH-;

$R_5$ is selected from the group consisting of H, $C_{1-6}$ alkyl and $C_{1-6}$ haloalkyl;

$R_{12}$ is selected from the group consisting of

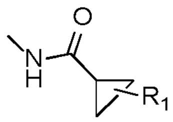 and 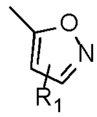 ;

$R_a$ is each independently selected from the group consisting of H, hydroxyl, amino, oxo, cyano, halogen, $C_{1-6}$ alkyl, $C_{1-6}$ haloalkyl, $C_{1-6}$ alkoxy, $-C_{0-3}$ alkylene-$OR_b$, $-OC(=O)C_{1-6}$ alkyl, $-C_{0-3}$ alkylene-$SR_b$, $-C_{0-3}$ alkylene-$N(R_b)_2$, $-C_{0-3}$ alkylene-$S(=O)R_b$, $-C_{0-3}$ alkylene-$S(=O)_2R_3$, $-C_{0-3}$ alkylene-$SR_b$, $-C_{0-3}$ alkylene-$S(R_b)_5$, $-C_{0-3}$ alkylene-$C(=O)R_b$, $-C_{0-3}$ alkylene-$C(=O)OR_b$, $-C_{0-3}$ alkylene-$C(=O)N(R_b)_2$, $C_{2-6}$ alkenyl,

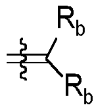,

$C_{2-6}$ alkynyl, $-C_{0-3}$ alkylene-$C_{3-14}$ cycloalkyl, $-C_{0-3}$ alkylene-(3- to 14-membered heterocyclyl), $-C_{0-3}$ alkylene-$C_{6-18}$ aryl and $-C_{0-3}$ alkylene-(5- to 18-membered heteroaryl), wherein $C_{1-6}$ alkyl, $C_{1-6}$ haloalkyl, $C_{1-6}$ alkoxy, $C_{2-6}$ alkenyl, $C_{2-6}$ alkynyl, $-C_{0-3}$ alkylene-$C_{3-14}$ cycloalkyl, $-C_{0-3}$ alkylene-(3- to 14-membered heterocyclyl), $-C_{0-3}$ alkylene-$C_{6-18}$ aryl or $-C_{0-3}$ alkylene-(5- to 18-membered heteroaryl) is optionally further substituted by one or more $R_b$; and

each $R_b$ is independently H, halogen, hydroxyl, cyano, $C_{1-6}$ alkyl, $C_{3-6}$ cycloalkyl, $C_{1-6}$ haloalkyl, $C_{3-14}$ cycloalkyl, 3- to 14-membered heterocyclyl, $C_{6-18}$ aryl or 5- to 18-membered heteroaryl, wherein $C_{1-6}$ alkyl, $C_{3-6}$ cycloalkyl, $C_{1-6}$ haloalkyl, $C_{3-14}$ cycloalkyl, 3- to 14-membered heterocyclyl, $C_{6-18}$ aryl or 5- to 18-membered heteroaryl is optionally further substituted by one or more halogen, $C_{1-6}$ alkyl or $C_{1-6}$ haloalkyl; or two $R_b$ bound to one atom together with the atom to which they are bound form $C_{3-14}$ cycloalkyl, 3- to 14-membered heterocyclyl, $C_{6-18}$ aryl or 5- to 18-membered heteroaryl, wherein $C_{3-14}$ cycloalkyl, 3- to 14-membered heterocyclyl, $C_{6-18}$ aryl or 5- to 18-membered heteroaryl is optionally further substituted by one or more halogen, amino, hydroxy, cyano, $C_{1-6}$ alkyl, $C_{3-6}$ cycloalkyl or $C_{1-6}$ haloalkyl.

In some embodiments of the present application, L is selected from the group consisting of

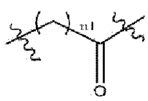, 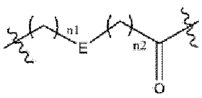, 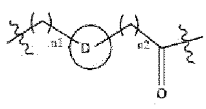,

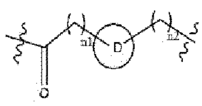, 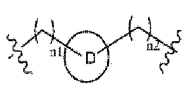, 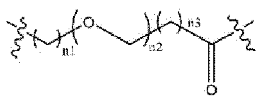, 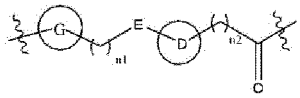,

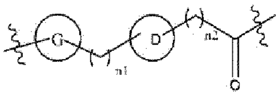 , 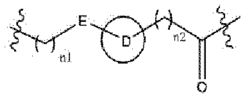 , 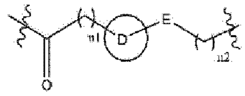 , 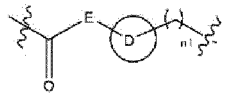 ,

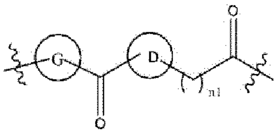 , 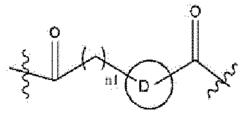 , 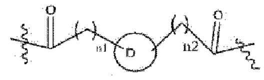 , 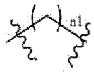 , 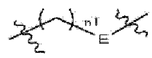 ,

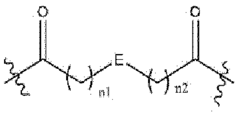 , 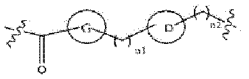 , 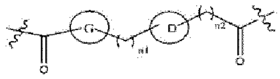 , 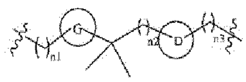 ,

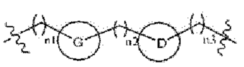 , 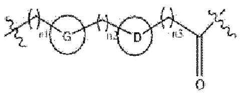 , 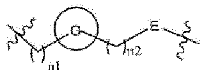 , 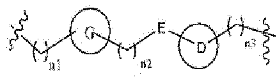 ,

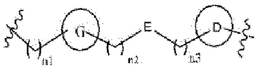 , 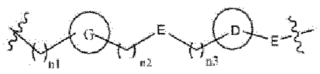 , 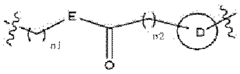 , 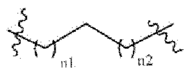 ,

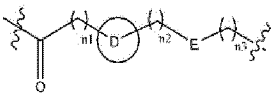 , 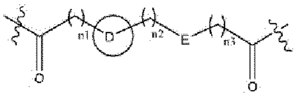 , 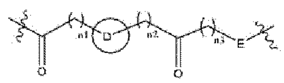 , 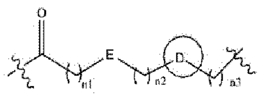

and

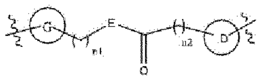 ;

wherein,

ring G or ring D is each independently selected from the group consisting of $C_{6-14}$ aryl, 5- to 14-membered heteroaryl, $C_{3-14}$ cycloalkyl and 3- to 14-membered heterocyclyl, wherein $C_{6-14}$ aryl, 5- to 14-membered heteroaryl or 3- to 14-membered heterocyclyl is optionally further substituted by one or more $R_a$;

E is each independently selected from the group consisting of -O-, -NH- and $-NCH_3-$;

n1, n2 and n3 are each independently selected from integers ranging from 0 to 10, and preferably n1, n2 and n3 are each independently 0, 1, 2, 3 or 4;

M is a VHL binding fragment represented by general formula (V) or (IV);

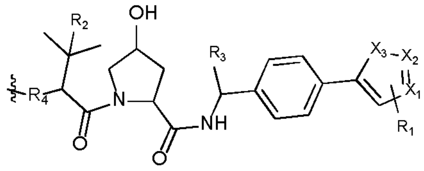

(V)

or

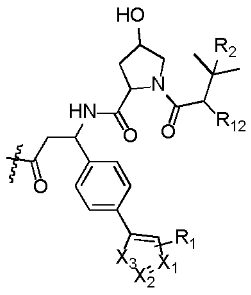

(IV)

$X_1$ is selected from the group consisting of C, CH and N;

$X_2$ is selected from the group consisting of C, CH and N;

$X_3$ is selected from the group consisting of N, NH, O and S;

$R_1$ is selected from the group consisting of H, $C_{1-6}$ alkyl, halogen and cyano, and is preferably methyl, ethyl, F or cyano;

$R_2$ is selected from the group consisting of H and $C_{1-6}$ alkyl, and is preferably H or methyl;

$R_3$ is selected from the group consisting of H, $C_{1-6}$ alkyl and $C_{1-6}$ hydroxylalkyl, and is preferably H, methyl or hydroxylmethyl;

$R_4$ is selected from the group consisting of

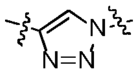

,

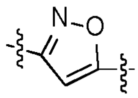

and -NH-; and

$R_{12}$ is selected from the group consisting of

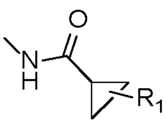

and

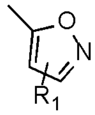

.

In some embodiments of the present application, L is selected from the group consisting of

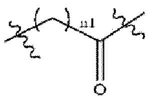

,

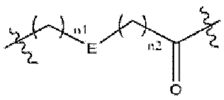

,

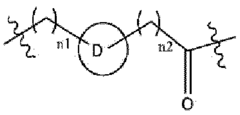

,

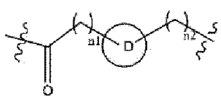

,

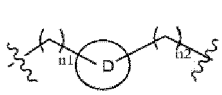

,

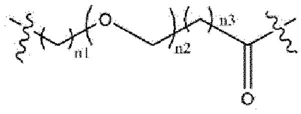

,

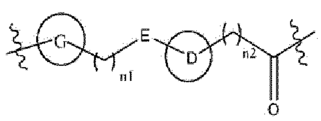

,

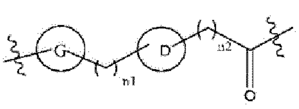

,

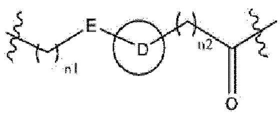

,

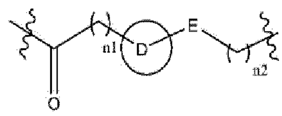

,

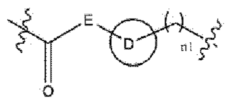, 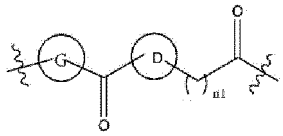, 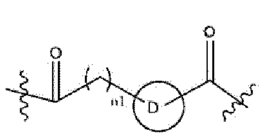, 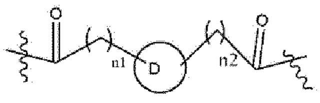,

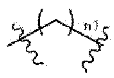, 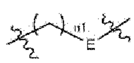, 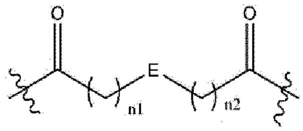, 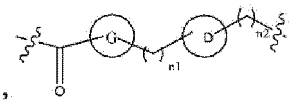, 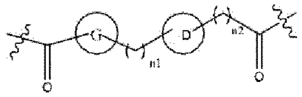,

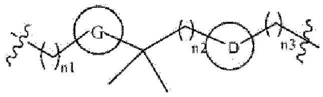, 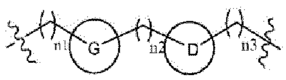, 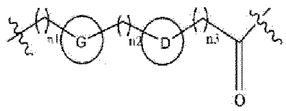, 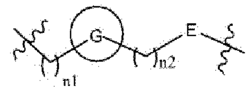,

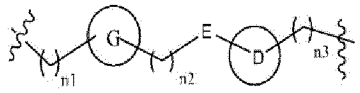, 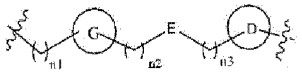, 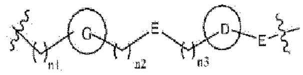,

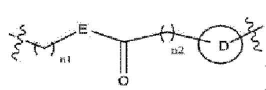 and 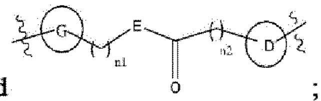;

ring G or ring D is each independently selected from the group consisting of $C_{6-14}$ aryl, 5- to 14-membered heteroaryl and 3- to 14-membered heterocyclyl, wherein $C_{6-14}$ aryl, 5- to 14-membered heteroaryl or 3- to 14-membered heterocyclyl is optionally further substituted by one or more $R_a$;

E is each independently selected from the group consisting of -O-, -NH- and $-NCH_3-$;

n1, n2 and n3 are each independently selected from integers ranging from 0 to 10, and preferably n1, n2 and n3 are each independently 0, 1, 2, 3 or 4;

M is a VHL binding fragment represented by general formula (V) or (IV);

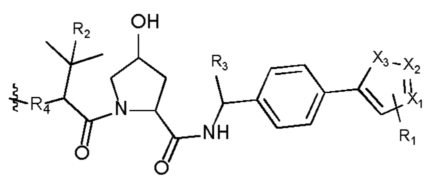

(V) or

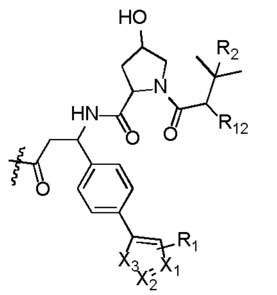

(IV)

$X_1$ is selected from the group consisting of C, CH and N;

$X_2$ is selected from the group consisting of C, CH and N;

$X_3$ is selected from the group consisting of N, NH, O and S;

$R_1$ is selected from the group consisting of H, $C_{1-6}$ alkyl, halogen and cyano, and is preferably methyl, ethyl, F or cyano;

$R_2$ is selected from the group consisting of H and $C_{1-6}$ alkyl, and is preferably H or methyl;

$R_3$ is selected from the group consisting of H, $C_{1-6}$ alkyl and $C_{1-6}$ hydroxylalkyl, and is preferably H, methyl or hydroxylmethyl;

$R_4$ is selected from the group consisting of

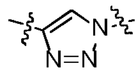, 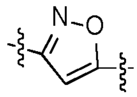

and -NH-;

$R_{12}$ is selected from the group consisting of

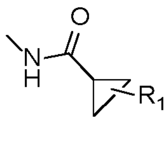 and 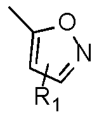.

$R_a$ is independently selected from the group consisting of H, hydroxyl, amino, oxo, cyano, halogen, $C_{1-6}$ alkyl, $C_{1-6}$ haloalkyl, $C_{1-6}$ alkoxy, -$C_{0-3}$ alkylene-$OR_b$, -OC(=O)$C_{1-6}$ alkyl, -$C_{0-3}$ alkylene-$SR_b$, -$C_{0-3}$ alkylene-N$(R_b)_2$, -$C_{0-3}$ alkylene-S(=O)$R_b$, -$C_{0-3}$ alkylene-S$(=O)_2R_3$, -$C_{0-3}$ alkylene-$SR_b$, -$C_{0-3}$ alkylene-S$(R_b)_5$, -$C_{0-3}$ alkylene-C(=O)$R_b$, -$C_{0-3}$ alkylene-C(=O)$OR_b$, -$C_{0-3}$ alkylene-C(=O)N$(R_b)_2$, $C_{2-6}$ alkenyl, $C_{2-6}$ alkynyl, -$C_{0-3}$ alkylene-$C_{3-14}$ cycloalkyl, -$C_{0-3}$ alkylene-(3- to 14-membered heterocyclyl), -$C_{0-3}$ alkylene-$C_{6-18}$ aryl and -$C_{0-3}$ alkylene-(5- to 18-membered heteroaryl), wherein $C_{1-6}$ alkyl, $C_{1-6}$ haloalkyl, $C_{1-6}$ alkoxy, $C_{2-6}$ alkenyl, $C_{2-6}$ alkynyl, -$C_{0-3}$ alkylene-$C_{3-14}$ cycloalkyl, -$C_{0-3}$ alkylene-(3- to 14-membered heterocyclyl), -$C_{0-3}$ alkylene-$C_{6-18}$ aryl or -$C_{0-3}$ alkylene-(5- to 18-membered heteroaryl) is optionally further substituted by one or more $R_b$; and

each $R_b$ is independently H, halogen, hydroxyl, cyano, $C_{1-6}$ alkyl, $C_{3-6}$ cycloalkyl, $C_{1-6}$ haloalkyl, $C_{3-14}$ cycloalkyl, 3- to 14-membered heterocyclyl, $C_{6-18}$ aryl or 5- to 18-membered heteroaryl, wherein $C_{1-6}$ alkyl, $C_{3-6}$ cycloalkyl, $C_{1-6}$ haloalkyl, $C_{3-14}$ cycloalkyl, 3- to 14-membered heterocyclyl, $C_{6-18}$ aryl or 5- to 18-membered heteroaryl is optionally further substituted by one or more halogen, $C_{1-6}$ alkyl or $C_{1-6}$ haloalkyl; or two $R_b$ bound to one atom together with the atom to which they are bound form $C_{3-14}$ cycloalkyl, 3- to 14-membered heterocyclyl, $C_{6-18}$ aryl or 5- to 18-membered heteroaryl, wherein $C_{3-14}$ cycloalkyl, 3- to 14-membered heterocyclyl, $C_{6-18}$ aryl or 5- to 18-membered heteroaryl is optionally further substituted by one or more halogen, amino, hydroxy, cyano, $C_{1-6}$ alkyl, $C_{3-6}$ cycloalkyl or $C_{1-6}$ haloalkyl.

In some embodiments of the present application, F is a KRAS protein binding fragment represented by general formula (I) or (II);

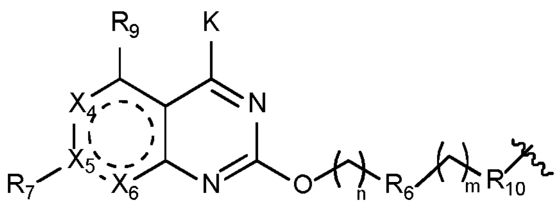

(I)

or

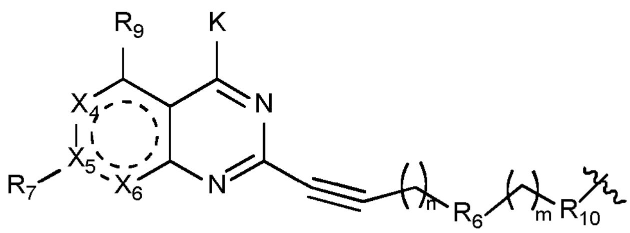

(II)

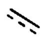

represents a bond independently selected from the group consisting of a single bond and a double bond;

$X_4$ is $CR_8$, $C(R_8)_2$, O, N or $NR_8$; $R_8$ is each independently selected from the group consisting of H, hydroxyl, oxo, halogen, cyano, $C_{1-6}$ alkyl, $C_{1-6}$ haloalkyl, -S-$C_{1-6}$ alkyl, -$C_{0-3}$ alkylene-$C_{2-4}$ alkenyl, $C_{1-6}$ alkoxy, $C_{1-6}$ haloalkoxy and $C_{3-14}$ cycloalkyl, wherein $C_{3-14}$ cycloalkyl or $C_{1-6}$ alkyl is optionally further substituted by one or more $R_a$;

$X_3$ is selected from the group consisting of C, CH and N;

$X_6$ is selected from the group consisting of $CR_{14}$, $C(R_{14})_2$, O, N and $NR_{14}$; $R_{14}$ is each independently selected from the group consisting of H, hydroxyl, oxo, halogen, cyano, $C_{1-6}$ alkyl, $C_{1-6}$ haloalkyl, -S-$C_{1-6}$ alkyl, -$C_{0-3}$ alkylene-$C_{2-4}$ alkenyl, $C_{1-6}$ alkoxy, $C_{1-6}$ haloalkoxy and $C_{3-14}$ cycloalkyl, wherein $C_{3-14}$ cycloalkyl or $C_{1-6}$ alkyl is optionally further substituted by one or more $R_a$;

$R_6$ is absent or is selected from the group consisting of $C_{3-14}$ cycloalkyl and 3- to 14-membered heterocyclyl, wherein $C_{3-14}$ cycloalkyl or 3- to 14-membered heterocyclyl is optionally further substituted by one or more $R_a$;

$R_9$ is selected from the group consisting of H, amino, substituted amino, cyano, $C_{1-6}$ alkyl, $C_{1-6}$ alkoxy, halogen, $C_{2-6}$ alkenyl and $C_{3-6}$ cycloalkyl, wherein $C_{1-6}$ alkyl, $C_{2-6}$ alkenyl or $C_{3-6}$ cycloalkyl is optionally further substituted by one or more $R_a$;

$R_{10}$ is absent or is selected from the group consisting of -O- and -$NR_{11}$-; $R_{11}$ is selected from the group consisting of H, $C_{1-6}$ alkyl, $C_{1-6}$ haloalkyl, $C_{1-6}$ hydroxylalkyl and $C_{1-6}$ aminoalkyl;

$R_7$ is selected from the group consisting of $C_{3-14}$ cycloalkyl, 3- to 14-membered heterocyclyl, $C_{6-18}$ aryl and 5- to 18-membered heteroaryl, wherein $C_{3-14}$ cycloalkyl, 3- to 14-membered heterocyclyl, $C_{6-18}$ aryl or 5- to 18-membered heteroaryl is optionally further substituted by one or more $R_a$;

K is selected from the group consisting of $C_{3-14}$ cycloalkyl and 3- to 14-membered heterocyclyl, wherein $C_{3-14}$ cycloalkyl or 3- to 14-membered heterocyclyl is a monocyclic ring, a fused ring, a spiro ring or a bridged ring, optionally further substituted by one or more $R_a$, wherein 3- to 14-membered heterocyclyl is not

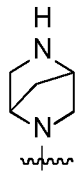, 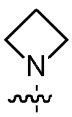, 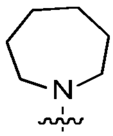, 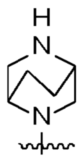, 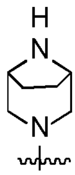, 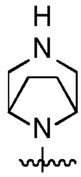

or

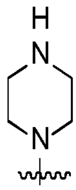;

and

m and n are each independently selected from the group consisting of 0, 1, 2, 3, 4 and 5.

In some embodiments of the present application, $R_4$ is selected from the group consisting of

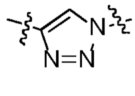

and -NH-.

In some embodiments of the present application, $R_4$ is

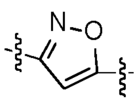.

In some embodiments of the present application, K is selected from the group consisting of $C_{3-14}$ cycloalkyl and 3- to 14-membered heterocyclyl, wherein $C_{3-14}$ cycloalkyl or 3- to 14-membered heterocyclyl is a monocyclic ring and is optionally further substituted by one or more $R_a$.

Further, K is selected from the group consisting of

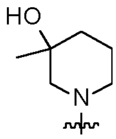, 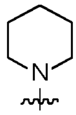, 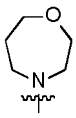, 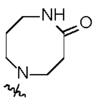,

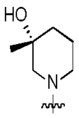, 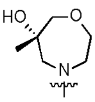, 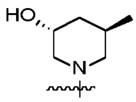, 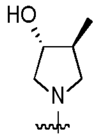, 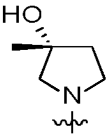, 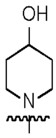, 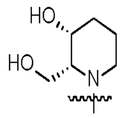, 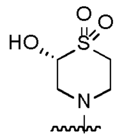, 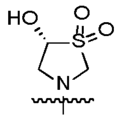,

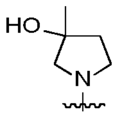 and 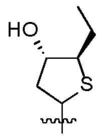.

In some embodiments of the present application, K is $C_{3-14}$ cycloalkyl or 3- to 14-membered heterocyclyl, wherein $C_{3-14}$ cycloalkyl or 3- to 14-membered heterocyclyl is a spiro ring and is optionally further substituted by one or more $R_a$.

Further, K is selected from the group consisting of

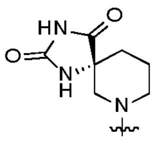, 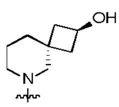, 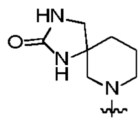,

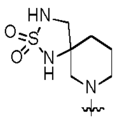, 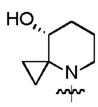, 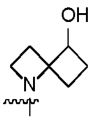, 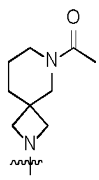, 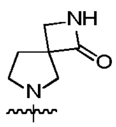, 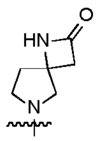, 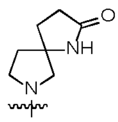, 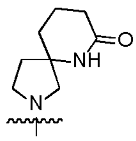, 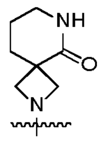,

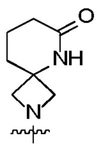 and 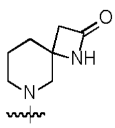.

In some embodiments of the present application, K is $C_{3-14}$ cycloalkyl or 3- to 14-membered heterocyclyl, wherein $C_{3-14}$ cycloalkyl or 3- to 14-membered heterocyclyl is a fused ring and is optionally further substituted by one or more $R_a$.

Further, K is selected from the group consisting of

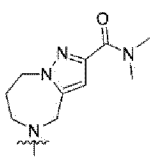, 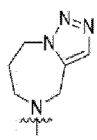, 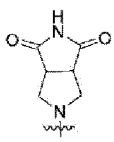, 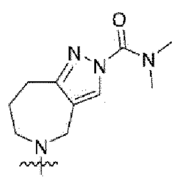, 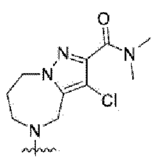,

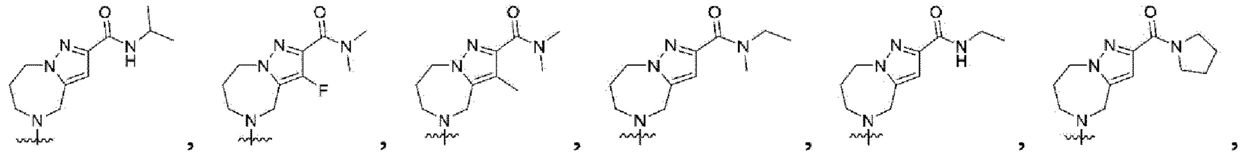
, , , , , ,

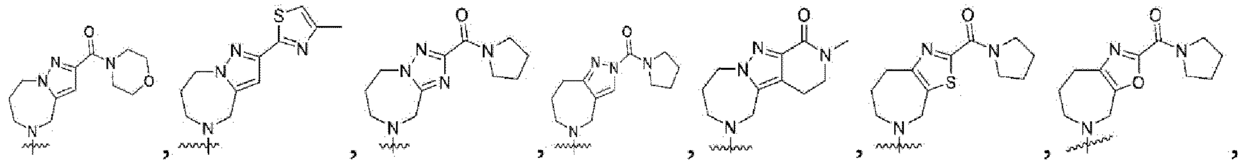
, , , , , , ,

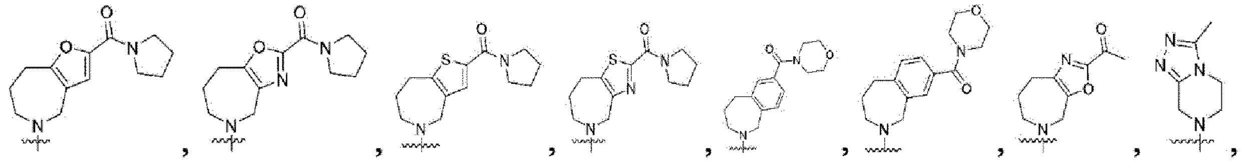
, , , , , , , ,

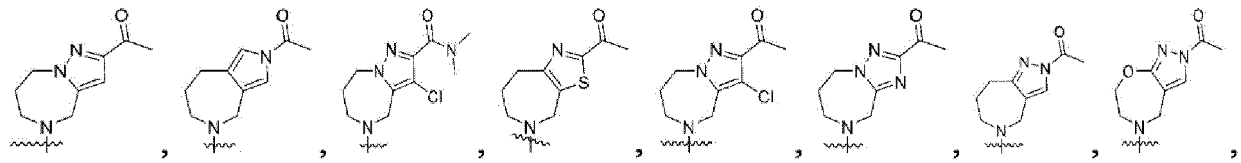
, , , , , , , ,

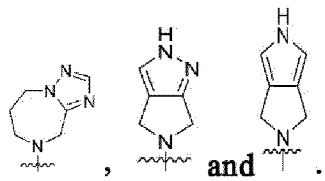
, and .

In some embodiments of the present application, K is $C_{3-14}$ cycloalkyl or 3- to 14-membered heterocyclyl, wherein $C_{3-14}$ cycloalkyl or 3- to 14-membered heterocyclyl is a bridged ring and is optionally further substituted by one or more $R_a$.

Further, K is selected from the group consisting of

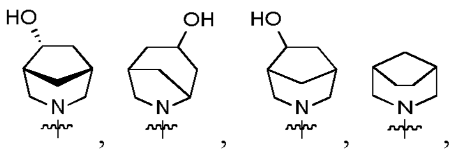
, , , ,

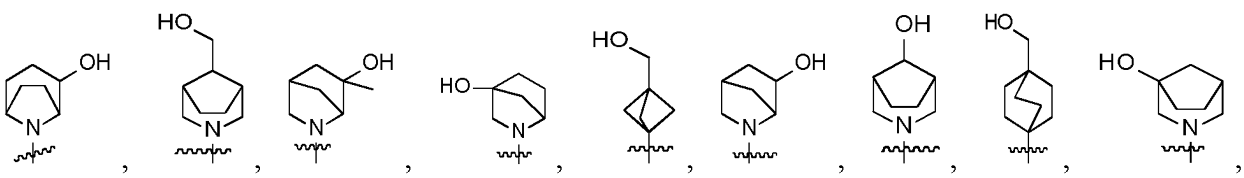
, , , , , , , , ,

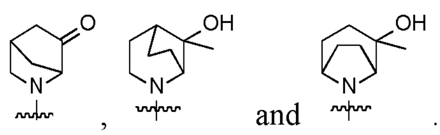
, and .

In some embodiments of the present application, K is selected from the group consisting of

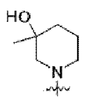, 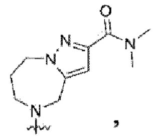, 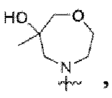, 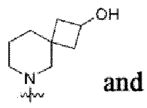 and 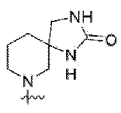.

In some embodiments of the present application, $R_7$ is selected from the group consisting of $C_{6-18}$ aryl and 5- to 18-membered heteroaryl, wherein $C_{6-18}$ aryl or 5- to 18-membered heteroaryl is optionally further substituted by one or more $R_a$, wherein $R_a$ is each independently selected from the group consisting of H, halogen, hydroxyl, amino, cyano, $C_{2-6}$ alkynyl, $C_{1-6}$ alkyl, $C_{1-6}$ haloalkyl and $C_{2-6}$ alkenyl.

In some embodiments of the present application, $R_7$ is selected from the group consisting of

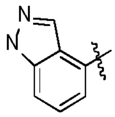, 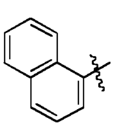, 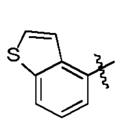, 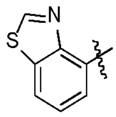, 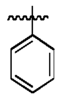, 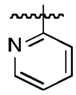, 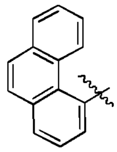, 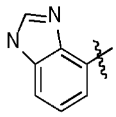, 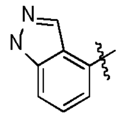,

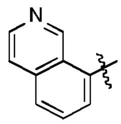 and 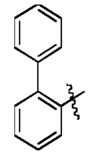,

wherein

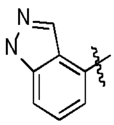, 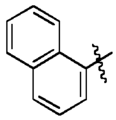, 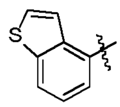, 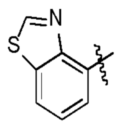, 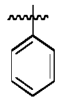, 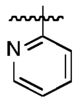,

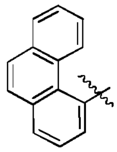, 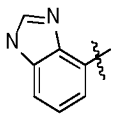, 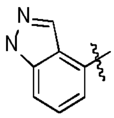, 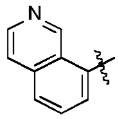 or 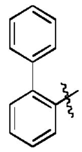

is optionally further substituted by one or more $R_a$, wherein $R_a$ is each independently selected from the group consisting of H, halogen, hydroxyl, amino, cyano, $C_{2-6}$ alkynyl, $C_{1-6}$ alkyl, $C_{1-6}$ haloalkyl and $C_{2-6}$ alkenyl.

In some embodiments of the present application, $R_7$ is selected from the group consisting of

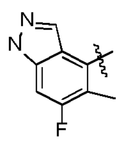, 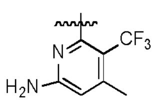, 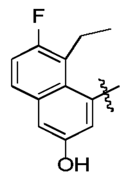, 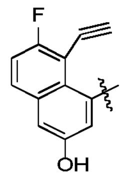, 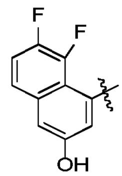, 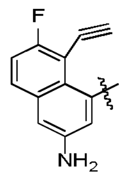, 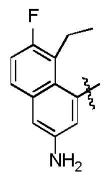, 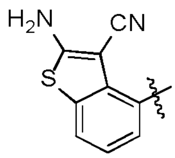,

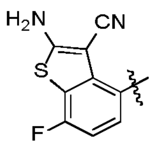, 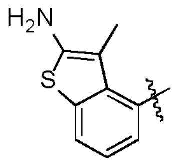 and 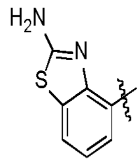.

In some embodiments of the present application, F is selected from the group consisting of

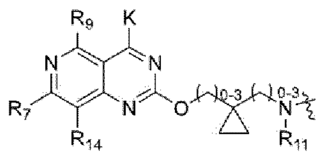,
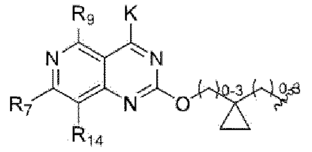,
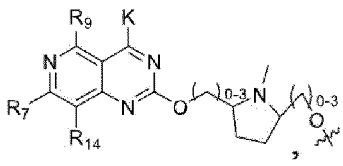,

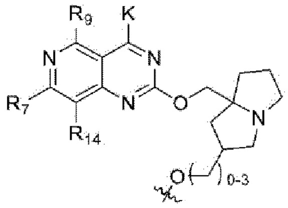,
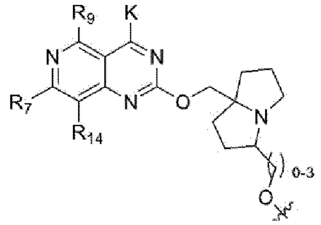,
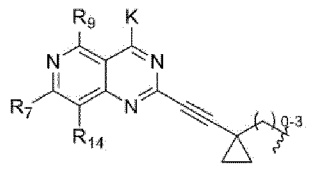,

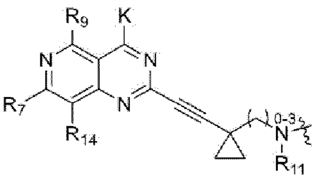,
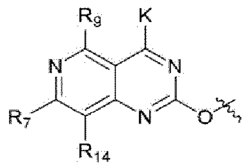,
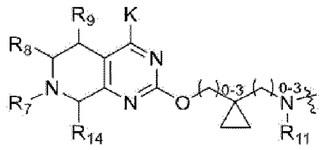,

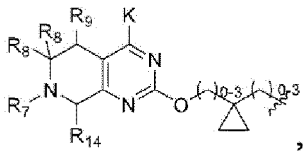,
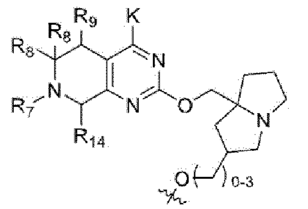,
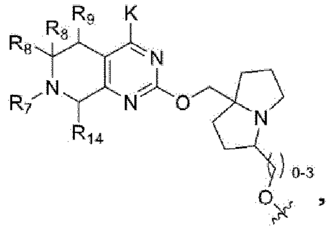,

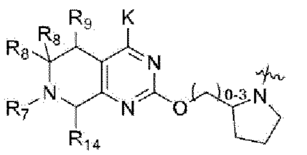,
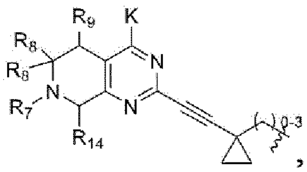,
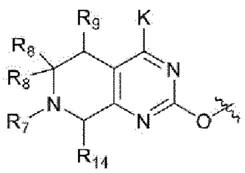,

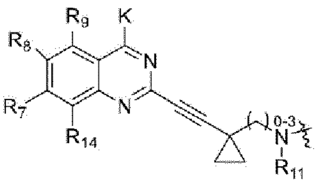,
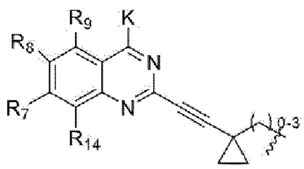,
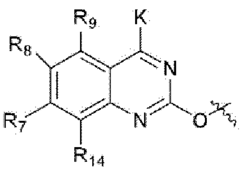,

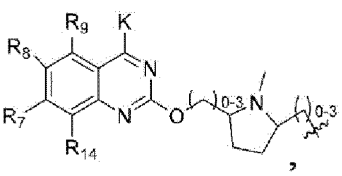,
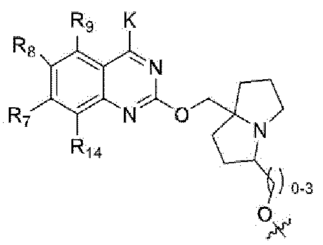,
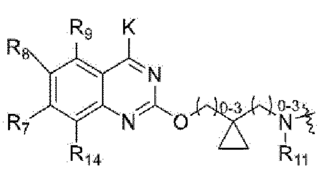,

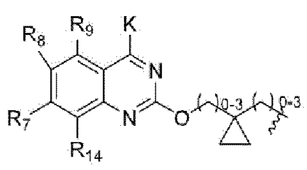 , 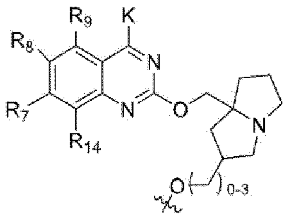 and 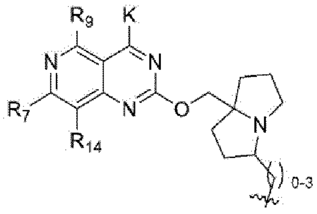 ,

wherein, the definitions of $R_7$, $R_9$, $R_8$, $R_{11}$ and $R_{14}$ are as defined in general formula (I) or (II), wherein K is selected from the group consisting of.

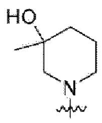 , 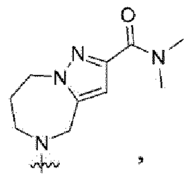 , 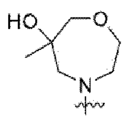 , 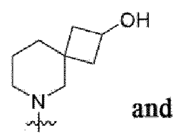 and 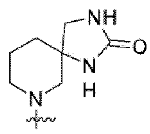 .

In some embodiments of the present application, K is selected from the group consisting of

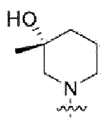 , 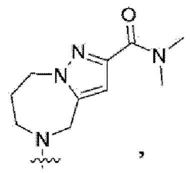 , 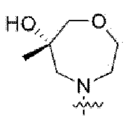 , 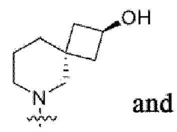 and 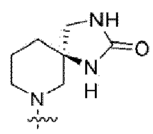 .

In some embodiments of the present application, F is selected from the group consisting of

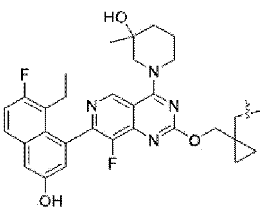 , 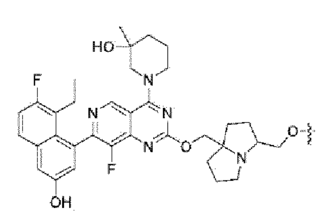 , 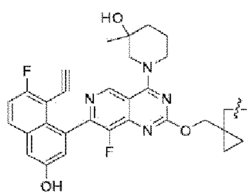 , 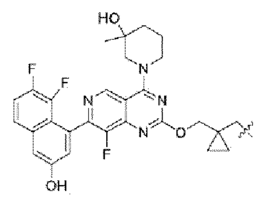 ,

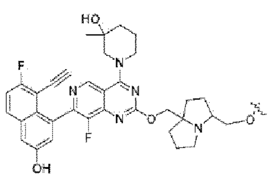 , 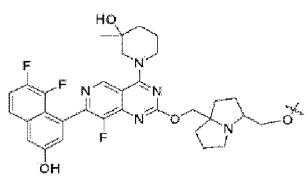 , 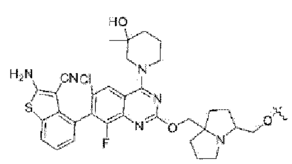 , 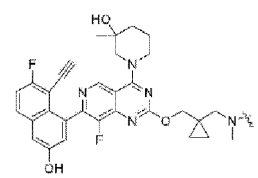 ,

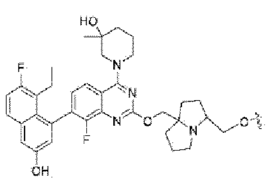 , 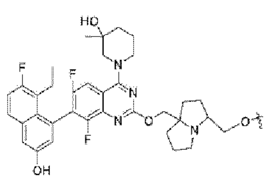 , 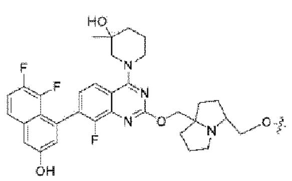 , 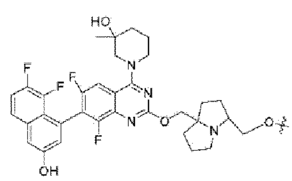 ,

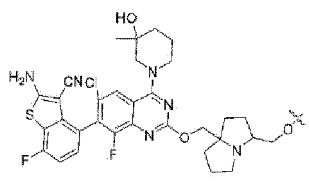, 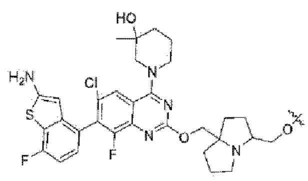, 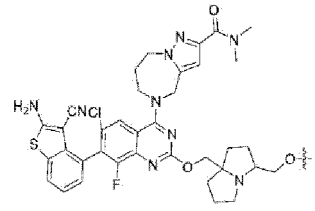, 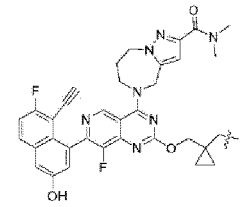,

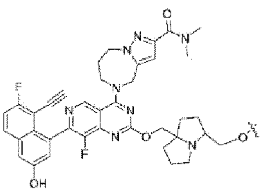, 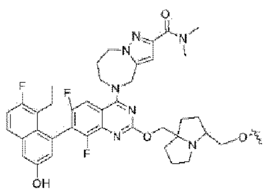, 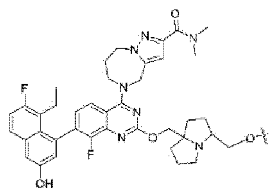, 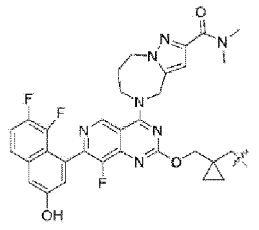,

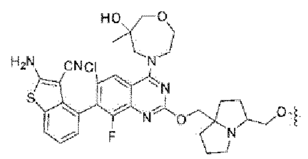, 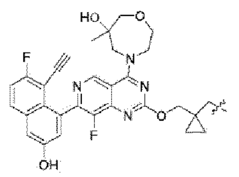, 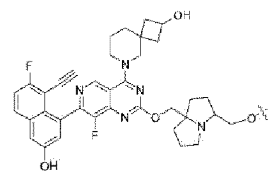, 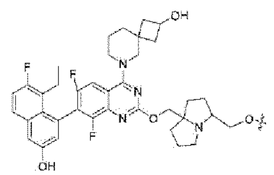,

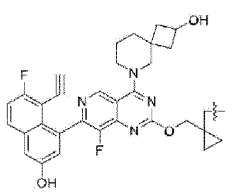, 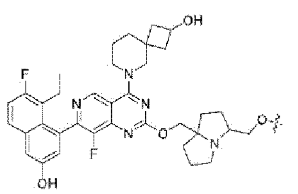, 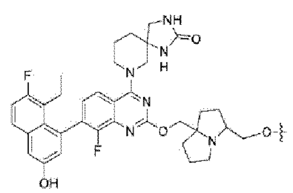, 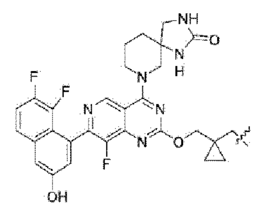,

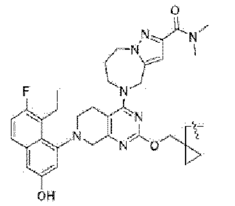, 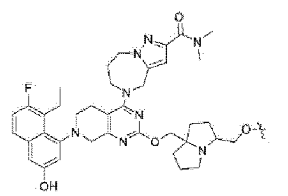, 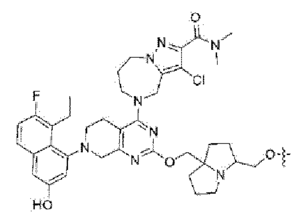, 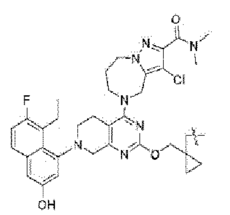,

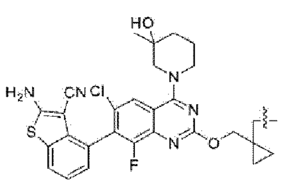, 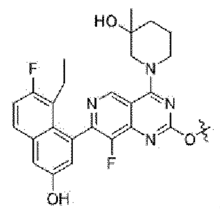, 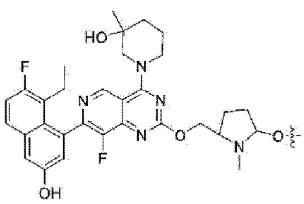, 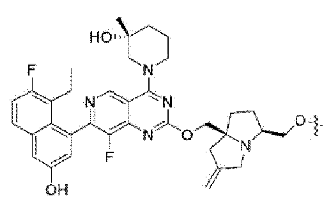,

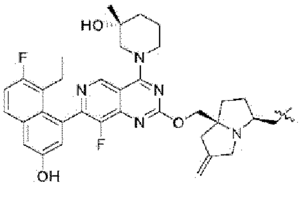, 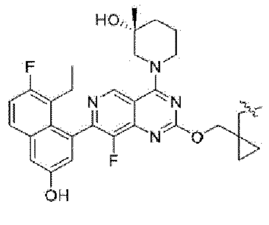, 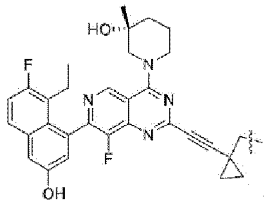 and 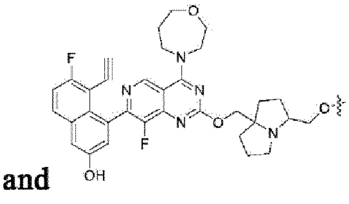.

In some embodiments of the present application, F is selected from the group consisting of

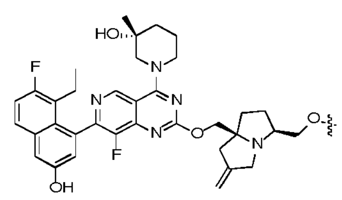, 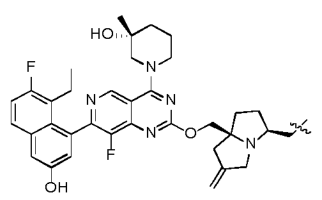, 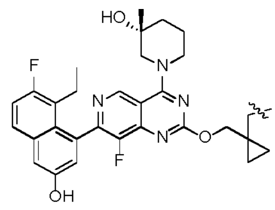

and

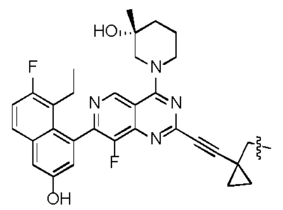.

In some embodiments of the present application, ring G or ring D in L is each independently selected from the group consisting of $C_{6-14}$ aryl, 5- to 14-membered heteroaryl, $C_{3-14}$ cycloalkyl and 3- to 14-membered heterocyclyl, wherein $C_{6-14}$ aryl, 5- to 14-membered heteroaryl, $C_{3-14}$ cycloalkyl or 3- to 14-membered heterocyclyl is optionally further substituted by halogen or $C_{1-3}$ alkyl;

$C_{6-14}$ aryl is preferably

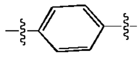;

5- to 14-membered heteroaryl is preferably selected from the group consisting of

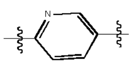, 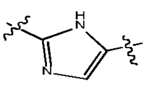, 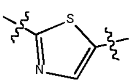, 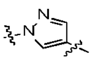, 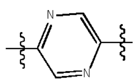, 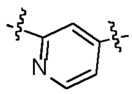, 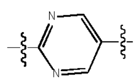,

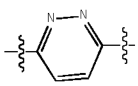, 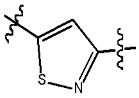, 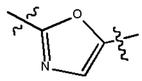 and 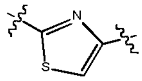;

3- to 14-membered heterocyclyl is preferably selected from the group consisting of

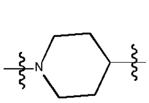, 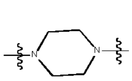, 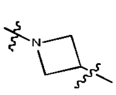, 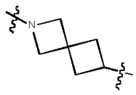, 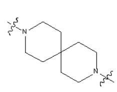, 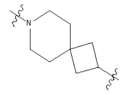, 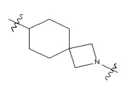, 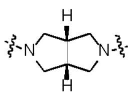,

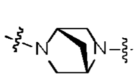, 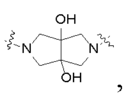, 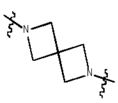 and 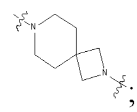;

and

$C_{3-14}$ cycloalkyl is preferably selected from the group consisting of

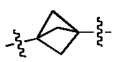

and

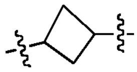

.

In some embodiments of the present application, ring G or ring D in L is each independently selected from the group consisting of $C_{6-14}$ aryl, 5- to 14-membered heteroaryl, $C_{3-14}$ cycloalkyl and 3- to 14-membered heterocyclyl, wherein $C_{6-14}$ aryl, 5- to 14-membered heteroaryl, $C_{3-14}$ cycloalkyl or 3- to 14-membered heterocyclyl is optionally further substituted by halogen or $C_{1-3}$ alkyl;

$C_{6-14}$ aryl is preferably

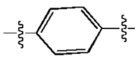

;

5- to 14-membered heteroaryl is preferably selected from the group consisting of

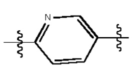 , 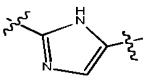 , 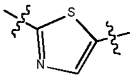 , 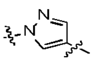 , 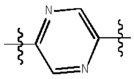 , 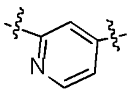 , 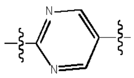 , 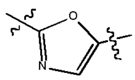

and

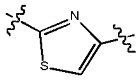

;

3- to 14-membered heterocyclyl is preferably selected from the group consisting of

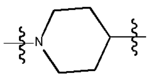 , 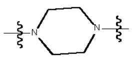 , 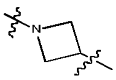 , 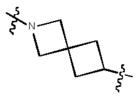 , 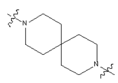 , 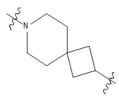 , 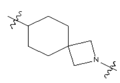 , 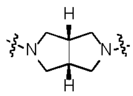 ,

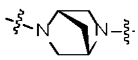 , 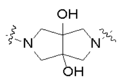 , 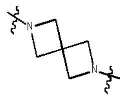 and 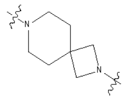 ;

and

$C_{3-14}$ cycloalkyl is preferably selected from the group consisting of

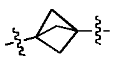

and

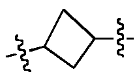

.

In some embodiments of the present application, ring G or ring D in L is each independently selected from the group consisting of $C_{6-14}$ aryl, 5- to 14-membered heteroaryl and 3- to 14-membered heterocyclyl, wherein $C_{6-14}$ aryl, 5- to 14-membered heteroaryl or 3- to 14-membered heterocyclyl is optionally further substituted by halogen or $C_{1-3}$ alkyl;

$C_{6-14}$ aryl is preferably

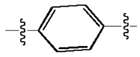;

5- to 14-membered heteroaryl is preferably selected from the group consisting of

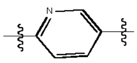, 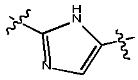, 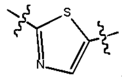, 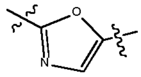 and 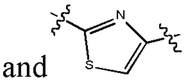;

and

3- to 14-membered heterocyclyl is preferably selected from the group consisting of

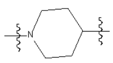, 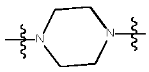, 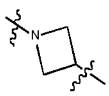, 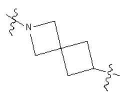, 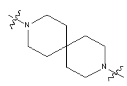, 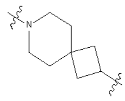, 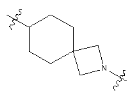, 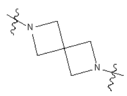,

and

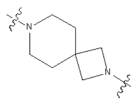.

In some embodiments of the present application, L is selected from the group consisting of

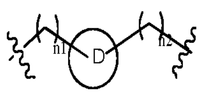, 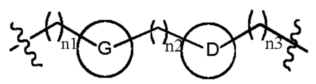, 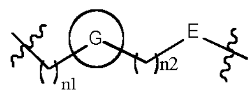, 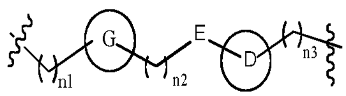,

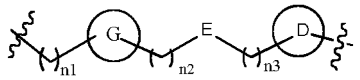, 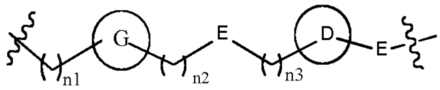 and 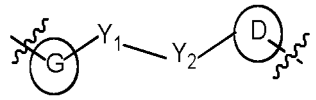;

wherein,

ring G or ring D is each independently selected from the group consisting of $C_{6-14}$ aryl, 5- to 14-membered heteroaryl, $C_{3-14}$ cycloalkyl and 3- to 14-membered heterocyclyl, wherein $C_{6-14}$ aryl, 5- to 14-membered heteroaryl or 3- to 14-membered heterocyclyl is optionally further substituted by one or more $R_a$, wherein $R_a$ is independently selected from the group consisting of H, hydroxyl, amino, oxo, cyano, halogen, $C_{1-6}$ alkyl, $C_{1-6}$ haloalkyl and $C_{1-6}$ alkoxy.

$Y_1$ and $Y_2$ are each independently absent or selected from the group consisting of -O-, -NH-, 3- to 6-membered N-containing heterocyclyl, $C_{1-6}$ alkylene, carbonyl, acetylenyl and $C_{3-6}$ cycloalkyl;

E is each independently selected from the group consisting of -O-, -NH- and $-NCH_3-$; and

n1, n2 and n3 are each independently selected from integers ranging from 0 to 10, and preferably n1, n2 and n3 are each independently 0, 1, 2, 3 or 4.

In some embodiments of the present application, L is selected from the group consisting of the following structures:

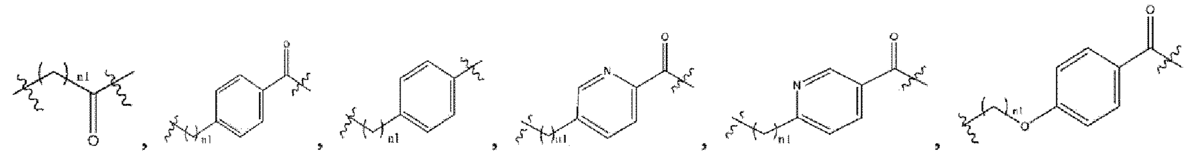
, , , , , ,

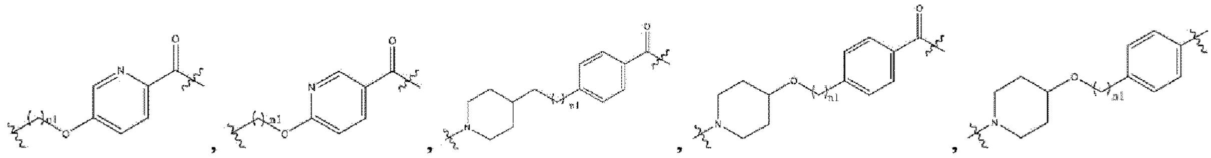
, , , , ,

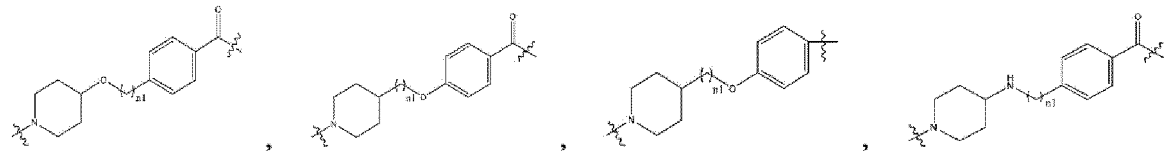
, , , ,

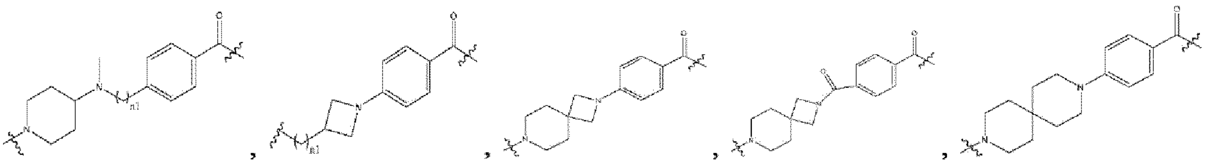
, , , , ,

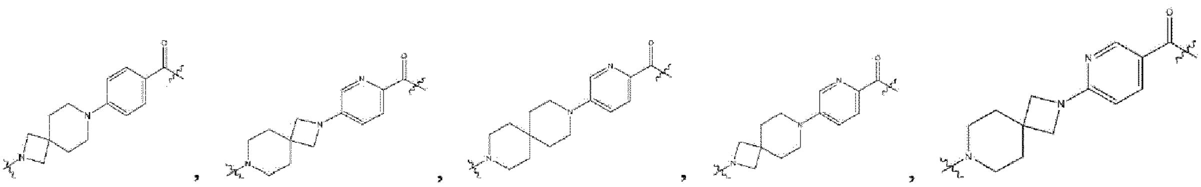
, , , , ,

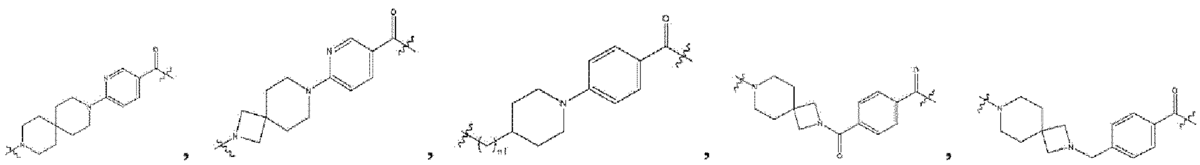
, , , , ,

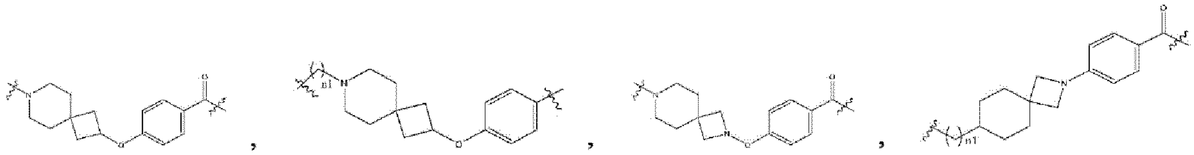
, , , ,

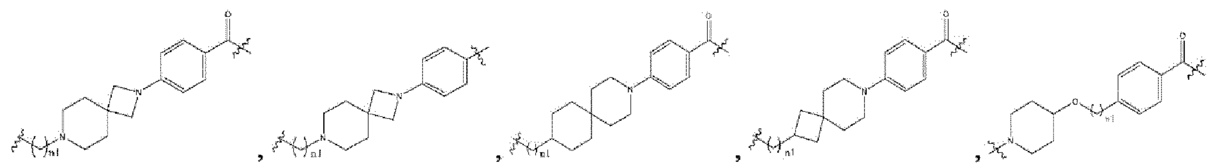
, , , , ,

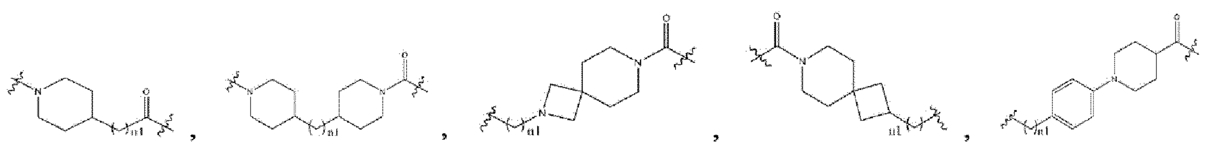
, , , , ,

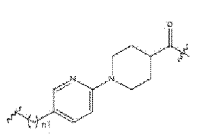 , 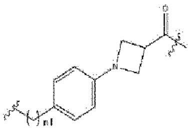 , 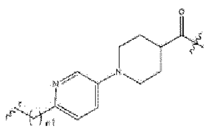 , 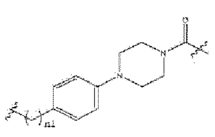 , 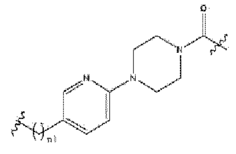 ,

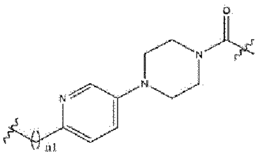 , 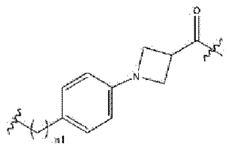 , 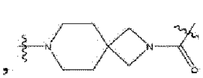 , 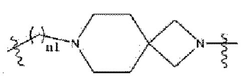 , 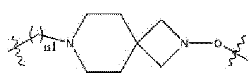 ,

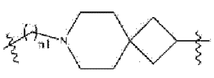 , 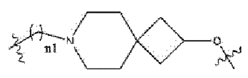 , 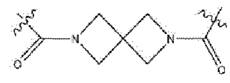 , 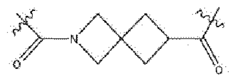 , 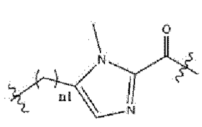 ,

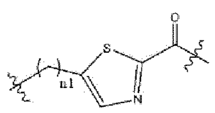 , 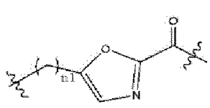 , 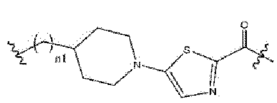 , 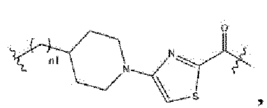 , 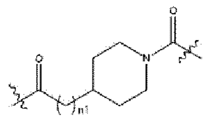 ,

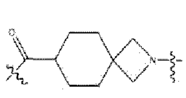 , 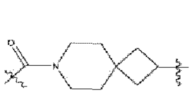 , 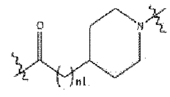 , 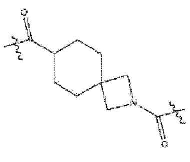 , 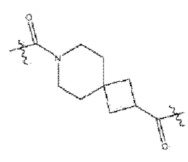 , 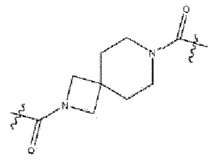 ,

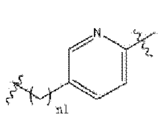 , 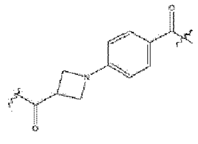 , 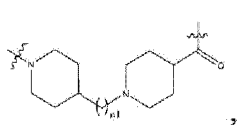 , 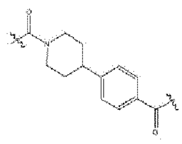 , 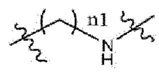 , 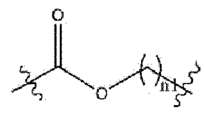 ,

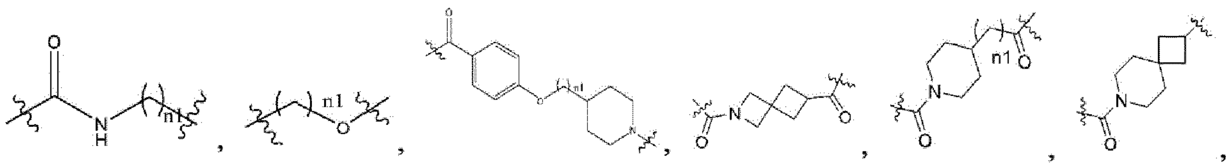 , , , , , ,

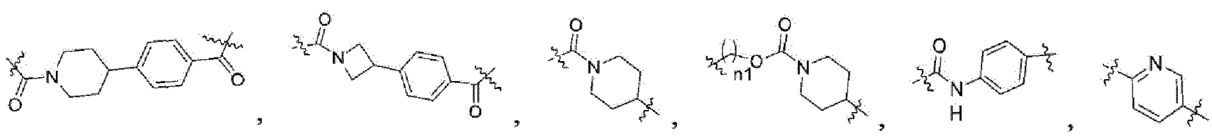 , , , , , ,

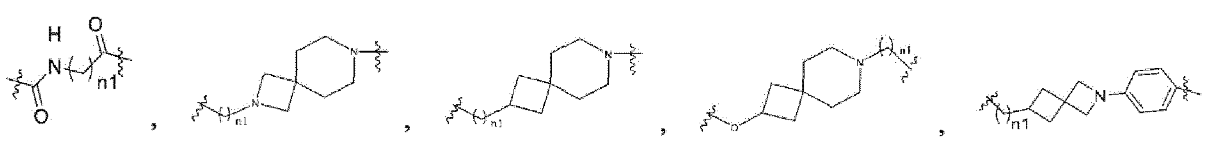 , , , , ,

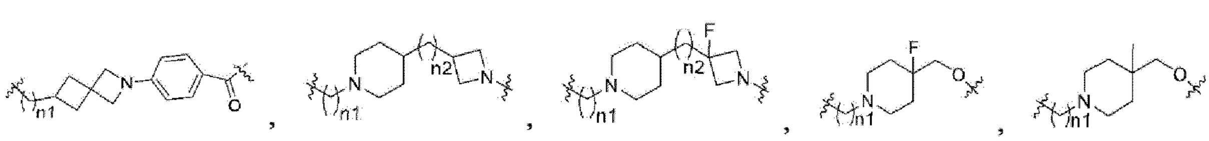 , , , , ,

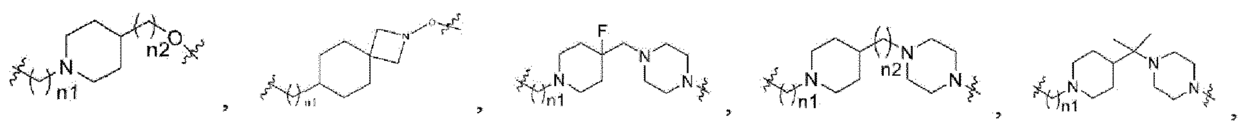

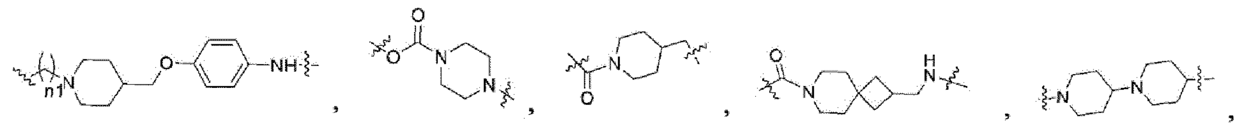

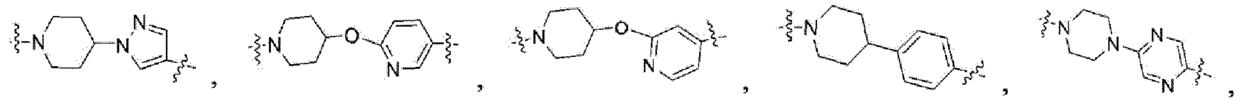

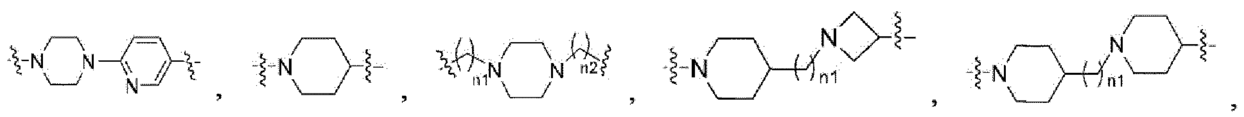

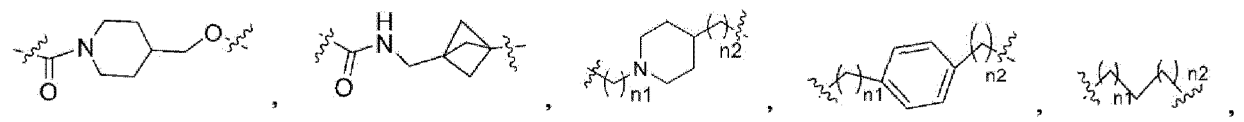

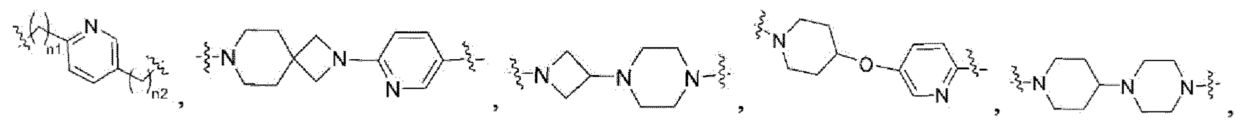

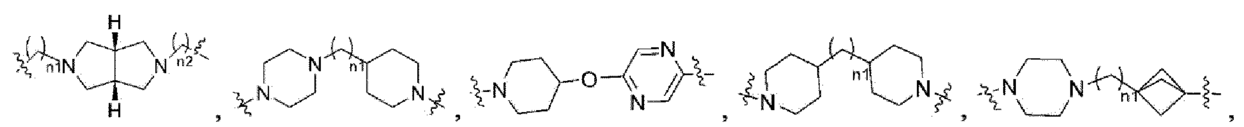

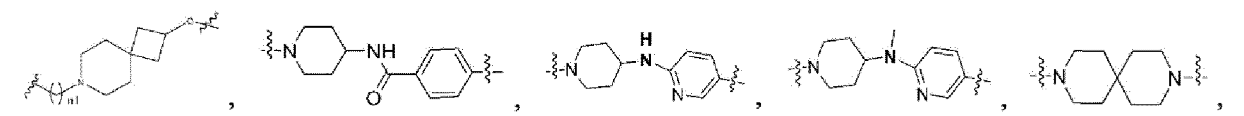

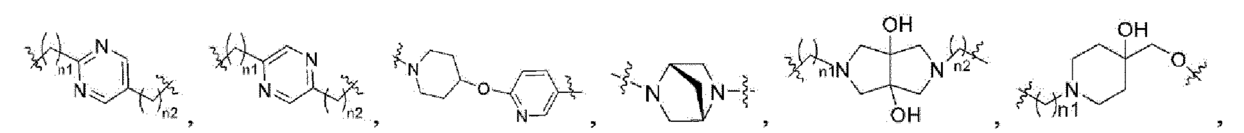

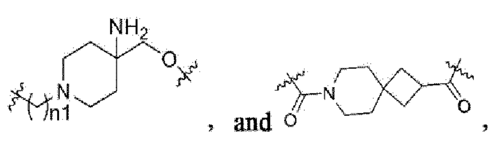

, and ,

wherein, n1 and n2 are each independently selected from integers ranging from 0 to 10, and preferably n1 and n2 are each independently 0, 1, 2 or 3.

In some embodiments of the present application, L is selected from the following structures:

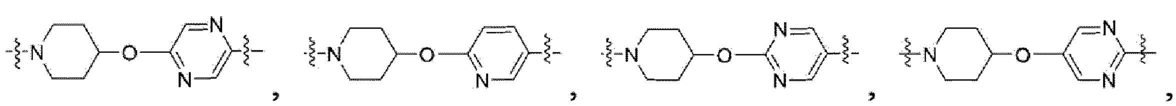

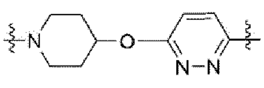, 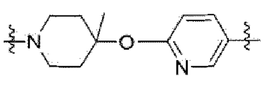, 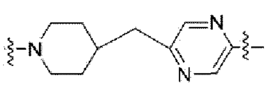, 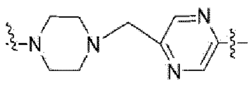,

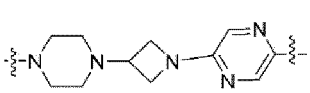, 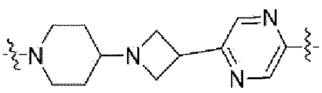, 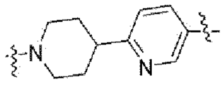, 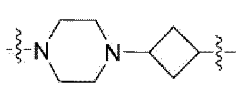,

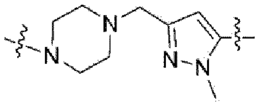, 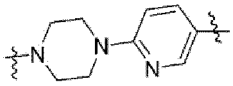, 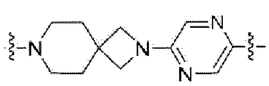, 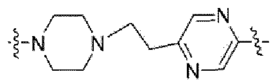,

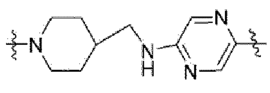, 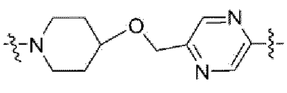, 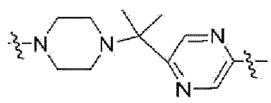, 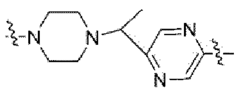,

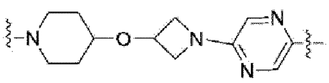, 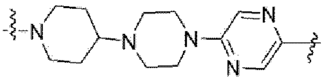, 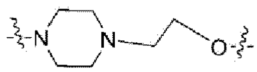,

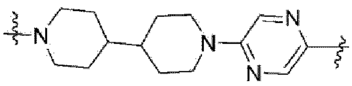, 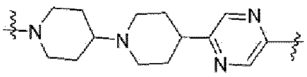, 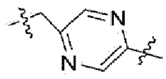, 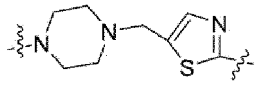,

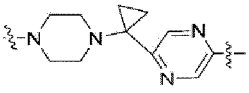, 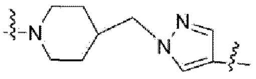, 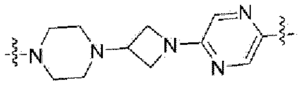, 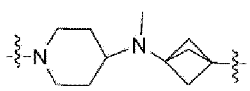,

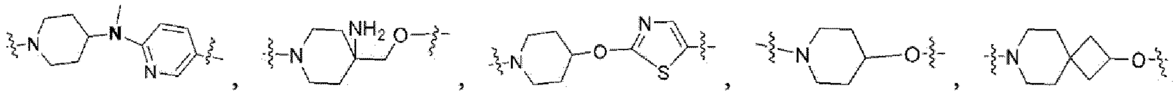,

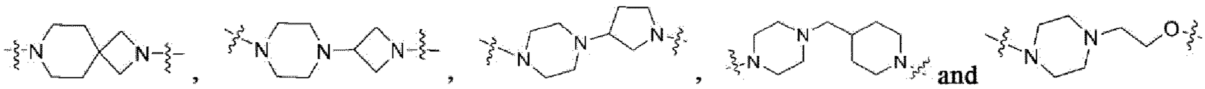

, , , and .

In some embodiments of the present application, M is selected from the group consisting of

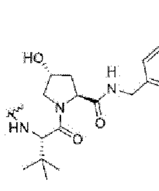, 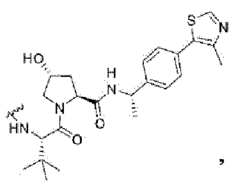, 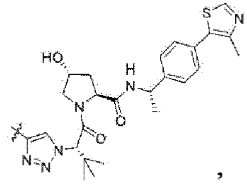, 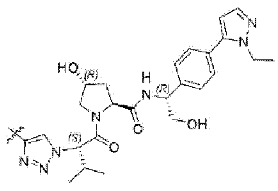,

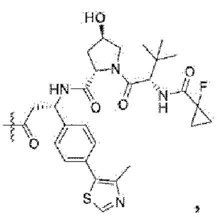, 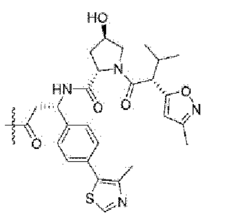, 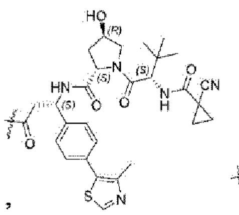, 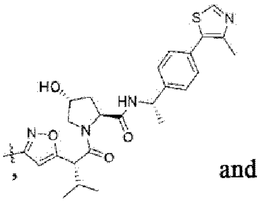 and 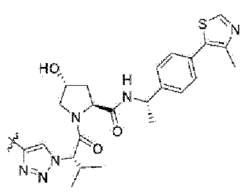.

In some embodiments of the present application, M is selected from the group consisting of

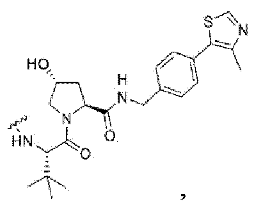, 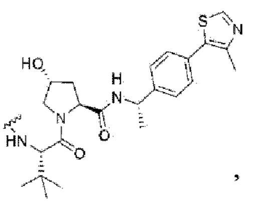, 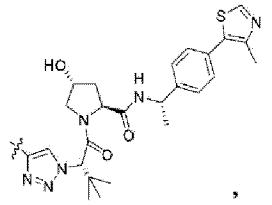, 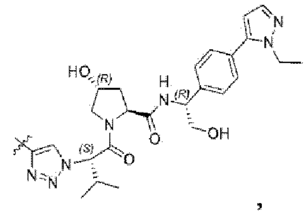, 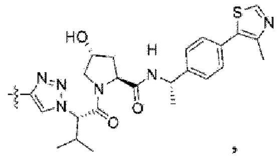, 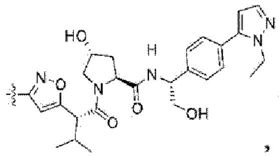, 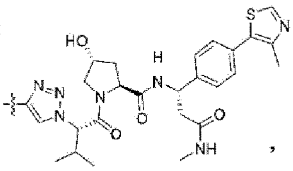, 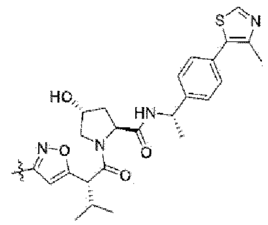, 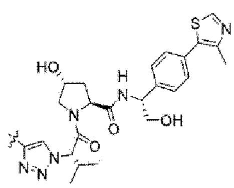, 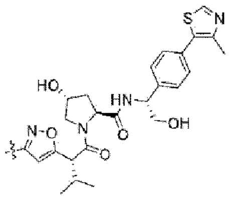, 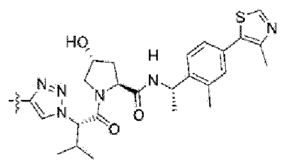 and 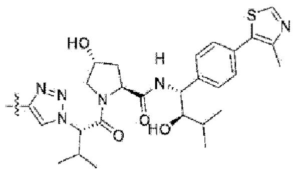.

In some embodiments of the present application, L is selected from the group consisting of

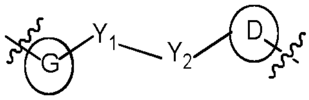 and 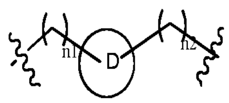;

wherein, ring G is selected from the group consisting of $C_{6-14}$ aryl, 5- to 14-membered heteroaryl, $C_{3-14}$ cycloalkyl and 3- to 14-membered heterocyclyl, wherein $C_{6-14}$ aryl, 5- to 14-membered heteroaryl or 3- to 14-membered heterocyclyl is optionally further substituted by one or more $R_a$, wherein $R_a$ is each independently selected from the group consisting of H, hydroxyl, amino, oxo, cyano, halogen, $C_{1-6}$ alkyl, $C_{1-6}$ haloalkyl and $C_{1-6}$ alkoxy;

ring D is 5- to 6-membered N-containing heteroaryl, and is preferably

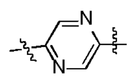

or

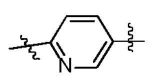;

$Y_1$ and $Y_2$ are each independently absent or selected from the group consisting of -O-, -NH-, 3- to 6-membered N-containing heterocyclyl, $C_{1-6}$ alkylene, carbonyl, acetylenyl and $C_{3-6}$ cycloalkyl; and

n1 and n2 are each independently 0, 1, 2, 3, or 4.

In some embodiments of the present application, L is selected from the group consisting of

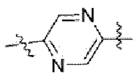, 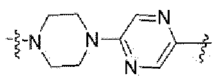, 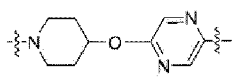,

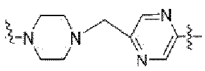, 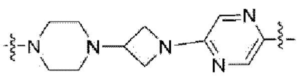, 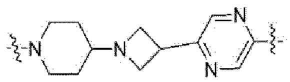, 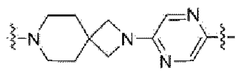,

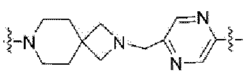, 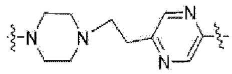, 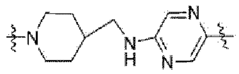, 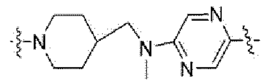

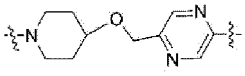, 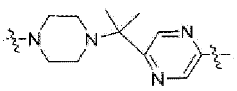, 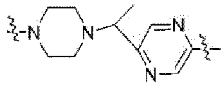, 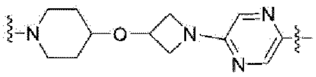,

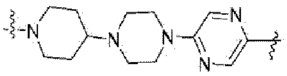, 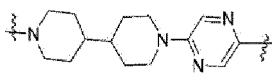, 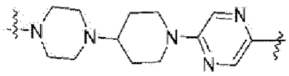 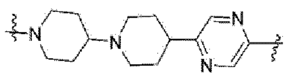,

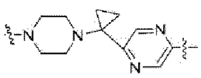, 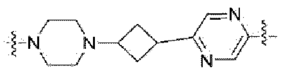, 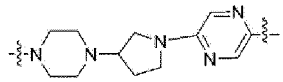, 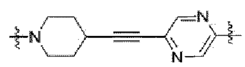,

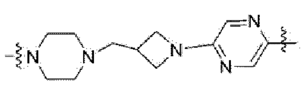, 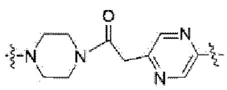, 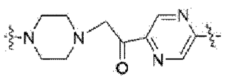, 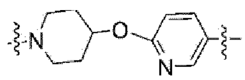,

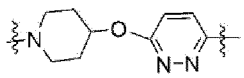, 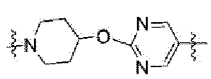, 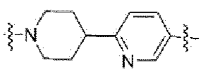 and 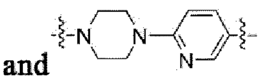.

In some embodiments of the present application, F is selected from the group consisting of

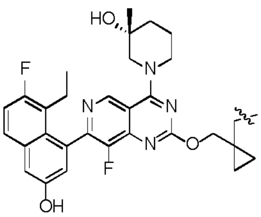 and 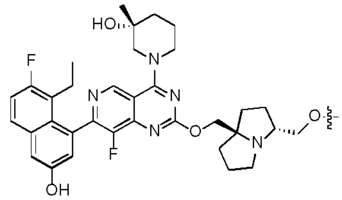,

L is selected from the group consisting of the following structures:

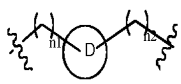,

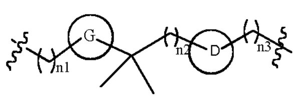, 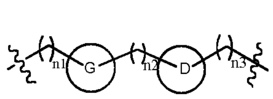, 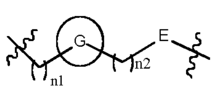, 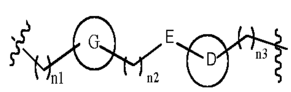,

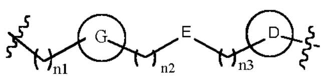,
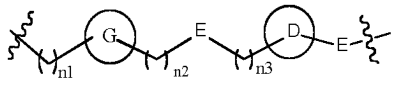
and
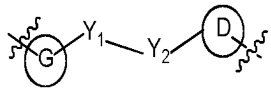;

wherein,

ring G or ring D is each independently selected from the group consisting of $C_{6-14}$ aryl, 5- to 14-membered heteroaryl, $C_{3-14}$ cycloalkyl and 3- to 14-membered heterocyclyl, wherein $C_{6-14}$ aryl, 5- to 14-membered heteroaryl, $C_{3-14}$ cycloalkyl or 3- to 14-membered heterocyclyl is optionally further substituted by one or more $R_a$;

$Y_1$ and $Y_2$ are each independently absent or selected from the group consisting of -O-, -NH-, 3- to 6-membered N-containing heterocyclyl, $C_{1-6}$ alkylene, carbonyl, acetylenyl and $C_{3-6}$ cycloalkyl;

E is each independently selected from the group consisting of -O-, -NH- and $-NCH_3-$;

n1, n2 and n3 are each independently selected from integers ranging from 0 to 10, and preferably n1, n2 and n3 are each independently 0, 1, 2, 3 or 4;

M is a VHL binding fragment represented by general formula (III);

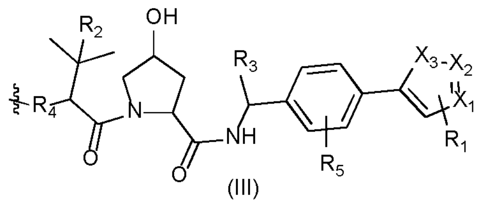

(III)

wherein, $X_1$ is selected from the group consisting of C, CH and N;

$X_2$ is selected from the group consisting of C, CH and N;

$X_3$ is selected from the group consisting of N, NH, O and S;

$R_1$ is selected from the group consisting of H, $C_{1-6}$ alkyl, halogen and cyano, and is preferably methyl, ethyl, F or cyano;

$R_2$ is selected from the group consisting of H and $C_{1-6}$ alkyl, and is preferably H or methyl;

$R_3$ is selected from the group consisting of H, $C_{1-6}$ alkyl, $C_{1-6}$ hydroxylalkyl and $-(CH_2)_n-CO-NH-(CH_2)_n-CH_3$, wherein n is selected from the group consisting of 0, 1, 2 and 3, and $R_3$ is preferably H, methyl, hydroxylmethyl,

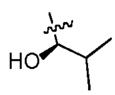

or $-CH_2-CO-NH-CH_3$;

$R_4$ is selected from the group consisting of

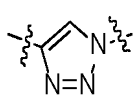,
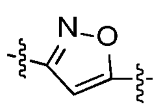

and -NH-;

$R_5$ is selected from the group consisting of H, $C_{1-6}$ alkyl and $C_{1-6}$ haloalkyl; and

$R_a$ is independently selected from the group consisting of H, hydroxyl, amino, oxo, cyano, halogen, $C_{1-6}$ alkyl, $C_{1-6}$ haloalkyl and $C_{1-6}$ alkoxy.

In some embodiments of the present application, the compound having an F-L-M structure is selected from the following compounds:

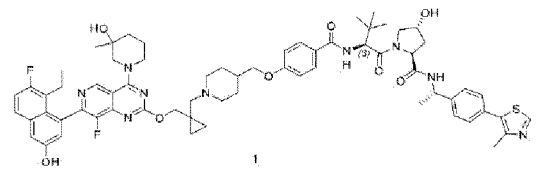

1

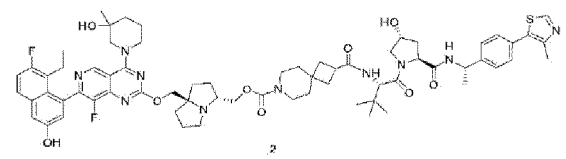

2

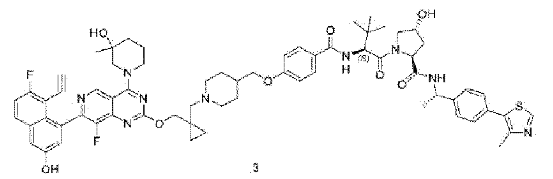

3

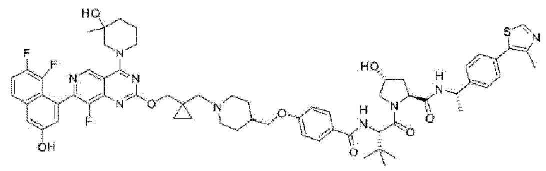

4

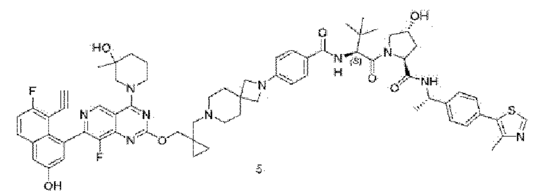

5

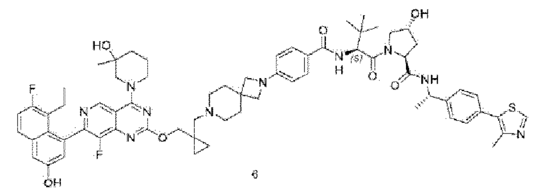

6

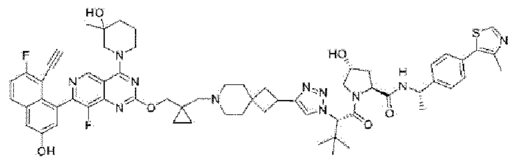

7

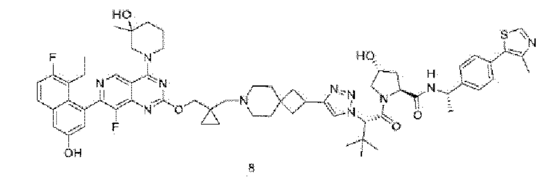

8

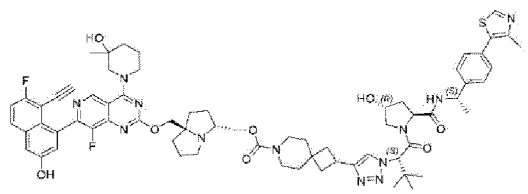

9

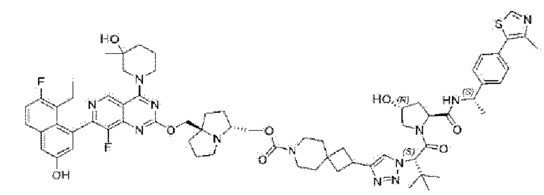

10

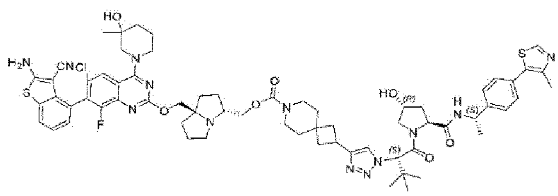

11

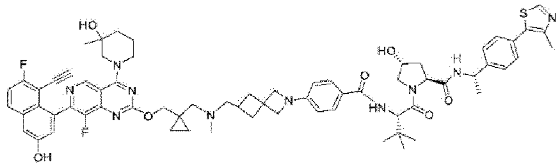

12

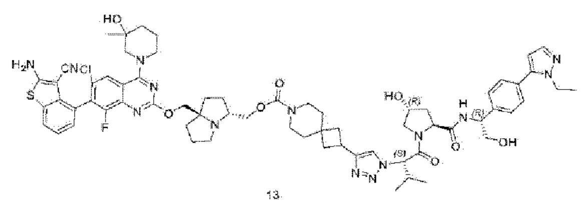

13

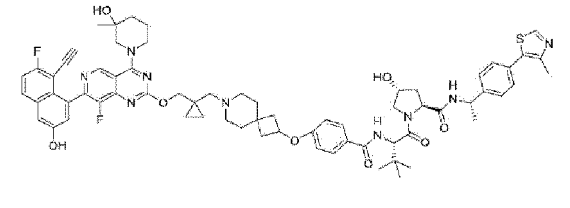

14

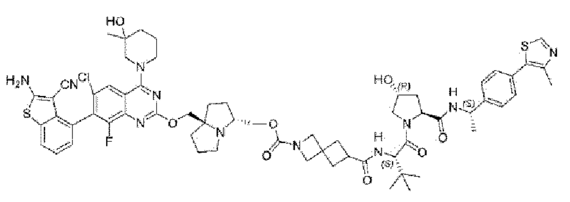

15

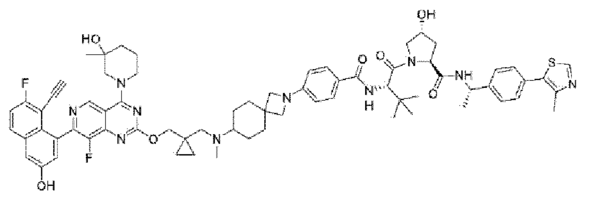

16

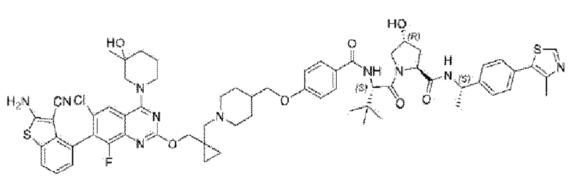

17

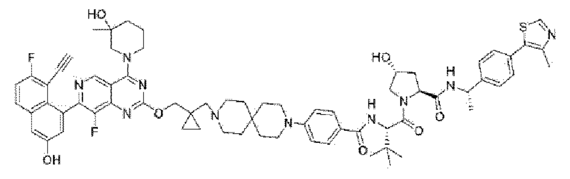

18

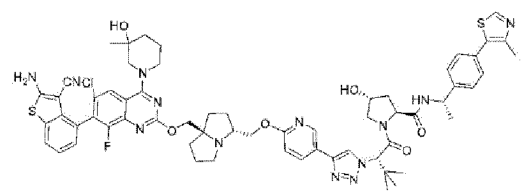

19

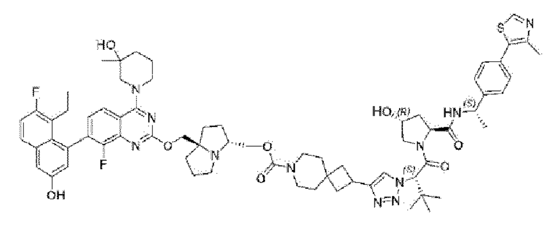

20

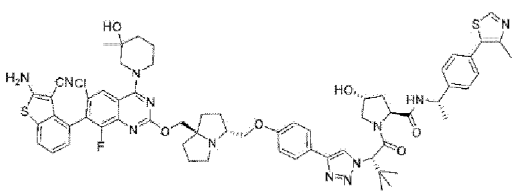

21

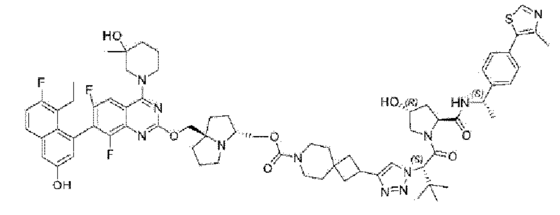

22

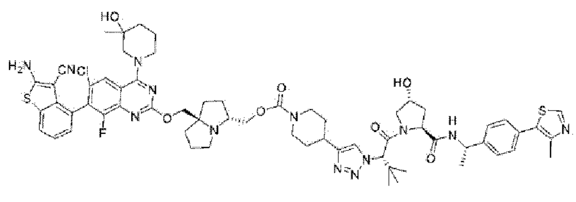

23

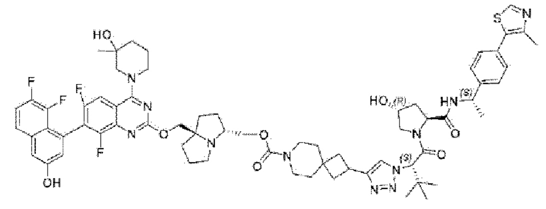

24

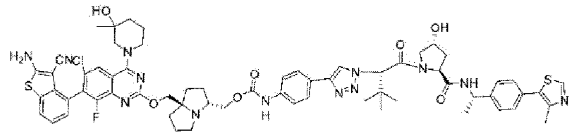

25

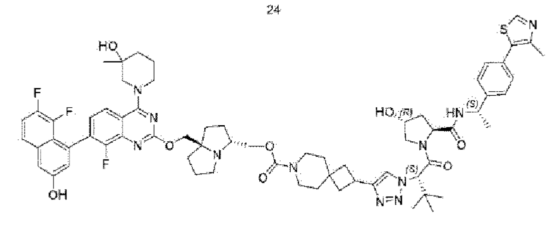

26

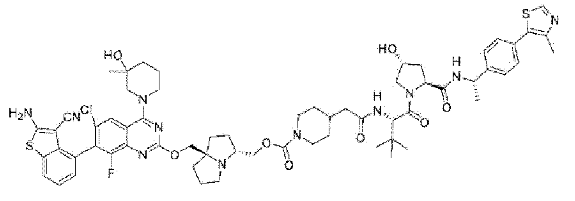

27

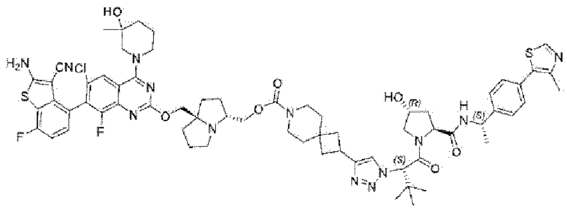

28

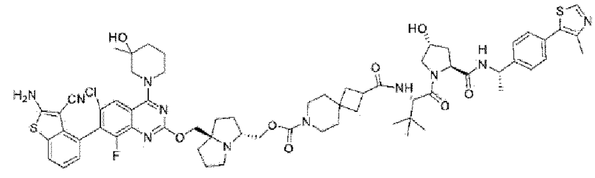

29

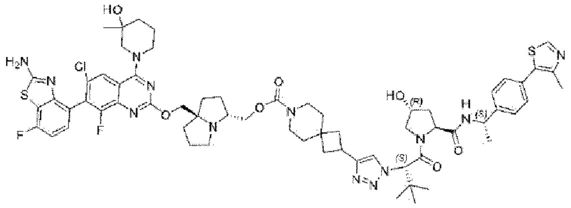

30

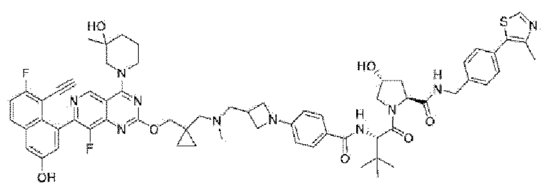

31

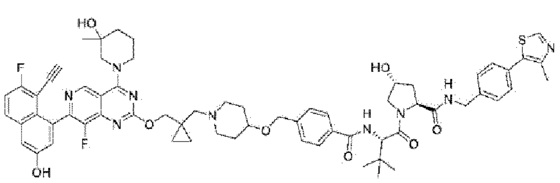

32

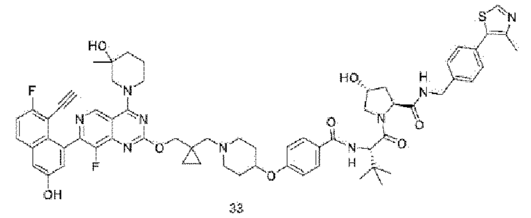

33

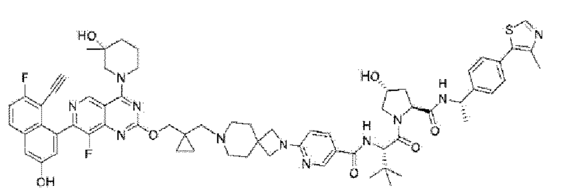

34

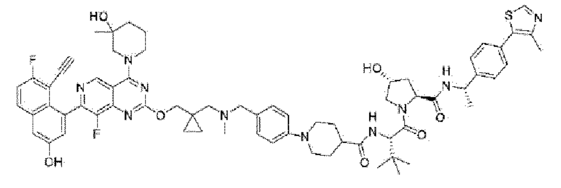

35

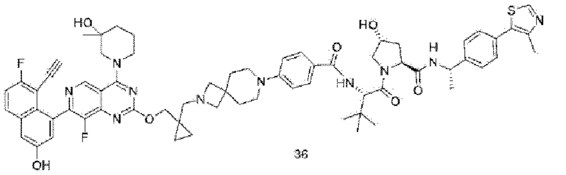

36

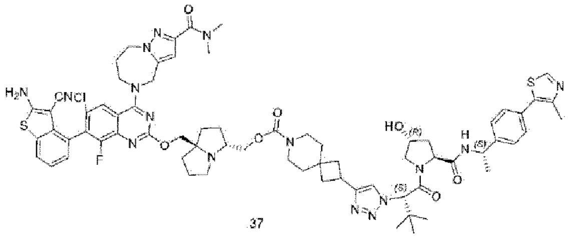

37

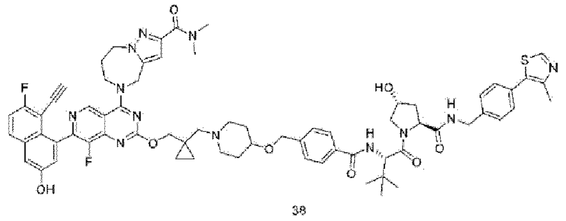

38

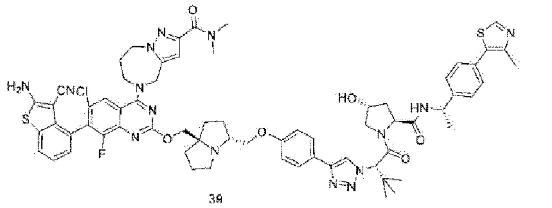

39

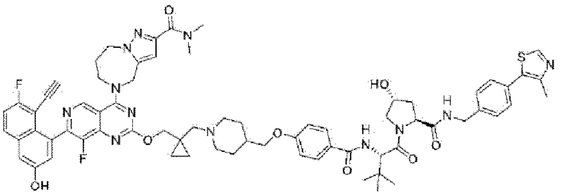

40

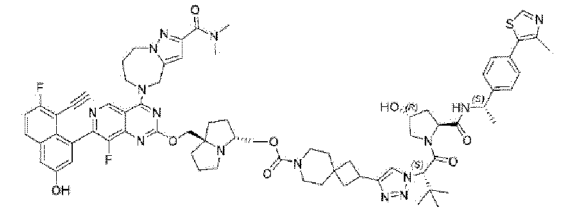

41

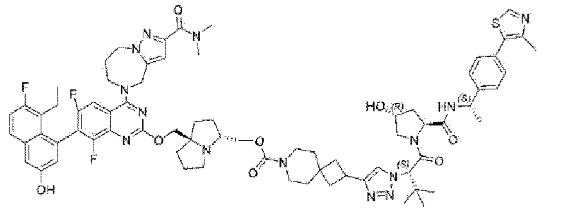

42

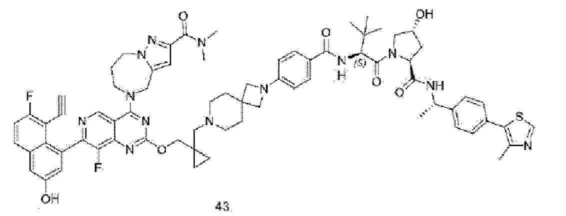

43

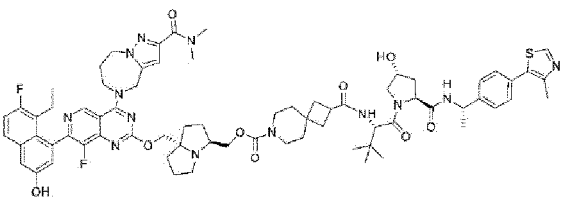

44

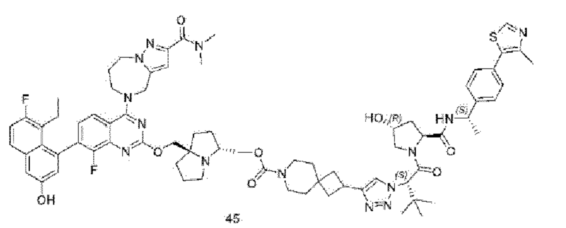

45

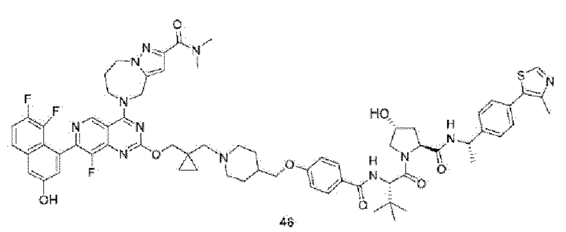

46

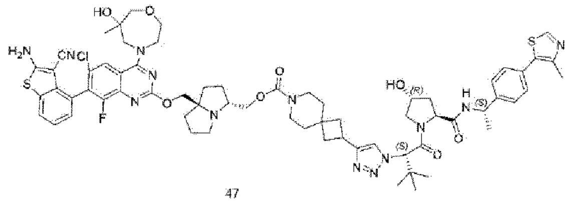
47

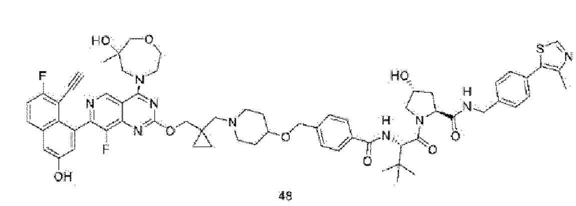
48

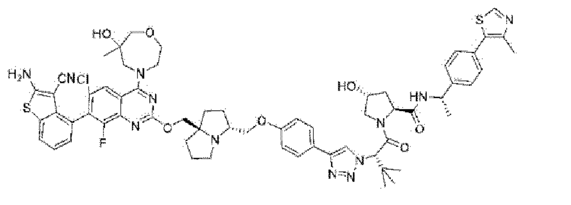
49

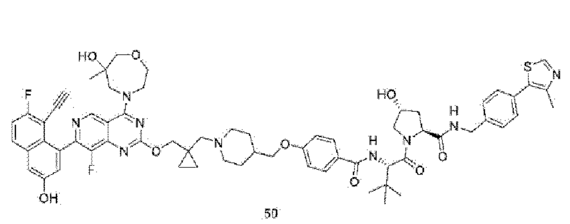
50

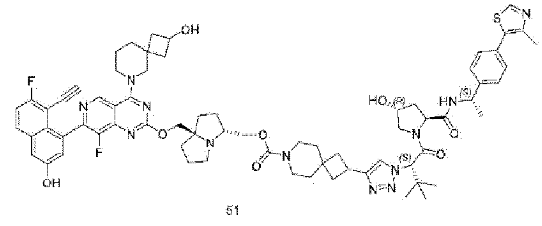
51

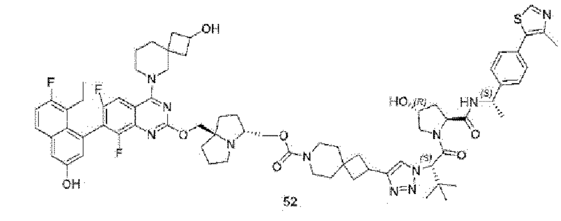
52

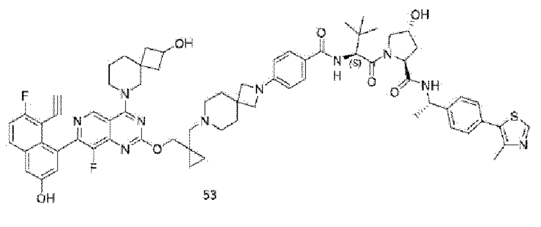
53

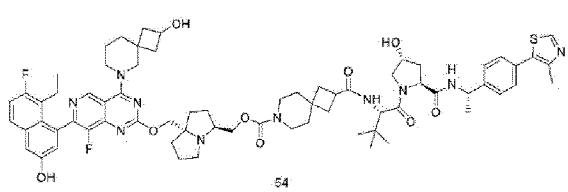
54

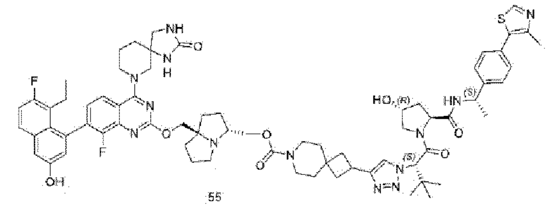
55

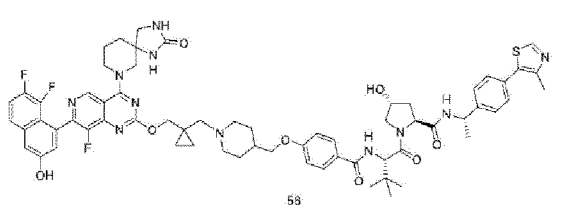
56

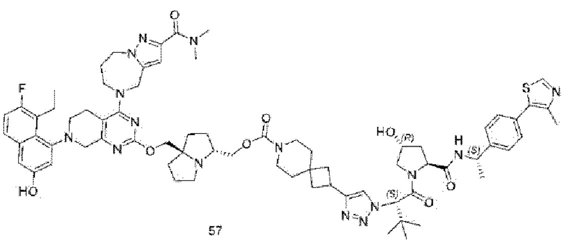
57

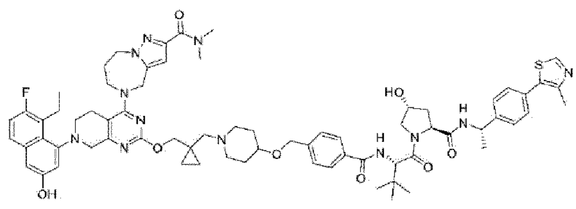
58

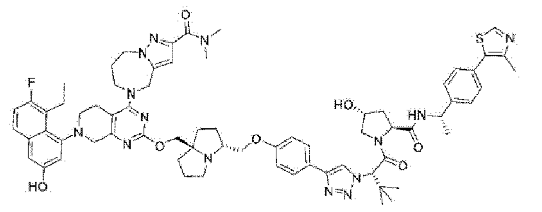
59

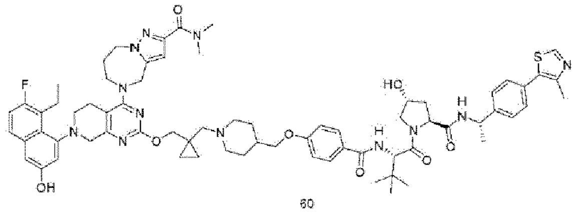
60

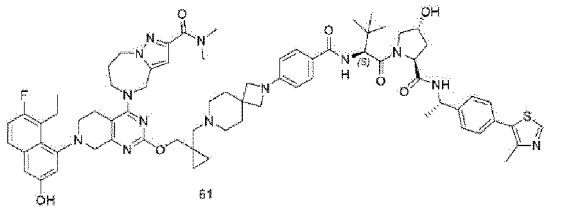
61

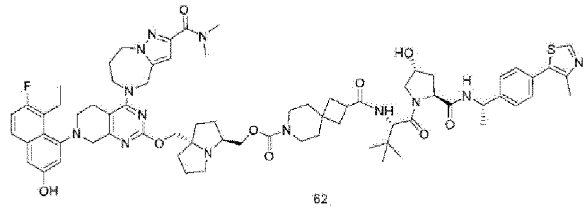
62

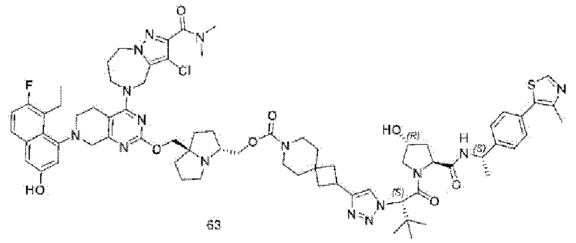
63

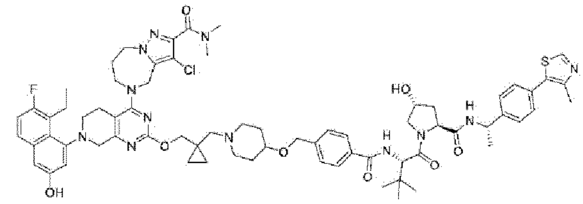
64

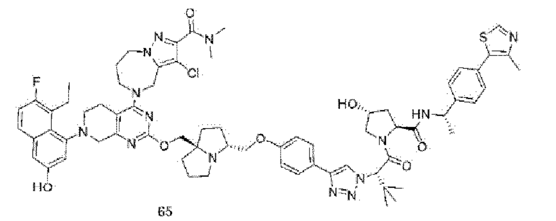
65

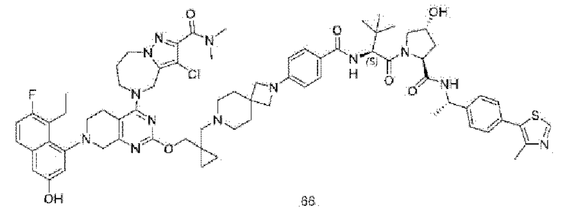
66

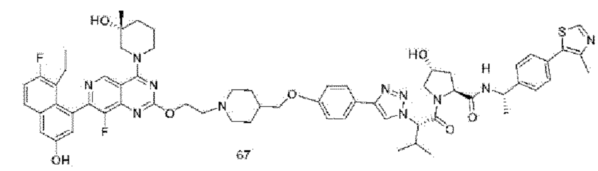
67

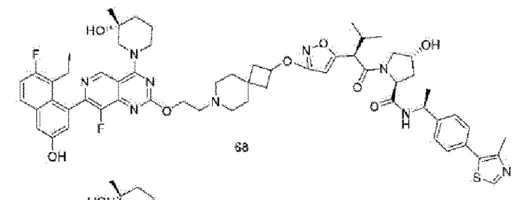
68

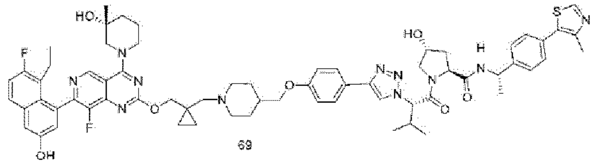
69

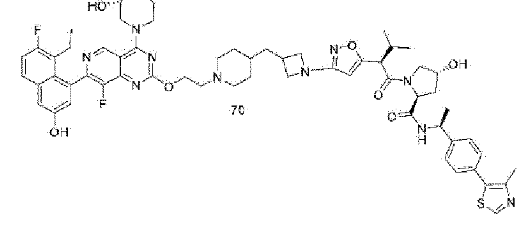
70

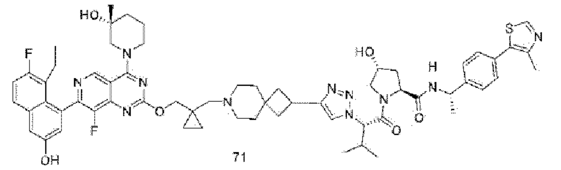
71

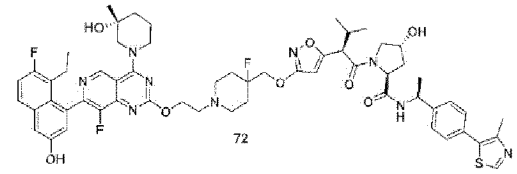
72

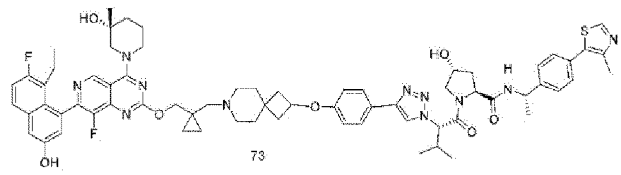

73

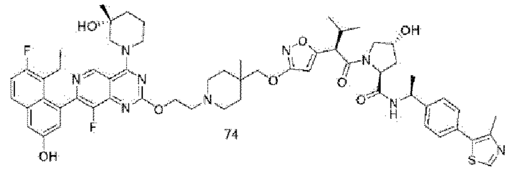

74

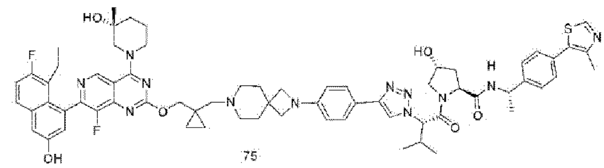

75

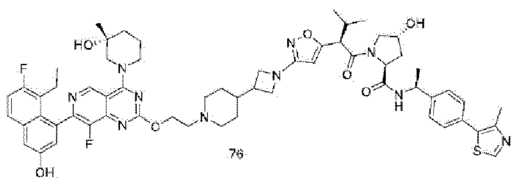

76

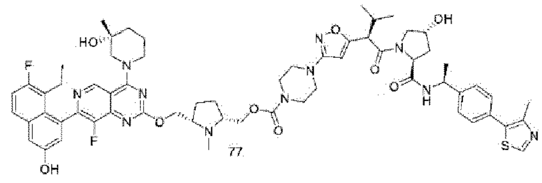

77

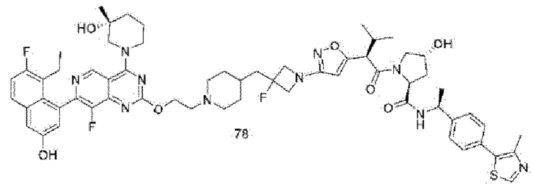

78

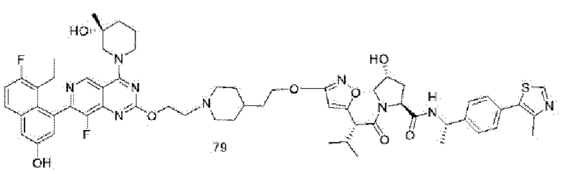

79

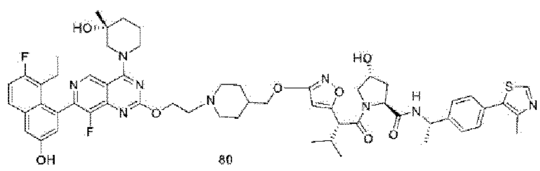

80

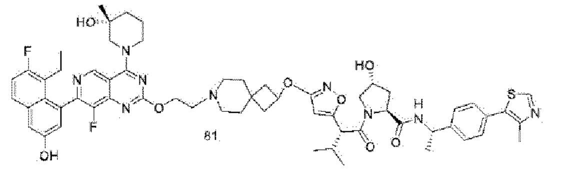

81

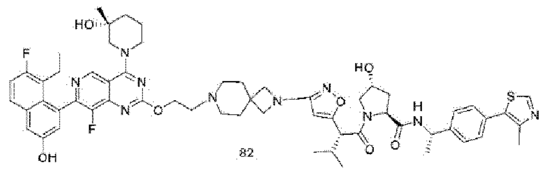

82

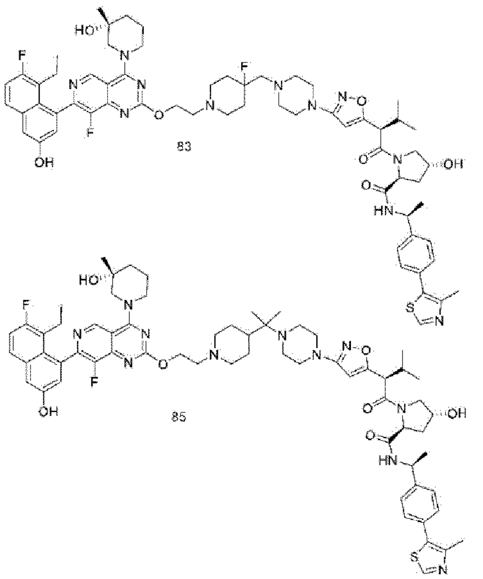

83

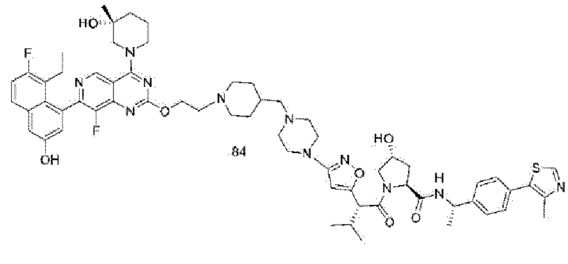

84

85

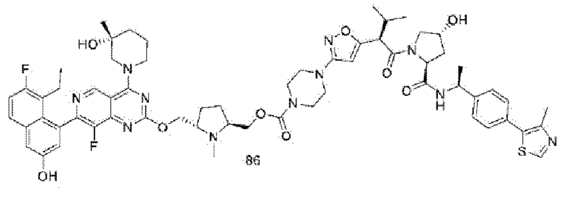

86

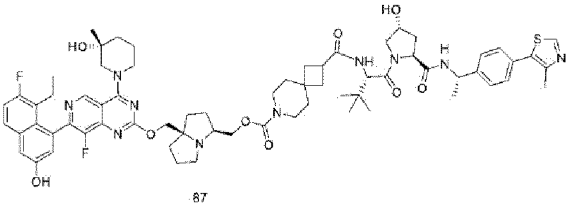

87

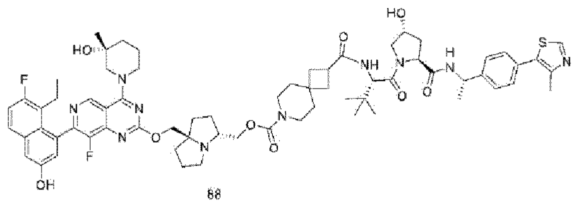

88

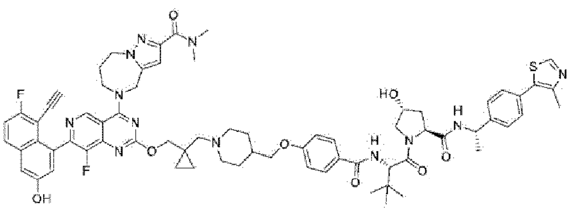

89

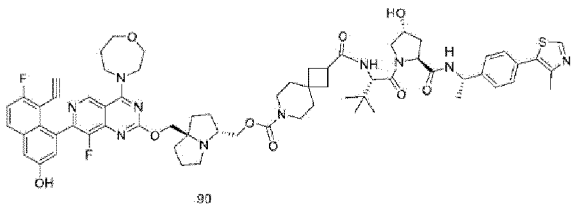

90

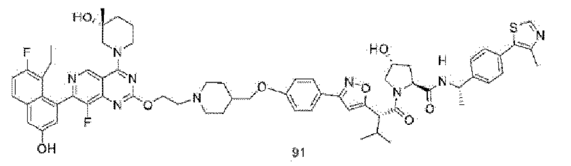

91

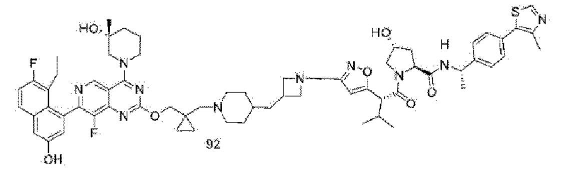

92

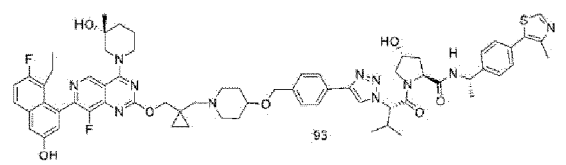

93

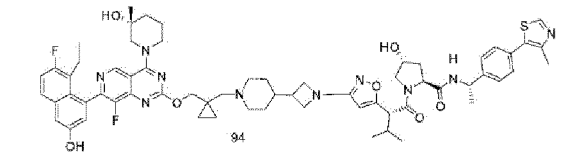

94

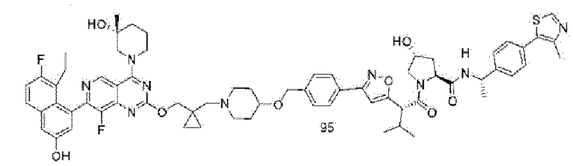

95

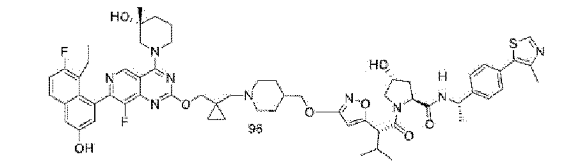

96

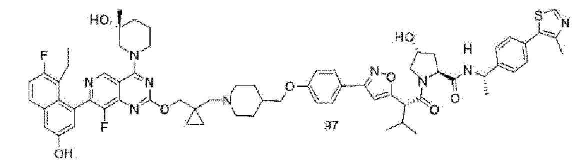

97

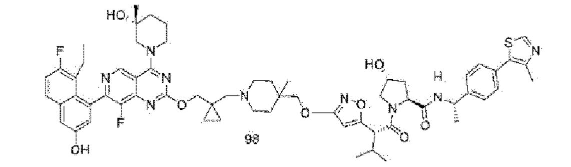

98

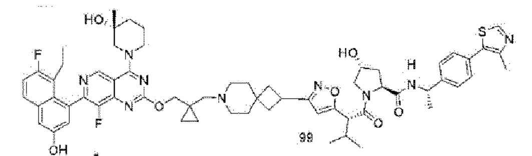

99

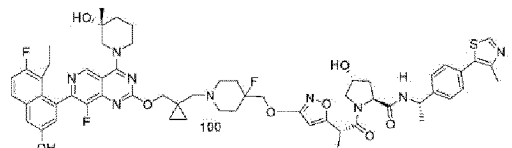

100

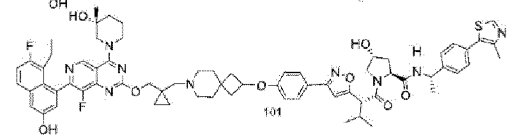

101

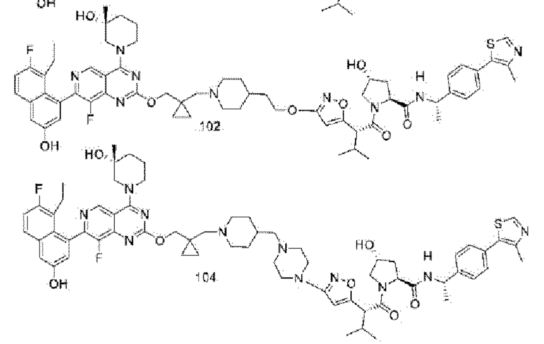

102

104

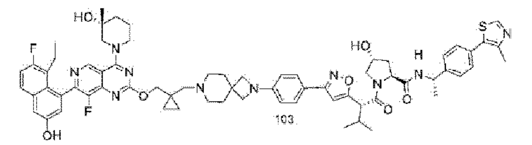

103

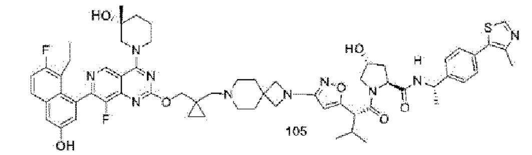

105

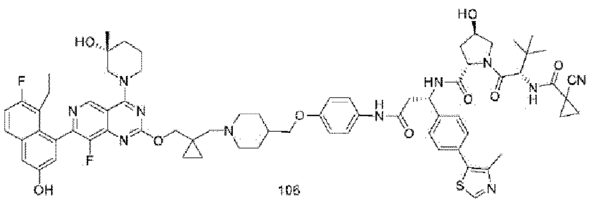

106

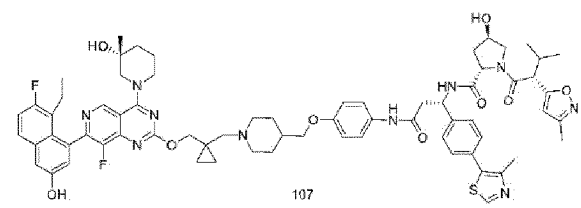

107

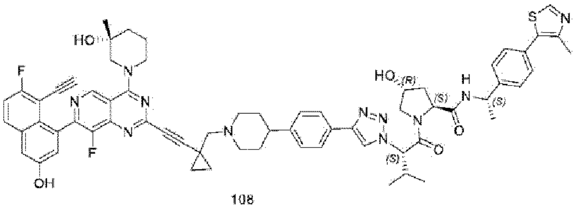

108

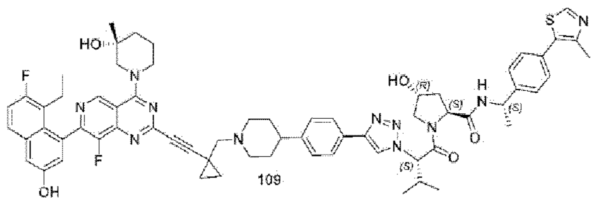

109

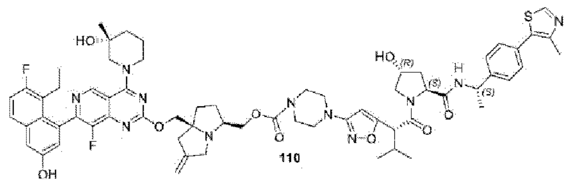

110

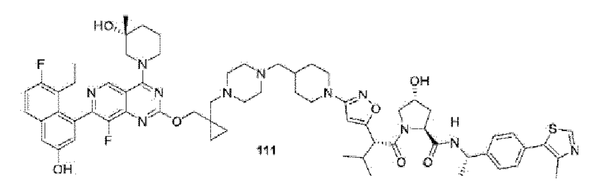

111

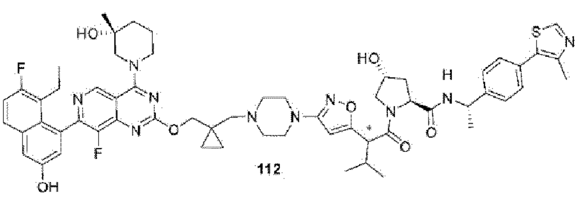

112

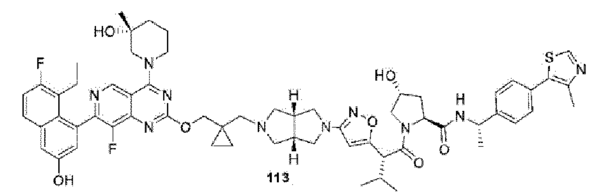

113

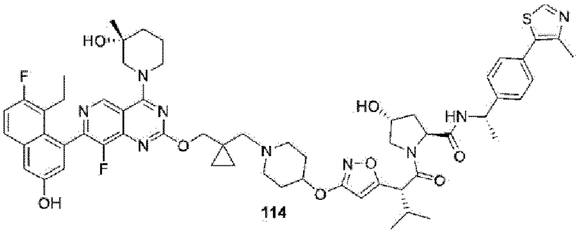
114

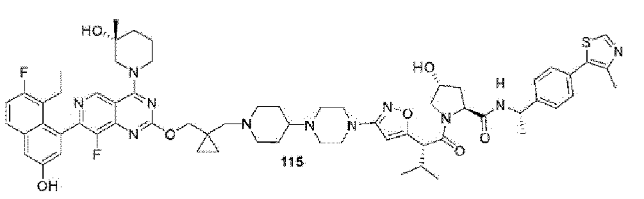
115

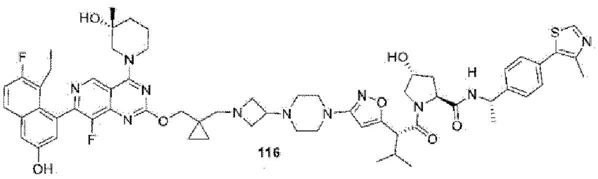
116

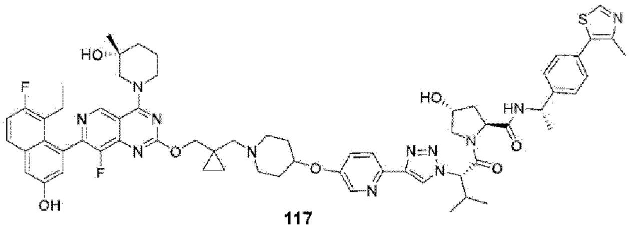
117

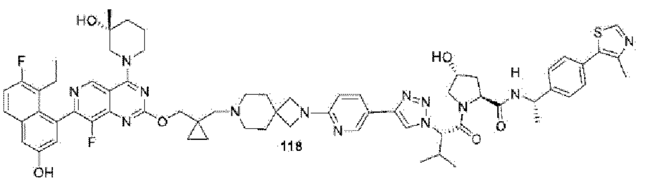
118

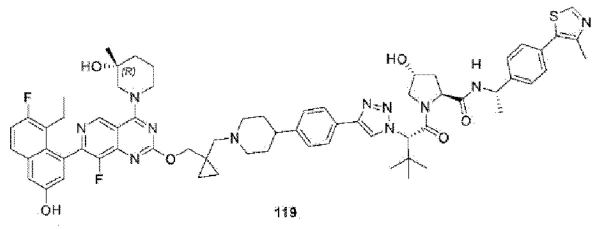
119

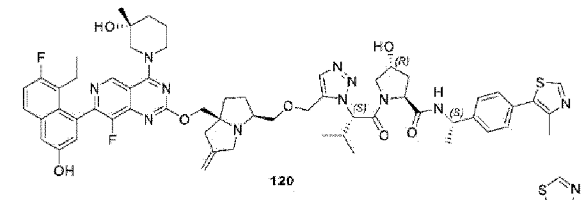
120

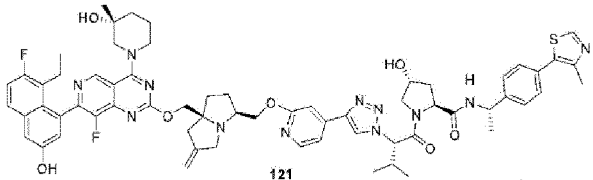
121

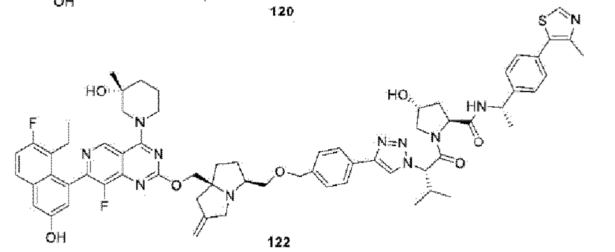
122

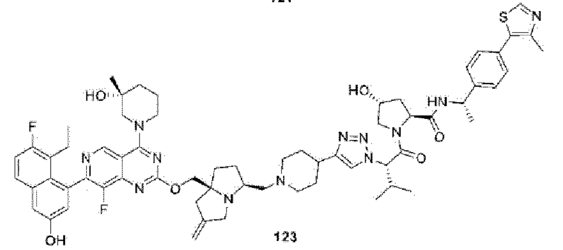
123

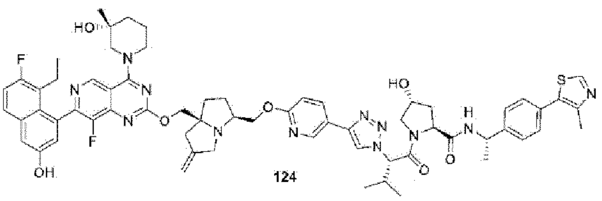
124

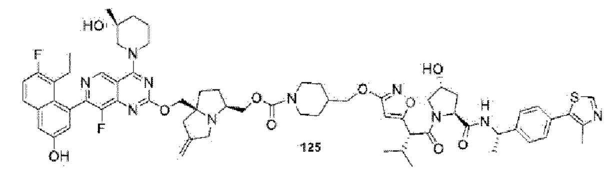
125

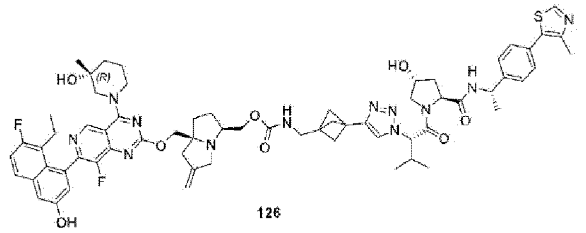
126

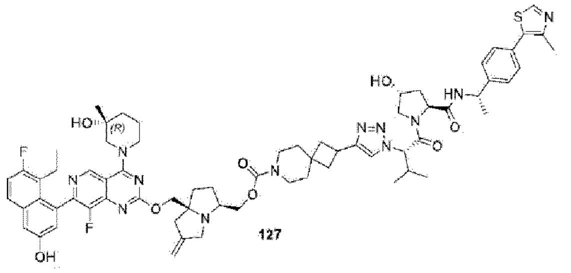
127

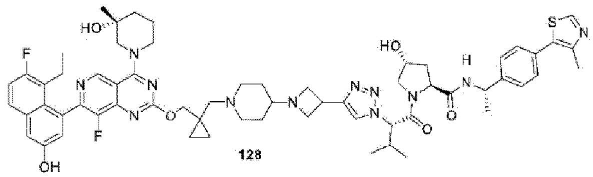
128

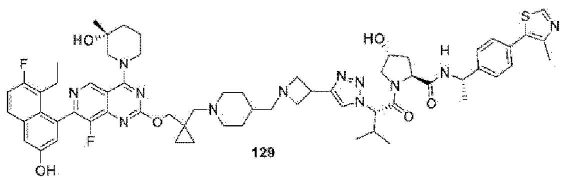
129

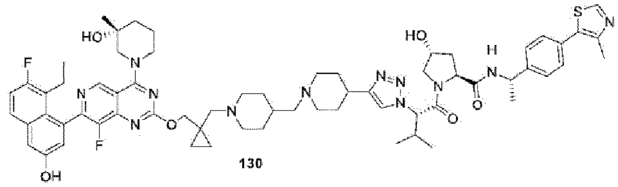
130

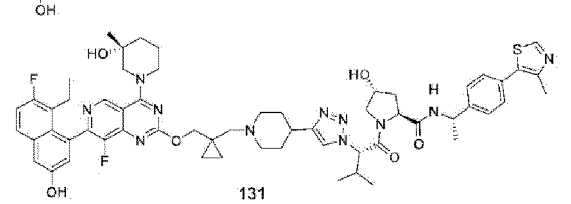
131

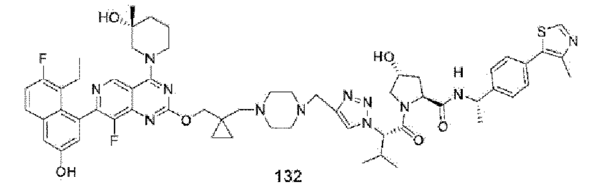
132

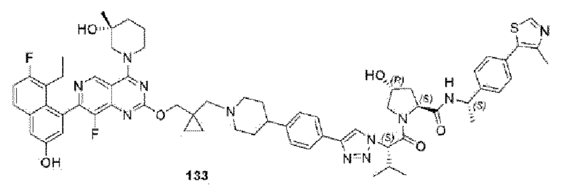
133

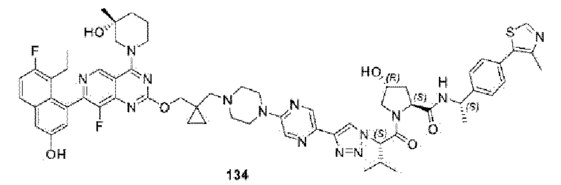
134

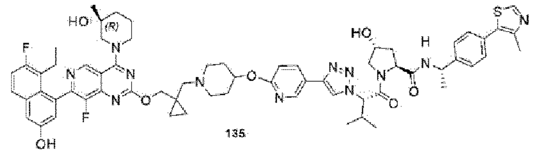
135

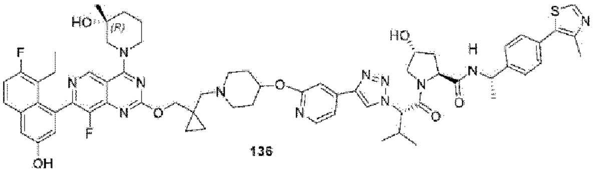
136

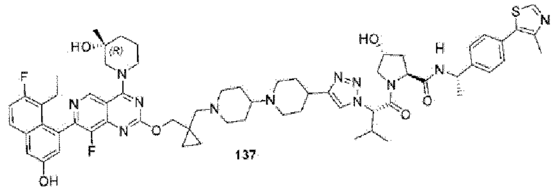
137

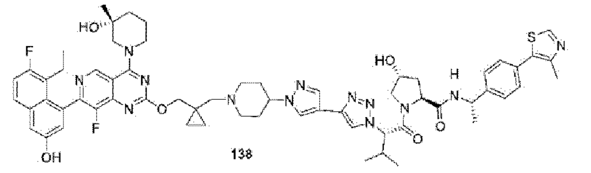
138

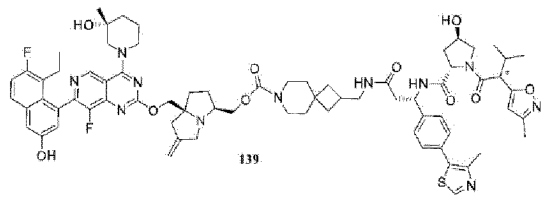
139

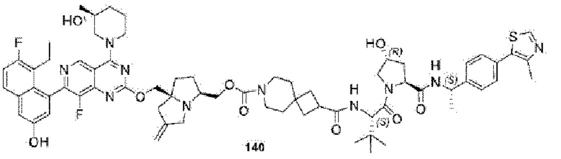
140

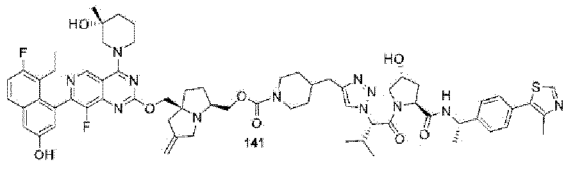
141

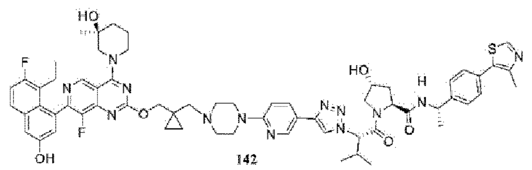
142

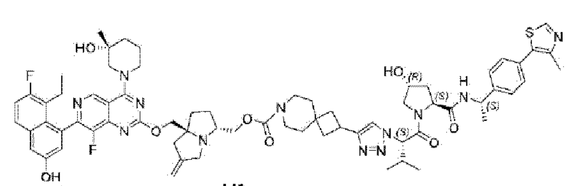

143

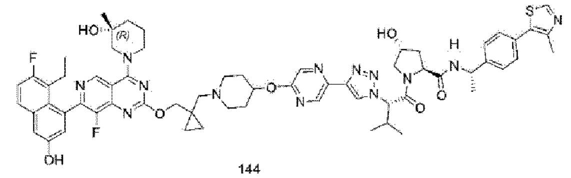

144

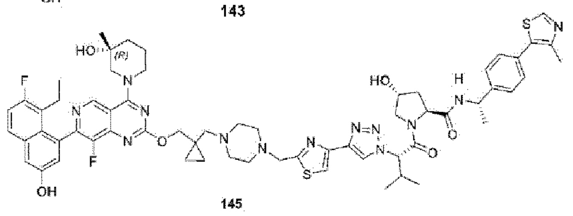

145

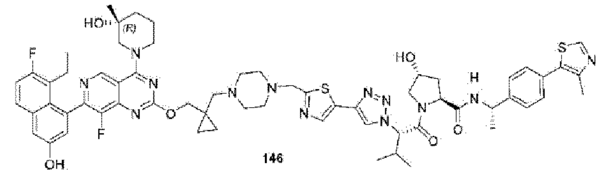

146

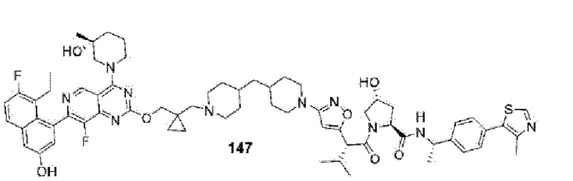

147

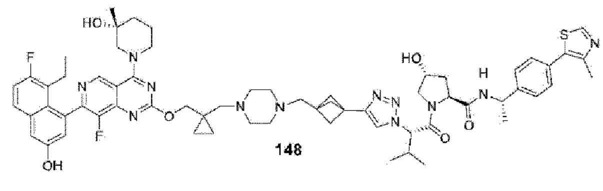

148

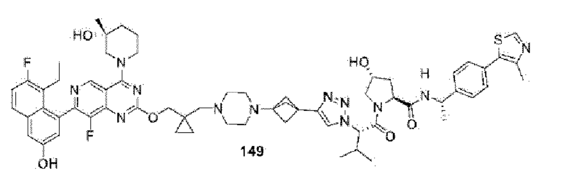

149

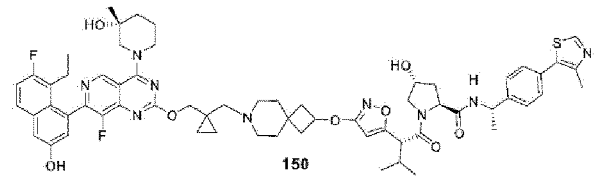

150

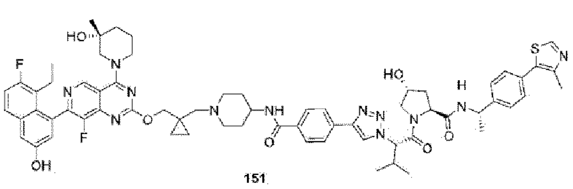

151

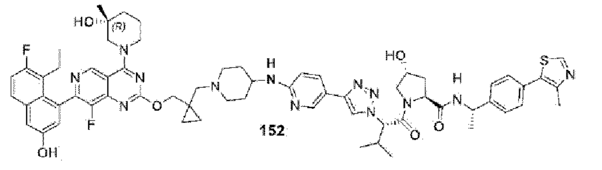

152

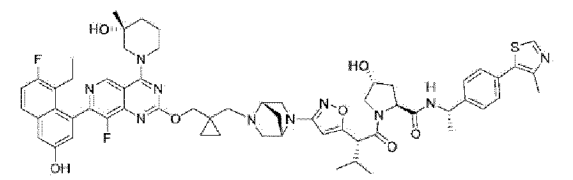

153

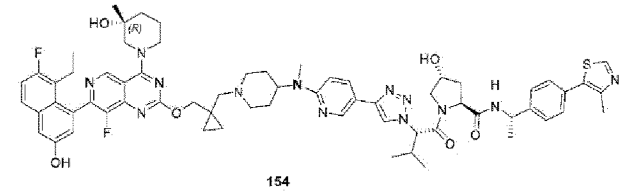

154

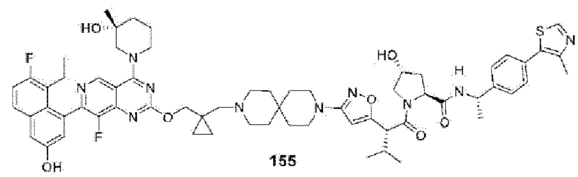

155

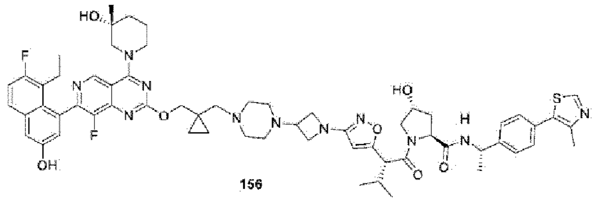

156

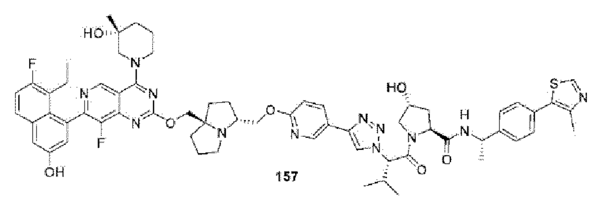
157

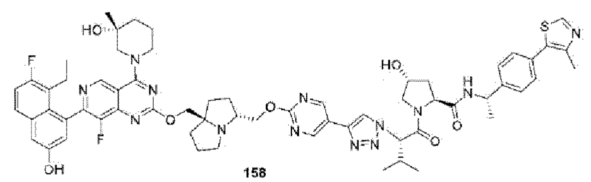
158

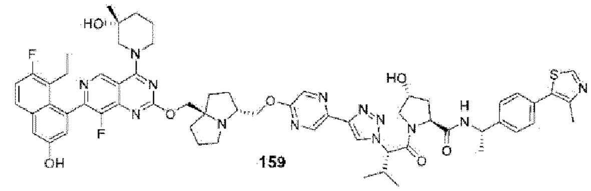
159

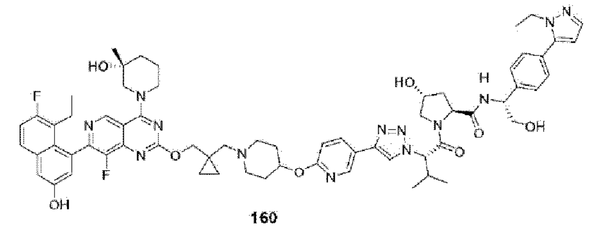
160

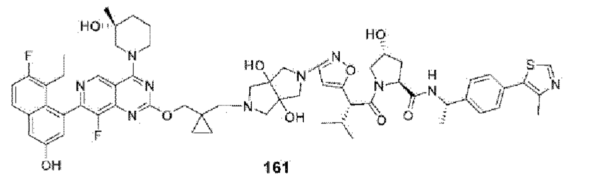
161

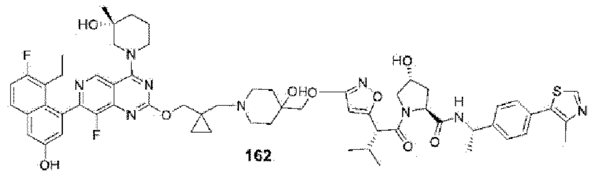
162

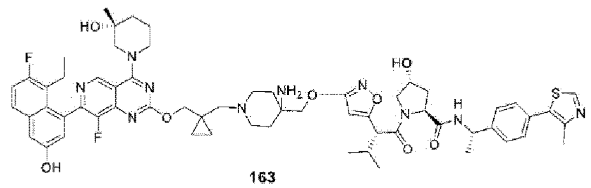
163

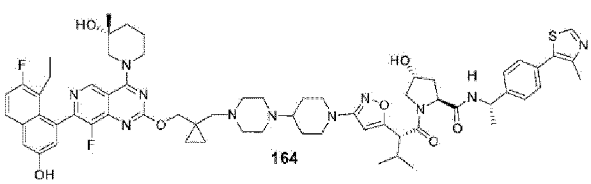
164

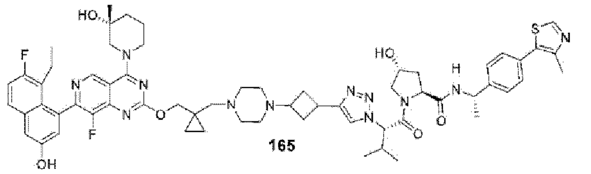
165

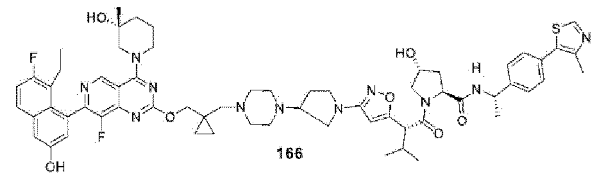
166

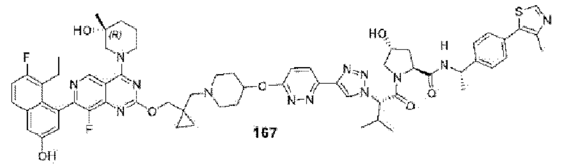
167

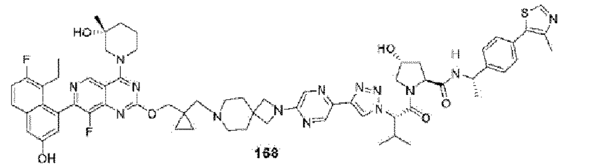
168

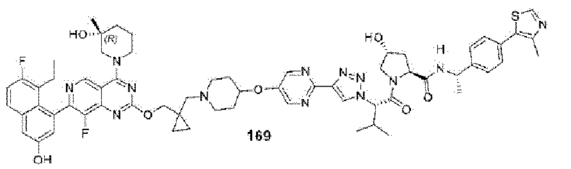
169

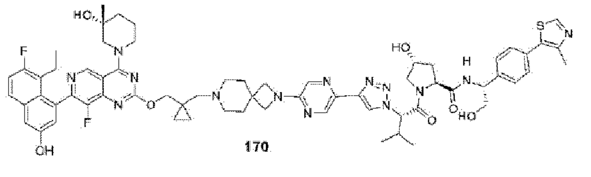
170

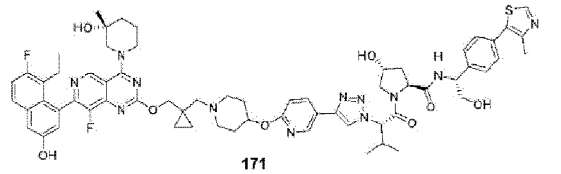
171

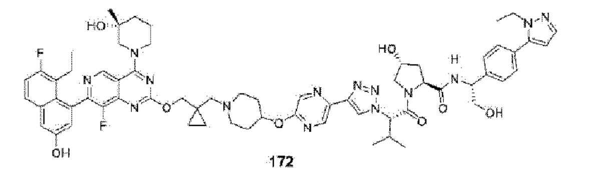
172

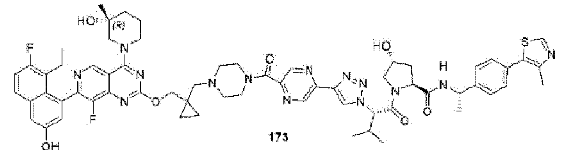

173

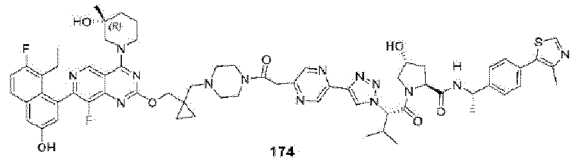

174

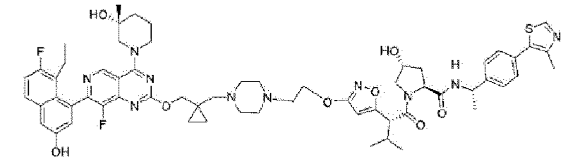

175

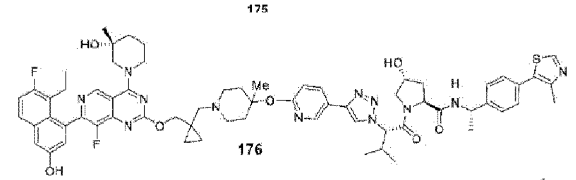

176

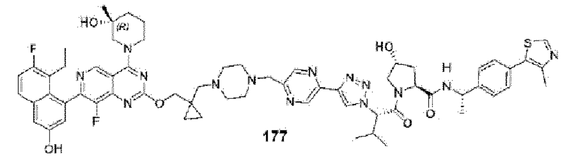

177

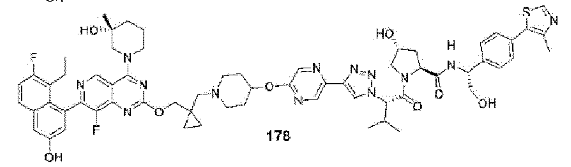

178

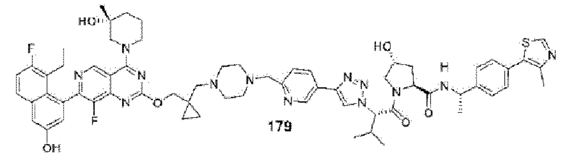

179

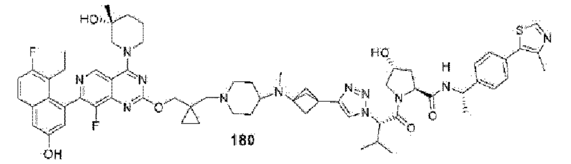

180

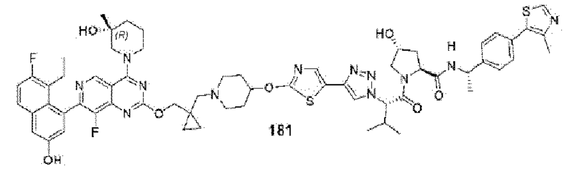

181

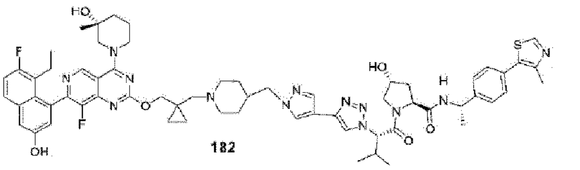

182

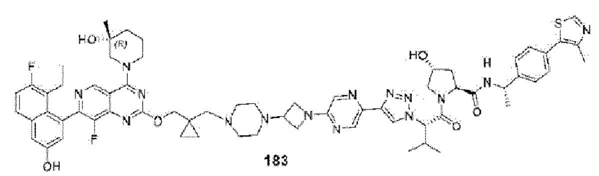

183

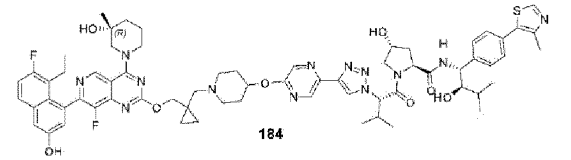

184

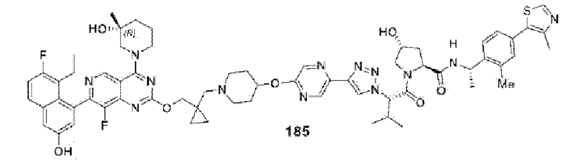

185

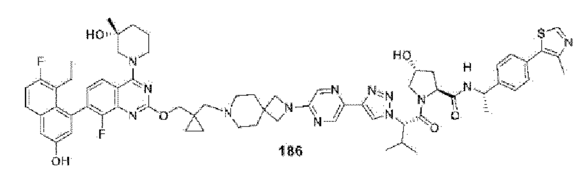

186

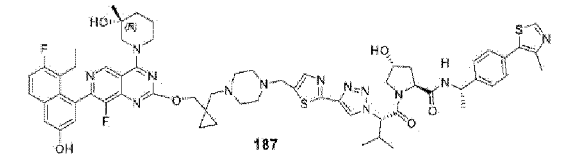

187

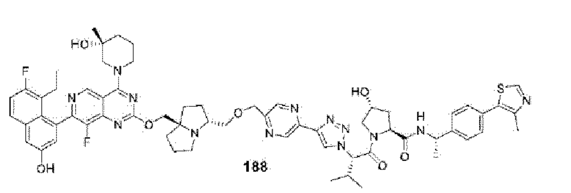

188

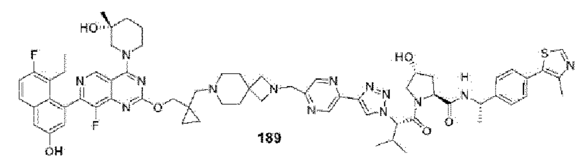

189

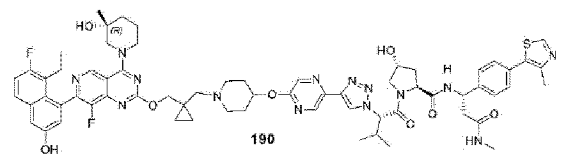

190

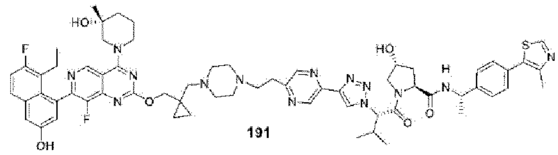

191

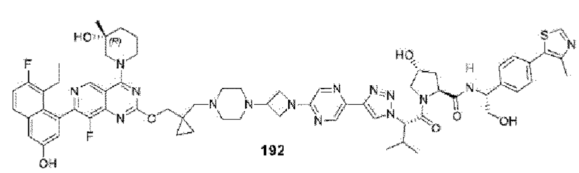

192

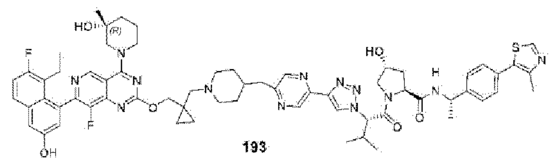

193

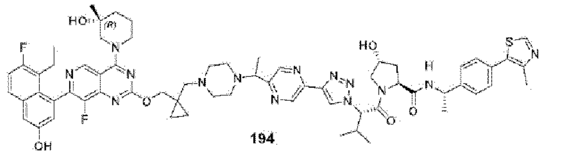

194

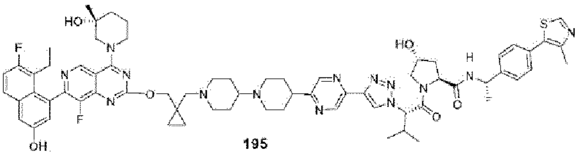

195

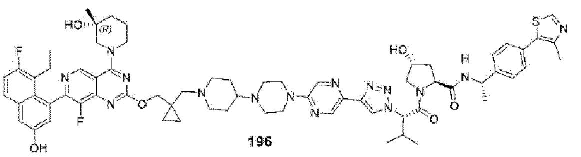

196

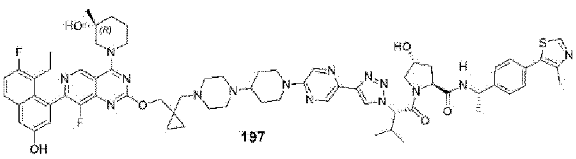

197

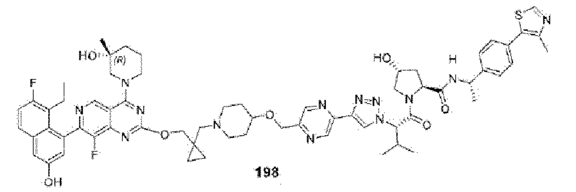

198

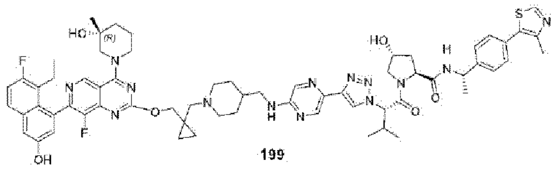

199

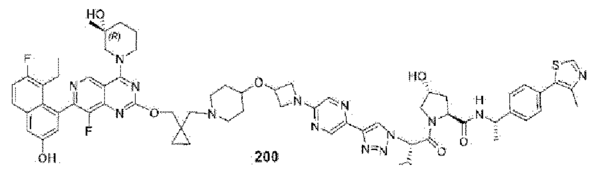

200

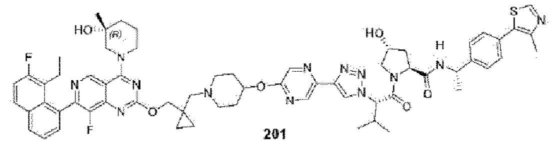

201

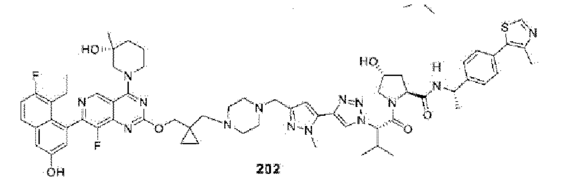

202

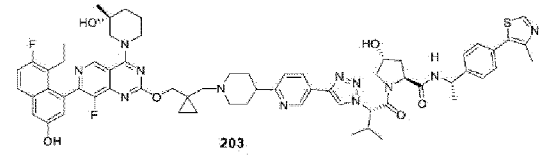

203

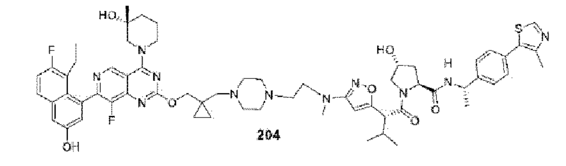

204

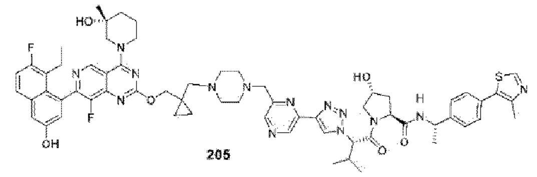

205

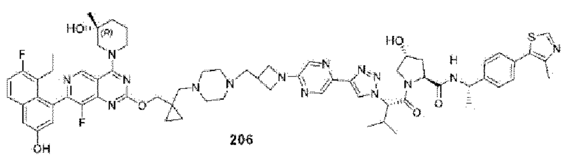
206

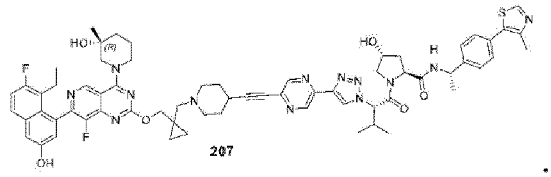
207

After extensive research, inventors of the present application found that the connecting chain of the compound in the present application has a significant impact on both the KRAS degradation ability and cell proliferation inhibitory activity of the compound. When 5- to 6-membered N-containing heteroaryl, especially pyrazine ring and pyridine ring, is added to the side close to the VHL ligand in the connecting chain, the KRAS degradation ability of the compound will be significantly enhanced, and at the same time, the cell proliferation inhibitory activity of the compound will increase. We speculate that the spatial position of the N-containing heteroaromatic ring is special, which will have interactions such as hydrogen bonds with the VHL protein, increasing the binding force between the compound and VHL, thereby enhancing the compound for the KRAS protein degradation ability. Studies show that, directly linking the N-containing heteroaromatic ring to the VHL ligand is a key factor in enhancing the degradation ability of the compound. In addition, this type of compound with N-containing heteroaromatic ring has excellent in vivo pharmacokinetic properties and high druggability.

In another aspect, the present application provides a pharmaceutical composition comprising the compound represented by a general formula having an F-L-M structure, or a tautomer, racemate, enantiomer, diastereomer thereof, or a mixture thereof, or a pharmaceutically acceptable salt thereof of the present application, and one or more pharmaceutically acceptable carriers, diluents or excipients.

The present application further provides use of any one of the compound represented by a general formula having an F-L-M structure, or the tautomer, racemate, enantiomer, diastereomer thereof, or the mixture thereof, or the pharmaceutically acceptable salt thereof, or the pharmaceutical composition comprising the same as described above in the manufacture of a medicament for regulating ubiquitination and degradation of KRAS protein in a subject.

The present application further provides use of any one of the compound represented by a general formula having an F-L-M structure, or the tautomer, racemate, enantiomer, diastereomer thereof, or the mixture thereof, or the pharmaceutically acceptable salt thereof, or the pharmaceutical composition comprising the same as described above in the manufacture of a medicament for treating and/or preventing a KRAS-mediated or dependent disease, wherein the KRAS-mediated disease is preferably a tumor.

In some embodiments, the disease is selected from the group consisting of breast cancer, multiple myeloma, bladder cancer, endometrial cancer, gastric cancer, cervical cancer, rhabdomyosarcoma, non-small cell lung cancer, small cell lung cancer, pleomorphic lung cancer, ovarian cancer, esophageal cancer, melanoma, colorectal cancer, hepatocellular carcinoma, head and neck tumor, intrahepatic cholangiocarcinoma, myelodysplastic syndrome, malignant glioma, prostate cancer, thyroid cancer, Schwann cell tumor, pulmonary squamous cell carcinoma, lichenoid keratosis, synovial sarcoma, skin cancer, pancreatic cancer, testicular cancer and liposarcoma.

Unless otherwise stated, a general chemical term used in a structural general formula has its common meaning.

For example, unless otherwise stated, the term "halogen" used in the present application refers to fluorine, chlorine, bromine or iodine.

In the present application, without any specific limitation on the connection sequence of the groups, they can be connected from left to right or from right to left.

In the present application, unless otherwise stated, "alkyl" includes a linear or branched monovalent saturated hydrocarbon group. For example, alkyl groups include methyl, ethyl, propyl, isopropyl, *n*-butyl, isobutyl, *sec*-butyl, *tert*-butyl, *n*-pentyl, 3-(2-methyl)butyl, 2-pentyl, 2-methylbutyl, neopentyl, *n*-hexyl, 2-hexyl, 2-methylpentyl, etc. Similarly, "1-6" in "$C_{1-6}$ alkyl" means that the group comprises 1, 2, 3, 4, 5 or 6 carbon atoms arranged in a linear or branched form.

The term "alkoxy" refers to an oxide ether form of the aforementioned linear or branched alkyl group, namely -O-alkyl.

The term "alkylene" refers to a divalent alkyl linking group. Formally, alkylene group refers to an alkane with two C-H bonds replaced by connection points between the alkylene group and the rest of the compound. Similarly, "$C_{1-3}$" in $C_{1-3}$ alkylene means the alkylene group contains 1, 2 or 3 carbon atoms, including, but is not limited to, methylene, 1,2-ethylene, 1,3-propylene or 1,2-isopropylene.

The term "haloalkyl" refers to an alkyl group with one or more H atoms replaced by halogen atoms.

The term "oxo" or "oxo group" refers to an oxygen atom in form of a divalent substituent, which forms a carbonyl group when it is connected to C, or forms a sulfoxide group, a sulfone group or an N-oxide group when it is connected to a heteroatom.

In the present application, unless otherwise stated, the term "aryl ring", "aromatic ring" or "aromatic heterocyclic ring" refers to a polyunsaturated carbon ring or heterocyclic ring with aromatic characteristics (having (4n+2) non-localized $\pi$ electrons, wherein n is an integer).

The term "aryl", in the present invention, unless otherwise stated, refers to an unsubstituted or substituted monocyclic or fused aryl group containing carbon ring atoms. $C_{6-18}$ aryl is preferred. More preferably, aryl is a $C_{6-10}$ monocyclic or bicyclic aromatic ring group. Phenyl or naphthalenyl is preferred; and naphthalenyl is the most preferred. The aryl ring can be fused onto a heteroaryl, heterocyclyl or cycloalkyl group, wherein the ring connected to the parent structure is the aryl ring. Non-limiting examples include, but are not limited to, benzocyclopentyl.

The term "heterocyclyl" refers to a ring system with at least one cyclic alkyl or cyclic alkenyl group containing a heteroatom, and the heteroatom is selected from the group consisting of N, O and/or S. The heterocyclyl may include monocyclic ring or polycyclic ring (for example, having 2, 3 or 4 fused rings, spiro rings, bridged rings, etc.). Heterocyclyl can be linked to another part of the compound via a ring-forming carbon atom or a ring-forming heteroatom. 3- to 14-membered heterocyclyl is preferred. In a 3- to 14-membered heterocyclyl, "3- to 14-membered" means the heterocyclyl is composed of 3 to 14 ring-forming atoms of C, N, O or S; wherein the nitrogen or sulfur heteroatom can be oxidized selectively, and the nitrogen heteroatom can be quaternized selectively. Examples of these heterocyclyl include, but are not limited to,

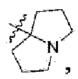, 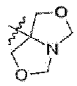, 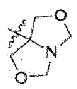, 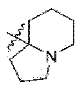, 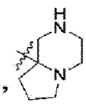, 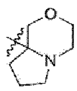,

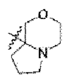, 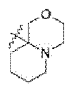, 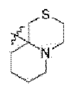, 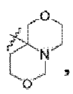, 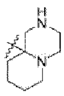, 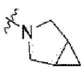, 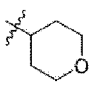, 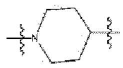, 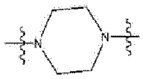, 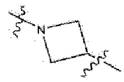, 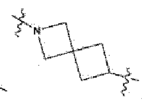,

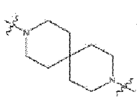 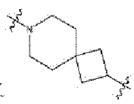, 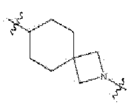, 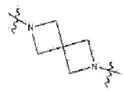, 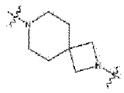, 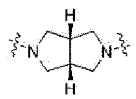, 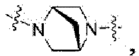, 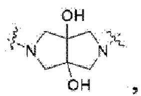,

azetidinyl, pyrrolidinyl, piperidinyl, piperazinyl, oxo-piperazinyl, oxo-piperidinyl, tetrahydrofuranyl, dioxolanyl, tetrahydroimidazolyl, tetrahydrothiazolyl, tetrahydrooxazolyl, tetrahydropyranyl, morpholinyl, thiomorpholinyl, thiomorpholinyl sulfoxide, thiomorpholinyl sulfone and tetrahydrooxadiazolyl. The heterocyclyl can be fused onto an aryl, heteroaryl or cycloalkyl ring, wherein the ring connected to the parent structure is the heterocyclyl.

The term "heteroaryl" in the present application, unless otherwise stated, refers to a monocyclic or polycyclic (e.g., having 2, 3 or 4 fused rings, spiro rings, bridged rings, etc.) heteroaryl ring with at least one heteroatom, wherein the heteroatom is selected from the group consisting of N, O and/or S, and wherein the nitrogen or sulfur heteroatom can be oxidized selectively, and the nitrogen heteroatom can be quaternized selectively. 5- to 18-membered heteroaryl is preferred, wherein "5- to 18-membered" in 5- to 18-membered heteroaryl means the heteroaryl composed of 5 to 18 ring atoms of C, N, O or S. 5- to 10-membered heteroaryl groups are more preferred. 5- to 6-membered heteroaryl groups are much more preferred. Examples of heteroaryl include, but are not limited to, thiophenyl, furanyl, imidazolyl, isoxazolyl, oxazolyl, pyrazolyl, pyrrolyl, thiazolyl, thiadiazolyl, triazolyl, pyridinyl, pyrazinyl, indolyl, azaindolyl, indazolyl, benzimidazolyl, benzofuranyl, benzothiophenyl, benzoisoxazolyl, benzothiazolyl, benzothiazolyl, benzothiadiazolyl, benzotriazolyl adenine, quinolyl or isoquinolyl. The heteroaryl can be fused onto an aryl, heterocyclyl or cycloalkyl ring, wherein the ring connected to the parent structure is the heteroaryl ring.

The term "cycloalkyl" refers to a cyclic system with at least one cyclic alkyl group. $C_{3-14}$ cycloalkyl is preferred, wherein "$C_{3-14}$" mans that the cycloalkyl group can have 3, 4, 5, 6, 7, 8, 9, 10, 11, 12, 13 or 14 ring-forming atoms. Cycloalkyl groups can include monocyclic ring and polycyclic ring (for example, having 2, 3 or 4 fused rings, spiro rings, bridged rings, etc.). In some embodiments, the cycloalkyl includes, but is not limited to, cyclopropyl, cyclobutyl, cyclopentyl, etc.; the cycloalkyl can be further fused onto an aryl, heterocyclyl or heterocyclyl ring, wherein the ring connected to the parent structure is the cycloalkyl.

The term "substituted" means that one or more hydrogen atoms in a group are substituted by the same or different substituents respectively. Typical substituents include, but are not limited to, halogen (F, Cl, Br or I), $C_{1-8}$ alkyl, $C_{3-12}$ cycloalkyl, $-OR^1$, $-SR^1$, =O, =S, $-C(O)R^1$, $-C(S)R^1$, $=NR^1$, $-C(O)OR^1$, $-C(S)OR^1$, $-NR^1R^2$, $-C(O)NR^1R^2$, cyano, nitro, $-S(O)_2R^1$, $-O-S(O_2)OR^1$, $-O-S(O)_2R^1$, and $-OP(O)(OR^1)(OR^2)$; wherein, $R^1$ and $R^2$ are independently selected from the group consisting of -H, $C_{1-6}$ alkyl, $C_{1-6}$ haloalkyl and $C_{3-6}$ cycloalkyl. In some embodiments, the substituent is independently selected from the groups including -F, -Cl, -Br, -I, -OH, trifluoromethoxy, ethoxy, propoxy, isopropoxy, *n*-butoxy, isobutoxy, *tert*-butoxy, $-SCH_3$, $-SC_2H_5$, formyl, $-C(OCH_3)$, cyano, nitro, $-CF_3$, $-OCF_3$, amino, dimethylamino, methylthio, sulfonyl and acetyl.

When the number of connecting group is 0, for example $-(CH_2)_0-$, it indicates that the connecting group is a bond.

The term "pharmaceutically acceptable salt" refers to a salt prepared from a non-toxic base or acid that are pharmaceutically acceptable.

When the compound provided by the present application is an acid, the corresponding salt can be conveniently prepared from a pharmaceutically acceptable non-toxic base, including inorganic bases and organic bases. Salts derived from inorganic bases include salts of aluminium, ammonium, calcium, copper (with high and low valence), trivalent iron, ferrous iron, lithium, magnesium, manganese (with high and low valence), potassium, sodium, zinc and the like. Salts of ammonium, calcium, magnesium, potassium and sodium are particularly preferred. Non-toxic organic bases that can be derived into pharmaceutically acceptable salts include primary amines, secondary amines and tertiary amines, as well as cyclic amines and amines containing substituents, such as naturally occurring and synthetic amines containing substituents. Other pharmaceutically acceptable non-toxic organic bases that can form salts include ion exchange resins as well as arginine, betaine, caffeine, choline, N',N'-dibenzylethylenediamine, diethylamine, 2-diethylaminoethanol, 2-dimethylaminoethanol, ethanolamine, ethylenediamine, N-ethylmorpholine, N-ethylpiperidine, reduced glucosamine, glucosamine, histidine, isopropylamine, lysine, methylglucosamine, morpholine, piperazine, piperidine, polyamine resin, procaine, chloroprocaine, purine, theobromine, triethylamine, trimethylamine, tripropylamine, trishydroxymethylaminomethane, etc.

When the compound provided in the present application is a base, the corresponding salt can be conveniently prepared from a pharmaceutically acceptable non-toxic acid, including inorganic and organic acids. Such acids include, for example, acetic acid, benzenesulfonic acid, benzoic acid, camphorsulfonic acid, citric acid, ethanesulfonic acid, formic acid, fumaric acid, gluconic acid, glutamic acid, hydrobromic acid, hydrochloric acid, hydroxyethanesulfonic acid, lactic acid, maleic acid, malic acid, mandelic acid, methanesulfonic acid, mucic acid, nitric acid, pamoic acid, pantothenic acid, phosphoric acid, succinic acid, sulfuric acid, oxalic acid, propionic acid, glycolic acid, hydriodic acid, perchloric acid, cyclohexylsulfamic acid, salicylic acid, 2-naphthalenesulfonic acid, saccharin acid, trifluoroacetic acid, tartaric acid, *p*-toluenesulfonic acid, etc. Citric acid, hydrobromic acid, formic acid, hydrochloric acid, maleic acid, phosphoric acid, sulfuric acid and tartaric acid are preferred. Formic acid and hydrochloric acid are more preferred.

Prodrugs of the compounds of the present invention are comprised within the protection scope of the present application. Generally, the prodrug refers to a functional derivative that can be easily converted into the desired compound in a body, for example, any pharmaceutically acceptable salt, ester, salt of ester or other derivative of the compound of the present application, which can directly or indirectly provide the compound of the present application or a pharmaceutically active metabolite or residue thereof after administration to a subject.

The compound described in the present application may contain one or more asymmetric centers, and may thereby resulting in diastereomers and optical isomers. The present application comprises all possible diastereomers and a racemic mixture thereof, a essentially pure separated enantiomer, all possible geometric isomers and medicinal salts thereof.

When the compound represented by formula (I) has tautomers, unless otherwise stated, the present application includes any possible tautomers and pharmaceutically acceptable salt thereof, as well as mixtures thereof.

The term "pharmaceutical composition" refers to a mixture consisting of one or more compounds or a pharmaceutically acceptable salt thereof of the present application and a pharmaceutically acceptable excipient. The aim of the pharmaceutical composition is to facilitate the administration of the compound of the present application to an organism.

In the present invention, "a", "an", "the", "at least one" and "one or more" can be used interchangeably. Therefore, for example, a mixture consisting of "a" pharmaceutically acceptable excipient can be interpreted as indicating that the pharmaceutical composition includes "one or more" pharmaceutically acceptable excipients.

The term "pharmaceutically acceptable excipients" refers to those excipients that have no obvious stimulating effect on an organism and do not damage the biological activity and performance of the active compound. Suitable excipients are well-known to those skilled in the art, such as carbohydrates, waxes, water-soluble and/or water-expellable polymers, hydrophilic or hydrophobic materials, gelatin, oil, solvents, water, etc.

The pharmaceutical composition of the present application can be prepared by combining the compound of the present application with an appropriate pharmaceutically acceptable excipient. For example, the pharmaceutical composition of the present application can be formulated into a solid, semi-solid, liquid or gaseous preparation, such as tablets, pills, capsules, powders, granules, ointments, emulsions, suspensions, suppositories, injections, inhalants, gels, microspheres, aerosols, etc.

The typical routes for administrating the compound or a pharmaceutically acceptable salt or pharmaceutical composition thereof of the present application include, but are not limited to, oral, rectal, local, inhalation, parenteral, sublingual, vaginal, intranasal, intraocular, intraperitoneal, intramuscular, subcutaneous, and intravenous administration.

The term "treatment" or any variant thereof generally refers to obtaining the desired pharmacological and/or physiological effect. This effect can be therapeutic depending on partially or completely stabilizing or curing the disease and/or the side effects resulting from the disease. The term "treatment" or any variant thereof used herein covers any treatment for a disease of a patient, including: (a) suppressing the symptom of the disease, that is, preventing its

progression; or (b) alleviating the symptom of the disease, that is, causing the disease or symptoms to regress.

The term "effective amount" means the dosage of the compound of the present application for (i) treating or preventing a specific disease, condition or disorder, (ii) alleviating, improving or eliminating one or more symptoms of a specific disease, condition or disorder, or (iii) preventing or delaying the onset of one or more symptoms of a specific disease, condition or disorder described herein. The amount of the compounds constituting the "therapeutic effective dose" of the present application varies depending on the compound, the disease status and severity thereof, the administration method, and the age of the mammal to be treated, but may be conventionally determined by a person skilled in the art based on their own knowledge and the content of the present disclosure.

**Brief Description of the Figures**

FIG. 1 is a figure showing the degradation effect of compound 2 on different KRAS in biological experiments.

FIG. 2 shows the Western Blotting experimental results of KRAS protein degradation by compound 135 on different cell lines in biological experiments.

FIG. 3 is a figure showing the efficacy investigation of compound 135 on a mouse xenograft tumor model in a biological experiment.

FIG. 4 is a figure showing the efficacy investigation of compound 144 on a mouse xenograft tumor model in a biological experiment.

FIG. 5 shows the Western Blotting experimental results of KRAS protein degradation by compound 144 on different cell lines in biological experiments.

**Examples**

In order to make the above disclosure more clear and definite, the following examples are described in the present application to further illustrate the technical solutions of the present application. The following examples are only used to illustrate the specific embodiments of the present invention, so that those skilled in the art can understand the present application, but are not intended to limit the protection scope of the present application. In specific embodiments of the present application, unless otherwise specified, the technical means or methods, etc. are conventional technical means or methods, etc. in the art.

Unless otherwise stated, all temperatures in the present application refer to degrees Celsius. The following abbreviations are used in the examples:

DMF: N,N-dimethylformamide;
NIS: N-iodosuccinimide;
THF: tetrahydrofuran;
EA: ethyl acetate;
PE: petroleum ether;
DCM: dichloromethane;
MeOH: methanol;
TBDPSCl: *tert*-butyldiphenylchlorosilane;
DPPA: diphenyl azidophosphate;
CDI: *N,N'*-carbonyldiimidazole;
DIPEA: *N*,*N*-diisopropylethylamine;
CataCXium A Pd G3:

mesylate[(di(1-adamantyl)-n-butylphosphine)-2-(2'-amino-1,1'-biphenyl)]palladium(II);
LAH/ $LiAlH_4$: lithium tetrahydroaluminate;
$POCl_3$ : phosphorus oxytrichloride;
DMSO: dimethylsulfoxide;
DMF-DMA: N,N-dimethylformamide dimethyl acetal;
TosMIC: *p*-toluenesulfonylmethyl isocyanide;
DCE: 1,2-dichloroethane;

DMAP: 4-dimethylaminopyridine;
$PhN(Tf)_2$: phenylbis(trifluoromethanesulfonyl)imide;
PBS: phosphate buffered saline;
Selectfluor: 1-chloromethyl-4-fluoro-1,4-diazabicyclo[2.2.2]octane bis(tetrafluoroborate);
TsOH: *p*-toluenesulfonic acid;
$(Boc)_2O$: di-tert-butyl dicarbonate;
TMSCl: chlorotrimethylsilane;
$Pd_2(dba)_3$: tris(dibenzylideneacetone)dipalladium;
HATU: 2-(7-azabenzotriazol-1-yl)-N,N,N',N'-tetramethyluronium hexafluorophosphate;
TFA: trifluoroacetic acid;
DABCOL: triethylene diamine;
DIBAL-H: diisobutyl aluminium hydride;
TMSCN: trimethylsilyl cyanide;
TMSOTf: trimethylsilyl trifluoromethanesulfonate;
LiHMDS: lithium bis(trimethylsilyl)amide;
$NaBH_3CN$: sodium cyanoborohydride;
TBAF: tetrabutylammonium fluoride;
NADPH: reduced nicotinamide adenine dinucleotide phosphate;
UDPGA: uridine diphosphateglucuronic acid;
UGT: uridine diphosphate glucuronosyltransferase;
TEA: triethylamine.

Synthesis of intermediate M1:

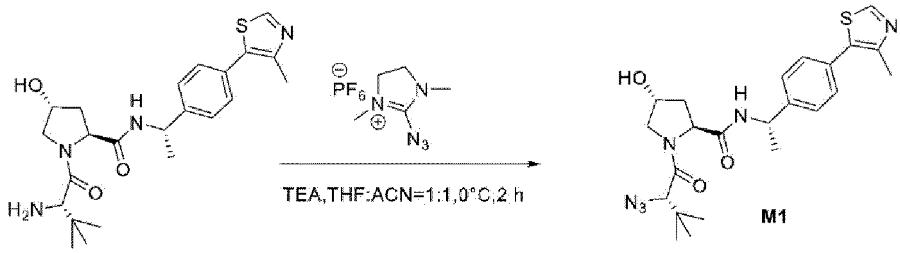


(2S,4R)-1-((S)-2-amino-3,3-dimethylbutanoyl)-4-hydroxy-N-((S)-1-(4-(4-methylthiazol-5-y l)phenyl)ethyl)pyrrolidine-2-carboxamide (1 g) was dissolved in THF (10 mL) and acetonitrile (10 mL). TEA (1.88 mL) was added, and a solution of 2-azido-1,3-dimethylimidazolinium hexafluorophosphate (0.64 g) in acetonitrile (2 mL) was added dropwise in an ice bath. The mixture was stirred in the ice bath for 2 h. 20.0 mL water was added to the reaction solution, and then the resultant was extracted with EA three times, 20 mL each time. The organic phases were combined, dried with sodium sulfate, filtered, concentrated, and then separated and purified by column chromatography to obtain intermediate M1 (0.8 g).

ESI-MS m/z: 471[M+H]$^+$.

Synthesis of intermediate M2:

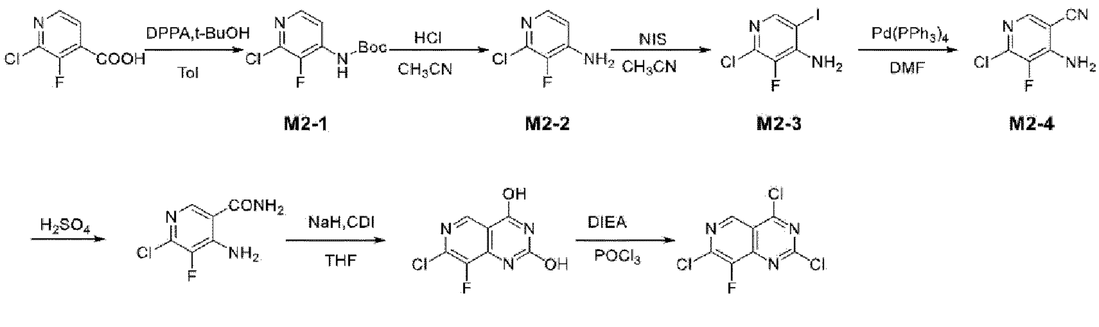


M2-1 M2-2 M2-3 M2-4

M2-5 M2-6 M2

Step 1: Synthesis of compound M2-1

At room temperature, 2-chloro-3-fluoro-pyridine-4-carboxylic acid (54.00 g), toluene (390.00 mL), *tert*-butanol (390.00 mL), triethylamine (128.27 mL), and powdered 4A molecular sieves (90.00 mL, pre-activated) were added successively. The temperature was maintained at reflux under nitrogen protection for half an hour (internal temperature 87°C). After natural cooling to room temperature, DPPA (99.44 mL) was added. The resultant was heated to reflux, and reacted under maintained temperature for 5 hours. The reaction solution was cooled to below 40°C, then diluted with 500 mL EA; and further cooled to room temperature. The resultant was filtered with diatomaceous earth to remove the added molecular sieves. The filter residue was rinsed multiple times with 1500 mL EA and dried under reduced pressure. The filtrate was collected, washed successively with 700 mL water and 700 mL saturated brine, and subjected to phase separation. The organic phases were dried with anhydrous sodium sulfate, filtered to remove the drying agent, and concentrated. The concentrate was separated and purified by column chromatography (PE/EA=30:1 to 20:1). The eluent was concentrated to finally obtain product M2-1 (68.2 g, 89.88% yield).
ESI-MS m/z: 247.1 $[M+H]^+$

Step 2: Synthesis of compound M2-2

Compound M2-1 (65.00 g) was dissolved in $CH_3CN$ (82.00 mL) at room temperature. The mixture was cooled in a water bath, and 4M hydrogen chloride (dioxane solution, 264 mL) was slowly added. The mixture was stirred at room temperature for about 16 hours, and a white solid precipitated in a suspended state. The reaction mixture was filtered, and the filter cake was rinsed with a small amount of acetonitrile, and dried under reduced pressure. The filtrate was discarded. The filter cake was collected and added to a mixture of 700 mL saturated sodium bicarbonate aqueous solution and 700 mL ethyl acetate. The resultant was alkalized, extracted, and subjected to phase separation. The aqueous phase was further extracted with 350 mL ethyl acetate and subjected to phase separation. The ethyl acetate phases were combined, washed with 300 mL saturated sodium chloride aqueous solution, and subjected to phase separation. The organic phase was dried with anhydrous sodium sulfate, filtered to remove the drying agent, and concentrated to obtain product M2-2 (36.3 g, 94.0% yield).

ESI-MS m/z: 147.1 $[M+H]^+$.

Step 3: Synthesis of compound M2-3

Compound M2-2 (36.00 g) was dissolved in acetonitrile (180.00 mL) at room temperature. NIS (66.32 g) and p-toluenesulfonic acid (2.12 g) were added thereto. The mixture was heated and maintained at 70°C for reaction under nitrogen protection. The reaction solution was cooled to 50°C, and 900 mL water was added. A pinkish-white solid powder precipitated. The resultant was slurried for half an hour and filtered. The filter cake was rinsed with water and dried under reduced pressure. The filter cake was collected and completely dissolved in 1200 mL ethyl acetate. The resultant was successively washed with 350 mL saturated sodium sulfite aqueous solution twice, then washed with 350 mL saturated brine and subjected to phase separation. The organic phase was dried with anhydrous sodium sulfate, filtered, and concentrated to obtain product M2-3 (63.2 g, 94.43% yield).
ESI-MS m/z: 272.9 $[M+H]^+$

Step 4: Synthesis of compound M2-4

Compound M2-3 (57.50 g) was dissolved in DMF (22.00 mL) at room temperature. Zinc cyanide (32.22 g), tetrakis(triphenylphosphine)palladium (12.19 g), and powdered 4Å molecular sieves (20.00 mL) were added thereto. The

mixture was heated and maintained at 100°C for reaction under nitrogen atmosphere for about 7 hours. The oil bath was removed, and the resultant was cooled naturally to room temperature for post-treatment. The reaction mixture was filtered with diatomaceous earth and dried under reduced pressure. The filtrate was collected and concentrated at 60°C to 70°C to obtain a pale yellow solid crude product. The filter residue was rinsed with 500 mL ethyl acetate and dried under reduced pressure. The rinsing liquid was collected, combined with the crude product, and then concentrated until no liquid was distilled out. The obtained solid crude product was dissolved in 700 mL ethyl acetate, washed with saturated brine three times, 250 mL each time, and subjected to phase separation. The organic phase was dried with anhydrous sodium sulfate, filtered, and concentrated to obtain a pale yellow solid. 160 mL PE/EA=3/1 mixture was added. The resultant was slurried for half an hour, filtered, and dried under reduced pressure. The filter cake was collected, concentrated in a 45°C water bath, then dried under reduced pressure with high-vacuum oil pump until constant weight. The final product M2-4 was obtained (36.1 g, 99.7% yield).

ESI-MS m/z: 172.0 $[M+H]^+$.

Step 5: Synthesis of compound M2-5

Concentrated sulfuric acid (61.37 mL) was added to a 500 mL single-necked flask at room temperature and cooled in an ice-water bath to below 10°C. Compound M2-4 (39.30 g) was added in batches. After the addition was completed, the mixture was stirred for 10 minutes under nitrogen atmosphere, maintained at 60°C in an oil bath, and reacted for about 1 hour. The reaction solution was cooled to room temperature, then carefully added to 1100 mL ice-water mixture for dilution and quenching. A small amount of yellow solid was precipitated. The resultant was stirred for 10 minutes and filtered. The filter cake was collected, added with 50 mL saturated sodium bicarbonate aqueous solution, slurried for 20 minutes, and filtered again. The two filtrates were collected and combined. Then, solid sodium carbonate was slowly added to adjust the pH to about 7, and an off-white solid was precipitated. The mixture was stirred for half an hour, filtered and dried under reduced pressure. The filter cake was rinsed with 100 mL water and dried under reduced pressure, which was repeated twice. The filter cake was collected, placed in a vacuum oven, and dried at 55°C to constant weight to obtain product M2-5 (33.6 g, 77.37% yield).

ESI-MS m/z: 190.0 $[M+H]^+$.

Step 6: Synthesis of compound M2-6

Tetrahydrofuran (470.00 mL) was added at room temperature. After nitrogen replacement, sodium hydride (10.00 g) was added under the protection of a slight nitrogen flow. The mixture was heated in an oil bath, maintained at 40°C to 45°C, and stirred for 15 minutes. Then, compound M2-5 (18.95 g) was added in batches. After the addition was completed, the resultant was mechanically stirred at maintained temperature for 20 minutes, and then CDI (24.31 g) was carefully added in batches. After the addition was completed, the resultant was stirred for 15 minutes, heated in an oil bath, and reacted at maintained temperature under reflux. The reaction solution was cooled to below 10°C in an ice-water bath. Then 500 mL saturated ammonium chloride aqueous solution was added. A pale yellow solid was precipitated. 1000 mL water was added. Then, the resultant was transferred into a 5 L beaker with 3000 mL additional water. After stirring for 1 hour, the resultant was filtered and dried under reduced pressure. The filter cake was collected, placed in a vacuum oven, and dried at 50°C to 55°C to constant weight to obtain product M2-6 (18.3 g, 84.93% yield).

ESI-MS m/z: 216.0 $[M+H]^+$.

Step 7: Synthesis of compound M2

Compound M2-6 (18.00 g) and DIPEA (36.00 mL) were dissolved in $POCl_3$ (180.00 mL) at room temperature. The mixture was heated and maintained at 100°C under nitrogen atmosphere for about 2.5 hours. The phosphorus oxychloride was removed by concentration under reduced pressure, followed by co-evaporation with 100 mL DCM twice. The concentrated residue was dissolved in 400 mL dichloromethane, which was then slowly added dropwise to 500 mL saturated sodium bicarbonate aqueous solution and cooled with ice-water. After stirring for 15 minutes, phase separation was performed. The aqueous phase was further extracted with 300 mL dichloromethane and subjected to phase separation. The dichloromethane phases were combined, washed with 300 mL saturated brine, and subjected to phase separation. The resultant was dried with anhydrous sodium sulfate, filtered, and concentrated. The concentrate was purified by silica gel column chromatography (PE/EA=90/10 to 75/25) to obtain product M2 (10.95 g, 51.94% yield).

ESI-MS m/z: 251.9 $[M+H]^+$.

Synthesis of intermediate M3:

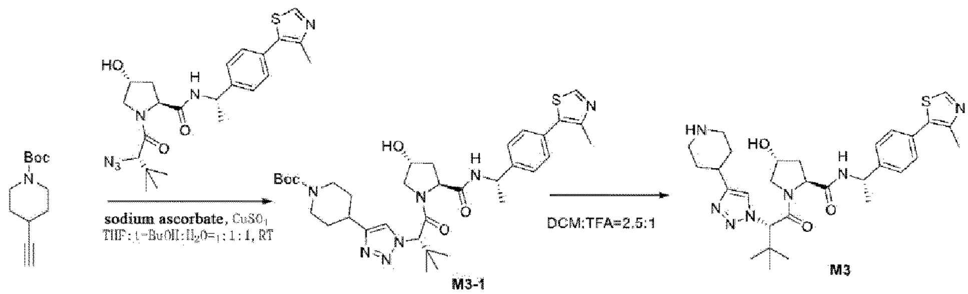


M3-1 M3

Step 1: Synthesis of compound M3-1

Tert-butyl 4-ethynylpiperidine-1-carboxylate (147 mg) and tert-butyl 4-(1-(((S)-1-((2S,4R)-4-hydroxy-2-((((S)-1-(4-(4-methylthiazol-5-yl)phenyl)ethyl)carbamoyl)pyr rolidin-1-yl)-3,3 -dimethyl-1-oxobutan-2-yl)-1H-1,2,3-triazol-4-yl)piperidine-1-carboxylate (330 mg) were dissolved in 6 mL THF, 6 mL $H_2O$, and 6 mL tert-butanol. Sodium ascorbate (360 mg) and anhydrous copper sulfate (100 mg) were successively added. The mixture was reacted at room temperature for 3 hours. 20.0 mL water was added to the reaction solution, and then the resultant was extracted with EA three times, 20 mL each time. The organic phases were combined and dried with sodium sulfate, filtered, concentrated, and then separated and purified by column chromatography to obtain compound M3-1 (420 mg).
ESI-MS m/z: 680[M+H]$^+$.

Step 2: Synthesis of compound M3

M3-1 (420 mg) was dissolved in 10 mL DCM, and then 3 mL TFA was added. The mixture was reacted at room temperature for 30 min. 1M NaOH aqueous solution was added to the reaction solution to free the acid. The resultant was extracted with DCM three times, 10 mL each time. The organic phases were combined, dried with anhydrous sodium sulfate, and filtered. The filtrate was subjected to distillation under reduced pressure to obtain compound M3 (365 mg).
ESI-MS m/z: 580 [M+H]$^+$.

Synthesis of intermediate M4:

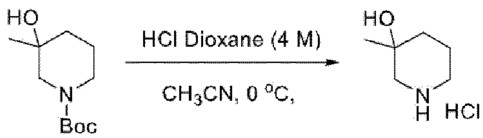


M4

N-(tert-butoxycarbonyl)-3-methyl-3-hydroxypiperidine (1.0 g) was added to a 25 mL single-necked flask. 3 mL acetonitrile was added, and 3 mL hydrochloric acid (4 M/Dioxane) was added at 0°C. The mixture was reacted at 0°C for 0.5 h. After the reaction was completed, the reaction solution was directly concentrated to obtain intermediate M4 (0.7 g, 99% yield), which was used directly in the next step.

Synthesis of intermediate M5:

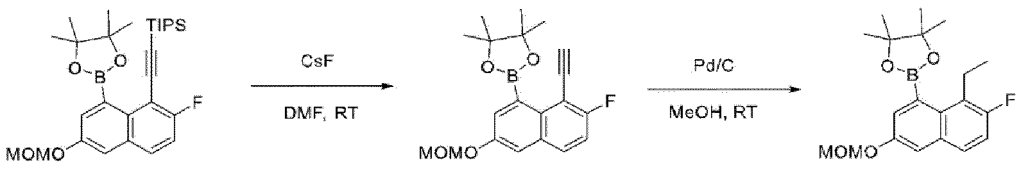


M5-1 M5

Step 1: Synthesis of compound M5-1

(((2-Fluoro-6-(methoxymethoxy)-8-(4,4,5,5-tetramethyl-1,3,2-dioxaborolan-2-yl)naphthale n-1-yl)ethynyl)triisopropylsilane (1.0 g) was added to a 50 mL single-necked flask. 6 mL DMF was added, followed by addition of cesium fluoride (5.9 g). The mixture was stirred at room temperature for 1 h. The raw materials were monitored by TLC until they were reacted completely. The reaction solution was slowly added dropwise to water (20 mL). A white solid was precipitated. The resultant was filtered, and the filter cake was collected and dried to obtain 0.65 g white solid, i.e. compound M5-1.

Step 2: Synthesis of compound M5

Compound M5-1 (0.65 g) was added to a 50 mL single-necked flask. 6 mL MeOH and Pd/C (600 mg, 10% purity) were added. The mixture was reacted under hydrogen atmosphere at room temperature for 20 min. After the reaction was completed, the resultant was filtered, and the filtrate was collected and concentrated. The concentrate was purified by column chromatography to obtain compound M5 (496 mg, 75.5% yield).

$^1$H NMR (500 MHz, Methanol-$d_4$) δ 7.56 (dd, *J* = 8.9, 5.8 Hz, 1H), 7.39 (d, *J* = 2.7 Hz, 1H), 7.36 (d, *J* = 2.7 Hz, 1H), 7.20 (t, *J* = 9.2 Hz, 1H), 5.27 (s, 2H), 3.50 (s, 3H), 3.12 (qd, *J* = 7.5, 2.5 Hz, 2H), 1.44 (s, 12H), 1.26 (t, *J* = 7.5 Hz, 3H).

Synthesis of intermediate M6:

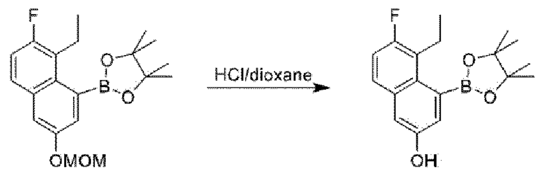


M5 M6

Intermediate M5 (1.0 g) was added to a 50 mL single-necked flask, and 10 mL 4M hydrogen chloride dioxane solution was added. The mixture was reacted at room temperature for half an hour. Then the reaction solution was neutralized with saturated sodium bicarbonate aqueous solution. The resultant was extracted with EA twice. The organic phases were combined and dried with anhydrous sodium sulfate and concentrated. The concentrate was purified by column chromatography (DCM : ammonia in methanol = 10 : 1) to obtain intermediate M6 (0.82 g).
$^1$H NMR (500 MHz, $CDCl_3$) δ 7.46 (dd, *J* = 8.9, 5.8 Hz, 1H), 7.27 (d, *J* = 2.7 Hz, 1H), 7.17 (dd,*J* = 12.5, 5.9 Hz, 1H), 7.12 (d, *J* = 2.7 Hz, 1H), 3.17 - 3.07 (m, 2H), 1.45 (s, 12H), 1.29 - 1.25 (m, 3H).

Synthesis of intermediate M7:

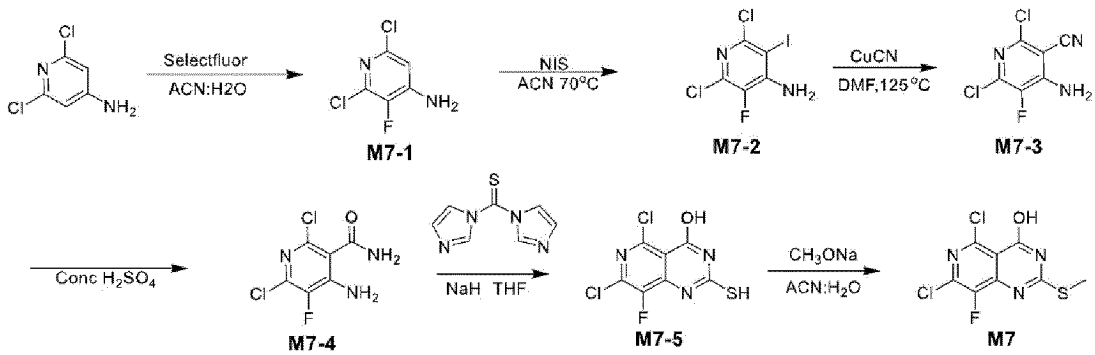


M7-1 M7-2 M7-3 M7-4 M7-5 M7

Step 1: Synthesis of compound M7-1

4-Amino-2,6-dichloropyridine (63 g) was added to a 1 L three-necked flask, and 440 mL ACN and 180 mL water

were added. The mixture was heated to 45°C, and selectfluor (164 g) was added. The reaction was exothermic and was allowed to cool naturally in air. After reacting for 10 min, the resultant was quenched with saturated sodium sulfite aqueous solution in an ice bath and extracted with EA three times. The organic phases were collected, combined, dried with anhydrous sodium sulfate and concentrated. The concentrate was purified by column chromatography (PE : EA =10 :1) to obtain compound M7-1 (42.6 g).

ESI-MS m/z: 163.9 $[M+H]^+$.

Step 2: Synthesis of compound M7-2

Compound M7-1 (54 g) was added to a 1 L three-necked flask, and 500 mL ACN, NIS (80 g), and TsOH (5.1 g) were added. The mixture was heated to 70°C and reacted for 1 h. 2.5 L water was added dropwise, and the resultant was filtered. The filter cake was dissolved in EA. After phase separation, the organic phase was concentrated to obtain compound M7-2 (94 g). ESI-MS m/z: 307.2 $[M+H]^+$.

Step 3: Synthesis of compound M7-3

Compound M7-2 (88 g) was added to a 1 L three-necked flask, 500 mL DMF was added, and CuCN (31 g) was added. The mixture was heated to 125°C and reacted for 16 h. 2 L water was added dropwise to the resultant. Then the resultant was added into 2 L water and filtered. The filter cake was washed with water and dissolved in EA. The aqueous phase was extracted with EA twice. The organic phases were collected, combined, and then concentrated to obtain compound M7-3 (55.4 g).

ESI-MS m/z: 207.0 $[M]^-$.

Step 4: Synthesis of compound M7-4

Compound M7-3 (88 g) was added to a 250 mL three-necked flask, and 83 mL concentrated sulfuric acid and 9 mL water were added. The mixture was reacted at 60°C for 16 h, then supplemented with 70 mL concentrated sulfuric acid and 5 mL water. The resultant was further reacted for 24 h. The reaction solution was cooled to 0°C, slowly poured into 2 L ice-water, and filtered. The filter cake was washed with water and dissolved in EA. The filtrate was extracted with EA twice. The organic phases were collected, combined, concentrated, and then slurried with PE:EA=1:1. The slurry was filtered to obtain compound M7-4 (59 g).

ESI-MS m/z: 224.1 $[M+H]^+$.

Step 5: Synthesis of compound M7-5

Compound M7-4 (58 g) was added to a 1 L three-necked flask, and 650 mL THF was added. The mixture was heated to 40°C, and NaH (16 g) was slowly added in batches. After stirring for 10 min, the resultant was heated to 60°C, and N,N'-thiocarbonyldiimidazole (69 g) was slowly added. After reacting for 1 h, the reaction was quenched with saturated ammonium chloride solution. Dilute hydrochloric acid was added dropwise to adjust the pH=4 to 5. THF was removed by rotary evaporation. The resultant was filtered, and the filter cake was washed with water and dissolved in methanol. The organic phase was concentrated to obtain crude product M7-5 (85 g).

ESI-MS m/z: 267.0 $[M+H]^+$.

Step 6: Synthesis of compound M7

Compound M7-5 (74 g) was added to a 1 L three-necked flask, 750 mL ACN was added, 22 mL methyl iodide was added, and sodium methoxide (23 g) aqueous solution (100 mL) was added. The mixture was reacted at room temperature for 15 min. The reaction solution was added to 3.5 L water. Dilute hydrochloric acid was added to adjust the pH=4 to 5. The resultant was filtered, and the filter cake was slurried with a mixed solvent of PE:EA=2:1. The slurry was filtered to obtain the filter cake, i.e. compound M7 (42.2 g).

ESI-MS m/z: 281.2 $[M+H]^+$.

Synthesis of intermediate M8:

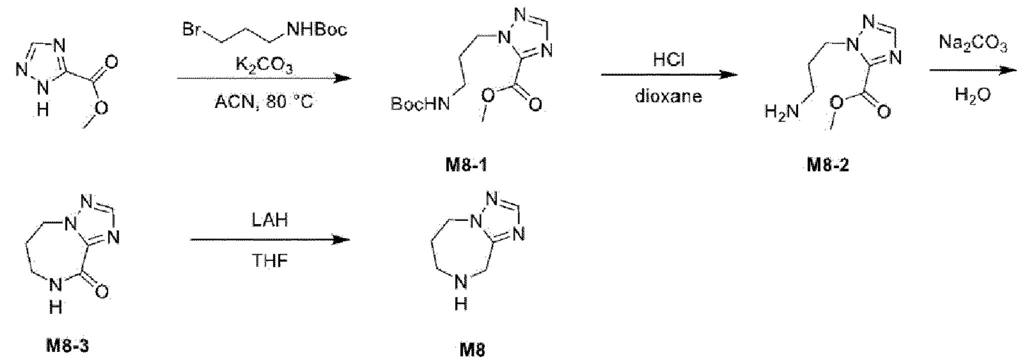

M8-1 M8-2 M8-3 M8

Step 1: Synthesis of compound M8-1

Potassium carbonate (4.78 g) was added to a suspension of methyl 1H-1,2,4-triazole-5-carboxylate (4.00 g) and N-tert-butoxy-3-aminobromopropyl bromide (7.49 g) in acetonitrile (40 mL) at room temperature. Then the system was stirred in an oil bath at 80°C. After reacting for 1 hour, ethyl acetate and water were added to the reaction system for extraction and phase separation. The aqueous phase was re-extracted with ethyl acetate. The organic phase was washed with saturated brine (50 mL) once, dried with anhydrous sodium sulfate, filtered, and concentrated to obtain crude product M8-1 (8.90 g, 99% yield). ESI-MS m/z = 285.2$[M+H]^+$.

Step 2: Synthesis of compound M8-2

Compound M8-1 (8.90 g) was dissolved in 4 N HCl in dioxane solution (40 mL), and stirred at room temperature for 1 hour. After the reaction was completed, the reaction solution was dried by rotary evaporation to obtain M8-2 (5.60 g, 97% yield), which was used directly in the next step. ESI-MS m/z = 185.1$[M+H]^+$.

Step 3: Synthesis of compound M8-3

Compound M8-2 (5.60 g) and sodium carbonate (9.96 g) were added to 100 mL water at room temperature. The reaction system was stirred at room temperature for 5 hours. After the reaction was completed, the reaction solution was adjusted to neutral with dilute hydrochloric acid, and then concentrated to remove water. The obtained solid residue was washed through a short silica gel column with organic solvent (DCM:MeOH = 20:1). The filtrate was concentrated to obtain compound M8-3 (1.32 g, 28% yield). ESI-MS m/z = 153.2$[M+H]^+$.

Step 4: Synthesis of compound M8

Compound M8-3 (1.20 g) was added to a suspension of $LiAlH_4$ (0.75 g) in THF (24 mL) at room temperature. The mixture was stirred at room temperature for 4 hours. After the reaction was completed, the resultant was cooled to 0°C. Water (0.75 mL), 15% sodium hydroxide solution (0.75 mL), and water (2.25 mL) were successively added. The resultant was then warmed to room temperature and stirred for 10 minutes. After that, an appropriate amount of anhydrous magnesium sulfate was added, followed by stirring for another 10 minutes. The resultant was filtered, and the filter residue was rinsed with an appropriate amount of organic solvent (DCM:MeOH = 10:1). The filtrate was concentrated to obtain a crude product. The crude product was purified by column chromatography (MeOH:DCM = 0% to 10%) to obtain compound M8 (0.62 g, 57% yield). ESI-MS m/z = 139.2$[M+H]^+$.

Synthesis of intermediate M9:

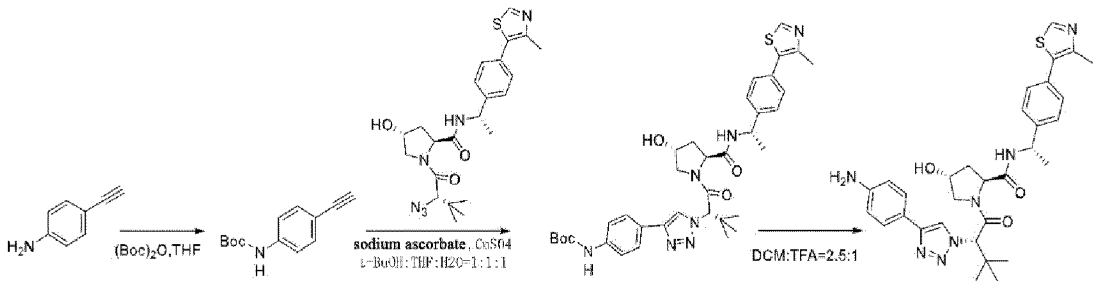


M9-1 M9-2 M9

Step 1: Synthesis of compound M9-1

4-Ethynylaniline (1g) was dissolved in 10 mL THF, and then $(Boc)_2O$ (2.24g) was added. The mixture was reacted at 55°C for 24 hours. 20.0 mL water was added to the reaction solution, and the resultant was extracted with EA three times, 20 mL each time. The organic phases were combined, dried with sodium sulfate, filtered, concentrated, and then separated and purified by column chromatography to obtain compound M9-1 (1.78 g). ESI-MS m/z: 218$[M+H]^+$.

Step 2: Synthesis of compound M9-2

M9-1 (152 mg) and M1 (330 mg) were dissolved in 6 mL THF, 6 mL $H_2O$, and 6 mL tert-butanol. Then, sodium ascorbate (360 mg) and anhydrous copper sulfate (100 mg) were successively added. The mixture was reacted at room temperature for 3 hours. 20.0 mL water was added to the reaction solution, and then the resultant was extracted with EA three times, 20 mL each time. The organic phases were combined, dried with sodium sulfate, filtered, concentrated, and then separated and purified by column chromatography to obtain compound M9-2 (450 mg). ESI-MS m/z: 688$[M+H]^+$.

Step 3: Synthesis of compound M9

M9-2 (450 mg) was dissolved in 10 mL DCM, and then 3 mL TFA was added. The mixture was reacted at room temperature for 30 min. 1 M NaOH aqueous solution was added to the reaction solution to adjust pH = 7, and then the resultant was extracted with DCM three times, 10 mL each time. The organic phases were combined, dried with anhydrous sodium sulfate and concentrated under reduced pressure to obtain compound M9 (320 mg). ESI-MS m/z: 588 $[M+H]^+$.

Synthesis of intermediate M10:

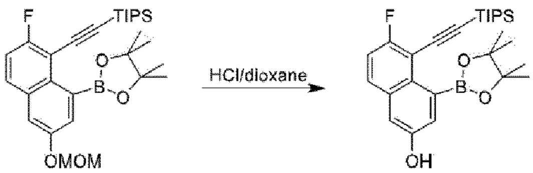


M10-1 M10

Compound M10-1 ((2-fluoro-6-(methoxymethoxy)-8-(4,4,5,5-tetramethyl-1,3,2-dioxa borolan-2-yl)naphthalen-1-yl)ethynyl)triisopropylsilane (10 g) was added to a 100 mL single-necked flask, and 30 mL 4 M hydrogen chloride dioxane solution was added. The mixture was reacted at room temperature for half an hour. Then saturated sodium bicarbonate aqueous solution was added in an ice bath to neutralize the reaction solution. The resultant was filtered, and the filter cake was washed with water twice, 20 mL each time. The resultant was dissolved in EA, dried with anhydrous sodium sulfate, and concentrated. The concentrate was purified by column chromatography (PE : EA =82 : 18) to obtain intermediate M10 (9.1 g).

Synthesis of intermediate M11:

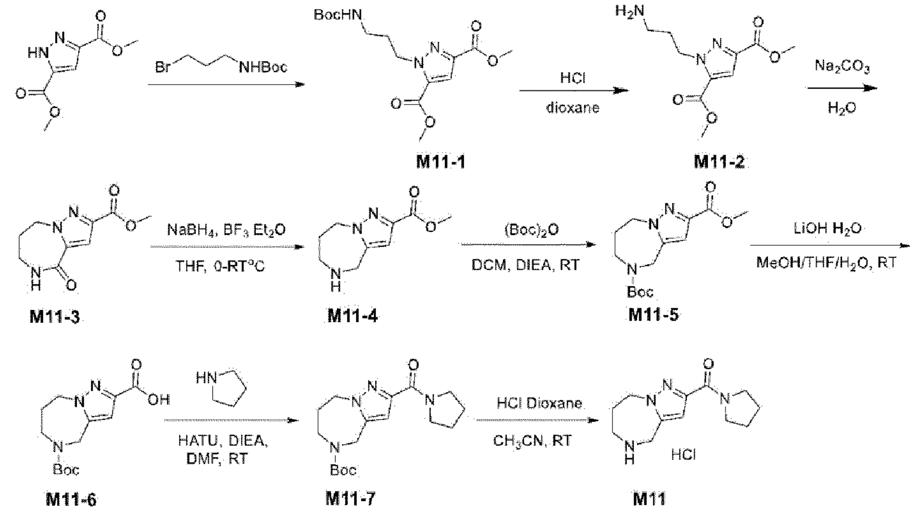


The synthesis of steps 1 to 3 refers to the synthesis of intermediate M8.

Step 4: Synthesis of compound M11-4

Compound M11-3 (3.4 g) was added to a 250 mL three-necked flask, and 100 mL THF was added. $NaBH_4$ (3.1 g) was added in batches. Then, boron trifluoride ethyl ether (14 mL) was slowly added dropwise at 0°C under $N_2$ atmosphere. After the addition was completed, the reaction solution was allowed to warm up naturally to room temperature, and the reaction continued overnight. After the reaction was completed, ethanol (50 mL) was slowly added to the reaction solution in an ice bath to quench the reaction. THF and ethanol were removed by concentration under reduced pressure. Ethanol (70 mL), water (20 mL), and 10% hydrochloric acid solution (10 mL) were added to the residue. The resultant was stirred at room temperature for 4 h, concentrated under reduced pressure, adjusted to pH = 8 with saturated $NaHCO_3$ solution, and then extracted with DCM/MeOH (10:1) three times, 40 mL each time. The organic phases were combined, dried with anhydrous sodium sulfate, filtered, and dried by rotary evaporation to obtain crude product M11-4 (2.8 g), which was used directly in the next step. ESI-MS m/z: 196.1 $[M+H]^+$.

Step 5: Synthesis of compound M11-5

Compound M11-4 (2.8 g) was added to a 100 mL single-necked flask, and 25 mL DCM and DIPEA (5.9 mL) were added. $(Boc)_2O$ (5.0 g) was added in an ice bath. The mixture was warmed up to room temperature, and reacted for 3 h. After the reaction was completed, the reaction solution was added to saturated ammonium chloride solution. DCM was added for extraction. The organic phase was collected, and the aqueous phase was re-extracted with DCM twice. The organic phases were combined, dried with anhydrous sodium sulfate, filtered, and concentrated. The concentrate was purified by column chromatography (PE:EA = 3:1) to obtain 3.0 g compound M11-5. ESI-MS m/z: 296.2 $[M+H]^+$.

Step 6: Synthesis of compound M11-6

Compound M11-5 (2.5 g) was added to a 100 mL single-necked flask, and 12 mL MeOH and 12 mL THF were added. Lithium hydroxide monohydrate (1.8 g, dissolved in 12 mL $H_2O$) was added. The mixture was stirred at room temperature for 0.5 h. After the reaction was completed, water (12 mL) was added to dilute the reaction solution. The resultant was concentrated under reduced pressure. The aqueous phase was adjusted to pH = 5 to 6 with acetic acid, and then extracted with DCM/MeOH (10:1) three times, 40 mL each time. The organic phases were combined, dried with anhydrous sodium sulfate, filtered, and concentrated to obtain crude product (3.1 g), which was used directly in the next step. ESI-MS m/z: 282.2 $[M+H]^+$.

Step 7: Synthesis of compound M11-7

Compound M11-6 (1.4 g) was added to a 100 mL single-necked flask, and 10 mL DMF was added. Pyrrolidine (0.71 g), DIPEA (3.29 mL), and HATU (2.84 g) were successively added. The mixture was stirred at room temperature for 1 h. After the reaction was completed, EA and water were added to the reaction solution for extraction. The organic phase was collected, and the aqueous phase was re-extracted with EA twice. The organic phases were combined, dried with anhydrous sodium sulfate, filtered, and concentrated. The concentrate was purified by column chromatography (DCM:EA

= 3:1) to obtain 1.5 g compound M11-7. ESI-MS m/z: 335.2 [M+H]+.

Step 8: Synthesis of compound M11

Compound M11-7 (1.5 g) was added to a 50 mL single-necked flask, and 5 mL $CH_3CN$ was added. 5 mL hydrogen chloride (4 M/Dioxane) was added. The mixture was stirred at room temperature for 0.5 h. After the reaction was completed, the reaction solution was directly concentrated to obtain intermediate M11 (0.98 g). ESI-MS m/z: 235.3 [M+H]+.

Synthesis of intermediate M12:

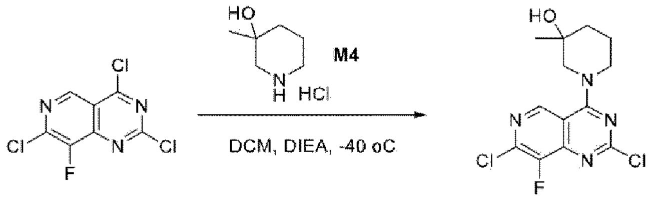


M2 M12

Intermediate M2 (500 mg) was added to a 25 mL single-necked flask. Intermediate M4 (275 mg) and *N,N*-diisopropylethylamine (1.0 g) were added at -40°C. The reaction was maintained at -40°C and continued for 0.5 h. After the reaction was completed, dichloromethane and water were added to the reaction solution for extraction. The organic phase was collected, and the aqueous phase was re-extracted with dichloromethane twice. The organic phases were collected, combined, dried with anhydrous sodium sulfate, and concentrated. The concentrate was purified by column chromatography (PE:EA = 3:2) to obtain compound M12 (385 mg, 64.6% yield). ESI-MS m/z: 331.3 [M+H]+.

Synthesis of intermediate M13:

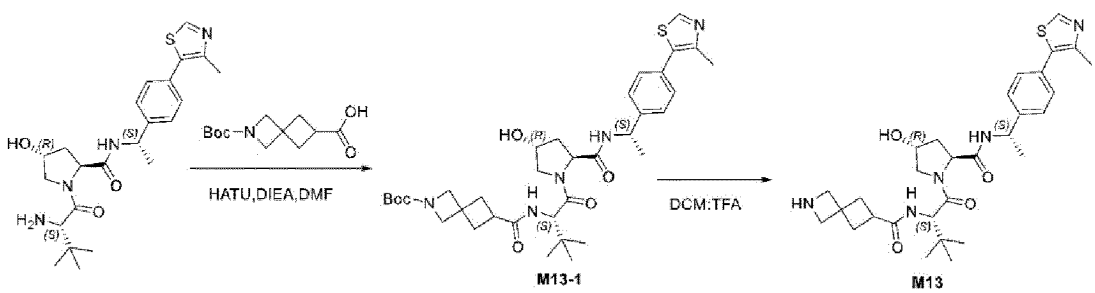


M13-1 M13

Step 1: Synthesis of compound M13-1

(2S,4R)-1-((S)-2-amino-3,3-dimethylbutanoyl)-4-hydroxy-N-((S)-1-(4-(4-methylthiazol-5-y l)phenyl)ethyl)pyrrolidine-2-carboxamide (304 mg), 2-(tert-butoxycarbonyl)-2-azaspiro[3.3]heptane-6-carboxylic acid (150 mg), and HATU (284 mg) were dissolved in 5 mL DMF. Then DIPEA (0.31 mL) was added. The mixture was reacted at room temperature for 30 minutes. 20.0 mL water was added to the reaction solution, and the resultant was then extracted with EA three times, 20 mL each time. The organic phase was combined, dried with sodium sulfate, filtered, concentrated, and then separated and purified by column chromatography to obtain compound M13-1 (400 mg). ESI-MS m/z: 668[M+H]+.

Step 2: Synthesis of compound M13

M13-1 (135 mg) was dissolved in 3 mL DCM, and then 1 mL TFA was added. The mixture was reacted at room temperature for 30 min. 1 M NaOH aqueous solution was added to the reaction solution to adjust pH=7. The resultant was extracted with chloroform:isopropanol = 3:1 three times, 5 mL each time. The organic phases were combined, dried with anhydrous sodium sulfate, filtered, and concentrated under reduced pressure to obtain M13 (110 mg). ESI-MS m/z: 568 [M+H]+.

Synthesis of intermediate M14:

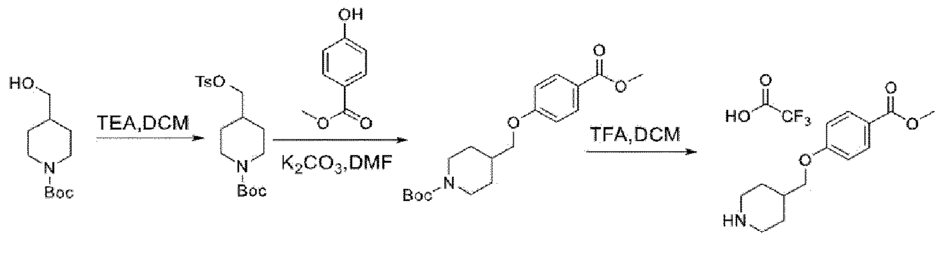


M14-1 M14-2 M14

Step 1: Synthesis of compound M14-1

Tert-butyl 4-(hydroxymethyl)piperidine-1-carboxylate (5.0 g) was dissolved in DCM (50 mL) at room temperature. Triethylamine (9.68 mL) and p-toluenesulfonyl chloride (5.31 g) were successively added. The mixture was reacted at room temperature for 15 h. An appropriate amount of water was added to the reaction solution to quench the reaction. Then an appropriate amount of ethyl acetate was added for extraction. The organic phase was collected, dried with anhydrous sodium sulfate, filtered, and evaporated under reduced pressure to remove the solvent to obtain 8.02 g product, i.e. compound M14-1, which was used directly in the next step.

Step 2: Synthesis of compound M14-2

Compound M14-1 (7.0 g) and methyl 4-hydroxybenzoate (2.6 g) were dissolved in DMF (30.0 mL). Potassium carbonate (4.72 g) was added. The mixture was reacted at 60°C for 10 h. An appropriate amount of water was added to the reaction solution to quench the reaction. Then an appropriate amount of ethyl acetate was added for extraction. The organic phase was collected, dried with anhydrous sodium sulfate, filtered, evaporated under reduced pressure to remove the solvent, and then purified by column chromatography to obtain 4.02 g product, i.e. compound M14-2. ESI-MS m/z: 350 $[M+H]^+$.

Step 3: Synthesis of compound M14

M14-2 (3.5 g) was dissolved in DCM (30.0 mL), then trifluoroacetic acid (10.0 mL) was added. The mixture was reacted at room temperature for 0.5 h. The reaction was monitored by LC-MS until it was completed. The reaction solution was directly concentrated, redissolved in 30.0 mL DCM, and then reconcentrated to obtain 2.5 g product trifluoroacetate salt, i.e. compound M14, which was used directly in the next step. ESI-MS m/z: 250 $[M+H]^+$.

Synthesis of intermediate M15:

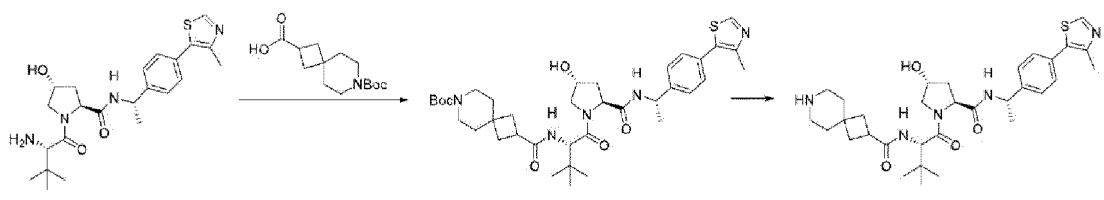

M15-1 M15

Step 1: Synthesis of compound M15-1

7-Tert-butoxycarbonyl-7-azaspiro[3.5]nonane-2-carboxylic acid (200.0 mg) and (2S,4R)-1-((S)-2-amino-3,3-dimethylbutanoyl)-4-hydroxy-N-((S)-1-(4-(4-methylthiazol-5-yl)ph enyl)ethyl)pyrrolidine-2-carboxamide (300.0 mg) were dissolved in 5.00 mL DMF. N,N-diisopropylethylamine (0.35 mL) and N,N,N',N'-tetramethyl-O-(7-azabenzotriazol-1-yl) uronium hexafluorophosphate (310.0 mg) were added. The mixture was reacted at room temperature for 30 minutes. The

reaction was monitored by LC-MS until it was completed. Water (100.0 mL) was added to the reaction solution, and the resultant was filtered to obtain a precipitated solid from the system, which was separated and purified by column chromatography to obtain 400.0 mg pale yellow solid, i.e. compound M15-1. ESI-MS m/z: 696[M+H]$^+$.

Step 2: Synthesis of compound M15

M15-1 (400.0 mg) was dissolved in 5.00 mL DCM, and then TFA (2.00 mL) was added. The mixture was reacted at room temperature for 0.5 h. The reaction solution was added dropwise to saturated sodium bicarbonate solution. An appropriate amount of DCM/MeOH=10/1 was added for extraction. The organic phase was collected, dried with anhydrous sodium sulfate, filtered, and concentrated under reduced pressure to obtain compound M15. ESI-MS m/z: 596 [M+H]$^+$.

Synthesis of intermediate M16:

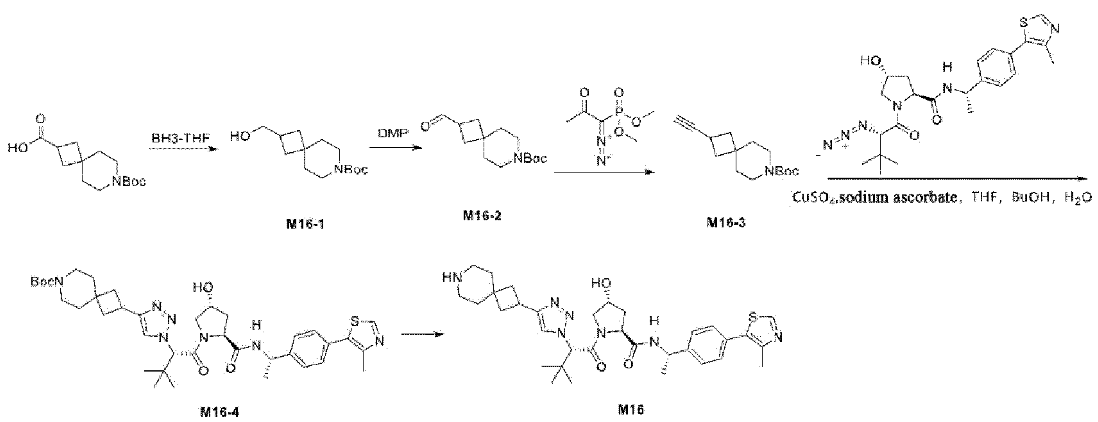


Step 1: Synthesis of compound M16-1

7-Tert-butoxycarbonyl-7-azaspiro[3.5]nonane-2-carboxylic acid (500.0 mg) was dissolved in 5.0 mL THF, and borane tetrahydrofuran solution (4.64 mL, 1 mol/L) was added. The mixture was reacted at room temperature for 1 h. 5 mL HCl/MeOH solution was slowly added to the reaction solution, and the mixture was stirred until gas evolution ceased. 1 mol/L NaOH solution was added to adjust the pH to alkaline, and then an appropriate amount of ethyl acetate was added for extraction. The organic phase was collected, dried with anhydrous sodium sulfate, filtered, and concentrated under reduced pressure to obtain compound M16-1 (450.0 mg). ESI-MS m/z: 200 [M+H-56]$^+$.

Step 2: Synthesis of compound M16-2

M16-1 (250.0 mg) was dissolved in 5.0 mL DCM, and Dess-Martin reagent (387.0 mg) was added. The mixture was reacted at room temperature for 1 h. An appropriate amount of ethyl acetate was added to the reaction solution. Solids were precipitated, filtered and removed. 1 mol/L NaOH solution was added to the filtrate to adjust the pH to alkaline, which was then extracted and separated. The organic phase was collected, dried with anhydrous sodium sulfate, filtered, and concentrated under reduced pressure to obtain compound M16-2 (180.0 mg). ESI-MS m/z: 198 [M+H-56]$^+$.

Step 3: Synthesis of compound M16-3

Compound M16-2 (180 mg) was dissolved in 3.0 mL anhydrous methanol under $N_2$ protection. Anhydrous potassium carbonate (167 mg) and dimethyl (1-diazo-2-oxopropyl)phosphonate (174 mg) were added. The mixture was reacted at room temperature for 3 h. An appropriate amount of water and ethyl acetate were added to the reaction solution for extraction. The organic phase was collected, dried with anhydrous sodium sulfate, filtered, and concentrated under reduced pressure to obtain compound M16-3 (110 mg). ESI-MS m/z: 194 [M+H-56]$^+$.

Step 4: Synthesis of compound M16-4

Compound M16-3 (110 mg), (2S,4R)-1-((S)-2-azido-3,3-dimethylbutanoyl)-4-hydroxy-N-((S)-1-(4-(4-methylthiazol-5-yl)phe nyl)ethyl)pyrrolidine-2-carboxamide (229 mg), anhydrous copper sulfate (63.3 mg), and sodium ascorbate (227 mg) were dissolved in a mixed solvent of 4.0 mL tert-butanol, 4.0 mL water and 4.0 mL THF under $N_2$ protection. The mixture was reacted at room temperature for 2 h. An appropriate amount of water and DCM/MeOH=10/1 solution were added to the reaction solution for extraction. The organic phase was collected, dried with anhydrous sodium sulfate, filtered, and concentrated under reduced pressure. The concentrate was purified by column chromatography to obtain compound M16-4 (250 mg). ESI-MS m/z: 664 $[M+H-56]^+$.

Step 5: Synthesis of compound M16

M16-4 (250 mg) was dissolved in 5.00 mL DCM under $N_2$ protection, then 2.00 mL TFA was added. The mixture was reacted at room temperature for 0.5 h. The reaction solution was added dropwise to saturated sodium bicarbonate solution. An appropriate amount of DCM/MeOH=10/1 was added for extraction. The organic phase was collected, dried with anhydrous sodium sulfate, filtered, and concentrated under reduced pressure to obtain compound M16 (150.0 mg). ESI-MS m/z: 620 $[M+H]^+$.

Synthesis of intermediate M17:

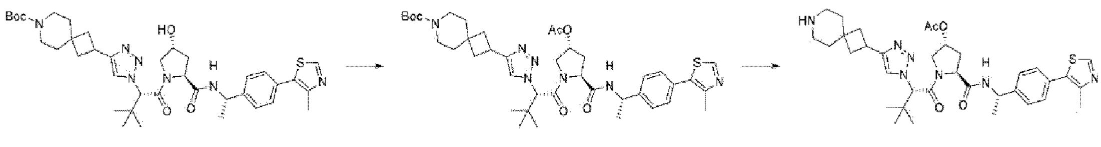

M16-4 M17-1 M17

Step 1: Synthesis of compound M17-1

M16-4 (100.0 mg) was dissolved in DCM (2.0 mL) in an ice bath. Triethylamine (0.046 mL), 4-dimethylaminopyridine (1.7 mg), and acetyl chloride (0.019 mL) were added successively. The mixture was reacted in an ice bath for 1 h. An appropriate amount of water and DCM were added to the reaction solution for extraction. The organic phase was collected, dried with anhydrous sodium sulfate, filtered, evaporated under reduced pressure to remove the solvent, and then purified by column chromatography to obtain 80 mg product, i.e. compound M17-1. ESI-MS m/z:762 $[M+H]^+$.

Step 2: Synthesis of compound M17

M17-1 (80.0 mg) was dissolved in DCM (2.0 mL) in an ice bath, and 1.00 mL TFA was added. The mixture was reacted at room temperature for 0.5 h. The reaction solution was concentrated directly, redissolved in 5.00 mL DCM, and reconcentrated to obtain 80 mg product trifluoroacetate salt, i.e. compound M17, which was used directly in the next step. ESI-MS m/z:662 $[M+H]^+$.

Synthesis of intermediate M18:

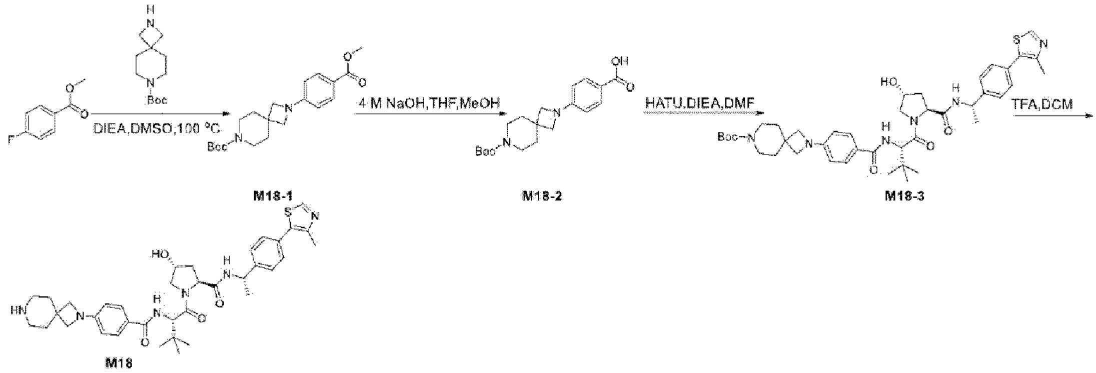


M18-1 M18-2 M18-3

M18

Step 1: Synthesis of compound M18-1

Methyl 4-fluorobenzoate (0.15 g) was added in a 25 mL single-necked flask, and dissolved in DMSO (3 mL). Tert-butyl 2,7-diazaspiro[3.5]nonane-7-carboxylate and DIPEA (0.48 mL) were added. The mixture was heated to 100°C and reacted overnight. The reaction solution was cooled to room temperature, and 10 mL water was added. Solid was participated and filtered. The filter cake was rinsed with water and dried to obtain 0.2 g compound M18-1.

Step 2: Synthesis of compound M18-2

Compound M18-1 (0.2 g) was added to a 25 mL single-necked flask, and dissolved in THF (1 mL). 4 M NaOH (1 mL) and MeOH (1 mL) were added. The mixture was heated to 40°C and reacted for 3 h. The reaction solution was cooled to room temperature and concentrated directly. 5 mL water was added to the reaction solution. The pH of the solution was adjusted to 3 to 4 with 1 M HCl solution. Solid was participated and filtered. The filter cake was rinsed with water and dried to obtain 0.17 g compound M18-2.

Step 3: Synthesis of compound M18-3

Compound M18-2 (140.0 mg) was added to a 25 mL single-necked flask, and dissolved in DMF (3 mL). (2S,4R)-1-((S)-2-amino-3,3-dimethylbutanoyl)-4-hydroxy-N-(4-(4-methylthiazol-5-yl)benzyl)pyrrolidine-2-carboxamide hydrochloride (180.0 mg), DIPEA (0.13 mL) and HATU (231.0 mg) were added. The mixture was reacted at room temperature for 2 h. Saturated brine (5 mL) was added to the reaction solution, and the resultant was extracted with EA. The organic phase was collected, dried with anhydrous sodium sulfate, and separated by column chromatography (DCM:MeOH=12:1) to obtain 276.0 mg compound M18-3.

Step 4: Synthesis of compound M18

Compound M18-3 (276.0 mg) was added to a 25 mL single-necked flask, and dissolved in DCM (2 mL). TFA (2 mL) was added. The mixture was reacted at room temperature for 1 h. The reaction solution was directly dried by evaporation. 5 mL DCM was added to the reaction solution. The pH of the solution was adjusted to 8 to 9 with 1 M NaOH solution. The resultant was extracted with DCM three times. The organic phase was washed with water and saturated brine, and dried by evaporation to obtain 214.0 mg compound M18.

Synthesis of intermediate M19:

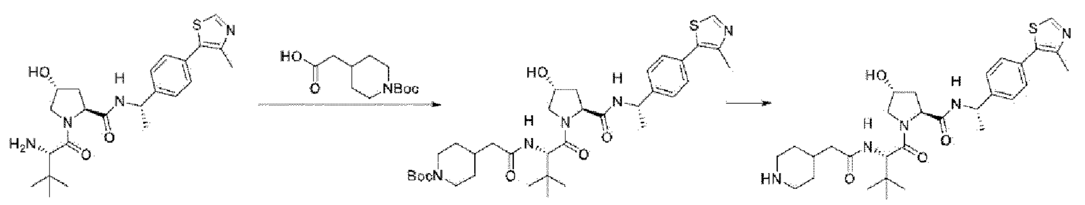

M19-1 M19

The synthetic steps of M19 refer to that of M18.

Synthesis of intermediate M20:

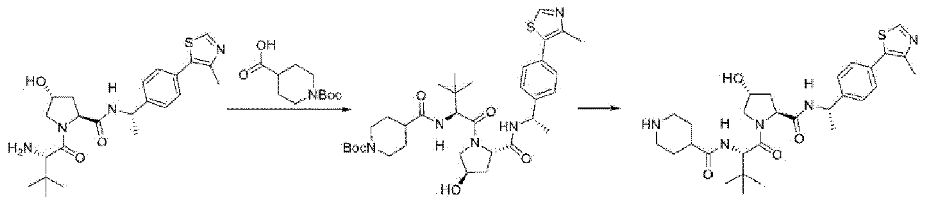

M20-1 M20

The synthetic steps of M20 refer to that of M18.

Synthesis of intermediate M21:

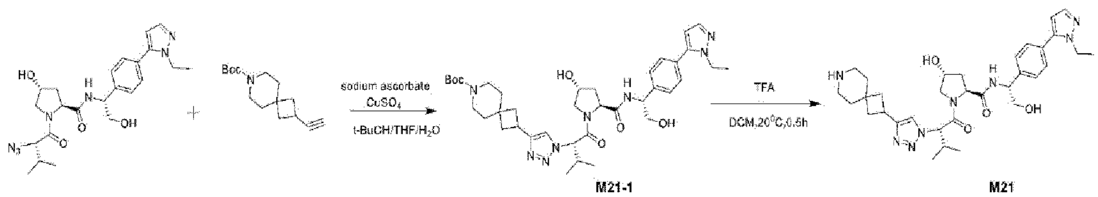


M21-1 M21

Step 1: Synthesis of compound M21-1

Compound (2S,4R)-1-((S)-2-azido-3-methylbutanoyl)-N-((R)-1-(4-(1-ethyl-1H-pyrazol -5-yl)phenyl)-2-hydroxyethyl)-4-hydroxypyrrolidine-2-carboxamide (0.08 g) and tert-butyl 2-ethynyl-7-azaspiro[3.5]nonane-7-carboxylate (0.044 g) were dissolved in tert-butanol (1 mL) and THF (1 mL). Copper sulfate (28 mg) and sodium ascorbate (105 mg) aqueous solution (1 mL) were added. The mixture was reacted at 20°C for 1 h. The reaction solution was added to 15 mL water, and 15 mL EA was added. The organic phase was separated, dried, filtered, and concentrated under reduced pressure. The concentrate was purified by prep-TLC (MeOH:DCM=1:20) to obtain 78 mg compound M21-1. ESI-MS m/z: 719.40[M+H]$^+$.

Step 2: Synthesis of compound M21

To compound M21-1 (78 mg) in DCM (2.0 mL), trifluoroacetic acid (0.4 mL) was added. The mixture was reacted at 20°C for 0.5 h. The reaction solution was added to 20 mL saturated sodium bicarbonate, and extracted with chloroform/isopropanol = 3:1. The organic phase was separated, dried, filtered, and concentrated to obtain 65 mg compound M21, which was used directly in the next step. ESI-MS m/z: 619.40 [M+H]$^+$.

Synthesis of intermediate M22:

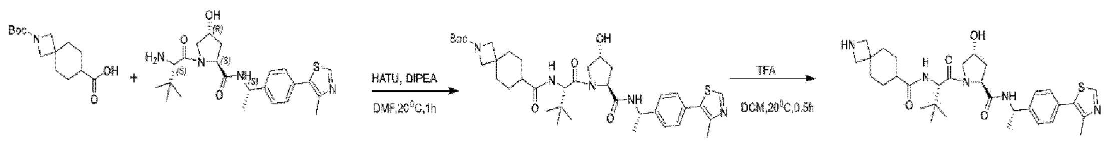


M22-1 M22

Step 1: Synthesis of compound M22-1

To compound 2-(tert-butoxycarbonyl)-2-azaspiro[3.5]nonane-7-carboxylic acid (0.1g) and (2S,4R)-1-((S)-2-amino-3,3-dimethylbutanoyl)-4-hydroxy-N-((S)-1-(4-(4-methylthiazol-5-yl)ph enyl)ethyl)pyrrolidine-2-carboxamide (0.178 g) in DMF (5 mL), HATU (169 mg) and DIPEA (145 mg) were added. The mixture was reacted at 20°C for 1 h. The reaction solution was added to 20 mL water, and 15 mL EA + 1 mL methanol were added. The organic phase was separated, dried, and filtered. The filtrate was concentrated under reduced pressure to obtain 0.22 g compound M22-1, which was used directly in the next step. ESI-MS m/z: 696.40[M+H]+.

Step 2: Synthesis of compound M22

To compound M22-1 (240 mg) in DCM (6.0 mL), trifluoroacetic acid (1.2 mL) was added. The mixture was reacted at 20°C for 0.5 h. The reaction solution was added to 20 mL saturated sodium bicarbonate, and extracted with chloroform/isopropanol = 3:1. The organic phase was separated, dried, filtered, and concentrated to obtain 200 mg compound M22, which was used directly in the next step. ESI-MS m/z: 596.40 [M+H]+.

Synthesis of intermediate M23:

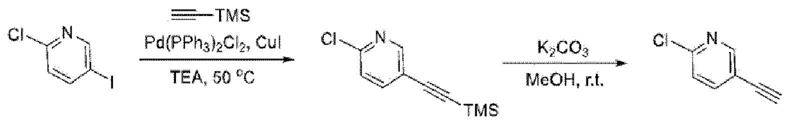


M23-1 M23

Step 1: Synthesis of compound M23-1

2-Chloro-5-iodopyridine (1 g) was added to a 50 mL three-necked flask. 10 mL TEA was added under $N_2$ protection, and trimethylsilylacetylene (1.03 g), cuprous iodide (80 mg), and $PdCl_2(PPh_3)_2$ (146 mg) were added. The mixture was heated to 50°C and reacted for 3 h. The reaction solution was filtered, and then the filtrate was collected and concentrated directly. The concentrate was separated and purified by column chromatography (PE : EA = 10 : 1) to obtain 0.85 g compound M23-1. ESI-MS m/z: 210 [M+H]+.

Step 2: Synthesis of compound M23

Compound M23-1 (500 mg) was added to a 50 mL single-necked flask, and dissolved in 5 mL MeOH. Anhydrous potassium carbonate (658 mg) was added. The mixture was stirred at room temperature for 2 h. The reaction solution was filtered, and then the filtrate was collected and concentrated directly. The concentrate was separated and purified by column chromatography (PE : EA = 20 : 1) to obtain 240 mg compound M23. ESI-MS m/z: 138 [M+H]+.

Synthesis of intermediate M24:

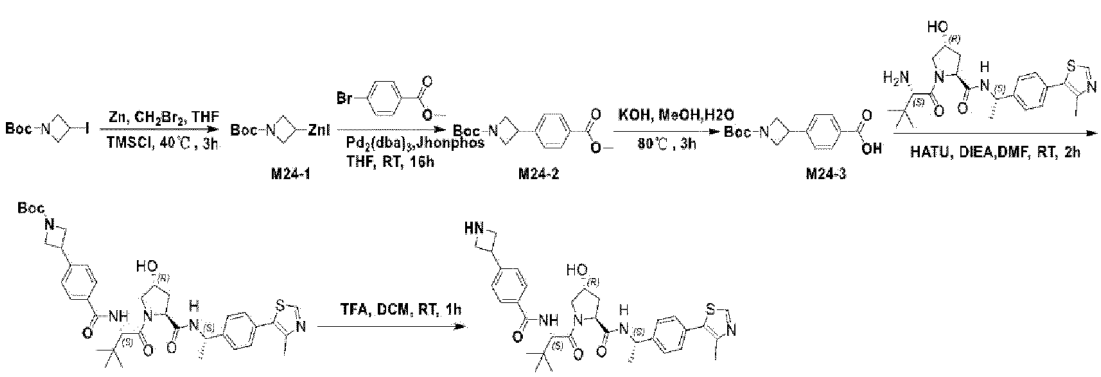


M24-1 M24-2 M24-3 M24-4 M24

Step 1: Synthesis of compound M24-1

Zinc powder (880 mg) and 80 mL anhydrous tetrahydrofuran were added to a 250 mL three-necked flask. Dibromomethane (200 mg) was added at room temperature under nitrogen protection. The mixture was heated to 65°C for 1 h. The resultant was cooled to room temperature, and TMSCl (115 mg) was added. The resultant was reacted at room temperature for 1 h. Tert-butyl 3-iodoazetidine-1-carboxylate (3.0 g) was added. The resultant was reacted at 40°C for 1 h to obtain M24-1, and it was sealed for later use.

Step 2: Synthesis of compound M24-2

Methyl 4-bromobenzoate (1.1 g), $Pd_2(dba)_3$ (235 mg), and Johnphos (153 mg) were added to the above M24-1 solution. The mixture was reacted at 40°C under nitrogen protection for 2 h. After the reaction was completed, ammonium chloride was added to quench the reaction. The resultant was extracted with EA, washed with brine, dried with sodium sulfate, and concentrated. The concentrate was purified by column chromatography (PE:EA = 10 : 1) to obtain compound M24-2 (660 mg).

Step 3: Synthesis of compound M24-3

Compound M24-2 (660 mg) was added to a 50 mL single-necked flask, and 10 mL MeOH and 2 mL water were added. Sodium hydroxide (410 mg) was added at room temperature. The mixture was heated to 60°C and reacted for 2 h. After the reaction was completed, water was added to the reaction solution, and pH was adjusted to 6. The resultant was extracted with EA twice, washed with brine, dried with anhydrous sodium sulfate, and concentrated to obtain compound M24-3 (550 mg).

Step 4: Synthesis of compound M24-4

Compound M24-3 (150 mg) was added to a 50 mL single-necked flask, and 3 mL DMF and HATU (250 mg) were added. DIPEA (350 mg) was added at 0°C under nitrogen protection. The mixture was reacted for 0.5 h. (2S,4R)-1-((S)-2-amino-3,3-dimethylbutanoyl)-4-hydroxy -N-((S)-1-(4-(4-methylthiazol-5-yl)phenyl)ethyl)pyrrolidine-2-carboxamide (290 mg) was added. The mixture was heated to room temperature and reacted for 1 h. After the reaction was completed, ammonium chloride was added to quench the reaction. The resultant was extracted with EA, washed with brine, dried with sodium sulfate, and concentrated. The concentrate was purified by column chromatography (DCM : methanol = 10 : 1) to obtain M24-4 (340 mg). ESI-MS m/z: 704.3 $[M+H]^+$.

Step 5: Synthesis of compound M24

Compound M24-4 (340 mg) was added to a 50 mL single-necked flask, and 5 mL DCM and 1 mL TFA were added. The mixture was reacted at room temperature for 1 h. After the reaction was completed, the resultant was concentrated. DCM was added for dilution. The resultant was poured into sodium bicarbonate solution, and then extracted with DCM:MeOH (10:1) three times. The organic phase was washed with brine, dried with anhydrous sodium sulfate, and concentrated to obtain M24 (250 mg). ESI-MS m/z: 604.3 $[M+H]^+$.

Synthesis of intermediate M25:

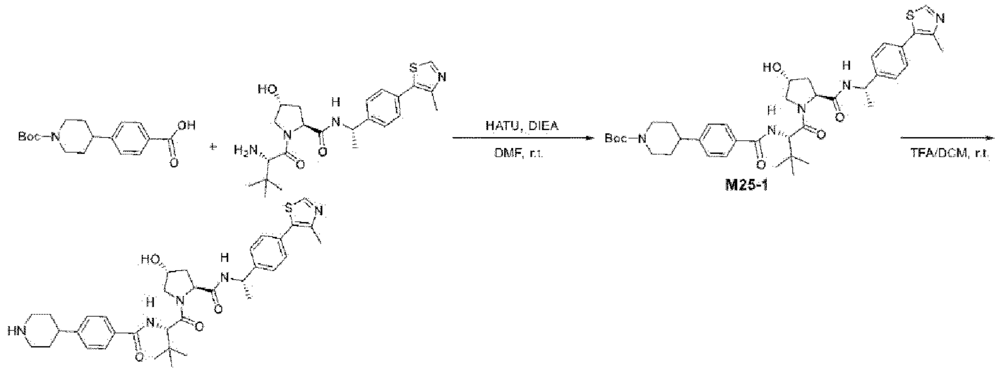


M25

Step 1: Synthesis of compound M25-1

4-(1-(Tert-butoxycarbonyl)piperidin-4-yl)benzoic acid (100.0 mg), tert-butyl 4-(1-(((*S*)-1-((2*S*,4*R*)-4-hydroxy-2-((((*S*)-1-(4-(4-methylthiazol-5-yl)phenyl)ethyl)carbamoyl)pyr rolidin-1-yl)-3,3-dimethyl-1-oxobutan-2-yl)-1*H*-1,2,3-triazol-4-yl)piperidine-1-carboxylate (174.0 mg), and HATU (149.4 mg) were dissolved in DMF (2 mL). DIPEA (0.27 mL) was added. The mixture was reacted at room temperature for 10 minutes. The reaction was monitored by LCMS until it was completed. DCM (10 mL) was added to the reaction solution. The mixture was washed once with saturated brine. The organic phase was dried with anhydrous sodium sulfate, filtered, concentrated, and separated and purified by column chromatography to obtain 231.0 mg compound M25-1. ESI-MS m/z: 732 $[M+H]^+$.

Step 2: Synthesis of compound M25

M25-1 (231.0 mg) was added to DCM (5 mL) and TFA (2 mL). The mixture was reacted at room temperature for 10 minutes. The reaction was monitored by LCMS until it was completed. The reaction solution was concentrated directly. 1 M NaOH (2 mL) was added, and the resultant was stirred at room temperature for 5 minutes and filtered under reduced pressure to obtain 181.0 mg pale yellow solid, i.e. compound M25. ESI-MS m/z: 632 $[M+H]^+$.

Synthesis of intermediate M26:

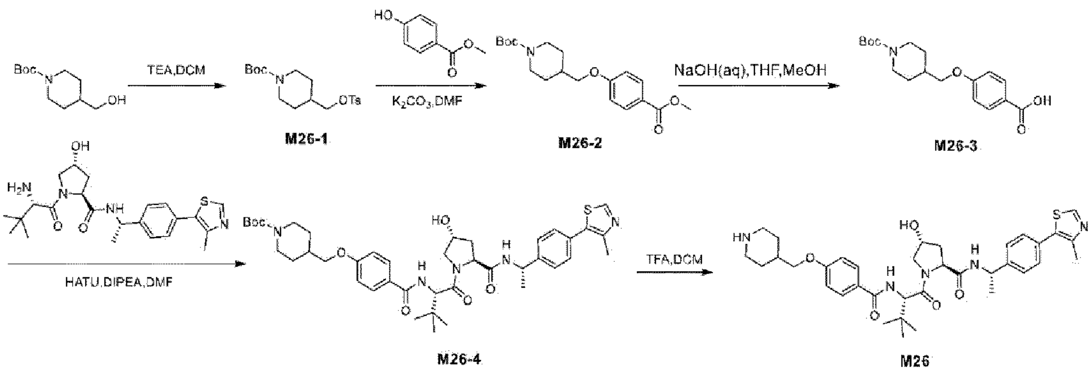


Step 1: Synthesis of intermediate M26-1

Tert-butyl 4-(hydroxymethyl)piperidine-1-carboxylate (5.0 g) was dissolved in DCM (50 mL) at room temperature. Triethylamine (9.68 mL) and p-toluenesulfonyl chloride (5.31 g) were successively added. The mixture was reacted at room temperature for 15 h. An appropriate amount of water was added to the reaction solution to quench the reaction. Then an appropriate amount of ethyl acetate was added for extraction. The organic phase was collected, dried with anhydrous sodium sulfate, filtered, and then concentrated under reduced pressure to obtain 8.02 g product, i.e. compound M26-1, which was used directly in the next step.

Step 2: Synthesis of intermediate M26-2

Intermediate M26-1 (7.0 g) and methyl 4-hydroxybenzoate (2.6 g) were dissolved in DMF (30.0 mL). Potassium carbonate (4.72 g) was added. The mixture was reacted at 60°C for 10 h. An appropriate amount of water was added to the reaction solution to quench the reaction. Then an appropriate amount of ethyl acetate was added for extraction. The organic phase was collected, dried with anhydrous sodium sulfate, filtered, and then concentrated under reduced pressure. The concentrate was purified by column chromatography to obtain 4.02 g product, i.e. compound M26-2.
ESI-MS m/z: 350 $[M+H]^+$.

Step 3: Synthesis of intermediate M26-3

Compound M26-2 (4.0 g) was dissolved in a mixed solution of THF (40.00 mL) and MeOH (40.00 mL). Sodium hydroxide (2.29 g) aqueous solution (20.0 mL) was added. The mixture was reacted at 40°C for 1 h. After the reaction solution was cooled to room temperature, 1 M sulfuric acid was added to adjust the mixture to acidic. An appropriate amount of DCM/MeOH was added for extraction. The organic phase was collected, dried with anhydrous sodium sulfate, filtered, and then concentrated under reduced pressure to obtain 3.50 g product, i.e. intermediate M26-3.
ESI-MS m/z: 336 $[M+H]^+$.

Step 4: Synthesis of intermediate M26-4

Intermediate M26-3, (2S,4R)-1-((S)-2-amino-3,3-dimethylbutanoyl)-4-hydroxy-N-((S)-1-(4-(4-methylthiazol-5-yl)ph enyl)ethyl)pyrrolidine-2-carboxamide (1.59 g) were dissolved in 15.00 mL DMF. Then, N,N-diisopropylethylamine (1.56 mL) and N,N,N',N'-tetramethyl-O-(7-azabenzotriazol-1-yl)uronium hexafluorophosphate (1.36 g) were added successively. The mixture was reacted at room temperature for 30 minutes. The reaction was monitored by LCMS until it was completed. 100.0 mL water was added to the reaction solution. The precipitated solid from the system was filtered, then separated and purified by column chromatography to obtain 1.95 g product, i.e. intermediate M26-4.
ESI-MS m/z: 762$[M+H]^+$.

Step 5: Synthesis of intermediate M26

Intermediate M26-4 (1.50 g) was dissolved in 20.00 mL DCM, and then TFA (5.00 mL) was added. The mixture was reacted at room temperature for 0.5 h. The reaction solution was added dropwise to saturated sodium bicarbonate solution. An appropriate amount of DCM/MeOH=10/1 was added for extraction. The organic phase was collected, dried with anhydrous sodium sulfate, filtered, and evaporated under reduced pressure to obtain 1.20 g intermediate M26.
ESI-MS m/z: 662 $[M+H]^+$.

Synthesis of intermediate M27:

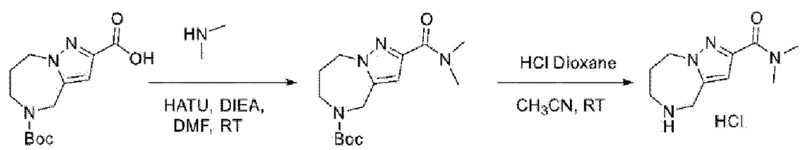


M11-6 M27-1 M27

Step 1: Synthesis of compound M27-1

To a solution of compound M11-6 (600 mg) in DMF (15 mL), dimethylamine (191 mg), DIPEA (1.76 mL), and HATU (973 mg) were added successively. The mixture was stirred and reacted at room temperature for 1 h. After the reaction was completed, EA and water were added to the reaction solution for extraction. The organic phase was collected. The aqueous phase was extracted with EA twice. The organic phases were combined and washed three times with saturated brine, dried with anhydrous sodium sulfate, and concentrated. The concentrate was purified by column chromatography (DCM : EA = 3 : 1) to obtain 580 mg compound M27-1.
ESI-MS m/z: 309.3 $[M+H]^+$.

Step 2: Synthesis of compound M27

M27-1 (580 mg) was dissolved in HCl/dioxane (4 N, 5 mL). The mixture was stirred and reacted at room temperature for 0.5 h. After the reaction was completed, the reaction solution was directly concentrated to obtain intermediate M27 (390 mg). ESI-MS m/z: 209.3 $[M+H]^+$.

Synthesis of intermediate M28: Synthesis of (R)-1-(((7-(8-ethyl-7-fluoro-3-(methoxymethoxy)naphthalen-1-yl)-8-fluoro-4-(3-hydroxy-3-met hylpiperidin-1-yl)pyrido[4,3-d]pyrimidin-2-yl)oxy)methyl)cyclopropane-1-carbaldehyde

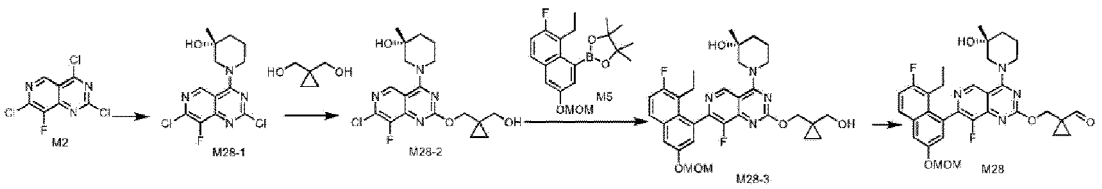

Step 1: Synthesis of compound M28-1

Intermediate M2 (5.0 g) was dissolved in DCM (20 mL) and cooled to about 40°C below zero. DIPEA (7.7 g) was added. The mixture was stirred at low temperature for 30 min. (3R)-3-methylpiperidin-3-ol hydrochloride (4.2 g) was added slowly. Stirring was continued, and the mixture was reacted for 1.0 h. After the reaction was completed, the mixture was extracted with DCM and $H_2O$, and washed with saturated brine. The organic phase was dried with anhydrous sodium sulfate, filtered, dried by rotary evaporation, and purified by column chromatography (EA:DCM 10%) to obtain compound M28-1 (5.3 g).

ESI-MS m/z: 331.3 $[M+H]^+$.

Step 2: Synthesis of compound M28-2

Compound M28-1 (5.3 g), cesium carbonate (10.4 g), DABCO (0.4 g), and 1,1-cyclopropane dimethanol (3.3 g) were dissolved in DMF (20 mL). The mixture was stirred and reacted at room temperature overnight. After the reaction was completed, the mixture was extracted with EA and $H_2O$, and washed with saturated brine. The organic phase was dried with anhydrous sodium sulfate, filtered, dried by rotary evaporation, and purified by column chromatography (EA:DCM 18%) to obtain compound M28-2 (5.0 g).

ESI-MS m/z: 397.1 $[M+H]^+$

Step 3: Synthesis of compound M28-3

Compound M28-2 (5.0 g), M5 (9.0 g), potassium carbonate (3.5 g), and CataCxium A Pd G3 (1.3 g) were dissolved in 1,4-dioxane (24 mL) and $H_2O$ (4 mL). The mixture was reacted at 90°C under nitrogen protection for 1 h. After the reaction was completed, the reaction solution was diluted with EA and $H_2O$, and further extracted with EA once. The organic phase was washed with saturated brine once, dried with anhydrous sodium sulfate, and concentrated. The concentrate was purified by column chromatography (dichloromethane : ammonia in methanol) to obtain M28-3 (6.0 g, 85% purity), which was used directly in the next step.

ESI-MS m/z:595.2 $[M+H]^+$.

Step 4: Synthesis of compound M28

To a solution of compound M28-3 (6.0 g) in DCM (50 mL), DMP (6.4 g) was added in batches. The mixture was stirred at room temperature for 3.0 h. After the reaction was completed, saturated sodium sulfite solution was added to quench the reaction. The resultant was extracted with DCM and $H_2O$. The aqueous phase was further extracted with DCM twice. The organic phases were combined, dried with anhydrous sodium sulfate, filtered, dried by rotary evaporation, and purified by column chromatography (PE:EA 50%) to obtain compound M28 (3.4 g).

ESI-MS m/z: 593.4 $[M+H]^+$.

Synthesis of intermediate M29: Synthesis of compound (2S,4R)-1-((S)-2-azido-3-methylbutanoyl)-4-hydroxy-N-((S)-1-(4-(4-methylthiazol-5-yl)phenyl) ethyl)pyrrolidine-2-carboxamide

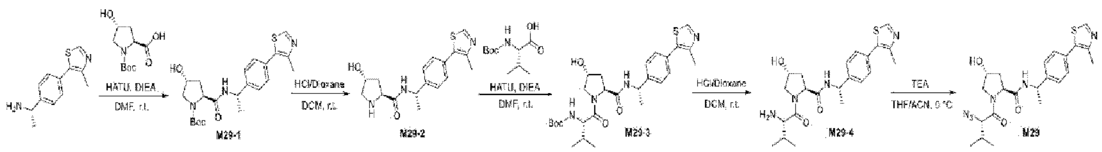


Step 1: Synthesis of compound M29-1

(S)-1-(4-(4-methylthiazol-5-yl)phenyl)ethanamine (12.7 g), Boc-L-hydroxyproline (13.5 g), and HATU (23.2 g) were dissolved in DMF (100 mL). DIPEA (38.5 mL) was added. The mixture was reacted at room temperature for 10 minutes. The reaction was monitored by LCMS until it was completed. EA (100 mL) and saturated brine (100 mL) were added to the reaction solution. The organic phase was collected, dried with anhydrous sodium sulfate, filtered, concentrated, and separated and purified by column chromatography to obtain 22.0 g white solid, i.e. compound M29-1.
ESI-MS m/z: 432 $[M+H]^+$.

Step 2: Synthesis of compound M29-2

M29-1 (22.0 g) was added to DCM (100 mL). A dioxane solution of hydrogen chloride (100 mL) was added in an ice-water bath. The mixture was reacted at room temperature for 60 minutes. The reaction was monitored by LCMS until it was completed. The reaction solution was concentrated directly. 1 M NaOH solution was added to adjust the reaction solution to pH = 8. DCM (100 mL) was added to the reaction solution. The organic phase was collected, washed with saturated brine once, dried with anhydrous sodium sulfate, filtered, and concentrated to obtain 16.8 g white solid, i.e. compound M29-2.
ESI-MS m/z: 332 $[M+H]^+$.

Step 3: Synthesis of compound M29-3

M29-2 (19.0 g), Boc-L-valine (12.5 g), and HATU (22.9 g) were dissolved in DMF (100 mL). DIPEA (28.4 mL) was added. The mixture was reacted at room temperature for 10 minutes. The reaction was monitored by LCMS until it was completed. EA (100 mL) and saturated brine (100 mL) were added to the reaction solution. The organic phase was collected, dried with anhydrous sodium sulfate, filtered, concentrated, and separated and purified by column chromatography to obtain 25.0 g white solid, i.e. compound M29-3.
ESI-MS m/z: 531 $[M+H]^+$.

Step 4: Synthesis of compound M29-4

M29-3 (25.0 g) was added to DCM (100 mL). A dioxane solution of hydrogen chloride (100 mL) was added in an ice-water bath. The mixture was reacted at room temperature for 60 minutes. The reaction was monitored by LCMS until it was completed. The reaction mixture was concentrated directly. 1 M NaOH solution was added to adjust the reaction mixture to pH = 8. DCM (100 mL) was added to the reaction solution. The organic phase was collected, washed with saturated brine once, dried with anhydrous sodium sulfate, filtered, and concentrated to obtain 17.0 g white solid, i.e. compound M29-4.
ESI-MS m/z: 431 $[M+H]^+$.

Step 5: Synthesis of compound M29

M29-4 (17.0 g) and TEA (32.9 mL) were dissolved in ACN (100 mL) and THF (130 mL). 2-Azido-1,3-dimethylimidazolinium hexafluorophosphate (22.5 g) was dissolved in ACN (30 mL) in an ice-water bath, which was slowly added to the system. The mixture was reacted at maintained temperature for 3 hours. The reaction was monitored by LCMS until it was completed. The reaction solution was concentrated directly. DCM (100 mL) was added to the reaction solution. The organic phase was collected, washed with saturated brine once, dried with anhydrous sodium sulfate, filtered, concentrated, and separated and purified by column chromatography to obtain 15.3 g pale yellow powder, i.e. compound M29.
ESI-MS m/z: 457 $[M+H]^+$.

$^1$H NMR (500 MHz, MeOD) δ 8.87 (s, 1H), 7.46-7.40 (m, 4H), 7.94 (q, $J$ = 7.0 Hz, 1H), 4.64-4.60 (m, 1H), 4.45-4.44 (m, 1H), 3.75 (d, $J$ = 8.0 Hz, 1H), 3.71-3.64 (m, 2H), 2.48 (s, 3H), 2.26-2.15 (m, 2H), 1.96-1.91 (m, 1H), 1.52 (d, $J$ = 7.0 Hz, 3H), 1.09-0.98 (m, 6H).

Synthesis of intermediate M30:

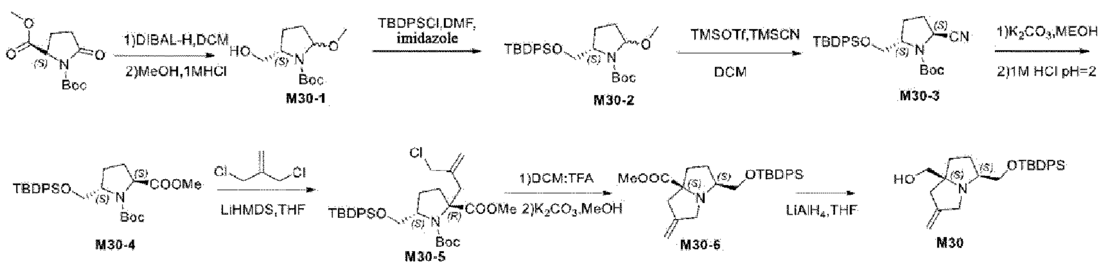


Step 1: Synthesis of compound M30-1

At -78°C under nitrogen atmosphere, to a stirred solution of 1-(tert-butyl) 2-methyl (S) -5-oxopyrrolidine-1,2-dicarboxylate (20 g) in DCM (300 mL), a solution of 1.0 M DIBAL-H in n-hexane (329 mL) was added dropwise. The mixture was stirred at -78°C for 30 min, then transferred to room temperature and stirred for 2 hours. After the raw materials were confirmed by TLC for being reacted completely, the mixture was cooled to 0°C. MeOH (300 mL) and 1 M HCl (200 mL) were added. The mixture was stirred at room temperature for 2 hours. The reaction endpoint was confirmed by TLC. After the reaction was completed, the resultant was extracted with EtOAc. The organic layers were combined, washed with brine, dried with anhydrous $Na_2SO_4$, concentrated, and purified by column chromatography to obtain a pale yellow liquid (13.9 g), i.e. compound M30-1.

Step 2: Synthesis of compound M30-2

M30-1 (13.9 g) was dissolved in 140 mL DMF. Imidazole (4.5g) was added at 0°C, followed by dropwise addition of TBDPSCl (17.2 mL). After the addition was completed, the mixture was transferred to room temperature and stirred for 3 h. The reaction endpoint was monitored by TLC. After the reaction was completed, 100.0 mL water was added to the reaction solution. The resultant was extracted with EA three times, 200 mL each time. The organic phases were combined, washed with saturated brine, dried with sodium sulfate, filtered, concentrated, and purified by column chromatography to obtain a pale yellow liquid (21.09 g), i.e. compound M30-2.

Step 3: Synthesis of compound M30-3

M30-2 (21.09g) was dissolved in 140 mL DCM. The reaction solution was cooled to -35°C. TMSCN (7.48 mL) was added, and the mixture was stirred at -35°C for 15 min. TMSOTf (0.14 mL) was added dropwise, and the mixture was stirred at -35°C for 10 min. After the reaction was monitored to be completed by TLC, saturated sodium bicarbonate solution : water = 15 mL : 90 mL was added. The mixture was stirred for 15 min and extracted with EA three times, 200 mL each time. The organic phases were combined, washed with saturated brine, dried with sodium sulfate, filtered, concentrated, and separated and purified by column chromatography to obtain a yellow liquid 16.2 g, i.e. compound M30-3.

Step 4: Synthesis of compound M30-4

A mixture of M30-3 (16.2 g) and $K_2CO_3$ (7.55 g) in MeOH (120 mL) was stirred at room temperature for 3 h. The mixture was acidified to pH = 2 with 1 M HCl aqueous solution. The obtained mixture was stirred at room temperature for 3h. The reaction was confirmed by TLC to be completed. The mixture was extracted with EtOAc. The organic layers were combined, washed with brine, dried with anhydrous $Na_2SO_4$, concentrated, and purified by column chromatography to obtain a yellow liquid (8.5 g), i.e. compound M30-4.

Step 5: Synthesis of compound M30-5

M30-4 (8.5 g) was dissolved in THF (85 mL). A tetrahydrofuran solution of LiHMDS (34.16 mL) was added dropwise at -78°C under nitrogen atmosphere. The obtained mixture was stirred at -78°C for 10 min. Then 3-chloro-2-(chloromethyl)propene (3.95 mL) was added dropwise under nitrogen atmosphere. The obtained mixture was stirred at room temperature under nitrogen atmosphere for 16 hours. The reaction was confirmed by LC-MS to be completed. The mixture was quenched with water at 0°C and then extracted with EA. The organic layers were combined, washed with brine, dried with anhydrous sodium sulfate, concentrated, and purified by column chromatography to obtain a yellow liquid (5 g), i.e. compound M30-5.

Step 6: Synthesis of compound M30-6

To a solution of M30-5 (5 g) in DCM (40 mL), TFA (20 mL) was added dropwise. The obtained mixture was stirred for 1 h. The reaction solution was concentrated under reduced pressure, which was repeated with DCM. The residue was dissolved in MeOH (40 mL), and $K_2CO_3$ (10 g) was added. The mixture was stirred at room temperature for 40 minutes. The reaction endpoint was confirmed by LC-MS. The reaction was quenched with water at room temperature. The mixture was extracted with EA. The organic layers were combined, washed with brine, dried with anhydrous $Na_2SO_4$, concentrated, and purified by column chromatography to obtain a yellow liquid (3.04 g), i.e. compound M30-6.

Step 7: Synthesis of compound M30

At -20°C under nitrogen atmosphere, to a solution of M30-6 (3.04 g) in THF (30 mL), a solution of $LiAlH_4$ in THF (1M, 6.8 mL) was added dropwise. The obtained mixture was stirred at 0°C for 20 min. The reaction was confirmed by LC-MS to be completed. Then sodium sulfate decahydrate was added at 0°C, and the mixture was stirred for 30 min. The resultant was filtered, washed with EA, concentrated and purified by neutral alumina column chromatography to obtain a yellow liquid (2 g), i.e. compound M30.

Intermediate M31: Synthesis of compound (R)-1-(7-(8-ethyl-7-fluoro-3-methoxymethoxy)naphthalen-1-yl)-8-fluoro-2-((5S,7aS)-5-hydroxy methyl-2-methylenetetrahydro-1H-pyrrolizine-7a(5H)-methoxy)pyrido[4,3-d]pyrimidin-4-yl)-3-methylpiperidin-3-ol

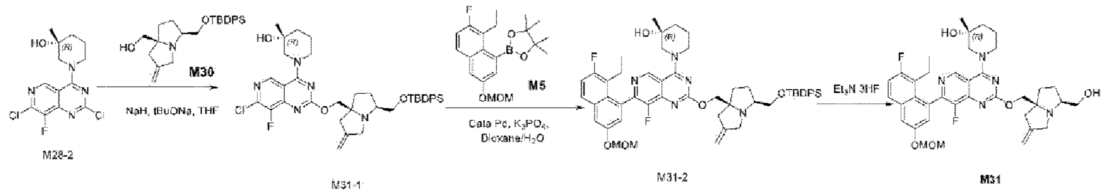


Step 1: Synthesis of compound M31-1

M28-2 (2.90 g) was dissolved in tetrahydrofuran (30.0 mL) in a 100 mL single-necked flask. Sodium hydride (1.40 g) was added. After stirring for 10 min, M30 (4.43 g) was added. The mixture was reacted at room temperature for 0.5 h. Saturated aqueous ammonium chloride solution was added to the reaction solution to quench the reaction. The mixture was extracted with dichloromethane three times. The organic phase was collected, and dried with anhydrous sodium sulfate, and then the solvent was removed by evaporating. The resultant was purified by column chromatography to obtain 5.30 g yellow solid, i.e. compound M31-1.

Step 2: Synthesis of compound M31-2

Compound M31-1 (5.0 g), M5 (2.62 g), CataCXium A Pd G3 (0.40 g), and potassium phosphate (3.56 g) were added successively to a 100 mL single-necked flask, and dissolved in 1,4-dioxane (50.0 mL) and water (5.0 mL). Nitrogen displacement was performed twice or three times. The mixture was reacted at 100°C for 3 h. An appropriate amount of water was added to the reaction solution. The resultant was extracted with ethyl acetate three times. The organic phase was collected, and dried with anhydrous sodium sulfate, and then the solvent was removed by evaporating. The resultant was purified by column chromatography to obtain 4.5 g yellow solid, i.e. compound M31-2.

Step 3: Synthesis of intermediate M31

Compound M31-2 (4.50 g) was added to a 100 mL single-necked flask in an ice bath, and dissolved in dichloromethane (50.0 mL). Hydrogen fluoride triethylamine (10.0 mL) was added. The mixture was reacted under the ice bath for 0.5 h. 5.0 mL methanol was added to the reaction solution. The resultant was extracted with 50.0 mL water three times, and washed with saturated brine. After that, the organic phase was collected, dried by rotary evaporation, and separated by column chromatography to obtain 2.3 g of yellow solid, i.e. intermediate M31.

Intermediate M32: Synthesis of compound ((3S,7aS)-7a-((7-(8-ethyl-7-fluoro-3-methoxymethoxy)naphthalen-1-yl)-8-fluoro-4-(R)-3-hydrox y-3-methylpiperidin-1-yl)pyrido[4,3-d]pyrimidin-2-yl)oxy)methyl)-6-methylenehexahydro-1H-p yrrolizin-3-yl)methyl(4-nitrophenyl)carbonate

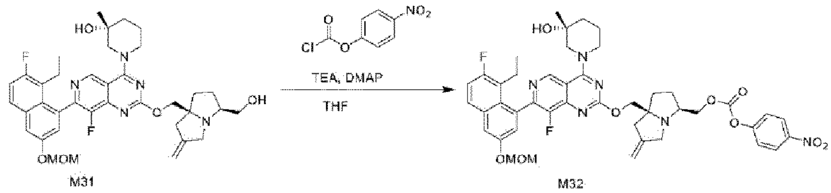


Step 1: Synthesis of compound M32

Compound M31 (100.0 mg), triethylamine (30.0 mg), DMAP (5.0 mg), and p-nitrophenyl chloroformate were added to a 10 mL single-necked flask, and tetrahydrofuran (4.00 mL) was added. The mixture was stirred at 20°C for 18 hours. The raw materials were confirmed by LCMS for being reacted completely. The reaction solution M32 was used directly in the next reaction.

Intermediate M33: Synthesis of compound (R)-2-((7-(8-ethyl-7-fluoro-3-hydroxynaphthalen-1-yl)-8-fluoro-4-(3-hydroxy-3-methylpiperidin -1-yl)pyrido[4,3-d]pyrimidin-2-yl)oxy)acetaldehyde

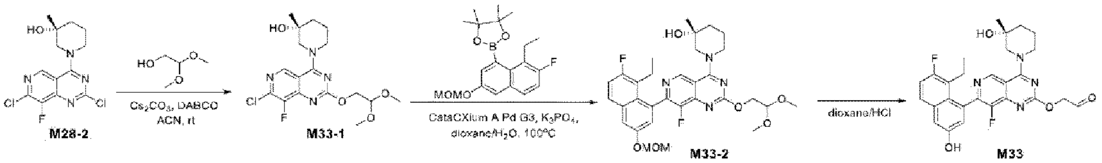


Step 1: Synthesis of compound M33-1

Compound M28-2 (2.50 g), acetonitrile (40.00 mL), 2,2-dimethoxyethanol (1.20 g), cesium carbonate (3.20 g), and triethylenediamine (0.08 g) were added to a reaction flask. The mixture was reacted at room temperature for 2 h. The solvent was concentrated at first. Water was added for dilution. The mixture was extracted with EA, dried, and concentrated. The resultant was purified by column chromatography (DCM:EA=3:1) to obtain yellow oil M33-1 (2.3 g). ESI-MS m/z: 401.28 $[M+H]^+$.

Step 2: Synthesis of compound M33-2

2-[8-ethyl-7-fluoro-3-methoxymethoxynaphthalen-1-yl]-4,4,5,5-tetramethyl-1,3,2-dioxabor olane (3.24 g), compound M33-1 (2.25 g), mesylate[(di(1-adamantyl)-n-butylphosphine)-2-(2'-amino-1,1'-biphenyl)]palladium(II) (0.61 g), potassium phosphate (2.98 g), and 1,4-dioxane (36.00 mL) were added to a reaction flask. After nitrogen displacement, the mixture was reacted at 100°C under nitrogen protection for 3 h. Water was added for dilution. The resultant was extracted with EA, dried, and concentrated. The resultant was seperated by column chromatography (DCM:EA=3:1) to obtain yellow oil M33-2 (3.3 g). ESI-MS m/z: 599.45 $[M+H]^+$.

Step 3: Synthesis of compound M33

Compound M33-2 (2.50 g), 1,4-dioxane (15.00 mL), and concentrated hydrochloric acid (9.00 mL) were added to a reaction flask. The mixture was reacted at room temperature for 1 h. Sodium bicarbonate aqueous solution was added in an ice bath to adjust the pH to 8. The mixture was extracted with EA and THF, dried, and concentrated to obtain yellow solid M33 (2.15 g). ESI-MS m/z: 509.19 [M+H]$^+$.

Synthesis of intermediate M34

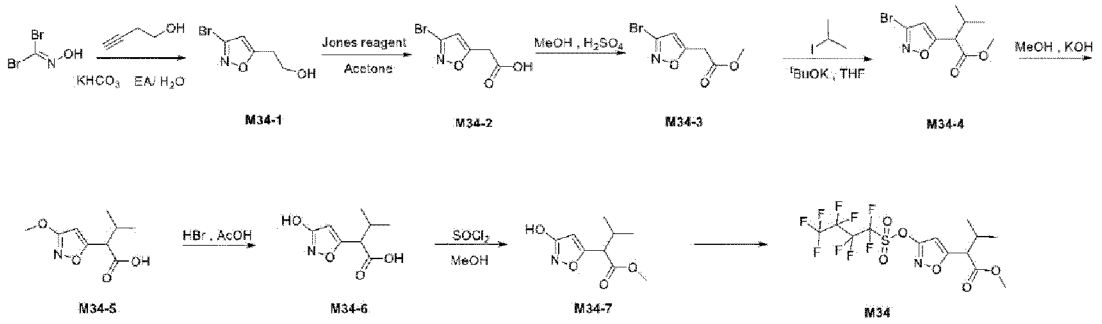


Step 1: Synthesis of compound M34-1

3-Butyn-1-ol (140 g) and potassium bicarbonate (150 g) were added with stirring to a 3 L single-necked flask containing ethyl acetate/water (1000/100 mL). After that, raw material 1,1-dibromoformaldoxime (100 g) was dissolved in ethyl acetate (250 mL), which was added dropwise to the 3 L single-necked flask. The mixture was stirred at room temperature for 16 h. After the reaction was completed, water was added to the reaction solution for dilution. The organic phase was separated and collected. The aqueous phase was continued to be re-extracted with ethyl acetate once. The organic phases were collected and combined, washed with brine once, dried with anhydrous sodium sulfate and concentrated. The concentrate was purified by column chromatography (PE : EA = 5: 1) to obtain compound M34-1 (93 g, 97% yield).

ESI-MS m/z: 191.9 [M+H]$^+$.

Step 2: Synthesis of compound M34-2

Compound M34-1 (93 g) was added to a 3 L single-necked flask, and acetone (1100 mL) was added. Jones reagent (450 mL) was further added dropwise to the reaction solution at zero degree Celsius. The mixture was heated to room temperature, and stirred for 12 h. After the reaction was completed, water was added to the reaction solution for dilution. Ethyl acetate was added for extraction. The organic phase was collected. The aqueous phase was continued to be re-extracted with ethyl acetate once. The organic phases were combined, washed with brine once, dried with anhydrous sodium sulfate, and concentrated to obtain compound M34-2 (60 g, 60% yield) as a yellow oil. ESI-MS m/z: 205.9 [M+H]$^+$.

Step 3: Synthesis of compound M34-3

Compound M34-2 (60 g) was added to a 1 L single-necked flask, and methanol (500 mL) was added. 5 mL concentrated sulfuric acid was added dropwise slowly in an ice bath. The mixture was heated to 70°C, and reacted for 2 h. After the reaction was completed, the resultant was concentrated under reduced pressure. Water was added for dilution. Ethyl acetate was then added for extraction. The aqueous phase was continued to be re-extracted with ethyl acetate twice. The organic phases were collected and combined, washed with brine once, dried with anhydrous sodium sulfate and concentrated. The concentrate was purified by column chromatography (PE : EA = 10: 1) to obtain compound M34-3 (49 g, 76% yield). ESI-MS m/z: 219.9 [M+H]$^+$.

Step 4: Synthesis of compound M34-4

Compound M34-3 (49 g) was added to a 1 L single-necked flask. Tetrahydrofuran (500 mL) was added, followed by addition of potassium tert-butoxide (45 g). 2-Iodopropane (49 g) was added dropwise in an ice bath. The mixture was

heated to room temperature, and reacted for 16 h. After the reaction was completed, an ice-water mixture was added to quench the reaction. Ethyl acetate was then added for extraction. The aqueous phase was continued to be re-extracted with ethyl acetate twice. The organic phases were collected and combined, washed with brine once, dried with anhydrous sodium sulfate and concentrated. The concentrate was purified by column chromatography (PE : EA = 100: 1) to obtain compound M34-4 (32 g, 55% yield). ESI-MS m/z: 262.0 $[M+H]^+$.

Step 5: Synthesis of compound M34-5

Compound M34-4 (32 g) was added to a 1 L single-necked flask. Methanol (250 mL) and potassium hydroxide (73 g) were added, followed by addition of potassium tert-butoxide (45 g). The mixture was stirred under reflux for 4 h. After the reaction was completed, the resultant was cooled to room temperature, and adjusted to pH = 5 with 1 N HCl solution. The reaction solution was concentrated. The concentrate was purified by reverse-phase column chromatography (5% to 40% ACN/water (0.1% trifluoroacetic acid)) to obtain compound M34-5 (21 g, 86% yield). ESI-MS m/z: 200.1 [M+H]+.

Step 6: Synthesis of compound M34-6

Compound M34-5 (21 g) was added to a 500 mL single-necked flask. Acetic acid (150 mL) and 33% hydrobromic acid aqueous solution (150 mL) were added. The mixture was heated to 60°C, and stirred for 10 h. After the reaction was completed, the reaction solution was cooled to room temperature and concentrated under reduced pressure. The concentrate was dissolved in DCM, then dried with anhydrous sodium sulfate, and concentrated to obtain compound M34-6 (15 g, 77% yield). ESI-MS m/z: 186.0 [M+H]+.

Step 7: Synthesis of compound M34-7

Compound M34-6 (15 g) was added to a 500 mL single-necked flask, and methanol (150 mL) was added. Thionyl chloride (75 mL) was added dropwise in an ice bath. The mixture was stirred at room temperature for 1.5 h. After the reaction was completed, the reaction solution was concentrated under reduced pressure. After that, water was added for dilution. Ethyl acetate was added for extraction. The organic phase was collected. The aqueous phase was continued to be re-extracted with ethyl acetate once. The organic phases were collected and combined, washed with brine once, dried with anhydrous sodium sulfate and concentrated. The concentrate was purified by column chromatography (DCM : MeOH = 100: 1) to obtain compound M34-7 (11 g, 69% yield). ESI-MS m/z: 200.1 $[M+H]^+$.

Step 8: Synthesis of compound M34

Compound M34-7 (7 g) was added to a 500 mL single-necked flask, and acetonitrile (75 mL) was added. Potassium carbonate (10g) was added in batches. Then, nonafluorobutanesulfonyl fluoride (14 g) was further added slowly dropwise. The mixture was stirred at room temperature for 2 h. After the reaction was completed, Water was added for dilution. Ethyl acetate was further added for extraction. The organic phase was collected. The aqueous phase was continued to be re-extracted with ethyl acetate once. The organic phases were collected and combined, washed with brine once, dried with anhydrous sodium sulfate and concentrated. The concentrate was purified by column chromatography (PE : THF = 100: 1) to obtain compound M34 (14.5 g, 80% yield).

ESI-MS m/z: 482.0 $[M+H]^+$. $^1$H NMR (500 MHz, DMSO-$d_6$) $\delta$ 7.02 (s, 1H), 4.01 (d, J = 7.9 Hz, 1H), 3.69 (s, 3H), 2.37 (dq, J = 13.6, 6.8 Hz, 1H), 0.93 (d, J = 6.7 Hz, 3H), 0.85 (d, J = 6.7 Hz, 3H).

Synthesis of intermediate M35

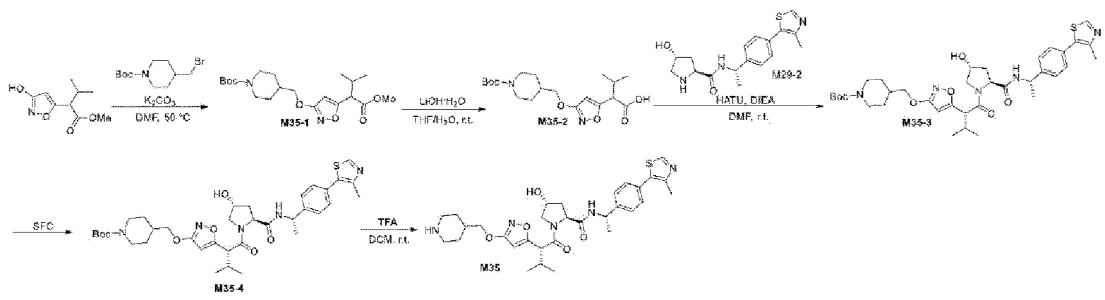

Step 1: Synthesis of compound M35-1

Methyl 2-(3-hydroxyisoxazol-5-yl)-3-methylbutanoate (450.0 mg), tert-butyl 4-(bromomethyl)piperidine-1-carboxylate (1.57 g), and potassium carbonate (936.7 mg) were dissolved in DMF (10 mL). The mixture was reacted at 50°C for 5 hours. The reaction was monitored by LCMS until it was completed. DCM (10 mL) was added to the reaction solution. The mixture was washed once with saturated brine. The organic phase was dried with anhydrous sodium sulfate, filtered, concentrated, and separated and purified by column chromatography to obtain 237.1 mg white solid, i.e. compound 35-1. ESI-MS m/z: 341.5 $[M+H]^+$.

Step 2: Synthesis of compound M35-2

M35-1 (237.1 mg) was added to methanol (4 mL) and water (1 mL). Lithium hydroxide monohydrate (125.4 mg) was added. The mixture was reacted at room temperature for 1 hour. The reaction was monitored by LCMS until it was completed. The pH of the reaction solution was adjusted to acidic, to which EA (20 mL) was added. The organic phase was collected, washed with saturated brine once, dried with anhydrous sodium sulfate, filtered, and concentrated to obtain 200.0 mg white solid, i.e. compound M35-2. ESI-MS m/z: 283.5 $[M+H]^+$.

Step 3: Synthesis of compound M35-3

M35-2 (200.0 mg), M29-2 (2S,4R)-4-hydroxy-N-((S)-1-(4-(4-methylthiazol-5-yl) phenyl)ethyl)pyrrolidine-2-carboxamide (218.4 mg), and HATU (250.5 mg) were dissolved in DMF (5 mL). DIPEA (0.45 mL) was added. The mixture was reacted at room temperature for 10 minutes. The reaction was monitored by LCMS until it was completed. EA (20 mL) and saturated brine (20 mL) were added to the reaction solution. The organic phase was collected, dried with anhydrous sodium sulfate, filtered, concentrated, and separated and purified by column chromatography to obtain 300.0 mg white solid, i.e. compound M35-3. ESI-MS m/z: 696.9 $[M+H]^+$.

Step 4: Synthesis of compound M35-4

M35-3 (300.0 mg) was subjected to SFC resolution. The resultant was concentrated to obtain 135.0 mg white solid, i.e. compound M35-4. ESI-MS m/z: 696.9 $[M+H]^+$.

Step 5: Synthesis of compound M35

M35-4 (135.0 mg) was added to DCM (5 mL) and TFA (2 mL). The mixture was reacted at room temperature for 10 minutes. The reaction was monitored by LC-MS until it was completed. The reaction solution was concentrated directly. 1 M NaOH solution was added to adjust the reaction solution to pH = 8. DCM (20 mL) was added to the reaction solution. The organic phase was collected, washed with saturated brine once, dried with anhydrous sodium sulfate, filtered, and concentrated to obtain 109.0 mg white solid, i.e. compound M35. ESI-MS m/z: 596.9 $[M+H]^+$.

Synthesis of intermediate M36

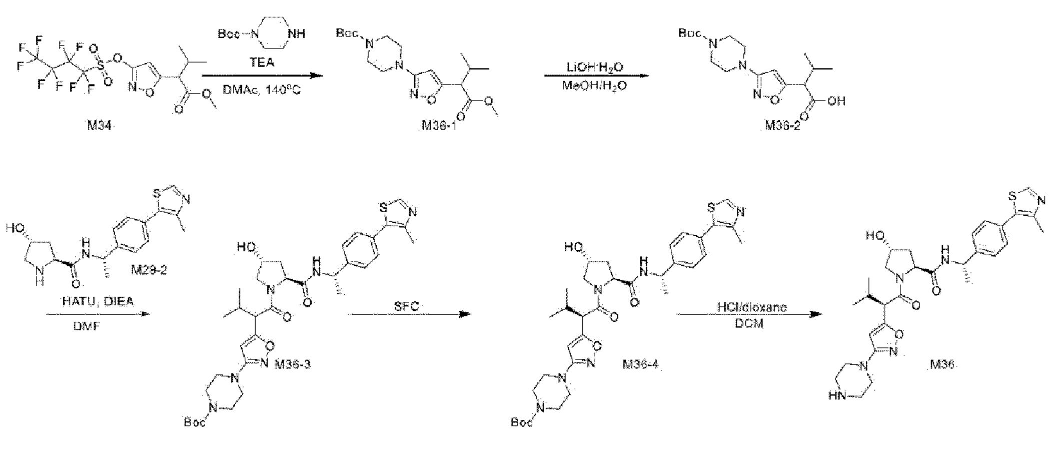

Step 1: Synthesis of compound M36-1

Tert-butyl piperazine-1-carboxylate (2.55 g) was added with N,N-dimethylacetamide (150 mL), followed by addition of triethylamine (5.2 mL). The mixture was heated to 145°C, and stirred for 0.5 hours. Finally, M34 (6.0 g) was added. The mixture was stirred at 145°C for 5 hours. The reaction was monitored by LCMS until it was completed. $H_2O$ was added for dilution. The mixture was extracted with EA twice. The organic layer was washed with saturated brine three times, and further dried with anhydrous sodium sulfate. The resultant was purified by column chromatography to obtain a pale yellow solid (4.2 g), i.e. compound M36-1. ESI-MS m/z: 368.1[M+H]$^+$.

Step 2: Synthesis of compound M36-2

Compound M36-1 (4.2 g) was added to tetrahydrofuran (5 mL), methanol (5 mL), and water (5 mL). Lithium hydroxide monohydrate (1.0 g) was added. The mixture was stirred at 20°C for 1 hour. The reaction was monitored by LCMS until it was completed. The resultant was concentrated under reduced pressure. Dilute hydrochloric acid was added to adjust to pH = 5. The resultant was further extracted with dichloromethane/isopropanol=5/1 twice. The organic layer was washed with saturated brine and dried with anhydrous sodium sulfate. The crude product was used directly in the next step. A pale yellow solid (3.6 g) was obtained, i.e. compound M36-2. ESI-MS m/z: 354.3[M+H]$^+$.

Step 3: Synthesis of compound M36-4

Compound M36-2 (3.5 g) and M29-2 (3.3 g) were added to a 25 mL single-necked flask. N,N-dimethylacetamide (30.00 mL), N,N,N',N'-tetramethyl-O-(7-azabenzotriazol-1-yl)uronium hexafluorophosphate (7.6 g), and N,N-diisopropylethylamine (4.9 mL) were added. The mixture was stirred at 20°C for 0.5 hours. The reaction was monitored by LCMS until it was completed. $H_2O$ was added for dilution. The mixture was extracted with EA twice. The organic layer was washed with saturated brine three times, and dried with anhydrous sodium sulfate. Crude product M36-3 was subjected to SFC resolution. A pale yellow solid (3.4 g) was obtained, i.e. compound M36-4. ESI-MS m/z: 667.2[M+H]$^+$.

Step 4: Synthesis of compound M36

Compound M36-4 (80.0 mg) was added to a 10 mL single-necked flask, and dichloromethane (2.00 mL) and hydrochloric acid (4 M in dioxane) (1.00 mL) were added. The mixture was stirred at 20°C for 0.5 hours. The reaction was monitored by LCMS until it was completed. The resultant was concentrated under reduced pressure. Then, saturated $NaHCO_3$ solution was added to adjust to pH = 8. The resultant was extracted with dichloromethane/isopropanol=5/1 twice. The organic layer was washed with saturated brine and then dried with anhydrous sodium sulfate. The crude product was used directly in the next step. A pale yellow solid (73.0 mg) was obtained, i.e. compound M36. ESI-MS m/z: 567.3[M+H]$^+$.

Synthesis of intermediate M37

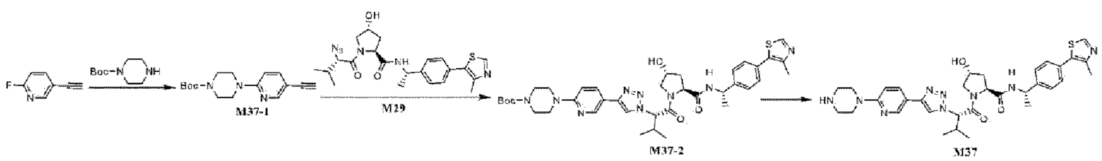

Step 1: Synthesis of compound M37-1

5-Ethynyl-2-fluoropyridine (500 mg), 1-tert-butoxycarbonyl-piperazine (922 mg), acetone (5 mL), and DIPEA (1.6 g) were added to a 50 mL flask. The mixture was heated to 60°C and reacted for 5 h. The solvent was removed. The resultant was dissolved in EA, washed with water and brine, dried with sodium sulfate, and filtered. The filtrate was concentrated under reduced pressure to remove the solvent. The crude product was purified by column chromatography to obtain compound M37-1 (1.02g).

Step 2: Synthesis of compound M37-2

M37-1 (200 mg), M29 (250 mg), sodium ascorbate (226 mg), and copper sulfate (108 mg), tert-butanol 3 mL, THF 3 mL, and water 3 mL were added to a 50 mL flask. The mixture was reacted at room temperature for 1 hour. The reaction

was monitored until the reaction was completed. EA was added for extraction. The organic phase was washed with water and brine, dried with sodium sulfate, and filtered. The filtrate was concentrated under reduced pressure to obtain a crude product. The crude product was purified by column chromatography to obtain compound M37-2 (198 mg).

Step 3: Synthesis of compound M37

M37-2 (198 mg) and a 4 M solution of hydrogen chloride in dioxane (5 mL) were added to a 50 mL flask. The mixture was reacted at room temperature for 1 hour. The solvent was removed under reduced pressure. About 200 mg crude product M37 was obtained.

Synthesis of intermediate M38

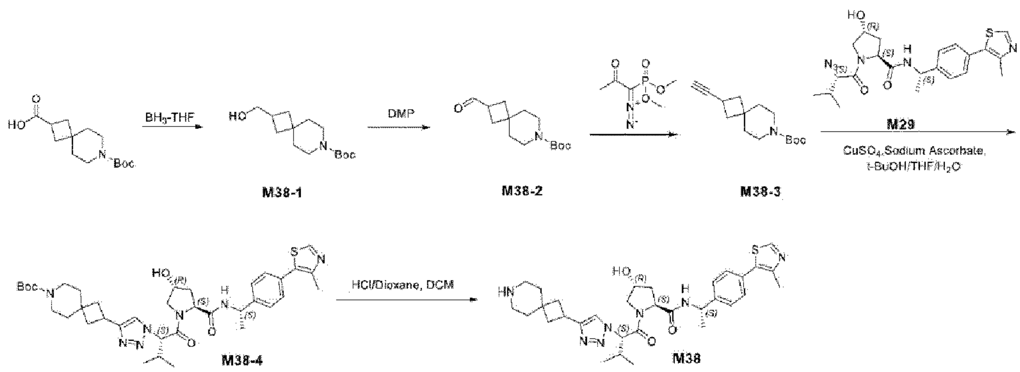


Step 1: Synthesis of compound M38-1

7-Tert-butoxycarbonyl-7-azaspiro[3.5]nonane-2-carboxylic acid (500.0 mg) was dissolved in 5.0 mL THF, and borane tetrahydrofuran solution (4.64 mL, 1 mol/L) was added. The mixture was reacted at room temperature for 1 h. 5 mL HCl/MeOH solution was slowly added to the reaction solution and stirred until gas evolution ceased. 1 mol/L NaOH solution was added to adjust the pH to alkaline, and then an appropriate amount of ethyl acetate was added for extraction. The organic phase was collected and dried with anhydrous sodium sulfate, filtered, and concentrated under reduced pressure to obtain compound M38-1 (450.0 mg). ESI-MS m/z: 200 $[M+H-56]^+$.

Step 2: Synthesis of compound M38-2

M38-1 (250.0 mg) was dissolved in 5.0 mL DCM, and Dess-Martin reagent (387.0 mg) was added. The mixture was reacted at room temperature for 1 h. An appropriate amount of ethyl acetate was added to the reaction solution. Solids were precipitated and removed by filtering. 1 mol/L NaOH solution was added to the filtrate to adjust the pH to alkaline, which was then extracted. The organic phase was collected, dried with anhydrous sodium sulfate, filtered, and concentrated under reduced pressure to obtain compound M38-2 (180.0 mg). ESI-MS m/z: 198 $[M+H-56]^+$.

Step 3: Synthesis of compound M38-3

Compound M38-2 (180 mg) was dissolved in 3.0 mL anhydrous methanol under $N_2$ protection. Anhydrous potassium carbonate (167 mg) and dimethyl (1-diazo-2-oxopropyl)phosphonate (174 mg) were added. The mixture was reacted at room temperature for 3 h. An appropriate amount of water and ethyl acetate were added to the reaction solution for extraction. The organic phase was collected, dried with anhydrous sodium sulfate, filtered, and concentrated under reduced pressure to obtain compound M38-3 (110 mg). ESI-MS m/z: 194 $[M+H-56]^+$.

Step 4: Synthesis of compound M38-4

Compound M29 (200 mg) and compound M38-3 (131 mg) were dissolved in tert-butanol (2 mL), tetrahydrofuran (2 mL), and water (2 mL). Anhydrous copper sulfate (63 mg) and sodium ascorbate (226 mg) were added. The mixture was reacted at room temperature for 2 h. The reaction solution was dried by rotary evaporation. DCM and water were added for extraction. The organic phase was collected, dried with anhydrous sodium sulfate, and filtered. The filtrate was collected,

concentrated, and then purified by column chromatography (DCM/MeOH = 15: 1) to obtain compound M38-4 (295 mg). ESI-MS m/z: 706 [M+H]+.

Step 5: Synthesis of compound M38

Compound M38-4 (295 mg) was dissolved in 6 mL DCM and 3 mL solution of hydrogen chloride in dioxane (4 mol/L). The mixture was reacted at room temperature for 0.5 h. The reaction solution was dried by rotary evaporation. DCM/isopropanol (3:1) and saturated sodium bicarbonate solution were added for extraction. The organic phase was collected, dried with anhydrous sodium sulfate, and filtered. The filtrate was collected and concentrated under reduced pressure to obtain compound M38 (240 mg). ESI-MS m/z: 606 [M+H]+.

Preparation of intermediate M39 refers to the preparation of 176-4 in Example 176 of WO2023138524A1.

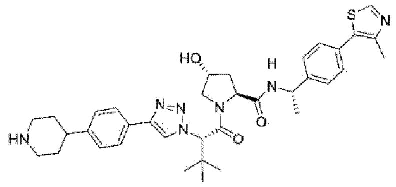

M39

Synthesis of intermediate M40

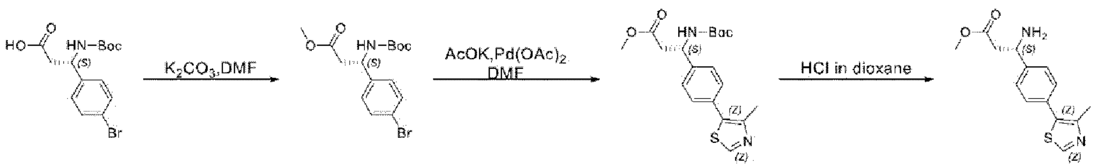


M40-1 M40-2 M40

Step 1: Synthesis of compound M40-1

Compound (S)-3-(4-bromophenyl)-3-((tert-butoxycarbonyl)amino)propanoic acid (1.5 g) was added to a 50 mL single-necked flask and dissolved in DMF (15 mL). Iodomethane (0.74 g) and potassium carbonate (1.2 g) were added. The mixture was reacted at room temperature for 2 h. Saturated brine (5 mL) was added to the reaction solution. The resultant was extracted with EA. The organic phase was dried with anhydrous sodium sulfate to obtain 1.4 g yellow solid, i.e. compound M40-1.

Step 2: Synthesis of compound M40-2

Compound M40-1 (1.4 g) was added to a 50 mL single-necked flask and dissolved in DMF (20 mL). 4-Methylthiazole (1.2 g), potassium acetate (1.2 g), and palladium acetate (0.1 g) were added. After nitrogen protection, the mixture was heated to 80°C and reacted for 5 h. Saturated brine (5 mL) was added to the reaction solution. The reaction solution was extracted with EA. The organic phase was dried with anhydrous sodium sulfate, and purified by column chromatography (PE: EA=4:1) to obtain 0.82 g yellow solid, i.e. compound M40-2.

Step 3: Synthesis of compound M40

Compound M40-2 (0.82 g) was added to a 25 mL single-necked flask and dissolved in a 4 M solution of hydrogen chloride in dioxane (20 mL). Methanol (1 mL) was added. The mixture was reacted at room temperature for 1 h. The reaction solution was concentrated under reduced pressure to obtain 0.55 g yellow solid, i.e. compound M40.

Synthesis of intermediate M42

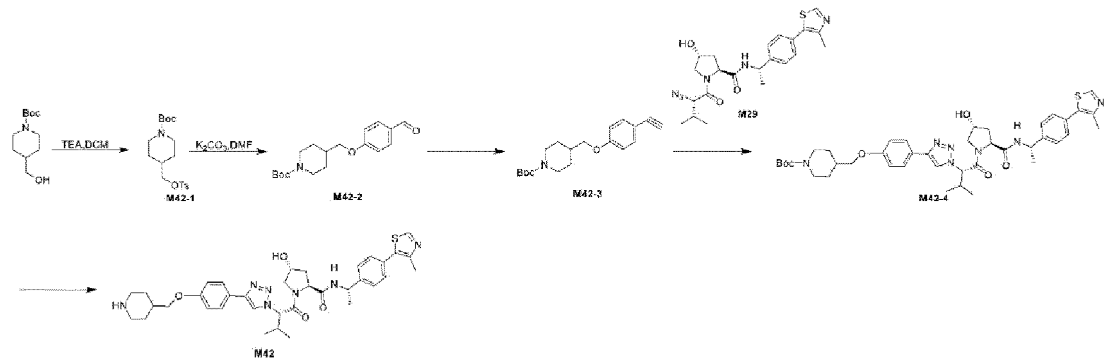


Step 1: Synthesis of compound M42-1

Tert-butyl 4-(hydroxymethyl)piperidine-1-carboxylate (5.00g, 23.22 mmol), TEA (6.46 mL, 46.45 mmol), and TsCl (6.64 g, 34.84 mmol) were added successively to DCM (50.00 mL). The mixture was reacted at room temperature for 16 h. 80 mL ice water was added to the reaction solution. The mixture was extracted with DCM (100.00 mL) twice, washed with saturated brine twice, and dried with anhydrous sodium sulfate. The resultant was purified by column chromatography (PE:EA=85%:15%) to obtain compound M42-1 (8.00 g, 93.23%).
ESI-MS m/z: 270.16 $[M+H]^+$.

Step 2: Synthesis of compound M42-2

Compound M42-1 (8000.00 mg, 21.65 mmol), p-hydroxybenzaldehyde (2908.62 mg, 23.82 mmol), and cesium carbonate (21164.30 mg, 64.96 mmol) were added to DMF (80.00 mL). The mixture was reacted at 80°C for 2 h. Water was added to the reaction solution. The product was precipitated, and further filtered. The filter cake was washed with water and dried to obtain compound M42-2 (6100.00 mg, 88.21%).
ESI-MS m/z: 220.15 $[M+H]^+$.

Step 3: Synthesis of compound M42-3

Compound M42-2 (3.00g, 9.39 mmol) and potassium carbonate (2.60 g, 18.79 mmol) were added to methanol (30.00 mL). Dimethyl (1-diazo-2-oxopropyl)phosphonate (1.83 mL, 12.21 mmol) was added under stirring. The mixture was reacted at room temperature for 6 h. The reaction solution was concentrated. 50 mL water was added. The mixture was extracted with EA (100.00 mL) twice. The combined organic phases were dried with anhydrous sodium sulfate and purified by column chromatography (PE:EA=90%:10%) to obtain compound M42-3 (2.30 g, 77.64%).
ESI-MS m/z: 216.19 $[M+H]^+$.

Step 4: Synthesis of compound M42-4

Compound M42-3 (276.36 mg, 0.88 mmol), M29 (200.00 mg, 0.44 mmol), copper sulfate (62.93 mg, 0.39 mmol), sodium ascorbate (225.66 mg, 1.14 mmol), water (2.00 mL), and tert-butanol (2.00 mL) were added successively to tetrahydrofuran (2.00 mL). The mixture was reacted at room temperature for 2 h. The reaction solution was concentrated, and 30 mL water was added. The resultant was extracted with DCM (80.00 mL) twice, dried with anhydrous sodium sulfate and purified by column chromatography (DCM:MeOH=95%:5%) to obtain compound M42-4 (300.00 mg, 88.71%).
ESI-MS m/z: 772.52 $[M+H]^+$.

Step 5: Synthesis of compound M42

Compound M42-4 (300.00 mg, 0.39 mmol) and trifluoroacetic acid (1.00 mL) were added successively to dichloromethane (2.00 mL). The mixture was reacted at room temperature for 1 h. Saturated aqueous sodium bicarbonate solution was added to the reaction solution to adjust to alkaline. The mixture was extracted with DCM (80.00 mL) twice, dried with anhydrous sodium sulfate and purified by column chromatography (DCM:MeOH=93%:7%) to obtain compound M42 (130.00 mg, 49.79%).
ESI-MS m/z: 672.72 $[M+H]^+$.

Synthesis of intermediate M43

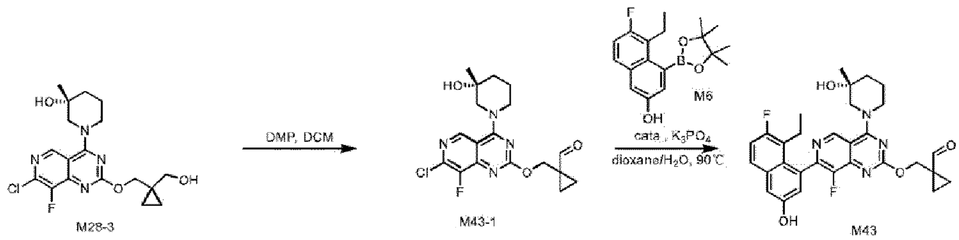


Step 1: Synthesis of compound M43-1

Compound M28-3 (16.5 g) was dissolved in DCM (200 mL). DMP (18.4 g) was added slowly in batches at 0°C. The mixture was warm naturally to room temperature and reacted for 2 hours. After the reaction was completed, water was added for dilution. The mixture was extracted with DCM. The organic phase was washed with saturated brine and purified by column chromatography to obtain compound M43-1 (9.5 g). ESI-MS m/z:395.2 $[M+H]^+$.

Step 2: Synthesis of compound M43

Compound M43-1 (9.5 g), M6 (15.21 g), mesylate[(di(1-adamantyl)-n-butylphosphine)-2-(2'-amino-1,1'-biphenyl)]palladium (II) (3.50 g), and potassium phosphate (15.3 g) were added to 1,4-dioxane (100.00 mL) and water (25 mL). The mixture was reacted at 90°C under nitrogen protection for 1.5 hours. After the reaction was completed, water was added for dilution. Ethyl acetate was added for extraction. The organic phase was washed with saturated brine and purified by column chromatography to obtain compound M43 (8.5 g). ESI-MS m/z:549.1 $[M+H]^+$.

Synthesis of intermediate M44

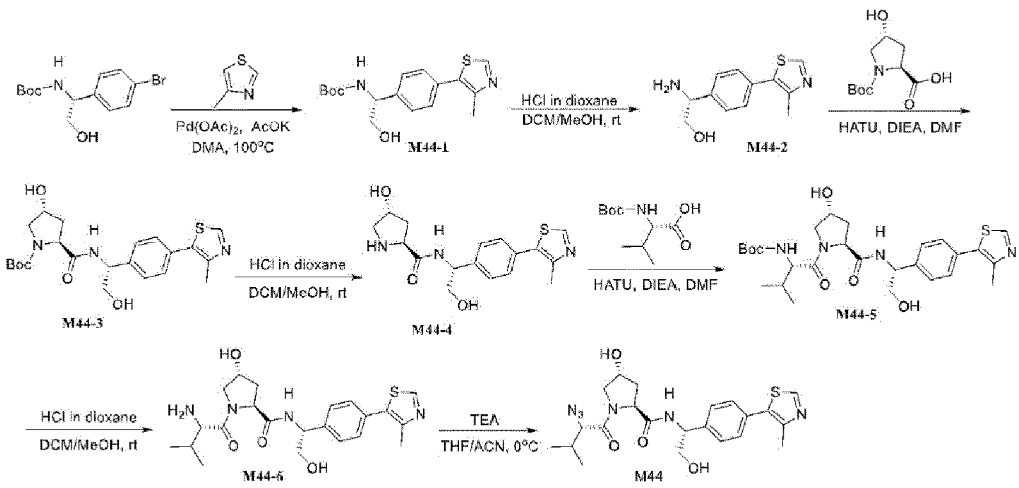


Step 1: Synthesis of compound M44-1

Commercially available tert-butyl N-((1R)-1-(4-bromophenyl)-2-hydroxyethyl)carbamate (2.50 g), 4-methylthiazole (1.57 g), palladium acetate (0.18 g), potassium acetate (1.55 g, 15.81 mmol), and N,N-dimethylacetamide (40.00 mL) were added to a reaction flask. After nitrogen displacement, the mixture was reacted at 100°C under nitrogen protection for 10 h. $H_2O$ (100 mL) was added for dilution. The mixture was extracted with EA (80 mL) twice. The organic layers were washed with saturated brine, further dried with anhydrous sodium sulfate, concentrated, and purified by column chromatography (DCM: ammonia in methanol = 20:1) to obtain a yellow solid, i.e. compound M44-1 (2.01 g). ESI-MS m/z: 335.29 $[M+H]^+$.

Step 2: Synthesis of compound M44-2

Compound M44-1 (2.01 g), dichloromethane (20.00 mL), and methanol (10.00 mL) were added to a reaction flask. A 4 M solution of hydrogen chloride in dioxane (10.00 mL) was added under stirring at 0°C. The mixture was reacted at room temperature for 1 h. The solvent was concentrated to obtain a yellow solid crude product M44-2 (1.85 g). ESI-MS m/z: 218.21 $[M-OH]^+$.

Step 3: Synthesis of compound M44-3

Compound M44-2 (1.50 g), commercially available (2S,4R)-4-hydroxy-1-[(2-methylpropan-2-yl)oxycarbonyl] pyrrolidine-2-carboxylic acid (1.48 g), N,N-dimethylformamide (25.00 mL), and N,N-diisopropylethylamine (3.17 mL) were added to a reaction flask. N,N,N',N'-tetramethyl-O-(7-azabenzotriazol-1-yl)uronium hexafluorophosphate (3.16 g) was added under stirring. The mixture was reacted at room temperature for 0.5 h. Saturated brine was added for dilution, and the resultant was extracted with EA. The aqueous phase was extracted with EA:isopropanol = 9:1. The organic phases were combined, washed once again with saturated brine, dried with anhydrous sodium sulfate, and concentrated. The resultant was purified by column chromatography (DCM:ammonia in methanol = 15:1) to obtain yellow solid M44-3 (2.02 g). ESI-MS m/z: 448.20 $[M+H]^+$.

Step 4: Synthesis of compound M44-4

Compound M44-3 (2.02 g), dichloromethane (20.00 mL), and methanol (10.00 mL) were added to a reaction flask. A 4 M solution of hydrogen chloride in dioxane (10.00 mL) was added under stirring at 0°C. The mixture was reacted at room temperature for 1 h. The solvent was concentrated to obtain a yellow solid crude product M44-4 (1.86 g). ESI-MS m/z: 348.18 $[M+H]^+$.

Step 5: Synthesis of compound M44-5

Compound M44-4 (1.55 g), commercially available (2S)-3-methyl-2-((2-methylpropan-2-yl)oxycarbonylamino) butanoic acid (1.07 g), N,N-dimethylformamide (20.00 mL), and N,N-diisopropylethylamine (2.21 mL) were added to a reaction flask. N,N,N',N'-tetramethyl-O-(7-azabenzotriazol-1-yl)uronium hexafluorophosphate (2.21 g) was added under stirring. The mixture was reacted at room temperature for 0.5 h. Saturated brine was added for dilution, and the resultant was extracted with EA. The aqueous phase was extracted with EA:isopropanol = 9:1. The organic phases were combined, washed once again with saturated brine, dried with anhydrous sodium sulfate, and concentrated. The product was isolated by column chromatography (DCM:ammonia in methanol = 15:1) to obtain yellow solid M44-5 (2.01 g). ESI-MS m/z: 547.30 $[M+H]^+$.

Step 6: Synthesis of compound M44-6

Compound M44-5 (2.01 g), dichloromethane (20.00 mL), and methanol (10.00 mL) were added to a reaction flask. A 4 M solution of hydrogen chloride in dioxane (10.00 mL) was added under stirring at 0°C. The mixture was reacted at room temperature for 1 h. The solvent was concentrated. Sodium bicarbonate aqueous solution was added in an ice bath to adjust to pH = 8. The resultant was purified by reverse-phase column ($MeOH/H_2O$=95%:5%) to obtain a pale yellow solid M44-6 (1.5 g). ESI-MS m/z: 447.30 $[M+H]^+$.

Step 7: Synthesis of compound M44

Compound M44-6 (1.50 g), tetrahydrofuran (15.00 mL), acetonitrile (7.50 mL), and triethylamine (1.87 mL) were added to a reaction flask. The mixture was cooled to 0°C. 2-azido-1,3-dimethylimidazolinium hexafluorophosphate (1.91 g, 6.72 mmol) was dissolved in acetonitrile (7.50 mL), and then the solution was slowly added to the reaction flask. After the addition was completed, the mixture was reacted at this temperature for 1 h. An appropriate amount of saturated brine was added to the reaction solution. The resultant was extracted with EA. The aqueous phase was further extracted with EA : isopropanol = 9 : 1. The organic phases were combined, washed with saturated brine, dried with anhydrous sodium sulfate, and concentrated. The resultant was purified by column chromatography (7% ammonia in MeOH/DCM) to obtain a pale yellow solid M44 (0.92 g). ESI-MS m/z: 473.20 $[M+H]^+$.

Synthesis of intermediate M45

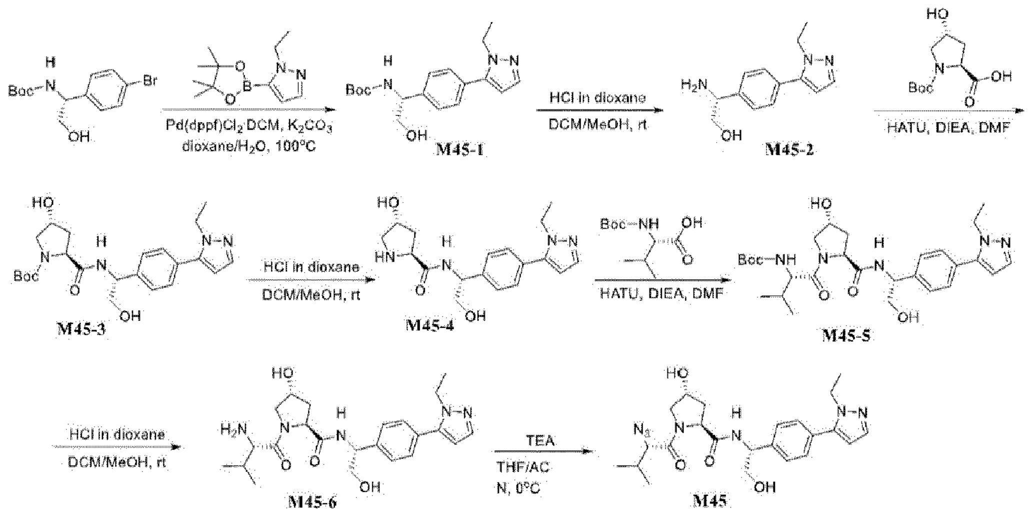


Step 1: Synthesis of compound M45-1

Commercially available 1-ethyl-5-(4,4,5,5-tetramethyl-1,3,2-dioxoboran-2-yl)pyrazole (1.83 g), tert-butyl N-((1R)-1-(4-bromophenyl)-2-hydroxyethyl)carbamate (2.00 g), potassium carbonate (1.75 g), [1,1'-bis(diphenylphosphino)ferrocene]palladium dichloride dichloromethane complex (0.52 g), 1,4-dioxane (35.00 mL) and water (7.00 mL) were added to a reaction flask. After nitrogen displacement, the mixture was reacted at 100°C under nitrogen protection for 2 h. Sampling showed the raw material was reacted completely. Water was added for dilution. The resultant was extracted with EA. The organic phase was dried with anhydrous sodium sulfate and concentrated. The resultant was purified by column chromatography (DCM:EA=3:1) to obtain a yellow solid, i.e. compound M45-1 (1.7 g). ESI-MS m/z: 332.29 $[M+H]^+$.

Step 2: Synthesis of compound M45-2

Compound M45-1 (1.7 g), dichloromethane (20.00 mL), and methanol (10.00 mL) were added to a reaction flask. A 4 M solution of hydrogen chloride in dioxane (10.00 mL) was added under stirring at 0°C. The mixture was reacted at room temperature for 1 h. The solvent was concentrated to obtain a yellow solid crude product M45-2 (1.35 g). ESI-MS m/z: 215.21 $[M-OH]^+$.

Step 3: Synthesis of compound M45-3

Compound M45-2 (1.30 g), commercially available (2S)-3-methyl-2-((2-methylpropan-2-yl)oxycarbonylamino) butanoic acid (0.82 g), N,N-dimethylformamide (1.00 mL), and N,N-diisopropylethylamine (1.25 mL) were added to a reaction flask. N,N,N',N'-tetramethyl-O-(7-azabenzotriazol-1-yl)uronium hexafluorophosphate (1.87 g) was added under stirring. The mixture was reacted at room temperature for 0.5 h. Saturated brine was added for dilution, and the resultant was extracted with EA. The aqueous phase was extracted with EA:isopropanol = 9:1. The organic phases were combined, washed with saturated brine once, dried with anhydrous sodium sulfate, and concentrated. The product was isolated by column chromatography (DCM:ammonia in methanol = 20:1) to obtain yellow solid M45-3 (1.75 g). ESI-MS m/z: 445.20 $[M+H]^+$.

Step 4: Synthesis of compound M45-4

Compound M45-3 (1.75 g), dichloromethane (20.00 mL), and methanol (10.00 mL) were added to a reaction flask. A 4 M solution of hydrogen chloride in dioxane (10.00 mL) was added under stirring at 0°C. The mixture was reacted at room temperature for 1 h. The solvent was concentrated to obtain a yellow solid crude product M45-4 (1.66 g). ESI-MS m/z: 345.18 $[M+H]^+$.

Step 5: Synthesis of compound M45-5

Compound M45-4 (1.30 g), commercially available (2S)-3-methyl-2-((2-methylpropan-2-yl)oxycarbonylamino) butanoic acid (0.82 g), N,N-dimethylformamide (1.00mL), and N,N-diisopropylethylamine (1.25 mL) were added to a

reaction flask. N,N,N',N'-tetramethyl-O-(7-azabenzotriazol-1-yl)uronium hexafluorophosphate (1.87 g) was added under stirring. The mixture was reacted at room temperature for 0.5 h. Saturated brine was added for dilution, and the resultant was extracted with EA. The aqueous phase was extracted with EA:isopropanol = 9:1. The organic phases were combined, washed once again with saturated brine, dried with anhydrous sodium sulfate, and concentrated. The resultant was isolated by column chromatography (DCM:ammonia in methanol = 20:1) to obtain yellow solid M45-5 (1.8 g). ESI-MS m/z: 544.30 $[M+H]^+$.

Step 6: Synthesis of compound M45-6

Compound M45-5 (1.8 g), dichloromethane (20.00 mL), and methanol (10.00 mL) were added to a reaction flask. A 4 M solution of hydrogen chloride in dioxane (10.00 mL) was added under stirring at 0°C. The mixture was reacted at room temperature for 1 h. The solvent was concentrated. Sodium bicarbonate aqueous solution was added in an ice bath to adjust to pH = 8. The resultant was purified by reverse-phase column to obtain a pale yellow solid M45-6 (1.12 g). ESI-MS m/z: 444.30 $[M+H]^+$.

Step 7: Synthesis of compound M45

Compound M45-6 (1.10 g), tetrahydrofuran (10.00 mL), acetonitrile (5.00 mL), and triethylamine (1.38 mL) were added to a reaction flask. The mixture was cooled to 0°C. 2-azido-1,3-dimethylimidazolinium hexafluorophosphate (1.41 g, 4.96 mmol) was dissolved in acetonitrile (5.00 mL), which was then added to the reaction solution. After the addition was completed, the mixture was reacted at this temperature for 1 h. An appropriate amount of saturated brine was added to the reaction solution for dilution. EA was added for extraction. The aqueous phase was further extracted with EA: isopropanol=9:1. The organic phases were combined, washed with saturated brine, dried with anhydrous sodium sulfate, and concentrated. The product was isolated by column chromatography to obtain a pale yellow solid M45 (0.8 g). ESI-MS m/z: 470.20 $[M+H]^+$.

Synthesis of intermediate M46

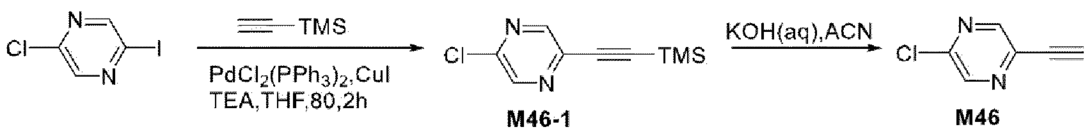


Step 1: Synthesis of compound M46-1

2-Chloro-5-iodopyrazine (1 g), trimethylsilylacetylene (0.88 mL), bis(triphenylphosphine)palladium chloride (0.36 g), and copper iodide (0.1 g) were dissolved in 10 mL tetrahydrofuran. Triethylamine (2.16 mL) was added. Nitrogen displacement was performed three times. The mixture was reacted at 80°C for 2 h. The reaction was monitored by LCMS until it was completed. 10 mL water was added to the reaction solution. The resultant was extracted with DCM:MeOH three times. The organic phases were combined, washed with saturated brine, dried with sodium sulfate, filtered, concentrated, and separated and purified by column chromatography to obtain 0.5 g white solid, i.e. compound M46-1. ESI-MS m/z: 211 $[M+H]^+$.

Step 2: Synthesis of compound M46

M46-1 (0.5 g) was dissolved in 2 mL ACN. A 5 M potassium hydroxide aqueous solution (1.25 mL) was added dropwise in an ice bath. The mixture was stirred at a maintained temperature for 15 min. The reaction was monitored by LCMS until it was completed. The mixture was extracted with EA three times, 10 mL each time. The organic phases were combined, washed with saturated brine, dried with sodium sulfate, filtered, concentrated, and separated and purified by column chromatography to obtain 0.17 g white solid, i.e. M46. ESI-MS m/z: 139$[M+H]^+$.

Synthesis of intermediate M47

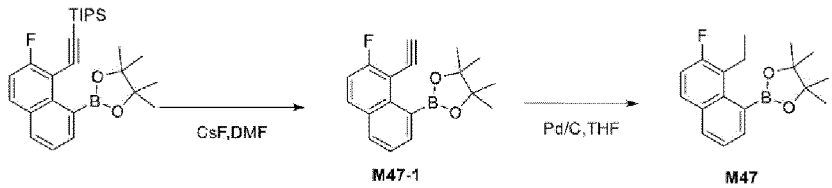


Step 1: Synthesis of compound M47-1

((2-Fluoro-8-(4,4,5,5-tetramethyl-1,3,2-dioxaborolan-2-yl)naphthalen-1-yl)ethynyl)triisopro pylsilane (1 g) was dissolved in 10 mL DMF. Cesium fluoride (3.36 g) was added. The mixture was reacted at 25°C for 5 h. The reaction was monitored by TLC until it was completed. Water was added for dilution. The mixture was extracted with EA three times. The organic phases were combined, dried with sodium sulfate, filtered, and concentrated to obtain 0.65 g white solid, i.e. M47-1. The obtained crude product was used directly in the next step without purification. ESI-MS m/z: 297[M+H]$^+$.

Step 2: Synthesis of compound M47

M47-1 (0.65 g) was dissolved in 10 mL THF, and then 10% Pd/C (0.4 g) was added. Hydrogen displacement was performed three times. The mixture was stirred at 25°C for 12h. The reaction was monitored by TLC until it was completed. The mixture was filtered by diatomaceous earth. The organic phase was concentrated, and separated and purified by column chromatography to obtain 0.48 g white solid, i.e. M47. ESI-MS m/z: 301[M+H]$^+$.

Synthesis of intermediate M48

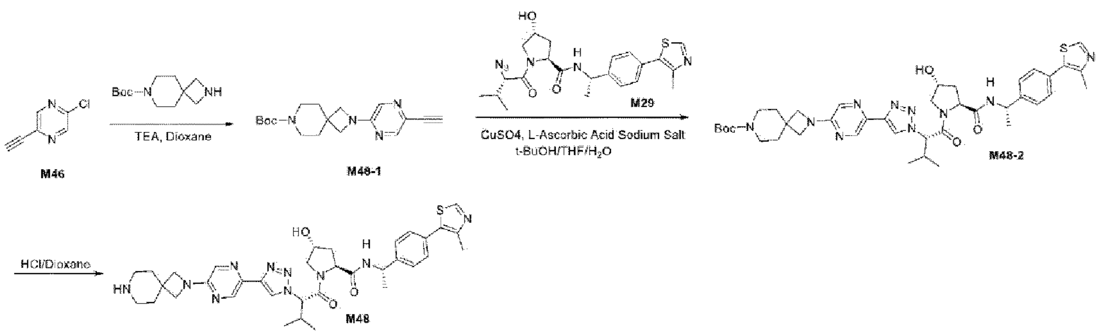


Step 1: Synthesis of compound M48-1

Compound M46 (0.83 g) and tert-butyl 2,7-diazaspiro[3.5]nonane-7-carboxylate (1.63 g) were dissolved in 15.0 mL 1,4-dioxane. Triethylamine (2.5 mL) was added. The mixture was heated to 80°C under $N_2$ atmosphere and reacted for 2 h. The reaction solution was concentrated under reduced pressure. The concentrate was purified by column chromatography (PE/EA = 65:35) to obtain compound M48-1 (1.6 g). ESI-MS m/z: 329.1 [M+H]$^+$.

Step 2: Synthesis of compound M48-2

Compound M48-1 (300 mg) and compound M29 (459 mg) were dissolved in tert-butanol (5 mL), tetrahydrofuran (5 mL), and water (5 mL). Anhydrous copper sulfate (131 mg) and sodium ascorbate (470 mg) were added. The mixture was reacted at room temperature for 2 h. The reaction solution was dried by rotary evaporation. DCM/MeOH (10:1) and water were added for extraction. The organic phase was collected, dried with anhydrous sodium sulfate, and filtered. The filtrate was collected and concentrated under reduced pressure, and then purified by column chromatography (DCM/MeOH = 10:1) to obtain compound M48-2 (646 mg). ESI-MS m/z: 785.3 [M+H]$^+$.

Step 3: Synthesis of intermediate M48

Compound M48-2 (600 mg) was dissolved in 8 mL DCM and 8 mL solution of hydrogen chloride in dioxane (4 M). The mixture was reacted at room temperature for 0.5 h. After the reaction was completed, the reaction solution was dried

by rotary evaporation. Water was added for dilution. The mixture was extracted with EA. The aqueous phase was collected. Anhydrous sodium carbonate was added to adjust the aqueous phase pH to basic. DCM/isopropanol (3:1) was added for extraction. The organic phases were collected, combined, dried with anhydrous sodium sulfate, and filtered. The filtrate was collected and concentrated under reduced pressure to obtain compound M48 (498 mg). ESI-MS m/z: 685.3 $[M+H]^+$.

Example 1: Synthesis of compound (2S,4R)-1-((2S)-2-(4-((1-((1-(((7-(8-ethyl-7-fluoro-3-hydroxynaphthalen-1-yl)-8-fluoro-4-(3-hyd roxy-3-methylpiperidin-1-yl)pyrido[4,3-d]pyrimidin-2-yl)oxy)methyl)cyclopropyl)methyl)piperi din-4-yl) methoxy)benzamido)-3,3-dimethylbutanoyl)-4-hydroxy-N-((S)-1-(4-(4-methylthiazol-5 -yl)phenyl)ethyl)pyrrolidine-2-carboxamide

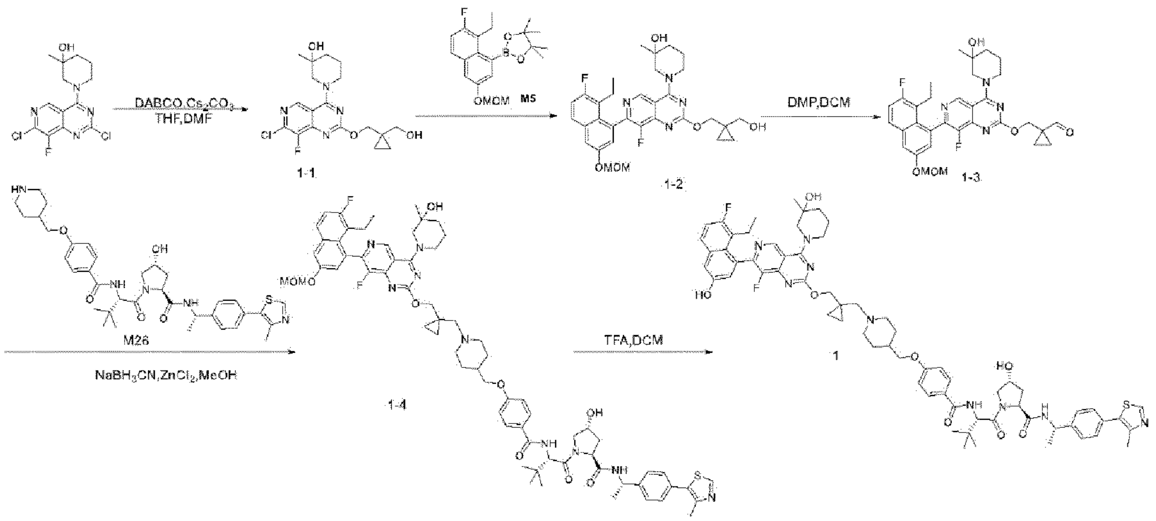


Step 1: Synthesis of compound 1-1

Compound M1 (0.25 g) was added to a 50 mL single-necked flask, dissolved in DMF (5 mL) and THF (5 mL). 1,1-Cyclopropane dimethanol (0.31 g), cesium carbonate (0.738 g), and DABCO (8.47 mg) were added. The mixture was reacted at 40°C under the protection of $N_2$ for 5 hours. After cooling to room temperature, an appropriate amount of water was added to the reaction solution. The solution was separated, and the aqueous phase was extracted with DCM. The organic phases were combined and dried with anhydrous sodium sulfate. The resultant was separated by column chromatography (DCM:MeOH = 10:1) to obtain 0.278 g of a light yellow solid, i.e., compound 1-1.

Step 2: Synthesis of compound 1-2

Compound 1-1 (200.0 mg) was added to a 25 mL single-necked flask, dissolved in THF (10 mL) and water (2 mL). Compound M5 (379.3 mg), $K_3PO_4$ (321.0 mg), and CataCXium A Pd G3 (36.7 mg) were added. The mixture was reacted at 75°C under the protection of $N_2$ for 2 hours. The reaction solution was directly evaporated to dryness. The resultant was separated and purified by column chromatography (DCM:MeOH = 10:1) to obtain 210.5 mg of a yellow solid, i.e., compound 1-2.

Step 3: Synthesis of compound 1-3

Compound 1-2 (210.0 mg) was added to a 25 mL single-necked flask, dissolved in DCM (10 mL), and DMP (299.5 mg) was added. The mixture was reacted at room temperature for 4 hours. The reaction solution was quenched by adding sodium thiosulfate solution. The solution was extracted three times with DCM, and the organic phase was washed with water and saturated brine. The resultant was evaporated to dryness, and separated by column chromatography ((DCM:MeOH = 10:1)) to obtain 192 mg of a light yellow solid, i.e., compound 1-3.

Step 4: Synthesis of compound 1-4

Compound 1-3 (100 mg) was added to a 25 mL single-necked flask, dissolved in MeOH (3 mL). Compound M26 (167.5 mg) and 1 M $ZnCl_2$/THF solution (0.1 mL) were added. After the reaction was heated to 40°C for 2 hours, $NaBH_3CN$ (53.1 mg) was added. The mixture was reacted overnight. The reaction solution was directly evaporated to dryness. The resultant was separated and purified by column chromatography (DCM:MeOH = 8:1) to obtain 84.0 mg of a light yellow solid, i.e., compound 1-4.

Step 5: Synthesis of compound 1

Compound 1-4 (84.0 mg) was added to a 25 mL single-necked flask, dissolved in DCM (2 mL), and TFA (1 mL) was added. The mixture was reacted at room temperature for 1 hour. The reaction solution was evaporated to dryness. The resultant was separated by reversed phase column chromatography ($CH_3CN/H_2O$ = 30%-55%) to obtain 30.6 mg of a white solid, i.e., compound 1 (purity 99.14%).

LCMS: 1/2$[M+2H]^+$= 598.31.

1H NMR (500 MHz, MeOD) δ 9.31 (s, 1H), 8.98 (s, 1H), 7.78 (d, J = 8.6 Hz, 2H), 7.71 - 7.62 (m, 2H), 7.49 - 7.37 (m, 4H), 7.33 (s, 1H), 7.25 (t, J = 9.3 Hz, 1H), 7.07 (s, 1H), 6.96 (d, J = 7.6 Hz, 2H), 5.03 (dd, J = 12.9, 6.2 Hz, 2H), 4.70 - 4.59 (m, 2H), 4.57 - 4.45 (m, 3H), 4.37 (t, J = 14.8 Hz, 2H), 4.05 - 3.94(m, 5H), 3.86 - 3.76 (m, 1H), 3.66 - 3.51 (m, 2H), 3.47 - 3.35 (m, 2H), 3.04 (t, J = 12.4 Hz, 2H), 2.51 - 2.39 (m, 4H), 2.28 - 2.09 (m, 7H), 1.92 - 1.80 (m, 3H), 1.77 - 1.68 (m, 2H), 1.52 (d, J = 6.8 Hz, 3H), 1.27 (d, J = 8.3 Hz, 3H), 1.17 - 1.06 (m, 9H), 1.06 - 0.98 (m, 2H), 0.92-0.86 (m, 2H), 0.82 (q, J = 7.6 Hz, 3H).

Example 2: Synthesis of compound ((3R,7aR)-7a-(((7-(8-ethyl-7-fluoro-3-hydroxy naphthalen-1-yl)-8-fluoro-4-(3-hydroxy-3-methylpiperidin-1-yl)pyrido[4,3-d]pyrimidin-2-yl)oxy )methyl)hexahydro-1H-pyrrolizin-3-yl)methyl 2-(((S)-1-((2S,4R)-4-hydroxy-2-(((S)-1-(4-(4-methylthiazol-5-yl)phenyl)ethyl)carbamoyl)pyrrolidin-1-yl)-3,3-dimethyl-1-oxobutan-2-yl)carba moyl)-7-azaspiro[3.5]nonane-7-carboxylate

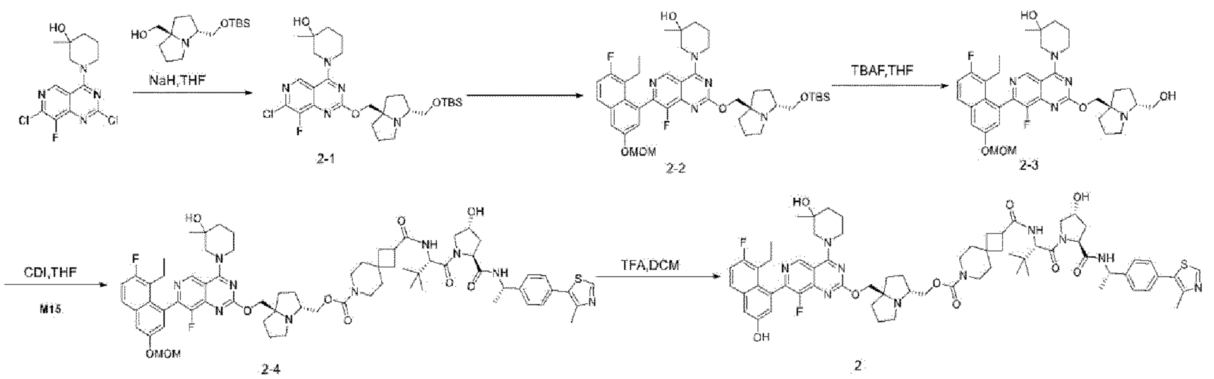


Step 1: Synthesis of compound 2-1

((3R,7aR)-3-((tert-butyldimethylsilyl)oxy)methyl)tetrahydro-1H-pyrrolizin-7a(5H)-yl)meth anol (100.0 mg) was added to a 25 mL single-necked flask, dissolved in THF (5 mL), and NaH (38.7 mg) was added. After the mixture was reacted at room temperature for 0.5 hours, compound M12 (82.0 mg) was added. The mixture was reacted at room temperature for 2 hours. The reaction solution was quenched by adding saturated ammonium chloride solution. The solution was extracted three times with EA, and the organic phase was washed with water and saturated brine. The resultant was concentrated under reduced pressure, and then separated by column chromatography ((DCM:MeOH = 10:1)) to obtain 104.0 mg of a light yellow solid, i.e., compound 2-1.

Step 2: Synthesis of compound 2-2

Compound 2-1 (104.0 mg) was added to a 25 mL single-necked flask, dissolved in THF (10 mL) and water (2 mL). Compound M5 (124.0 mg), $K_3PO_4$ (109.8 mg), and CataCXium A Pd G3 (12.5 mg) were added. The mixture was reacted at 75°C under the protection of $N_2$ for 2 hours. The reaction solution was concentrated under reduced pressure, and then separated and purified by column chromatography (DCM:MeOH = 10:1) to obtain 106.0 mg of a yellow solid, i.e., compound 2-2.

Step 3: Synthesis of compound 2-3

Compound 2-2 (106.0 mg) was added to a 25 mL single-necked flask, dissolved in THF (3 mL), and 1 M tetrabutylammonium fluoride in THF (2 mL) was added. The mixture was reacted at room temperature for 4 hours. The reaction solution was concentrated under reduced pressure, and then separated and purified by column chromatography (DCM:MeOH = 10:1) to obtain 67.0 mg of a yellow solid, i.e., compound 2-3.

Step 4: Synthesis of compound 2-4

Compound 2-3 (67.0 mg) was added to a 25 mL single-necked flask, dissolved in THF (4 mL), and CDI (24.6 mg) was added. After the reaction was heated to 40°C for 2 hours, compound M15 (300.6 mg) was added. The reaction was heated to 80°C overnight. The reaction solution was concentrated under reduced pressure, and then separated and purified by column chromatography (DCM:MeOH = 10:1) to obtain 62.0 mg of a light yellow solid, i.e., compound 2-4.

Step 5: Synthesis of compound 2

Compound 2-4 (62.0 mg) was added to a 25 mL single-necked flask, dissolved in DCM (2 mL), and TFA (1 mL) was added. The mixture was reacted at room temperature for 1 hour. The reaction solution was concentrated under reduced pressure, and then separated by reversed phase column chromatography ($CH_3CN/H_2O$ = 30%-55%) to obtain 18.5 mg of a white solid, i.e., compound 2 (purity 96.48%).

LCMS: $1/2[M+2H]^+$= 621.93.

$^1H$ NMR (500 MHz, Methanol-$d_4$) δ 9.30 (s, 1H), 8.92 (s, 1H), 7.69 (dd, J = 8.9, 5.9 Hz, 1H), 7.43 (q, J = 8.3 Hz, 4H), 7.32 (d, J = 2.5 Hz, 1H), 7.26 (t, J = 9.3 Hz, 1H), 7.07 (t, J = 2.8 Hz, 1H), 5.00 (dd, J = 13.8, 6.9 Hz, 2H), 4.73 (dd, J = 12.3, 4.5 Hz, 1H), 4.68-4.60 (m, 3H), 4.57 - 4.23 (m, 7H), 3.87 (d, J = 10.9 Hz, 1H), 3.75 (dd, J = 10.9, 3.7 Hz, 1H), 3.70 - 3.53 (m, 3H), 3.50 - 3.36 (m, 6H), 3.24 - 3.13 (m, 1H), 2.49 (s, 3H), 2.45 - 2.30 (m, 3H), 2.27 - 1.92 (m, 14H), 1.90 - 1.75 (m, 3H), 1.66-1.56 (m, 3H), 1.54-1.47 (m, 4H), 1.31 (d, J = 10.8 Hz, 3H), 1.07 - 0.99 (s, 9H), 0.84 - 0.78 (m, 3H).

Example 67: Synthesis of compound (2S,4R)-1-(S)-2-(4-(1-(2-(7-(8-ethyl-7-fluoro-3-hydroxynaphthalen-1-yl)-8-fluoro-4-((R)-3-hydr oxy-3-methylpiperidin-1-yl)pyrido[4,3-d]pyrimidin-2-yl)oxy)ethyl)piperidin-4-yl)methoxy)phen yl)-1H-1,2,3-triazol-1-yl)-3-methylbutanoyl)-4-hydroxy-N-((S)-1-(4-methylthiazol-5-yl)phenyl) ethyl)pyrrolidine-2-carboxamide

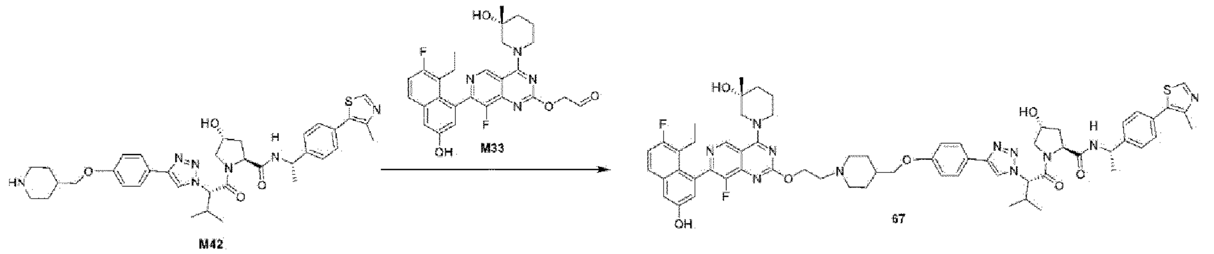


Step 1: Synthesis of compound 67

Compound M42 (80.00 mg), compound M33 (78.96 mg), and acetic acid (0.05 mL) were sequentially added to dichloromethane (2.00 mL) and isopropanol (0.50 mL). After the mixture was reacted at room temperature for 0.5 hours, 2-methylpyridine borane (38.22 mg) was added, followed by reacting at room temperature for 2 hours. Saturated aqueous sodium bicarbonate solution (30 mL) was added to the reaction solution. The solution was extracted three times with DCM, and the organic phases were combined and concentrated. The resultant was separated and purified by Pre-HPLC to obtain compound 67 (42.4 mg, 29.43%).

ESI-MS m/z: 583.27 $1/2[M+2H]^+$.

$^1H$ NMR (500 MHz, Methanol-$d_4$) δ 9.21 (d, J = 4.1 Hz, 1H), 8.87 (s, 1H), 8.35 (s, 1H), 7.73 (d, J = 8.6 Hz, 2H), 7.67 (dd, J = 8.8, 5.9 Hz, 1H), 7.47 - 7.36 (m, 4H), 7.30 (d, J = 2.4 Hz, 1H), 7.24 (t, J = 9.3 Hz, 1H), 7.06 (s, 1H), 6.97 (d, J = 8.6 Hz, 2H), 5.32 (d, J = 10.3 Hz, 1H), 5.04 (q, J = 6.9 Hz, 1H), 4.68 (t, J = 5.4 Hz, 2H), 4.53 (t, J = 8.3 Hz, 2H), 4.47 (s, 1H), 4.28 (t, J = 13.0 Hz, 1H), 3.93 - 3.84 (m, 4H), 3.64 (d, J = 13.3 Hz, 1H), 3.58 (d, J = 13.5 Hz, 1H), 3.45 (dd, J = 20.6, 9.8 Hz, 1H), 3.18 (d, J = 10.9 Hz, 2H), 2.93 (d, J = 4.8 Hz, 2H), 2.68 - 2.57 (m, 1H), 2.48 (s, 3H), 2.45 (s, 1H), 2.29 (t, J = 11.2 Hz, 2H), 2.24 - 2.13 (m,

3H), 2.03 (s, 1H), 2.01 - 1.94 (m, 1H), 1.87 (t, J = 14.3 Hz, 4H), 1.77 (t, J = 9.4 Hz, 2H), 1.51 (dd, J = 18.2, 9.6 Hz, 5H), 1.29 (d, J = 3.5 Hz, 3H), 1.27 (s, 1H), 1.15 (t, J = 7.7 Hz, 3H), 0.84 - 0.78 (m, 6H).

Example 69: Synthesis of compound (2S,4R)-1-((S)-2-(4-(4-(1-(1-(((7-(8-ethyl-7-fluoro-3-hydroxynaphthalen-1-yl)-8-fluoro-4-(((R)-3-hydroxy-3-methylpiperidin-1-yl)pyrido[4,3-d]pyrimidin-2-yl)oxy)methyl)cyclopropyl)methyl) piperidin-4-yl)methoxy)phenyl)-1H-1,2,3-triazol-1-yl)-3-methylbutanoyl)-4-hydroxy-N-(((S-1-( 4-(4-methylthiazol-5-yl)phenyl)ethyl) pyrrolidine-2-carboxamide

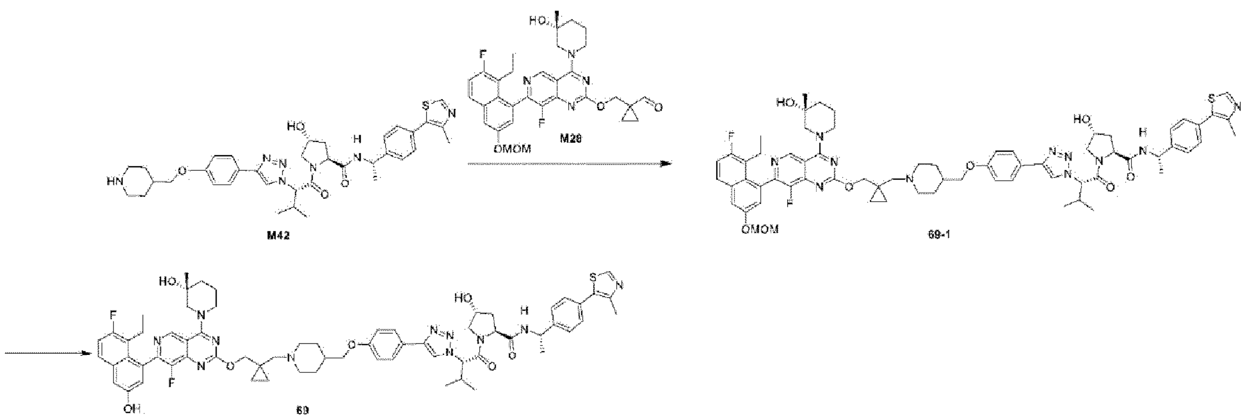
M42 M28 69-1 69

Step 1: Synthesis of compound 69-1

Compound M42 (62.35 mg, 0.09 mmol), intermediate compound M28 (50.00 mg, 0.08 mmol), and a THF solution of zinc chloride (0.08 mL, 1.00 mol/L, 0.08 mmol) were added to methanol (2.00 mL) and 1,2-dichloroethane (2.00 mL). The mixture was reacted at 40°C for 1 hour, and then sodium cyanoborohydride (53.04 mg, 0.84 mmol) was added, followed by reacting at 40°C for 3 hours. The reaction solution was concentrated, and 30 mL of water was added. The solution was extracted twice with DCM (80.00 mL), and dried with anhydrous sodium sulfate. The resultant was separated by column chromatography (DCM:MeOH = 90%:10%) to obtain compound 69-1 (80.00 mg, 75.92%).

ESI-MS m/z:625.35, 1/2$[M+2H]^+$.

Step 2: Synthesis of compound 69

Compound 69-1 (80.00 mg, 0.06 mmol) and TFA/trifluoroacetic acid (1.00 mL) were added to dichloromethane (2.00 mL). The mixture was reacted at room temperature for 1 hour. The reaction solution was concentrated, and then separated and purified by Pre-HPLC to obtain compound 69 (19.00 mg, 23.75%).

ESI-MS m/z: 603.18, 1/2$[M+2H]^+$.

$^1$H NMR (500 MHz, MeOD) δ 9.19 (d, J = 1.8 Hz, 1H), 8.86 (d, J = 7.8 Hz, 1H), 8.34 (d, J = 4.2 Hz, 1H), 7.72 (dd, J = 8.8, 1.4 Hz, 2H), 7.66 (dd, J = 9.0, 5.8 Hz, 1H), 7.46 - 7.37 (m, 4H), 7.29 (d, J = 2.5 Hz, 1H), 7.23 (t, J = 8.9 Hz, 1H), 7.06 (t, J = 2.6 Hz, 1H), 6.96 (d, J = 7.9 Hz, 2H), 5.32 (d, J = 10.3 Hz, 1H), 5.05 (q, J = 7.0 Hz, 1H), 4.55 - 4.42 (m, 5H), 4.24 (t, J = 14.2 Hz, 1H), 3.88 (ddd, J = 23.4, 11.5, 4.7 Hz, 4H), 3.62 (dd, J = 34.4, 13.3 Hz, 1H), 3.50 - 3.41 (m, 1H), 3.14 (dd, J = 20.3, 7.1 Hz, 2H), 2.67 - 2.58 (m, 1H), 2.54 - 2.37 (m, 7H), 2.24 - 2.13 (m, 3H), 2.04 - 1.94 (m, 4H), 1.89 - 1.72 (m, 7H), 1.53 (d, J = 7.0 Hz, 3H), 1.45 - 1.37 (m, 2H), 1.34 (s, 2H), 1.15 (dd, J = 10.8, 4.8 Hz, 4H), 0.84 - 0.78 (m, 7H), 0.72 (s, 2H), 0.51 (s, 2H).

Example 71: Synthesis of compound (2S,4R)-1-((S)-2-(4-(1-(7-(8-ethyl-7-fluoro-3-hydroxynaphthalen-1-yl)-8-fluoro-4-((R)-3-hydrox y-3-methylpiperidin-1-yl)pyrido[4,3-d]pyrimidin-2-yl)oxy)methyl)cyclopropyl)methyl)-7-azaspi ro[3.5] nonan-2-yl)-1H-1,2,3-triazol-1-yl)-3-methylbutanoyl)-4-hydroxy-N-((S)-1-(4-methylthiaz ol-5-yl)phenyl)ethyl)pyrrolidine-2-carboxamide

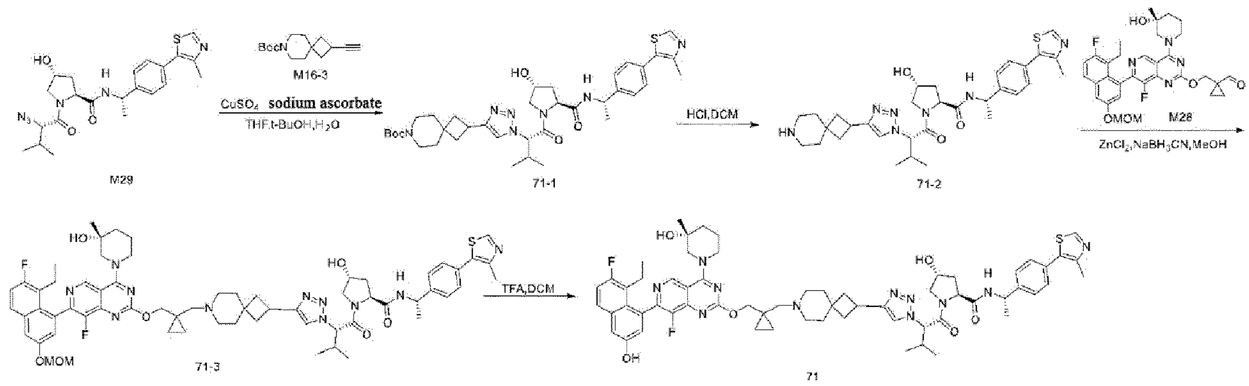


M29 71-1 71-2 71-3 71

Step 1: Synthesis of compound 71-1

Compound M29 (200.00 mg) and M16-3 (142.00 mg) were added to tetrahydrofuran (4.00 mL), tert-butanol (2.00 mL) and water (2.00 mL). Then copper sulfate (62.93 mg) and sodium ascorbate (225.67 mg) were added. The mixture was stirred at 20°C for 6 hours. The reaction was monitored by LCMS until completion. The resultant was concentrated under reduced pressure, and then purified by column chromatography to obtain a light yellow solid (291.00 mg, yield 94.10%), i.e., compound 71-1.

Step 2: Synthesis of compound 71-2

Compound 71-1 (291.00 mg) was added to a 25 mL single-necked flask, and dichloromethane (5.00 mL) and a solution of hydrogen chloride in dioxane (2.00 mL) were added. The mixture was stirred at 20°C for 0.5 hours. The reaction was monitored by LCMS until completion. The resultant was concentrated under reduced pressure, and then saturated $NaHCO_3$ solution was added until pH=8. The solution was extracted twice with dichloromethane/isopropanol = 5/1. The organic phase was washed with saturated brine and dried with anhydrous sodium sulfate. The crude product was used in the next step directly. A yellow solid (290.00 mg) was obtained, i.e., compound 71-2.

Step 3: Synthesis of compound 71-3

Compound M28 (80.00 mg) and 71-2 (98.14 mg) was added to a 25 mL single-necked flask, methanol (2.00 mL) was added, and then zinc chloride (0.20 mL, 1.00 mol/L) was added. The reaction was heated to 40°C and stirred for 2 hours. Finally, sodium cyanoborohydride (29.69 mg) was added, and the mixture was stirred at 40°C for 12 hours. The reaction was monitored by LCMS until completion. The resultant was concentrated under reduced pressure, and then purified by column chromatography to obtain a light yellow solid (130.00 mg, yield 81.44%), i.e., compound 71-3.

Step 4: Synthesis of compound 71

Compound 71-3 (130.00 mg) was added to a 25 mL single-necked flask, dichloromethane (4.00 mL) was added, and then TFA was added (2.00 mL). The mixture was stirred at 20°C for 0.5 hours. The reaction was monitored by LCMS until completion. The resultant was concentrated under reduced pressure, and then the crude product was purified by HPLC to obtain a pure product, which is a light yellow solid (50.00 mg, yield 37.47%), i.e., compound 71.

ESI-MS m/z: 570.23 1/2[M+2H]$^+$.

$^1$H NMR (500 MHz, Methanol-$d_4$) δ 9.11 (s, 1H), 8.77 (s, 1H), 7.81 (d, *J* = 8.4 Hz, 1H), 7.57 (t, *J* = 7.5 Hz, 1H), 7.33 (q, *J* = 8.1 Hz, 4H), 7.23 - 7.10 (m, 2H), 6.96 (s, 1H), 5.15 (d, *J* = 10.3 Hz, 1H), 4.94 (d, *J* = 6.9 Hz, 1H), 4.52 - 4.29 (m, 6H), 4.16 (t, *J* = 13.9 Hz, 1H), 3.83 - 3.68 (m, 2H), 3.59 - 3.43 (m, 2H), 3.35 (q, *J* = 11.7, 11.3 Hz, 1H), 2.55 (s, 3H), 2.45 (s, 1H), 2.38 (s, 4H), 2.18 (d, *J* = 10.9 Hz, 2H), 2.09 (s, 2H), 1.88 (d, *J* = 12.2 Hz, 3H), 1.77 - 1.73 (m, 2H), 1.71 - 1.66 (m, 2H), 1.61 (s, 2H), 1.42 (d, *J* = 7.0 Hz, 3H), 1.28 (d, *J* = 23.5 Hz, 2H), 1.02 (d, *J* = 6.8 Hz, 3H), 0.82 - 0.75 (m, 5H), 0.70 (t, *J* = 9.4 Hz, 5H), 0.65 (d, *J* = 6.7 Hz, 3H), 0.49 (s, 2H).

Example 73: Synthesis of compound (2S,4R)-1-((S)-2-(4-(7-((1-((7-(8-ethyl-7-fluoro-3-hydroxynaphthalen-1-yl)-8-fluoro-4-(((R)-3-h ydroxy-3-methylpiperidin-1-yl)pyrido[4,3-d]pyrimidin-2-yl)oxy)methyl)cyclopropyl)methyl)-7-azaspiro [3.5]nonan-2-yl)-1H-1,2,3-triazol-1-yl)-3-methylbutanoyl)-4-hydroxy-N-((S)-1-(4-(4-me thylthiazol-5-yl)phenyl)ethyl) pyrrolidine-2-carboxamide

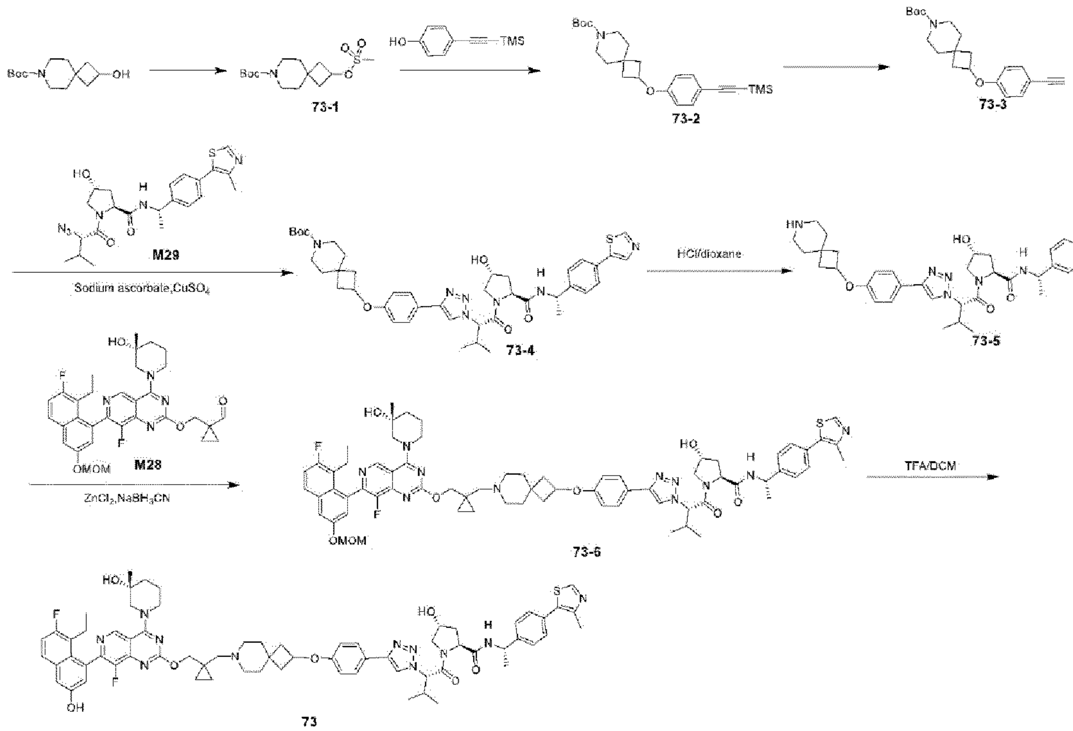


Step 1: Synthesis of compound 73-1

7-Tert-butoxycarbonyl-7-azaspiro[3.5]-2-nonanol (500 mg) was dissolved in 6 mL of DCM, triethylamine (0.58 mL) was added, and methanesulfonyl chloride (0.19 mL) was added in an ice bath. After returning to room temperature and stirring for 2 hours, the reaction was quenched with water in an ice bath. The solution was extracted once with EA and extracted once with DCM. The organic phases were collected, combined, and dried with anhydrous sodium sulfate, and concentrated to obtain crude product 73-1 (660 mg). ESI-MS m/z: 320.1 $[M+H]^+$.

Step 2: Synthesis of compound 73-2

Compound 73-1 (660 mg) was added to a 50 mL single-necked flask, 10 mL of DMF was added. 4-((Trimethylsilyl)ethynyl)phenol (511 mg), and cesium carbonate (2.01 g) were added. The mixture was reacted at 60°C for 3 hours. 50 mL of water was added dropwise to the reaction solution, followed by filtration, and the filter cake was washed with water. The filter cake was dissolved in DCM, washed with saturated brine, and the organic phases were collected, combined, dried with anhydrous sodium sulfate, and concentrated. The concentrate was purified by column chromatography to obtain compound 73-2 (330 mg). ESI-MS m/z: 413.3 $[M+H]^+$.

Step 3: Synthesis of compound 73-3

Compound 73-2 (330 mg) was added to a 10 mL single-necked flask, 3 mL of DMF was added, and cesium fluoride (500 mg) was added. The mixture was stirred at room temperature for 3 hours. 20 mL of water was added dropwise to the reaction solution, followed by filtration, and the filter cake was washed with water and dried to obtain compound 73-3 (300 mg). ESI-MS m/z: 342.2 $[M+H]^+$.

Step 4: Synthesis of compound 73-4

Compound 73-3 (300 mg) was added to a 50 mL single-necked flask, 3 mL of THF, 3 mL of tert-butanol and 3 mL of water were added. Then, M29 (269 mg), copper sulfate (95 mg), and sodium ascorbate (338 mg) were added. The mixture was reacted at room temperature for 30 minutes. The reaction solution was diluted with water, and the solution was extracted twice with EA. The organic phases were collected, combined, dried with anhydrous sodium sulfate, and the concentrate was separated and purified by column chromatography to obtain compound 73-4 (280 mg). ESI-MS m/z: 798.4 $[M+H]^+$.

Step 5: Synthesis of compound 73-5

Compound 73-4 (280 mg) was added to a 10 mL single-necked flask, and 4 M hydrogen chloride in dioxane (3 mL) was added. The mixture was reacted at room temperature for 30 minutes. The reaction solution was neutralized with saturated sodium bicarbonate solution, and the solution was extracted twice with EA. The organic phases were collected, combined, dried with anhydrous sodium sulfate, and concentrated to obtain crude product compound 73-5 (220 mg). ESI-MS m/z: 698.4 $[M+H]^+$.

Step 6: Synthesis of compound 73-6

M28 (80 mg) was added to a 10 mL single-necked flask, 1 mL of methanol was added, and compound 73-5 (94 mg) was added. Then, 1 M zinc chloride in tetrahydrofuran (0.2 mL) was added, and the mixture was stirred at room temperature for 3 hours. Sodium cyanoborohydride (25 mg) was added, and the mixture was stirred at room temperature for 16 hours. The reaction solution was quenched with saturated ammonium chloride solution, and the solution was extracted twice with EA. The organic phases were collected, combined, dried with anhydrous sodium sulfate, and concentrated to obtain crude product compound 73-6 (100 mg). ESI-MS m/z: 638.7 1/2$[M+2H]^+$.

Step 7: Synthesis of compound 73

Compound 73-6 (100 mg) was added to a 10 mL single-necked flask, 2 mL of DCM was added, and 2 mL of TFA was added. The mixture was reacted at room temperature for 1 hour. After rotary evaporation, the resultant was purified by Pre-HPLC to obtain compound 73 (51.4 mg).

ESI-MS m/z: 616.4 1/2$[M+2H]^+$.

$^1$H NMR (500 MHz, DMSO-$d_6$) δ 9.21 (s, 1H), 8.99 (s, 1H), 8.57 (s, 1H), 8.53 - 8.48 (m, 1H), 7.80 - 7.72 (m, 3H), 7.45 (d, J = 8.4 Hz, 2H), 7.37 (d, J = 8.2 Hz, 2H), 7.32 (d, J = 2.8 Hz, 1H), 7.03 (d, J = 2.5 Hz, 1H), 6.88 (d, J = 8.8 Hz, 2H), 5.31 (t, J = 8.3 Hz, 1H), 5.22 - 5.10 (m, 1H), 4.93 (t, J = 7.2 Hz, 1H), 4.74 (t, J = 6.7 Hz, 2H), 4.40 (t, J = 8.1 Hz, 1H), 4.36 - 4.23 (m, 4H), 4.04 (dd, J = 27.4, 13.5 Hz, 1H), 3.81 - 3.69 (m, 1H), 3.70 - 3.49 (m, 1H), 2.45 (d, J = 3.9 Hz, 3H), 2.40-2.21 (m, 9H), 2.19 - 1.93 (m, 5H), 1.81 - 1.64 (m, 6H), 1.59 - 1.47 (m, 5H), 1.38 (d, J = 7.0 Hz, 3H), 1.17 (d, J = 12.1 Hz, 4H), 1.08 (d, J = 6.5 Hz, 3H), 0.85 (t, J = 6.9 Hz, 1H), 0.76 - 0.70 (m, 6H), 0.64 (s, 2H), 0.40 (s, 2H).

Example 75: Synthesis of compound (2S,4R)-1-((S)-2-(4-(4-(7-(7-(8-ethyl-7-fluoro-3-hydroxynaphthalen-1-yl)-8-fluoro-4-((R)-3-hyd roxy-3-methylpiperidin-1-yl)pyrido[4,3-d]pyrimidin-2-yl)oxy)methyl)cyclopropyl)methyl)-2,7-d iazaspiro [3.5]nonan-2-yl)phenyl)-1H-1,2,3-triazol-1-yl)-3-methylbutanoyl)-4-hydroxy-N-((S)-1-(4-(4-methylthiazol-5-yl)phenyl) ethyl)pyrrolidine-2-carboxamide

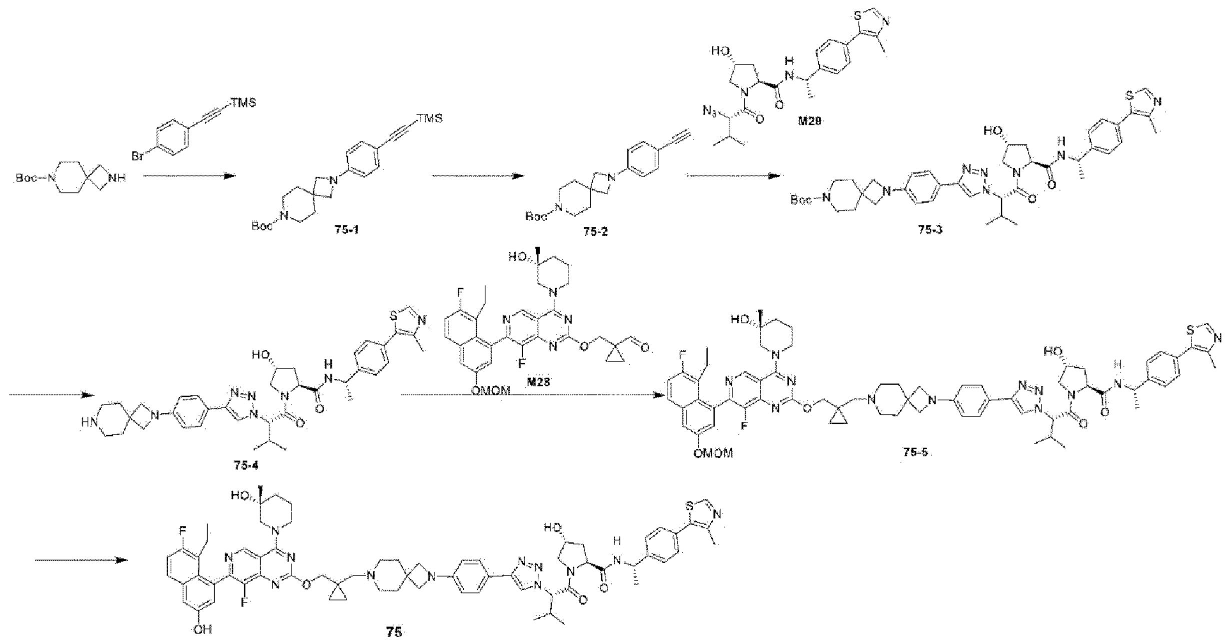

M29 75-1 75-2 75-3 75-4 M28 75-5 75

Step 1: Synthesis of compound 75-1

Tert-butyl 2,7-diazaspiro[3.5]nonane-7-carboxylate (3.00 g), ((4-bromophenyl)ethynyl)trimethylsilane (5.03 g), tris(dibenzylideneacetone)dipalladium (2.43 g), 1.1'-binaphthyl-2.2'-diphemyl phosphine (3.30 g), and cesium carbonate (12.96 g) were added to toluene (30.00 mL), and then the reaction system was purged with nitrogen. The mixture was reacted at 90°C for 7 hours. The reaction solution was filtrated, and the filter cake was washed with DCM. The filtrate was concentrated, and separated by column chromatography (PE:EA = 93%:7%) to obtain compound 75-1 (1.40 g, 26.50%).

ESI-MS m/z: 398.28 $[M+H]^+$.

Step 2: Synthesis of compound 75-2

Compound 75-1 (1400.00 mg) and potassium carbonate (1456.26 mg) were added to methanol (20.00 mL). The mixture was reacted at room temperature for 16 hours. The reaction solution was concentrated, diluted with DCM and filtered. Then, the filter cake was washed with DCM. The filtrate was concentrated, and separated by column chromatography (PE:EA = 90%:10%) to obtain compound 75-2 (650.00 mg, 56.69%).

ESI-MS m/z: 327.25 $[M+H]^+$.

Step 3: Synthesis of compound 75-3

Compound M29 (200.00 mg), compound 75-2 (286.02 mg), sodium ascorbate (216.98 mg), and copper sulfate (69.93 mg) were added to tetrahydrofuran (2.00 mL), water (2.00 mL), and tert-butanol (2.00 mL). The mixture was reacted at room temperature for 3 hours. 50 mL of water was added to the reaction solution. The solution was extracted twice with DCM, and dried with anhydrous sodium sulfate. The resultant was separated by column chromatography (DCM:MeOH = 95%:5%) to obtain compound 75-3 (230 mg, 67.05%).

ESI-MS m/z: 783.59 $[M+H]^+$.

Step 4: Synthesis of compound 75-4

Compound 75-3 (230.00 mg) and trifluoroacetic acid (1.00 mL) were sequentially added to dichloromethane (2.00 mL). The mixture was reacted at room temperature for 1 hour. 40 mL of saturated sodium bicarbonate solution was added to the reaction solution to adjust to alkaline. The solution was extracted twice with DCM, and dried with anhydrous sodium sulfate. The resultant was separated by column chromatography (DCM:MeOH = 90%:10%) to obtain compound 75-4 (129.00 mg, 64.32%).

ESI-MS m/z: 683.49 $[M+H]^+$.

Step 5: Synthesis of compound 75-5

Compound 75-4 (120.00 mg), compound M28 (104.13 mg), and 1.0 M zinc chloride in tetrahydrofuran (0.35 mL) were added to methanol (3.00 mL) and 1,2-dichloroethane (3.00 mL). The mixture was reacted at 40°C for 2 hours, and then sodium cyanoborohydride (55.20 mg) was added, followed by reacting at 40°C for 3 hours. The reaction solution was concentrated, and 30 mL of water was added. The solution was extracted twice with DCM, and dried with anhydrous sodium sulfate. The resultant was separated by column chromatography (DCM:MeOH = 90%:10%) to obtain compound 75-5 (110.00 mg, 49.71%).
ESI-MS m/z: 630.71 1/2[M+2H]$^+$.

Step 6: Synthesis of compound 75

Compound 75-5 (110.00 mg) and trifluoroacetic acid (1.50 mL) were added to dichloromethane (3.00 mL). The mixture was reacted at room temperature for 1 hour. The reaction solution was concentrated, and then separated and purified by Pre-HPLC to obtain compound 75 (26.10 mg, 23.43%).
ESI-MS m/z: 608.68 1/2[M+2H]$^+$.
$^1$H NMR (500 MHz, Methanol-$d_4$) $\delta$ 9.21 (d, *J* = 5.5 Hz, 1H), 8.87 (d, *J* = 6.9 Hz, 1H), 8.25 (s, 1H), 7.67 (dd, *J* = 9.0, 5.8 Hz, 1H), 7.62 (d, *J* = 8.5 Hz, 2H), 7.49 - 7.35 (m, 4H), 7.30 (d, *J* = 2.6 Hz, 1H), 7.24 (t, *J* = 9.3 Hz, 1H), 7.06 (t, *J* = 2.4 Hz, 1H), 6.51 (d, *J* = 8.7 Hz, 2H), 5.30 (d, *J* = 10.4 Hz, 1H), 5.05 (q, *J* = 7.0 Hz, 1H), 4.56 - 4.40 (m, 5H), 4.25 (t, *J* = 14.4 Hz, 1H), 3.92 (dd, *J* = 11.0, 3.9 Hz, 1H), 3.85 (d, *J* = 11.1 Hz, 1H), 3.69 - 3.52 (m, 5H), 3.51 - 3.41 (m, 1H), 2.67 - 2.36 (m, 11H), 2.25 - 2.13 (m, 3H), 2.10 - 1.94 (m, 2H), 1.89 - 1.74 (m, 7H), 1.53 (d, *J* = 7.0 Hz, 2H), 1.35 - 1.24 (m, 8H), 1.14 (dd, *J* = 15.1, 6.5 Hz, 3H), 0.83 - 0.79 (m, 5H), 0.73 (s, 2H), 0.52 (s, 2H).

Example 79: Synthesis of compound (2S,4R)-1-((R)-2-(3-(2-(1-(2-((7-(8-ethyl-7-fluoro-3-hydroxynaphthalen-1-yl)-8-fluoro-4-((R)-3-hydroxy-3-methylpiperidin-1-yl)pyrido[4,3-d]pyrimidin-2-yl)oxy)ethyl)piperidin-4-yl)ethoxy)is oxazol-5-yl)-3-methylbutanoyl)-4-hydroxy-N-(((S)-1-(4-(4-methylthiazol-5-yl)phenyl)ethyl)pyrr olidine-2-carboxamide

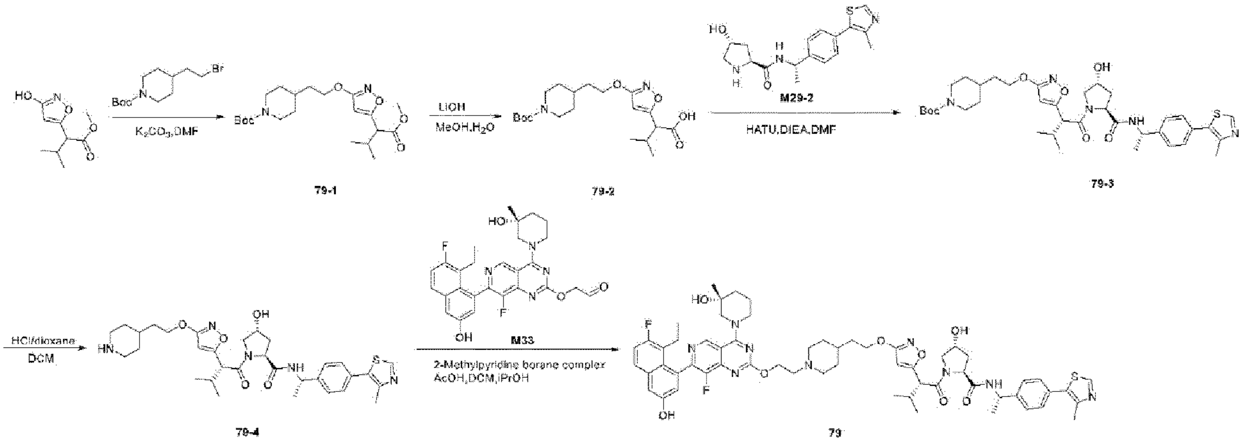


79-1 79-2 79-3 79-4 79

Step 1: Synthesis of compound 79-1

Methyl 2-(3-hydroxyisoxazol-5-yl)-3-methylbutyrate (500 mg), tert-butyl 4-(2-bromoethyl)piperidine-1-carboxylate (1.03 g), and potassium carbonate (1.04 g) were dissolved in N,N-dimethylformamide (10 mL), and the mixture was reacted at 50°C for 2 hours. After the reaction was completed, the resultant was diluted with water and extracted with ethyl acetate. The organic phase was washed three times with saturated brine and purified by column chromatography (petroleum ether/ethyl acetate) to obtain compound 79-1 (920 mg). ESI-MS m/z: 433.22 [M+Na]$^+$.

Step 2: Synthesis of compound 79-2

Compound 79-1 (920 mg) was dissolved in methanol (10 mL) and water (2 mL), and lithium hydroxide (268 mg) was added. The mixture was reacted at 25°C for 1 hour. After the reaction was completed, citric acid was added until pH = 4. The solution was extracted three times with dichloromethane/isopropanol (10:1), dried with anhydrous sodium sulfate,

filtered, and concentrated to obtain compound 79-2 (883 mg). ESI-MS m/z: 297.31 $[M+H-100]^+$.

Step 3: Synthesis of compound 79-3

Compound 79-2 (883 mg), M29-2 (775 mg), HATU (1.27 g), and N,N-diisopropylethylamine (1.1 mL) were dissolved in N,N-dimethylformamide (10 mL). The mixture was reacted at 25°C for 1 hour. After the reaction was completed, the resultant was diluted with water and extracted with ethyl acetate. The organic phase was washed with saturated brine, and the resultant was purified by column chromatography (dichloromethane/methanol) and separated by chiral column to obtain compound 79-3 (263 mg). ESI-MS m/z: 710.53 $[M+H]^+$.

Step 4: Synthesis of compound 79-4

Compound 79-3 (263 mg) was dissolved in dichloromethane (5 mL), and 4 M hydrogen chloride in dioxane (1 mL) was added. The mixture was reacted at 25°C for 20 minutes. After the reaction was completed, the resultant was dissolved in water, neutralized with sodium bicarbonate solution, extracted with dichloromethane/isopropanol (10:1), dried, and concentrated to obtain compound 79-4 (203 mg). ESI-MS m/z: 610.41 $[M+H]^+$.

Step 5: Synthesis of compound 79

Compound 79-4 (100 mg), M33 (83 mg), and glacial acetic acid (49 mg) were dissolved in dichloromethane (5 mL) and isopropanol (0.5 mL). The mixture was reacted at 25°C for 20 minutes. Then, 2-methylpyridine-N-borane (87 mg) was added, and the mixture was reacted at 25°C for 1 hour. After the reaction was completed, the reaction was quenched with sodium bicarbonate solution, and the resultant was extracted with dichloromethane/isopropanol (10:1), concentrated and purified by column chromatography (dichloromethane/methanol) and Pre-TLC to obtain compound 79 (32.5 mg).

ESI-MS m/z: 552.18 1/2$[M+2H]^+$.
$^1$H NMR (500 MHz, DMSO-$d_6$) δ 10.00 (s, 1H), 9.26 (s, 1H), 8.99 (d, *J* = 2.7 Hz, 1H), 8.43 (d, *J* = 7.4 Hz, 1H), 7.77 (dd, *J* = 9.1, 6.0 Hz, 1H), 7.48 - 7.41 (m, 2H), 7.39 - 7.32 (m, 4H), 7.29 (s, 1H), 7.05 (s, 1H), 6.68 (s, 1H), 6.07 (s, 1H), 5.15 - 5.13 (m, 1H), 4.91 (p, *J* = 7.2 Hz, 1H), 4.82 - 4.65 (m, 3H), 4.43 - 4.31 (m, 2H), 4.30 - 4.25 (m, 1H), 4.21 - 4.17 (m, 2H), 4.10 (dd, *J* = 25.0, 13.4 Hz, 1H), 3.70 (dd, *J* = 10.6, 4.4 Hz, 1H), 3.64 (d, *J* = 10.0 Hz, 2H), 3.58 - 3.50 (m, 2H), 3.49 - 3.39 (m, 2H), 3.10 - 2.95 (m, 2H), 2.46 (s, 3H), 2.39 - 2.29 (m, 1H), 2.28 - 2.08 (m, 3H), 2.05 - 1.97 (m, 3H), 1.92 - 1.85 (m, 2H), 1.81 - 1.77 (m, 1H), 1.76 (s, 3H), 1.72 - 1.63 (m, 6H), 1.45 (d, *J* = 7.0 Hz, 1H), 1.37 (d, *J* = 7.0 Hz, 3H), 1.17 (d, *J* = 11.6 Hz, 3H), 0.95 (d, *J* = 6.5 Hz, 3H), 0.86 - 0.80 (m, 1H), 0.79 (d, *J* = 6.7 Hz, 2H), 0.74 (q, *J* = 7.7 Hz, 3H).

Example 80: Synthesis of compound (2S,4R)-1-(R)-2-(3-(1-(7-(8-ethyl-7-fluoro-3-hydroxynaphthalen-1-yl)-8-fluoro-4-((R)-3-hydrox y-3-methylpiperidin-1-yl)pyrido[4,3-d]pyrimidin-2-yl)oxy)ethyl)piperidin-4-yl)methoxy)isoxazo 1-5-yl)-3-methylbutanoyl)-4-hydroxy-N-((S)-1-(4-(4-methylthiazol-5-yl)phenyl)ethyl)pyrrolidine -2-carboxamide

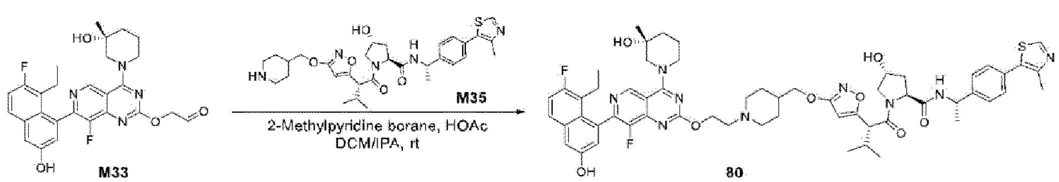


Intermediate M33 (30 mg), intermediate M35 (35.15 mg), dichloromethane (1.00 mL), isopropanol (0.10 mL), and acetic acid (0.02 mL) were added to a reaction flask. After reacting at room temperature for 10 minutes, dimethylpyridineborane (31.27 mg) was added, and the mixture was reacted at room temperature for 1 hour. Sodium carbonate solution was added until pH = 8. The solution was extracted with DCM, dried and concentrated. The resultant was separated by Pre-HPLC to obtain compound 80 (12 mg). ESI-MS m/z: 545.19 1/2$[M+2H]^+$.
$^1$H NMR (500 MHz, Methanol-$d_4$) δ 8.99 (t, *J* = 75.2 Hz, 1H), 8.66 (s, 1H), 7.92 (d, *J* = 6.7 Hz, 1H), 7.61 - 7.46 (m, 1H), 7.35 (s, 4H), 7.28 (s, 1H), 7.18 (m, 2H), 5.73 (d, *J* = 8.0 Hz, 1H), 5.05 (td, *J* = 7.1, 2.3 Hz, 1H), 4.76 - 4.37 (m, 5H), 4.35 - 4.18 (m, 2H), 3.99 (m, 3H), 3.65 (d, *J* = 8.8 Hz, 1H), 3.45 - 3.34 (m, 2H), 3.28 - 3.05 (m, 5H), 2.85 (s, 2H), 2.49 (s, 3H), 2.38 - 2.07 (m, 8H), 2.01 (d, *J* = 4.5 Hz, 5H), 1.70 (d, *J* = 12.7 Hz, 4H), 1.52 - 1.46 (m, 4H), 0.99 (d, *J* = 6.3 Hz, 3H), 0.87 - 0.74 (m, 6H).

Example 88: Synthesis of compound ((3R,7aR)-7a-(((7-(8-ethyl-7-fluoro-3-hydroxynaphthalen-1-yl)-8-fluoro-4-((R)-3-hydroxy-3-me thylpiperidin-1-yl)pyrido[4,3-d]pyrimidin-2-yl)oxy)methyl)hexahydro-1H-pyrrolizin-3-yl)methy l 2-(((S)-1-((2S,4R)-4-hydroxy-2-((((S)-1-(4-(4-methylthiazol-5-yl)phenyl)ethyl)carbamoyl)pyrrol idin-1-yl)-3,3-dimethyl-1-oxobutan-2-yl)carbamoyl)-7-azaspiro[3.5]nonane-7-carboxylate

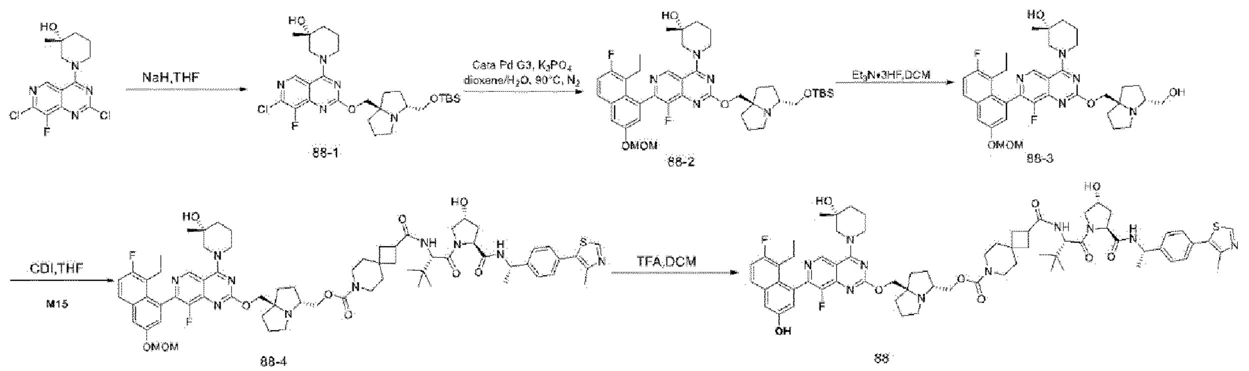


Step 1: Synthesis of compound 88-1

(R)-1-(2,7-dichloro-8-fluoropyrido[4,3-d]pyrimidin-4-yl)-3-methylpiperidin-3-ol (300 mg) was dissolved in 2mL of THF under the protection of nitrogen. NaH (72 mg) was added, then ((3R,7aR)-3-((tert-butyldimethylsilyl)oxy)methyl) tetrahydro-1H-pyrrolizin-7a(5H)-yl)methanol (259 mg) in THF (1 mL) was added. The mixture was stirred at 25°C for 2 hours. The reaction was monitored by LC-MS until completion. The reaction solution was quenched by slowly adding 10 mL of saturated ammonium chloride solution. The solution was extracted three times with EA, and the organic phases were combined, dried with anhydrous sodium sulfate. The resultant was concentrated under reduced pressure, and then separated and purified by column chromatography to obtain 315 mg of a light yellow solid, i.e., compound 88-1.

ESI-MS m/z: 580 $[M+H]^+$;

Step 2: Synthesis of compound 88-2

Compound 88-1 (315 mg), 2-(8-ethyl-7-fluoro-3-(methoxymethoxy)naphthalen-1-yl)-4,4,5,5-tetramethyl-1,3,2-dioxaborola ne (187 mg), potassium phosphate (340 mg), CataCXium A Pd G3 (39 mg) were dissolved in 5 mL of dioxane and 1 mL of water, and then the reaction system was purged with nitrogen. The mixture was reacted at 90°C for 3 hours. The reaction was monitored by LC-MS until completion. 20.0 mL of water was added to reaction solution, and the solution was extracted three times with EA and 30 mL for each time, the organic phases were combined, washed with saturated brine, dried with anhydrous sodium sulfate, filtered and concentrated. The resultant was separated and purified by column chromatography to obtain 146 mg of a light yellow solid, i.e., compound 88-2. ESI-MS m/z: 778 $[M+H]^+$.

Step 3: Synthesis of compound 88-3

Compound 88-2 (146 mg) was dissolved in 3 mL of DCM, then 1.5 mL of triethylamine trihydrofluoride was added. The mixture was reacted at room temperature for 2 hours. The reaction was monitored by LCMS until completion. 10.0 mL of water was added to reaction solution, and the solution was extracted with a extract of DCM:MeOH = 10:1. The organic phases were combined, washed with saturated brine, dried with sodium sulfate, filtered and concentrated. The resultant was separated and purified by column chromatography to obtain 80 mg of a light yellow solid, i.e., compound 88-3.

Step 4: Synthesis of compound 88-4

Compound 88-3 (43 mg) was dissolved in 3 mL of THF, then 14 mg of CDI was added, the mixture was reacted at 40°C for 1 hour, the intermediate was monitored by LC-MS. Then, compound M15 (58 mg) was added, and the mixture was stirred at 80°C for 12 hours. The reaction was monitored by LC-MS until completion. The reaction solution was quenched by adding 5.00 mL of methanol. The organic phase was concentrated, and then separated and purified by column chromatography to obtain 29 mg of a light yellow solid, i.e., compound 88-4.

ESI-MS m/z: 643 1/2$[M+2H]^+$.

Step 5: Synthesis of compound 88

Compound 88-4 (29 mg) was dissolved in 3 mL of DCM, then 1 mL of TFA was added. The mixture was reacted at room temperature for 30 minutes. The reaction was monitored by LCMS until completion. The reaction was monitored by LCMS until completion. The reaction solution was concentrated directly, and the resultant was separated by preparative liquid chromatography to obtain 13.6 mg of a light yellow powder, i.e., compound 88.

ESI-MS m/z: 621 1/2[M+2H]$^+$.

$^1$H NMR (500 MHz, Methanol-$d_4$) δ 9.29 (s, 1H), 8.92 (s, 1H), 7.70-7.67 (m, 1H),7.43 (q, *J* = 8.2 Hz, 4H), 7.32 - 7.24 (m, 2H), 7.06-7.05 (m, 1H), 5.03 - 4.98 (m, 1H), 4.73 (d, *J* = 12.2 Hz, 1H), 4.67-4.60 (m, 3H), 4.57 - 4.48 (m, 2H), 4.43(s, 1H), 4.39-4.28 (m, 3H), 3.87 (d, *J* = 10.9 Hz, 1H), 3.76-3.73 (m,1H), 3.67 - 3.53 (m, 2H), 3.45 - 3.40 (m, 3H), 3.17-3.16 (m, 1H), 2.48-2.35 (m, 6H), 2.24-2.17 (m, 5H), 2.14 - 2.01 (m, 7H), 1.99 - 1.86 (m, 4H), 1.82-1.80 (m, 3H), 1.61(s, 3H), 1.51-1.49(m, 5H), 1.33-1.29 (m, 7H), 1.02 (s, 9H), 0.84-0.78 (m, 3H).

Example 89: Synthesis of compound 5-(7-(8-ethynyl-7-fluoro-3-hydroxynaphthalen-1-yl)-8-fluoro-2-(1-(4-(4-((S)-1-((2S,4R)-4-hydro xy-2-(((S)-1-(4-(4-methylthiazol-5-yl)phenyl)ethyl)carbamoyl)pyrrolidin-1-yl)-3,3-dimethyl-1-o xobutan-2-yl)carbamoyl)phenoxy)methyl)piperidin-1-yl)methylcyclopropyl)methoxy)pyrido[4,3 -d] pyrimidin-4-yl)-N,N-dimethyl-5,6,7,8-tetrahydro-4H-pyrazolo[1,5-a][1,4]diazepine-2-carbox amide

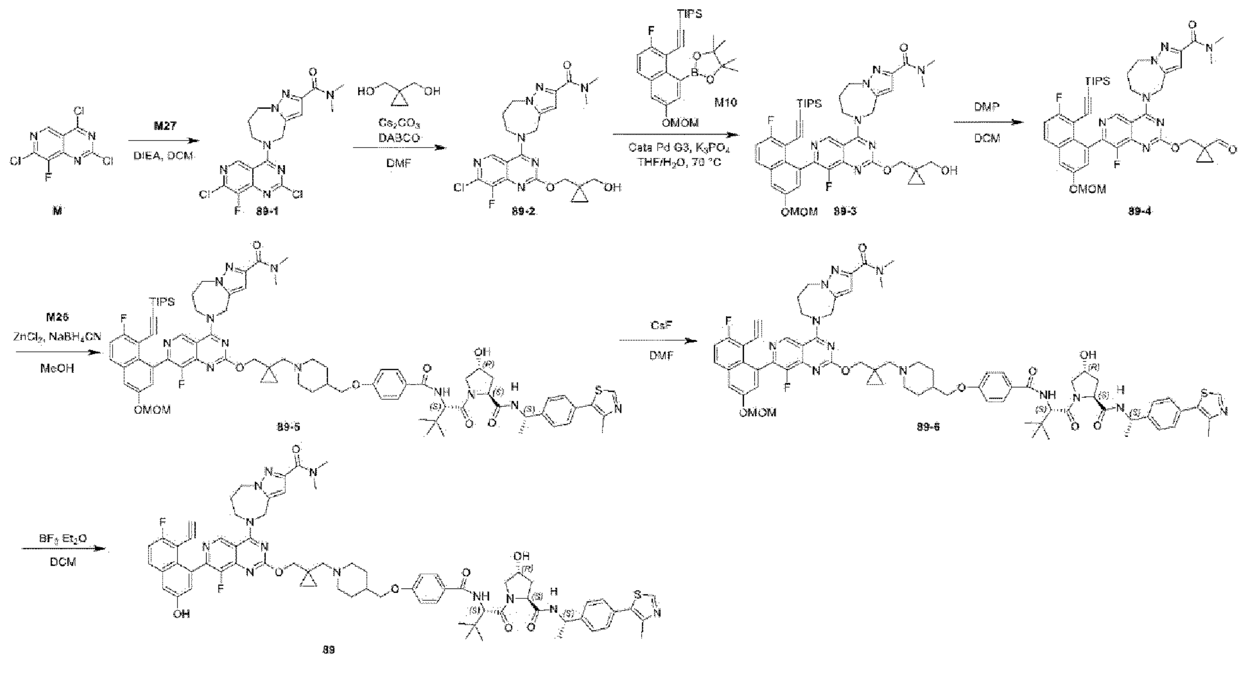


Step 1: Synthesis of compound 89-1

DIPEA (0.83 mL) and 2,4,7-trichloro-8-fluoropyrido[4,3-d]pyrimidine (255 mg) were added to a solution of intermediate compound M27 (270 mg) in DCM (5 mL) at -40°C. The reaction was stirred at this temperature for 1 hour. After the reaction was completed, the reaction system was concentrated, and the concentrate was purified by column chromatography (0-4% MeOH/DCM) to obtain compound 89-1 (416 mg).

ESI-MS m/z: 424.3 [M+H]$^+$.

Step 2: Synthesis of compound 89-2

1,1-Cyclopropane dimethanol (150 mg), $Cs_2CO_3$ (639 mg), and DABCO (22 mg) were added to a solution of compound 89-1 (416 mg) in DMF (5 mL) at room temperature. The reaction was stirred at this temperature for 12 hours. After the reaction was completed, EA and water were added to the reaction system, then the aqueous phase was extracted twice with EA. The organic phases were combined and washed three times with saturated brine, followed by drying with anhydrous sodium sulfate, and concentrated. The concentrate was purified by column chromatography (0-6% MeOH/DCM) to obtain compound 89-2 (220 mg).

ESI-MS m/z: 490.3 $[M+H]^+$.

Step 3: Synthesis of compound 89-3

Compound 89-2 (220 mg), compound M10 ((2-fluoro-6-(methoxymethoxy)-8-(4,4,5,5-tetramethyl-1,3,2-dioxyborolan-2-yl)naphthalen-1-yl) ethynyl)triisopropylsilane (335 mg), CataCXium A Pd G3 (33 mg), and $K_3PO_4$ (286 mg) were added to a 50 mL single-necked flask. THF (5 mL) and $H_2O$ (1 mL) were added, and then the reaction system was purged with nitrogen, the mixture was stirred at 70°C for 1 hour. After the reaction was completed, EA and saturated brine were added to the reaction system, then the aqueous phase was extracted once with EA. The organic phases were combined and dried with anhydrous sodium sulfate, and then concentrated. The concentrate was purified by preparative thin-layer chromatography (8% MeOH/DCM) to obtain compound 89-3 (196 mg).
ESI-MS m/z: 840.7 $[M+H]^+$.

Step 4: Synthesis of compound 89-4

DMP (148 mg) was added to a solution of compound 89-3 (196 mg) in DCM (5 mL) at room temperature. The reaction was stirred at this temperature for 3 hours. After the reaction was completed, the reaction was quenched by adding saturated sodium sulfite solution to the reaction system. Then, the solution was extracted with an appropriate amount of DCM, and then separated. The aqueous phase was extracted twice with DCM. The organic phases were combined and washed once with saturated brine, followed by drying with anhydrous sodium sulfate, and concentrated. The concentrate was purified by column chromatography (0-5% MeOH/DCM) to obtain compound 89-4 (180 mg).
ESI-MS m/z: 838.7 $[M+H]^+$.

Step 5: Synthesis of compound 89-5

$ZnCl_2$ (0.2 mL) was added to a solution of compound 89-4 (180 mg) and compound M26 (156 mg) in MeOH (5 mL) at room temperature. The system was stirred at room temperature for 1 hour. Then, $NaBH_3CN$ (67 mg) was added to the system. The reaction was stirred at 40°C overnight. After the reaction was completed, the reaction was quenched by adding saturated sodium bicarbonate solution to the reaction system. Then, the solution was extracted with a mixed solvent (DCM/MeOH = 9:1) and then separated. The aqueous phase was extracted twice with the mixed solvent. The organic phases were combined and dried with anhydrous sodium sulfate, and concentrated. The concentrate was purified by preparative thin-layer chromatography (10% MeOH/DCM) to obtain compound 89-5 (23 mg).
ESI-MS m/z: 742.5 1/2$[M+2H]^+$.

Step 6: Synthesis of compound 89-6

CsF (47 mg) was added to a solution of compound 89-5 (23 mg) in DCM (1 mL) at room temperature. The reaction was stirred at this temperature for 3 hours. After the reaction was completed, EA and saturated brine were added to the reaction system for extraction and phase separation, then the aqueous phase was washed three times with saturated brine. The organic phases were dried with anhydrous sodium sulfate, and concentrated. The concentrate was purified by preparative thin-layer chromatography (11% MeOH/DCM) to obtain compound 89-6 (17 mg).
ESI-MS m/z: 664.3 1/2$[M+2H]^+$.

Step 7: Synthesis of compound 89

Boron trifluoride etherate (0.4 mL) was added to a solution of compound 89-6 (17 mg) in DCM (2 mL) at room temperature. The reaction was stirred at this temperature for 1 hour. After the reaction was completed, the reaction system was poured into cooled saturated sodium carbonate solution, and then extracted three times with a mixed solvent (DCM/MeOH = 9:1). The organic phases were combined and dried with anhydrous sodium sulfate, and concentrated. The concentrate was purified by preparative thin-layer chromatography (12% MeOH/DCM) to obtain compound 89 (7 mg).
ESI-MS m/z: 642.8 1/2$[M+2H]^+$.
$^1H$ NMR (500 MHz, Methanol-$d_4$) δ 9.12 (s, 1H), 8.93 - 8.83 (m, 1H), 7.85 (dd, *J* = 9.1, 5.7 Hz, 1H), 7.79 - 7.75 (m, 2H), 7.46 - 7.36 (m, 4H), 7.36 - 7.33 (m, 1H), 7.31 (t, *J*= 8.9 Hz, 1H), 7.22 - 7.19 (m, 1H), 6.97 - 6.92 (m, 2H), 6.74 (s, 1H), 5.36 - 5.28 (m, 1H), 5.20 (d, *J*= 16.1 Hz, 1H), 5.02 (q, *J* = 6.8 Hz, 1H), 4.63 - 4.53 (m, 3H), 4.51 - 4.32 (m, 5H), 3.95 (d, *J* = 10.9 Hz, 1H), 3.88 - 3.77 (m, 3H), 3.32 (s, 3H), 3.06 (s, 3H), 2.48 (s, 3H), 2.44 - 2.38 (m, 2H), 2.20 (dd, *J* = 19.4, 12.0 Hz, 2H), 2.06 - 1.94 (m, 2H), 1.91 - 1.81 (m, 3H), 1.52 (d, *J* = 7.0 Hz, 3H), 1.31 - 1.27 (m, 9H), 1.11 (s, 9H), 0.77 (s, 2H), 0.57 (s, 2H).

Example 90: Synthesis of compound (3R,7aR)-7a-((7-(8-ethynyl-7-fluoro)-3-hydroxynaphthalen-1-yl)-8-fluoro-4-(1,4-oxolan-4-yl)py rido[4,3-d]pyrimidin-2-yl)oxy)methyl)hexahydro-1H-pyrrolizin-3-yl)methyl 2-(((S)-1-((2S,4R)-4-hydroxy-2-(((S)-1-(4-(4-methylthiazol-5-yl)phenyl)ethyl)carbamoyl)pyrroli din-1-yl)-3,3-dimethyl-1-oxobutan-2-yl)carbamoyl)-7-azaspiro[3.5]nonane-7-carboxylate

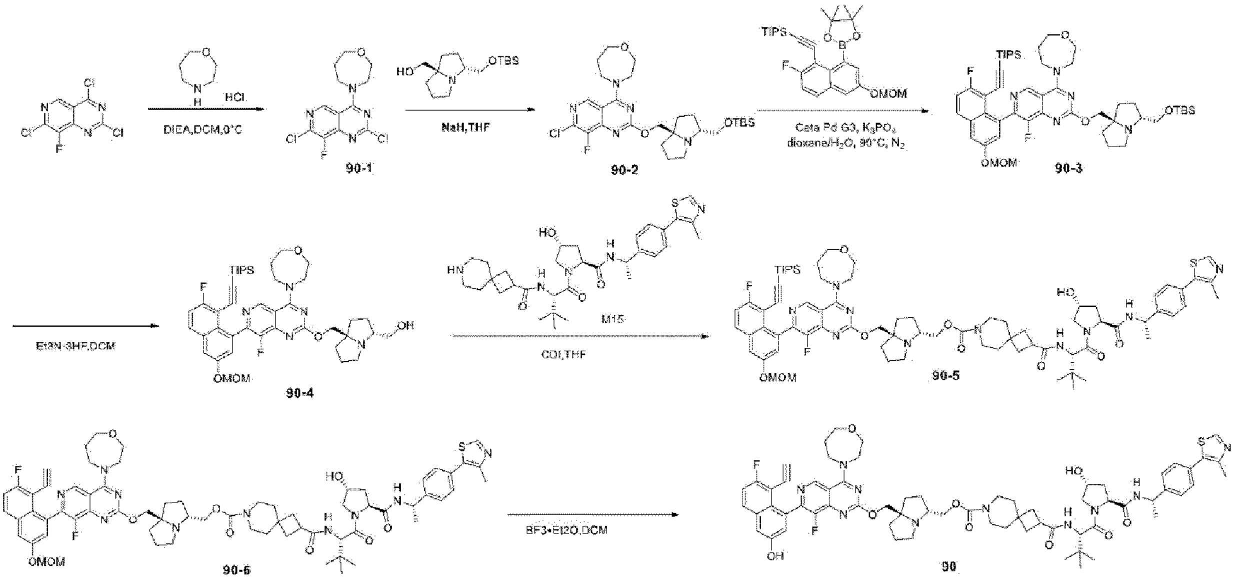


Step 1: Synthesis of compound 90-1

2,4,7-Trichloro-8-fluoropyrido[4,3-d]pyrimidine (1 g) was dissolved in 10 mL of DCM, DIPEA (1.96 mL) was added, and the mixture was placed in an ice bath. Then, a solution of homomorpholine hydrochloride (1 g) in DCM (3 mL) was added dropwise. A nitrogen balloon was inserted and the mixture was stirred at 0°C for 0.5 hours. The reaction was monitored by LC-MS until completion. 10 mL of water was added to the reaction solution. The solution was extracted three times with DCM, and the organic phases were combined, dried with anhydrous sodium sulfate. The resultant was concentrated under reduced pressure, and then separated and purified by column chromatography to obtain 1.26 g of a light yellow solid, i.e., compound 90-1.

ESI-MS m/z: 317 $[M+H]^+$;

Step 2: Synthesis of compound 90-2

((3R,7aR)-3-((tert-butyldimethylsilyl)oxy)methyl)tetrahydro-1H-pyrrolizin-7a(5H)-yl)meth anol (1.08 g) was dissolved in 4 mL of THF. Under the protection of nitrogen, NaH (230 mg) was added, and the mixture was stirred at room temperature for 0.5 hours. Then, a solution of compound 90-1 (600 mg) in THF (2 mL) was added. The mixture was stirred at room temperature for 0.5 hours. The reaction was monitored by LC-MS until completion. The reaction solution was quenched by adding 10 mL of saturated ammonium chloride solution slowly. The solution was extracted three times with EA, and the organic phases were combined, dried with anhydrous sodium sulfate. The resultant was concentrated under reduced pressure, and then separated and purified by column chromatography to obtain 790 mg of a light yellow solid, i.e., compound 90-2.

ESI-MS m/z: 566 $[M+H]^+$;

Step 3: Synthesis of compound 90-3

Compound 90-2 (790 mg), ((2-fluoro-6-(methoxymethoxy)-8-(4,4,5,5-tetramethyl-1,3,2-dioxyborolan-2-yl) naphthalen-1-yl) ethynyl)triisopropylsilane (537 mg), potassium phosphate (887mg), CataCXium A Pd G3 (51 mg) were dissolved in 8 mL of dioxane and 1.6 mL of water, and then the reaction system was purged with nitrogen, the mixture was reacted at 90°C for 3 hours. The reaction was monitored by LC-MS until completion. 20.0 mL of water was added to reaction solution, and the solution was extracted three times with EA and 30 mL for each time, the organic phases were combined, washed with saturated brine, dried with anhydrous sodium sulfate, filtered and concentrated. The resultant was

separated and purified by column chromatography to obtain 620 mg of a light yellow solid, i.e., compound 90-3.
ESI-MS m/z: 916 $[M+H]^+$.

Step 4: Synthesis of compound 90-4

Compound 90-3 (620 mg) was dissolved in 6 mL of DCM, then 3 mL of triethylamine trihydrofluoride was added. The mixture was reacted at room temperature for 2 hours. The reaction was monitored by LC-MS until completion. 20.0 mL of water was added to reaction solution, and the solution was extracted with a extract of DCM:MeOH = 10:1. The organic phases were combined, washed with saturated brine, dried with anhydrous sodium sulfate, filtered and concentrated. The resultant was separated and purified by column chromatography to obtain 500 mg of a light yellow solid, i.e., compound 90-4.

Step 5: Synthesis of compound 90-5

Compound 90-4 (89 mg) was dissolved in 3 mL of THF, then 23 mg of CDI was added, the mixture was reacted at 40°C for 1 hour, and the intermediate was monitored by LC-MS. Then, intermediate compound M15 (99 mg) was added, and the mixture was stirred at 80°C for 12 hours. The reaction was monitored by LC-MS until completion. The reaction solution was quenched by adding 5.00 mL of methanol. The organic phase was concentrated, and then separated and purified by column chromatography to obtain 101 mg of a light yellow solid, i.e., compound 90-5.
ESI-MS m/z: 712 1/2$[M+2H]^+$.

Step 6: Synthesis of compound 90-6

Compound 90-5 (101 mg) was dissolved in 3 mL of DMF, then CsF (108 mg) was added. The mixture was reacted at room temperature for 1 hour. The reaction was monitored by LCMS until completion. The solution was extracted with water and ethyl acetate, and the organic phases were combined, dried with anhydrous sodium sulfate and filtered. The resultant was concentrated under reduced pressure, and then separated and purified by column chromatography to obtain 70 mg of a light yellow solid, i.e., compound 90-6.
ESI-MS m/z: 634 1/2$[M+2H]^+$;

Step 7: Synthesis of compound 90

Compound 90-6 (70 mg) was dissolved in 3 mL of DCM, then 1.5 mL of boron trifluoride diethyl etherate was added. The mixture was reacted at room temperature for 30 minutes. The reaction was monitored by LCMS until completion. A aqueous sodium carbonate solution was added dropwise to the reaction solution, extracted with DCM:isopropanol = 10:1. The organic phases were combined, dried with anhydrous sodium sulfate and filtered. The resultant was concentrated under reduced pressure, and then separated by preparative liquid chromatography to obtain 33.2 mg of a light yellow solid, i.e., compound 90.
ESI-MS m/z: 612 1/2$[M+2H]^+$.
$^1$H NMR (500 MHz, Methanol-$d_4$) δ 9.18 (d, *J* = 5.3 Hz, 1H), 8.93(s, 1H), 7.90 - 7.87 (m, 1H), 7.45 - 7.33 (m, 6H), 7.25 (d, *J* = 2.6 Hz, 1H), 4.99 (q, *J* = 6.9 Hz, 1H), 4.76 - 4.71 (m, 2H), 4.64 - 4.54 (m, 3H), 4.48-4.43 (m, 2H), 4.36 - 4.26 (m, 6H). 4.07 (t, *J*= 4.9 Hz, 2H), 3.89 - 3.86 (m, 3H), 3.77-3.74 (m, 1H), 3.56 (d, *J*= 11.9 Hz, 1H), 3.46-3.41(m, 4H), 3.16(s, 1H), 2.48(s, 3H), 2.42-2.34 (m, 2H), 2.22 - 2.11 (m, 5H), 2.09-1.93(m, 10H), 1.60(s, 3H), 1.50 (d, *J* = 7.4 Hz, 5H), 1.33 - 1.28 (m, 2H), 1.01 (s, 9H).

Example 92: Synthesis of compound (2S,4R)-1-((R)-2-(3-((4-((1-((7-(8-ethyl-7-fluoro-3-hydroxynaphthalen-1-yl)-8-fluoro-4-((R)-3-h ydroxy-3-methylpiperidin-1-yl)pyrido[4,3-d]pyrimidin-2-yl)oxy)methyl)cyclopropyl)methyl)pip erazin-1-yl)methyl)azetan-1-yl)isoxazol-5-yl)-3-methylbutanoyl)-4-hydroxy-N-((S)-1-(4-(4-met hylthiazol-5-yl)phenyl)ethyl)pyrrolidine-2-carboxamide

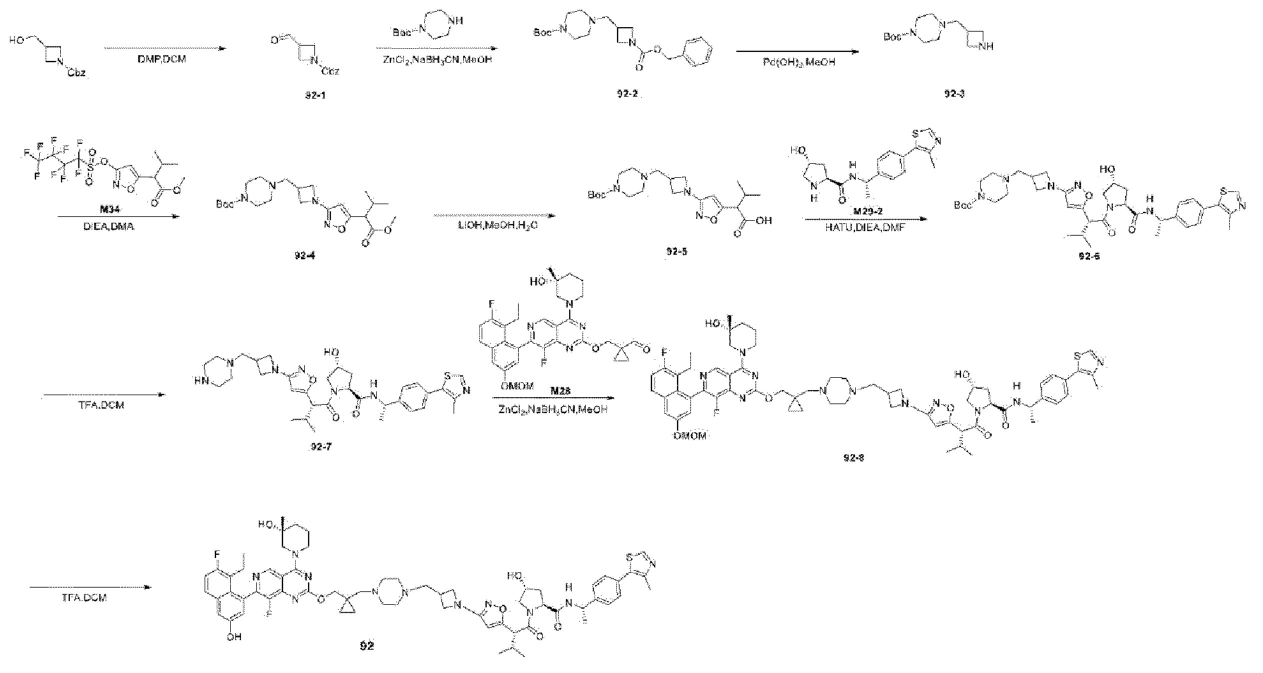


Step 1: Synthesis of compound 92-1

Benzyl 3-(hydroxymethyl)azetane-1-carboxylate (2 g) was added to 20 mL of DCM, and after dissolution, Dess-Martin periodinane (4.6 g) was added in batches in an ice bath. After returning to room temperature and stirring for 3 hours, the reaction solution was filtered. The filtrate was washed with an appropriate amount of saturated sodium bicarbonate solution, and the solution was extracted once with DCM. The organic phases were collected, combined, washed once with saturated brine, dried with anhydrous sodium sulfate, and concentrated to obtain crude product compound 92-1 (2.3 g). ESI-MS m/z: 220.1 $[M+H]^+$.

Step 2: Synthesis of compound 92-2

Compound 92-1 (2.3 g) was added to a 100 mL single-necked flask, 30 mL of toluene was added, tert-butyl piperazine-1-carboxylate (5.86 g) was added, and titanium tetraisopropanolate (2.98 g) was added. The mixture was reacted at 60°C for 1 hour. Then, sodium cyanoborohydride (1.32 g) was added to the reaction solution, and the reaction mixture was reacted for another 5 hours. After the reaction was completed, silica gel was added to the reaction solution for concentrating, and the concentrate was purified by column chromatography to obtain compound 92-2 (1.6 g). ESI-MS m/z: 390.2 $[M+H]^+$.

Step 3: Synthesis of compound 92-3

Compound 92-2 (1.6 g) was added to a 100 mL single-necked flask, 15 mL of methanol was added, and palladium hydroxide (1 g) was added. The mixture was stirred at room temperature for 17 hours under a hydrogen atmosphere. The resultant was filtered, and the filtrate was concentrated to obtain compound 92-3 (850 mg) ESI-MS m/z: 256.2 $[M+H]^+$.

Step 4: Synthesis of compound 92-4

Compound 92-3 (850 mg) was added to a 50 mL single-necked flask, intermediate M34 (1.4 g) was added, followed by the addition of DMA (15 mL). Then, DIPEA (1.55 mL) was added dropwise, and the mixture was reacted at 140°C for 2 hours. The reaction solution was diluted with water, and the solution was extracted twice with EA. The organic phases were collected, combined, dried with anhydrous sodium sulfate, and the concentrate was separated and purified by column chromatography to obtain compound 92-4 (205 mg). ESI-MS m/z: 437.3 $[M+H]^+$.

Step 5: Synthesis of compound 92-5

Compound 92-4 (205 mg) was added to a 25 mL single-necked flask, and 1 mL of methanol, 1 mL of THF, 1 mL of water were added, followed by the addition of lithium hydroxide (24 mg). The mixture was reacted at room temperature for

30 minutes. The reaction solution was concentrated, and diluted with water. Saturated sodium bicarbonate solution was added until pH = 5, and the solution was extracted twice with a solution of dichloromethane:isopropanol (5:1). The organic phases were collected, combined, wash with saturated brine, dried with anhydrous sodium sulfate, and concentrated to obtain compound 92-5 (150 mg). ESI-MS m/z: 423.3 $[M+H]^+$.

Step 6: Synthesis of compound 92-6

Compound 92-5 (150 mg), compound M29-2 (140 mg), HATU (200 mg), and N,N-diisopropylethylamine (0.37 mL) were dissolved in N,N-dimethylformamide (5.00 mL). The mixture was reacted at 25°C for 1 hour. After the reaction was completed, the resultant was extracted with ethyl acetate and water. The organic phase was washed with saturated brine, purified by column chromatography (dichloromethane/methanol) and separated by chiral column to obtain compound 92-6 (47 mg). ESI-MS m/z: 736.4 $[M+H]^+$.

Step 7: Synthesis of compound 92-7

Compound 92-6 (43 mg) was dissolved in dichloromethane (5 mL), and TFA (1 mL) was added. The mixture was reacted at 25°C for 20 minutes. After the reaction was completed, the resultant was concentrated, and diluted with water. Saturated sodium bicarbonate solution was added until pH = 8 to 9. The solution was extracted with dichloromethane/isopropanol (10:1), dried with anhydrous sodium sulfate, and concentrated to obtain compound 92-7 (35 mg). ESI-MS m/z: 636.3 $[M+H]^+$.

Step 8: Synthesis of compound 92-8

Compound 92-7 (35 mg) was added to a 25 mL single-necked flask, 1 mL of methanol was added, and compound M28 (50 mg) was added. Followed by the addition of 0.1 mL of zinc chloride (1 mol/L in THF), the mixture was stirred at room temperature for 1 hour. Sodium cyanoborohydride (25 mg) was added, and the mixture was stirred at 50°C for 3 hours. The reaction was quenched with saturated ammonium chloride solution, and the solution was extracted twice with EA. The organic phases were collected, combined, dried with anhydrous sodium sulfate, and concentrated to obtain compound 92-8 (100 mg). ESI-MS m/z: 607.8 1/2$[M+2H]^+$.

Step 9: Synthesis of compound 92

Compound 92-8 (100 mg) was added to a 10 mL single-necked flask, 2 mL of DCM was added, and 1 mL of TFA was added. The mixture was reacted at room temperature for 1 hour. After rotary evaporation, the resultant was purified by Pre-HPLC to obtain compound 92 (18.3 mg). ESI-MS m/z: 585.3 1/2$[M+2H]^+$.

Example 93: Synthesis of compound (2S,4R)-1-((S)-2-(4-((1-((1-((7-(8-ethyl-7-fluoro-3-hydroxynaphthalen-1-yl)-8-fluoro-4-((R)-3-h ydroxy-3-methylpiperidin-1-yl)pyrido[4,3-d]pyrimidin-2-yl)oxy)methyl)cyclopropyl)methyl)pip eridin-4-yl)oxy)methyl)phenyl)-1H-1,2,3-triazol-1-yl)-3-methylbutanoyl)-4-hydroxy-N-(((S)-1-( 4-(4-methylthiazol-5-yl)phenyl) ethyl)pyrrolidine-2-carboxamide

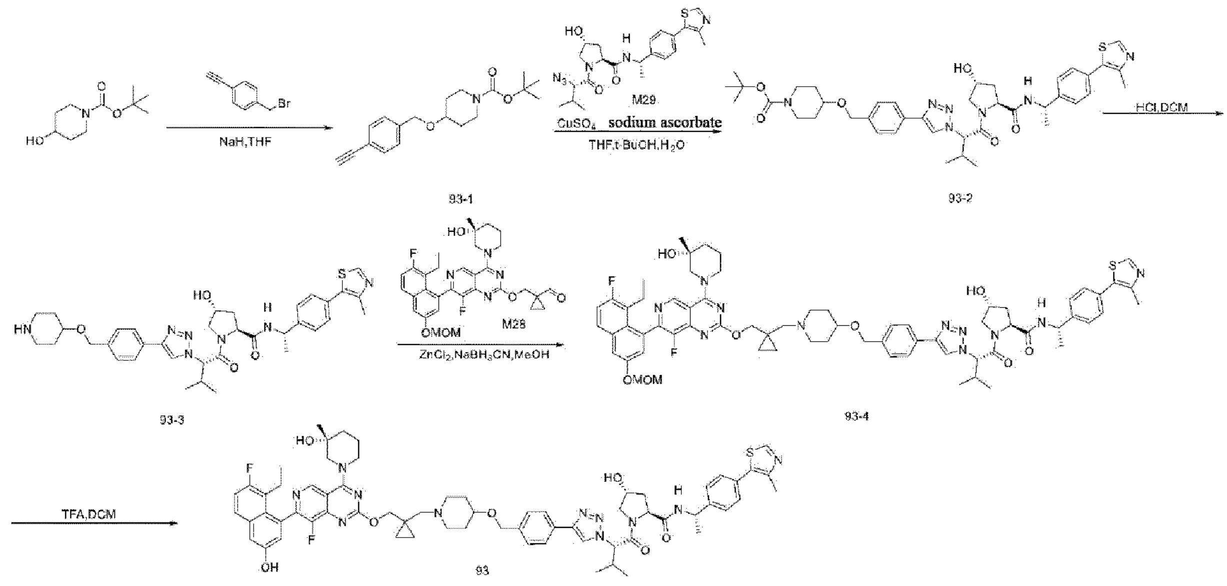


93-1 93-2 93-3 93-4 93

Step 1: Synthesis of compound 93-1

Compound N-tert-butoxycarbonyl-4-hydroxypiperidine (300.00 mg) and 1-(bromomethyl)-4-ethynylbenzene (377.98 mg) were added to tetrahydrofuran (5.00 mL), and sodium hydride (149.06 mg, 60%) was added in batches at 0°C. The reaction was heated to 50°C and stirred for 3 hours. The reaction was monitored by LCMS until completion. The resultant was diluted with $H_2O$, and extracted twice with EA. The organic phase was washed with saturated brine, and dried with anhydrous sodium sulfate. The resultant was purified by column chromatography to obtain a light yellow solid (249.00 mg, yield 52.96%), i.e., compound 93-1.

Step 2: Synthesis of compound 93-2

Compound M29 (280.00 mg) and compound 93-1 (251.47 mg) were added to tetrahydrofuran (6.00 mL), tert-butanol (3.00 mL) and water (3.00 mL). Then copper sulfate (88.10 mg) and sodium ascorbate (315.91 mg) were added. The mixture was stirred at 20°C for 4 hours. The reaction was monitored by LCMS until completion. The resultant was concentrated under reduced pressure, and then purified by column chromatography to obtain a light yellow solid (306.00 mg, yield 64.63%), i.e., compound 93-2.

Step 3: Synthesis of compound 93-3

Compound 93-2 (306.00 mg) was added to a 25 mL single-necked flask, and dichloromethane (5.00 mL) and a solution of hydrogen chloride in dioxane (2.00 mL) were added. The mixture was stirred at 20°C for 0.5 hours. The reaction was monitored by LCMS until completion. The resultant was concentrated under reduced pressure, and then saturated $NaHCO_3$ solution was added until pH = 8. The solution was extracted twice with dichloromethane/isopropanol = 5/1. The organic phase was washed with saturated brine and dried with anhydrous sodium sulfate. The crude product was used in the next step directly. A light yellow solid (278.00 mg) was obtained, i.e., compound 93-3.

Step 4: Synthesis of compound 93-4

Compound M28 (80.00 mg) and compound 93-3 (108.84 mg) was added to a 25 mL single-necked flask, methanol (2.00 mL) was added, and then zinc chloride (0.20 mL, 1.00 mol/L) was added. The reaction was heated to 40°C and stirred for 2 hours. Finally, sodium cyanoborohydride (29.69 mg) was added, and the mixture was stirred at 40°C for 12 hours. The reaction was monitored by LCMS until completion. The resultant was concentrated under reduced pressure, and then purified by column chromatography to obtain a light yellow solid (77.00 mg, yield 45.68%), i.e., compound 93-4.

Step 5: Synthesis of compound 93

Compound 93-4 (77.00 mg) was added to a 25 mL single-necked flask, dichloromethane (3.00 mL) was added, and then trifluoroacetic acid (1.50 mL) was added. The mixture was stirred at 20°C for 0.5 hours. The reaction was

monitored by LCMS until completion. The resultant was concentrated under reduced pressure, and then the crude product was purified by Pre-HPLC to obtain a pure product, which is a white solid (33.00 mg, yield 42.19%), i.e., compound 93.

ESI-MS m/z: 603.18 1/2[M+2H]$^+$.

$^1$H NMR (500 MHz, Methanol-$d_4$) δ 9.12 (d, *J* = 4.0 Hz, 1H), 8.79 (d, *J* = 8.0 Hz, 1H), 8.39 (s, 1H), 7.73 (d, *J*= 7.8 Hz, 2H), 7.59 (dd, *J*= 9.1, 5.8 Hz, 1H), 7.34 (h, *J*= 8.2 Hz, 6H), 7.22 (d, *J* = 2.7 Hz, 1H), 7.16 (t, *J* = 9.3 Hz, 1H), 6.99 (t, *J* = 2.8 Hz, 1H), 5.28 (d, *J*= 10.3 Hz, 1H), 4.97 (d, *J* = 7.0 Hz, 2H), 4.48 (d, *J* = 9.5 Hz, 3H), 4.40 (s, 2H), 4.36 (s, 2H), 4.16 (t, *J* = 14.8 Hz, 1H), 3.87 - 3.78 (m, 2H), 3.60 - 3.48 (m, 1H), 3.39 (t, *J* = 11.4 Hz, 2H), 2.85 (d, *J* = 11.4 Hz, 2H), 2.56 (dq, *J* = 10.2, 6.7 Hz, 1H), 2.39 (d, *J*= 11.6 Hz, 4H), 2.34 (dd, *J*= 12.9, 5.7 Hz, 1H), 2.16 - 2.08 (m, 4H), 1.93 - 1.89 (m, 1H), 1.89 - 1.83 (m, 2H), 1.77 (d, *J* = 12.6 Hz, 1H), 1.69 (q, *J*= 10.9, 7.5 Hz, 2H), 1.61 - 1.49 (m, 3H), 1.45 (d, *J* = 7.0 Hz, 2H), 1.28 - 1.24 (m, 1H), 1.20 (d, *J* = 14.1 Hz, 4H), 1.09 (d,*J* = 6.8 Hz, 3H), 0.74 (t, *J* = 8.0 Hz, 5H), 0.64 (s, 2H), 0.42 (s, 2H).

Example 100: Synthesis of compound (2S,4R)-1-((R)-2-(3-((1-(1-((((7-(8-ethyl-7-fluoro-3-hydroxynaphthalen-1-yl)-8-fluoro-4-(((R-)-3-hydroxy-3-methylpiperidin-1-yl)pyrido[4,3-d]pyrimidin-2-yl)oxy)methyl)cyclopropyl)methyl) -4-fluoropiperidin-4-yl)methoxy)isoxazol-5-yl)-3-methylbutanoyl)-4-hydroxy-N-((S)-1-(4-(4-me thylthiazol-5-yl)phenyl)ethyl)pyrrolidine-2-carboxamide

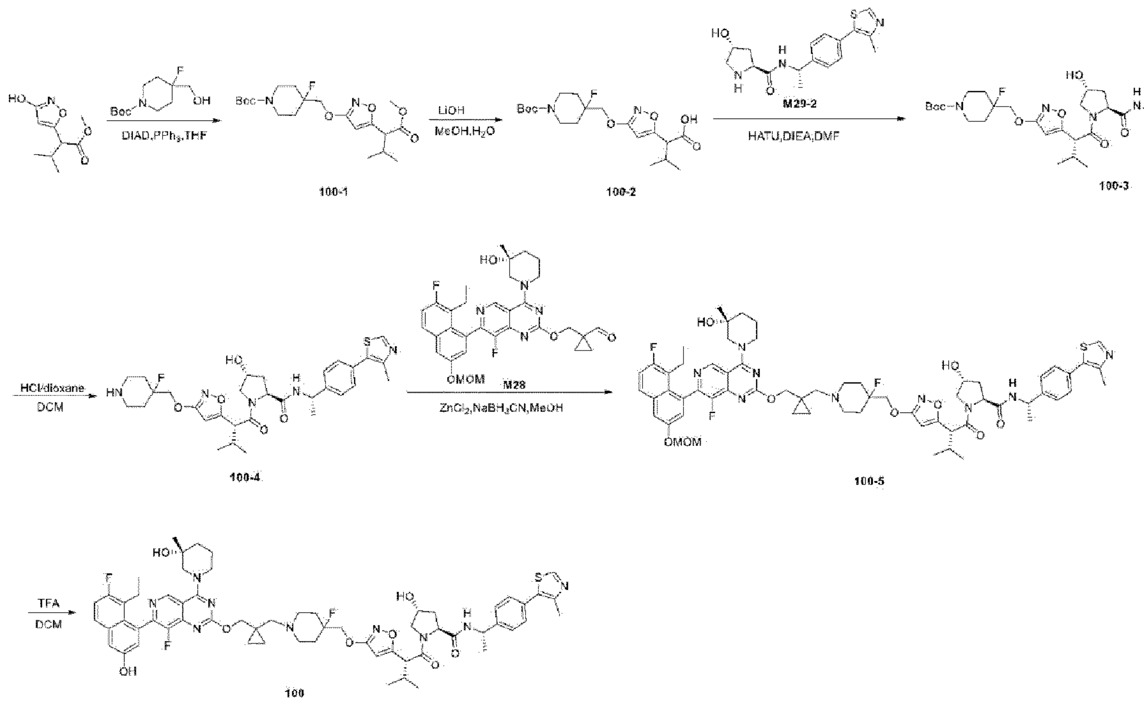


Step 1: Synthesis of compound 100-1

Methyl 2-(3-hydroxyisoxazol-5-yl)-3-methylbutyrate (500 mg), tert-butyl 4-fluoro-4-(hydroxymethyl)piperidine-1-carboxylate (585 mg), triphenylphosphine (1.32 g), and DIAD (1.02 g) were dissolved in tetrahydrofuran (10 mL), and the mixture was reacted at 60°C for 1 hour. After the reaction was completed, the resultant was concentrated and purified by column chromatography (petroleum ether/ethyl acetate) to obtain compound 100-1 (786 mg). ESI-MS m/z: 315.21 [M+H-100]$^+$.

Step 2: Synthesis of compound 100-2

Compound 100-1 (786 mg) was dissolved in methanol (10 mL) and water (2 mL), and lithium hydroxide (227 mg) was added. The mixture was reacted at 25°C for 1 hour. After the reaction was completed, citric acid was added until pH = 4. The solution was extracted three times with dichloromethane/isopropanol (10:1), dried with anhydrous sodium sulfate, filtered, and concentrated to obtain compound 100-2 (683 mg). ESI-MS m/z: 301.22 [M+H-100]$^+$.

Step 3: Synthesis of compound 100-3

Compound 100-2 (300 mg), compound M29-2 (248 mg), HATU (427 mg), and N,N-diisopropylethylamine (0.37 mL) were dissolved in N,N-dimethylformamide (5.00 mL). The mixture was reacted at 25°C for 1 hour. After the reaction was completed, the resultant was diluted with water and extracted with ethyl acetate. The organic phase was washed with saturated brine, purified by column chromatography (dichloromethane/methanol) and separated by chiral column to obtain compound 100-3 (170 mg). ESI-MS m/z: 714.54 $[M+H]^+$.

Step 4: Synthesis of compound 100-4

Compound 100-3 (170 mg) was dissolved in dichloromethane (5 mL), and 4 M hydrogen chloride in dioxane (1 mL) was added. The mixture was reacted at 25°C for 20 minutes. After the reaction was completed, the resultant was neutralized with sodium bicarbonate solution, extracted with dichloromethane/isopropanol (10:1), dried, and concentrated to obtain compound 100-4 (118 mg). ESI-MS m/z: 614.50 $[M+H]^+$.

Step 5: Synthesis of compound 100-5

Compound 100-4 (118 mg), compound M28 (114 mg), and zinc chloride (0.18 mL, 1.00 mol/L) were dissolved in methanol (10 mL). The mixture was reacted at 40°C for 2 hours. Then, sodium cyanoborohydride (18 mg) was added, and the mixture was reacted at 40°C for 8 hours. After the reaction was completed, the reaction was quenched with ammonium chloride solution, concentrated and purified by column chromatography (dichloromethane/methanol) to obtain compound 100-5 (177 mg). ESI-MS m/z: 596.27 1/2$[M+2H]^+$.

Step 6: Synthesis of compound 100

Compound 100-5 (177 mg) was dissolved in dichloromethane (4 mL), and trifluoroacetic acid (1 mL) was added. The mixture was reacted at 25°C for 1 hour. After the reaction was completed, the resultant was concentrated and then separated and purified by Pre-HPLC (C18 column, A: 0.05% trifluoroacetic acid aqueous solution, B: acetonitrile, concentration gradient: 20% B to 75% B, 11 minutes, flow rate: 15 mL/min, 254 nm), concentrated, and neutralized with sodium bicarbonate solution. The product was precipitated and filtered to obtain compound 100 (29.9 mg). ESI-MS m/z:574.10 1/2$[M+2H]^+$.
$^1$H NMR (500 MHz, Methanol-$d_4$) δ 9.21 - 9.19 (m, 1H), 8.87 - 9.86 (m, 1H), 7.68 - 7.65 (m, 1H), 7.46 - 7.35 (m, 4H), 7.30 - 7.27 (m, 1H), 7.26 - 7.22 (m, 1H), 7.07 - 7.04 (m, 1H), 6.03 (d, *J* = 3.7 Hz, 1H), 5.04 - 4.98 (m, 1H), 4.52 - 4.40 (m, 5H), 4.27 - 4.16 (m, 3H), 3.82 (dd, *J* = 10.9, 4.2 Hz, 1H), 3.69 - 3.64 (m, 1H), 3.63 - 3.57 (m, 1H), 3.50 - 3.41 (m, 1H), 2.93 - 2.83 (m, 2H), 2.54 - 2.43 (m, 6H), 2.39 - 2.28 (m, 3H), 2.25 - 2.09 (m, 3H), 1.97 - 1.82 (m, 5H), 1.81 - 1.72 (m, 4H), 1.57 (d, *J* = 7.0 Hz, 1H), 1.51 (d, *J* = 7.0 Hz, 2H), 1.29 - 1.25 (m, 3H), 1.04 (d, *J*= 6.5 Hz, 3H), 0.91 - 0.87 (m, 3H), 0.83 - 0.78 (m, 3H), 0.74 - 0.70 (m, 2H), 0.54 - 0.46 (m, 2H).

Example 104: Synthesis of compound (2S,4R)-1-((R)-2-(3-(1-((1-((7-(8-ethyl-7-fluoro-3-hydroxynaphthalen-1-yl)-8-fluoro-4-((R)-3-h ydroxy-3-methylpiperidin-1-yl)pyrido[4,3-d]pyrimidin-2-yl)oxy)methyl)cyclopropyl)methyl)pip eridin-4-yl)methyl)piperazin-1-yl)isoxazol-5-yl)-3-methylbutanoyl)-4-hydroxy-N-((S)-1-(4-(4-m ethylthiazol-5-yl)phenyl)ethylpyrrolidine-2-carboxamide

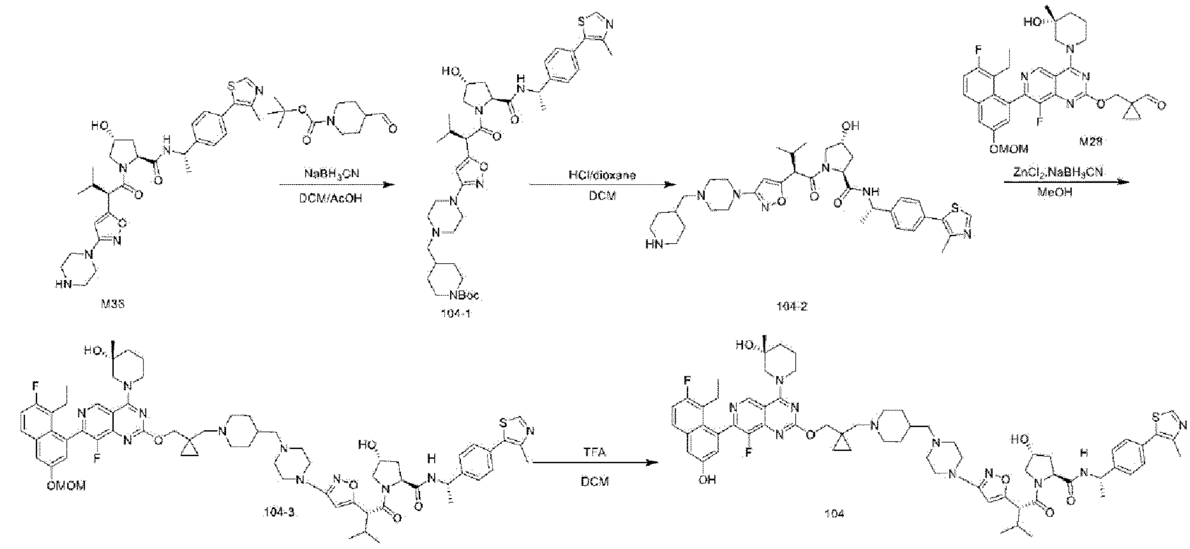


Step 1: Synthesis of compound 104-1

Compound M36 (80.0 mg) and 1-tert-butoxycarbonyl-piperidine-4-carboxaldehyde (30.0 mg) were dissolved in dichloromethane (5 mL), one drop of acetic acid was added, and the mixture was reacted at room temperature for 1 hour. Sodium cyanoborohydride (17.8 mg) was then added, and the mixture reacted at room temperature for 10 minutes. The reaction was monitored by LCMS until completion. The reaction was quenched by adding saturated sodium bicarbonate solution (5.0 mL). DCM (20 mL) was added to the reaction solution, and the organic phase was collected, washed once with saturated brine, dried with anhydrous sodium sulfate, filtered, and concentrated to obtain 73.0 mg of a white solid, i.e., compound 104-1. ESI-MS m/z: 764.3 $[M+H]^+$.

Step 2: Synthesis of compound 104-2

Compound 104-1 (73.0 mg) was dissolved in DCM (1 mL) and TFA (1 mL), and the mixture was reacted at room temperature for 10 minutes. The reaction was monitored by LCMS until completion. The reaction solution was concentrated directly, and 1 M NaOH solution was added until pH of the reaction solution reached 8. DCM (20 mL) was added to the reaction solution, and the organic phase was collected, washed once with saturated brine, dried with anhydrous sodium sulfate, filtered, and concentrated to obtain 55.0 mg of a white solid, i.e., compound 104-2. ESI-MS m/z: 664.1 $[M+H]^+$.

Step 3: Synthesis of compound 104-3

Compound 104-2 (55.0 mg) and compound M28 (61.6 mg) were dissolved in methanol (8 mL), 1 M zinc chloride in diethyl ether (0.25 mL) was added. The mixture was reacted at 40°C for 1 hour. Sodium cyanoborohydride (29.2 mg) was then added, and the mixture reacted at 60°C for 8 hours. The reaction was monitored by LCMS until completion. The reaction was quenched by adding saturated sodium bicarbonate solution (5.0 mL). DCM (20 mL) was added to the reaction solution, and the organic phase was collected, washed once with saturated brine, dried with anhydrous sodium sulfate, filtered, and concentrated to obtain 78.0 mg of a white solid, i.e., compound 104-3. ESI-MS m/z: 621.4 1/2$[M+2H]^+$.

Step 4: Synthesis of compound 104

Compound 104-3 (78.0 mg) was dissolved in DCM (2 mL) and TFA (1 mL), and the mixture was reacted at room temperature for 10 minutes. The reaction was monitored by LCMS until completion. The reaction solution was concentrated directly, and the resultant was separated by preparative liquid chromatography to obtain 15.3 mg of a white powder, i.e., compound 104.

ESI-MS m/z: 599.2 1/2$[M+2H]^+$.

Example 105: Synthesis of compound (2S,4R)-1-(R)-2-(3-(7-(8-ethyl-7-fluoro-3-hydroxynaphthalen-1-yl)-8-fluoro-4-((R)-3-hydroxy-3 -methylpiperidin-1-yl)pyrido[4,3-d]pyrimidin-2-yl)oxy)methyl)cyclopropyl)methyl)-2,7-diazaspi ro [3.5]nonan-2-yl)isoxazol-5-yl)-3-methylbutanoyl)-4-hydroxy-N-((S)-1-(4-(4-methylthiazol-5-y l)phenyl)ethyl)pyrrolidine-2-carboxamide

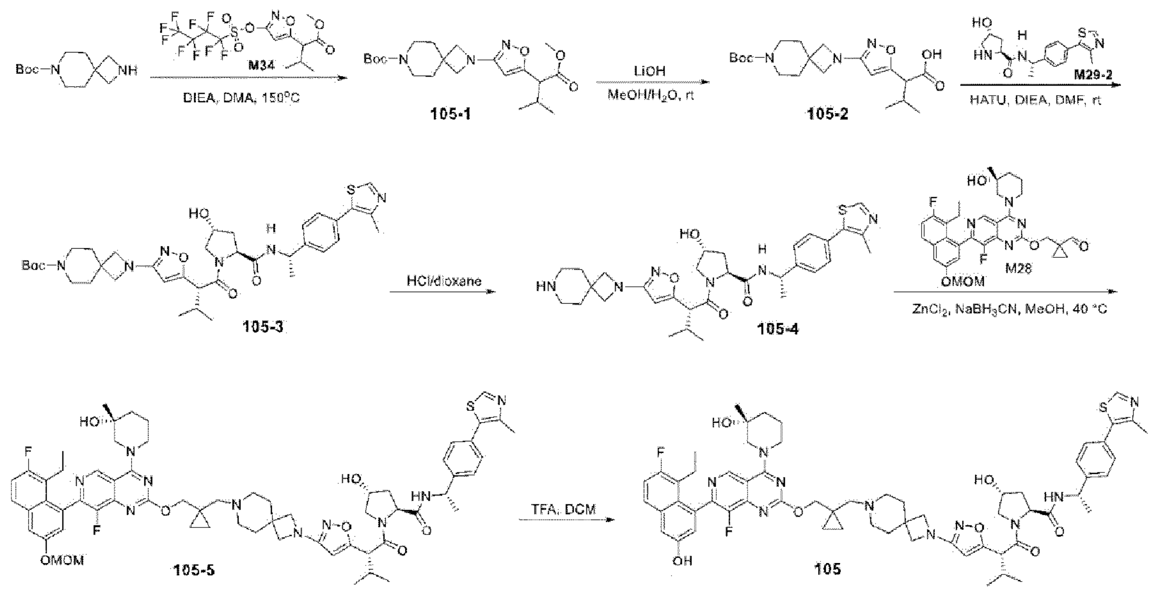


Step 1: Synthesis of compound 105-1

Commercially available tert-butyl 2,7-diazaspiro[3.5]nonane-7-carboxylate (310.00 mg), intermediate M34 (791.14 mg), N,N-dimethylacetamide (6.00 mL), and N,N-diisopropylethylamine (0.68 mL) were added to a reaction flask. The mixture was reacted at 150°C for 1 hour under microwave. The resultant was extracted with EA and water, dried, and concentrated. The resultant was separated by column chromatography to obtain a yellow solid of compound 105-1 (300 mg). ESI-MS m/z: 408.24 $[M+H]^+$.

Step 2: Synthesis of compound 105-2

Compound 105-1 (300 mg), methanol (4.00 mL), water (1.00 mL), and lithium hydroxide monohydrate (185.35 mg) were added to a reaction flask, and the mixture was reacted at room temperature for 2 hours. The solvent was concentrated first, then dilute hydrochloric acid was added until pH = 3. The resultant was extracted with DCM and methanol, dried, and concentrated to obtain a yellow oil of compound 105-2 (270 mg). ESI-MS m/z: 394.23 $[M+H]^+$.

Step 3: Synthesis of compound 105-3

Compound 105-2 (260 mg), compound M29-2 (2S,4R)-4-hydroxy-N-((S)-1-(4-(4-methylthiazol-5-yl) phenyl) ethyl)pyrrolidine-2-carboxamide (241 mg), N,N-dimethylformamide (5 mL), and N,N-diisopropylethylamine (0.33 mL) were added to a reaction flask. Then N,N,N',N'-tetramethyl-O-(7-azabenzotriazol-1-yl)uronium hexafluorophosphate (301 mg) was added under stirring. The mixture was reacted at room temperature for 0.5 hours. The resultant was diluted with water, extracted with EA, dried, and concentrated, and then separated by column chromatography (DCM:MeOH = 20:1) to obtain 310 mg of a yellow solid. Subsequently, the resultant was separated by SFC to obtain a single compound 105-3 (105 mg). ESI-MS m/z: 707.35 $[M+H]^+$.

Step 4: Synthesis of compound 105-4

Compound 105-3 (105 mg) and dichloromethane (3 mL) were added to a reaction flask, and 4 M hydrogen chloride in dioxane (0.5 mL) was added under stirring. The mixture was reacted at room temperature for 0.2 hours. A large amount of solid was precipitated, and the supernatant was decanted. The solid was dissolved by adding methanol, and the resultant was diluted with DCM, and aqueous sodium bicarbonate solution was added until pH = 8. The resultant was

extracted, dried, and concentrated to obtain a yellow solid of compound 105-4 (70 mg). ESI-MS m/z: 707.35 $[M+H]^+$.

Step 5: Synthesis of compound 105-5

Intermediate M28 (50 mg), compound 105-4 (56 mg), methanol (1.50 mL), 1,2-dichloroethane (1.50 mL), 1 M zinc chloride in diethyl ether (0.08 mL) were added to a reaction flask. The mixture was reacted at 40°C for 1 hour, and then sodium cyanoborohydride (53.04 mg) was added. The mixture was reacted at 40°C for 8 hours. The resultant was diluted with aqueous sodium bicarbonate solution, extracted with DCM, dried, and concentrated. The resultant was separated by Pre-TLC using DCM:ammonia in methanol = 15:1 as the developing solvent to obtain a white solid of compound 105-5 (50 mg). ESI-MS m/z: 592.68 1/2$[M+2H]^+$.

Step 6: Synthesis of compound 105

Compound 105-5 (50.00 mg), dichloromethane (1.50 mL), and trifluoroacetic acid (1.50 mL) were added to a reaction flask. The mixture was reacted at room temperature for 0.5 hours. The resultant was concentrated and then separated by Pre-HPLC to obtain compound 105 (21 mg).

ESI-MS m/z: 570.62 1/2$[M+2H]^+$.

$^1$H NMR (500 MHz, $CDCl_3$) δ 9.32 - 9.01 (m, 1H), 8.75 - 8.50 (m, 1H), 7.58 - 7.47 (m, 1H), 7.33 (dt, *J* = 9.1, 4.7 Hz, 2H), 7.27 (d, *J*= 7.7 Hz, 4H), 7.21 - 7.10 (m, 2H), 6.85 (d, *J* = 126.4 Hz, 1H), 5.67 (d, *J* = 9.9 Hz, 1H), 4.99 - 4.90 (m, 1H), 4.76 - 4.68 (m, 1H), 4.55 (d, *J* = 4.8 Hz, 1H), 4.36 - 4.12 (m, 3H), 3.99 - 3.77 (m, 1H), 3.66 - 3.42 (m, 7H), 3.29 - 3.01 (m, 2H), 2.53 - 2.34 (m, 8H), 2.24 - 1.96 (m, 5H), 1.87 - 1.48 (m, 12H), 1.39 - 1.21 (m, 9H), 1.04 - 0.84 (m, 6H), 0.83 - 0.74 (m, 3H).

Example 110: Synthesis of compound (3S,7aS)-7a-((7-(8-ethyl-7-fluoro-3-hydroxynaphthalen-1-yl)-8-fluoro-4-((R)-3-hydroxy-3-meth ylpiperidin-1-yl)pyrido[4,3-d]pyrimidin-2-yl)oxy)methyl)-6-methylenehexahydro-1H-pyrrolizin-3-yl) methyl 4-(5-((R)-1-((2S,4R)-4-hydroxy-2-((S)-1-(4-(4-methylthiazol-5-yl)phenyl)ethylcarbamoyl)pyrrol idin-1-yl)-3-methyl-1-oxobutan-2-yl)isoxazol-3-yl)piperazine-1-carboxylate

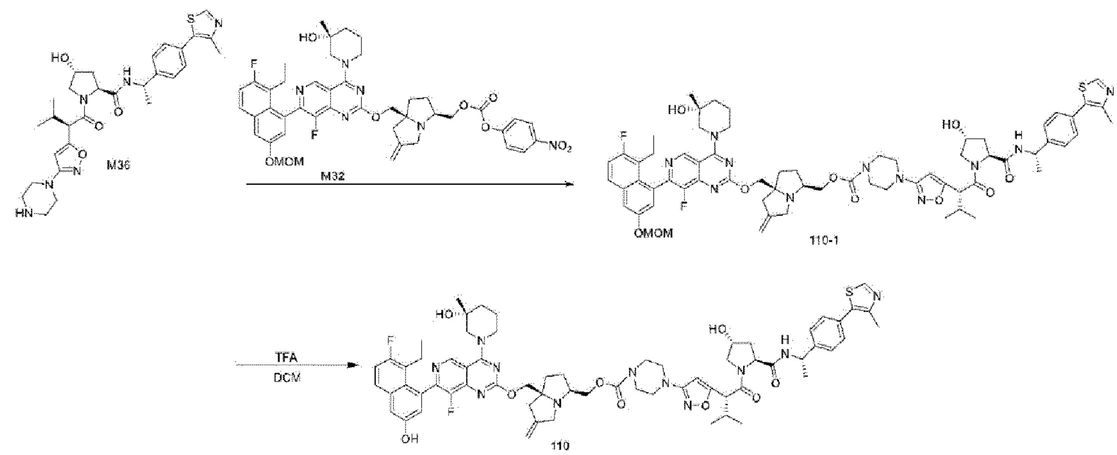


Step 1: Synthesis of compound 110-1

Compound M36 (73.0 mg) was added to the reaction solution of intermediate M32, and the mixture was stirred at 20°C for 1 hour. The reaction was monitored by LCMS until completion. The resultant was diluted with $H_2O$, and extracted twice with EA. The organic phase was washed three times with saturated brine, and dried with anhydrous sodium sulfate. The resultant was purified by column chromatography to obtain a light yellow solid (55.1 mg), i.e., compound 110-1. ESI-MS m/z: 635.2 1/2$[M+2H]^+$.

Step 2: Synthesis of compound 110

Compound 110-1 (55.1 mg) was added to DCM (2 mL) and TFA (1 mL), and the mixture was reacted at room temperature for 10 minutes. The reaction was monitored by LCMS until completion. The reaction solution was concentrated directly, and the resultant was separated by preparative liquid chromatography to obtain 22.2 mg of a white

powder, i.e., compound 110.

ESI-MS m/z: 613.2 1/2[M+2H]$^+$.

$^1$H NMR (500 MHz, Methanol-$d_4$) δ 9.22 (d, *J* = 9.5 Hz, 1H), 8.87 (s, 1H), 7.88 (s, 1H), 7.68-7.65 (m, 1H), 7.44-7.38 (m, 4H), 7.29 (d, *J* = 3.0 Hz, 1H), 7.24 (t, *J* = 4.0 Hz, 1H), 7.08-7.06 (m, 1H), 6.11 (d, *J*= 10.5 Hz, 1H), 5.03-5.01(m, 1H), 4.97-4.93 (m, 1H), 4.85-4.80(m, 1H), 4.50-4.49 (m, 2H), 4.19-4.08(m, 2H), 4.00-3.79 (m, 2H), 3.79-3.38 (m, 12H), 3.33-3.19 (m,4H), 3.10-3.00 (m, 1H), 2.65-2.71 (m, 1H), 2.50-2.41 (m, 4H), 2.40-2.30 (m, 2H), 2.29-1.90(m, 6H), 1.89-1.72 (m, 5H), 1.55-1.45(m, 3H), 1.40-1.20 (m, 2H), 1.10-0.79 (m, 9H).

Example 111: Synthesis of compound (2S,4R)-1-(R)-2-(3-(4-(4-((1-((7-(8-ethyl-7-fluoro-3-hydroxynaphthalen-1-yl)-8-fluoro-4-((R)-3-hydroxy-3-methylpiperidin-1-yl)pyrido[4,3-d]pyrimidin-2-yl)oxy)methyl)cyclopropyl)methyl)pi perazin-1-yl)isoxazol-5-yl)-3-methylbutanoyl)-4-hydroxy-N-((S)-1-(4-(4-methylthiazol-5-yl)phe nyl)ethyl)pyrrolidine-2-carboxamide

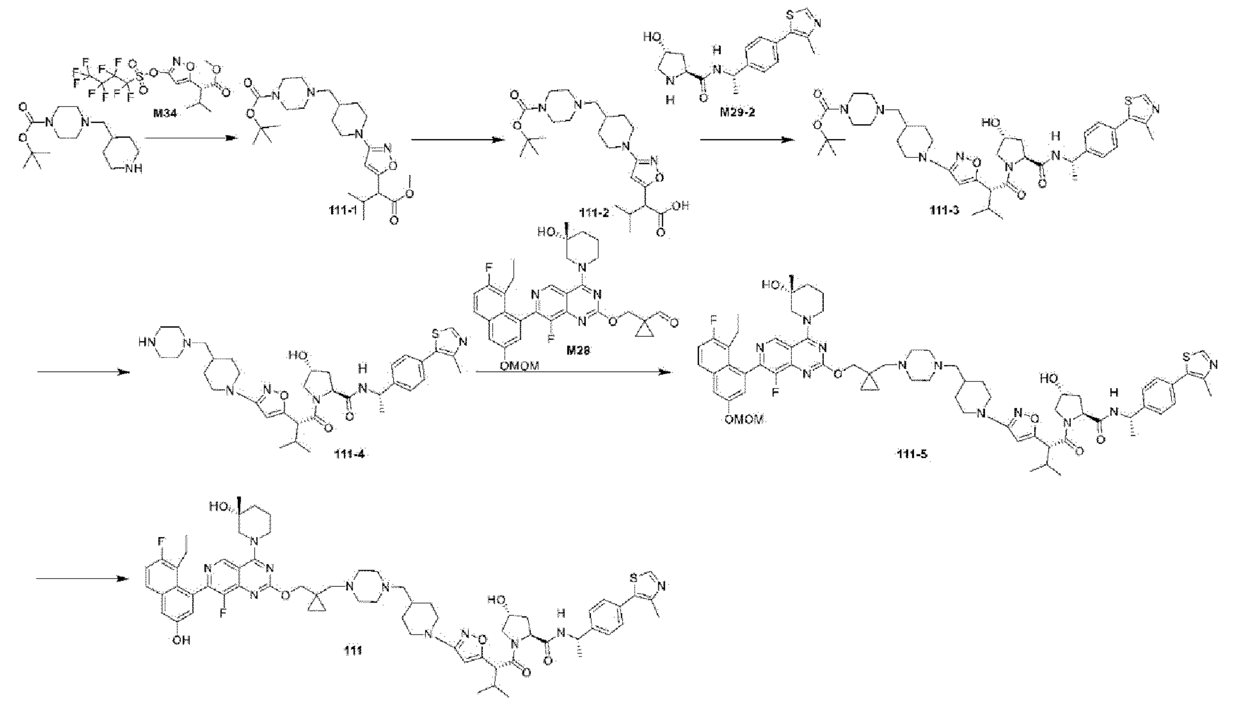

Step 1: Synthesis of compound 111-1

Tert-butyl 4-[(piperidin-4-yl)methyl]piperazine-1-carboxylate (400.00 mg), compound M34 (815.16 mg), and DIPEA (0.70 mL, 4.23 mmol) were added to DMA (3.00 mL). The mixture was reacted at 150°C for 2 hours. The resultant was diluted with 50 mL of water, extracted twice with EA, washed with saturated brine, and separated by column chromatography (PE:EA = 1:1) to obtain compound 111-1 (380.00 mg, 57.95%).

ESI-MS m/z: 465.33 [M+H]$^+$.

Step 2: Synthesis of compound 111-2

Compound 111-1 (370.00 mg) and anhydrous lithium hydroxide (47.68 mg) were added to water (2.00 mL), methanol (2.00 mL) and tetrahydrofuran (2.00 mL). The mixture was reacted at room temperature for 1 hour. The resultant was diluted with water, added with 1 N hydrochloric acid aqueous solution until pH = 3, extracted three times with DCM, dried with anhydrous sodium sulfate, and concentrated to obtain compound 111-2 (350 mg, 97.54%).

ESI-MS m/z: 451.35 [M+H]$^+$.

Step 3: Synthesis of compound 111-3

Compound 111-2 (340.00 mg), DIPEA (0.62 mL), HATU (373.01 mg) and compound M29-2 (250.10 mg) were

sequentially added to dichloromethane (8.00 mL). The mixture was reacted at room temperature for 2 hours. Water was added, and the resultant was extracted with DCM, washed with saturated brine, dried, and separated by column chromatography (DCM:MeOH = 91%: 9%) and then separated by chiral column to obtain compound 111-3 (100 mg, 17.35%).

ESI-MS m/z: 764.55 $[M+H]^+$.

Step 4: Synthesis of compound 111-4

Compound 111-3 (100.00 mg) and trifluoroacetic acid (1.00 mL) were added to dichloromethane (2.00 mL). The mixture was reacted at room temperature for 1 hour. 40 mL of saturated sodium bicarbonate solution was added to the reaction solution to adjust to alkaline. The solution was extracted twice with DCM, dried with anhydrous sodium sulfate, and concentrated to obtain compound 111-4 (80.00 mg, 92.06%).

ESI-MS m/z: 664.49 $[M+H]^+$.

Step 5: Synthesis of compound 111-5

Compound 111-4 (80.00 mg), compound M28 (71.41 mg), and 1.0 M zinc chloride in tetrahydrofuran (0.24 mL) were added to methanol (3.00 mL) and 1,2-dichloroethane (3.00 mL). The mixture was reacted at 40°C for 2 hours, and then sodium cyanoborohydride (75.72 mg) was added, followed by reacting at 40°C for 3 hours. The reaction solution was concentrated, and 30 mL of water was added. The solution was extracted twice with DCM, and dried with anhydrous sodium sulfate. The resultant was separated by column chromatography (DCM:MeOH = 90%:10%) to obtain compound 111-5 (70.00 mg, 46.83%).

ESI-MS m/z: 621.24 1/2$[M+2H]^+$.

Step 6: Synthesis of compound 111

Compound 111-5 (70.00 mg) and trifluoroacetic acid (1.00 mL) were added to dichloromethane (2.00 mL). The mixture was reacted at room temperature for 1 hour. The reaction solution was concentrated, then separated and purified by Pre-HPLC to obtain compound 111 (25.40 mg, 36.67%).

ESI-MS m/z: 599.17 1/2 $[M+2H]^+$.

$^1$H NMR (500 MHz, Methanol-$d_4$) δ 9.20 (d, J = 8.0 Hz, 1H), 8.87 (s, 1H), 7.67 (dd, J = 8.8, 5.9 Hz, 1H), 7.46 - 7.36 (m, 4H), 7.30 (d, J = 2.3 Hz, 1H), 7.24 (t, J = 9.3 Hz, 1H), 7.05 (s, 1H), 6.08 (s, 1H), 5.06 - 4.99 (m, 1H), 4.49 (dt, J = 10.1, 5.9 Hz, 3H), 4.45 - 4.38 (m, 2H), 4.28 - 4.20 (m, 1H), 3.84 (dd, J = 10.8, 4.1 Hz, 1H), 3.68 - 3.42 (m, 8H), 2.81 (t, J = 12.2 Hz, 2H), 2.66 - 2.29 (m, 15H), 2.18 (t, J = 11.9 Hz, 6H), 1.95 (ddd, J = 8.6, 6.6, 3.4 Hz, 1H), 1.89 1.68 (m, 7H), 1.58 (d, J = 7.0 Hz, 1H), 1.52 (d, J = 7.0 Hz, 3H), 1.26 - 1.16 (m, 3H), 1.04 (d, J = 6.5 Hz, 3H), 0.92 - 0.86 (m, 4H), 0.82 (dd, J = 16.2, 7.6 Hz, 3H), 0.72 (s, 2H), 0.51 (s, 2H).

Example 112: Synthesis of compound (2S,4R)-1-(R)-2-(3-(7-(8-ethyl-7-fluoro-3-hydroxynaphthalen-1-yl)-8-fluoro-4-((R)-3-hydroxy-3 -methylpiperidin-1-yl)pyrido[4,3-d]pyrimidin-2-yl)oxy)methyl)cyclopropyl)methyl)piperazin-1-yl) isoxazol-5-yl)-3-methylbutanoyl)-4-hydroxy-N-((S)-1-(4-(4-methylthiazol-5-yl)phenyl)ethyl) pyrrolidine-2-carboxamide

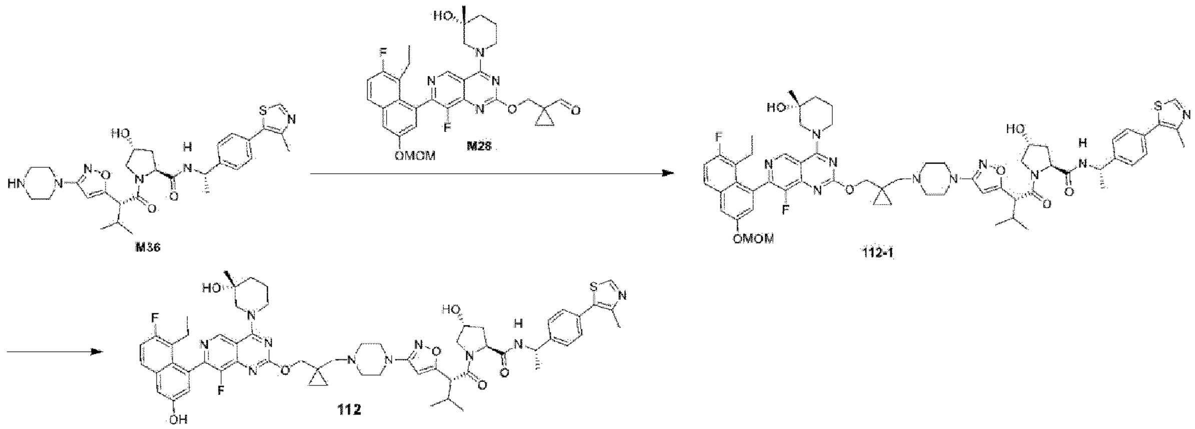
M28
M36
112-1
112

Step 1: Synthesis of compound 112-1

Compound M36 (50.00 mg), compound M28 (52.27 mg), and 1.0 M zinc chloride in tetrahydrofuran (0.18 mL) were added to methanol (2.00 mL) and 1,2-dichloroethane (2.00 mL). The mixture was reacted at 40°C for 2 hours, and then sodium cyanoborohydride (27.71 mg) was added, followed by reacting at 40°C for 3 hours. The reaction solution was concentrated, and 30 mL of water was added. The solution was extracted twice with DCM, and dried with anhydrous sodium sulfate. The resultant was separated by column chromatography (DCM:MeOH = 90%:10%) to obtain compound 112-1 (15.00 mg, 14.87%).

ESI-MS m/z: 572.74 1/2[M+2H]$^+$.

Step 2: Synthesis of compound 112

Compound 112-1 (15.00 mg) and trifluoroacetic acid (0.50 mL) were added to dichloromethane (1.00 mL). The mixture was reacted at room temperature for 1 hour. The reaction solution was concentrated, then separated and purified by Pre-HPLC to obtain compound 112 (8.50 mg, 56.67%).

ESI-MS m/z: 550.57 1/2[M+2H]$^+$.

Example 113: Synthesis of compound (2S,4R)-1-(R)-2-(3-(3aR,6aS)-5-(1-((7-(8-ethyl-7-fluoro-3-hydroxynaphthalen-1-yl)-8-fluoro-4-( (R)-3-hydroxy-3-methylpiperidin-1-yl)pyrido[4,3-d]pyrimidin-2-yl)oxy)methyl)cyclopropyl)met hyl)hexahydropyrrolo[3,4-c]pyrrol-2(1H)-yl)isoxazol-5-yl)-3-methylbutanoyl)-4-hydroxy-N-((S) -1-(4-(4-methylthiazol-5-yl)phenyl)ethyl)pyrrolidine-2-carboxamide

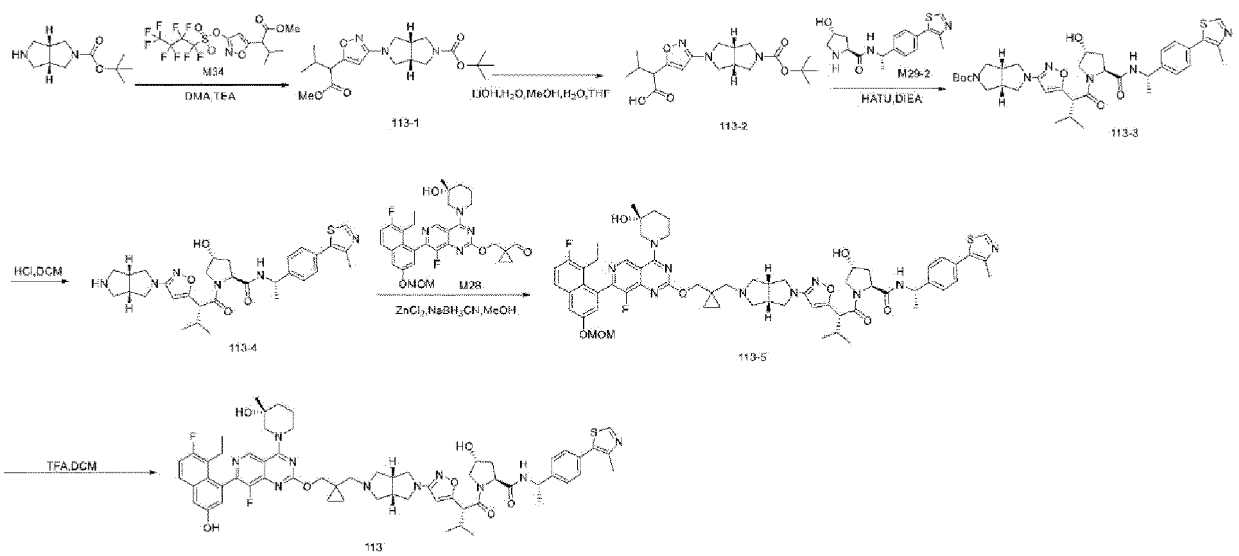


Step 1: Synthesis of compound 113-1

Compound tert-butyl (3aR,6aS)-hexahydropyrrolo[3,4-c]pyrrole-2(1H)-carboxylate (242.60 mg) was added to N,N-dimethylacetamide (2.50 mL), then triethylamine (0.72 mL) was added. The reaction was heated to 120°C and stirred for 0.5 hours. Finally, compound M34 (500.00 mg) was added. The mixture was stirred at 120°C for 5 hours. The reaction was monitored by LCMS until completion. The resultant was diluted with $H_2O$, and extracted twice with EA. The organic phase was washed three times with saturated brine, and dried with anhydrous sodium sulfate. The resultant was purified by column chromatography to obtain a light yellow solid (212.00 mg, yield 51.86%), i.e., compound 113-1.

Step 2: Synthesis of compound 113-2

Compound 113-1 (212.00 mg) was added to tetrahydrofuran (2.00 mL), methanol (2.00 mL) and water (2.00 mL), and then lithium hydroxide monohydrate (113.04 mg, 2.69 mmol) was added. The mixture was stirred at 20°C for 1 hours. The reaction was monitored by LCMS until completion. The resultant was concentrated under reduced pressure, and then dilute hydrochloric acid was added until pH = 5. The solution was then extracted twice with dichloromethane/isopropanol =

5/1. The organic phase was washed with saturated brine and dried with anhydrous sodium sulfate. The crude product was used in the next step directly. A light yellow solid (209.00 mg) was obtained, i.e., compound 113-2.

Step 3: Synthesis of compound 113-3

Compound 113-2 (201.53 mg) and compound M29-2 (160.00 mg) were added to a 25 mL single-necked flask, then N,N-dimethylacetamide (3.00 mL), N,N,N',N'-tetramethyl-O-(7-azabenzotriazol-1-yl)uronium hexafluorophosphate (238.63 mg, 0.63 mmol) and N,N-diisopropylethylamine (0.40 mL, 2.41 mmol) were added. The mixture was stirred at 20°C for 0.5 hours. The reaction was monitored by LCMS until completion. The resultant was diluted with $H_2O$, and extracted twice with EA. The organic phase was washed three times with saturated brine, and dried with anhydrous sodium sulfate. The crude product was separated by SFC to obtain a light yellow solid (96.00 mg), i.e., compound 113-3.

Step 4: Synthesis of compound 113-4

Compound 113-3 (96.00 mg) was added to a 25 mL single-necked flask, and dichloromethane (2.00 mL) and 4 M hydrogen chloride in dioxane (1.00 mL) were added. The mixture was stirred at 20°C for 0.5 hours. The reaction was monitored by LCMS until completion. The resultant was concentrated under reduced pressure, and then saturated $NaHCO_3$ solution was added until pH = 8. The solution was extracted twice with dichloromethane/isopropanol = 5/1. The organic phase was washed with saturated brine and dried with anhydrous sodium sulfate. The crude product was used in the next step directly. A light yellow solid (84.00 mg) was obtained, i.e., compound 113-4.

Step 5: Synthesis of compound 113-5

Compound M28 (70.00 mg) and compound 113-4 (83.99 mg) was added to a 25 mL single-necked flask, methanol (2.00 mL) was added, and then zinc chloride (0.18 mL, 1.00 mol/L) was added. The reaction was heated to 40°C and stirred for 2 hours. Finally, sodium cyanoborohydride (25.98 mg, 0.41 mmol) was added, and the mixture was stirred at 50°C for 12 hours. The reaction was monitored by LCMS until completion. The resultant was concentrated under reduced pressure, and then purified by column chromatography to obtain a light yellow solid (88.00 mg, yield 63.72%), i.e., compound 113-5.

Step 6: Synthesis of compound 113

Compound 113-5 (88.00 mg) was added to a 25 mL single-necked flask, dichloromethane (4.00 mL) was added, and then trifluoroacetic acid (1.50 mL)was added. The mixture was stirred at 20°C for 0.5 hours. The reaction was monitored by LCMS until completion. The resultant was concentrated under reduced pressure, and then the crude product was purified by preparative HPLC to obtain a pure product, which is a white solid (42.20 mg, yield 48.01%), i.e., compound 113.

ESI-MS m/z: 563.70 1/2$[M+2H]^+$.

$^1H$ NMR (500 MHz, Methanol-$d_4$) δ 9.08 (dd, *J* = 7.3, 2.6 Hz, 1H), 8.77 (d, *J* = 6.3 Hz, 1H), 7.57 (dd, *J*= 9.1, 5.7 Hz, 1H), 7.39 - 7.23 (m, 5H), 7.20 (dt, *J* = 4.3, 2.6 Hz, 1H), 7.14 (td, *J* = 9.3, 4.7 Hz, 1H), 7.01 - 6.95 (m, 1H), 5.89 - 5.85 (m, 1H), 4.92 (d, *J*= 7.0 Hz, 1H), 4.50 (s, 2H), 4.39 (dt, *J* = 11.7, 4.1 Hz, 2H), 4.35 - 4.30 (m, 2H), 4.19 - 4.09 (m, 1H), 3.71 (dt, *J*= 10.7, 3.8 Hz, 1H), 3.58 - 3.48 (m, 2H), 3.38 - 3.31 (m, 1H), 3.25 (d, *J*= 6.3 Hz, 2H), 3.04 (q, *J* = 9.7, 8.3 Hz, 2H), 2.83 (s, 3H), 2.49 (d, *J* = 31.0 Hz, 3H), 2.37 (d, *J* = 2.0 Hz, 3H), 2.26 (dt, *J* = 9.7, 6.6 Hz, 1H), 2.12 - 2.02 (m, 3H), 1.87 - 1.80 (m, 1H), 1.77 - 1.72 (m, 1H), 1.67 (dt, *J* = 11.7, 7.0 Hz, 2H), 1.46 (dd, *J* = 7.0, 3.7 Hz, 2H), 1.40 (d, *J* = 7.0 Hz, 3H), 0.93 (dt, *J* = 6.6, 3.1 Hz, 3H), 0.80 - 0.77 (m, 4H), 0.76 (d, *J* = 6.3 Hz, 3H), 0.71 (d, *J* = 7.2 Hz, 2H), 0.63 (s, 2H), 0.45 (s, 2H).

Example 114: Synthesis of compound (2S,4R)-1-((R)-2-(3-((1-((1-(((7-(8-ethyl-7-fluoro-3-hydroxynaphthalen-1-yl)-8-fluoro-4-((R)-3-hydroxy-3-methylpiperidin-1-yl)pyrido[4,3-d]pyrimidin-2-yl)oxy)methyl)cyclopropyl)methyl)pi peridin-4-yl)oxy)isoxazol-5-yl)-3-methylbutanoyl)-4-hydroxy-N-((S)-1-(4-(4-methylthiazol-5-yl )phenyl)ethyl)pyrrolidine-2-carboxamide

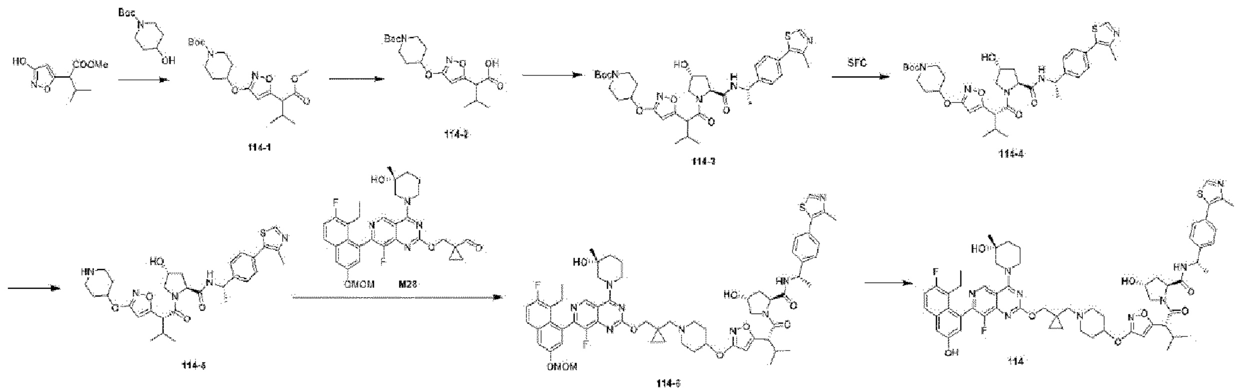


Step 1: Synthesis of compound 114-1

Methyl 2-(3-hydroxyisoxazol-5-yl)-3-methylbutanoate (567.0 mg), tert-butyl 4-hydroxypiperidine-1-carboxylate (573.0 mg), and triphenylphosphine (1.12 g) were dissolved in THF (10 mL) under the protection of nitrogen. Diisopropyl azodicarboxylate (863 mg) was added slowly, and the mixture was reacted at 60°C for 4 hours. The reaction was monitored by LCMS until completion. EA (20 mL) was added to the reaction solution, and the resultant was washed once with saturated brine. The organic phase was dried with anhydrous sodium sulfate, filtered, concentrated, and then separated and purified by column chromatography to obtain 850.0 mg of a white solid, i.e., compound 114-1. ESI-MS m/z: 383.2 $[M+H]^+$.

Step 2: Synthesis of compound 114-2

Compound 114-1 (850.0 mg) was added to methanol (10 mL) and water (2 mL), and lithium hydroxide monohydrate (467.0 mg) was added. The mixture was reacted at room temperature for 1 hour, and the reaction was monitored by LCMS until completion. The pH of the reaction solution was adjusted to 3, and EA (20 mL) was added. The organic phase was collected, washed once with saturated brine, dried with anhydrous sodium sulfate, filtered, and concentrated to obtain 825.0 mg of a white solid, i.e., compound 114-2. ESI-MS m/z: 369.2 $[M+H]^+$.

Step 3: Synthesis of compound 114-3

Compound 114-2 (825.0 mg), (2S,4R)-4-hydroxy-N-((S)-1-(4-(4-methylthiazol-5-yl)phenyl)ethyl)pyrrolidine-2-carboxamide (821 mg), and HATU (1028 mg) were dissolved in DMF (10 mL). DIPEA (1.18 mL) was added. The mixture was reacted at room temperature for 10 minutes, and the reaction was monitored by LCMS until completion. EA (20 mL) and saturated brine (20 mL) were added to the reaction solution. The organic phase was collected, dried with anhydrous sodium sulfate, filtered, concentrated, and purified by column chromatography to obtain 1291.0 mg of a white solid, i.e., compound 114-3. ESI-MS m/z: 682.3 $[M+H]^+$.

Step 4: Synthesis of compound 114-4

Compound 114-3 (1291 mg) was separated by SFC and concentrated to obtain 502 mg of a white solid, i.e., compound 114-4. ESI-MS m/z: 682.3 $[M+H]^+$.

Step 5: Synthesis of compound 114-5

Compound 114-4 (502.0 mg) was added to DCM (5 mL) and TFA (2 mL), and the mixture was reacted at room temperature for 10 minutes. The reaction was monitored by LCMS until completion. The reaction solution was concentrated directly, and 1 M NaOH solution was added until pH of the reaction solution reached 8. DCM (20 mL) was added to the reaction solution, and the organic phase was collected, washed once with saturated brine, dried with anhydrous sodium sulfate, filtered, and concentrated to obtain 426 mg of a white solid, i.e., compound 114-5. ESI-MS m/z: 582.3 $[M+H]^+$.

Step 6: Synthesis of compound 114-6

Compound 114-5 (200.0 mg) and compound M28 (207 mg) were dissolved in methanol (10 mL), 1 M zinc chloride

in diethyl ether (0.52 mL) was added. The mixture was reacted at 40°C for 1 hour. Sodium cyanoborohydride (64.8 mg) was then added, and the mixture reacted at 60°C for 12 hours. The reaction was monitored by LCMS until completion. The reaction was quenched by adding saturated sodium bicarbonate solution (5.0 mL). DCM (20 mL) was added to the reaction solution, and the organic phase was collected, washed once with saturated brine, dried with anhydrous sodium sulfate, filtered, and concentrated to obtain 310 mg of a white solid, i.e., compound 114-6. ESI-MS m/z: 579.8 1/2[M+2H]$^+$.

Step 7: Synthesis of compound 114

Compound 114-6 (310 mg) was added to DCM (10 mL) and TFA (2 mL), and the mixture was reacted at room temperature for 10 minutes. The reaction was monitored by LCMS until completion. The reaction solution was concentrated directly, and the resultant was separated by preparative liquid chromatography to obtain 48.0 mg of a white powder, i.e., compound 114.

ESI-MS m/z: 558.1 1/2[M+2H]$^+$.

$^1$H NMR (500 MHz, Methanol-$d_4$) δ 9.19 (d, *J* = 8.4 Hz, 1H), 8.86 (s, 1H), 7.66 (dd, J = 9.0, 5.9 Hz, 1H), 7.47 - 7.35 (m, 4H), 7.29 (t, J = 2.5 Hz, 1H), 7.24 (dd, J = 12.5, 6.2 Hz, 1H), 7.07 (t, J = 2.4 Hz, 1H), 5.98 (d, J = 1.8 Hz, 1H), 5.08 - 4.96 (m, 1H), 4.63 - 4.35 (m, 7H), 4.24 (t, J = 13.8 Hz, 1H), 3.86 - 3.78 (m, 1H), 3.67 - 3.39 (m, 4H), 2.87 (s, 2H), 2.57 - 2.29 (m, 9H), 2.25 - 2.10 (m, 3H), 2.02 (d, J = 5.6 Hz, 3H), 1.95 (ddd, J = 13.2, 8.7, 4.7 Hz, 1H), 1.82 (dd, J = 35.3, 10.7 Hz, 7H), 1.51 (d, J = 7.0 Hz, 4H), 1.27 (s, 2H), 1.03 (t, J = 15.0 Hz, 4H), 0.81 (dd, J = 16.3, 7.5 Hz, 4H), 0.72 (s, 3H), 0.50 (s, 2H).

Example 115: Synthesis of compound (2S,4R)-1-(R)-2-(3-(4-(1-((7-(8-ethyl-7-fluoro-3-hydroxynaphthalen-1-yl)-8-fluoro-4-((R)-3-hyd roxy-3-methylpiperidin-1-yl)pyrido[4,3-d]pyrimidin-2-yl)oxy)methyl)cyclopropyl)methyl)piperi din-4-yl)piperazin-1-yl)isoxazol-5-yl)-3-methylbutanoyl)-4-hydroxy-N-((S)-1-(4-methylthiazol-5-yl)phenylethyl)pyrrolidine-2-carboxamide

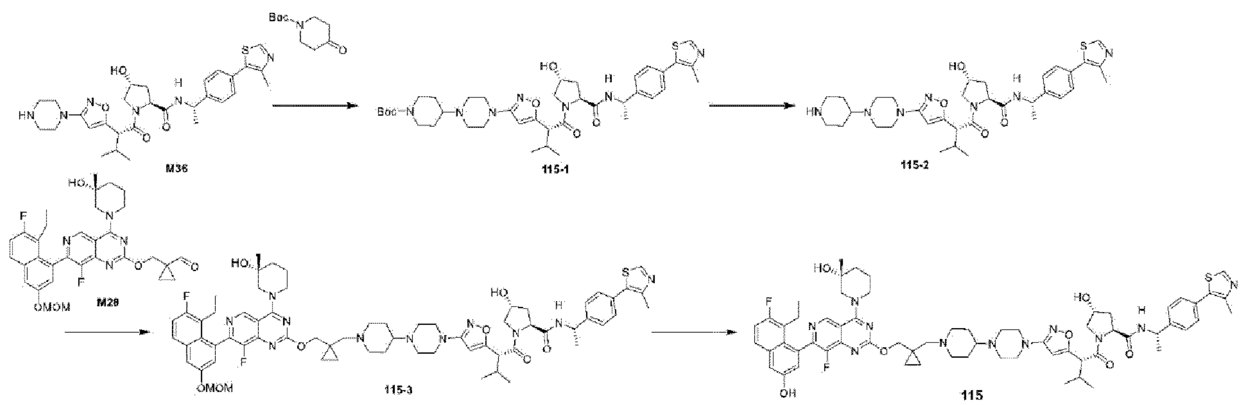
M36 115-1 115-2 M28 115-3 115

Step 1: Synthesis of compound 115-1

Compound M36 (90.00 mg), tert-butyl 4-[(piperidin-4-yl)methyl]piperazine-1-carboxylate (63.28 mg), and 1.0 M zinc chloride in tetrahydrofuran (0.32 mL, 1.00 mol/L) were added to methanol (2.00 mL) and 1,2-dichloroethane (2.00 mL). The mixture was reacted at 40°C for 2 hours, and then sodium cyanoborohydride (49.90 mg) was added, followed by reacting at 40°C for 3 hours. The reaction solution was concentrated, and 30 mL of water was added. The solution was extracted twice with DCM, and dried with anhydrous sodium sulfate. The resultant was separated by column chromatography (DCM:MeOH = 90%:10%) to obtain compound 115-1 (87.00 mg, 73.35%).

ESI-MS m/z: 750.59 [M+H]$^+$.

Step 2: Synthesis of compound 115-2

Compound 115-1 (87.00 mg) and trifluoroacetic acid (1.00 mL) were added to dichloromethane (2.00 mL). The mixture was reacted at room temperature for 1 hour. 40 mL of saturated sodium bicarbonate solution was added to the reaction solution to adjust to alkaline. The solution was extracted twice with DCM, and dried with anhydrous sodium sulfate. The resultant was separated by column chromatography (DCM:MeOH = 90%:10%) to obtain compound 115- 2 (60.00 mg, 79.59%).

ESI-MS m/z: 650.48 [M+H]$^+$.

Step 3: Synthesis of compound 115-3

Compound 115-2 (50.00 mg), compound M28 (42.19 mg), and 1.0 M zinc chloride in tetrahydrofuran (0.15 mL) were added to methanol (2.00 mL) and 1,2-dichloroethane (2.00 mL). The mixture was reacted at 40°C for 2 hours, and then sodium cyanoborohydride (24.16 mg) was added, followed by reacting at 40°C for 3 hours. The reaction solution was concentrated, and 30 mL of water was added. The solution was extracted twice with DCM, and dried with anhydrous sodium sulfate. The resultant was purified by column chromatography (DCM:MeOH = 90%:10%) to obtain compound 115-3 (35.00 mg, 38.49%).
ESI-MS m/z: 614.18 1/2[M+2H]$^+$.

Step 4: Synthesis of compound 115

Compound 115-3 (35.00 mg) and trifluoroacetic acid (0.50 mL) were added to dichloromethane (1.00 mL). The mixture was reacted at room temperature for 1 hour. The reaction solution was concentrated, then separated and purified by Pre-HPLC to obtain compound 115 (11.40 mg, 33.59%).
ESI-MS m/z: 592.27 1/2[M+2H]$^+$.

Example 116: Synthesis of compound (2S,4R)-1-(R)-2-(3-(4-(1-((7-(8-ethyl-7-fluoro-3-hydroxynaphthalen-1-yl)-8-fluoro-4-(R)-3-hydr oxy-3-methylpiperidin-1-yl)pyrido[4,3-d]pyrimidin-2-yl)oxy)methyl)cyclopropyl)methyl)azetan-3-yl)piperazin-1-yl)isoxazol-5-yl)-3-methylbutanoyl)-4-hydroxy-N-(((S)-1-(4-(4-methylthiazol-5 -yl)phenyl)ethyl)pyrrolidine-2-carboxamide

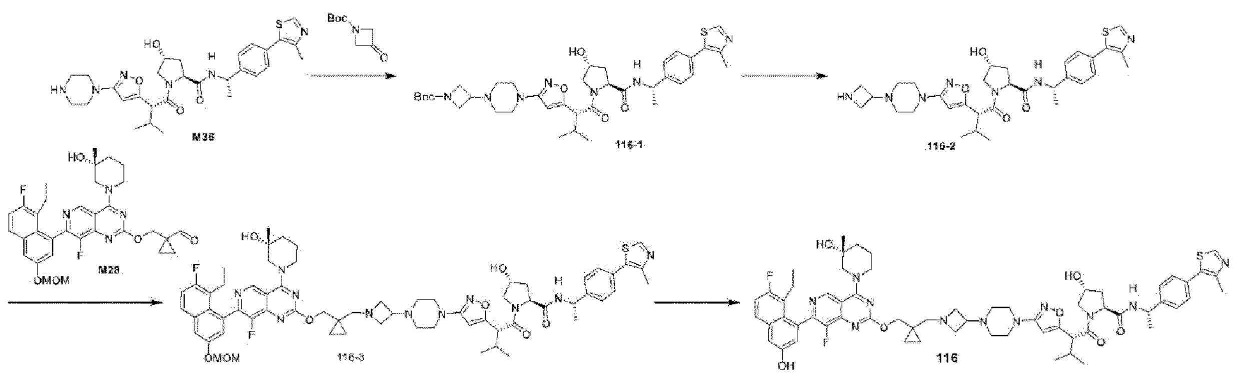
M36
116-1
116-2
M28
116-3
116

Step 1: Synthesis of compound 116-1

Compound M36 (80.00 mg), tert-butyl 3-oxoazetidine-1-carboxylate (72.52 mg), and 1.0 M zinc chloride in tetrahydrofuran (0.28 mL, 1.00 mol/L) were added to methanol (2.00 mL) and 1,2-dichloroethane (2.00 mL). The mixture was reacted at 40°C for 2 hours, and then sodium cyanoborohydride (44.37 mg) was added, followed by reacting at 40°C for 3 hours. The reaction solution was concentrated, and 30 mL of water was added. The solution was extracted twice with DCM, and dried with anhydrous sodium sulfate. The resultant was purified by column chromatography (DCM:MeOH = 90%:10%) to obtain compound 116-1 (60.00 mg, 58.56%).
ESI-MS m/z:722.57[M+H]$^+$.

Step 2: Synthesis of compound 116-2

Compound 116-1 (60.00 mg) and trifluoroacetic acid (1.00 mL) were added to dichloromethane (2.00 mL). The mixture was reacted at room temperature for 1 hour. 40 mL of saturated sodium bicarbonate solution was added to the reaction solution to adjust to alkaline. The solution was extracted twice with DCM, and dried with anhydrous sodium sulfate. The resultant was separated by column chromatography (DCM:MeOH = 90%:10%) to obtain compound 116-2 (40.00 mg, 77.41%).
ESI-MS m/z: 622.46 [M+H]$^+$.

Step 3: Synthesis of compound 116-3

Compound 116-2 (40.00 mg), compound M28 (45.75 mg), and 1.0 M zinc chloride in tetrahydrofuran (0.13 mL) were added to methanol (2.00 mL) and 1,2-dichloroethane (2.00 mL). The mixture was reacted at 40°C for 2 hours, and then sodium cyanoborohydride (22.20 mg) was added, followed by reacting at 40°C for 3 hours. The reaction solution was concentrated, and 30 mL of water was added. The solution was extracted twice with DCM, and dried with anhydrous sodium sulfate. The resultant was purified by column chromatography (DCM:MeOH = 90%:10%) to obtain compound 116-3 (30.00 mg, 38.93%).

ESI-MS m/z: 600.30 1/2[M+2H]$^+$.

Step 4: Synthesis of compound 116

Compound 116-3 (30.00mg) and trifluoroacetic acid (0.50 mL) were added to dichloromethane (1.00 mL). The mixture was reacted at room temperature for 1 hour. The reaction solution was concentrated, then separated and purified by Pre-HPLC to obtain compound 116 (15.00 mg, 49.67%).

ESI-MS m/z: 578.14 1/2[M+2H]$^+$.

$^1$H NMR (500 MHz, Methanol-$d_4$) δ 9.24 (d, *J* = 3.7 Hz, 1H), 8.87 (d, *J* = 3.2 Hz, 1H), 7.67 (dt, *J* = 9.1, 5.5 Hz, 1H), 7.46 - 7.38 (m, 4H), 7.31 - 7.28 (m, 1H), 7.27 - 7.22 (m, 1H), 7.06 (t, *J* = 2.6 Hz, 1H), 6.10 - 6.00 (m, 1H), 5.07 - 4.99 (m, 1H), 4.59 - 4.48 (m, 2H), 4.45 - 4.35 (m, 3H), 4.30 (t, *J* = 13.4 Hz, 1H), 4.16 (s, 2H), 3.84 (dd, *J* = 10.8, 4.2 Hz, 1H), 3.62 (dt, *J* = 29.2, 9.1 Hz, 3H), 3.47 (dd, *J*= 17.9, 8.2 Hz, 1H), 2.50 - 2.29 (m, 10H), 2.24 - 2.10 (m, 3H), 2.03 (s, 1H), 2.00 - 1.92 (m, 2H), 1.85 (s, 1H), 1.82 - 1.74 (m, 2H), 1.58 (dd, *J* = 7.0, 2.9 Hz, 1H), 1.52 (d, *J*= 7.0 Hz, 3H), 1.31 - 1.26 (m, 10H), 1.05 (d, *J* = 6.5 Hz, 3H), 0.85 (ddd, *J* = 24.4, 15.5, 7.1 Hz, 12H).

Example 117: Synthesis of compound (2S,4R)-1-((S)-2-(4-(5-(1-((7-(8-ethyl-7-fluoro-3-hydroxynaphthalen-1-yl)-8-fluoro-4-((R)-3-hy droxy-3-methylpiperidin-1-yl)pyrido[4,3-d]pyrimidin-2-yl)oxy)methyl)cyclopropyl)methyl)piper idin-4-yl)oxy)pyridin-2-yl)-1H-1,2,3-triazol-1-yl)-3-methylbutanoyl)-4-hydroxy-N-(((S)-1-(4-m ethylthiazol-5-yl)phenyl)ethyl) pyrrolidine-2-carboxamide

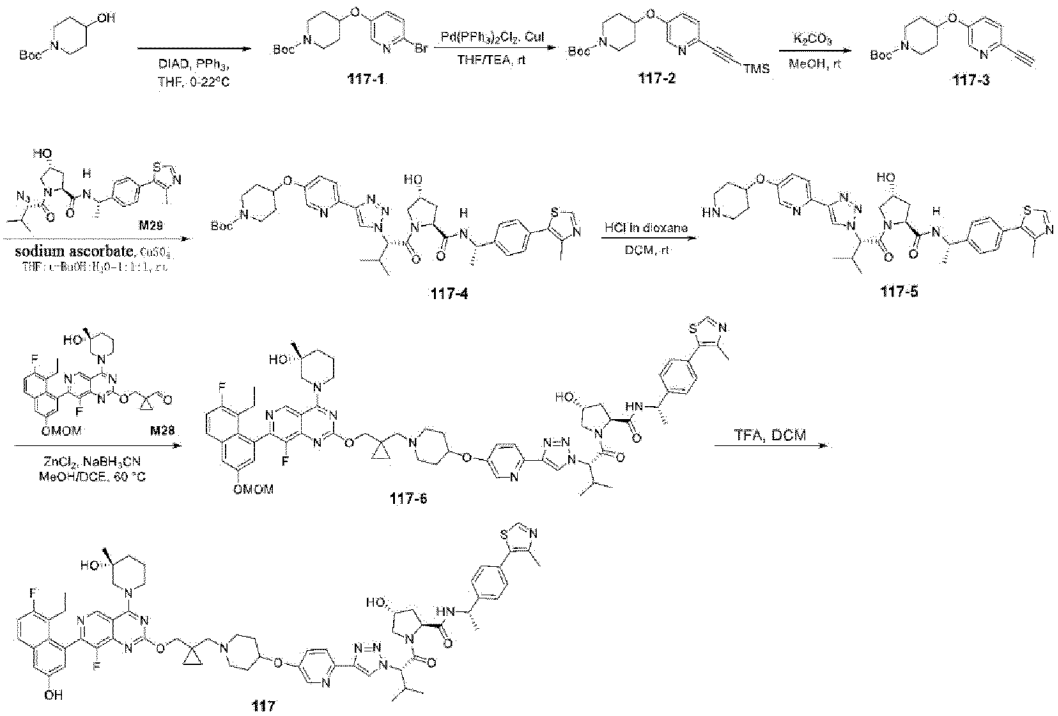


Step 1: Synthesis of compound 117-1

Tert-butyl 4-hydroxypiperidine-1-carboxylate (3.47 g), 6-bromopyridin-3-ol (2.00 g), THF (50.00 mL), and triphenylphosphine (3.92 g) were added to a reaction flask. The mixture was cooled to 0°C, followed by slow addition

of diisopropyl azodicarboxylate (3.02 g). After the addition was completed, followed by returning to room temperature, the mixture was reacted for 6 hours. The resultant was diluted with water, extracted with EA, dried and concentrated. The resultant was separated and purified by column chromatography (PE:EA = 4:1) to obtain a colorless liquid of compound 117-1 (3.3 g). ESI-MS m/z: 301.1 $[M+H-t-Bu]^+$.

Step 2: Synthesis of compound 117-2

Compound 117-1 (1.00 g), bis(triphenylphosphine)palladium dichloride (0.10 g), triethylamine (4.67 mL), and tetrahydrofuran (5.00 mL) were added to a reaction flask, then the reaction system was purged with nitrogen, and the mixture was reacted at room temperature for 10 minutes. Then, cuprous iodide (0.03 g) and ethynyltrimethylsilane (0.41 g) were added. After the reaction system was purged with nitrogen, the mixture was reacted at room temperature for 2.5 hours. The reaction solution was directly purified by column chromatography (PE:EA = 4:1) to obtain a yellow solid (930 mg) of compound 117-2. ESI-MS m/z: 375.2 $[M+H]^+$.

Step 3: Synthesis of compound 117-3

Compound 117-2 (930.00 mg), methanol (3.00 mL), and potassium carbonate (686.35 mg) were added to a reaction flask. The mixture was reacted at room temperature for 1 hour. The resultant was filtered, and the filter cake was washed three times with EA. The filtrate was concentrated and purified by column chromatography (PE:EA = 2:1) to obtain compound 117-3(702 mg) as a yellow solid. ESI-MS m/z: 303.4 $[M+H]^+$.

Step 4: Synthesis of compound 117-4

Compound 117-3 (99.33 mg), compound M29 (100.00 mg), copper sulfate (31.46 mg), sodium ascorbate (112.80 mg), tetrahydrofuran (1.50 mL), tert-butanol (1.50 mL), and water (1.50 mL) were added to a reaction flask. The mixture was reacted at 60°C for 2 hours. Then, the resultant was concentrated, extracted with DCM, dried and concentrated. The resultant was purified by column chromatography (DCM:ammonia in methanol = 25:1) to obtain a light yellow solid of compound 117-4 (91 mg). ESI-MS m/z: 759.4 $[M+H]^+$.

Step 5: Synthesis of compound 117-5

Compound 117-4 (91.00 mg), dichloromethane (3 mL) and 4 M hydrogen chloride in dioxane (0.18 mL) were added to a reaction flask. The mixture was reacted at room temperature for 0.2 hours. A large amount of solid was precipitated, and the reaction solution was decanted. The solid was dissolved by adding methanol. the resultant was diluted with DCM, and aqueous sodium bicarbonate solution was added until pH = 8. The resultant was extracted, dried, and concentrated to obtain compound 117-5 (72 mg). ESI-MS m/z: 659.4 $[M+H]^+$.

Step 6: Synthesis of compound 117-6

Compound 117-5 (61.14 mg), intermediate M28 (50.00 mg), methanol (1.00 mL), 1,2-dichloroethane (1.00 mL), and zinc chloride (11.50 mg) were added to a reaction flask. The mixture was reacted at 40°C for 1 hour. Then sodium cyanoborohydride (53.04 mg) was added, and the mixture was reacted for another 4 hours. The solvent was concentrated, diluted with water, and extracted with DCM and MeOH. The resultant was dried, concentrated, and then separated and purified by Pre-TLC to obtain compound 117-6 (52 mg) as a yellow solid. ESI-MS m/z: 618.8 1/2$[M+2H]^+$.

Step 7: Synthesis of compound 117

Compound 117-6 (51.00 mg), dichloromethane (1.00 mL), trifluoroacetic acid (1.00 mL) were added to a reaction flask. The mixture was reacted at room temperature for 0.6 hours. The resultant was concentrated and dissolved by adding DCM. The reaction solution was added to iced aqueous sodium bicarbonate solution until pH = 8. Then the solution was extracted with DCM and methanol, dried and concentrated. The resultant was purified by Pre-TLC with a developing agent of DCM:MeOH = 11:1 to obtain compound 117 (24 mg).

ESI-MS m/z: 596.66 1/2 $[M+2H]^+$.

$^1$H NMR (500 MHz, $CDCl_3$) $\delta$ 9.12 (d, *J*= 12.6 Hz, 1H), 8.65 (s, 1H), 8.52 - 8.35 (m, 1H), 8.11 (d, *J* = 27.8 Hz, 1H), 7.92 - 7.70 (m, 2H), 7.53 - 7.39 (m, 1H), 7.28 (m, 1H), 7.23 - 7.03 (m, 6H), 5.18 - 5.00 (m, 2H), 4.38 (m, 8H), 3.70 (m, 2H), 3.24 (d, *J* = 73.9 Hz, 3H), 2.77 (s, 2H), 2.49 (m, 8H), 2.33 (d, *J* = 8.2 Hz, 4H), 2.13 - 1.84 (m, 7H), 1.79 - 1.53 (m, 5H), 1.45 (t, *J* = 9.8 Hz, 3H), 1.34 - 1.17 (m, 5H), 1.06 (s, 3H), 0.75 (s, 6H).

Example 118: Synthesis of compound (2S,4R)-1-((S)-2-(4-(6-(7-(7-(8-ethyl-7-fluoro-3-hydroxynaphthalen-1-yl)-8-fluoro-4-(R)-3-hydr oxy-3-methylpiperidin-1-yl)pyrido[4,3-d]pyrimidin-2-yl)oxy)methyl)cyclopropyl)methyl)-2,7-di azaspiro [3.5]nonan-2-yl)pyridin-3-yl)-1H-1,2,3-triazol-1-yl)-3-methylbutanoyl)-4-hydroxy-N-(( S)-1-(4-methylthiazol-5-yl)phenylethyl)pyrrolidine-2-carboxamide

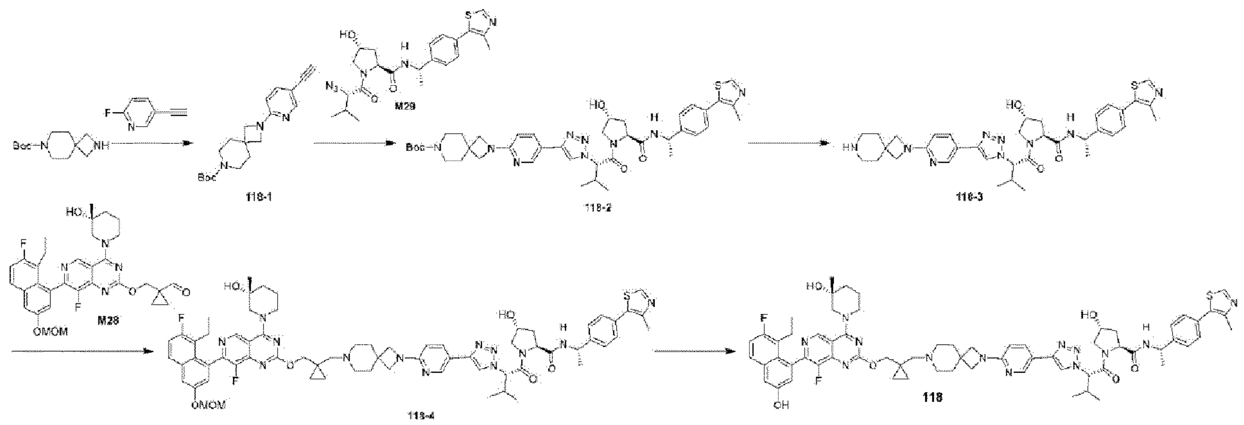


Step 1: Synthesis of compound 118-1

Tert-butyl 2,7-diazaspiro[3.5]nonane-7-carboxylate (280.00 mg), 5-ethynyl-2-fluoropyridine (224.76 mg), and cesium carbonate (1209.31 mg) were added to DMSO (3.00 mL), and the mixture was reacted at 50°C for 3 hours. 40 mL of water was added to the reaction solution. The solution was extracted twice with EA, and dried with anhydrous sodium sulfate. The resultant was separated by column chromatography (PE:EA = 75%:25%) to obtain compound 118-1 (290.00 mg, 71.59%).
ESI-MS m/z: 328.33 $[M+H]^+$.

Step 2: Synthesis of compound 118-2

Compound 118-1 (121.90 mg), compound M29 (170.00 mg), sodium ascorbate (184.40 mg), and copper sulfate (59.42 mg) were added to THF/tetrahydrofuran (2.00 mL), water (2.00 mL), and tert-butanol (2.00 mL). The mixture was reacted at room temperature for 3 hours. 50 mL of water was added to the reaction solution. The solution was extracted twice with EA, and dried with anhydrous sodium sulfate. The resultant was separated by column chromatography (DCM:MeOH = 93%:7%) to obtain compound 118-2 (230 mg, 78.80%).
ESI-MS m/z: 784.61 $[M+H]^+$.

Step 3: Synthesis of compound 118-3

Compound 118-2 (230.00 mg) and trifluoroacetic acid (1.00 mL) were sequentially added to dichloromethane (2.00 mL). The mixture was reacted at room temperature for 1 hour. 40 mL of saturated sodium bicarbonate solution was added to the reaction solution to adjust to alkaline. The solution was extracted twice with DCM, dried with anhydrous sodium sulfate, and concentrated to obtain compound 118-3 (200.00 mg, 99.68%).
ESI-MS m/z: 684.50 $[M+H]^+$.

Step 4: Synthesis of compound 118-4

Compound 118-3 (190.00 mg), compound M28 (164.63 mg), and 1.0 M zinc chloride in tetrahydrofuran (0.56 mL, 1.00 mol/L) were added to methanol (3.00 mL) and 1,2-dichloroethane (3.00 mL). The mixture was reacted at 60°C for 2 hours, and then sodium cyanoborohydride (87.28 mg) was added, followed by reacting at 60°C for 3 hours. The reaction solution was concentrated, and 30 mL of water was added. The solution was extracted twice with DCM, and dried with anhydrous sodium sulfate. The resultant was separated by column chromatography (DCM:MeOH = 90%:10%) to obtain compound 118-4 (100.00 mg, 28.56%).
ESI-MS m/z: 631.21 $1/2[M+2H]^+$.

Step 5: Synthesis of compound 118

Compound 118-4 (100.00 mg) and TFA/trifluoroacetic acid (1.00 mL) were added to dichloromethane (2.00 mL). The mixture was reacted at room temperature for 1 hour. The reaction solution was separated and purified by Pre-HPLC to obtain compound 118 (42.40 mg, 43.40%).

ESI-MS m/z: 609.32 1/2[M+2H]$^+$.

$^1$H NMR (500 MHz, Methanol-$d_4$) δ 9.11 (d, J = 5.8 Hz, 1H), 8.76 (d, J = 6.8 Hz, 1H), 8.38 (d, J = 1.7 Hz, 1H), 8.28 (d, J = 2.7 Hz, 1H), 7.83 (dt, J = 6.9, 3.1 Hz, 1H), 7.57 (dd, J = 9.0, 5.8 Hz, 1H), 7.37 7.26 (m, 4H), 7.20 (d, J = 2.6 Hz, 1H), 7.14 (t, J = 9.3 Hz, 1H), 6.97 (t, J = 2.7 Hz, 1H), 6.36 (dd, J = 8.8, 3.3 Hz, 1H), 5.23 (d, J = 10.3 Hz, 1H), 4.95 (q, J = 7.0 Hz, 1H), 4.46 - 4.29 (m, 5H), 4.15 (t, J = 13.4 Hz, 1H), 3.80 (dt, J = 25.0, 7.5 Hz, 2H), 3.65 (s, 4H), 3.55 (dd, J = 12.3, 4.8 Hz, 1H), 3.36 (q, J = 10.5 Hz, 1H), 2.60 - 2.30 (m, 11H), 2.17 - 2.01 (m, 3H), 1.93 (s, 1H), 1.87 (ddd, J = 19.5, 9.9, 5.3 Hz, 1H), 1.79 - 1.65 (m, 7H), 1.42 (d, J = 7.0 Hz, 3H), 1.22 - 1.15 (m, 6H), 1.05 (t, J = 8.2 Hz, 3H), 0.75 - 0.68 (m, 6H), 0.64 (s, 2H), 0.43 (s, 2H).

Example 119: Synthesis of compound (2S,4R)-1-((S)-2-(4-(1-((7-(8-ethyl-7-fluoro-3-hydroxynaphthalen-1-yl)-8-fluoro-4-((R)-3-hydro xy-3-methylpiperidin-1-yl)pyrido[4,3-d]pyrimidin-2-yl)oxy)methyl)cyclopropyl)methyl)piperidi n-4-yl) phenyl)-1H-1,2,3-triazol-1-yl)-3,3-dimethylbutanoyl)-4-hydroxy-N-((S)-1-(4-(4-methylthi azol-5-yl)phenyl)ethyl)pyrrolidine-2-carboxamide

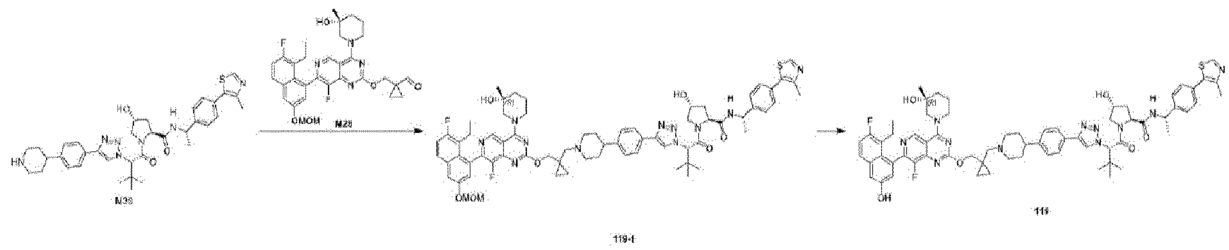

M39 119-1 119

Step 1: Synthesis of compound 119-1

Compound M39 (0.155 mg) and compound M28 (0.100 g) were dissolved in anhydrous methanol (4.00 mL), 1 M zinc chloride in diethyl ether (0.337 mL) was added. The mixture was reacted at 40°C for 1 hour. Sodium cyanoborohydride (106 mg) was then added, and the mixture reacted at 40°C for 4 hours. The reaction solution was quenched by adding 50 mL of saturated aqueous sodium bicarbonate solution. The solution was extracted three times with a mixed solvent of DCM/MeOH = 10/1, and the organic phases were combined, dried with anhydrous sodium sulfate. The resultant was concentrated under reduced pressure, and then purified by column chromatography (DCM/MeOH = 10/1) to obtain 0.150 g of a yellow solid product, i.e., compound 119-1. ESI-MS m/z: 617.3 1/2[M+2H]$^+$;

Step 2: Synthesis of compound 119

Compound 119-1 (150 mg) was dissolved in 5.00 mL of dichloromethane at room temperature, and then 2 mL of trifluoroacetic acid was added. The mixture was reacted at room temperature for 1 hour. The solvent was removed by evaporation under reduced pressure, the residue was redissolved in dichloromethane, and the solvent was evaporated again. The residue was then redissolved in 5.00 mL of THF, followed by the addition of 1.00 mL of water. Solid sodium carbonate was added, and the mixture was stirred for 20 minutes. The resultant was dried with anhydrous sodium sulfate, filtered, and concentrated under reduced pressure, and purified by Pre-HPLC to obtain 66.5 mg of a white solid product, i.e., compound 119.

ESI-MS m/z: 595.3 1/2[M+2H]$^+$.

$^1$H NMR (500 MHz, Methanol-$d_4$) δ 9.22 (s, 1H), 8.91 - 8.83 (m, 1H), 8.57 - 8.46 (m, 1H), 7.79 - 7.62 (m, 3H), 7.43 (td, *J*= 13.1, 12.4, 8.2 Hz, 3H), 7.34 - 7.18 (m, 4H), 7.05 (dt, *J* = 6.5, 3.2 Hz, 1H), 5.57 (s, 1H), 5.05 (p, *J* = 6.5, 6.0 Hz, 1H), 4.60 - 4.37 (m, 5H), 4.25 (t, *J*= 15.0 Hz, 1H), 3.93 - 3.77 (m, 2H), 3.63 (dd, *J* = 34.6, 13.4 Hz, 2H), 3.48 (t, *J* = 11.2 Hz, 1H), 3.22 (dd, *J*= 21.4, 11.5 Hz, 3H), 2.54 (s, 2H), 2.47 - 2.39 (m, 2H), 2.27 - 2.01 (m, 5H), 1.76 (dd, *J* = 23.1, 11.4 Hz, 5H), 1.55 (dd, *J* = 7.0, 1.9 Hz, 2H), 1.33 - 1.21 (m, 4H), 1.12 (s, 3H), 0.85 - 0.67 (m, 5H), 0.53 (s, 2H).

Example 120: Synthesis of compound (2S,4R)-1-((S)-2-(5-(((3S,7aS)-7a-(((7-(8-ethyl-7-fluoro-3-hydroxynaphthalen-1-yl)-8-fluoro-4-( (R)-3-hydroxy-3-methylpiperidin-1-yl)pyrido[4,3-d]pyrimidin-2-yl)oxy)methyl)-6-methylenehexahydro-1H-pyrrolidin-3-yl)methoxy)methyl)-1H-1,2,3-triazol-1-yl)-3-methylbutanoyl)-4-hydrox y-N-(((S)-1-(4-(4-methylthiazol-5-yl)phenyl)ethyl)pyrrolidine-2-carboxamide

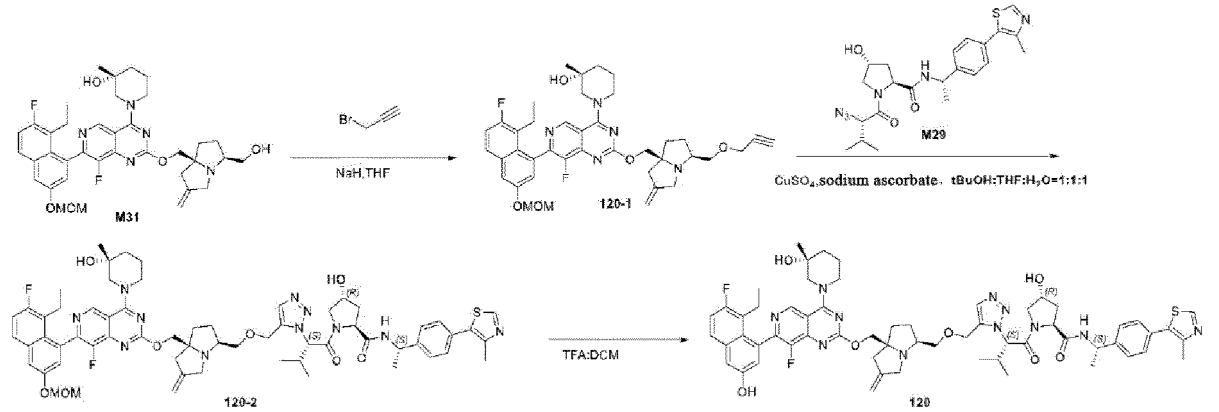


Step 1: Synthesis of compound 120-1

Compound M31 (100 mg) was dissolved in 3 mL of THF, and NaH (12 mg) was added. The mixture was stirred at room temperature for 30 minutes, and then 3-bromopropyne (15 μL) was added. A nitrogen balloon was inserted and the mixture was stirred at 25°C for 2 hours. The reaction was monitored by LC-MS until completion. The reaction solution was quenched by adding 10 mL of saturated ammonium chloride solution slowly. The solution was extracted three times with EA, and the organic phases were combined, dried with anhydrous sodium sulfate. The solvent was removed by evaporation under reduced pressure, and then the resultant purified by column chromatography to obtain 65 mg of a light yellow solid, i.e., compound 120-1. ESI-MS m/z: 714 $[M+H]^+$;

Step 2: Synthesis of compound 120-2

Compound 120-1 (65 mg) and compound M29 (50 mg) were dissolved in 2 mL of THF, 2 mL of $H_2O$, and 2 mL of tert-butanol. Then sodium ascorbate (47 mg) and anhydrous copper sulfate (13 mg) were sequentially added. The mixture was reacted at room temperature for 0.5 hours. The reaction was monitored by LCMS until completion. 20.0 mL of water was added to reaction solution, and the solution was extracted three times with DCM:MeOH = 4:1, 20 mL each time. The organic phases were combined, dried with anhydrous sodium sulfate, filtered and concentrated. The resultant was separated and purified by column chromatography to obtain 54 mg of a light yellow solid, i.e., compound 120-2. ESI-MS m/z: 586 1/2$[M+2H]^+$.

Step 3: Synthesis of compound 120

Compound 120-2 (54 mg) was dissolved in 2 mL of DCM, then 1 mL of TFA was added. The mixture was reacted at room temperature for 30 minutes. The reaction was monitored by LCMS until completion. The reaction was monitored by LCMS until completion. The reaction solution was concentrated directly, and the resultant was separated by preparative liquid chromatography to obtain 18.4 mg of a white powder, i.e., compound 120.

ESI-MS m/z: 564 1/2$[M+2H]^+$.

$^1$H NMR (500 MHz, Methanol-$d_4$) δ 9.21 - 9.20 (m, 1H), 8.87 (s, 1H), 8.14 (s, 1H), 7.68-7.65 (m, 1H), 7.44 - 7.39 (m, 4H), 7.30 - 7.29 (m, 1H), 7.26 - 7.21 (m, 1H), 7.07 - 7.06(m, 1H), 5.31 (d, $J$ = 10.2 Hz, 1H), 4.62 - 4.60 (m, 10H), 4.54 - 4.45 (m, 4H), 4.39 - 4.17 (m, 3H), 3.90-3.80 (m, 2H), 3.73 - 3.57 (m, 2H), 3.51 - 3.39 (m, 3H), 3.02-3.01 (m, 1H), 2.57 - 2.51 (m, 1H), 2.48 - 2.39 (m, 4H), 2.29 - 2.04 (m, 6H), 1.97 - 1.91 (m, 1H), 1.89-1.85 (m, 1H), 1.78-1.71 (m, 2H), 1.69-1.62 (m, 2H), 1.53 - 1.49 (m, 2H), 1.30 - 1.27 (m, 5H), 1.14 - 1.08 (m, 3H), 0.83 - 0.73 (m, 6H).

Example 121: Synthesis of compound (2S,4R)-1-((S)-2-(4-(2-((3S,7aS)-7a-(((7-(7-(8-ethyl-7-fluoro-3-hydroxynaphthalen-1-yl)-8-fluor o-4-((R)-3-hydroxy-3-methylpiperidin-1-yl)pyrido[4,3-d]pyrimidin-2-yl)oxy)methyl)-6-methyen ehexahydro-1H-pyrrolizin-3-yl)methoxy)pyridin-4-yl)-1H-1,2,3-triazol-1-yl)-4-hydroxy-N-((S)-1-(4-(4-methylthiazol-5-yl)phenyl) ethyl)pyrrolidine-2-carboxamide

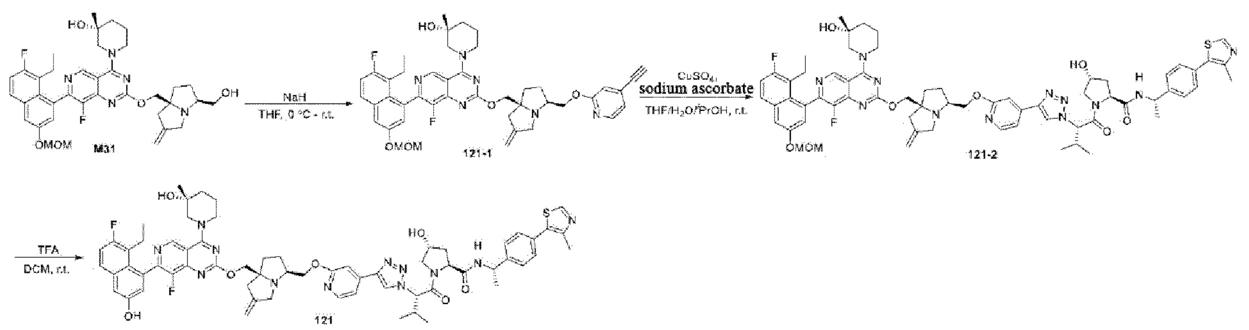


Step 1: Synthesis of compound 121-1

Sodium hydride (26.6 mg) and compound M31 (150.0 mg) were dissolved in THF (5 mL). The mixture was stirred for 0.5 hours. 4-Ethynyl-2-fluoropyridine (35.0 mg) was added to the reaction mixture, and the mixture was reacted at room temperature for 3 hours. The reaction was monitored by LCMS until completion. DCM (10 mL) was added to the reaction solution, and the resultant was washed once with saturated brine. The organic phase was dried with anhydrous sodium sulfate, filtered, concentrated, and then separated and purified by column chromatography to obtain 23.0 mg of a yellow solid, i.e., compound 121-1. ESI-MS m/z: 777.9 $[M+H]^+$.

Step 2: Synthesis of compound 121-2

Compound 121-1 (23.0 mg), (2S,4R)-1-((S)-2-azido-3-methylbutanoyl)-4-hydroxy-N-((S)-1-(4-(4-methylthiazol-5-yl)phenyl) ethyl)pyrrolidine-2-carboxamide (13.5 mg), anhydrous copper sulfate (4.3 mg), and sodium ascorbate (15.3 mg) were dissolved in a mixed solvent of THF/tert-butanol/water (1/1/1 mL). The mixture was reacted at room temperature for 1 hour. The reaction was monitored by LCMS until completion. DCM (10 mL) was added to the reaction solution, and the resultant was washed once with saturated brine. The organic phase was dried with anhydrous sodium sulfate, filtered, concentrated, and then separated and purified by column chromatography to obtain 15.0 mg of a yellow solid, i.e., compound 121-2. ESI-MS m/z: 618.3 1/2$[M+2H]^+$.

Step 3: Synthesis of compound 121

Compound 121-2 (15.0 mg) was added to DCM (2 mL) and TFA (1 mL), and the mixture was reacted at room temperature for 10 minutes. The reaction was monitored by LCMS until completion. The reaction solution was concentrated directly, and the resultant was separated by preparative liquid chromatography to obtain 5.7 mg of a white powder, i.e., compound 121.

ESI-MS m/z: 595.8 1/2$[M+2H]^+$.

$^1$H NMR (500 MHz, Methanol-$d_4$) δ 9.21 - 9.20 (m, 1H), 8.87 (s, 1H), 8.14 (s, 1H), 7.68-7.65 (m, 1H), 7.44 - 7.39 (m, 4H), 7.30 - 7.29 (m, 1H), 7.26 - 7.21 (m, 1H), 7.07 - 7.06(m, 1H), 5.31 (d, $J$ = 10.2 Hz, 1H), 4.62 - 4.60 (m, 10H), 4.54 - 4.45 (m, 4H), 4.39 - 4.17 (m, 3H), 3.90-3.80 (m, 2H), 3.73 - 3.57 (m, 2H), 3.51 - 3.39 (m, 3H), 3.02-3.01 (m, 1H), 2.57 - 2.51 (m, 1H), 2.48 - 2.39 (m, 4H), 2.29 - 2.04 (m, 6H), 1.97 - 1.91 (m, 1H), 1.89-1.85 (m, 1H), 1.78-1.71 (m, 2H), 1.69-1.62 (m, 2H), 1.53 - 1.49 (m, 2H), 1.30 - 1.27 (m, 5H), 1.14 - 1.08 (m, 3H), 0.83 - 0.73 (m, 6H).

Example 122: Synthesis of compound (2S,4R)-1-((S)-2-(4-(4-(((3S,7aS)-7a-((((7-(8-ethyl-7-fluoro-3-hydroxynaphthalen-1-yl)-8-fluoro -4-((R)-3-hydroxy-3-methylpiperidin-1-yl)pyrrolopyrimidin-2-yl)oxy)methyl)-[4,3-d]pyrimidin-2-yl)butanoyl)-4-hydroxy-N-((S)-1-(4-(4-methylthiazol-5-yl)phenyl)ethyl)pyrrolidine-2-carboxa mide

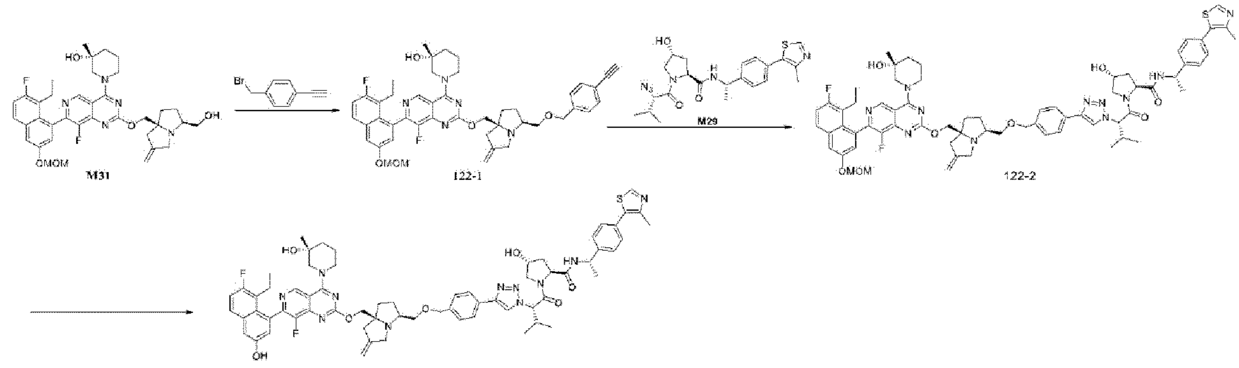


M31 122-1 122-2

122

Step 1: Synthesis of compound 122-1

140 mg of (R)-1-(7-(8-ethyl-7-fluoro-3-(methoxymethoxy)naphthalen-1-yl)-8-fluoro-2-(((5S,7aS)-5-(hydro xymethyl)-2-methyltetrahydro-1H-pyrrolizin-7a(5H)-yl)methoxy)pyrido[4,3-d]pyrimidin-4-yl)-3 -methylpiperidin-3-ol (compound M31) and 10 mL of THF were added to a 50 mL flask. The mixture was cooled to 0°C, then 41 mg of NaH was added. The mixture was warmed to room temperature and reacted for 0.5 hours. Then, 1-(bromomethyl)-4-ethynylbenzene (80 mg) was added, and the mixture was heated to 45°C and reacted for 1 hour. After cooling to 0°C, the reaction was quenched with saturated ammonium chloride solution and the reaction solution was extracted with EA. The organic phase was washed with water and brine, dried with sodium sulfate, and filtered. The filtrate was precipitated under reduced pressure to obtain the crude product. The resultant was purified by column chromatography to obtain compound 122-1 (49 mg).

Step 2: Synthesis of compound 122-2

Compound 122-1 (49 mg), (2S,4R)-1-((S)-2-azido-3-methylbutanoyl)-4-hydroxy-N-((S)-1-(4-(4-methylthiazol-5-yl)phenyl) ethyl)pyrrolidine-2-carboxamide (M29) (32 mg), sodium ascorbate (33 mg), and copper sulfate (10 mg), 1 mL of tert-butanol, 1 mL of THF, and 1 mL of water were added to a 50 mL flask. The mixture was reacted at room temperature for 1 hour. The reaction was monitored until completion. Then the resultant was extracted with EA, and the organic phase was washed with water and brine, dried with sodium sulfate, and filtered. The filtrate was precipitated under reduced pressure to obtain the crude product, which was purified by column chromatography to obtain compound 122-2 (32 mg).

Step 3: Synthesis of compound 122

Compound 122-2 (32 mg), 3 mL of DCM, and 1 mL of TFA were added to a 50 mL flask. The mixture was reacted at room temperature for 0.5 hours. The reaction was monitored, then the solvent was removed under reduced pressure. The resultant was dissolved by adding DCM, and saturated aqueous sodium bicarbonatesolution was added until pH = 7-8. The mixture was stirred, and the layers were separated, washed with brine, dried with sodium sulfate, and filtered. The filtrate was subjected to solvent removal under reduced pressure to obtain the crude product, which was purified by Pre-HPLC to obtain compound 122 (13.3 mg).

ESI-MS m/z: 602.14 1/2[M+2H]$^+$.

Example 123: Synthesis of compound (2S,4R)-1-((S)-2-(4-(1-(((3S,7aS)-7a-(((7-(8-ethyl-7-fluoro-3-hydroxynaphthalen-1-yl)-8-fluoro-4-((R)-3-hydroxy-3-methylpiperidin-1-yl)pyrido[4,3-d]pyrimidin-2-yl)oxy)methyl)-6-methylene hexahydro-1H-pyrrolizin-3-yl)methyl)piperidin-4-yl)-1H-1,2,3-triazol-1-yl)-3-methylbutanoyl)-4-hydroxy-N-(((S)-1-(4-(4-methylthiazol-5-yl)phenyl)ethyl)pyrrolidine-2-carboxamide

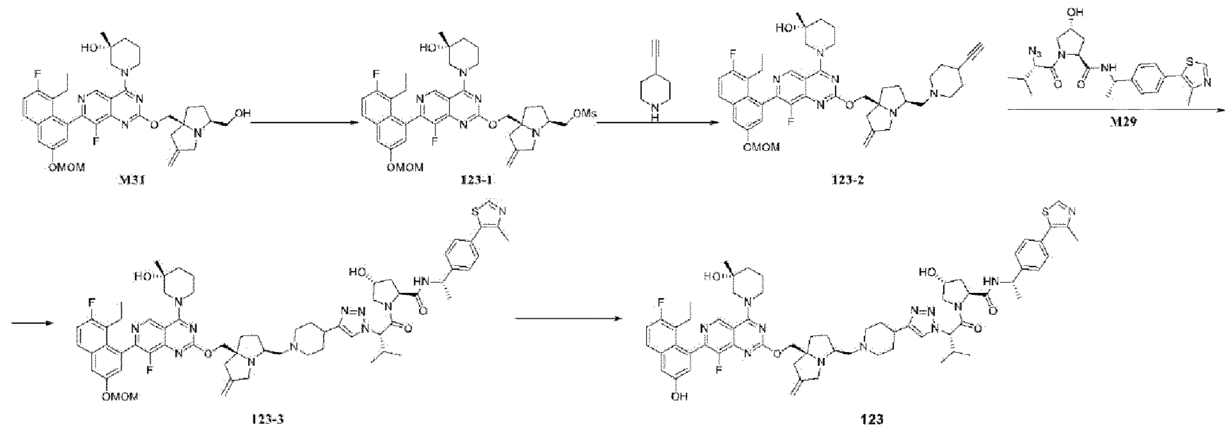

M31 123-1 123-2 M29 123-3 123

Step 1: Synthesis of compound 123-1

Compound M31 (100 mg), 2 mL of DCM, 75 mg of TFA and 23 μL of methanesulfonyl chloride were added to a 10 mL flask. The mixture was reacted at room temperature for 2 hours. The resultant was diluted with DCM and washed with saturated aqueous sodium bicarbonate solution. The layers were separated, dried with sodium sulfate, and filtered. The filtrate was subjected to solvent removal under reduced pressure to obtain the crude product, which was purified by column chromatography to obtain compound 123-1 (45 mg).

Step 2: Synthesis of compound 123-2

Compound 123-1 (45 mg), 2 mL of DCM, cesium carbonate (145 mg) and 4-ethynylpiperidine (43 mg) were added to a 10 mL flask. The mixture was heated to 60°C and reacted for 3 hours. The resultant was diluted with EA, washed with water and brine, then dried with sodium sulfate and filtered. The filtrate was subjected to solvent removal under reduced pressure to obtain a crude product, which was purified by column chromatography to obtain compound 123-2 (39 mg).

Step 3: Synthesis of compound 123-3

Compound 123-2 (39 mg), compound M29 (2S,4R)-1-((S)-2-azido-3-methylbutanoyl)-4-hydroxy-N-((S)-1-(4-(4-methylthiazol-5-yl)phenyl) ethyl)pyrrolidine-2-carboxamide (32 mg), sodium ascorbate (33 mg), copper sulfate (10 mg), 1 mL of tert-butanol, 1 mL of THF, and 1 mL of water were added to a 10 mL flask. The mixture was reacted at room temperature for 1 hour. The reaction was monitored until completion. Then the resultant was extracted with EA, and the organic phase was washed with water and brine, dried with sodium sulfate, and filtered. The filtrate was subjected to solvent removal under reduced pressure to obtain a crude product, which was purified by column chromatography to obtain compound 123-3 (25 mg).

Step 4: Synthesis of compound 123

Compound 123-3 (25 mg), 3 mL of DCM, and 1 mL of TFA were added to a 10 mL flask. The mixture was reacted at room temperature for 0.5 hours. The reaction was monitored, then the solvent was removed under reduced pressure, and saturated aqueous sodium bicarbonate solution was added until pH = 7-8. The mixture was stirred, and the layers were separated, dried with sodium sulfate, and filtered. The filtrate was subjected to solvent removal under reduced pressure to obtain a crude product 123, which was purified by Pre-HPLC to obtain compound 123 (1.7 mg).
ESI-MS m/z: 590.80 1/2$[M+2H]^+$.

Example 124: Synthesis of compound (2S,4R)-1-((S)-2-(4-(6-(((3S,7aS)-7a-((7-(8-ethyl-7-fluoro-3-hydroxynaphthalen-1-yl)-8-fluoro-4-((R)-3-hydroxy-3-methylpiperidin-1-yl)pyrido[4,3-d]pyrimidin-2-yl)oxy)methyl)-6-methylene hexahydro-1H-pyrrolizin-3-yl)methoxy)pyridin-3-yl)-1H-1,2,3-triazol-1-yl)-3-methylbutanoyl)-4-hydroxy-N-((S)-1-(4-(4-methylthiazol-5-yl)phenyl)ethyl)pyrrolidine-2-carboxamide

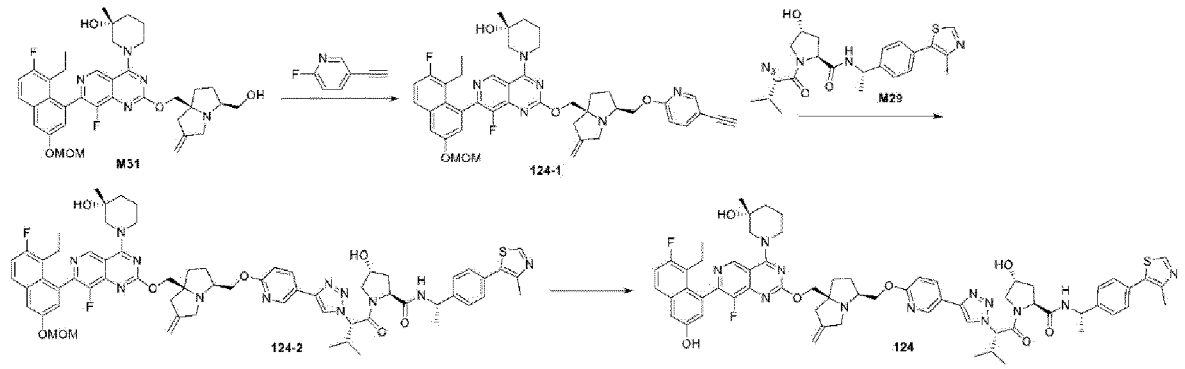

M31 124-1 M29 124-2 124

Step 1: Synthesis of compound 124-1

Sodium hydride (26.64 mg, 60%, 0.67 mmol) was added to a solution of compound M31 (150.00 mg, 0.22 mmol) in tetrahydrofuran (6.00 mL), which was cooled in an ice bath under the protection of nitrogen. The mixture was reacted at 0°C for 0.5 hours, followed by the addition of 5-ethynyl-2-fluoropyridine (53.77 mg, 0.44 mmol). The mixture was then reacted at 20°C for 4 hours. The reaction solution was added to 20 mL of saturated ammonium chloride solution, and 15 mL of EA was added. The organic phase was separated, and the aqueous phase was further extracted once with 5 mL of EA. The organic phases were combined, dried, filtered, and concentrated under reduced pressure. The resultant was separated and purified by column chromatography to obtain 85 mg of a yellow foam solid, i.e., compound 124-1. ESI-MS m/z: 777.30 $[M+H]^+$.

Step 2: Synthesis of compound 124-2

Copper sulfate (12.32 mg) and sodium ascorbate (45.88 mg) in water (0.75 mL) were added to a solution of compound 124-1 (60.00 mg) and compound M29 (35.25 mg) in tert-butanol (1.50 mL) and THF (1.50 mL). The mixture was reacted at 20°C for 1 hour. The reaction was monitored by LCMS until completion. The reaction solution was added to 20 mL of water and extracted with EA. The organic phase was dried, filtered, and concentrated under reduced pressure. The residue was purified by column chromatography (MEOH:DCM = 1% to 7%) to obtain 75.00 mg of a yellow foam solid, i.e., compound 124-2. ESI-MS m/z: 617.7 1/2$[M+2H]^+$.

Step 3: Synthesis of compound 124

Trifluoroacetic acid (0.96 mL) was added to a solution of compound 124-2 (80.00 mg) in dichloromethane (4.00 mL). The mixture was reacted at 20°C for 0.5 hours. The reaction was monitored by LCMS until completion. The reaction solution was concentrated without heating, and purified by Pre-HPLC to obtain 31.80 mg of a white powder, i.e., compound 124.

ESI-MS m/z: 595.4 1/2$[M+2H]^+$.

1H NMR (500 MHz, DMSO-$d_6$) δ 9.95 (s, 1H), 9.21 (s, 1H), 8.99 (s, 1H), 8.72 (s, 1H), 8.67 (s, 1H), 8.54-8.52 (m, 1H), 8.19-8.17 (m, 1H), 7.78-7.75 (m, 1H), 7.46-7.44 (m, 2H), 7.38-7.36 (m, 2H), 7.03 (s, 1H), 6.91-6.88 (m, 1H), 5.34 (s, 1H), 5.19-5.18 (m, 1H), 4.94-4.91 (m, 1H), 4.77-4.75 (s, 1H), 4.42-4.38 (m, 3H), 4.23-4.20 (m, 1H), 4.16-4.11 (m, 2H), 4.03-3.96 (m, 2H), 3.79-3.69 (m, 2H), 2.39-2.34 (m, 3H), 2.12-1.99 (m, 8H), 1.67-1.62 (m, 3H), 1.39-1.34 (m, 5H), 1.17 (s, 3H), 1.15 (s, 3H), 1.08 (s, 3H), 0.72 (s, 6H).

Example 125: Synthesis of compound (3S,7aS)-7a-((7-(7-(8-ethyl-7-fluoro-3-hydroxynaphthalen-1-yl)-8-fluoro-4-((R)-3-hydroxy-3-m ethylpiperidin-1-yl)pyrido[4,3-d]pyrimidin-2-yl)pyrimidin-2-yl)oxy)methyl)-6-methylenehexahy dro-1H-pyrrolizin-3-yl)methyl-4-(((5-(R)-1-(2S,4R)-4-hydroxy-2-(((S)-1-(4-(4-methylthiazol-5-yl)phenyl)ethyl)carbamoyl)pyrrolidin-1-yl)-3-methyl-1-oxobutan-2-yl)isoxazol-3-yl)oxy)methyl )piperidine-1-carboxylate

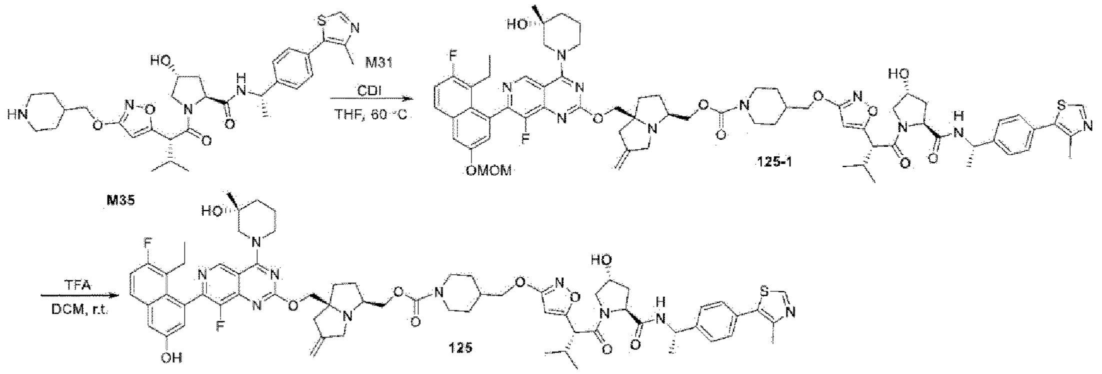


Step 1: Synthesis of compound 125-1

Compound M35 (30.0 mg) was dissolved in THF (2 mL), followed by the addition of CDI (10.8 mg). The mixture was reacted at 40°C for 1 hour, after which compound M31 (39.7 mg) was added, and the mixture was reacted at 60°C for 12 hours. The reaction was monitored by LCMS until completion. The reaction was quenched by adding methanol (5.0 mL). The reaction solution was concentrated directly, and then separated and purified by column chromatography to obtain 35.0 mg of a light yellow solid, i.e., compound 125-1. ESI-MS m/z: 650.3 1/2[M+2H]$^+$.

Step 2: Synthesis of compound 125

Compound 125-1 (35.0 mg) was added to DCM (5 mL) and TFA (2 mL), and the mixture was reacted at room temperature for 10 minutes. The reaction was monitored by LCMS until completion. The reaction solution was concentrated directly, and the resultant was purified by Pre-HPLC to obtain 15.3 mg of a white powder, i.e., compound 125.

ESI-MS m/z: 627.8 1/2[M+2H]$^+$.

$^1$H NMR (500 MHz, Methanol-$d_4$) δ 9.21 (d, *J* = 7.0 Hz, 1H), 8.87 (s, 1H), 7.68-7.65 (m, 1H), 7.44-7.36 (m, 4H), 7.29 (d, *J* = 2.5 Hz, 1H), 7.24 (t, *J* = 4.5 Hz, 1H), 7.09-7.06 (m, 1H), 5.98-5.92 (m, 1H), 5.26 (d, *J*= 10.0 Hz, 1H), 5.03 (d, *J*= 7.0 Hz, 1H), 4.52-4.49 (m, 2H), 4.43-4.40 (m, 1H), 4.34-4.24 (m, 2H), 4.17-4.13 (m, 2H), 4.07-4.01 (m, 2H), 3.96-3.92 (m, 1H), 3.84-3.81 (m, 1H), 3.70-3.57 (m, 4H), 3.48-3.42 (m, 2H), 3.07-3.04 (m, 1H), 2.93-2.83 (m, 3H), 2.71-2.68 (m, 1H), 2.47 (s, 3H), 2.43-2.35 (m, 2H), 2.23-2.15 (m, 5H), 2.06-1.92 (m, 4H), 1.85-1.77 (m, 6H), 1.51 (d, *J* = 7.0 Hz, 2H), 1.33-1.23 (m, 6H), 1.05 (d, *J* = 6.5 Hz, 3H), 0.91-0.87 (m, 3H), 0.83-0.79 (m, 3H).

Example 126: Synthesis of compound (7-(8-ethyl-7-fluoro-3-hydroxynaphthalen-1-yl)-8-fluoro-4-(R)-3-hydroxy-3-methylpiperidin-1-y l)pyrido[4,3-d]pyrimidin-2-yl)oxy)methyl)-6-methylenehexahydro-1H-pyrrolizin-3-yl)methyl ((3-(1-((S)-1-(2S,4R)-4-hydroxy-2-(S)-1-(4-methylthiazol-5-yl)phenyl)ethyl)carbamoyl)pyrrolidi n-1-yl)-3-methyl-1-oxobutan-2-yl)-1H-1,2,3-triazol-4-yl)bicyclo[1.1.1]pentan-1-yl)methyl)carba mate

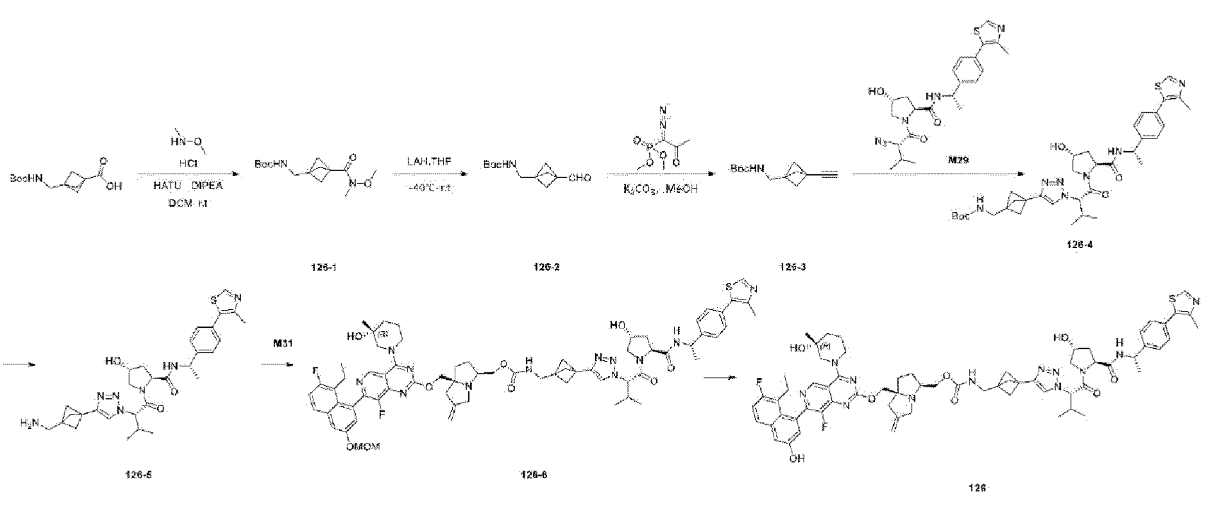

Step 1: Synthesis of compound 126-1

3-(((Tert-butoxycarbonyl)amino)methyl)bicyclo[1.1.1]pentane-1-carboxylic acid (1.00 g) and dimethylhydroxylamine hydrochloride (0.510 g) were dissolved in 10.0 mL of DCM at 0°C, followed by sequential addition of N,N-diisopropylethylamine (2.17 mL) and N,N,N',N'-tetramethyl-O-(7-azabenzotriazol-1-yl)uronium hexafluorophosphate (2.99 g). The mixture was reacted at room temperature for 3 hours. Then the reaction solution was quenched by slowly adding 50 mL of saturated sodium bicarbonate solution. The resultant was extracted three times with EA, and the organic phases were combined, washed three times with saturated sodium chloride, dried with anhydrous sodium sulfate, and concentrated under reduced pressure to obtain 1.40 g of a colorless oil product, i.e., compound 126-1. ESI-MS m/z: 285 $[M+H]^+$; $^1H$ NMR (500 MHz, Chloroform-*d*) δ 4.51 (s, 1H), 3.66 (s, 3H), 3.18 (s, 3H), 2.80 (s, 2H), 2.02 (s, 6H), 1.44 (s, 9H).

Step 2: Synthesis of compound 126-2

Compound 126-1 (1.40 g) was dissolved in 20.0 mL of THF and cooled to -40°C. Lithium aluminum hydride (0.330 g) was added, and the mixture was raised naturally to room temperature and reacted for 2 hours. The reaction solution was then cooled to 0°C, followed by sequential dropwise addition of 0.33 mL of water, 0.33 mL of 15% NaOH solution, and 0.33 mL of water. After stirring for 30 minutes, the resultant was filtered with diatomaceous earth to remove insoluble impurities. The filter cake was eluted three times with 20.0 mL of EA, and the filtrate was dried with anhydrous sodium sulfate, filtered, and concentrated under reduced pressure to obtain 0.910 g of a light yellow oil product, i.e., compound 126-2. $^1H$ NMR (500 MHz, Chloroform-*d*) δ 9.50 (s, 1H), 4.50 (s, 1H), 3.15 (s, 2H), 1.88 (s, 6H), 1.38 (s, 9H).

Step 3: Synthesis of compound 126-3

Compound 126-2 (0.910 g) was dissolved in 10.0 mL of anhydrous methanol under the protection of $N_2$, followed by sequential addition of anhydrous potassium carbonate (0.920 g) and dimethyl (1-diazo-2-oxopropyl)phosphonate (0.960 g). The mixture was reacted at room temperature for 2 hours. The reaction solution was concentrated under reduced pressure, and then separated and purified by column chromatography (PE/EA = 5/1) to obtain 0.480 g of a white solid, i.e., compound 126-3. $^1H$ NMR (500 MHz, Chloroform-*d*) δ 4.47 (s, 1H), 3.16 (s, 2H), 2.09 (s, 1H), 1.97 (s, 6H), 1.44 (s, 9H).

Step 4: Synthesis of compound 126-4

Compound 126-3 (0.400 g), compound M29 (2S,4R)-1-((S)-2-azido-3-methylbutanoyl)-4-hydroxy-N-((S)-1-(4-(4-methylthiazol-5-yl)phenyl) ethyl)pyrrolidine-2-carboxamide (0.818 g), sodium ascorbate (0.838 g), and anhydrous copper sulfate (0.234 g) were dissolved in a mixed solvent of 4.00 mL of tert-butanol, 4.00 mL of tetrahydrofuran and 4 mL of water. The mixture was reacted at room temperature for 1 hour. The reaction solution was quenched by adding 50 mL of water. The solution was extracted three times with a mixed solvent of DCM/MeOH = 10/1, and the organic phases were combined, dried with anhydrous sodium sulfate. The resultant was concentrated under reduced pressure, and then purified by column chromatography (DCM/MeOH = 10/1) to obtain 1.050 g of a yellow solid product, i.e., compound 126-4. ESI-MS m/z: 678 $[M+H]^+$;

Step 5: Synthesis of compound 126-5

Compound 126-4 (0.800 g) was dissolved in 10.0 mL of dichloromethane at room temperature, and then 3.00 mL of trifluoroacetic acid was added. The mixture was reacted at room temperature for 1 hour. The solvent was removed by evaporation under reduced pressure, the residue was redissolved in dichloromethane, and the solvent was evaporated again. The residue was then redissolved in 5.00 mL of tetrahydrofuran, followed by the addition of 1.00 mL of water. Solid sodium carbonate was added, and the mixture was stirred for 20 minutes. The resultant was dried with anhydrous sodium sulfate, filtered, concentrated under reduced pressure, and purified by column chromatography (DCM/MeOH = 10/1) to obtain 0.600 g of a yellow solid product, i.e., compound 126-5. ESI-MS m/z: 578 $[M+H]^+$;

Step 6: Synthesis of compound 126-6

Compound M31 (100 mg) was dissolved in tetrahydrofuran (2.00 mL) at room temperature, followed by sequential addition of triethylamine (0.165 mL), p-nitrophenyl chloroformate (60.0 mg), and 4-dimethylaminopyridine (5.50 mg). The mixture was reacted at room temperature overnight, then compound 126-5 (103 mg) was added, and reacted at room temperature for 0.5 hours. An appropriate amount of water was added to the reaction solution, and the solution was extracted three times with DCM, and the organic phases were combined, dried with anhydrous sodium

sulfate. The resultant was concentrated under reduced pressure, and then purified by column chromatography (DCM/MeOH = 10/1) to obtain 150 mg of a yellow solid product, i.e., compound 126-6. ESI-MS m/z: 640.7 1/2[M+2H]$^+$;

Step 7: Synthesis of compound 126

Compound 126-6 (150 mg) was dissolved in 5.00 mL of dichloromethane at room temperature, and then 2.00 mL of trifluoroacetic acid was added. The mixture was reacted at room temperature for 1 hour. The resultant was resultant under reduced pressure, the residue was redissolved in dichloromethane, and the solvent was evaporated again. The residue was then redissolved in 5.00 mL of tetrahydrofuran, followed by the addition of 1.00 mL of water. Solid sodium carbonate was added, and the mixture was stirred for 20 minutes. The resultant was dried with anhydrous sodium sulfate, filtered, concentrated under reduced pressure, and purified by Pre-HPLC to obtain 25.1 mg of a white solid product, i.e., compound 126.

ESI-MS m/z: 618.8 1/2[M+2H]$^+$;

$^1$H NMR (500 MHz, Methanol-$d_4$) δ 9.20 (s, 1H), 8.87 (d, *J* = 3.1 Hz, 1H), 7.90 (d, *J*= 11.8 Hz, 1H), 7.66 (dd, *J* = 9.1, 5.8 Hz, 1H), 7.42 (qd, *J* = 8.4, 7.6, 2.8 Hz, 4H), 7.29 (d, *J* = 2.7 Hz, 1H), 7.23 (t, *J* = 9.3 Hz, 1H), 7.06 (dd, *J* = 5.7, 2.6 Hz, 1H), 5.25 (d, *J* = 10.3 Hz, 1H), 5.03 (q, *J* = 6.5, 6.1 Hz, 1H), 4.94 (s, 2H), 4.55 - 4.40 (m, 3H), 4.36 - 4.22 (m, 2H), 4.17 (d, *J* = 10.6 Hz, 1H), 4.03 (d, *J*= 11.3 Hz, 1H), 3.93 (dd, *J* = 10.8, 6.0 Hz, 1H), 3.90 - 3.79 (m, 2H), 3.68 (dd, *J*= 29.6, 13.7 Hz, 2H), 3.51 - 3.38 (m, 2H), 3.23 (s, 2H), 3.04 (s, 1H), 2.84 (s, 1H), 2.70 (d, *J* = 15.5 Hz, 1H), 2.51 (d, *J*= 7.1 Hz, 2H), 2.41 (d, *J* = 15.6 Hz, 2H), 2.28 - 2.11 (m, 4H), 2.08 - 1.92 (m, 8H), 1.89 - 1.73 (m, 5H), 1.51 (d, *J*= 7.0 Hz, 3H), 1.28 (d, *J*= 13.8 Hz, 3H), 1.10 (t, *J* = 6.9 Hz, 3H), 0.81 (q, *J*= 7.2 Hz, 3H), 0.73 (d, *J*= 6.7 Hz, 3H).

Example 127: Synthesis of compound ((7-(8-ethyl-7-fluoro-3-hydroxynaphthalen-1-yl)-8-fluoro-4-(R)-3-hydroxy-3-methylpiperidin-1-yl)pyrido[4,3-d]pyrimidin-2-yl)oxy)methyl)-6-methylenehexahydro-1H-pyrrolizin-3-yl)methyl 2-(1-(S)-1-(2S,4R)-4-hydroxy-2-(S)-1-(4-(4-methylthiazol-5-yl)phenyl)ethylcarbamoyl)pyrrolidi n-1-yl)-3-methyl-1-oxobutan-2-yl)-1H-1,2,3-triazol-4-yl)-7-azaspiro[3.5]nonane-7-carboxylate

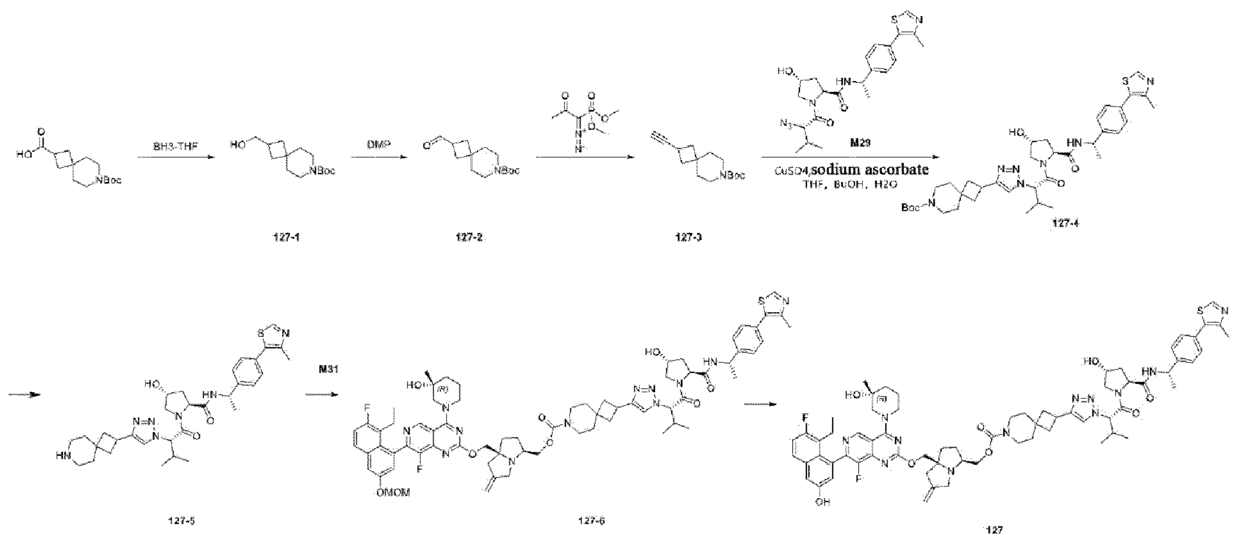


Step 1: Synthesis of compound 127-1

7-Tert-butoxycarbonyl-7-azaspiro[3.5]nonane-2-carboxylic acid (500.0 mg) was dissolved in 5.0 mL of THF, then borane in tetrahydrofuran solution (4.64 mL, 1 mol/L) was added. The mixture was reacted at room temperature for 1 hour. 5 mL of HCl/MeOH solution was slowly added to the reaction solution, and the reaction solution was stirred until no bubble generation. The pH was adjusted to alkaline with 1 mol/L NaOH, and the phases was extracted with an appropriate amount of ethyl acetate. The organic phase was collected, dried with anhydrous sodium sulfate, filtered, and the solvent was removed by evaporation under reduced pressure to obtain 450.0 mg of a colorless oil product, i.e., compound 127-1. ESI-MS m/z: 200 [M+H-56]$^+$.

Step 2: Synthesis of compound 127-2

Compound 127-1 (250.0 mg) was dissolved in 5.0 mL DCM, and Dess-Martin periodinane (387.0 mg) was added. The mixture was reacted at room temperature for 1 hour. An appropriate amount of ethyl acetate was added to the reaction solution, and the solid was precipitated and removed by filtration. The pH of the filtrate was adjusted to alkaline with 1 mol/L NaOH, the phases was extracted and separated, and the organic phase was collected, dried with anhydrous sodium sulfate, filtered, and the solvent was removed by evaporation under reduced pressure to obtain 180.0 mg of a colorless oil product, i.e., compound 127-2. ESI-MS m/z: 198 $[M+H-56]^+$.

Step 3: Synthesis of compound 127-3

Compound 127-2 (180 mg) was dissolved in 3.0 mL of anhydrous methanol under the protection of $N_2$, followed by addition of anhydrous potassium carbonate (167 mg) and dimethyl (1-diazo-2-oxopropyl)phosphonate (174 mg). The mixture was reacted at room temperature for 3 hours. An appropriate amount of water was added to the reaction solution, and the solution was extracted with ethyl acetate. The organic phase was collected, dried with anhydrous sodium sulfate, filtered, and the solvent was removed by evaporation under reduced pressure, and the resultant was purified by column chromatography to obtain 110 mg of a colorless oil product, i.e., compound 127-3. ESI-MS m/z: 194 $[M+H-56]^+$.

Step 4: Synthesis of compound 127-4

Compound 127-3 (120 mg), compound M29 (2S,4R)-1-((S)-2-azido-3-methylbutanoyl)-4-hydroxy-N-((S)-1-(4-(4-methylthiazol-5-yl)phenyl) ethyl)pyrrolidine-2-carboxamide (218 mg), sodium ascorbate (223 mg), and anhydrous copper sulfate (62.0 mg) were dissolved in a mixed solvent of 2.00 mL of tert-butanol, 2.00 mL of tetrahydrofuran and 2.00 mL of water. The mixture was reacted at room temperature for 1 hour. The reaction solution was quenched by adding 50 mL of water. The solution was extracted three times with a mixed solvent of DCM/MeOH = 10/1, and the organic phases were combined, dried with anhydrous sodium sulfate. The solvent was removed by evaporation under reduced pressure, and the resultant was then purified by column chromatography (DCM/MeOH = 10/1) to obtain 300 mg of a yellow solid product, i.e., compound 127-4. ESI-MS m/z: 706 $[M+H]^+$;

Step 5: Synthesis of compound 127-5

Compound 127-4 (300 mg) was dissolved in 5.00 mL of dichloromethane at room temperature, and then 2 mL of trifluoroacetic acid was added. The mixture was reacted at room temperature for 1 hour. The solvent was removed by evaporation under reduced pressure, the residue was redissolved in dichloromethane, and the solvent was evaporated again. The residue was then redissolved in 5.00 mL of tetrahydrofuran, followed by the addition of 1.00 mL of water. Solid sodium carbonate was added, and the mixture was stirred for 20 minutes. The resultant was dried with anhydrous sodium sulfate, filtered. The solvent was removed by evaporation under reduced pressure, and the resultant was purified by column chromatography (DCM/MeOH = 10/1) to obtain 225 mg of a yellow solid product, i.e., compound 127-5. ESI-MS m/z: 606 $[M+H]^+$;

Step 6: Synthesis of compound 127-6

Compound M31 (100 mg) was dissolved in tetrahydrofuran (2.00 mL) at room temperature, followed by sequential addition of triethylamine (0.165 mL), p-nitrophenyl chloroformate (60.0 mg), and 4-dimethylaminopyridine (5.50 mg). The mixture was reacted at room temperature overnight, then compound 127-5 (99.2 mg) was added, and reacted at room temperature for 0.5 hours. An appropriate amount of water was added to the reaction solution, and the solution was extracted three times with DCM, and the organic phases were combined, dried with anhydrous sodium sulfate. The solvent was removed by evaporation under reduced pressure, and the resultant was then purified by column chromatography (DCM/MeOH = 10/1) to obtain 155 mg of a yellow solid product, i.e., compound 127-6. ESI-MS m/z: 654.8 1/2$[M+2H]^+$;

Step 7: Synthesis of compound 127

Compound 127-6 (155 mg) was dissolved in 5.00 mL of dichloromethane at room temperature, and then 2 mL of trifluoroacetic acid was added. The mixture was reacted at room temperature for 1 hour. The solvent was removed by evaporation under reduced pressure, the residue was redissolved in dichloromethane, and the solvent was evaporated again. The residue was then redissolved in 5.00 mL of tetrahydrofuran, followed by the addition of 1.00 mL of water. Solid sodium carbonate was added, and the mixture was stirred for 20 minutes. The resultant was dried with anhydrous sodium

sulfate and filtered. The solvent was removed by evaporation under reduced pressure, and the resultant was then purified by Pre-HPLC to obtain 26.8 mg of a white solid product, i.e., compound 127.

ESI-MS m/z: 632.9 1/2 [M+2H]+;

$^1$H NMR (500 MHz, Methanol-$d_4$) δ 9.22 (d, *J* = 4.4 Hz, 1H), 8.87 (d, *J*= 3.5 Hz, 1H), 7.90 (s, 1H), 7.66 (dd, *J* = 9.1, 5.8 Hz, 1H), 7.42 (q, *J*= 8.6, 7.8 Hz, 3H), 7.34 - 7.22 (m, 2H), 7.07 (dd, *J*= 10.1, 2.6 Hz, 1H), 5.25 (d, *J*= 10.3 Hz, 1H), 5.12 - 5.01 (m. 3H), 4.94 (s, 5H), 4.59 (s, 3H), 4.52 (t, *J* = 8.5 Hz, 2H), 4.43 (d, *J* = 25.9 Hz, 1H), 4.30 (ddd, *J* = 29.2, 17.1, 12.1 Hz, 2H), 4.16 (d, *J* = 10.9 Hz, 1H), 4.06 (dd, *J* = 10.5, 4.9 Hz, 1H), 3.97 - 3.86 (m, 2H), 3.83 (s, 1H), 3.66 (dd, *J* = 24.0, 13.8 Hz, 2H), 3.61 - 3.55 (m, 1H), 3.46 (d, *J* = 12.2 Hz, 2H), 3.40 - 3.36 (m, 1H), 3.05 (s, 1H), 2.70 (d, *J* = 15.4 Hz, 1H), 2.55 (dt, *J*= 10.9, 6.5 Hz, 1H), 2.41 (d, *J= 15.8* Hz, 1H), 2.31 - 2.14 (m, 5H), 2.10 - 1.94 (m, 4H), 1.85 (s, 1H), 1.84 - 1.74 (m, 3H), 1.69 (d, *J*= 5.6 Hz, 2H), 1.54 (dd, *J* = 25.8, 6.3 Hz, 4H), 1.30 (dd, *J* = 15.5, 11.4 Hz, 5H), 1.12 (d, *J* = 6.7 Hz, 3H), 0.86 - 0.72 (m, 5H).

Example 128: Synthesis of compound (2S,4R)-1-((S)-2-(4-((1-((1-(((7-(8-ethyl-7-fluoro-3-hydroxynaphthalen-1-yl)-8-fluoro-4-((R)-3-hydroxy-3-methylpiperidin-1-yl)pyrido[4,3-d]pyrimidin-2-yl)oxy)methyl)cyclopropyl)methyl)pi peridin-4-yl)azetidin-3-yl)-1H-1,2,3-triazol-1-yl)-3-methylbutanoyl)-4-hydroxy-N-((S)-1-(4-(4-methylthiazol-5-yl)phenyl)ethyl)pyrrolidine-2-carboxamide

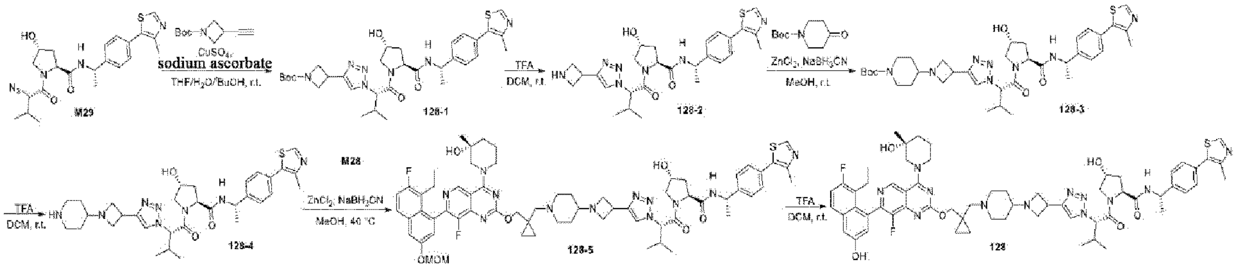


Step 1: Synthesis of compound 128-1

Compound M29 (1000.0 mg), tert-butyl 3-ethynylazetidine-1-carboxylate (396.9 mg), anhydrous copper sulfate (314.6 mg), and sodium ascorbate (1128.2 mg) were dissolved in a mixed solvent of THF/tert-butanol/water (10/10/10 mL). The mixture was reacted at room temperature for 3 hours. The reaction was monitored by LCMS until completion. DCM (30 mL) was added to the reaction solution, and the resultant was washed once with saturated brine. The organic phase was dried with anhydrous sodium sulfate, filtered, concentrated, and the separated and purified by column chromatography to obtain 628.0 mg of a yellow solid, i.e., compound 128-1. ESI-MS m/z: 638.3 [M+H]+.

Step 2: Synthesis of compound 128-2

Compound 128-1 (295.0 mg) was added to DCM (5 mL) and TFA (2 mL), and the mixture was reacted at room temperature for 10 minutes. The reaction was monitored by LCMS until completion. The reaction solution was concentrated directly, and 1 M NaOH solution was added until pH of the reaction solution reached 8. DCM (20 mL) was added to the reaction solution, and the organic phase was collected, washed once with saturated brine, dried with anhydrous sodium sulfate, filtered, and concentrated to obtain 165.0 mg of a white solid, i.e., compound 128-2. ESI-MS m/z: 538.3 [M+H]+.

Step 3: Synthesis of compound 128-3

Compound 128-2 (165.0 mg) and N-tert-butoxycarbonyl-4-piperidone (61.2 mg) were dissolved in methanol (5 mL), 1 M zinc chloride in diethyl ether (0.46 mL) was added. The mixture was reacted at 40°C for 1 hour. Sodium cyanoborohydride (57.9 mg) was then added, and the mixture reacted at 40°C for 10 minutes. The reaction was monitored by LCMS until completion. The reaction was quenched by adding saturated sodium bicarbonate solution (5.0 mL). DCM (20 mL) was added to the reaction solution, and the organic phase was collected, washed once with saturated brine, dried with anhydrous sodium sulfate, filtered, and concentrated to obtain 120.0 mg of a white solid, i.e., compound 128-3. ESI-MS m/z: 721.3 [M+H]+.

Step 4: Synthesis of compound 128-4

Compound 128-3 (120.0 mg) was added to DCM (5 mL) and TFA (2 mL), and the mixture was reacted at room temperature for 10 minutes. The reaction was monitored by LCMS until completion. The reaction solution was concentrated directly, and 1 M NaOH solution was added until pH of the reaction solution reached 8. DCM (20 mL) was added to the reaction solution, and the organic phase was collected, washed once with saturated brine, dried with anhydrous sodium sulfate, filtered, and concentrated to obtain 80.0 mg of a white solid, i.e., compound 128-4. ESI-MS m/z: 621.3 $[M+H]^+$.

Step 5: Synthesis of compound 128-5

Compound 128-4 (80.0 mg) and compound M28 (76.4 mg) were dissolved in methanol (5 mL), 1 M zinc chloride in diethyl ether (0.19 mL) was added. The mixture was reacted at 40°C for 1 hour. Sodium cyanoborohydride (24.3 mg) was then added, and the mixture reacted at 60°C for 12 hours. The reaction was monitored by LCMS until completion. The reaction was quenched by adding saturated sodium bicarbonate solution (5.0 mL). DCM (20 mL) was added to the reaction solution, and the organic phase was collected, washed once with saturated brine, dried with anhydrous sodium sulfate, filtered, and concentrated to obtain 84.0 mg of a white solid, i.e., compound 128-5. ESI-MS m/z: 600.3 $1/2[M+2H]^+$.

Step 6: Synthesis of compound 128

Compound 128-5 (84.0 mg) was added to DCM (5 mL) and TFA (2 mL), and the mixture was reacted at room temperature for 10 minutes. The reaction was monitored by LCMS until completion. The reaction solution was concentrated directly, and the resultant was separated by preparative liquid chromatography to obtain 28.9 mg of a white powder, i.e., compound 128.

ESI-MS m/z: 578.2 $1/2[M+2H]^+$.

$^1$H NMR (500 MHz, Methanol-$d_4$) δ 9.20 (d, $J$ = 2.5 Hz, 1H), 8.87 (s, 1H), 8.04 (s, 1H), 7.68-7.65 (m, 1H), 7.45-7.39 (m, 4H), 7.29 (d, $J$ = 2.5 Hz, 1H), 7.24 (t, $J$ = 4.5 Hz, 1H), 7.06 (t, $J$ = 2.5 Hz, 1H), 5.27 (d, $J$ = 10.0 Hz, 1H), 5.03 (q, $J$ = 7.0 Hz, 1H), 4.50 (t, $J$ = 8.5 Hz, 2H), 4.47-4.44 (m, 1H), 4.42-4.39 (m, 2H), 4.25 (t, $J$= 14.5 Hz, 1H), 3.90-3.87 (m, 1H), 3.82-3.78 (m, 2H), 3.75-3.71 (m, 2H), 3.67-3.58 (m, 1H), 3.49-3.43 (m, 1H), 3.26 (q, $J$ = 7.5 Hz, 2H), 3.10-3.04 (m, 2H), 2.59-2.56 (m, 1H), 2.47 (s, 1H), 2.43-2.39 (m, 1H), 2.23-2.17 (m, 2H), 2.01-1.93 (m, 2H), 1.87-1.85 (m, 1H), 1.81-1.73 (m, 4H), 1.51 (d, $J$= 7.0 Hz, 2H), 1.30-1.27 (m, 6H), 1.12 (d, $J$ = 7.0 Hz, 3H), 0.90-0.86 (m, 2H), 0.84-0.79 (m, 3H), 0.75 (d, $J$ = 6.5 Hz, 3H), 0.71 (s, 2H), 0.49 (s, 1H).

Example 129: Synthesis of compound (2S,4R)-1-((S)-2-(4-(1-((1-((1-(((7-(8-ethyl-7-fluoro-3-hydroxynaphthalen-1-yl)-8-fluoro-4-((R)-3-hydroxy-3-methylpiperidin-1-yl)pyrido[4,3-d]pyrimidin-2-yl)oxy)methyl)piperidin-4-yl)methy l)azetidin-3-yl)-1H-1,2,3-triazol-1-yl)-3-methylbutanoyl)-4-hydroxy-N-((S)-1-(4-(4-methylthiaz ol-5-yl)phenyl)ethyl)pyrrolidine-2-carboxamide

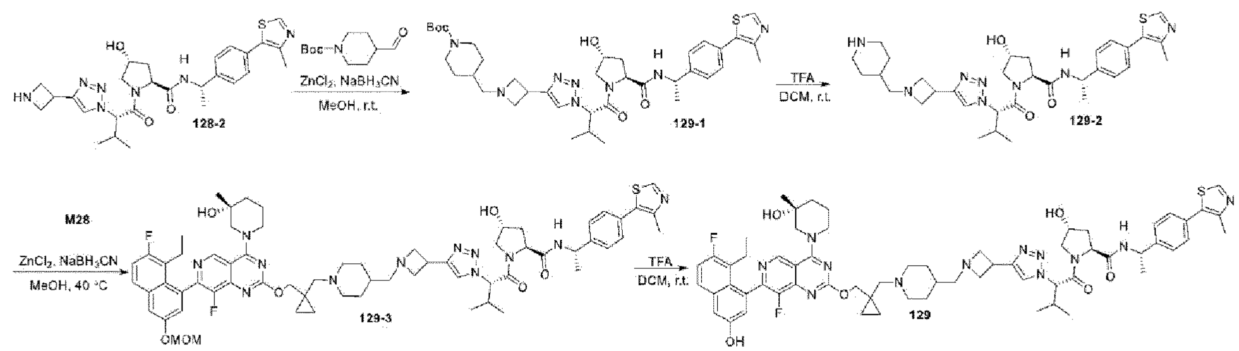


Step 1: Synthesis of compound 129-1

Compound 128-2 (300.0 mg) and 1-tert-butoxycarbonyl-4-piperidone (119.0 mg) were dissolved in methanol (8 mL), 1 M zinc chloride in diethyl ether (0.61 mL) was added. The mixture was reacted at 40°C for 1 hour. Sodium cyanoborohydride (105.2 mg) was then added, and the mixture reacted at 40°C for 10 minutes. The reaction was monitored by LCMS until completion. The reaction was quenched by adding saturated sodium bicarbonate solution (5.0

mL). DCM (20 mL) was added to the reaction solution, and the organic phase was collected, washed once with saturated brine, dried with anhydrous sodium sulfate, filtered, and concentrated to obtain 267.0 mg of a white solid, i.e., compound 129-1. ESI-MS m/z: 735.4 $[M+H]^+$.

Step 2: Synthesis of compound 129-2

Compound 129-1 (267.0 mg) was added to DCM (5 mL) and TFA (2 mL), and the mixture was reacted at room temperature for 10 minutes. The reaction was monitored by LCMS until completion. The reaction solution was concentrated directly, and 1 M NaOH solution was added until pH of the reaction solution reached 8. DCM (20 mL) was added to the reaction solution, and the organic phase was collected, washed once with saturated brine, dried with anhydrous sodium sulfate, filtered, and concentrated to obtain 230.0 mg of a white solid, i.e., compound 129-2. ESI-MS m/z: 635.4 $[M+H]^+$.

Step 3: Synthesis of compound 129-3

Compound M28 (100 mg) and compound 129-2 (128.5 mg) were dissolved in methanol (8 mL), 1 M zinc chloride in diethyl ether (0.25 mL) was added. The mixture was reacted at 40°C for 1 hour. Sodium cyanoborohydride (31.8 mg) was then added, and the mixture reacted at 60°C for 12 hours. The reaction was monitored by LCMS until completion. The reaction was quenched by adding saturated sodium bicarbonate solution (5.0 mL). DCM (20 mL) was added to the reaction solution, and the organic phase was collected, washed once with saturated brine, dried with anhydrous sodium sulfate, filtered, and concentrated to obtain 82.0 mg of a white solid, i.e., compound 129-3. ESI-MS m/z: 607.3 1/2$[M+2H]^+$.

Step 4: Synthesis of compound 129

Compound 129-3 (82.0 mg) was added to DCM (5 mL) and TFA (2 mL), and the mixture was reacted at room temperature for 10 minutes. The reaction was monitored by LCMS until completion. The reaction solution was concentrated directly, and the resultant was separated by preparative liquid chromatography to obtain 37.6 mg of a white powder, i.e., compound 129.

ESI-MS m/z: 585.2 1/2$[M+2H]^+$.

$^1$H NMR (500 MHz, Methanol-$d_4$) $\delta$ 9.20 (d, $J$ = 8.0 Hz, 1H), 8.87 (s, 1H), 8.03 (d, $J$ = 4.0 Hz, 1H), 7.67-7.64 (m, 1H), 7.44-7.39 (m, 4H), 7.29 (d, $J$ = 2.5 Hz, 1H), 7.23 (t, $J$ = 9.0 Hz, 1H), 7.06 (t, $J$ = 2.5 Hz, 1H), 5.27 (d, $J$ = 10.5 Hz, 1H), 5.03 (q, $J$= 7.0 Hz, 1H), 4.52-4.39 (m, 5H), 4.24 (t, $J$= 13.5 Hz, 1H), 3.90-3.87 (m, 1H), 3.82-3.78 (m, 2H), 3.74-3.71 (m, 2H), 3.66-3.56 (m, 1H), 3.49-3.41 (m, 1H), 3.26 (q, $J$= 7.5 Hz, 2H), 3.10-3.03 (m, 2H), 2.57-2.53 (m, 1H), 2.47 (s, 1H), 2.41-2.37 (m, 3H), 2.21-2.16 (m, 2H), 1.98-1.91 (m, 2H), 1.86-1.84 (m, 1H), 1.80-1.74 (m, 2H), 1.69-1.66 (m, 2H), 1.51 (d, $J$ = 7.0 Hz, 2H), 1.29-1.27 (m, 6H), 1.12 (d, $J$ = 6.5 Hz, 3H), 0.91-0.86 (m, 2H), 0.83-0.79 (m, 3H), 0.75-0.74 (m, 3H), 0.70 (s, 2H), 0.49 (s, 1H).

Example 130: Synthesis of compound (2S,4R)-1-((S)-2-(4-(1-((1-((1-(((7-(8-ethyl-7-fluoro-3-hydroxynaphthalen-1-yl)-8-fluoro-4-((R)-3-hydroxy-3-methylpiperidin-1-yl)pyrido[4,3-d]pyrimidin-2-yl)oxy)methyl)cyclopropyl)methyl) piperidin-4-yl)methyl)piperidin-4-yl)-1H-1,2,3-triazol-1-yl)-3-methylbutanoyl)-4-hydroxy-N-((S )-1-(4-(4-methylthiazol-5-yl) phenyl)ethyl)pyrrolidine-2-carboxamide

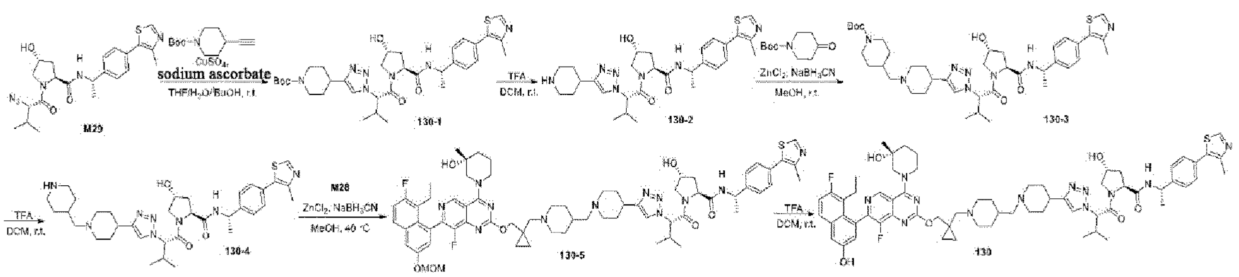


Step 1: Synthesis of compound 130-1

Compound M29 (800.0 mg), 1-(tert-butoxycarbonyl)-4-ethynylpiperidine (476.7 mg), anhydrous copper sulfate (251.7 mg), and sodium ascorbate (902.5 mg) were dissolved in a mixed solvent of THF/tert-butanol/water (10/10/10 mL).

The mixture was reacted at room temperature for 3 hours. The reaction was monitored by LCMS until completion. DCM (30 mL) was added to the reaction solution, and the resultant was washed once with saturated brine. The organic phase was dried with anhydrous sodium sulfate, filtered, concentrated, and purified by column chromatography to obtain 800.0 mg of a yellow solid, i.e., compound 130-1. ESI-MS m/z: 666.3 $[M+H]^+$.

Step 2: Synthesis of compound 130-2

Compound 130-1 (800.0 mg) was added to DCM (5 mL) and TFA (2 mL), and the mixture was reacted at room temperature for 10 minutes. The reaction was monitored by LCMS until completion. The reaction solution was concentrated directly, and 1 M NaOH solution was added until pH of the reaction solution reached 8. DCM (20 mL) was added to the reaction solution, and the organic phase was collected, washed once with saturated brine, dried with anhydrous sodium sulfate, filtered, and concentrated to obtain 577.0 mg of a white solid, i.e., compound 130-2. ESI-MS m/z: 566.3 $[M+H]^+$.

Step 3: Synthesis of compound 130-3

Compound 130-2 (577.0 mg) and 1-tert-butoxycarbonyl-4-piperidone (217.5 mg) were dissolved in methanol (10 mL), 1 M zinc chloride in diethyl ether (1.53 mL) was added. The mixture was reacted at 40°C for 1 hour. Sodium cyanoborohydride (192.3 mg) was then added, and the mixture reacted at 40°C for 10 minutes. The reaction was monitored by LCMS until completion. The reaction was quenched by adding saturated sodium bicarbonate solution (5.0 mL). DCM (20 mL) was added to the reaction solution, and the organic phase was collected, washed once with saturated brine, dried with anhydrous sodium sulfate, filtered, and concentrated to obtain 356.0 mg of a white solid, i.e., compound 130-3. ESI-MS m/z: 764.1 $[M+H]^+$.

Step 4: Synthesis of compound 130-4

Compound 130-3 (356.0 mg) was added to DCM (5 mL) and TFA (2 mL), and the mixture was reacted at room temperature for 10 minutes. The reaction was monitored by LCMS until completion. The reaction solution was concentrated directly, and 1 M NaOH solution was added until pH of the reaction solution reached 8. DCM (20 mL) was added to the reaction solution, and the organic phase was collected, washed once with saturated brine, dried with anhydrous sodium sulfate, filtered, and concentrated to obtain 198.0 mg of a white solid, i.e., compound 130-4. ESI-MS m/z: 664.1 $[M+H]^+$.

Step 5: Synthesis of compound 130-5

Compound 130-4 (100.0 mg) and compound M28 (134.2 mg) were dissolved in methanol (10 mL), 1 M zinc chloride in diethyl ether (0.25 mL) was added. The mixture was reacted at 40°C for 1 hour. Sodium cyanoborohydride (31.8 mg) was then added, and the mixture reacted at 60°C for 12 hours. The reaction was monitored by LCMS until completion. The reaction was quenched by adding saturated sodium bicarbonate solution (5.0 mL). DCM (20 mL) was added to the reaction solution, and the organic phase was collected, washed once with saturated brine, dried with anhydrous sodium sulfate, filtered, and concentrated to obtain 90.0 mg of a white solid, i.e., compound 130-5. ESI-MS m/z: 621.3 1/2$[M+2H]^+$.

Step 6: Synthesis of compound 130

Compound 130-5 (90.0 mg) was added to DCM (5 mL) and TFA (2 mL), and the mixture was reacted at room temperature for 10 minutes. The reaction was monitored by LCMS until completion. The reaction solution was concentrated directly, and the resultant was separated by preparative liquid chromatography to obtain 41.2 mg of a white powder, i.e., compound 130.

ESI-MS m/z: 599.2 1/2$[M+2H]^+$.

$^1$H NMR (500 MHz, Methanol-$d_4$) δ 9.20 (d, *J* = 8.0 Hz, 1H), 8.87 (s, 1H), 7.89 (s, 1H), 7.67-7.64 (m, 1H), 7.44-7.39 (m, 4H), 7.29 (d, *J* = 3.0 Hz, 1H), 7.23 (t,*J* = 9.5 Hz, 1H), 7.06 (d, *J* = 3.0 Hz, 1H), 5.25 (d, *J* = 10.0 Hz, 1H), 5.04 (q, *J* = 6.5 Hz, 1H), 4.53-4.39 (m, 5H), 4.23 (t, *J* = 13.0 Hz, 1H), 3.90-3.87 (m, 1H), 3.81 (d, *J* = 11.0 Hz, 1H), 3.66-3.56 (m, 1H), 3.49-3.41 (m, 1H), 3.08 (q, *J* = 11.5 Hz, 2H), 2.93 (d, *J* = 11.5 Hz, 2H), 2.75-2.69 (m, 1H), 2.58-2.54 (m, 1H), 2.47 (s, 1H), 2.41-2.38 (m, 1H), 2.22-2.14 (m, 4H), 2.09-2.04 (m, 2H), 1.99-1.93 (m, 4H), 1.86-1.84 (m, 1H), 1.79-1.70 (m, 6H), 1.57-1.51 (m, 4H), 1.29-1.26 (m, 6H), 1.12 (d, *J*= 6.5 Hz, 3H), 0.89-0.86 (m, 2H), 0.84-0.79 (m, 3H), 0.74-0.73 (m, 3H), 0.71 (s, 2H), 0.50 (s, 1H).

Example 131 Synthesis of compound (2S,4R)-1-((S)-2-(4-(1-((((7-(8-ethyl-7-fluoro-3-hydroxynaphthalen-1-yl)-8-fluoro-4-((R)-3-hydr oxy-3-methylpiperidin-1-yl)pyrido[4,3-d]pyrimidin-2-yl)oxy)methyl)cyclopropyl)methyl)piperid in-4-yl)-1H-1,2,3-triazol-1-yl)-3-methylbutanoyl)-4-hydroxy-N-(((S)-1-(4-(4-methylthiazol-5-yl) phenyl)ethyl)pyrrolidine-2-carboxamide

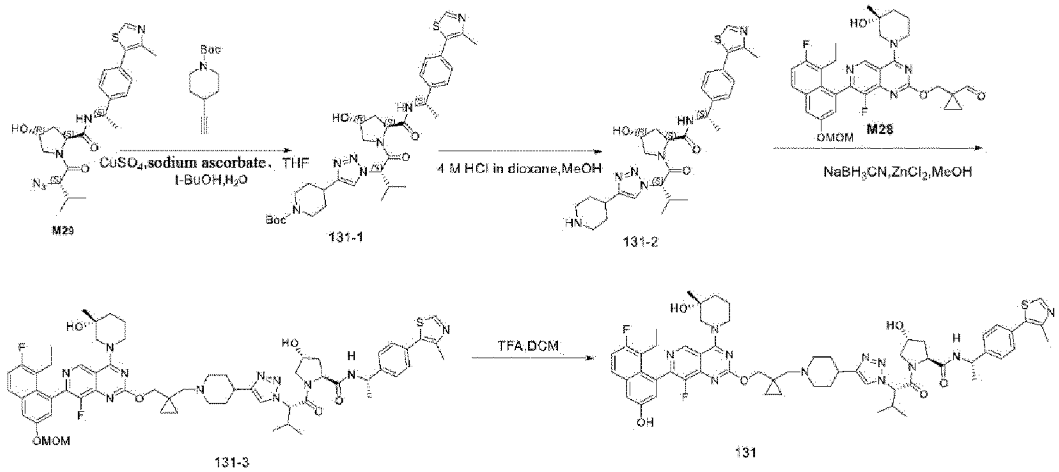


Step 1: Synthesis of compound 131-1

Compound M29 (100.0 mg) was added to a 25 mL single-necked flask, and dissolved in THF (2 mL). Then, 1-Boc-4-ethynylpiperidine (55.0 mg), copper sulfate (35.2 mg), water (2 mL), tert-butanol (2 mL), and sodium ascorbate (160.0 mg) were added. The mixture was reacted at room temperature for 0.5 hours. The reaction solution was directly evaporated to dryness. The resultant was separated and purified by column chromatography (DCM:MeOH = 12:1) to obtain 104.2 mg of a light yellow solid, i.e., compound 131-1. ESI-MS m/z: 666 $[M+H]^+$

Step 2: Synthesis of compound 131-2

Compound 131-1 (104.2 mg) was added to a 25 mL single-necked flask, and dissolved in 4 M hydrogen chloride in dioxane (5 mL). Then, methanol (1 mL) was added. The mixture was reacted at room temperature for 1 hour. The reaction solution was directly evaporated to dryness. After 5 mL of saturated sodium bicarbonate solution was added to the reaction solution to adjust the pH of the solution until pH=7 to 8, the reaction solution was extracted with a mixed solution of dichloromethane and isopropanol. The organic phase was dried with anhydrous sodium sulfate to obtain 79.0 mg of a yellow solid, i.e., compound 131-2. ESI-MS m/z: 566 $[M+H]^+$

Step 3: Synthesis of compound 131-3

Compound 131-2 (79.0 mg) was added to a 25 mL single-necked flask, and dissolved in methanol (3 mL). Then, M28 (100.00 mg) and 1M zinc chloride in tetrahydrofuran (0.1 mL) were added. After the reaction was heated to 40°C for 2 hours, $NaBH_3CN$ (35.2 mg) was added. The mixture was reacted overnight. The reaction solution was directly evaporated to dryness. The resultant was separated and purified by column chromatography (DCM:MeOH = 10:1) to obtain 108.2 mg of a light yellow solid, i.e., compound 131-3. ESI-MS m/z: 572 1/2 $[M+2H]^+$

Step 4: Synthesis of compound 131

Compound 131-3 (108.2 mg) was added to a 25 mL single-necked flask, and dissolved in DCM (2 mL). Then, TFA (2 mL) was added. The mixture was reacted at room temperature for 1 hour. The reaction solution was directly evaporated to dryness. After 5 mL of saturated sodium bicarbonate solution was added to the reaction solution to adjust the pH of the solution until pH=7 to 8, the reaction solution was extracted with a mixed solution of dichloromethane and isopropanol. The organic phase was dried with anhydrous sodium sulfate, and the resultant was separated and purified by Pre-HPLC ($CH_3CN/H_2O$ = 30%-55%) to obtain 67.6 mg of a white solid, i.e., compound 131.
ESI-MS m/z: 550.1 1/2$[M+2H]^+$

$^1H$ NMR (500 MHz, Methanol-$d_4$) δ 9.27 - 9.19 (m, 1H), 8.87 (s, 1H), 7.87 (t, J= 9.3 Hz, 1H), 7.66 (dd, J = 8.9, 5.9 Hz, 1H), 7.46 - 7.35 (m, 4H), 7.29 (d, J = 2.5 Hz, 1H), 7.23 (t, J = 9.2 Hz, 1H), 7.09 - 7.04 (m, 1H), 5.25 (d, J = 10.2 Hz, 1H), 5.08 - 4.99 (m, 1H), 4.59 - 4.41 (m, 5H), 4.25 (dd, J = 28.6, 13.2 Hz, 1H), 3.88 (dd, J = 10.9, 3.8 Hz, 1H), 3.79 (dd, J = 19.9, 9.0 Hz, 1H), 3.70 - 3.58 (m, 1H), 3.45 (d, J = 6.9 Hz, 1H), 3.22 - 3.06 (m, 2H), 2.94 (dd, J = 14.3, 7.1 Hz, 1H), 2.68 (d, J= 29.7 Hz, 2H), 2.59 - 2.35 (m, 8H), 2.14 (dd, J= 22.5, 11.5 Hz, 6H), 1.99 - 1.83 (m, 4H), 1.81 - 1.58 (m, 5H), 1.54 - 1.46 (m, 3H), 1.26 (dd, J = 17.7, 10.1 Hz, 4H), 1.11 (t, J= 8.2 Hz, 3H), 0.85 - 0.68 (m, 9H), 0.51 (s, 2H).

Example 132 Synthesis of compound (2S,4R)-1-((S)-2-(4-((4-((1-(((7-(8-ethyl-7-fluoro-3-hydroxynaphthalen-1-yl)-8-fluoro-4-((R)-3-hydroxy-3-methylpiperidin-1-yl)pyrido[4,3-d]pyrimidin-2-yl)oxy)methyl)cyclopropyl)methyl)pi perazin-1-yl)methyl-1H-1,2,3-triazol-1-yl)-3-methylbutanoyl)-4-hydroxy-N-((S)-1-(4-(4-methylt hiazol-5-yl)phenyl)ethyl)pyrrolidine-2-carboxamide

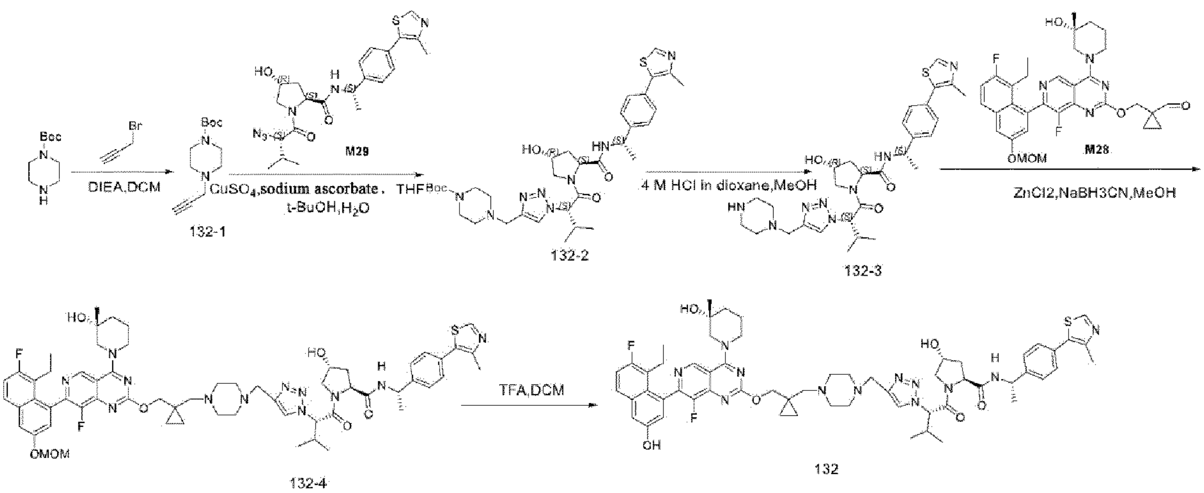


Step 1: Synthesis of compound 132-1

Compound M1 (1.0 g) was added to a 25 mL single-necked flask, and dissolved in DCM (2 mL). Then, DIPEA (1.4 mL) and 3-bromopropyne (0.7 g) were added. The mixture was reacted at room temperature for 5 hours. The reaction solution was directly evaporated to dryness. The resultant was separated and purified by column chromatography (PE: EA = 1:1) to obtain 1.05 g of a light yellow solid, i.e., compound 132-1.

Step 2: Synthesis of compound 132-2

Compound M29 (100.0 mg) was added to a 25 mL single-necked flask, and dissolved in THF (2 mL). Then, compound 132-1 (55.0 mg), copper sulfate (35.2 mg), water (2 mL), tert-butanol (2 mL), and sodium ascorbate (160.0 mg) were added. The mixture was reacted at room temperature for 0.5 hours. The reaction solution was directly evaporated to dryness. The resultant was separated and purified by column chromatography (DCM:MeOH = 12:1) to obtain 105.7 mg of a light yellow solid, i.e., compound 132-2.

Step 3: Synthesis of compound 132-3

Compound 132-2 (105.7 mg) was added to a 25 mL single-necked flask, and dissolved in 4 M hydrogen chloride in dioxane (5 mL). Then, methanol (1 mL) was added. The mixture was reacted at room temperature for 1 hour. The reaction solution was directly evaporated to dryness. After 5 mL of saturated sodium bicarbonate solution was added to the reaction solution to adjust the pH of the solution until pH=7 to 8, the reaction solution was extracted with dichloromethane and isopropanol. The organic phase was dried with anhydrous sodium sulfate to obtain 83.2 mg of a yellow solid, i.e., compound 132-3.

Step 4: Synthesis of compound 132-4

Compound 132-3 (83.2 mg) was added to a 25 mL single-necked flask, and dissolved in methanol (3 mL). Then,

M28 (100.00 mg) and 1M zinc chloride in tetrahydrofuran (0.1 mL) were added. After the reaction was heated to 40°C for 2 hours, $NaBH_3CN$ (35.2 mg) was added. The mixture was reacted overnight. The reaction solution was directly evaporated to dryness. The resultant was separated and purified by column chromatography (DCM:MeOH = 10:1) to obtain 94.3 mg of a light yellow solid, i.e., compound 132-4.

Step 5: Synthesis of compound 132

Compound 132-4 (94.3 mg) was added to a 25 mL single-necked flask, and dissolved in DCM (2 mL). Then, TFA (2 mL) was added. The mixture was reacted at room temperature for 1 hour. The reaction solution was directly evaporated to dryness. After 5 mL of saturated sodium bicarbonate solution was added to the reaction solution to adjust the pH of the solution until pH=7 to 8, the reaction solution was extracted with a mixed solution of dichloromethane and isopropanol. The organic phase was dried with anhydrous sodium sulfate, and the resultant was separated and purified by Pre-HPLC ($CH_3CN/H_2O$ = 30%-55%) to obtain 36.1 mg of a white solid, i.e., compound 132.

LCMS: 1/2[M+2H]$^+$= 557.57

$^1$H NMR (500 MHz, DMSO-$d_6$) δ 9.22 (s, 1H), 8.98 (s, 1H), 8.50 (d, *J* = 7.2 Hz, 1H), 7.97 (d, *J* = 13.6 Hz, 1H), 7.81 - 7.72 (m, 1H), 7.44 (d, *J* = 7.9 Hz, 2H), 7.40 - 7.29 (m, 4H), 7.03 (s, 1H), 5.24 (d, *J* = 10.1 Hz, 1H), 5.11 *(d, J*= 44.3 Hz, 1H), 4.97 - 4.84 (m, 1H), 4.75 (d, *J* = 5.7 Hz, 1H), 4.44 - 4.23 (m, 5H), 4.04 (dd, *J* = 26.8, 13.5 Hz, 1H), 3.74 (d, *J*= 6.9 Hz, 1H), 3.65 (s, 1H), 3.52 (d, *J* = 15.4 Hz, 3H), 2.41 - 2.27 (m, 9H), 2.09 (d, *J* = 48.3 Hz, 4H), 1.83 - 1.59 (m, 5H), 1.37 *(d, J*= 6.8 Hz, 3H), 1.17 (d, *J* = 11.3 Hz, 3H), 1.02 (d, *J* = 6.8 Hz, 6H), 0.74 (d, *J* = 7.3 Hz, 3H), 0.63 (s, 5H), 0.40 (s, 2H).

Example 133 Synthesis of compound (2S,4R)-1-((S)-2-(4-(1-((7-(8-ethyl-7-fluoro-3-hydroxynaphthalen-1-yl)-8-fluoro-4-((R)-3-hydro xy-3-methylpiperidin-1-yl)pyrido[4,3-d]pyrimidin-2-yl)oxy)methyl)cyclopropyl)methyl)piperidi n-4-yl) phenyl)-1H-1,2,3-triazol-1-yl)-3-methylbutanoyl)-4-hydroxy-N-((S)-1-(4-(4-methylthiazo l-5-yl)phenyl)ethyl)pyrrolidine-2-carboxamide

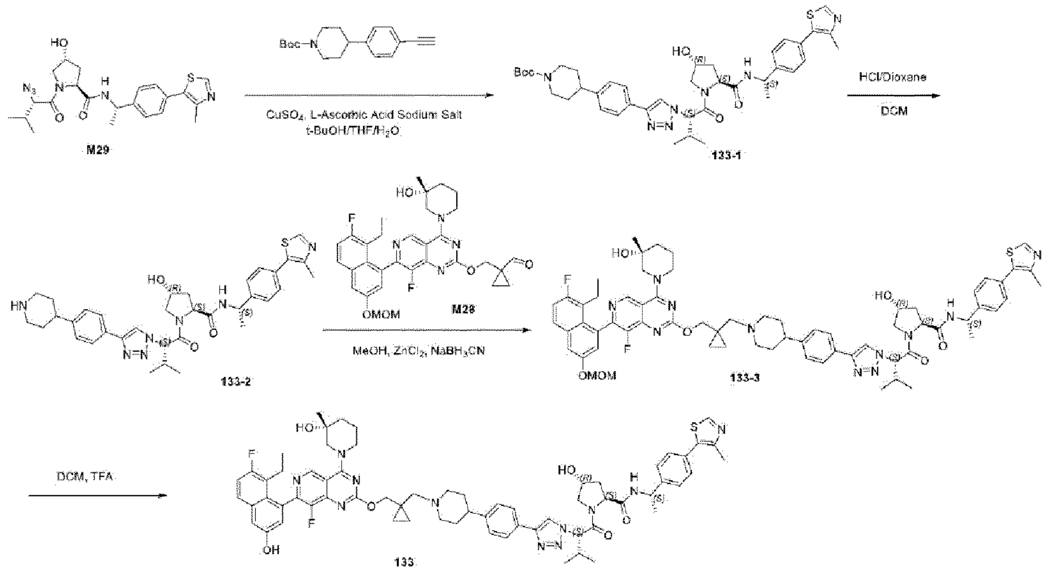


Step 1: Synthesis of compound 133-1

M29 (380 mg) and tert-butyl 4-(4-ethynylphenyl)piperidin-1-carboxylate (310 mg) were dissolved in tert-butanol (4 mL), tetrahydrofuran (4 mL) and water (4 mL), and then anhydrous copper sulfate (120 mg), and sodium ascorbate (429 mg) were added. The mixture was reacted at room temperature for 2 hours. The reaction solution was rotary evaporated to dryness, and extracted by adding DCM and water. The organic phase was collected, dried with anhydrous sodium sulfate, and filtered. The filtrate was collected, and concentrated under reduced pressure. The resultant was purified by column chromatography (DCM:MeOH = 15:1) to obtain compound 133-1 (560 mg), ESI-MS m/z: 742.3 [M+H]$^+$.

Step 2: Synthesis of compound 133-2

Compound 133-1 (300 mg) was dissolved in 6 mL of DCM and 4 M hydrogen chloride in dioxane (3 mL). The mixture was reacted at room temperature for 0.5 hours. The reaction solution was concentrated under reduced pressure, and saturated sodium bicarbonate solution was added. The mixture was extracted with DCM/isopropanol (3:1), and the organic phase was collected, dried with anhydrous sodium sulfate, and filtered. The filtrate was collected, and concentrated under reduced pressure to obtain compound 133-2 (254 mg), ESI-MS m/z: 642.3 $[M+H]^+$.

Step 3: Synthesis of compound 133-3

Compound 133-2 (60.0 mg) and M28 (66.0 mg) were dissolved in methanol (5 mL), and then zinc chloride (0.14 mL, 1 M in THF) was added. The mixture was reacted at 40°C for 1 hour, and then sodium cyanoborohydride (18 mg) was added. The reaction was kept at 40°C for 12 hours. The reaction was quenched by adding saturated sodium bicarbonate solution (5.0 mL). The reaction solution was extracted by adding DCM (20 mL), and the organic phase was collected, washed once with saturated brine, dried with anhydrous sodium sulfate, and filtered. The filtrate was collected, and concentrated under reduced pressure. The resultant was purified by column chromatography (DCM:MeOH = 10:1) to obtain compound 133-3 (59 mg). ESI-MS m/z: 609.7 1/2$[M+2H]^+$.

Step 4: Synthesis of compound 133

Compound 133-3 (59 mg) was dissolved in DCM (3 mL), and then 3 mL of trifluoroacetic acid was added. The mixture was reacted at room temperature for 1 hour. The reaction solution was concentrated directly, and redissolved with 5.00 mL of DCM, then concentrated again. The resultant was separated by preparative liquid chromatography to obtain 20.9 mg of white powder, i.e., compound 133.

ESI-MS m/z: 588.2 1/2$[M+2H]^+$.

$^1$H NMR (500 MHz, DMSO-$d_6$) δ: 9.93 (d, *J* = 2.5 Hz, 1H), 9.23 (s, 1H), 8.98 (s, 1H), 8.63 (s, 1H), 8.52 (d, *J* = 7.5 Hz, 1H), 7.82 - 7.73 (m, 3H), 7.44 (d, *J* = 8.0 Hz, 2H), 7.39 - 7.32 (m, 4H), 7.30 - 7.25 (m, 2H), 7.03 (d, *J* = 2.5 Hz, 1H), 5.32 (d, *J* = 10.0 Hz, 1H), 5.17 (d, J = 3.5 Hz, 1H), 4.95 - 4.89 (m, 1H), 4.75 (d, *J* = 10.0 Hz, 1H), 4.44 - 4.27 (m, 5H), 4.09 - 4.01 (m, 1H), 3.78 (dd, *J* = 10.5, 4.0 Hz, 1H), 3.70 (d, *J* = 10.5 Hz, 1H), 3.64 - 3.51 (m, 1H), 3.41 - 3.36 (m, 1H), 3.08 (s, 2H), 2.39 - 2.36 (m, 3H), 2.18 - 2.12 (m, 1H), 2.10 - 1.93 (m, 5H), 1.85 - 1.53 (m, 9H), 1.38 (d, J = 7.0 Hz, 3H), 1.23 (s, 3H), 1.17 (d, J = 11.5 Hz, 3H), 1.08 (d, J = 6.5 Hz, 3H), 0.77 - 0.64 (m, 8H), 0.44 (s, 2H).

Example 134 Synthesis of compound (2S,4R)-1-((S)-2-(4-(5-((1-(7-(8-ethyl-7-fluoro-3-hydroxynaphthalen-1-yl)-8-fluoro-4-((R)-3-hy droxy-3-methylpiperidin-1-yl)pyrido[4,3-d]pyrimidin-2-yl)oxy)methyl)cyclopropyl)methyl)piper azin-1-yl)pyrazin-2-yl)-1H-1,2,3-triazol-1-yl)-3-methylbutanoyl)-4-hydroxy-N-(((S)-1-(4-methyl thiazol-5-yl)phenyl)ethyl)pyrrolidine-2-carboxamide

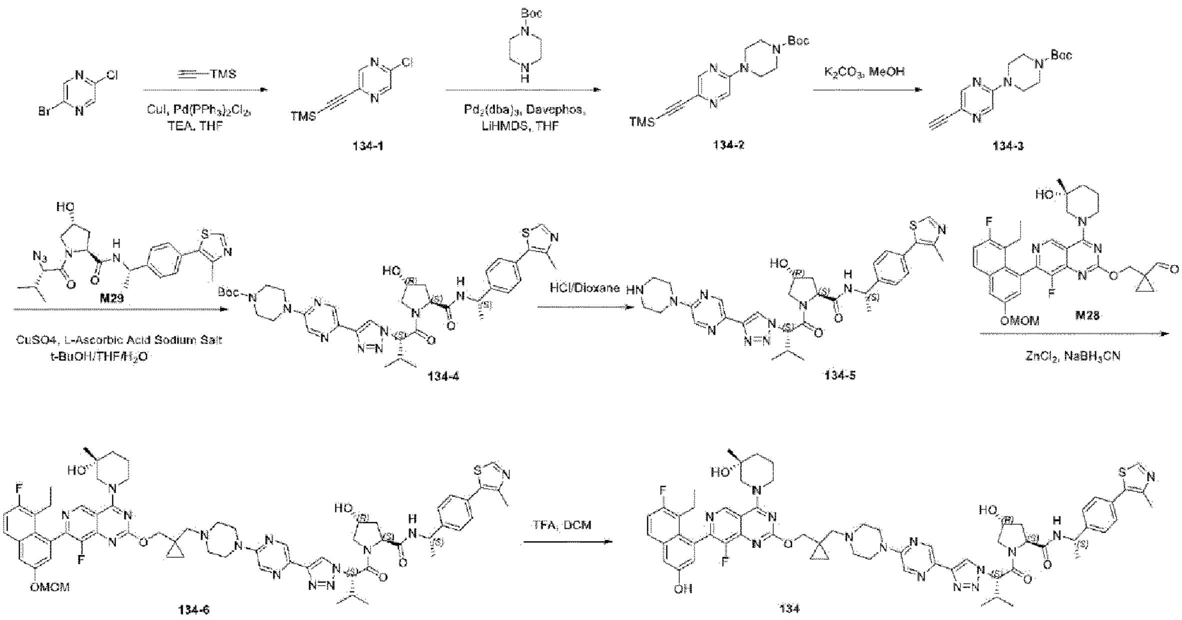


Step 1: Synthesis of compound 134-1

2-Bromo-5-chloropyrazine (3.0 g), trimethylsilyne (1.83 g) were dissolved in THF (20 mL), and then cuprous iodide (0.3 g), palladium(II)bis(triphenylphosphine) dichloride (1.09 g), triethylamine (6.47 mL) were added. The reaction was heated to 80°C for 2 hours under $N_2$ atmosphere. The reaction solution was rotary evaporated to dryness, diluted with water, and extracted with EA. The organic phase was collected, dried with anhydrous sodium sulfate, and filtered. The filtrate was collected, and concentrated under reduced pressure. The resultant was purified by column chromatography (PE: EA = 10:1) to obtain product 134-1 (2.9 g). ESI-MS m/z: 211.0 $[M+H]^+$.

Step 2: Synthesis of compound 134-2

Compound 134-1 (2.9 g) was dissolved in THF (20 mL), and then 1-Boc-piperazine (6.41 g), tris(dibenzylideneacetone)dipalladium (0.63 g), 2-dicyclohexylphosphine-2'-(N,N-dimethylamine)-biphenyl (0.27 g), lithium bis(trimethylsilyl)amide (27.5 mL/1M in THF) were added. The reaction was heated to 80°C for 2 hours under $N_2$ atmosphere. The reaction solution was rotary evaporated to dryness, diluted with water, and extracted with EA. The organic phase was collected, dried with anhydrous sodium sulfate, and filtered. The filtrate was collected, and concentrated under reduced pressure. The resultant was purified by column chromatography (PE: EA = 85: 15) to obtain product 134-2 (728 mg). ESI-MS m/z: 631.2 $[M+H]^+$.

Step 3: Synthesis of compound 134-3

Compound 134-2 (728 mg) was dissolved in MeOH (15 mL), and then potassium carbonate (140 mg) was added. The mixture was reacted at room temperature for 1 hour. The reaction solution was rotary evaporated to dryness, diluted with water, and extracted with EA. The organic phase was collected, dried with anhydrous sodium sulfate, and filtered. The filtrate was collected, and concentrated under reduced pressure. The resultant was purified by column chromatography (PE: EA = 4:1) to obtain product 134-3 (525 mg). ESI-MS m/z: 289.1 $[M+H]^+$.

Step 4: Synthesis of compound 134-4

Compound 134-3 (200 mg) and M29 (317 mg) were dissolved in tert-butanol (4 mL), tetrahydrofuran (4 mL) and water (4 mL), and then anhydrous copper sulfate (100 mg), and sodium ascorbate (357 mg) were added. The mixture was reacted at room temperature for 3 hours. The reaction solution was rotary evaporated to dryness, diluted with water, and extracted with DCM. The organic phase was collected, dried with anhydrous sodium sulfate, and filtered. The filtrate was collected, and concentrated under reduced pressure. The resultant was purified by column chromatography (DCM: MeOH = 10:1) to obtain compound 134-4 (440 mg), ESI-MS m/z: 745.3 $[M+H]^+$.

Step 5: Synthesis of compound 134-5

Compound 134-4 (440 mg) was dissolved in 6 mL of DCM and hydrogen chloride in 3 mL of dioxane (4 mol/L). The mixture was reacted at room temperature for 0.5 hours. The reaction solution was rotary evaporated to dryness, and diluted by adding saturated sodium bicarbonate solution. The mixture was extracted with DCM/isopropanol (3:1), and the organic phase was collected, dried with anhydrous sodium sulfate, and filtered. The filtrate was collected, and concentrated under reduced pressure to obtain compound 134-5 (290 mg), ESI-MS m/z: 645.3 $[M+H]^+$.

Step 6: Synthesis of compound 134-6

Compound 134-5 (90.0 mg) and M28 (100.0 mg) were dissolved in methanol (5 mL), and then 1 M zinc chloride in tetrahydrofuran (0.21 mL) was added. The mixture was reacted at 40°C for 1 hour, and then sodium cyanoborohydride (27 mg) was added. The reaction was kept at 40°C for 12 hours. The reaction was quenched by adding saturated sodium bicarbonate solution (5.0 mL). The reaction solution was extracted by adding DCM (20 mL), and the organic phase was collected, washed once with saturated brine, dried with anhydrous sodium sulfate, and filtered. The filtrate was collected, and concentrated under reduced pressure. The resultant was purified by column chromatography (DCM:MeOH = 10:1) to obtain compound 134-6 (115 mg). ESI-MS m/z: 611.2 1/2$[M+2H]^+$.

Step 7: Synthesis of compound 134

Compound 134-6 (115 mg) was dissolved in DCM (3 mL), and then 3 mL of trifluoroacetic acid was added. The mixture was reacted at room temperature for 1 hour. The reaction solution was concentrated directly, and redissolved with 5.00 mL of DCM, then concentrated again. The resultant was separated by preparative liquid chromatography to obtain 68.1 mg of white powder, i.e., compound 134.

ESI-MS m/z: 589.6 1/2$[M+2H]^+$.

$^1$H NMR (500 MHz, DMSO-$d_6$) δ: 9.95 (s, 1H), 9.21 (d, *J* = 2.0 Hz, 1H), 8.98 (s, 1H), 8.67 (d, *J* = 1.5 Hz, 1H), 8.52 (d, *J* = 7.5 Hz, 1H), 8.43 (s, 1H), 8.33 (s, 1H), 7.75 (dd, J = 9.0, 6.0 Hz, 1H), 7.46 - 7.42 (m, 2H), 7.37 - 7.35 (m, 2H), 7.34 - 7.30 (m, 2H), 7.03 (d, *J* = 2.5 Hz, 1H), 5.36 (d, *J* = 10.5 Hz, 1H), 5.18 (d, *J* = 4.0 Hz, 1H), 4.95 - 4.89 (m, 2H), 4.74 (d, *J* = 8.5 Hz, 1H), 4.42 - 4.29 (m, 5H), 4.08 - 4.00 (m, 1H), 3.81 - 3.74 (m, 1H), 3.68 (d, *J* = 11.0 Hz, 1H), 3.62 - 3.50 (m, 5H), 2.54 (s, 2H), 2.39 - 2.29 (m, 4H), 2.16 - 1.97 (m, 4H), 1.1.81 - 1.61 (m, 5H), 1.38 (d, *J* = 7.0 Hz, 3H), 1.24 (s, 3H), 1.16 (d, *J* = 13.0 Hz, 3H), 1.07 (d, *J* = 6.5 Hz, 3H), 0.73 - 0.69 (m, 8H), 0.45 (s, 2H).

Example 135 Synthesis of compound (2S,4R)-1-((S)-2-(4-(6-(1-(1-(((7-(8-ethyl-7-fluoro-3-hydroxynaphthalene-1-yl)-8-fluoro-4-(((R) -3-hydroxy-3-methylpiperidin-1-yl)pyrido[4,3-d]pyrimidin-2-yl)oxy)methylcyclopropyl)methyl) piperidin-4-yl)oxy)pyridin-3-yl)-1H-1,2,3-triazol-1-yl-3-methylbutanoyl)-4-hydroxy-N-((S)-1-(4 -(4-methylthiazol-5-yl)phenyl)ethyl) pyrrolidine-2-carboxamide

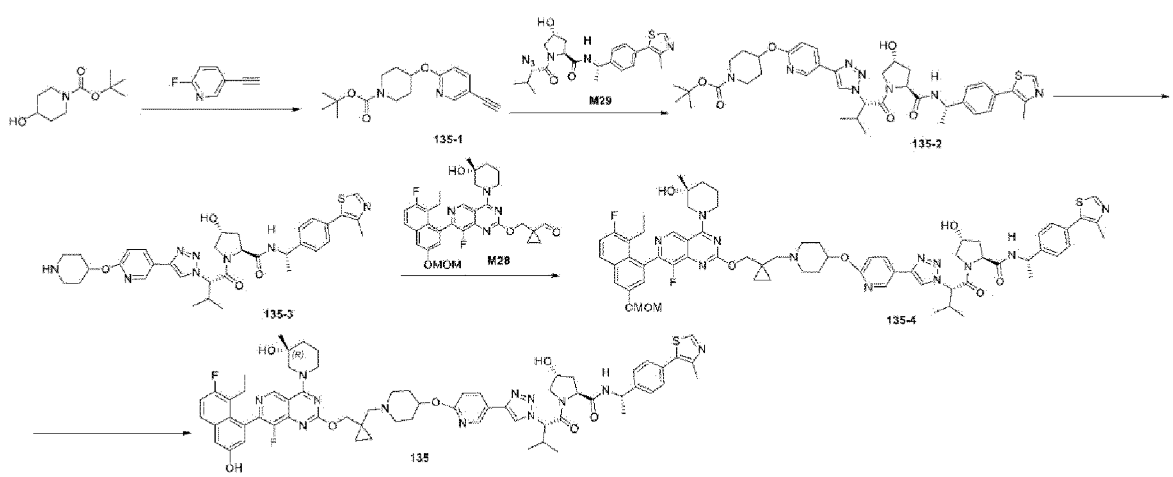


Step 1: Synthesis of compound 135-1

Sodium hydride (29.81 mg, 60%, 0.75 mmol) was added to N-tert-butoxycarbonyl-4-hydroxypiperidine (50 mg) in

tetrahydrofuran (1.00 mL), cooled in an ice water bath under the protection of nitrogen. The mixture was reacted at 0°C for 0.5 hours, and then 5-ethynyl-2-fluoropyridine (36.10 mg, 0.30 mmol) was added. The mixture was reacted at 20°C for 2 hours. The reaction solution was added to 20 mL of saturated ammonium chloride, and then 20 mL of EA was added. The organic phase was separated, dried, filtered and concentrated under reduced pressure. The residue was purified by column chromatography (EA:PE = 1:5) to obtain 60.00 mg of a colorless solid, i.e., compound 135-1. ESI-MS m/z: 303.20 $[M+H]^+$.

Step 2: Synthesis of compound 135-2

Copper sulfate (31.67 mg, 0.20 mmol) and an aqueous solution containing sodium ascorbate (117.92 mg, 0.60 mmol) were added to compound 135-1 (60.00 mg, 0.20 mmol), M29 (1.50 mL) and tetrahydrofuran (1.50 mL). The mixture was reacted at 20°C for 1 hour. The reaction was monitored by LCMS until completion. The reaction solution was added to 15 mL of water, and extracted 4 times with EA. The organic phases were combined, dried, filtered and concentrated under reduced pressure. The residue was purified by column chromatography (EA:DCM = 1:10) to obtain 105.00 mg of a brownish-yellow solid, i.e., compound 135-2. ESI-MS m/z: 759.40 $[M+H]^+$.

Step 3: Synthesis of compound 135-3

Trifluoroacetic acid (0.59 mL, 7.91 mmol) was added to compound 135-2 (120.00 mg, 0.16 mmol) in dichloromethane (3.00 mL), and the mixture was reacted at 20°C for 0.5 hours. The reaction was monitored by LCMS until completion. The reaction solution was slowly added to 20 mL of saturated sodium bicarbonate solution, and extracted 4 times with DCM. The organic phases were combined, dried, filtered, and concentrated under reduced pressure. 95.00 mg of a brown solid was obtained, i.e., compound 135-3. ESI-MS m/z: 659.30 $[M+H]^+$. The crude product was directly fed into the next step.

Step 4: Synthesis of compound 135-4

1 M zinc chloride in diethyl ether (0.27 mL) was added to compound 135-3 (106.73 mg, 0.16 mmol) and M28 (80.00 mg, 0.14 mmol) in methanol (0.27 mL). The reaction was heated to 40°C and stirred for 2 hours. Then, sodium cyanoborohydride (33.93 mg, 0.54 mmol) was added, and the reaction was stirred at 40°C for 4 hours. The reaction solution was added to 20 mL of water, and extracted with DCM. The organic phase was dried, filtered and concentrated under reduced pressure. The resultant was purified by column chromatography (MeOH:DCM = 1:10) to obtain 90.00 mg of a colorless solid, i.e., compound 135-4. ESI-MS m/z: 618.7 1/2$[M+2H]^+$.

Step 5: Synthesis of compound 135

Trifluoroacetic acid (1.20 mL, 16.18 mmol) was added to compound 135-4 (100.00 mg, 0.08 mmol) in dichloromethane (5.00 mL), and the mixture was reacted at 20°C for 0.5 hours. The reaction was monitored by LCMS until completion. The reaction solution was concentrated under reduced pressure and the resultant was purified by Pre-HPLC to obtain 44.90 mg of white powder, i.e., compound 135.

ESI-MS m/z: 596.6 1/2$[M+2H]^+$.

$^1$H NMR (500 MHz, Methanol-$d_4$) $\delta$ 9.20 (s, 1H), 8.87 (s, 1H), 8.57 (s, 1H), 8.47 (s, 1H), 8.08-8.08 (m, 1H), 7.68-7.65 (m, 1H), 7.45-7.39 (m, 4H), 7.29 (s, 1H), 7.25-7.22 (m, 1H), 7.07 (s, 1H), 6.81 (d, *J* = 10.0 Hz, 1H), 5.37-5.35 (d, *J* = 10.0 Hz, 1H), 5.05-5.02 (m, 3H), 4.55-4.52 (m, 1H), 4.46 (s, 2H), 4.27-4.22 (m, 1H), 3.93-3.86 (m, 2H), 3.58 (s, 1H), 3.48-3.46 (m, 1H), 2.91-2.88 (m, 3H), 2.65-2.63 (m, 1H), 2.51-2.43 (m, 6H), 2.35 (s, 2H), 2.22-2.16 (m, 3H), 2.03-1.98 (m, 4H), 1.80-1.76 (m, 5H), 1.53-1.52 (d, *J*= 5.0 Hz, 3H), 1.30-1.27 (d, *J* = 15.0 Hz, 3H), 1.17-1.16 (d, *J* = 5.0 Hz, 3H), 0.83-0.79 (m, 6H).

Example 136 Synthesis of compound (2S,4R)-1-((S)-2-(4-(2-(1-(1-((7-(8-ethyl-7-fluoro-3-hydroxynaphthalen-1-yl)-8-fluoro-4-((R)-3-hydroxy-3-methylpiperidin-1-yl)pyrido[4,3-d]pyrimidin-2-yl)oxy)methyl)cyclopropyl)methyl)pi peridin-4-yl)oxy)pyridin-4-yl)-1H-1,2,3-triazol-1-yl)-3-methylbutanoyl)-4-hydroxy-N-(((S)-1-(4 -methylthiazol-5-yl)phenyl)ethyl) pyrrolidine-2-carboxamide

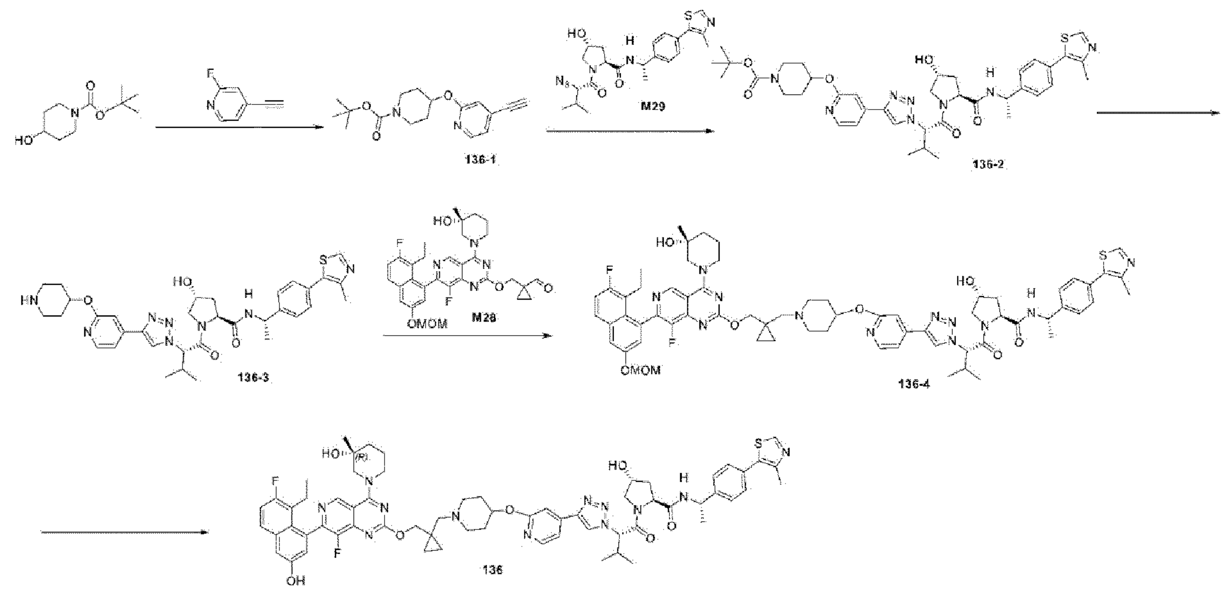


Step 1: Synthesis of compound 136-1

Sodium hydride (32.80 mg, 60%, 0.82 mmol) was added to N-tert-butoxycarbonyl-4-hydroxypiperidine (55.00 mg, 0.27 mmol) in tetrahydrofuran (3.00 mL) cooled in an ice water bath under the protection of nitrogen. The mixture was reacted at 0°C for 0.5 hours, and then 4-ethynyl-2-fluoropyridine (33.10 mg, 0.27 mmol) was added. The mixture was reacted at 20°C for 2 hours. The reaction solution was added to 20 mL of saturated ammonium chloride, and then 20 mL of EA was added. The organic phase was separated, dried, filtered and concentrated under reduced pressure. The residue was purified by column chromatography (EA:PE = 1% to 16%) to obtain 38.00 mg of a colorless solid, i.e., compound 136-1. ESI-MS m/z: 303.20 $[M+H]^+$.

Step 2: Synthesis of compound 136-2

Sodium ascorbate (55.03 mg, 0.28 mmol) and copper sulfate (14.78 mg, 0.09 mmol) were added to compound 136-1 (28.00 mg, 0.09 mmol) and M29 (46.52 mg, 0.10 mmol) in tert-butanol (1.50 mL), tetrahydrofuran (1.50 mL) and water (0.75mL). The mixture was reacted at 20°C for 1 hour. The reaction was monitored by LCMS until completion. The reaction solution was added to 15 mL of water, and extracted 4 times with EA. The organic phases were combined, dried, filtered and concentrated under reduced pressure. The residue was purified by column chromatography (MEOH:DCM = 1% to 10%) to obtain 50.00 mg of a colorless solid, i.e., compound 136-2. ESI-MS m/z: 759.40 $[M+H]^+$.

Step 3: Synthesis of compound 136-3

Trifluoroacetic acid (0.44 mL, 5.93 mmol) was added to compound 136-2 (45.00 mg, 0.06 mmol) in dichloromethane (2.00 mL), and the mixture was reacted at 20°C for 0.5 hours. The reaction was monitored by LCMS until completion. The reaction solution was slowly added to 20 mL of saturated sodium bicarbonate solution, and extracted 4 times with DCM. The organic phases were combined, dried, filtered, and concentrated under reduced pressure. Then, 35.00 mg of a brown solid was obtained, i.e., compound 136-3. The resultant was directly fed into the next step. ESI-MS m/z: 659.30 $[M+H]^+$.

Step 4: Synthesis of compound 136-4

1 M zinc chloride in diethyl ether (0.10 mL) was added to compound 136-3 (36.70 mg, 0.06 mmol), and M28 (30.00 mg, 0.05 mmol) in methanol (2.00 mL). The reaction was heated to 40°C and stirred for 2 hours. Then, sodium cyanoborohydride (12.72 mg, 0.20 mmol) was added. The reaction was stirred at 40°C for 16 hours. The reaction was monitored by LCMS until completion. The reaction solution was added to 20 mL of water, and extracted with DCM. The organic phase was dried, filtered and concentrated under reduced pressure. The resultant was purified by column chromatography (MeOH:DCM = 1:10) to obtain 30.00 mg of a white solid, i.e., compound 136-4. ESI-MS m/z: 618.7 1/2 $[M+2H]^+$.

Step 5: Synthesis of compound 136

Trifluoroacetic acid (0.30 mL, 4.04 mmol) was added to compound 136-4 (25.00 mg, 0.02 mmol) in dichloromethane (2.00 mL), and the mixture was reacted at 20°C for 0.5 hours. The reaction was monitored by LCMS until completion. The reaction solution was concentrated under reduced pressure without heating, and the residue was purified by Pre-HPLC to obtain 10.10 mg of a white solid, i.e., compound 136.

ESI-MS m/z: 596.3 1/2[M+2H]$^+$.

$^1$H NMR (500 MHz, Methanol-$d_4$) δ 9.20-9.19 (s, $J$ = 5.0 Hz,1H), 8.87 (s, 1H), 8.66 (s, 1H), 8.13 (s, 1H), 7.68-7.65 (m, 1H), 7.45-7.37 (m, 5H), 7.30 (s, 1H), 7.28-7.22 (m, 2H), 7.07 (s, 1H), 5.41-5.39 (d, $J$ = 10.0 Hz, 1H), 5.05-5.02 (m, 3H), 4.55-4.52 (m, 1H), 4.46 (s, 2H), 4.27-4.22 (m, 1H), 3.93-3.86 (m, 2H), 3.58 (s, 1H), 3.48-3.46 (m, 1H), 2.91-2.88 (m, 3H), 2.65-2.63 (m, 1H), 2.51-2.43 (m, 6H), 2.35 (s, 2H), 2.22-2.16 (m, 3H), 2.03-1.98 (m, 4H), 1.80-1.76 (m, 5H), 1.53-1.51 (d, $J$ = 10.0 Hz, 3H), 1.30-1.27 (d, $J$ = 15.0 Hz, 3H), 1.17-1.16 (d, $J$ = 5.0 Hz, 3H), 0.83-0.79 (m, 6H).

Example 137 Synthesis of compound (2S,4R)-1-((1-(7-(8-ethyl-7-fluoro-3-hydroxynaphthalen-1-yl)-8-fluoro-4-((R)-3-hydroxy-3-met hylpiperidin-1-yl)pyrido[4,3-d]pyrimidin-2-yl)oxy)methyl)cyclopropyl)methyl)-[1,4'-bipiperidin ]-4-yl)-1H-1,2,3-triazol-1-yl)-3-methylbutanoyl)-4-hydroxy-N-((S)-1-(4-methylthiazol-5-yl)phen yl)ethyl)pyrrolidine-2-carboxamide

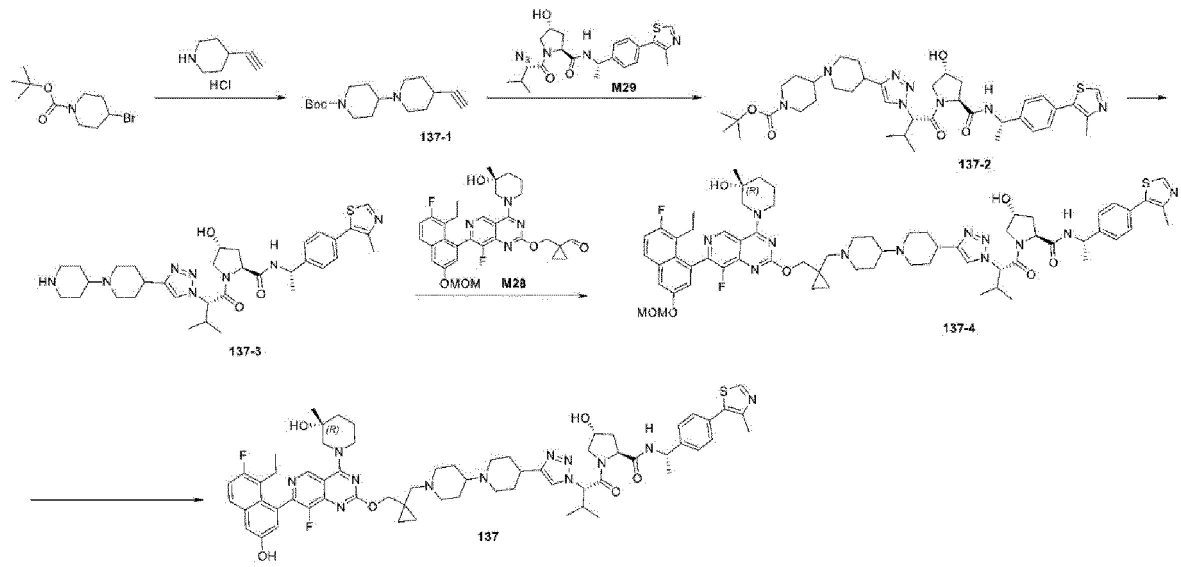


Step 1: Synthesis of compound 137-1

Tert-butyl 4-bromopiperidine-1-carboxylate (437.00 mg, 1.65 mmol), potassium carbonate (476.34 mg, 3.45 mmol ), and potassium iodide (45.77 mg, 0.28 mmol ) was added to 4-ethynylpiperidine hydrochloride (200.00 mg, 1.38 mmol) in acetonitrile (4.00 mL). The mixture was reacted at 100°C for 16 hours under the protection of nitrogen. The reaction solution was filtered and the filtrate was concentrated under reduced pressure. The resultant was purified by column chromatography (MeOH:DCM = 1:20) to obtain 90.00 mg of colorless liquid, i.e., compound 137-1. ESI-MS m/z: 293.30 [M+H]$^+$.

Step 2: Synthesis of compound 137-2

Sodium ascorbate (142.28 mg, 0.72 mmol) and copper sulfate (38.21 mg, 0.24 mmol) were added to compound 137-1 (70.00 mg, 0.24 mmol) and M29 (120.21mg, 0.26 mmol) in tert-butanol (1.50 mL), tetrahydrofuran (1.50 mL) and water (1.00 mL). The mixture was reacted at 20°C for 1 hour. The reaction was monitored by LCMS until completion. The reaction solution was added to 30 mL of water, extracted with DCM, and a little insoluble solid was precipitated, and dissolved with appropriate amount of methanol. The organic phases were combined, dried, filtered and concentrated under reduced pressure. The residue was purified by column chromatography (MeOH:DCM = 15%:85%) to obtain 134.00 mg of a brown solid, i.e., compound 137-2. ESI-MS m/z: 749.30 [M+H]$^+$.

Step 3: Synthesis of compound 137-3

Trifluoroacetic acid (1.39 mL 18.69 mmol) was added to compound 137-2 (140.00 mg, 0.19 mmol) in dichloromethane (5.20 mL), and the mixture was reacted at 20°C for 0.5 hours. The reaction solution was added to 20 mL of saturated sodium bicarbonate solution, and extracted 5 times with chloroform/ isopropanol. The organic phases were combined, dried, filtered, and concentrated under reduced pressure. Then, 110.00 mg of a brown solid was obtained, i.e., compound 137-3. The resultant was directly fed into the next step. ESI-MS m/z: 649.30 $[M+H]^+$.

Step 4: Synthesis of compound 137-4

Zinc chloride (0.34 mL, 1.00 mol/L, 0.34 mmol) was added to compound 137-3 (120.43 mg, 0.19 mmol), and M28 (100.00 mg, 0.17 mmol) in methanol (2.00 mL). The reaction was heated to 40°C and stirred for 2 hours. Then, sodium cyanoborohydride (42.40 mg, 0.67 mmol) was added. The reaction was stirred at 40°C for 16 hours. The reaction solution was added to 25 mL of water, and 15mL of DCM was supplemented. The organic phase was separated, dried and concentrated under reduced pressure. The residue was purified by column chromatography (MeOH:DCM = 15%:85%) to obtain 110.00 mg of a white solid, i.e., compound 137-4. ESI-MS m/z: 613.7 1/2$[M+2H]^+$.

Step 5: Synthesis of compound 137

Trifluoroacetic acid (0.48 mL, 6.53 mmol) was added to compound 137-4 (80.00 mg, 0.07 mmol) in dichloromethane (2.00 mL), and the mixture was reacted at 20°C for 0.5 hours. The reaction solution was concentrated under reduced pressure without heating and the residue was purified by Pre-HPLC to obtain 40.00 mg of white powder, i.e., compound 137.

ESI-MS m/z: 591.8 1/2$[M+2H]^+$.

$^1$H NMR (500 MHz, Methanol-$d_4$) δ 9.21 (s, 1H), 8.87 (s, 1H), 7.90 (s, 1H), 7.68-7.65 (m, 1H), 7.45-7.40 (m, 4H), 7.30 (s, 1H), 7.28-7.22 (m, 1H), 7.06 (s, 1H), 5.27-5.25 (d, *J*= 10.0 Hz, 1H), 5.05-5.02 (m, 2H), 4.51-4.39 (m, 6H), 4.27-4.22 (m, 1H), 3.90-3.80 (m, 2H), 3.67-3.58 (s, 1H), 3.47-3.45 (m, 1H), 3.34-3.30 (m, 3H), 3.21-3.15 (m, 2H), 3.00-2.93 (m, 2H), 2.75 (s, 1H), 2.54-2.48 (m, 6H),2.37 (s, 4H), 2.22-2.18 (m, 1H), 2.03-1.94 (m, 4H),1.85-1.70 (m, 6H), 1.53-1.51 (d, *J*= 10.0 Hz, 3H), 1.30-1.27 (d, *J*= 15.0 Hz, 3H), 1.13-1.12 (d, *J*= 5.0 Hz, 3H), 0.84-0.81 (m, 2H), 0.74-0.73 (m, 6H), 0.50 (s, 2H).

Example 138 Synthesis of compound (2S,4R)-1-((1-(1-(7-(8-ethyl-7-fluoro-3-hydroxynaphthalen-1-yl)-8-fluoro-4-((R)-3-hydroxy-3-m ethylpiperidin-1-yl)pyrido[4,3-d]pyrimidin-2-yl)oxy)methyl)cyclopropyl)methyl)piperidin-4-yl)-1H-pyrazol-4-yl)-1H-1,2,3-triazol-1-yl)-3-methylbutanoyl)-4-hydroxy-N-(((S)-1-(4-methylthiaz ol-5-yl)phenyl)ethyl) pyrrolidine-2-carboxamide

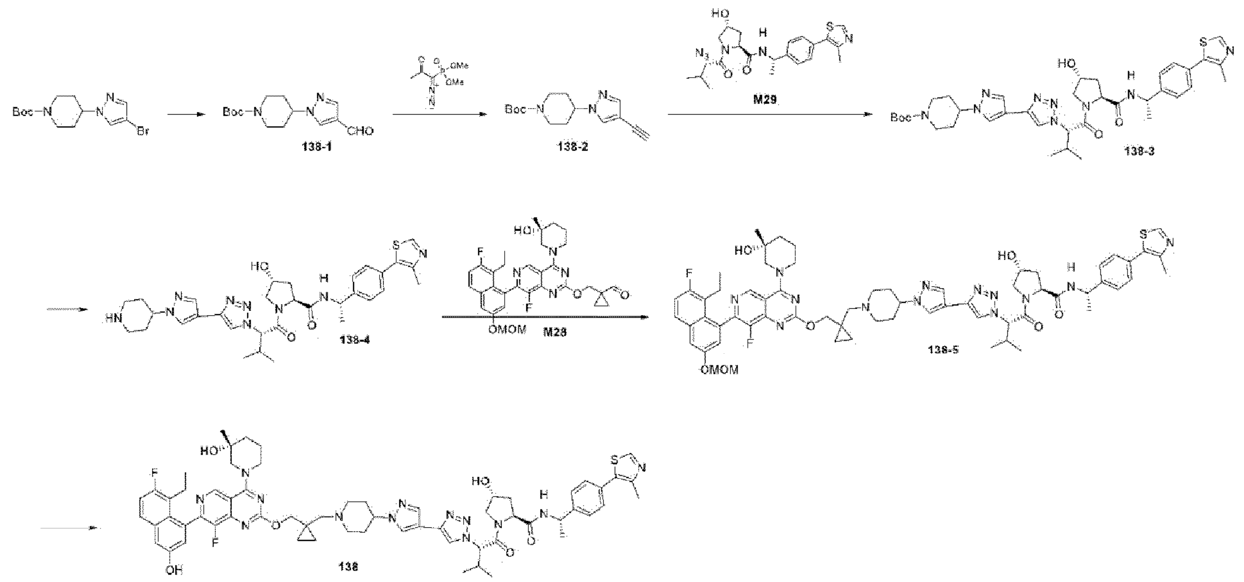

Step 1: Synthesis of compound 138-1

n-Butyl lithium (1.6 M in hexane) (1.45 mL, 2.50 mol/L, 3.63 mmol) was added to tert-butyl 4-(4-bromopyrazol-1-yl)piperidine-1-carboxylate (1000.00 mg, 3.03 mmol) in tetrahydrofuran (10.00 mL) under the protection of nitrogen and cooled to -78°C, and the mixture was reacted at -78°C for 15 minutes. Then N,N-dimethylformamide (0.28 mL, 3.63 mmol) was added dropwise, and the mixture was reacted at -78°C for 0.5 hours. The reaction was monitored by LCMS until completion. The reaction solution was added to 20 mL of saturated ammonium chloride, and extracted with EA. The organic phase was separated, dried, filtered and concentrated under reduced pressure. The residue was purified by column chromatography (EA:PE = 35%:65%) to obtain 680.00 mg of colorless liquid, i.e., compound 138-1. ESI-MS m/z: 280.30 $[M+H]^+$.

Step 2: Synthesis of compound 138-2

Dimethyl (1-diazo-2-oxopropyl) phosphonate (649.93 mg, 3.38 mmol) was added dropwise to compound 138-1 (630.00 mg, 2.26 mmol), and potassium carbonate (935.16 mg, 6.77 mmol) in methanol (15.00 mL). The mixture was reacted at 20°C for 16 hours. The reaction solution was added to 50 mL of water, and extracted with EA. The organic phase was separated, dried, filtered and concentrated under reduced pressure. The residue was purified by column chromatography (EA:PE = 30%:70%) to obtain 500.00 mg of a white solid, i.e., compound 138-2. ESI-MS m/z: 276.30 $[M+H]^+$.

Step 3: Synthesis of compound 138-3

Sodium ascorbate (431.72 mg, 2.18 mmol) and copper sulfate (115.94 mg, 0.73 mmol) were added to compound 138-2 (200.00 mg, 0.73 mmol), and M29 (364.79 mg, 0.80 mmol) in tert-butanol (5.00 mL), tetrahydrofuran (5.00 mL) and water (2.50 mL). The mixture was reacted at 20°C for 1 hour. The reaction was monitored by LCMS until completion. The reaction solution was added to 30 mL of water, and extracted three times with DCM. The organic phases were combined, dried, filtered and concentrated under reduced pressure. The residue was purified by column chromatography (MeOH:DCM = 10%:90%) to obtain 430.00 mg of a brown solid, i.e., compound 138-3. ESI-MS m/z: 732.10 $[M+H]^+$.

Step 4: Synthesis of compound 138-4

Trifluoroacetic acid (4.36 mL, 58.75 mmol) was added to compound 138-3 (430.00 mg, 0.59 mmol) in dichloromethane (17.00 mL), and the mixture was reacted at 20°C for 0.5 hours. The reaction solution was added to 20 mL of saturated sodium bicarbonate solution, and the mixture was stirred at room temperature for 15 minutes. The resultant was extracted with chloroform/ isopropanol, and the organic phases were combined, dried, filtered, and concentrated under reduced pressure. Then, 360.00 mg of a brown solid was obtained, i.e., compound 138-4. The resultant was directly fed into the next step. ESI-MS m/z: 632.30 $[M+H]^+$.

Step 5: Synthesis of compound 138-5

1 M zinc chloride in diethyl ether (0.35 mL) was added to M28 (120.00 mg, 0.17 mmol) and compound 138-4 (120.92 mg, 0.19 mmol) in methanol (6.00 mL). The reaction was heated to 40°C and stirred for 2 hours. Then, sodium cyanoborohydride (43.74 mg, 0.70 mmol) was added. The reaction was stirred at 40°C for 16 hours. The reaction was monitored by LCMS until completion. The reaction solution was added to 25 mL of water, and 20 mL of DCM was supplemented. The organic phase was separated, dried, filtered and concentrated under reduced pressure. The residue was purified by column chromatography (MeOH:DCM = 10%:90%) to obtain 80.00 mg of a colorless solid, i.e., compound 138-5. ESI-MS m/z: 605 1/2$[M+2H]^+$.

Step 6: Synthesis of compound 138

Trifluoroacetic acid (0.74 mL, 9.94 mmol) was added to compound 138-5 (60.00 mg, 0.05 mmol) in dichloromethane (3.00 mL), and the mixture was reacted at 20°C for 0.5 hours. The reaction was monitored by LCMS until completion. The reaction solution was concentrated under reduced pressure and the residue was purified by Pre-HPLC to obtain 38.90 mg of white powder, i.e., compound 138.

ESI-MS m/z: 583.1 1/2$[M+2H]^+$.

$^1$H NMR (500 MHz, Methanol-$d_4$) δ 9.21 (s, 1H), 8.87 (s, 1H), 8.25 (s, 1H), 8.05 (s, 1H), 7.84 (s, 1H), 7.67-7.64 (m, 1H), 7.45-7.40 (m, 4H), 7.29 (s, 1H), 7.25-7.21 (m, 1H), 7.05 (s, 1H), 5.32-5.30 (d, *J*= 10.0 Hz, 1H), 5.05-5.02 (m, 3H), 4.54-4.44 (m, 6H), 4.27-4.22 (m, 2H), 3.90-3.85 (m, 2H), 3.67-3.64 (s, 1H), 3.47-3.45 (m, 1H), 3.21-3.15 (m, 2H), 2.47 (s, 3H), 2.19-2.13 (m, 6H), 2.08-1.97 (m, 5H), 1.79-1.77 (m, 2H),1.52-1.51 (d, *J*= 5.0 Hz, 3H), 1.29-1.27 (d, *J*= 10.0 Hz, 3H),

1.15-1.14 (d, $J$= 5.0 Hz, 3H), 0.81-0.78 (m, 6H), 0.73 (s, 2H), 0.52 (s, 2H),

Example 139 Synthesis of compound (3S,7aS)-7a-(((7-(8-ethyl-7-fluoro-3-hydroxy naphthalen-1-yl)-8-fluoro-4-((R)-3-hydroxy-3-methylpiperidin-1-yl)pyrido[4,3-d]pyrimidin-2-yl )oxy)methyl)-6-methylenehexahydro-1H-pyrrazin-3-yl) methyl 2-(((S)-3-((2S,4R)-4-hydroxy -1-(3-methyl-2-(3-methylisoxazole-5-yl)-2$\lambda^3$-butanoyl)pyrrolidin-2-carboxamide)-3-(4-(4-methylthiazole-5-yl)phenyl)propanamide)methyl)-7-azaspiro[3.5]nonane-7-carboxylate

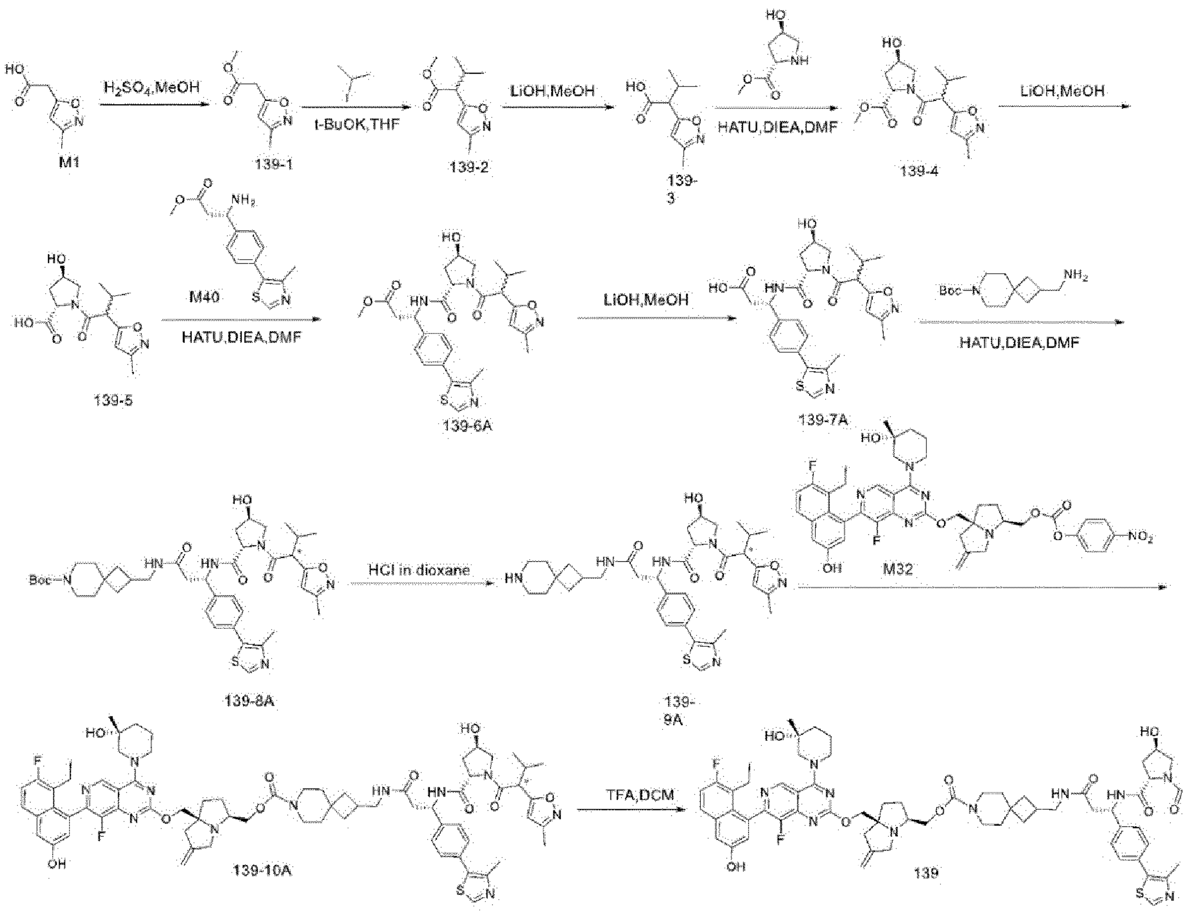


Step 1: Synthesis of compound 139-1

Compound M1 (2.0 g) was added to a 50 mL single-necked flask, dissolved in methanol (30 mL), and then concentrated sulfuric acid (1.0 mL) was added. The reaction was heated to 80°C overnight. The reaction solution was directly evaporated to dryness to obtain 2.1 g of a light yellow oil, i.e., compound 139-1.

Step 2: Synthesis of compound 139-2

Compound 139-1 (2.1 g) was added to a 50 mL single-necked flask, and dissolved in THF (20 mL). Then, 2-iodopropane (3.45 g), and potassium tert-butoxide (3.34 g) were added. The mixture was reacted at room temperature for 5 hours. The reaction solution was directly evaporated to dryness. The resultant was separated and purified by column chromatography (PE: EA = 5:1) to obtain 2.1 g of a light yellow solid, i.e., compound 139-2.

Step 3: Synthesis of compound 139-3

Compound 139-2 (2.1 g) was added to a 50 mL single-necked flask, and dissolved in methanol (20 mL). Then, lithium hydroxide (0.89 g) and water (4 mL) were added. The mixture was reacted at room temperature for 2 hours. The pH of the reaction solution was adjusted to pH=2 to 3 with dilute sulfuric acid, and then saturated brine (5 mL) was added to the reaction solution. The mixture was extracted with EA, and the organic phase was dried with anhydrous sodium sulfate to

obtain 1.78 g of a yellow solid, i.e., compound 139-3.

Step 4: Synthesis of compound 139-4

Compound 139-3 (1.78 g) was added to a 50 mL single-necked flask, and dissolved in DMF (20 mL). Then, methyl (2S,4R)-4-hydroxypyrrolidine-2-carboxylate (1.69 g), DIPEA (4.82 g) and HATU (5.54 g) were added. The mixture was reacted at room temperature for 1 hour. Saturated brine (5 mL) was added to the reaction solution. The mixture was extracted with EA, and the organic phase was dried with anhydrous sodium sulfate. The resultant was separated by column chromatography (PE: EA = 1:1) to obtain 2.21 g of a yellow solid, i.e., compound 139-4.

Step 5: Synthesis of compound 139-5

Compound 139-4 (2.2 g) was added to a 50 mL single-necked flask, and dissolved in methanol (2 mL). Then, lithium hydroxide (1.5 g) and water (4 mL) were added. The mixture was reacted at room temperature for 2 hours. The pH of the reaction solution was adjusted to pH=2 to 3 with dilute sulfuric acid, and then saturated brine (5 mL) was added to the reaction solution. The mixture was extracted with EA, and the organic phase was dried with anhydrous sodium sulfate to obtain 1.98 g of a yellow solid, i.e., compound 139-5.

Step 6: Synthesis of compound 139-6A

Compound 139-5 (0.98 g) was added to a 50 mL single-necked flask, and dissolved in DMF (10 mL). Then, M40 (1.1 g), DIPEA (1.4 mL) and HATU (1.9 g) were added. The mixture was reacted at room temperature for 1 hour. Saturated brine (5 mL) was added to the reaction solution. The mixture was extracted with EA, and the organic phase was dried with anhydrous sodium sulfate. The resultant was separated by column chromatography (PE: EA = 1:1) and resolved by chiral column to obtain 0.34 g of a white solid, i.e., compound 139-6A.

Step 7: Synthesis of compound 139-7A

Compound 139-6A (0.3 g) was added to a 25 mL single-necked flask, and dissolved in methanol (5 mL). Then, lithium hydroxide (0.1 g) and water (1 mL) were added. The mixture was reacted at room temperature for 2 hours. The pH of the reaction solution was adjusted to pH=2 to 3 with dilute sulfuric acid, and then saturated brine (5 mL) was added to the reaction solution. The mixture was extracted with EA, and the organic phase was dried with anhydrous sodium sulfate to obtain 0.27 g of a yellow solid, i.e., compound 139-7A.

Step 8: Synthesis of compound 139-8A

Compound 139-7A (0.27 g) was added to a 25 mL single-necked flask, and dissolved in DMF (20 mL). Then, 2-aminomethyl-7-tert-butoxycarbonyl-7-azaspiro[3.5]nonane (0.14 g), DIPEA (0.2 mL) and HATU (0.28 g) were added. The mixture was reacted at room temperature for 1 hour. Saturated brine (5 mL) was added to the reaction solution. The mixture was extracted with EA, and the organic phase was dried with anhydrous sodium sulfate. The resultant was separated by column chromatography (DCM:MeOH = 10:1) to obtain 0.27 g of a light yellow solid, i.e., compound 139-8A.

Step 9: Synthesis of compound 139-9A

Compound 139-8A (0.27 g) was added to a 25 mL single-necked flask, and dissolved in 4 M hydrogen chloride in dioxane (10 mL). Then, methanol (1 mL) was added. The mixture was reacted at room temperature for 1 hour. The reaction solution was directly evaporated to dryness to obtain 0.16 g of a yellow solid, i.e., compound 139-9A.

Step 10: Synthesis of compound 139-10A

Compound 139-9A (0.16 g) was added to a 25 mL single-necked flask, and dissolved in THF (10 mL). Then, M32 (0.23 g) was added. The mixture was reacted at room temperature for 1 hour. The reaction solution was directly evaporated to dryness to obtain 0.11 g of a light yellow solid, i.e., compound 139-10A.

Step 11: Synthesis of compound 139

Compound 139-10A (45.0 mg) was added to a 25 mL single-necked flask, and dissolved in DCM (2 mL). Then, TFA (2 mL) was added. The mixture was reacted at room temperature for 1 hour. The reaction solution was directly

evaporated to dryness. After 5 mL of saturated sodium bicarbonate solution was added to the reaction solution to adjust the pH of the solution until pH=7 to 8, the reaction solution was extracted with a mixed solution of dichloromethane and isopropanol. The organic phase was dried with anhydrous sodium sulfate, and the resultant was separated by reverse-phase chromatography ($CH_3CN/H_2O$ = 30%-55%) to obtain 1.2 mg of a white solid, i.e., compound 139.

ESI-MS m/z: 668.28 1/2[M+2H]$^+$.
$^1$H NMR (500 MHz, Methanol-$d_4$) δ 9.21 (d, *J* = 4.2 Hz, 1H), 8.87 (d, *J* = 5.7 Hz, 1H), 7.69 - 7.63 (m, 1H), 7.39 - 7.34 (m, 5H), 7.30 (d, *J*= 2.5 Hz, 1H), 7.24 (t, *J* = 8.9 Hz, 1H), 7.06 (dd, *J* = 10.0, 2.7 Hz, 1H), 4.61 - 4.57 (m, 8H), 4.51 - 4.42 (m, 5H), 4.35 - 4.21 (m, 4H), 4.17 - 4.08 (m, 2H), 4.06 - 3.99 (m, 5H), 3.87-3.81 (m, 3H), 3.75 - 3.57 (m, 5H), 3.01 - 2.88 (m, 3H), 2.79 - 2.63 (m, 6H), 2.50 - 2.36 (m, 3H), 2.27 - 2.15 (m, 4H), 2.03 - 1.82 (m, 5H), 1.63 - 1.37 (m, 5H), 1.38 - 1.25 (m, 2H), 1.05 - 0.90 (m, 3H), 0.83 - 0.76 (m, 3H).

Example 140 Synthesis of compound ((3 S,7aS)-7a-(((7-(8-ethyl-7-fluoro-3-hydroxynaphthalen-1-yl)-8-fluoro-4-((R)-3-hydroxy-3-met hylpiperidin-1-yl)pyrido[4,3-d]pyrimidin-2-yl)oxy)methyl)-6-methylenehexahydro-1H-pyrrolidi n-3-yl) methyl 2-(((S)-1-((2S,4R)-4-hydroxy-2-(((S)-1-(4-(4-methylthiazol-5-yl)phenyl)ethyl)carbamoyl)pyrroli din-1-yl)-3,3-dimethyl-1-oxobutan-2-yl)carbamoyl-7-azaspiro[3.5]nonane-7-carboxylate

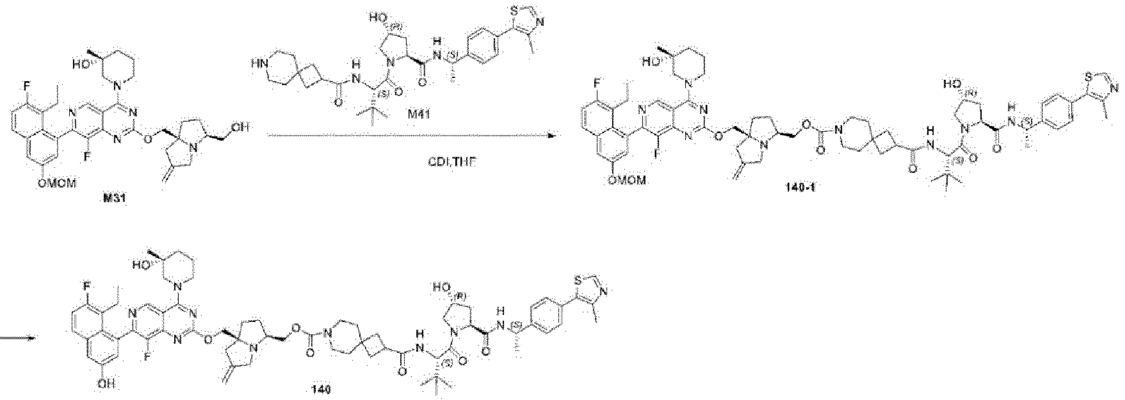


Step 1: Synthesis of compound 140-1

M31 (28 mg) was dissolved in 3 mL of THF, and then 9 mg of CDI was added. The mixture was reacted at 40°C for 1 hour, and the intermediate was monitored by LC-MS. Then, M15 N-((S)-1-((2S,4R)-4-hydroxy-2-(((S)-1-(4-(4-methylthiazol-5-yl)phenyl)ethyl)carbamoyl)pyrroli din-1-yl)-3,3-dimethyl-1-oxobutan-2-yl)-7-azaspiro[3.5]nonane-2-carboxamide (37 mg) was added. The mixture was stirred at 80°C for 12 hours, and the reaction was monitored by LC-MS until completion. The reaction was quenched by adding 5.00 mL of methanol, and the organic phase was concentrated. The resultant was separated and purified by column chromatography to obtain 24 mg of a light yellow solid, i.e., compound 140-1. ESI-MS m/z: 650, 1/2[M+2H]$^+$.

Step 2: Synthesis of compound 140

Compound 140-1 (24 mg) was dissolved in 1.5 mL of DCM, then 0.5 mL of TFA was added. The mixture was reacted at room temperature for 30 minutes, and the reaction was monitored by LCMS until completion. The reaction was monitored by LCMS until completion. The reaction solution was concentrated directly, and the resultant was separated by preparative liquid chromatography to obtain 6 mg of white powder, i.e., compound 140. ESI-MS m/z: 628, 1/2[M+2H]$^+$.
$^1$H NMR (500 MHz, DMSO-$d_6$) δ 9.93 (s, 1H), 9.22 (d, *J* = 3.6 Hz, 1H), 8.98 (s, 1H), 8.38 (d, *J* = 7.7 Hz, 1H), 7.76 (s, 1H), 7.63 (d, *J* = 8.2 Hz, 1H), 7.44 - 7.20 (m, 6H), 7.04 - 7.02 (m, 1H), 5.11 (d, *J* = 2.8 Hz, 1H), 4.95- 4.91 (m, 3H), 4.75 (d, *J* = 11.4 Hz, 1H), 4.50 (d, *J*= 9.2 Hz, 1H), 4.41 (t, *J* = 8.0 Hz, 1H), 4.32 - 4.21 (m, 2H), 4.11 - 4.00 (m, 2H), 3.96- 3.90 (m, 2H), 3.82-3.78 (m, 1H), 3.63-3.51 (m, 4H), 3.28-3.17 (m, 5H), 2.94 (s, 1H), 2.64 - 2.53 (m, 1H), 2.45 (s, 4H), 2.34 (d, *J* = 16.4 Hz, 2H), 2.12- 1.87 (m, 8H), 1.80 - 1.65 (m, 8H), 1.49 (s, 2H), 1.41-1.30(m, 5H), 1.16 (d, *J* = 11.6 Hz, 3H), 0.92 (s, 9H), 0.73 (q, *J* = 7.3 Hz, 3H).

Example 141 Synthesis of compound ((3S,7aS)-7a-(((7-(8-ethyl-7-fluoro-3-hydroxynaphthalen-1-yl)-8-fluoro-4-((R)-3-hydroxy-3-met hylpiperidin-1-yl)pyrido[4,3-d]pyrimidin-2-yl)oxy)methyl)-6-methylenehexahydro-1H-pyrrolizi n-3-yl) methyl 4-((1-((S)-1-((2S,4R)-4-hydroxy-2-(((S)-1-(4-(4-methylthiazol-5-yl) phenyl) ethyl)carbamoyl)pyrrolidin-1-yl)-3-methyl-1-oxobutan-2-yl)-1H-1,2,3-triazol-4-yl)methyl)piperi dine-1-carboxylate

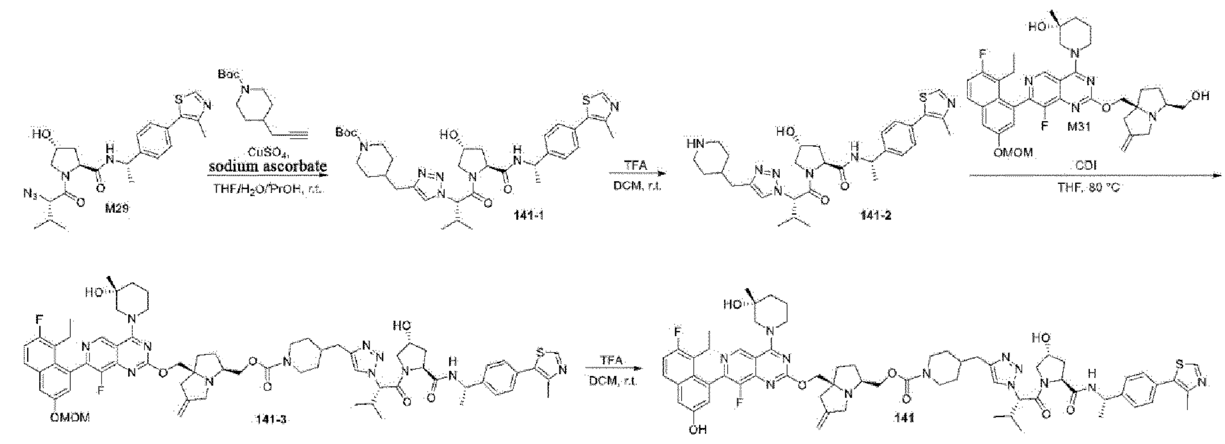


Step 1: Synthesis of compound 141-1

M29 (100.0 mg), tert-butyl 4-(prop-2-yn-1-yl)piperidin-1-carboxylate (97.8 mg), anhydrous copper sulfate (31.5 mg) and sodium ascorbate (112.8 mg) were dissolved in a mixed solvent of THF/t-butanol/water (2/2/2 mL). The mixture was reacted at room temperature for 3 hours. The reaction was monitored by LC-MS until completion, DCM (10 mL) was added to the reaction solution. The mixture was washed once with saturated brine, and the organic phase was dried with anhydrous sodium sulfate, filtered and concentrated. The resultant was separated and purified by column chromatography to obtain 95.0 mg of a yellow solid, i.e., compound 141-1. ESI-MS m/z: 680 $[M+H]^+$.

Step 2: Synthesis of compound 141-2

Compound 141-1 (95.0 mg) was added to DCM (5 mL) and TFA (2 mL), and the mixture was reacted at room temperature for 10 minutes, the reaction was monitored by LCMS until completion. The reaction solution was concentrated directly, and 1M NaOH was added to the reaction solution until pH=8. DCM (20 mL) was added to the reaction solution. The organic phase was collected, washed once with saturated brine, dried with anhydrous sodium sulfate, filtered and concentrated to obtain 80.0 mg of a white solid, i.e., compound 141-2. ESI-MS m/z: 580 $[M+H]^+$.

Step 3: Synthesis of compound 141-3

M31 (30.0 mg) was dissolved in THF (2 mL), and then CDI (10.8 mg) was added. The mixture was reacted at 40°C for 1 hour, and then compound 141-2 (38.6 mg) was added. The mixture was reacted at 80°C for 4 hours, and the reaction was monitored by LCMS until completion. The reaction was quenched by adding methanol (5.0 mL), and the reaction solution was concentrated directly. The resultant was separated and purified by column chromatography to obtain 29.0 mg of a light yellow solid, i.e., compound 141-3. ESI-MS m/z: 1282 $[M+H]^+$.

Step 4: Synthesis of compound 141

Compound 141-3 (29.0 mg) was added to DCM (5 mL) and TFA (2 mL), and the mixture was reacted at room temperature for 10 minutes, the reaction was monitored by LCMS until completion. The reaction solution was concentrated directly, and the resultant was separated by preparative liquid chromatography to obtain 11.3 mg of white powder, i.e., compound 141.

ESI-MS m/z: 620 1/2$[M+2H]^+$.

$^1$H NMR (500 MHz, Methanol-$d_4$) δ 9.22 (d, *J* = 9.5 Hz, 1H), 8.87 (s, 1H), 7.88 (s, 1H), 7.68-7.65 (m, 1H), 7.44-7.38 (m, 4H), 7.30 (d, *J* = 3.0 Hz, 1H), 7.24 (t, *J* = 4.0 Hz, 1H), 7.08-7.06 (m, 1H), 5.26 (d, *J* = 10.5 Hz, 1H), 5.03 (d, *J* = 7.0 Hz, 1H),

4.30-4.24 (m, 3H), 4.17-4.07 (m, 5H), 3.94-3.79 (m, 3H), 3.69-3.56 (m, 3H), 3.47-3.42 (m, 2H), 3.07-2.98 (m, 3H), 2.81-2.63 (m, 6H), 2.47 (s, 1H), 2.23-2.18 (m, 5H), 2.05-2.03 (m, 2H), 1.98-1.93 (m, 1H), 1.85-1.78 (m, 5H), 1.67-1.61 (m, 3H), 1.51 (d, *J* = 7.0 Hz, 2H), 1.30-1.28 (m, 6H), 1.12 (d, *J* = 6.5 Hz, 3H), 0.83-0.79 (m, 3H), 0.74 (d, *J* = 6.5 Hz, 3H).

Example 142 Synthesis of compound (2S,4R)-1-((S)-2-(4-(6-(4-((1-(((7-(8-ethyl-7-fluoro-3-hydroxynaphthalen-1-yl))-8-fluoro-4-((R)-3-hydroxy-3-methylpiperidin-1-yl)pyrido[4,3-d]pyrimidin-2-yl)oxy)methyl)cyclopropyl)methyl) piperazin-1-yl)pyridin-3-yl)-1H-1,2,3-triazol-1-yl)-3-methylbutanoyl)-4-hydroxy-N-((S)-1-(4-(4-methylthiazol-)5-yl)phenyl)ethyl)pyrrolidine-2-carboxamide

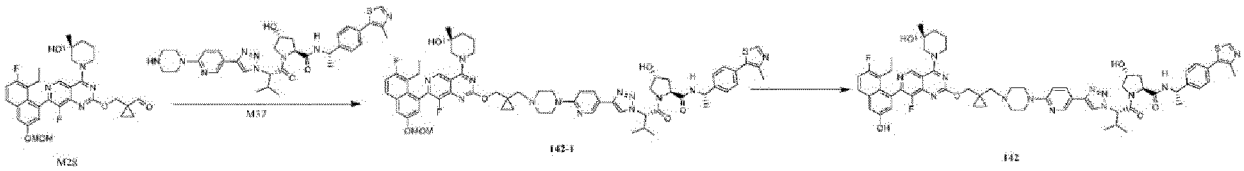


Step 1: Synthesis of compound 142-1

M28 (100 mg), M37 (100 mg), and MeOH (5 mL) were added to a 50 mL flask, and then 1 M zinc chloride in diethyl ether (0.3 μL) was added. The reaction was heated to 45°C for 1 hour. Sodium cyanoborohydride (100 mg) was then added, and the reaction was continued at 45°C for 12 hours. The reaction solution was diluted by adding DCM, washed with water and then washed with brine, dried with anhydrous sodium sulfate, and filtered. The filtrate was precipitated under reduced pressure to obtain a crude product, and the crude product was purified by column chromatography to obtain compound 142-1 (85 mg).

Step 2: Synthesis of compound 142

Compound 142-1 (70 mg), DCM (3 mL), and TFA (1 mL) were added to a 50 mL flask, and the mixture was reacted at room temperature for 0.5 hours. After detection, the mixture was precipitated under reduced pressure, dissolved by adding DCM, and the pH of the solution was adjusted to pH=7 to 8 with saturated aqueous sodium bicarbonate solution. The mixture was stirred, the layers were separated, washed with brine, dried with sodium sulfate, and filtered. The filtrate was precipitated under reduced pressure to obtain a crude product. The resultant was purified by Pre-HPLC to obtain compound 142 (33.5 mg).

ESI-MS m/z: 589.28 1/2[M+2H]$^+$.

$^1$H NMR (500 MHz, DMSO-$d_6$) ppm 9.94 (s, 1H), 9.22 (s, 1H), 8.99 (s, 1H), 8.68-8.46 (m, 111), 8.00 (dd, *J* = 8.77, 2.07 Hz, 1H), 7.76 (dd, *J* = 9.07, 6.03 Hz, 1H), 7.52-7.27 (m, 1H), 7.04 (d, *J* = 2.51 Hz, 1H), 6.88 (d, *J* = 8.89 Hz, 1H), 5.80-5.72 (m, 1H), 5.32 (d, *J* = 10.26 Hz, 1H), 5.25-4.69 (m, 1H), 4.48-4.23 (m, 1H), 4.14-3.95 (m, 1H), 3.86-3.17 (m, 1H), 2.63-2.25 (m, 1H), 2.23-1.91 (m, 1H), 1.87-1.51 (m, 1H), 1.48-0.60 (m, 1H), 0.46 (s, 1H).

Example 143 Synthesis of compound ((7-(8-ethyl-7-fluoro-3-hydroxynaphthalen-1-yl)-8-fluoro-4-((R)-3-hydroxy-3-methylpiperidin-1 -yl)pyrido[4,3-d]pyrimidin-2-yl)oxy)methyl)-6-methylenehexahydro-1H-pyrrolidin-3-yl)methyl 2-(1-((S)-1-(2S,4R)-4-hydroxy-2-((S)-1-(4-(4-methylthiazol-5-yl)phenyl)ethylcarbamoyl)pyrroli din-1-yl)-3-methyl-1-oxobutan-2-yl)-1H-1,2,3-triazol-4-yl)-7-azaspiro[3.5]nonane-7-carboxylate

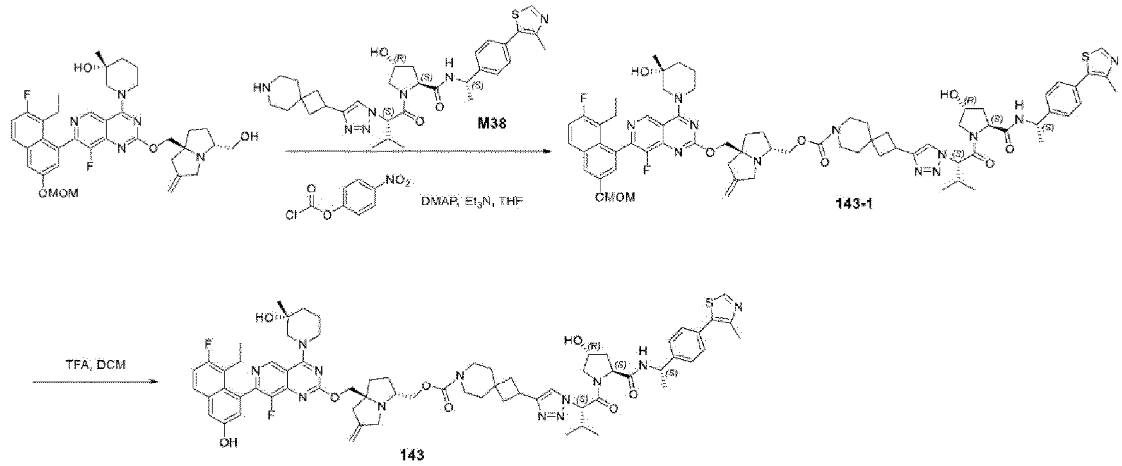


Step 1: Synthesis of compound 143-1

Compound M38 (70 mg) was dissolved in 5 mL of THF, and then triethylamine (0.12 mL), 4-dimethylaminopyridine (4 mg), and p-nitrophenyl chloroformate (32 mg) were added. The mixture was stirred at room temperature for 12 hours, and then compound (R)-1-(7-(8-ethyl-7-fluoro-3-(methoxymethoxy)naphthalen-1-yl)-8-fluoro-2-(((5R,7aS)-5-(hydro xymethyl)-2-methyltetrahydro-1H-pyrrolizin-7a(5H)-yl)methoxy]pyrido[4,3-d]pyrimidin-4-yl)-3 -methylpiperidin-3-ol (88 mg) was added. The reaction was continued for 0.5 hours. The reaction solution was quenched by adding methanol, and concentrated under reduced pressure. The resultant was purified by column chromatography (DCM/ammonia in methanol = 20:1) to obtain compound 143-1 (96 mg), ESI-MS m/z: 654 1/2$[M+2H]^+$.

Step 2: Synthesis of compound 143

Compound 143-1 (96 mg) was dissolved in 4 mL of DCM, and then 4 mL of trifluoroacetic acid was added. The mixture was reacted at room temperature for 1 hour. The reaction solution was concentrated directly, and redissolved with 5.00 mL of DCM, then concentrated again. The resultant was separated by preparative liquid chromatography to obtain 24.6 mg of white powder, i.e., compound 143.

ESI-MS m/z: 632 1/2$[M+2H]^+$.

$^1$H NMR (500 MHz, DMSO-$d_6$) δ: 9.93 (d, $J$ = 2.5 Hz, 1H), 9.22 (d, $J$ = 4.0 Hz, 1H), 8.99 (s, 1H), 8.50 (d, $J$ = 7.5 Hz, 1H), 7.98 (s, 1H), 7.76 (dd, $J$ = 9.0, 6.0 Hz, 1H), 7.44 (d, $J$ = 8.0 Hz, 2H), 7.39 - 7.30 (m, 4H), 7.03 (d, $J$ = 2.5 Hz, 1H), 5.21 (d, $J$ = 10.4 Hz, 1H), 5.16 (d, $J$ = 3.5 Hz, 1H), 4.96 - 4.84 (m, 3H), 4.75 (d, $J$ = 11.0 Hz, 1H), 4.40 - 4.26 (m, 3H), 4.20 - 4.00 (m, 5H), 3.75 (d, $J$ = 10.5 Hz, 4.0 Hz, 1H), 3.64 (d, $J$ = 11.2 Hz, 1H), 3.62 - 3.48 (m, 3H), 3.40 - 3.34 (m, 4H), 3.27 - 3.22 (s, 2H), 2.65 - 2.62 (m, 1H), 2.46 (s, 3H), 2.43 - 2.31 (m, 4H), 2.21 - 2.10 (m, 3H), 2.08 - 1.97 (m, 2H), 1.96 - 1.83 (m, 4H), 1.82 - 1.55 (m, 8H), 1.51 - 1.45 (m, 2H), 1.37 (d, J = 7.0 Hz, 3H), 1.17 (d, J = 10.5 Hz, 3H), 1.04 (d, J = 6.5 Hz, 3H), 0.75 - 0.70 (m, 3H), 0.64 (d, J = 6.5 Hz, 3H) .

Example 144 Synthesis of compound (2S,4R)-1-((S)-2-(4-(5-(1-((((7-(8-ethyl-7-fluoro-3-hydroxynaphthalen-1-yl)-8-fluoro-4-(((R)-3-hydroxy-3-methylpiperidin-1-yl)pyrido[4,3-d]pyrimidin-2-yl)oxy)methylcyclopropyl)methyl)pip eridin-4-yl)oxy)pyrazin-2-yl)-1H-1,2,3-triazol-1-yl-3-methylbutanoyl)-4-hydroxy-N-((S)-1-(4-(4 -methylthiazol-5-yl)phenyl)ethyl) pyrrolidine-2-carboxamide

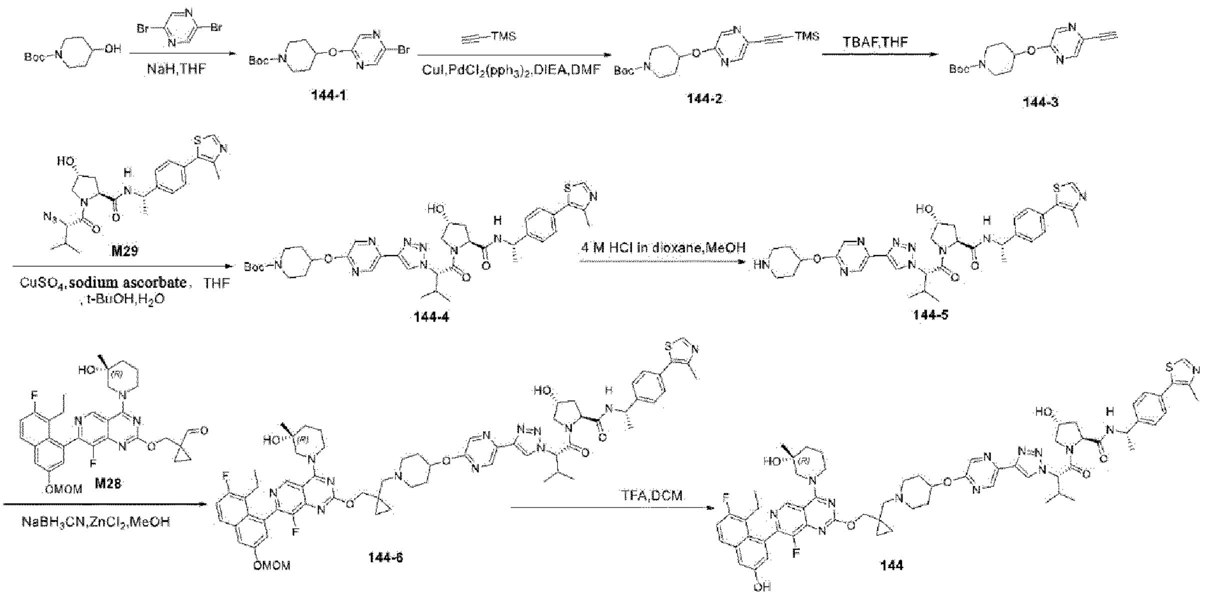

144-1 144-2 144-3 M29 144-4 144-5 M28 144-6 144

Step 1: Synthesis of compound 144-1

Compound N-tert-butoxycarbonyl-4-hydroxypiperidine (1.0 g) was added to a 25 mL single-necked flask, and dissolved in THF (10 mL). Then, NaH (300.0 mg) and 2,5-dibromopyrazine (1.18 g) were added. The reaction was heated to 50°C for 5 hours. The reaction solution was directly evaporated to dryness. The resultant was separated and purified by column chromatography (PE:EA = 6:1) to obtain 1.38 g of a light yellow solid, i.e., compound 144-1.

Step 2: Synthesis of compound 144-2

Compound 144-1 (690.0 mg) was added to a 25 mL single-necked flask, and dissolved in DMF (10 mL). Then, trimethylethynylsilane (283.7 mg), CuI (73.3 mg), $PdCl_2(PPh_3)_2$ (135.2 mg) and DIPEA (0.95 mL) were added. After the protection of nitrogen, the reaction was heated to 50°C for 3 hours. Saturated brine (5 mL) was added to the reaction solution. The mixture was extracted with EA, and the organic phase was dried with anhydrous sodium sulfate. The resultant was separated by column chromatography (PE:EA = 6:1) to obtain 522.2 mg of a light yellow solid, i.e., compound 144-2.

Step 3: Synthesis of compound 144-3

Compound 144-2 (522.2 mg) was added to a 25 mL single-necked flask, and dissolved in THF (10 mL). Then, 1 M TBAF in tetrahydrofuran (2.2 mL) was added. The mixture was reacted at room temperature for 2 hours. The reaction solution was directly evaporated to dryness. The resultant was separated and purified by column chromatography (PE:EA = 4:1) to obtain 378.2 mg of a light yellow solid, i.e., compound 144-3.

Step 4: Synthesis of compound 144-4

Compound M29 (100.0 mg) was added to a 25 mL single-necked flask, and dissolved in THF (2 mL). Then, compound 144-3 (80.2 mg), copper sulfate (35.2 mg), water (2 mL), tert-butanol (2 mL), and sodium ascorbate (160.0 mg) were added. The mixture was reacted at room temperature for 0.5 hours. The reaction solution was directly evaporated to dryness. The resultant was separated and purified by column chromatography (DCM:MeOH = 12:1) to obtain 132.3 mg of a light yellow solid, i.e., compound 144-4.

Step 5: Synthesis of compound 144-5

Compound 144-4 (132.3 mg) was added to a 25 mL single-necked flask, and dissolved in 4M hydrogen chloride in dioxane (5 mL). Then, methanol (1 mL) was added. The mixture was reacted at room temperature for 1 hour. The reaction solution was directly evaporated to dryness. After 5 mL of saturated sodium bicarbonate solution was added to the reaction solution to adjust the pH of the solution until pH=7 to 8, the reaction solution was extracted with a mixed solvent of dichloromethane and isopropanol. The organic phase was dried with anhydrous sodium sulfate, and concentrated under

reduced pressure to obtain 92.2 mg of a yellow solid, i.e., compound 144-5.

Step 6: Synthesis of compound 144-6

Compound 144-5 (92.2 mg) was added to a 25 mL single-necked flask, and dissolved in MeOH (5 mL). Then, M28 (100.0 mg) and 1 M $ZnCl_2$/THF (0.1 mL) were added. After the reaction was heated to 40°C for 2 hours, $NaBH_3CN$ (35.2 mg) was added. The mixture was reacted overnight. The reaction solution was directly evaporated to dryness. The resultant was separated and purified by column chromatography (DCM:MeOH = 10:1) to obtain 84.6 mg of a light yellow solid, i.e., compound 144-6.

Step 7: Synthesis of compound 144

Compound 144-6 (84.6 mg) was added to a 25 mL single-necked flask, and dissolved in DCM (2 mL). Then, TFA (2 mL) was added. The mixture was reacted at room temperature for 1 hour. The reaction solution was directly evaporated to dryness. After 5 mL of saturated sodium bicarbonate solution was added to the reaction solution to adjust the pH of the solution until pH=7 to 8, the reaction solution was extracted with a mixed solution of dichloromethane and isopropanol. The organic phase was dried with anhydrous sodium sulfate, and the resultant was separated and purified by Pre-HPLC ($CH_3CN/H_2O$ = 55%-45%) to obtain 29.3 mg of a white solid, i.e., compound 144 (purity 99.38%).
LCMS: 1/2[M+2H]$^+$= 597.17
$^1$H NMR (500 MHz, Methanol-$d_4$) $\delta$ 9.20 (d, $J$ = 5.0 Hz, 1H), 8.86 (d, $J$ = 7.2 Hz, 1H), 8.72 (s, 1H), 8.54 (d, $J$ = 12.7 Hz, 1H), 8.17 (s, 1H), 7.71 - 7.62 (m, 1H), 7.48 - 7.35 (m, 4H), 7.29 (s, 1H), 7.23 (t, $J$ = 9.2 Hz, 1H), 7.07 (s, 1H), 5.39 (d, $J$ = 10.0 Hz, 1H), 5.12 - 5.00 (m, 3H), 4.58 - 4.41 (m, 5H), 4.24 (t, $J$ = 13.5 Hz, 1H), 3.88 (dd, $J$ = 27.5, 9.3 Hz, 2H), 3.61 - 3.54 (m, 2H), 2.90 (s, 2H), 2.61 (d, $J$ = 6.5 Hz, 1H), 2.53 - 2.42 (m, 6H), 2.35 (s, 2H), 2.24 - 2.11 (m, 3H), 2.08 - 1.94 (m, 3H). 1.89 - 1.71 (m, 5H), 1.52 (d, $J$ = 6.9 Hz, 3H), 1.36 - 1.24 (m, 4H), 1.16 (d, $J$ = 6.5 Hz, 3H), 0.88 - 0.77 (m, 5H), 0.72 (s, 2H), 0.51 (s, 2H),

Example 147 Synthesis of compound (2S,4R)-1-((R)-2-(3-(1-((1-((7-(8-ethyl-7-fluoro-3-hydroxynaphthalen-1-yl)-8-fluoro-4-((R)-3-h ydroxy-3-methylpiperidin-1-yl)pyrido[4,3-d]pyrimidin-2-yl)oxy)methyl)cyclopropyl)methyl)pip eridin-1-yl)isoxazol-5-yl)-3-methylbutanoyl)-4-hydroxy-N-((S)-1-(4-(4-methylthiazol-5-yl)phen yl)ethyl)pyrrolidine-2-carboxamide

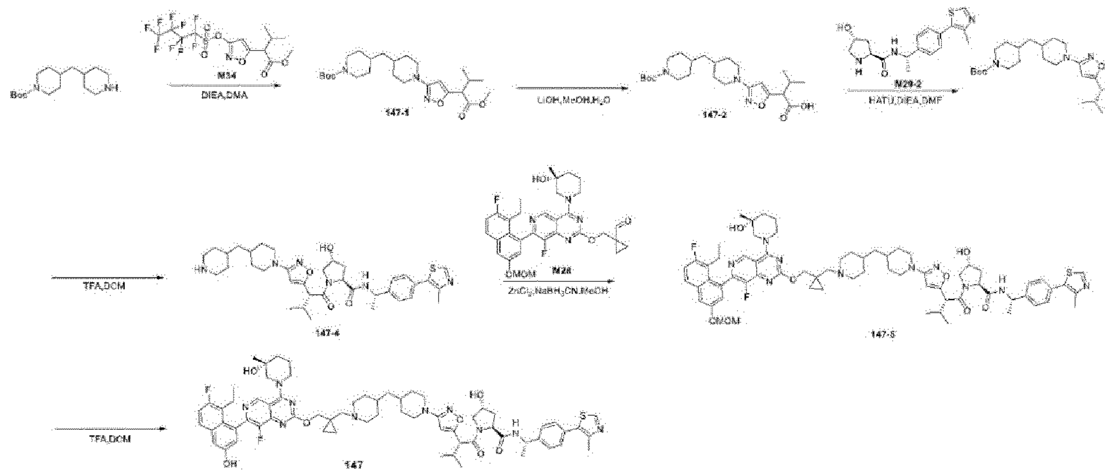


Step 1: Synthesis of compound 147-1

Tert-butyl 4-(piperidin-4-ylmethyl)piperidine-1-carboxylate (270 mg) was added to a 25 mL single-necked flask, and intermediate M3 (550 mg) was added, then DMA (6 mL) was added, and then DIPEA (0.5 mL) was added dropwise. The mixture was reacted at 140°C for 2 hours. The reaction mixture was diluted with water, extracted twice with EA. The organic phases were collected and combined, dried with anhydrous sodium sulfate, and the concentrate was purified by column chromatography to obtain compound 147-1 (234 mg). ESI-MS m/z: 434.3 [M+H]$^+$.

Step 2: Synthesis of compound 147-2

Compound 147-1 (234 mg) was added to a 25 mL single-necked flask, 2 mL of methanol, 2 mL of tetrahydrofuran, and 2 mL of water were added, and then lithium hydroxide (105 mg) was added. The mixture was reacted at room temperature for 30 minutes. The reaction solution was concentrated, and then diluted with water. The pH of the solution was adjusted to pH=5 with saturated sodium bicarbonate solution. The reaction solution was extracted twice with a solution of dichloromethane:isopropanol (5:1), and the organic phases were collected and combined, washed with saturated saline, dried with anhydrous sodium sulfate and concentrated to obtain compound 147-2 (210 mg). ESI-MS m/z: 450.3 $[M+H]^+$.

Step 3: Synthesis of compound 147-3

Compound 147-2 (210 mg), M29-2 (180 mg), HATU (250 mg) and N,N-diisopropylethylamine (0.3 mL) were dissolved in N,N-dimethylformamide (5.00 mL), and the mixture was reacted at 25°C for 1 hour. After the reaction was completed, the mixture was diluted with water and extracted with ethyl acetate. The organic phase was washed with saturated saline and the resultant was purified by column chromatography (dichloromethane/methanol), and resolved by chiral column to obtain compound 147-3 (160 mg). ESI-MS m/z:763.4 $[M+H]^+$.

Step 4: Synthesis of compound 147-4

Compound 147-3 (160 mg) was dissolved in dichloromethane (5 mL), and then trifluoroacetic acid (1 mL) was added. The mixture was reacted at 25°C for 20 minutes. After the reaction was completed, the reaction solution was concentrated, and then diluted with water. The pH of the solution was adjusted to pH=8 to 9 with saturated sodium bicarbonate solution. The reaction solution was extracted twice with dichloromethane:isopropanol (10:1), dried with anhydrous sodium sulfate and concentrated to obtain compound 147-4 (110 mg). ESI-MS m/z:663.4 $[M+H]^+$.

Step 5: Synthesis of compound 147-5

Compound 147-4 (110 mg) was added to a 50 mL single-necked flask, 3 mL of methanol was added, M28 (120 mg) was added, and then 1M zinc chloride in tetrahydrofuran (0.1 mL) was added. The mixture was stirred at room temperature for 1 hour. Sodium cyanoborohydride (40 mg) was added, and the mixture was stirred at 50°C for 3 hours. The reaction solution was quenched with saturated ammonium chloride solution, extracted twice with EA. The organic phases were collected and combined, dried with anhydrous sodium sulfate, and concentrated to obtain compound 147-5 (190 mg). ESI-MS m/z: 598.3 1/2$[M+2H]^+$.

Step 6: Synthesis of compound 147

Compound 147-5 (190 mg) was added to a 25 mL single-necked flask, 2 mL of DCM and 1 mL of trifluoroacetic acid were added. The mixture was reacted at room temperature for 5 hours. The reaction solution was concentrated under reduced pressure, and then the resultant was purified by pre-HPLC to obtain compound 147 (95.5 mg). ESI-MS m/z: 585.3 1/2$[M+2H]^+$.

Example 148 Synthesis of compound (2S,4R)-1-((S)-2-(4-(3-((4-((1-((7-(8-ethyl-7-fluoro-3-hydroxynaphthalen-1-yl)-8-fluoro-4-((R)-3-hydroxy-3-methylpiperidin-1-yl)pyrido[4,3-d]pyrimidin-2-yl)oxy)methyl)cyclopropyl)methyl) piperazin-1-yl)methyl)bicyclic[1.1.1]pent-1-yl)-1H-1,3-triazol-1-yl)-3-methylbutanoyl)-4-hydro xy-N-((S)-1-(4-(4-methylthiazol-5-yl) phenyl)ethyl)pyrrolidine-2-carboxamide

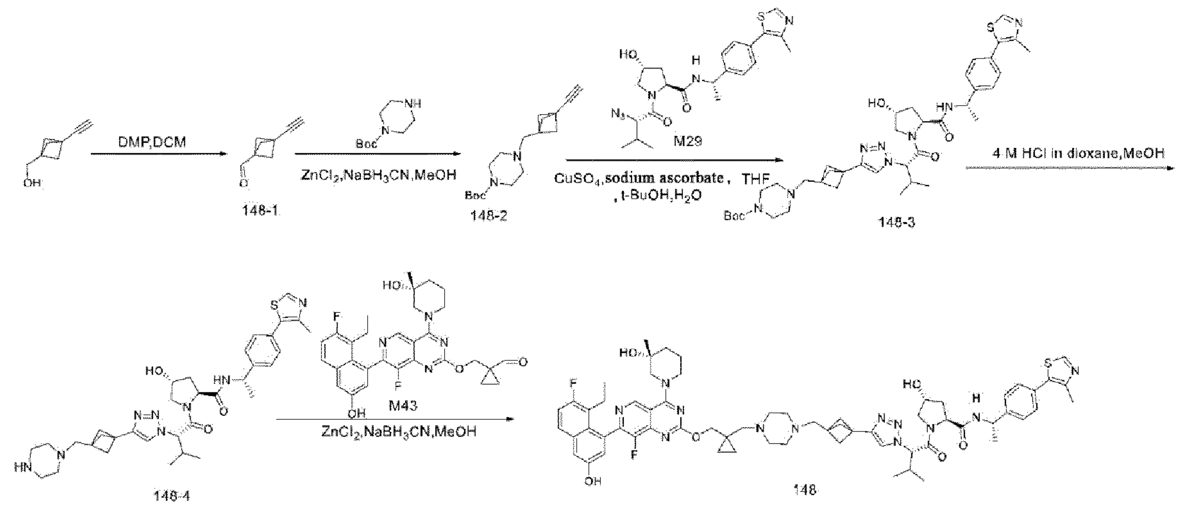


Step 1: Synthesis of compound 148-1

Compound (3-ethylbicyclo[1.1.1]pentan-1-yl)methanol (840.0 mg) was added to a 25 mL single-necked flask, and dissolved in DCM (10 mL). Then, DMP (3.52 g) was added. The mixture was reacted at room temperature for 3 hours. Saturated brine (10 mL) was added to the reaction solution. The mixture was extracted with DCM, and the organic phase was dried with anhydrous sodium sulfate. The resultant was separated by column chromatography (PE:EA = 6:1) to obtain 634.2 mg of a light yellow solid, i.e., compound 148-1.

Step 2: Synthesis of compound 148-2

Compound 148-1 (634.2 mg) was added to a 25 mL single-necked flask, and dissolved in MeOH (5 mL). Then, N-tert-butoxycarbonyl-piperazine (1.2 g) and 1 M zinc chloride in tetrahydrofuran (0.1 mL) were added. After the reaction was heated to 40°C for 2 hours, $NaBH_3CN$ (908.7 mg) was added, and the mixture was reacted overnight. The reaction solution was directly evaporated to dryness, and the resultant was separated and purified by column chromatography (PE:EA = 1:1) to obtain 845.8 mg of a light yellow solid, i.e., compound 148-2.

Step 3: Synthesis of compound 148-3

Compound M29 (100.0 mg) was added to a 25 mL single-necked flask, and dissolved in THF (2 mL). Then, compound 148-2 (70.0 mg), copper sulfate (35.2 mg), water (2 mL), tert-butanol (2 mL), and sodium ascorbate (160.0 mg) were added. The mixture was reacted at room temperature for 0.5 hours. The reaction solution was directly evaporated to dryness, and the resultant was separated and purified by column chromatography (DCM:MeOH = 12:1) to obtain 101.2 mg of a light yellow solid, i.e., compound 148-3.

Step 4: Synthesis of compound 148-4

Compound 148-3 (101.2 mg) was added to a 20 mL single-necked flask, and dissolved in 4 M hydrogen chloride in dioxane (5 mL). Then, methanol (1 mL) was added. The mixture was reacted at room temperature for 1 hour. The reaction solution was directly evaporated to dryness. After 5 mL of saturated sodium bicarbonate solution was added to the reaction solution to adjust the pH of the solution until pH=7 to 8, the reaction solution was extracted with a mixed solution of dichloromethane and isopropanol. The organic phase was dried with anhydrous sodium sulfate to obtain 76.7 mg of a yellow solid, i.e., compound 148-4.

Step 5: Synthesis of compound 148

Compound 148-4 (76.7 mg) was added to a 25 mL single-necked flask, and dissolved in MeOH (5 mL). Then, M43 (71.4 mg) and 1 M $ZnCl_2$/THF (0.1 mL) were added. After the reaction was heated to 40°C for 2 hours, $NaBH_3CN$ (29.8 mg) was added, and the mixture was reacted overnight. The reaction solution was directly evaporated to dryness, and the resultant was separated by Pre-HPLC ($CH_3CN/H_2O$ = 55%-45%) to obtain 18.9 mg of a white solid, i.e., compound 148, (purity 98.43%).
LCMS: 1/2[M+2H]$^+$= 590.72

$^1H$ NMR (500 MHz, Methanol-$d_4$) δ 9.20 (d, J = 7.0 Hz, 1H), 8.87 (d, J = 2.7 Hz, 1H), 7.91 (d, J = 11.5 Hz, 1H), 7.66 (dd, J = 8.4, 5.9 Hz, 1H), 7.48 - 7.35 (m, 4H), 7.33 - 7.17 (m, 2H), 7.06 (t, J = 2.7 Hz, 1H), 5.26 (d, J = 10.4 Hz, 1H), 5.12 - 4.94 (m, 1H), 4.59 (s, 1H), 4.55 - 4.38 (m, 5H), 4.32 - 4.18 (m, 1H), 3.88 (dd, J = 11.0, 3.9 Hz, 1H), 3.81 (d, J = 11.1 Hz, 1H), 3.63 (dd, J = 32.6, 13.3 Hz, 1H), 3.53 - 3.41 (m, 1H), 2.96 (s, 1H), 2.61 - 2.51 (m, 5H), 2.47 (d, J = 3.5 Hz, 5H), 2.40 (d, J = 12.7 Hz, 2H), 2.25 - 2.12 (m, 3H), 2.09 (d, J = 1.3 Hz, 6H), 1.95 (ddd, J = 9.1, 6.8, 3.6 Hz, 1H), 1.90 - 1.82 (m, 1H), 1.79 (d, J = 10.6 Hz, 2H), 1.52 (d, J = 7.0 Hz, 3H), 1.29 (d, J = 13.6 Hz, 5H), 1.15 - 1.05 (m, 3H), 0.82 (dd, J = 16.3, 7.5 Hz, 3H), 0.76 - 0.68 (m, 5H), 0.50 (d, J = 7.1 Hz, 2H).

Example 149 Synthesis of compound (2S,4R)-1-((S)-2-(4-(3-(4-((1-(((7-(8-ethyl-7-fluoro-3-hydroxynaphthalen-1-yl)-8-fluoro-4-((R)-3-hydroxy-3-methylpiperidin-1-yl)pyrido[4,3-d]pyrimidin-2-yl)oxy)methyl)cyclopropyl)methyl) piperazin-1-yl)bicyclic[1.1.1]pent-1-yl)-1H-1,2,3-triazol-1-yl)-3-methylbutanoyl)-4-hydroxy-N-( (S)-1-(4-(4-methylthiazol-5-yl)phenyl)ethyl)pyrrolidine-2-carboxamide

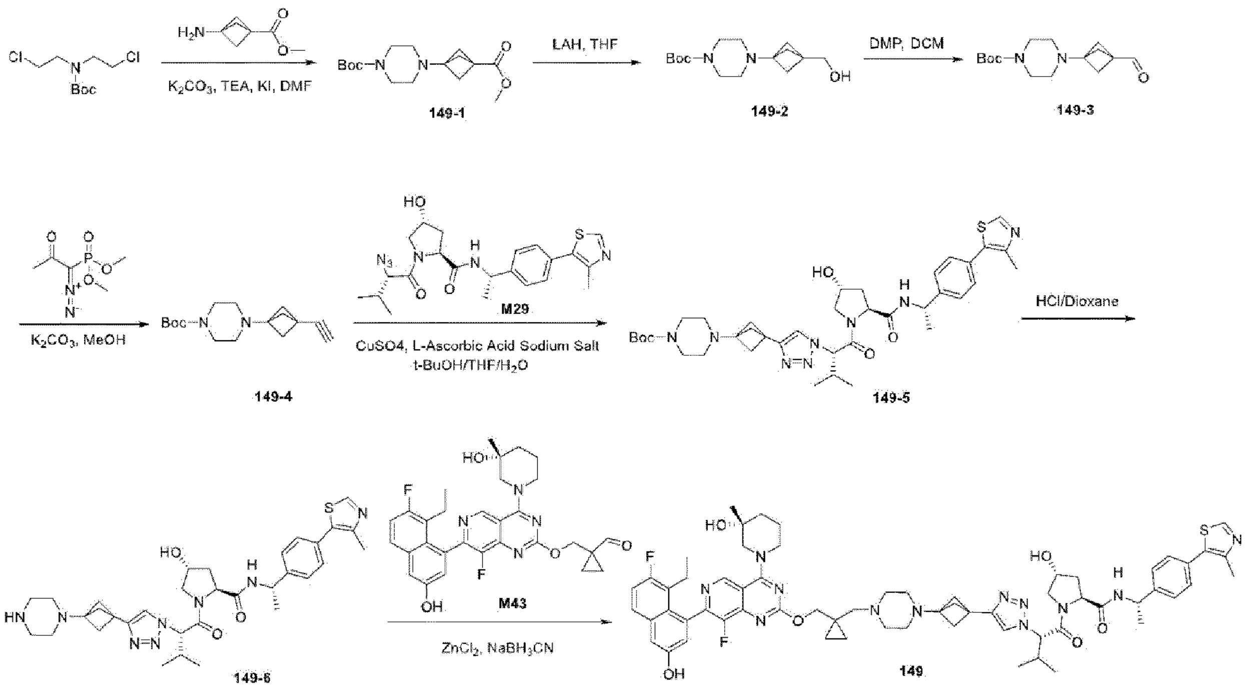


Step 1: Synthesis of compound 149-1

Tert-butyl *N,N*-bis(2-chloroethyl)carbamate (1.2 g) and 3-aminobicyclo[1.1.1]pentan-1-carboxylate (0.7 g) were dissolved in 15 mL of DMF, and then $K_2CO_3$ (2.0 g) and KI (1.65 g) were added. The reaction was heated to 70°C for 12 hours under $N_2$ atmosphere. The reaction solution was added to water, and extracted by adding EA. The organic phase was collected, dried with anhydrous sodium sulfate, and filtered. The filtrate was collected, and concentrated under reduced pressure. The resultant was purified by column chromatography (PE: EA = 3:2) to obtain compound 149-1 (167 mg), ESI-MS m/z: 311.1 $[M+H]^+$.

Step 2: Synthesis of compound 149-2

Compound 149-1 (167 mg) was dissolved in 5 mL of THF, $LiAlH_4$ (31 mg) was added under ice bath, and the reaction was maintained under ice bath for 0.5 hours. After the reaction was completed, the reaction solution was diluted by adding an appropriate amount of EA, and sodium sulfate decahydrate (173 mg) was added. The reaction solution was stirred for 10 minutes to quench the reaction, and dried with anhydrous sodium sulfate. The filtrate was collected, and rotary evaporated to dryness to obtain compound 149-2 (151 mg), ESI-MS m/z: 283.1 $[M+H]^+$.

Step 3: Synthesis of compound 149-3

Compound 149-2 (151 mg) was dissolved in 6 mL of DCM, DMP (261 mg) was added in an ice bath, and then the

reaction was turned to room temperature for 1 hour. The reaction solution was filtered, and extracted by adding DCM and water. The organic phase was collected, and the insoluble solid was removed. The filtrate was extracted by adding DCM and water, dried with anhydrous sodium sulfate, and filtered. The filtrate was collected, and concentrated under reduced pressure. The resultant was purified by column chromatography (PE/EA = 3:2) to obtain compound 149-3 (131 mg), ESI-MS m/z: 281.1 $[M+H]^+$.

Step 4: Synthesis of compound 149-4

Compound 149-3 (131 mg) was dissolved in 6 mL of MeOH, dimethyl (1-diazo-2-oxopropyl)phosphonate (0.10 mL), and potassium carbonate (128 mg) were added. The mixture was reacted at room temperature for 3 hours. The reaction solution was added to water, and extracted by adding EA. The organic phase was collected, dried with anhydrous sodium sulfate, and filtered. The filtrate was collected, and concentrated under reduced pressure. The resultant was purified by column chromatography (PE: EA = 3:2) to obtain compound 149-4 (90 mg), ESI-MS m/z: 277.1 $[M+H]^+$.

Step 5: Synthesis of compound 149-5

Compound 149-4 (90 mg) and M29 (164 mg) were dissolved in a solution of tert-butanol (3 mL), tetrahydrofuran (3 mL) and water (3 mL), and then anhydrous copper sulfate (47 mg), and sodium ascorbate (168 mg) were added. The mixture was reacted at room temperature for 3 hours. The reaction solution was rotary evaporated to dryness, and extracted by adding DCM and water. The organic phase was collected, dried with anhydrous sodium sulfate, and filtered. The filtrate was collected, and concentrated under reduced pressure. The resultant was purified by column chromatography (DCM:MeOH = 10:1) to obtain compound 149-5 (231 mg), ESI-MS m/z: 732.3 $[M+H]^+$.

Step 6: Synthesis of compound 149-6

Compound 149-5 was dissolved in 5 mL of DCM and 5 mL of hydrogen chloride in dioxane (4M). The mixture was reacted at room temperature for 0.5 hours. After the reaction was completed, the reaction solution was rotary evaporated to dryness, and extracted by adding EA and water. The aqueous phase was collected, and anhydrous sodium carbonate was added to the aqueous phase to adjust the pH to alkaline, then extracted by adding DCM/isopropanol (3:1). The organic phase was collected, dried with anhydrous sodium sulfate, and filtered. The filtrate was collected, and rotary evaporated to dryness to obtain compound 149-6 (177 mg), ESI-MS m/z: 633.3 $[M+H]^+$.

Step 7: Synthesis of compound 149

Compound 149-6 (80 mg) and compound M43 (85 mg) were dissolved in methanol (5 mL), and then zinc chloride solution (0.2 mL/1 M in THF) was added. The mixture was reacted at 40°C for 1 hour, then sodium cyanoborohydride (24 mg) was added, and the reaction was heated to 60°C for 6 hours. The reaction was quenched by adding saturated ammonium chloride solution (5.0 mL). The reaction solution was extracted by adding DCM, and the organic phase was collected, washed once with saturated brine, dried with anhydrous sodium sulfate, and filtered. The filtrate was collected, and rotary evaporated to dryness to obtain a crude product. The product was further separated by preparative liquid chromatography to obtain 27.8 mg of white powder, i.e., compound 149. ESI-MS m/z: 583.2 1/2$[M+2H]^+$.
$^1$H NMR (500 MHz, DMSO-$d_6$) $\delta$: 9.93 (d, $J$ = 2.5 Hz, 1H), 9.22 (d, $J$ = 2.0 Hz, 1H), 8.99 (s, 1H), 8.51 (d, $J$ = 7.5 Hz, 1H), 7.96 (s, 1H), 7.76 (dd, $J$ = 9.0, 6.0 Hz, 1H), 7.44 (d, $J$ = 8.5 Hz, 2H), 7.40 - 7.31 (m, 4H), 7.03 (d, $J$ = 2.5 Hz, 1H), 5.23 (d, $J$= 10.5 Hz, 1H), 5.17 (d, $J$ = 3.5 Hz, 1H), 4.91 (t, $J$ = 7.5 Hz, 1H), 4.75 (d, $J$ = 9.5 Hz, 1H), 4.40 - 4.24 (m, 5H), 4.08 - 4.00 (m, 1H), 4.76 - 3.73 (m, 1H), 3.68 - 3.50 (m, 2H), 3.43 - 3.37 (m, 1H), 2.46 - 2.26 (m, 14H), 2.19 - 1.96 (m, 10H), 1.80 - 1.62 (m, 4H), 1.37 (d, $J$ = 7.0 Hz, 3H), 1.17 (d, $J$ = 12.0 Hz, 3H), 1.03 (d, $J$ = 6.5 Hz, 3H), 0.76 - 0.71 (m, 3H), 0.65 - 0.63 (m, 5H), 0.41 (s, 2H).

Example 150 Synthesis of compound (2S,4R)-1-((R)-2-(3-((7-((1-((7-((7-(8-ethyl-7-fluoro-3-hydroxynaphthalen-1-yl)-8-fluoro-4-((R) -3-hydroxy-3-methylpiperidin-1-yl)pyrido[4,3-d]pyrimidin-2-yl)oxy)methyl)cyclopropyl)methyl )-7-azaspiro[3.5]nonan-2-yl)oxy)isoxazol-5-yl)-3-methylbutanoyl)-4-hydroxy-N-((S)-1-(4-(4-me thylthiazol-5-yl)phenyl) ethyl)pyrrolidine-2-carboxamide

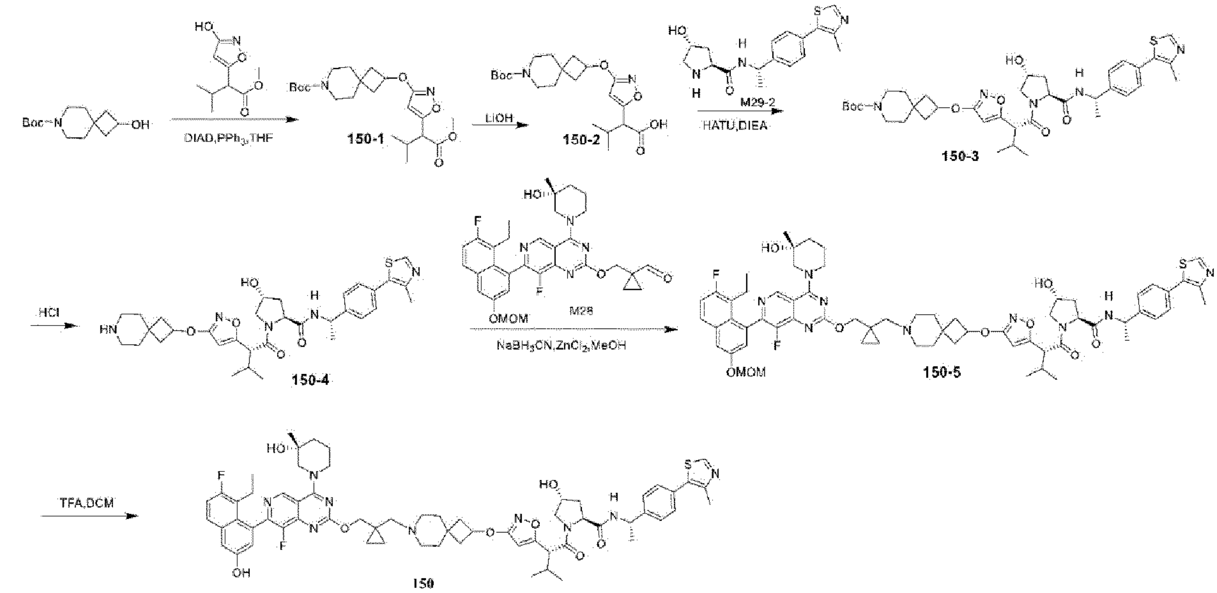


Step 1: Synthesis of compound 150-1

Compounds tert-butyl 2-hydroxy-7-azaspiro[3.5]nonane-7-carboxylate (500 mg), methyl (R)-2-(3-hydroxyisoxazol-5yl)-3-methylbutyrate (412 mg), triphenylphosphine (1.08 g), and DIAD (0.84 g) were dissolved in tetrahydrofuran (10 mL), and the mixture was reacted at 60°C for 1 hour. After the reaction was completed, the reaction solution was concentrated and the resultant was purified by column chromatography (PE:EA=10:1) to obtain compound 150-1 (710 mg). ESI-MS m/z:423.21 $[M+H]^+$.

Step 2: Synthesis of compound 150-2

Compound 150-1 (710 mg) was dissolved in methanol (10 mL) and water (10 mL), and then lithium hydroxide (140 mg) was added. The mixture was reacted at room temperature for 1 hour. After the reaction was completed, the pH of the solution was adjusted to pH=2 with dilute hydrochloric acid. The reaction solution was extracted three times with dichloromethane:isopropanol (10:1), dried with anhydrous sodium sulfate, filtered and concentrated to obtain compound 150-2 (550 mg). ESI-MS m/z:409.22 $[M+H]^+$.

Step 3: Synthesis of compound 150-3

Compound 150-2 (550 mg), M29-2 (491 mg), HATU (1.1 g) and N,N-diisopropylethylamine (900 mg) were dissolved in N,N-dimethylformamide (5.00 mL), and the mixture was reacted at room temperature for 1 hour. After the reaction was completed, the reaction solution was diluted by adding water, and extracted three times with ethyl acetate. The organic phase was washed with saturated brine, dried with anhydrous magnesium sulfate, filtered, and rotary evaporated to dryness. The concentrate was purified by column chromatography (pure ethyl acetate), and then resolved by chiral column (column: CHIRAL ART IB (20.0 mm × 250 mm, 5 μm); column temperature: 35°C; wavelength: 254 nm; flow rate: 25 mL/min; mobile phase: 0.1% diethylamine in n-hexane: 0.1% diethylamine in ethanol (75:25)) to obtain compound 150-3 (280 mg). ESI-MS m/z:722.14 $[M+H]^+$.

Step 4: Synthesis of compound 150-4

Compound 150-3 (150 mg) was dissolved in dichloromethane (6 mL), and then 4M hydrogen chloride in dioxane (2 mL) was added. The mixture was reacted at room temperature for 30 minutes. After the reaction was completed, the reaction solution was rotary evaporated to dryness, and diluted by adding water, extracted by adding dichloromethane:isopropanol (10:1), dried with anhydrous sodium sulfate, filtered and concentrated to obtain compound 150-4 (105 mg). ESI-MS m/z: 622.40 $[M+H]^+$.

Step 5: Synthesis of compound 150-5

Compound 150-4 (150 mg), M28 (52.27 mg), and 1M zinc chloride in tetrahydrofuran (0.5 mL) were dissolved in

methanol (10 mL). The mixture was reacted at 40°C for 1 hour. Sodium cyanoborohydride (25 mg) was added, and the mixture was stirred at 60°C overnight. After the reaction was completed, the reaction solution was quenched by adding ammonium chloride solution, extracted three times with dichloromethane/isopropanol (10:1), dried with anhydrous sodium sulfate, filtered and concentrated. The resultant was purified by column chromatography (dichloromethane/-methanol) to obtain compound 150-5 (100 mg). ESI-MS m/z:600.27 $[M+H]^+$.

Step 6: Synthesis of compound 150

Compound 150-5 (100 mg) was dissolved in dichloromethane (5 mL), and then trifluoroacetic acid (1 mL) was added. The mixture was reacted at room temperature for 1 hour. After completion of the reaction, the reaction solution was concentrated and the resultant was prepared by Pre-HPLC (SunFire C18 column (19.0 mm×250 mm, 5μm); A: 0.05% aqueous trifluoroacetic acid solution, B: acetonitrile, concentration gradient: 20% B to 60% B; 8.03-8.45 min; flow rate: 20mL/min, 254nm) to obtain compound 150 (37.8 mg).

ESI-MS m/z:578.20 $[M+H]^+$.

$^1$H NMR (500 MHz, DMSO-$d_6$) δ 9.21 (d, J = 3.4 Hz, 1H), 8.99 (d, J = 9.0 Hz, 1H), 8.32 (d, J = 7.0 Hz, 1H), 7.78 - 7.71 (m, 1H), 7.46 (q, J = 8.4 Hz, 1H), 7.40 (d, J = 8.2 Hz, 2H), 7.35 (d, J = 9.6 Hz, 2H), 7.31 (d, J = 8.2 Hz, 2H), 7.07 (d, J = 2.4 Hz, 1H), 6.06 (s, 1H), 5.19 (d, J = 3.6 Hz, 1H), 4.92 - 4.83 (m, 1H), 4.83 - 4.71 (m, 2H), 4.41 (t, J = 7.7 Hz, 1H), 4.30 (dd, J = 22.0, 10.0 Hz, 5H), 4.09 - 3.95 (m, 1H), 3.74 (d, J = 8.5 Hz, 1H), 3.62 (d, J = 13.3 Hz, 1H), 3.60 - 3.48 (m, 2H) 2.45 (d, J = 3.9 Hz, 3H), 2.40-2.21 (m, 9H), 2.19 - 1.93 (m, 5H), 1.81 - 1.64 (m, 6H), 1.59 - 1.47 (m, 5H), 1.38 (d, J = 7.0 Hz, 3H), 1.17 (d, J = 12.1 Hz, 4H), 1.08 (d, J = 6.5 Hz, 3H), 0.85 (t, J = 6.9 Hz, 1H), 0.76 - 0.70 (m, 6H), 0.64 (s, 2H), 0.40 (s, 2H).

Example 152 Synthesis of compound (2S,4R)-1-((S)-2-(4-(6-(1-((1-((7-(8-ethyl-7-fluoro-3-hydroxynaphthalen-1-yl)-8-fluoro-4-((R)-3 -hydroxy-3-methylpiperidin-1-yl)pyrido[4,3-d]pyrimidin-2-yl)oxy)methyl)cyclopropyl)methyl)p iperidin-4-yl)amino)pyridin-3-yl)-1H-1,2,3-triazol-1-yl)-3-methylbutanoyl)-4-hydroxy-N-((S)-1-(4-hydroxyl-N-(4-(4-methylthiazol-5-yl)phenyl)ethyl)pyrrolidine-2-carboxamide

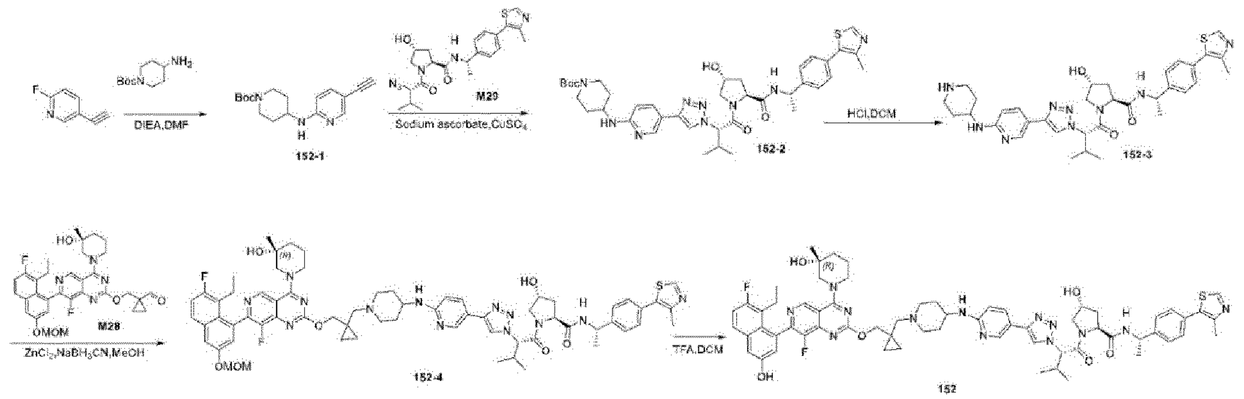


Step 1: Synthesis of compound 152-1

1-Tert-butoxycarbonyl-4-aminopiperidine (1550 mg) was dissolved in 10 mL of N,N-dimethylacetamide, and 5-ethynyl-2-fluoropyridine (625 mg) was added, and then N,N-diisopropylethylamine (3.41 mL) was added. The reaction was stirred at 100°C for 8 hours. The reaction was monitored by LCMS until completion. The reaction mixture was diluted with water, extracted twice with ethyl acetate. The organic phase was washed three times with saturated brine, collected and combined, dried with anhydrous sodium sulfate, and concentrated under reduced pressure. The resultant was purified by column chromatography to obtain compound 152-1 (411 mg). ESI-MS m/z: 302.2 $[M+H]^+$.

Step 2: Synthesis of compound 152-2

Compound 152-1 (103 mg) was added to a 50 mL single-necked flask, 4 mL of tetrahydrofuran, 2 mL of tert-butanol and 2 mL of water were added, and then M1 (120 mg), copper sulfate (38 mg), and sodium ascorbate (135 mg) were added. The mixture was stirred at 20°C for 12 hours. The reaction was monitored by LCMS until completion. The reaction solution was concentrated and the resultant was purified by column chromatography to obtain compound 152-2 (102 mg). ESI-MS m/z: 758.4 $[M+H]^+$.

Step 3: Synthesis of compound 152-3

Compound 152-2 (102 mg) was added to a 10 mL single-necked flask, 3 mL of dichloromethane, and 1 mL of 4M hydrogen chloride in dioxane were added. The mixture was stirred at 20°C for 0.5 hours. The reaction solution was neutralized with saturated sodium bicarbonate solution, extracted twice with dichloromethane:isopropanol (10:1), and the organic phases were collected and combined, dried with anhydrous sodium sulfate and concentrated to obtain compound 152-3 (88 mg). ESI-MS m/z:658.3 $[M+H]^+$.

Step 4: Synthesis of compound 152-4

M28 (70 mg) was added to a 50 mL single-necked flask, 3 mL of methanol, compound 152-3 (88 mg) were added, and then 1M zinc chloride in tetrahydrofuran (0.18 mL) was added. The mixture was stirred at 40°C for 2 hours. Sodium cyanoborohydride (30 mg) was added, and the mixture was stirred at 50°C for 12 hours. The reaction was monitored by LCMS until completion. The reaction solution was concentrated under reduced pressure and the resultant was purified by column chromatography to obtain compound 152-4 (62 mg). ESI-MS m/z:617.8 $[M+H]^+$.

Step 5: Synthesis of compound 152

Compound 152-4 (62 mg) was added to a 10 mL single-necked flask, 2 mL of DCM and 1 mL of trifluoroacetic acid were added. The mixture was reacted at room temperature for 0.5 hours. The reaction was monitored by LCMS until completion. The reaction solution was concentrated under reduced pressure and the resultant was purified by pre-HPLC to obtain compound 152 (11.1 mg).

ESI-MS m/z: 596.32 1/2$[M+2H]^+$.

$^1$H NMR (500 MHz, Methanol-$d_4$) δ 9.10 (d, J = 1.1 Hz, 1H), 8.77 (d, J = 7.5 Hz, 1H), 8.30 (d, J = 2.3 Hz, 1H), 8.23 (d, J = 2.2 Hz, 1H), 7.72 (dt, J = 8.7, 2.6 Hz, 1H), 7.57 (dd, J = 9.0, 5.7 Hz, 1H), 7.36 - 7.29 (m, 4H), 7.20 (d, J = 2.6 Hz, 1H), 7.14 (t, J = 9.4 Hz, 1H), 6.96 (t, J = 2.6 Hz, 1H), 6.48 (d, J = 8.7 Hz, 1H), 5.26 - 5.19 (m, 1H), 4.95 (t, J = 6.9 Hz, 1H), 4.43 (t, J = 8.3 Hz, 1H), 4.38 - 4.33 (m, 2H), 4.17 (t, J = 14.4 Hz, 1H), 3.85 - 3.72 (m, 2H), 3.68 - 3.53 (m, 2H), 3.48 (dd, J = 17.5, 11.3 Hz, 1H), 3.36 (d, J = 10.6 Hz, 1H), 2.98 (s, 2H), 2.56 - 2.49 (m, 1H), 2.43 (d, J = 13.5 Hz, 1H), 2.38 (s, 3H), 2.36 (d, J = 4.8 Hz, 1H), 2.14 - 2.05 (m, 5H), 1.93 - 1.86 (m, 3H), 1.76 (d, J = 8.1 Hz, 1H), 1.68 (t, J = 11.2 Hz, 3H), 1.48 (d, J = 14.4 Hz, 3H), 1.43 (d, J = 7.0 Hz, 3H), 1.06 (d, J = 6.7 Hz, 2H), 0.82 - 0.78 (m, 6H), 0.72 (d, J = 2.2 Hz, 2H), 0.71 (d, J = 3.2 Hz, 2H), 0.63 (s, 2H), 0.43 (s, 2H).

Example 154 Synthesis of compound (2S,4R)-1-((S)-2-(4-(6-(1-((1-((7-(8-ethyl-7-fluoro-3-hydroxynaphthalen-1-yl)-8-fluoro-4-((R)-3 -hydroxy-3-methylpiperidin-1-yl)pyrido[4,3-d]pyrimidin-2-yl)oxy)methyl)cyclopropyl)methyl)p iperidin-4-yl)methyl)amino)pyridin-3-yl)-1H-1,2,3-triazol-1-yl)-3-methylbutanoyl)-4-hydroxy-N -((S)-1-(4-(4-methylthiazol-5-yl) phenyl)ethyl)pyrrolidine-2-carboxamide

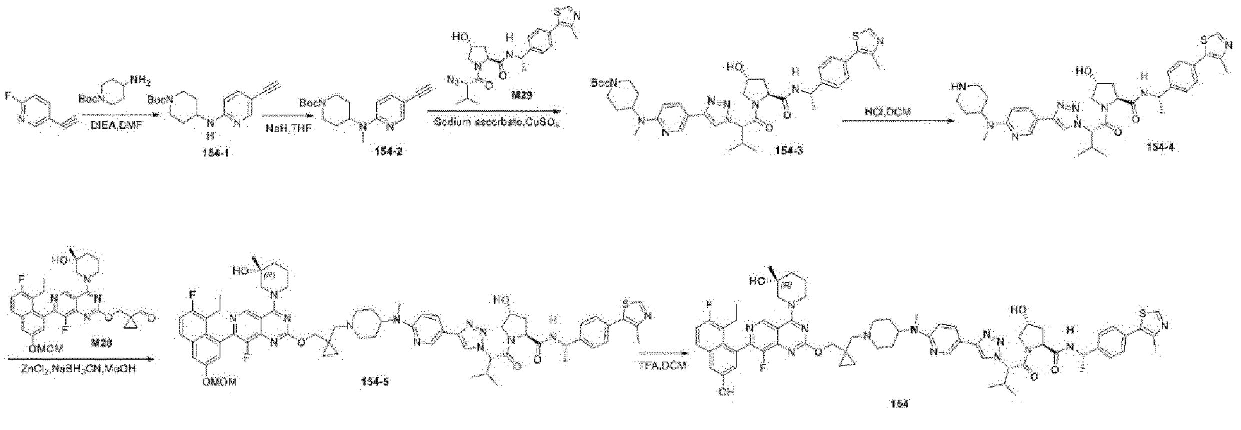


Step 1: Synthesis of compound 154-1

1-Tert-butoxycarbonyl-4-aminopiperidine (1550 mg) was dissolved in 10 mL of N,N-dimethylacetamide, and 5-ethynyl-2-fluoropyridine (625 mg) was added, and then N,N-diisopropylethylamine (3.41 mL) was added. The reaction

was stirred at 100°C for 8 hours. The reaction was monitored by LCMS until completion. The reaction mixture was diluted with water, extracted twice with ethyl acetate. The organic phase was washed three times with saturated brine. Then, the organic phases were collected and combined, dried with anhydrous sodium sulfate, and concentrated under reduced pressure. The resultant was purified by column chromatography to obtain compound 154-1 (411 mg). ESI-MS m/z: 302.2 $[M+H]^+$.

Step 2: Synthesis of compound 154-2

Compound 154-1 (140 mg) was dissolved in 3 mL of tetrahydrofuran, sodium hydride (56 mg) was added, and then methyl iodide (132 mg) was added. The reaction was stirred at 50°C for 12 hours. The reaction was monitored by LCMS until completion. The reaction mixture was diluted with water, extracted twice with ethyl acetate. The organic phases were collected and combined, dried with anhydrous sodium sulfate, and concentrated under reduced pressure. The resultant was purified by column chromatography to obtain compound 154-2 (122 mg). ESI-MS m/z: 316.2 $[M+H]^+$.

Step 3: Synthesis of compound 154-3

Compound 154-2 (122 mg) was added to a 50 mL single-necked flask, 4 mL of tetrahydrofuran, 2 mL of tert-butanol and 2 mL of water were added, and then M29 (130 mg), copper sulfate (41 mg), sodium ascorbate (147 mg) were added. The mixture was stirred at 20°C for 12 hours. The reaction was monitored by LCMS until completion. The reaction solution was concentrated and the resultant was purified by column chromatography to obtain compound 154-3 (118 mg). ESI-MS m/z: 772.4 $[M+H]^+$.

Step 4: Synthesis of compound 154-4

Compound 154-3 (118 mg) was added to a 10 mL single-necked flask, 3 mL of dichloromethane, and 1 mL of 4M hydrogen chloride in dioxane were added. The mixture was stirred at 20°C for 0.5 hours. The reaction solution was neutralized with saturated sodium bicarbonate solution, extracted twice with dichloromethane:isopropanol (10:1), and the organic phases were collected and combined, dried with anhydrous sodium sulfate and concentrated to obtain crude compound 154-4 (103 mg). ESI-MS m/z:672.3 $[M+H]^+$.

Step 5: Synthesis of compound 154-5

M28 (80 mg) was added to a 50 mL single-necked flask, 3 mL of methanol, compound 154-4 (103 mg) were added, and then 1M zinc chloride in tetrahydrofuran (0.20 mL) was added. The mixture was stirred at 40°C for 2 hours. Sodium cyanoborohydride (34 mg) was added, and the mixture was stirred at 50°C for 12 hours. The reaction was monitored by LCMS until completion. The reaction solution was concentrated and the resultant was purified by column chromatography to obtain compound 154-5 (112 mg). ESI-MS m/z:624.8 $[M+H]^+$.

Step 6: Synthesis of compound 154

Compound 154-5 (112 mg) was added to a 10 mL single-necked flask, 2 mL of dichloromethane was added, and then 1 mL of trifluoroacetic acid was added at 0°C. The reaction was stirred at 20°C for 1 hour. The reaction was monitored by LCMS until completion. The reaction solution was neutralized with saturated sodium bicarbonate solution, extracted twice with dichloromethane:isopropanol (5:1), and the organic phases were collected and combined, dried with anhydrous sodium sulfate and concentrated under reduced pressure. The resultant was purified by pre-HPLC to obtain compound 154 (41.7 mg).

ESI-MS m/z:603.39 $[M+H]^+$.

$^1$H NMR (500 MHz, Methanol-$d_4$) δ 9.22 (d, J = 2.1 Hz, 1H), 8.86 (d, J = 8.3 Hz, 1H), 8.52 (d, J = 2.4 Hz, 1H), 8.35 (d, J = 1.9 Hz, 1H), 7.92 (dt, J = 9.0, 3.2 Hz, 1H), 7.67 (dd, J = 9.0, 5.7 Hz, 1H), 7.48 - 7.38 (m, 4H), 7.30 (d, J = 2.7 Hz, 1H), 7.24 (t, J = 9.3 Hz, 1H), 7.06 (t, J = 2.6 Hz, 1H), 6.72 (dd, J = 9.1, 3.2 Hz, 1H), 5.33 (d, J = 10.3 Hz, 1H), 5.05 (q, J = 7.2 Hz, 1H), 4.63 - 4.39 (m, 7H), 4.27 (t, J = 13.6 Hz, 1H), 3.95 - 3.83 (m, 2H), 3.69 - 3.57 (m, 1H), 3.48 (t, J = 11.3 Hz, 1H), 2.89 (d, J = 1.4 Hz, 3H), 2.71 - 2.52 (m, 3H), 2.47 (d, J = 11.7 Hz, 4H), 2.34 - 2.11 (m, 5H), 2.01 - 1.83 (m, 6H), 1.79 (d, J = 10.4 Hz, 2H), 1.73 - 1.64 (m, 2H), 1.53 (d, J = 7.1 Hz, 3H), 1.33 - 1.25 (m, 4H), 1.16 (d, J = 6.8 Hz, 2H), 0.85 - 0.80 (m, 5H), 0.78 (d, J = 9.8 Hz, 2H), 0.58 (s, 2H).

Example 155 Synthesis of compound (2S,4R)-1-((R)-2-(3-(9-((1-((7-(8-ethyl-7-fluoro-3-hydroxynaphthalen-1-yl)-8-fluoro-4-((R)-3-h ydroxy-3-methylpiperidin-1-yl)pyrido[4,3-d]pyrimidin-2-yl)oxy)methyl)cyclopropyl)methyl)-3, 9-diazaspiro[5.5]nonan-3-yl)isoxazol-5-yl)-3-methylbutanoyl)-4-hydroxy-N-((S)-1-(4-(4-(4-met hylthiazol-5-yl)phenyl)ethyl)pyrrolidine-2-carboxamide

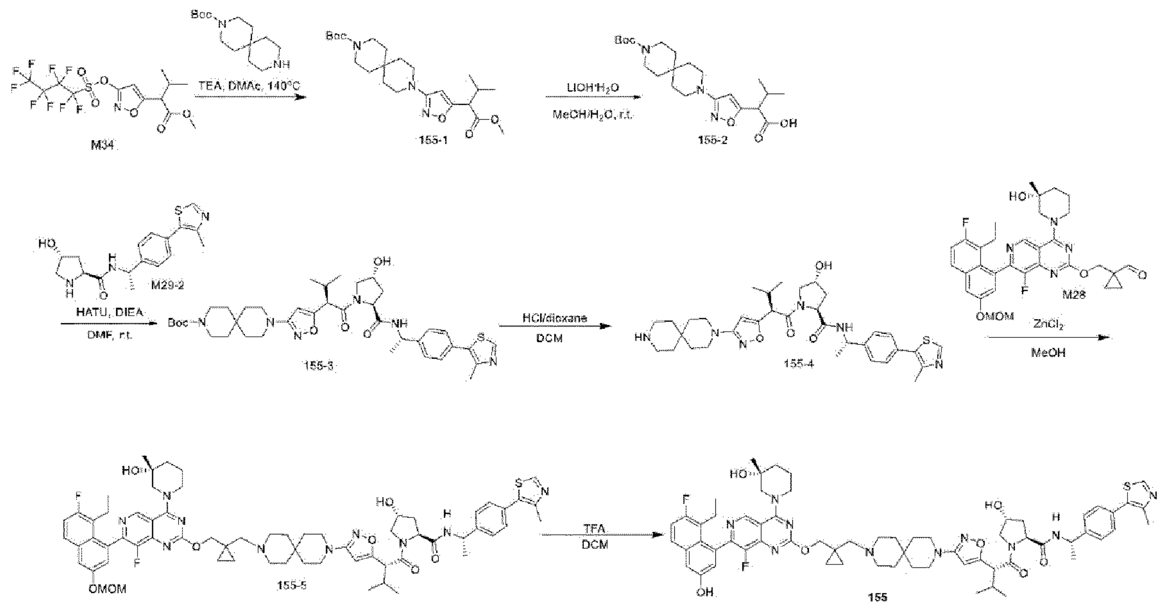


Step 1: Synthesis of compound 155-1

Compound tert-butyl 3,9-diazaspiro[5.5]undecane-3-carboxylate (1.15 g), compound M34 (0.61 g), and triethylamine (1 mL) were dissolved in N,N-dimethylacetamide (3 mL), and the mixture was reacted at 145°C for 3 hours. After the reaction was completed, the reaction solution was diluted with water, extracted three times with ethyl acetate, dried with anhydrous sodium sulfate, filtered and concentrated to obtain a crude product. The product was purified by column chromatography to obtain compound 155-1 (240 mg). ESI-MS m/z:436.2 $[M+H]^+$.

Step 2: Synthesis of compound 155-2

Compound 155-1 (240 mg) was dissolved in methanol (10 mL) and water (2 mL), and then lithium hydroxide (227 mg) was added. The mixture was reacted at 25°C for 1 hour. After the reaction was completed, the pH of the solution was adjusted to pH=4 with citric acid. The reaction solution was extracted three times with dichloromethane:isopropanol (10:1), dried with anhydrous sodium sulfate, filtered and concentrated to obtain compound 155-2 (210 mg). ESI-MS m/z:422.1 $[M+H]^+$.

Step 3: Synthesis of compound 155-3

Compound 155-2 (210 mg), M29-2 (170 mg), HATU (230 mg) and N,N-diisopropylethylamine (0.25 mL) were dissolved in N,N-dimethylformamide (5.00 mL), and the mixture was reacted at 25°C for 1 hour. After the reaction was completed, the reaction solution was extracted with ethyl acetate and water. The organic phase was washed with saturated saline and the resultant was purified by column chromatography (dichloromethane/methanol), and resolved by chiral column to obtain compound 155-3 (80 mg). ESI-MS m/z:735.3 $[M+H]^+$.

Step 4: Synthesis of compound 155-4

Compound 155-3 (80 mg) was dissolved in dichloromethane (5 mL), and then 4M hydrogen chloride in dioxane (1 mL) was added. The mixture was reacted at 25°C for 20 minutes. After the reaction was completed, the reaction solution was neutralized with saturated sodium bicarbonate solution, extracted with dichloromethane/isopropanol (10:1), dried and concentrated to obtain compound 155-4 (65 mg). ESI-MS m/z:635.40 $[M+H]^+$.

Step 5: Synthesis of compound 155-5

Compound 155-4 (65 mg), M28 (90 mg), and 1M zinc chloride in tetrahydrofuran (0.32 mL) were dissolved in methanol (5 mL). The mixture was reacted at 40°C for 2 hours. Sodium cyanoborohydride (50 mg) was added, and the mixture was stirred at 40°C for 8 hours. After the reaction was completed, the reaction solution was quenched by adding ammonium chloride solution, and concentrated. The resultant was purified by column chromatography (dichloromethane/methanol) to obtain compound 155-5 (86 mg). ESI-MS m/z:606.27 $[M+H]^+$.

Step 6: Synthesis of compound 155

Compound 155-5 (86 mg) was dissolved in dichloromethane (5 mL), and then trifluoroacetic acid (1 mL) was added. The mixture was reacted at 25°C for 1 hour. After completion of the reaction, the reaction solution was concentrated and the resultant was separated and purified by Pre-HPLC (C18 column, A: 0.05% aqueous trifluoroacetic acid solution, B: acetonitrile, concentration gradient: 20% B to 75% B, 11 min, flow rate: 15mL/min, 254 nm), concentrated, and neutralized with sodium bicarbonate solution. The resultant was precipitated and filtered to obtain compound 155 (30.7 mg).

ESI-MS m/z: 584.10 $[M+H]^+$.

Example 156 Synthesis of compound (2S,4R)-1-((R)-2-(3-(3-(4-((1-((7-(8-ethyl-7-fluoro-3-hydroxynaphthalen-1-yl)-8-fluoro-4-((R)-3 -hydroxy-3-methylpiperidin-1-yl)pyrido[4,3-d]pyrimidin-2-yl)oxy)methyl)cyclopropyl)methyl)p iperazin-1-yl)azetan-1-yl)isoxazol-5-yl)-3-methylbutanoyl)-4-hydroxy-N-((S)-1-(4-(4-methylthia zol-5-yl)phenyl)ethyl)pyrrolidine-2-carboxamide

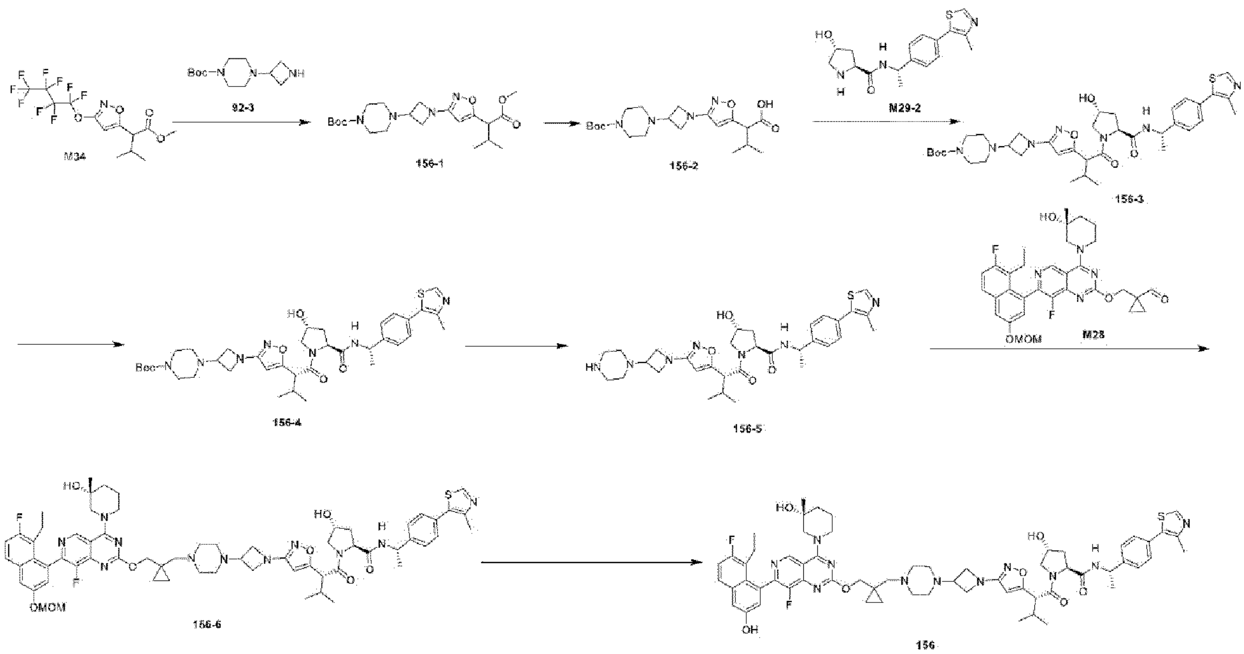

Step 1: Synthesis of compound 156-1

Methyl 3-methyl-2-(3-(perfluorobutoxy)isoxazol-5-yl)butyrate M34 (2.75 g), and tert-butyl 4-(azetan-3-yl)piperazin-1-carboxylate 92-3 (1.00 g) were dissolved in DMA (15 mL), and then TEA (1.73 mL) was slowly added. The mixture was reacted at 140°C for 2 hours. The reaction was monitored by LC-MS until completion, and quenched by adding saturated ammonium chloride (5 mL) to the reaction solution. The reaction solution was extracted with EA, washed once with saturated brine, and dried with anhydrous sodium sulfate, filtered and concentrated. The resultant was separated and purified by column chromatography to obtain 450.0 mg of a white solid, i.e., compound 156-1. ESI-MS m/z: 423.3 $[M+H]^+$.

Step 2: Synthesis of compound 156-2

Compound 156-1 (450 mg) was dissolved in MeOH (10 mL) and water (3 mL), and then lithium hydroxide

monohydrate (230 mg) was slowly added. The mixture was reacted at room temperature for 2 hours. The reaction was monitored by LC-MS until completion. The reaction was quenched by adding DCM (20 mL) to the reaction solution. The reaction solution was washed once with saturated brine, and the organic phase was dried with anhydrous sodium sulfate, filtered and concentrated to obtain 435 mg of a white solid, i.e., compound 156-2. ESI-MS m/z: 409.2 $[M+H]^+$.

Step 3: Synthesis of compound 156-3

Compound 156-2 (435 mg), compound M29-2 (468 mg) and HATU (614 mg) were dissolved in DMF (10 mL), and then DIPEA (0.70 mL) was slowly added under ice bath. The reaction was slowly elevated to room temperature for 1 hour. The reaction was monitored by LC-MS until completion. The reaction was quenched by adding DCM to the reaction solution. The reaction solution was washed once with saturated brine, and the organic phase was dried with anhydrous sodium sulfate, filtered and concentrated. The resultant was separated and purified by column chromatography to obtain 690 mg of a white solid, i.e., compound 156-3. ESI-MS m/z: 722.4 $[M+H]^+$.

Step 4: Synthesis of compound 156-4

Compound 156-3 (690 mg) was resolved by SFC and concentrated to obtain 275 mg of a white solid, i.e., compound 156-4. ESI-MS m/z: 722.3 $[M+H]^+$.

Step 5: Synthesis of compound 156-5

Compound 156-4 (275 mg) was added to DCM (5 mL) and 1M hydrogen chloride in dioxane (5 mL), and the mixture was reacted at room temperature for 10 minutes. The reaction was monitored by LCMS until completion. The reaction solution was concentrated directly, and saturated sodium carbonate solution was added to the reaction solution until pH=8. DCM (20 mL) was added to the reaction solution. The organic phase was collected, washed once with saturated brine, dried with anhydrous sodium sulfate, filtered and concentrated to obtain 237 mg of a white solid, i.e., compound 156-5. ESI-MS m/z: 622.3 $[M+H]^+$.

Step 6: Synthesis of compound 156-6

Compound 156-5 (237 mg) and compound M28 (237 mg) were dissolved in methanol (10 mL), and then 1 M zinc chloride in tetrahydrofuran (0.69 mL) was added. The mixture was reacted at 40°C for 1 hour, and then sodium cyanoborohydride (87 mg) was added. The mixture was reacted at 60°C for 12 hours. The reaction was monitored by LCMS until completion. The reaction was quenched by adding saturated sodium bicarbonate solution (5.0 mL), and DCM (20 mL) was added to the reaction solution. The organic phase was collected, and concentrated. The resultant was separated and purified by column chromatography to obtain 345 mg of white powder, i.e., compound 156-6. ESI-MS m/z: 600.5 1/2$[M+2H]^+$.

Step 7: Synthesis of compound 156

Compound 156-6 (310 mg) was added to DCM (10 mL) and TFA (2 mL), and the mixture was reacted at room temperature for 10 minutes, the reaction was monitored by LCMS until completion. The reaction solution was concentrated directly, and the resultant was separated by preparative liquid chromatography to obtain 88.4 mg of white powder, i.e., compound 156.

ESI-MS m/z: 578.1 1/2$[M+2H]^+$.

$^1$H NMR (500 MHz, DMSO-$d_6$) δ 9.96 (s, 1H), 9.22 (s, 1H), 8.99 (s, 1H). 8.42 (d, *J* = 7.6 Hz, 1H), 7.77 (dd, *J* = 9.0, 6.1 Hz, 1H), 7.44 (d, *J* = 8.2 Hz, 2H), 7.36 (dd, *J* = 12.5, 8.9 Hz, 4H), 7.04 (d, *J* = 2.3 Hz, 1H), 5.84 (s, 1H), 5.12 (d, *J* = 3.6 Hz, 1H), 4.95 - 4.86 (m, 1H), 4.76 (d, *J* = 7.1 Hz, 1H), 4.42 - 4.24 (m, 5H), 4.05 (dd, *J* = 26.0, 13.1 Hz, 1H), 3.87 (t, *J* = 6.0 Hz, 2H), 3.69 (dt, *J* = 14.7, 7.4 Hz, 1H), 3.62 (d, *J* = 12.8 Hz, 3H), 3.58 (d, *J* = 9.7 Hz, 1H), 3.56 - 3.44 (m, 1H), 3.43 - 3.38 (m, 2H), 3.34 - 3.30 (m, 1H), 3.21 (d, *J* = 5.8 Hz, 1H), 2.46 (s, 3H), 2.38 - 2.10 (m, 10H), 2.05 - 1.97 (m, 2H), 1.82 - 1.62 (m, 4H), 1.41 (dd, *J* = 32.6, 7.0 Hz, 3H), 1.17 (d, *J* = 11.7 Hz, 4H), 0.95 (t, *J* = 7.4 Hz, 3H), 0.87 - 0.70 (m, 7H), 0.65 (s, 2H), 0.41 (s, 2H).

Example 157 Synthesis of compound (2S,4R)-1-((S)-2-(4-(6-((3R,7aR)-7a-(((7-(8-ethyl-7-fluoro-3-hydroxynaphthalen-1-yl)-8-fluoro-4-((R)-3-hydroxy-3-methylpiperidin-1-yl)pyrido[4,3-d]pyrimidin-2-yl)oxy)methyl)cyclopropyl)h exahydro-1H-pyrrolizin-3-yl)methoxy)pyridin-3-yl)-1H-1,2,3-triazol-1-yl)-3-methylbutanoyl)-4-hydroxy-N-(((S)-1-(4-(4-methylthiazol-5-yl)phenyl)ethyl)pyrrolidine-2-carboxamide

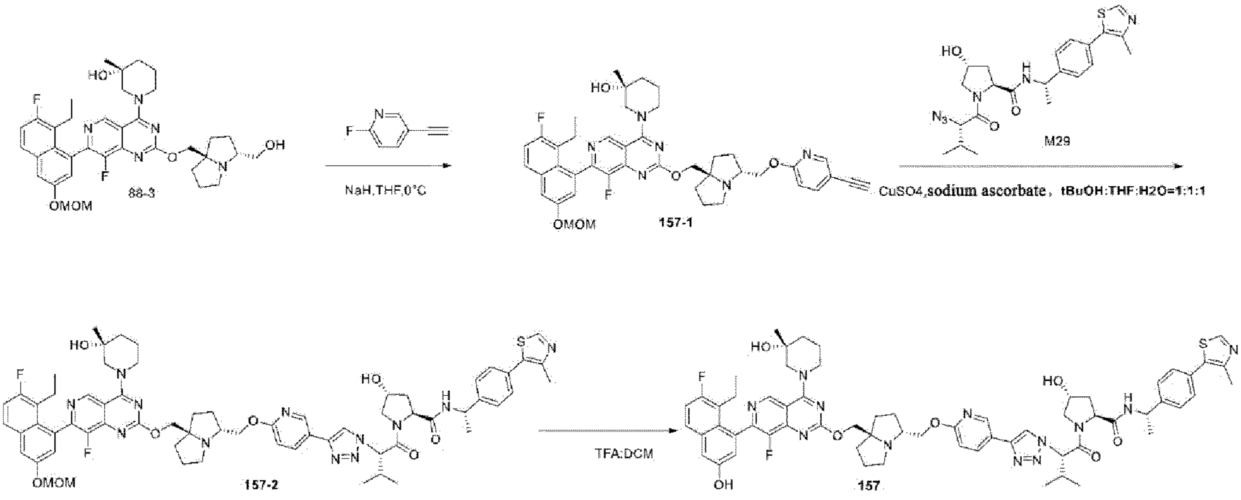


Step 1: Synthesis of compound 157-1

Compound 88-3 (R)-1-(7-(8-ethyl-7-fluoro-3-(methoxy-methoxy)naphthalen-1-yl)-8-fluoro-2-(((3R,7aR)-3-(hydr oxymethyl)tetrahydro-1H-pyrrolizin-7a(5H)-yl)methoxypyrido[4,3-d]pyrimidin-4-yl)-3-methylpi peridin-3-ol (130 mg) was dissolved in 3 mL of THF, and NaH (21 mg) was added under the protection of nitrogen. The mixture was stirred at room temperature for 30 minutes, then 5-ethynyl-2-fluoropyridine (31 mg) was added and the mixture was stirred at 25°C for 2 hours. The reaction was monitored by LC-MS until completion. The reaction was quenched by slowly adding 10 mL of saturated ammonium chloride to the reaction solution. The reaction solution was extracted three times with EA, and the organic phases were combined, dried with anhydrous sodium sulfate, and evaporated under reduced pressure. The resultant was separated and purified by column chromatography to obtain 80 mg of a light yellow solid, i.e., compound 157-1. ESI-MS m/z: 765 $[M+H]^+$;

Step 2: Synthesis of compound 157-2

Compound 157-1 (60 mg) and M29 (2S, 4R)-1-((S)-2-azido-3-methylbutanoyl)-4-hydroxy-N-((S)-1-(4-(4-methylthiazol-5-yl)phenyl)ethy 1)pyrrolidine-2-carboxamide (57 mg) were dissolved in 2 mL of THF, 2 mL of $H_2O$, and 2 mL of tert-butanol. Then, sodium ascorbate (54 mg) and anhydrous copper sulfate (15 mg) were added in turn. The mixture was reacted at room temperature for 0.5 hours. The reaction was monitored by LCMS until completion. 20 mL of water was added to the reaction solution, and the reaction solution was extracted three times with DCM:isopropanol = 4:1, 20 mL each time. The organic phases were combined, dried with sodium sulfate, filtered and concentrated. The resultant was separated and purified by column chromatography to obtain 85 mg of a light yellow solid, i.e., compound 157-2. ESI-MS m/z: 611 1/2$[M+2H]^+$.

Step 3: Synthesis of compound 157

Compound 157-2 (85 mg) was dissolved in 2 mL of DCM, then 1 mL of TFA was added. The mixture was reacted at room temperature for 30 minutes, and the reaction was monitored by LCMS until completion. The reaction was monitored by LCMS until completion. The reaction solution was concentrated directly, and the resultant was separated by preparative liquid chromatography to obtain 22.3 mg of white powder, i.e., compound 157.

ESI-MS m/z: 589 1/2$[M+2H]^+$.

$^1$H NMR (500 MHz, Methanol-$d_4$) δ 9.20 (s, 1H), 8.87 (d, *J* = 6.1 Hz, 1H), 8.62 (d, *J* = 2.3 Hz, 1H), 8.49 (s, 1H), 8.12 (d, *J* = 8.8 Hz, 1H), 7.65 (d, *J* = 5.7 Hz, 1H), 7.48 - 7.37 (m, 4H), 7.28-7.22 (m, 2H), 7.05 (s, 1H), 6.93 (d, *J* = 8.7 Hz, 1H), 5.36 (d, *J* = 10.2 Hz, 1H), 4.62-4.48 (m, 7H), 4.41 (d, *J* = 10.5 Hz, 1H), 4.30 (d, *J* = 10.5 Hz, 2H), 3.93-.3.86 (m, 2H), 3.71 - 3.64 (m, 2H), 3.60-3.57( m, 1H), 3.44 (s, 3H), 3.16 (s, 1H), 2.97-2.90 (m, 3H), 2.63-2.60 (m, 1H), 2.20-2.19 (m, 5H), 2.07-2.00 (m, 1H), 2.01 - 1.89 (m, 6H), 1.79 - 1.75 (m, 4H), 1.53 (d, *J* = 7.0 Hz, 3H), 1.35 - 1.24 (m, 6H), 1.17-1.14 (m, 3H), 0.86 - 0.76 (m, 4H).

Example 158 Synthesis of compound (2S,4R)-1-((S)-2-(4-(2-(((3R,7aR)-7a-(((7-(8-ethyl-7-fluoro-3-hydroxynaphthalen-1-yl)-8-fluoro -4-((R)-3-hydroxy-3-methylpiperidin-1-yl)pyrido[4,3-d]pyrimidin-2-yl)oxy)methyl)cyclopropyl) hexahydro-1H-pyrrolizin-3-yl)methoxy)pyrimidin-5-yl)-1H-1,2,3-triazol-1-yl)-3-methylbutanoyl )-4-hydroxy-N-(((S)-1-(4-(4-methylthiazol-5-yl)phenyl)ethyl)pyrrolidine-2-carboxamide

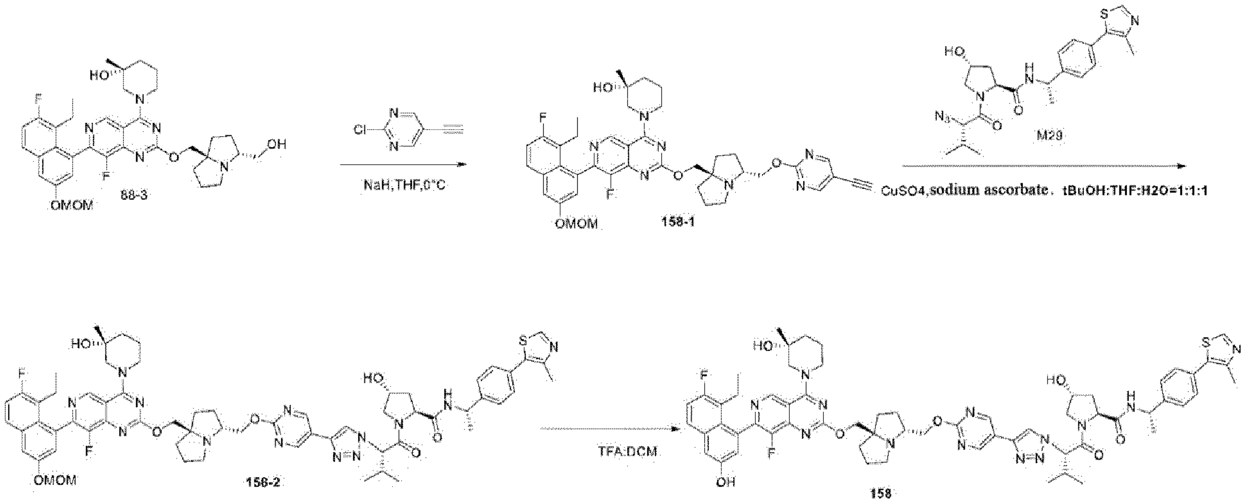


88-3 158-1 158-2 158

Step 1: Synthesis of compound 158-1

Compound 88-3 (R)-1-(7-(8-ethyl-7-fluoro-3-(methoxy-methoxy)naphthalen-1-yl)-8-fluoro-2-(((3R,7aR)-3-(hydr oxymethyl)tetrahydro-1H-pyrrolizin-7a(5H)-yl)methoxypyrido[4,3-d]pyrimidin-4-yl)-3-methylpi peridin-3-ol (100 mg) was dissolved in 3 mL of THF, and NaH (16 mg) was added under the protection of nitrogen. The mixture was stirred at room temperature for 30 minutes, then 2-chloro-5-ethynylpyrimidine (28 mg) was added and the mixture was stirred at 25°C for 2 hours. The reaction was monitored by LC-MS until completion. The reaction was quenched by slowly adding 10 mL of saturated ammonium chloride to the reaction solution. The reaction solution was extracted three times with EA, and the organic phases were combined, dried with anhydrous sodium sulfate, and evaporated under reduced pressure. The resultant was separated and purified by column chromatography to obtain 60 mg of a light yellow solid, i.e., compound 158-1. ESI-MS m/z: 766 $[M+H]^+$;

Step 2: Synthesis of compound 158-2

Compound 158-1 (60 mg) and M29 (2S,4R)-1-((S)-2-azido-3-methylbutanoyl)-4-hydroxy-N-((S)-1-(4-(4-methylthiazol-5-yl)phenyl) ethyl)pyrrolidine-2-carboxamide (43 mg) were dissolved in 2 mL of THF, 2 mL of $H_2O$, and 2 mL of tert-butanol. Then, sodium ascorbate (40 mg) and anhydrous copper sulfate (12 mg) were added in turn. The mixture was reacted at room temperature for 0.5 hours. The reaction was monitored by LCMS until completion. 20.0 mL of water was added to the reaction solution, and the reaction solution was extracted three times with DCM:isopropanol = 4:1, 20 mL each time. The organic phases were combined, dried with sodium sulfate, filtered and concentrated. The resultant was separated and purified by column chromatography to obtain 74 mg of a light yellow solid, i.e., compound 158-2. ESI-MS m/z: 612 1/2$[M+2H]^+$.

Step 3: Synthesis of compound 158

Compound 158-2 (74 mg) was dissolved in 2 mL of DCM, then 1 mL of TFA was added. The mixture was reacted at room temperature for 30 minutes, and the reaction was monitored by LCMS until completion. The reaction was monitored by LCMS until completion. The reaction solution was concentrated directly, and the resultant was separated by preparative liquid chromatography to obtain 39.6 mg of white powder, i.e., compound 158. ESI-MS m/z: 590 1/2$[M+2H]^+$.

Example 159 Synthesis of compound (2S,4R)-1-((S)-2-(4-(5-(((3R,7aR)-7a-((7-(8-ethyl-7-ethyl-7-fluoro-3-hydroxy-naphthalen-1-yl)-8 -fluoro-4-((R)-3-hydroxy-3-methylpiperidin-1-methyl)pyrido[4,3-d]pyrimidin-2-yl)oxy)methyl)h exahydro-1H-pyrrolizin-3-yl)methoxy)pyrazin-1H-1,2,3-triazol-yl)-4-methylpentanoyl)-4-hydro xy-N-(((S)-1-(4-(4-methylthiazol-5-yl)phenyl)ethyl)pyrrolidine-2-carboxamide

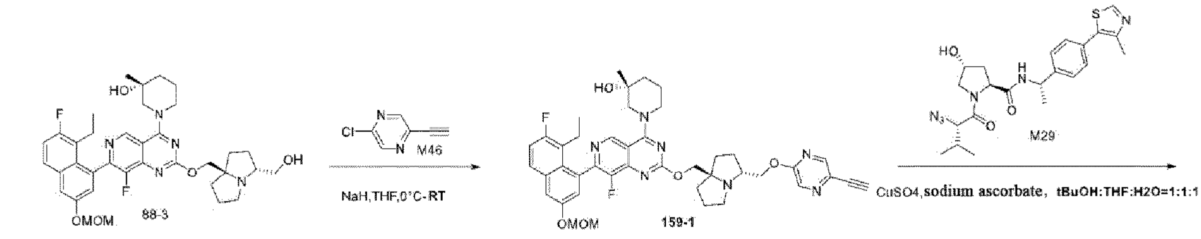


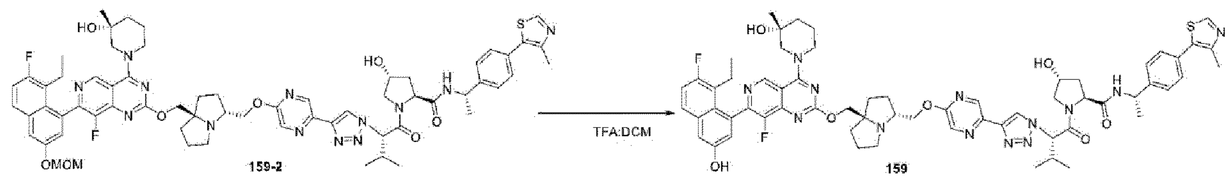


Step 1: Synthesis of compound 159-1

Compound 88-3 (R)-1-(7-(8-ethyl-7-fluoro-3-(methoxy-methoxy)naphthalen-1-yl)-8-fluoro-2-(((3R,7aR)-3-(hydr oxymethyl)tetrahydro-1H-pyrrolizin-7a(5H)-yl)methoxypyrido[4,3-d]pyrimidin-4-yl)-3-methylpi peridin-3-ol (90 mg) was dissolved in 3 mL of THF, and NaH (15mg) was added under the protection of nitrogen. The mixture was stirred at room temperature for 30 minutes, then M46 2-chloro-5-ethynylpyrazine (25 mg) was added and the mixture was stirred at 25°C for 2 hours. The reaction was monitored by LC-MS until completion. The reaction was quenched by slowly adding 10 mL of saturated ammonium chloride to the reaction solution. The reaction solution was extracted three times with EA, and the organic phases were combined, dried with anhydrous sodium sulfate, and evaporated under reduced pressure. The resultant was separated and purified by column chromatography to obtain 58mg of a light yellow solid, i.e., compound 159-1. ESI-MS m/z: 766 $[M+H]^+$;

Step 2: Synthesis of compound 159-2

Compound 159-1 (58 mg) and M29 (2S,4R)-1-((S)-2-azido-3-methylbutanoyl)-4-hydroxy-N-((S)-1-(4-(4-methylthiazol-5-yl)phenyl) ethyl)pyrrolidine-2-carboxamide (42 mg) were dissolved in 1.5 mL of THF, 1.5 mL of $H_2O$, and 1.5 mL of tert-butanol. Then, sodium ascorbate (39 mg) and anhydrous copper sulfate (11 mg) were added in turn. The mixture was reacted at room temperature for 0.5 hours. The reaction was monitored by LCMS until completion. 20 mL of water was added to the reaction solution, and the reaction solution was extracted three times with DCM:isopropanol = 4:1, 20 mL each time. The organic phases were combined, dried with sodium sulfate, filtered and concentrated. The resultant was separated and purified by column chromatography to obtain 25 mg of a light yellow solid, i.e., compound 159-2. ESI-MS m/z: 612 1/2$[M+2H]^+$.

Step 3: Synthesis of compound 159

Compound 159-2 (25 mg) was dissolved in 2 mL of DCM, then 1 mL of TFA was added. The mixture was reacted at room temperature for 30 minutes, and the reaction was monitored by LCMS until completion. The reaction was monitored by LCMS until completion. The reaction solution was concentrated directly, and the resultant was separated by preparative liquid chromatography to obtain 18.4 mg of white powder, i.e., compound 159. ESI-MS m/z: 590 1/2$[M+2H]^+$.

Example 160 Synthesis of compound (2S,4R)-N-((R)-1-(4-(1-ethyl-1H-pyrazol-5-yl)phenyl)-2-hydroxyethyl)-1-((S)-2-(4-(1-((7-(8-eth yl-7-fluoro-3-hydroxynaphthalen-1-yl)-8-fluoro-4-((R)-3-hydroxy-3-methylpiperidin-1-yl)pyrido [4,3-d]pyrimidin-2-yl)oxy)methylcyclopropyl)methylpiperidin-4-yloxy)pyridin-3-yl)-1H-1,2,3-tr iazol-1-yl)-3-methylbutanoyl)-4-hydroxypyrrolidine-2-carboxamide

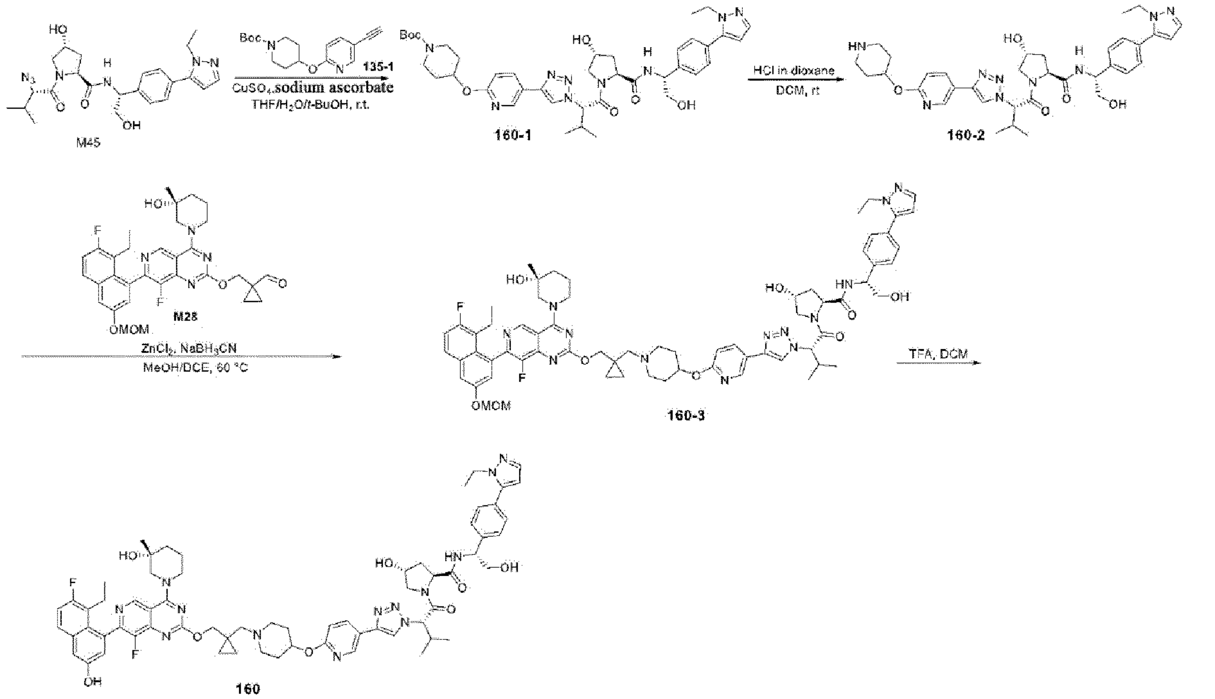


Step 1: Synthesis of compound 160-1

Intermediate M45 (100.00 mg), compound 135-1 (96.61 mg), copper sulfate (30.60 mg), sodium ascorbate (109.71 mg), tetrahydrofuran (1.50 mL), tert-butanol (1.50 mL), and water (1.50 mL) were added to a reaction flask. The reaction system was substituted with nitrogen and reacted at room temperature for 2 hours. The reaction solution was concentrated, diluted with water, extracted with DCM and methanol, dried with anhydrous sodium sulfate, and concentrated. The resultant was separated by column chromatography (DCM/ammonia in methanol = 20:1) to obtain a white solid compound 160-1 114 mg), ESI-MS m/z: 772.4 $[M+H]^+$.

Step 2: Synthesis of compound 160-2

Compound 160-1 (114 mg), dichloromethane (2.00 mL), and 4M hydrochloride in dioxane (0.40 mL) were added to a reaction flask, and the mixture was reacted at room temperature for 0.5 hours. After the reaction was completed, the supernatant was decanted, the insoluble substance was dissolved by adding methanol, and the pH of the solution was adjusted to pH=8 with aqueous sodium bicarbonate solution. The reaction solution was extracted with DCM and methanol, dried with anhydrous sodium sulfate, and concentrated to obtain compound 160-2 (85 mg), ESI-MS m/z: 672.4 $[M+H]^+$.

Step 3: Synthesis of compound 160-3

Compound 160-2 (62.34 mg), M28 (50.00 mg), methanol (2.00 mL), 1,2-dichloroethane (2.00 mL) and zinc chloride (11.50 mg) were added to a reaction flask, and the mixture was reacted at 40°C for 1 hour. Then, sodium cyanoborohydride (53.04 mg, 0.84 mmol) was added, the mixture was reacted at 60°C for 3 hours, diluted with water, extracted with DCM, dried with anhydrous sodium sulfate, and concentrated. The resultant was prepared by Pre-TLC (DCM/ammonia in methanol = 18:1) to obtain a yellow solid, i.e., compound 160-3 (56 mg), ESI-MS m/z: 625.3 1/2 $[M+2H]^+$.

Step 4: Synthesis of compound 160

Compound 160-3 (56.00 mg), dichloromethane (1.00 mL), and trifluoroacetic acid (1.00 mL) were added to a reaction flask, and the mixture was reacted at room temperature for 0.6 hours, concentrated and dissolved in DCM. The reaction solution was added to an ice-cold aqueous sodium bicarbonate solution to adjust pH=8, extracted with DCM and methanol, dried with anhydrous sodium sulfate, and concentrated. The resultant was prepared by Pre-TLC (DCM/ammonia in methanol = 10:1) to obtain compound 160 (35.5 mg).

ESI-MS m/z: 603.25 1/2[M+2H]$^+$.

$^1$H NMR (500 MHz, Methanol-$d_4$) $\delta$ 9.20 (d, *J* = 8.1 Hz, 1H), 8.57 (d, *J* = 2.2 Hz, 1H), 8.50 (d, *J* = 12.8 Hz, 1H), 8.06 (dd, *J* = 8.6, 2.4 Hz, 1H), 7.65 (dd, *J* = 8.9, 5.9 Hz, 1H), 7.53 - 7.37 (m, 5H), 7.30 (d, *J* = 2.6 Hz, 1H), 7.23 (t, *J* = 9.0 Hz, 1H), 7.08 (dd, *J* = 4.9, 2.6 Hz, 1H), 6.81 (d, *J* = 8.7 Hz, 1H), 6.28 (dd, *J* = 8.6, 1.9 Hz, 1H), 5.38 (d, *J* = 10.2 Hz, 1H), 5.07 (t, *J* = 6.0 Hz, 2H), 4.73 (s, 1H), 4.61 (t, *J* = 8.3 Hz, 1H), 4.46 (t, *J* = 8.9 Hz, 4H), 4.19 (m, 3H), 3.95 - 3.82 (m, 3H), 3.67 - 3.53 (m, 1H), 3.43 (dd, *J* = 25.9, 11.7 Hz, 1H), 3.31 (m, 1H), 3.00 (s, 2H), 2.69 - 2.52 (m, 6H), 2.18 - 2.04 (m, 6H), 1.77 (m, 5H), 1.36 - 1.24 (m, 6H), 1.16 (d, *J* = 6.6 Hz, 3H), 0.81 (t, *J* = 7.2 Hz, 6H), 0.75 (s, 2H), 0.56 (s, 2H).

Example 164 Synthesis of compound (2S,4R)-1-((R)-2-(3-(4-((1-((7-(8-ethyl-7-fluoro-3-hydroxynaphthalen-1-yl)-8-fluoro-4-((R)-3-h ydroxy-3-methylpiperidin-1-yl)pyrido[4,3-d]pyrimidin-2-yl)oxy)methyl)cyclopropyl)methyl)pip erazin-1-yl)piperidin-1-yl)isoxazol-5-yl)-3-methylbutanoyl)-4-hydroxy-N-((R)-1-((4-(4-methylth iazol-5-yl)phenyl)ethyl)pyrrolidine-2-carboxamide

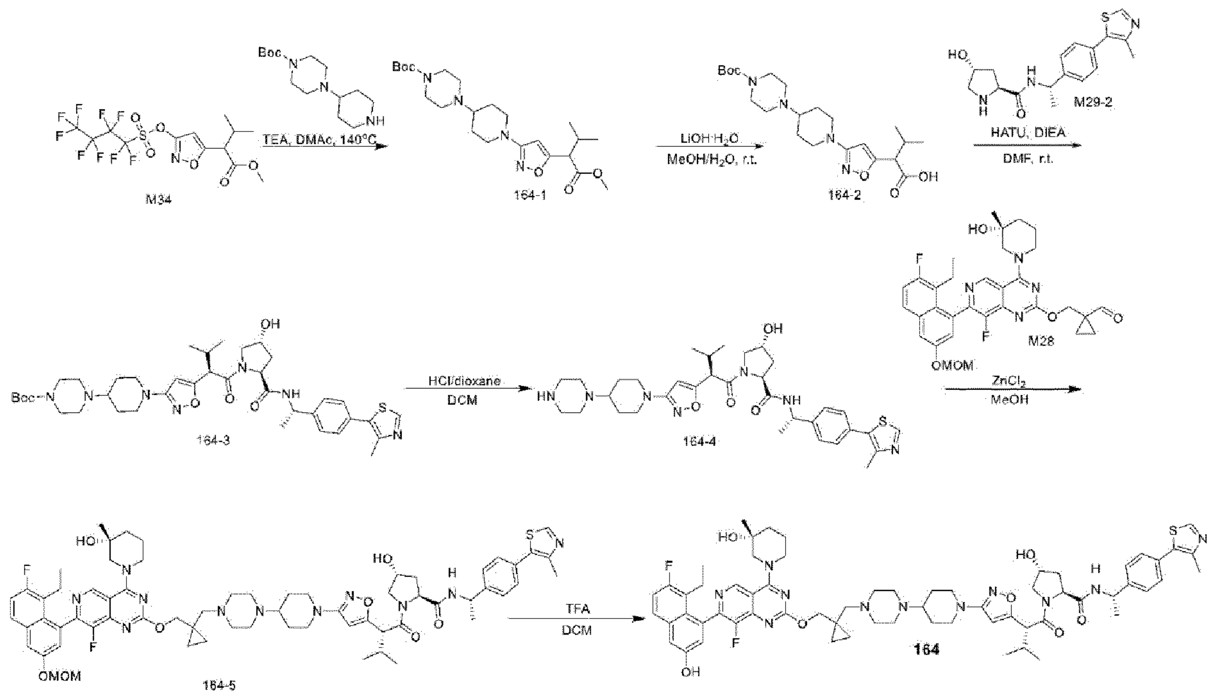


Step 1: Synthesis of compound 164-1

Tert-butyl 4-(piperidin-4-yl)piperazin-1-carboxylate (0.30 g), compound M34 (0.54 g), and triethylamine (0.47 mL) were dissolved in N,N-dimethylacetamide (3 mL), and the mixture was reacted at 145°C for 3 hours. After the reaction was completed, the reaction solution was diluted with water, extracted three times with EA, dried with anhydrous sodium sulfate, filtered and concentrated. The crude product was purified by column chromatography to obtain compound 164-1 (190 mg). ESI-MS m/z:451.2 [M+ H-100]$^+$.

Step 2: Synthesis of compound 164-2

Compound 164-1 (190 mg) was dissolved in methanol (10 mL) and water (2 mL), and then lithium hydroxide (40 mg) was added. The mixture was reacted at 25°C for 1 hour. After the reaction was completed, the pH of the solution was

adjusted to pH=4 with citric acid. The reaction solution was extracted three times with dichloromethane:isopropanol (10:1), dried with anhydrous sodium sulfate, filtered and concentrated to obtain compound 164-2 (180 mg). ESI-MS m/z:437.1 $[M+ H-100]^+$.

Step 3: Synthesis of compound 164-3

Compound 164-2 (180 mg), M29-2 (150 mg), HATU (190 mg) and N,N-diisopropylethylamine (0.20 mL) were dissolved in N,N-dimethylformamide (5.00 mL), and the mixture was reacted at 25°C for 1 hour. After the reaction was completed, the mixture was diluted by adding water and extracted with ethyl acetate. The organic phase was washed with saturated saline and the resultant was purified by column chromatography (dichloromethane/methanol), and resolved by chiral column to obtain compound 164-3 (81 mg). ESI-MS m/z:750.3 $[M+H]^+$.

Step 4: Synthesis of compound 164-4

Compound 164-3 (81 mg) was dissolved in dichloromethane (5 mL), and then 4M hydrogen chloride in dioxane (1 mL) was added. The mixture was reacted at 25°C for 20 minutes. After the reaction was completed, the reaction solution was neutralized with sodium bicarbonate solution, extracted with dichloromethane/isopropanol (10:1), dried and concentrated to obtain compound 164-4 (55 mg). ESI-MS m/z:650.4 $[M+H]^+$.

Step 5: Synthesis of compound 164-5

Compound 164-4 (55 mg), M28 (62 mg), and 1M zinc chloride in tetrahydrofuran (0.19 mL) were dissolved in methanol (5 mL). The mixture was reacted at 40°C for 2 hours. Sodium cyanoborohydride (34 mg) was added, and the mixture was stirred at 40°C for 8 hours. After the reaction was completed, the reaction solution was quenched by adding ammonium chloride solution, and concentrated. The resultant was purified by column chromatography (dichloromethane/methanol) to obtain compound 164-5 (43 mg). ESI-MS m/z:613.7 $[M+H]^+$.

Step 6: Synthesis of compound 164

Compound 164-5 (43 mg) was dissolved in dichloromethane (5 mL), and then trifluoroacetic acid (1 mL) was added. The mixture was reacted at 25°C for 1 hour. After completion of the reaction, the reaction solution was concentrated and the resultant was separated and purified by Pre-HPLC (C18 column, A: 0.05% aqueous trifluoroacetic acid solution, B: acetonitrile, concentration gradient: 20% B to 75% B, 11 min, flow rate: 15 mL/min, 254 nm), concentrated, and neutralized with sodium bicarbonate solution. The resultant was precipitated and filtered to obtain compound 164 (7.1 mg). ESI-MS m/z:591.70 $[M+H]^+$.

Example 165 Synthesis of compound (2S,4R)-1-((S)-2-(4-(3-(4-((1-(((7-(8-ethyl-7-fluoro-3-hydroxynaphthalen-1-yl)-8-fluoro-4-((R)-3-hydroxy-3-methylpiperidin-1-yl)pyrido[4,3-d]pyrimidin-2-yl)oxy)methyl)cyclopropyl)methyl) piperazin-1-yl)cyclobutyl)-1H-1,2,3-triazol-1-yl)-3-methylbutanoyl)-4-hydroxy-N-((S)-1-(4-(4-methylthiazol-5-yl)phenyl)ethyl)pyrrolidine-2-carboxamide

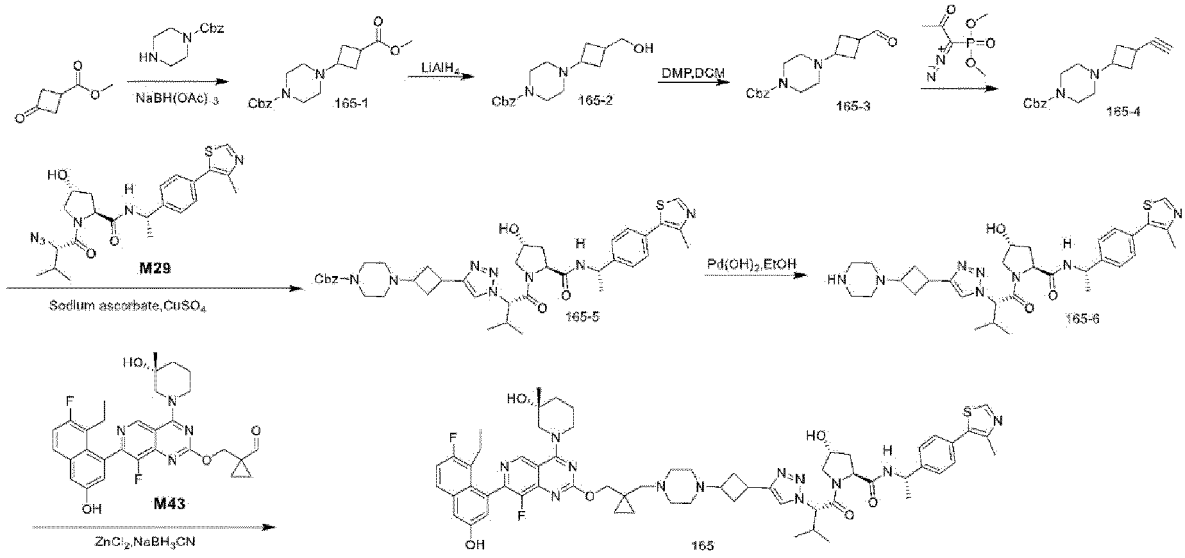


Step 1: Synthesis of compound 165-1

Methyl 3-oxocyclobutan-1-carboxylate (10.0 g) and benzyl piperazin-1-carboxylate (20.63 g) were dissolved in 100 mL of dichloroethane, and glacial acetic acid (8 mL) was added. The mixture was stirred at room temperature for 30 minutes. Then, sodium triacetoxyborohydride was added, and the mixture was stirred at room temperature overnight. After the reaction was completed, the reaction solution was diluted by adding water, and extracted three times with DCM. The organic phases were collected and combined, washed with saturated saline, and dried with anhydrous sodium sulfate, filtered and concentrated. The resultant was purified by column chromatography to obtain compound 165-1 (13.0 g). ESI-MS m/z: 333.2 $[M+H]^+$.

Step 2: Synthesis of compound 165-2

Compound 165-1 (13.0 g) was added to a 250 mL single-necked flask, and 100 mL of tetrahydrofuran was added. The mixture was cooled to below 0°C, followed by slow addition of lithium aluminum hydride (2.9 g) in batches, and the reaction was turned to room temperature for 3 hours. After the reaction was completed, the mixture was cooled to 0°C. 2.9 mL of water was slowly added dropwise to the reaction solution, and 2.9 mL of aqueous sodium hydroxide solution (15% of mass fraction) was slowly added dropwise. The mixture was stirred for 10 minutes, and then 9 mL of water was slowly added dropwise to the reaction solution, followed by the addition of anhydrous sodium sulfate. The mixture was stirred for 1 hour. The reaction solution was filtered, and the filtrate was concentrated. The concentrate was purified by column chromatography to obtain compound 165-2 (10.0 g). ESI-MS m/z: 305.3 $[M+H]^+$.

Step 3: Synthesis of compound 165-3

Compound 165-2 (10.0 g) was added to a 250 mL single-necked flask, and 100 mL of DCM was added, followed by the addition of DMP (20.0 g) in batches. The mixture was stirred at room temperature for 3 hours. After the reaction was quenched with saturated aqueous sodium thiosulfate solution, filtered, extracted twice with DCM, dried with anhydrous sodium sulfate, filtered and concentrated. The filtrate was purified by column chromatography to obtain compound 165-3 (12.0 g). ESI-MS m/z: 303.4 $[M+H]^+$.

Step 4: Synthesis of compound 165-4

Compound 165-3 (12.0 g) and dimethyl (1-diazo-2-oxopropyl)phosphonate (9.93 mL) were added to a 100 mL single-necked flask in turn, and dissolved by adding 30 mL of methanol, followed by slow addition of potassium carbonate (22.3g). The mixture was stirred at room temperature overnight. The reaction mixture was diluted with water, extracted twice with EA. The organic phases were collected and combined, dried with anhydrous sodium sulfate, and the concentrate was purified by column chromatography to obtain compound 165-4 (1.4 g). ESI-MS m/z:299.4 $[M+H]^+$.

Step 5: Synthesis of compound 165-5

Compound 165-4 (140 mg), sodium ascorbate (280 mg), copper sulfate (70 mg) and M29 (260 mg) were added to a 50 mL single-necked flask in turn, and then 3 mL of each of methanol, tetrahydrofuran and water were added. The mixture was reacted at room temperature for 30 minutes. The solvent was rotary evaporated to dryness, and extracted twice with EA. The organic phases were collected and combined, and dried with anhydrous sodium sulfate. The concentrate was separated and purified by column chromatography to obtain compound 165-5 (100 mg). ESI-MS m/z: 755.4 $[M+H]^+$.

Step 6: Synthesis of compound 165-6

Compound 165-5 (100 mg) was added to a 50 mL single-necked flask, dissolved by adding 10 mL of ethanol, and palladium hydroxide (20 mg) was added. The reaction system was substituted five times with hydrogen and stirred at room temperature overnight. After the reaction was completed, the resultant was filtered with diatomaceous earth and the filtrate was rotary evaporated to dryness, and concentrated to obtain crude product compound 165-6 (50 mg), which was directly fed into the next step. ESI-MS m/z: 621.3 $[M+H]^+$.

Step 7: Synthesis of compound 165

Compound 165-6 (50 mg), M43 (44 mg), and 1M zinc chloride in tetrahydrofuran (0.25 mL) were dissolved in methanol (8 mL). The mixture was reacted at 40°C for 1 hour. Sodium cyanoborohydride (16 mg) was added, and the mixture was stirred at 70°C for 4 hours. After the reaction was completed, the reaction was quenched by adding ammonium chloride solution, and the reaction solution was concentrated and the resultant was purified by column chromatography (dichloromethane/methanol). The concentrate was purified by Pre-HPLC (column: SunFire C18 column, A: 0.05% trifluoroacetic acid aqueous solution, B: acetonitrile, concentration gradient: 20% B to 70% B, 6.36-6.71 min, flow rate: 20 mL/min, 254 nm) to obtain compound 165 (23.0 mg).

$^1$H NMR (500 MHz, DMSO-$d_6$) ) δ 9.94 (s, 1H), 9.22 (s, 1H), 8.98 (s, 1H), 8.49 (d, J = 7.1 Hz, 1H), 7.96 (s, 1H), 7.76 (s, 1H), 7.44 (d, J = 8.2 Hz, 2H), 7.36 (d, J = 8.4 Hz, 2H), 7.33 (s, 1H), 7.03 (s, 1H), 5.19 (d, J = 24.9 Hz, 2H), 4.91 (t, J = 6.8 Hz, 1H), 4.74 (d, J = 4.0 Hz, 1H), 4.33 (d, J = 34.8 Hz, 5H), 4.05 (s, 1H), 3.74 (s, 1H), 3.64 (s, 1H), 2.64 (s, 1H), 2.36 (s, 6H), 2.12 (s, 3H), 2.01 (s, 4H), 1.91 (s, 1H), 1.74 (t, J = 25.2 Hz, 5H), 1.49 (d, J = 36.2 Hz, 2H), 1.37 (d, J = 7.0 Hz, 3H), 1.27 - 1.14 (m, 11H), 1.03 (d, J = 6.2 Hz, 3H), 0.85 (s, 1H), 0.74 (d, J = 7.5 Hz, 3H), 0.64 (d, J = 6.5 Hz, 5H), 0.42 (s, 2H).

Example 166 Synthesis of compound (2S,4R)-1-((2R)-2-(3-(3-(4-((1-((7-(8-ethyl-7-fluoro-3-hydroxynaphthalen-1-yl)-8-fluoro-4-((R) 3-hydroxy-3-methylpiperidin-1-yl)pyrido[4,3-d]pyrimidin-2-yl)oxy)methyl)cyclopropyl)methyl) piperazin-1-yl)pyrrolidin-1-yl)isoxazol-5-yl)-3-methylbutanoyl)-4-hydroxy-N-((S)-1-(4-(4-meth ylthiazol-5-yl)phenyl)ethyl)pyrrolidine-2-carboxamide

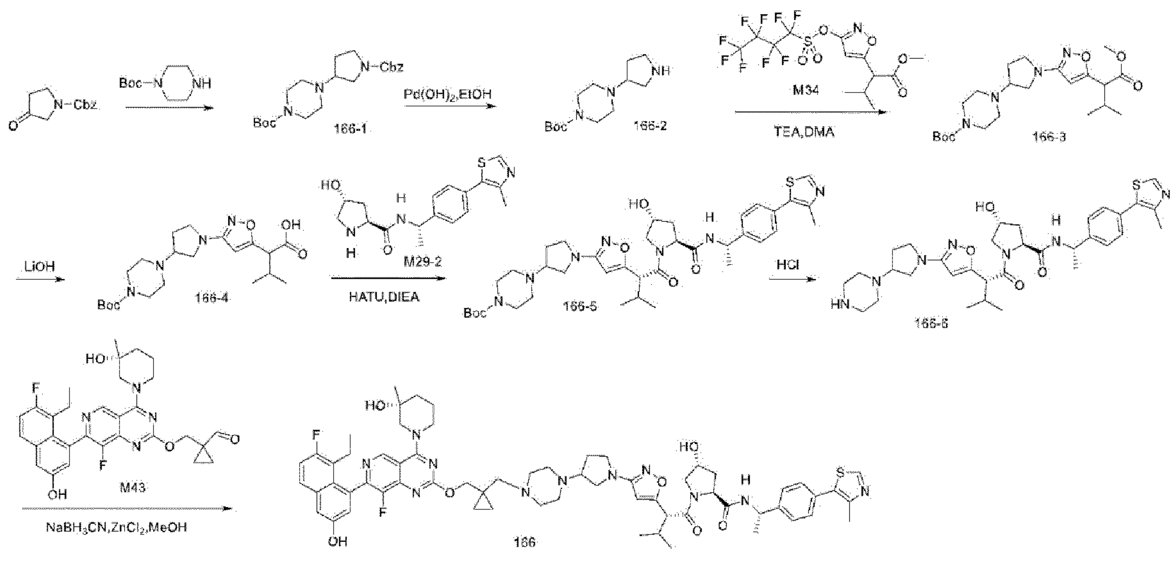

Step 1: Synthesis of compound 166-1

Benzyl 3-oxopyrrolidin-1-carboxylate (1.5 g) and benzyl piperazin-1-carboxylate (1.53 g) were dissolved in 30 mL of dichloroethane, and glacial acetic acid (0.82 g) was added. The mixture was stirred at room temperature for 30 minutes. Then, sodium triacetoxyborohydride (3.6 g) was added, and the mixture was stirred at room temperature overnight. After the reaction was completed, the reaction solution was diluted by adding water, and extracted three times with DCM. The organic phases were combined, washed with saturated saline, and dried with anhydrous sodium sulfate, filtered and concentrated. The resultant was purified by column chromatography (PE:EA = 4:6) to obtain compound 166-1 (3.0 g). ESI-MS m/z: 390.2 $[M+H]^+$.

Step 2: Synthesis of compound 166-2

Compound 166-1 (3.0 g) was added to a 50 mL single-necked flask, dissolved by adding 15 mL of ethanol, and palladium hydroxide (220 mg) was added. The reaction system was substituted five times with hydrogen and stirred at room temperature overnight. After the reaction was completed, the resultant was filtered with diatomaceous earth and the filtrate was rotary evaporated to dryness, and concentrated to obtain crude product compound 166-2 (2.1 g), which was directly fed into the next step. ESI-MS m/z: 256.3 $[M+H]^+$.

Step 3: Synthesis of compound 166-3

Compound 166-2 (2.1 g), M34 (3.3 g), and DIPEA (4.3 g) were dissolved in DMF (8 mL). The mixture was reacted at 140°C for 1 hour. After the reaction was completed, the reaction system was cooled to room temperature. The reaction solution was diluted by adding water, and extracted three times with ethyl acetate. The organic phases were collected and combined, washed with saturated saline, and dried with anhydrous sodium sulfate, filtered and concentrated. The resultant was purified by column chromatography to obtain compound 166-3 (1.4 g). ESI-MS m/z: 437.1 $[M+H]^+$.

Step 4: Synthesis of compound 166-4

Compound 166-3 (1.4 g) was dissolved in methanol (10 mL) and water (10 mL), and then lithium hydroxide (260 mg) was added. The mixture was reacted at room temperature for 1 hour. After the reaction was completed, the pH of the solution was adjusted to pH=2 with dilute hydrochloric acid. The reaction solution was extracted three times with dichloromethane:isopropanol (10:1), dried with anhydrous sodium sulfate, filtered and concentrated to obtain compound 166-4 (800 mg). ESI-MS m/z:423.3 $[M+H]^+$.

Step 5: Synthesis of compound 166-5

Compound 166-4 (800 mg), M29-2 (720 mg), HATU (1.4 g) and N,N-diisopropylethylamine (800 mg) were dissolved in N,N-dimethylformamide (10 mL), and the mixture was reacted at room temperature for 1 hour. After the reaction was completed, the reaction solution was diluted by adding water, and extracted three times with ethyl acetate. The organic phase was washed with saturated brine, dried with anhydrous sodium sulfate, filtered, and rotary evaporated to dryness. The concentrate was purified by column chromatography, and resolved by chiral column (column: CHIRAL PAK IE (20.0 mm × 250 mm, 5 μm); column temperature: 35°C; wavelength: 254 nm; flow rate: 25 mL/min; mobile phase: 0.1% diethylamine in n-hexane: 0.1% diethylamine in ethanol (15:85)) to obtain compound 166-5 (100 mg). ESI-MS m/z: 736.1 $[M+H]^+$.

Step 6: Synthesis of compound 166-6

Compound 166-5 (100 mg) was dissolved in dichloromethane (5 mL), and then 4M hydrogen chloride in dioxane (2 mL) was added. The mixture was reacted at room temperature for 20 minutes. After the reaction was completed, the solvent was rotary evaporated to dryness, washed twice with dichloromethane and rotary evaporated to dryness, and dissolved by adding water. The reaction solution was neutralized with sodium bicarbonate solution, extracted with dichloromethane/isopropanol (10:1), dried and concentrated to obtain compound 166-6 (80 mg). ESI-MS m/z:636.4 $[M+H]^+$.

Step 7: Synthesis of compound 166

Compound 166-6 (80 mg), M43 (69 mg), and 1 M zinc chloride in tetrahydrofuran (0.5 mL) were dissolved in methanol (10 mL). The mixture was reacted at 40°C for 1 hour. Sodium cyanoborohydride (32 mg) was added, and the

mixture was stirred at 70°C for 5 hours. After the reaction was completed, the reaction system was cooled to room temperature. The reaction was quenched by adding ammonium chloride solution, and the reaction solution was extracted with dichloromethane and methanol (10:1), dried with anhydrous sodium sulfate, filtered, and concentrated. The resultant was purified by column chromatography (DCM:MeOH = 85:15). The crude product was prepared by Pre-HPLC (C18 column, A: 0.05% diethylamine aqueous solution, B: acetonitrile, concentration gradient: 30% B to 80% B, 6.52-7.40 min, flow rate: 20 mL/min, 254 nm) to obtain compound 166 (42.4 mg).

ESI-MS m/z:585.2 $[M+H]^+$.

$^1H$ NMR (500 MHz, DMSO-$d_6$) δ 9.94 (s, 1H), 9.22 (s, 1H), 8.98 (s, 1H), 8.49 (d, J = 7.1 Hz, 1H), 7.96 (s, 1H), 7.76 (s, 1H), 7.44 (d, J = 8.2 Hz, 2H), 7.36 (d, J = 8.4 Hz, 2H), 7.33 (s, 1H), 7.03 (s, 1H), 5.93 (s, 1H), 5.10 (s, 1H), 4.96 (s, 1H), 4.86 (s, 1H), 4.74 (d, J = 6.0 Hz, 1H), 4.54 - 4.21 (m, 5H), 4.10 - 3.98 (m, 1H), 3.65 (dd, J = 22.6, 10.8 Hz, 1H), 3.54 (s, 2H), 2.92 (s, 2H), 2.74 (s, 2H), 2.64 (s, 1H), 2.34 (d, J = 27.3 Hz, 10H), 2.13 (s, 2H), 2.01 (s, 3H), 1.79 (s, 1H), 1.75 (s, 1H), 1.67 (s, 3H), 1.45 (d, J = 7.1 Hz, 1H), 1.33 (s, 3H), 1.24 (s, 3H), 1.16 (d, J = 11.5 Hz, 3H), 1.12 - 1.03 (m, 1H), 0.95 (d, J = 6.3 Hz, 2H), 0.81 (d, J = 6.2 Hz, 4H), 0.73 (d, J = 7.4 Hz, 4H), 0.64 (s, 2H), 0.40 (s, 2H).

Example 167 Synthesis of compound (2S,4R)-1-((S)-2-(4-(6-((1-((1-((7-(8-ethyl-7-fluoro-3-hydroxynaphthalen-1-yl)-8-fluoro-4-((R)-3-hydroxy-3-methylpiperidin-1-yl)pyrido[4,3-d]pyrimidin-2-yl)oxy)methyl)cyclopropyl)methyl) piperidin-4-yl)oxy)pyridazin-3-yl)-1H-1,2,3-triazol-1-yl)-3-methylbutanoyl)-4-hydroxy-N-((S)-1 -(4-(4-methylthiazol-5-yl)phenyl) ethyl)pyrrolidine-2-carboxamide

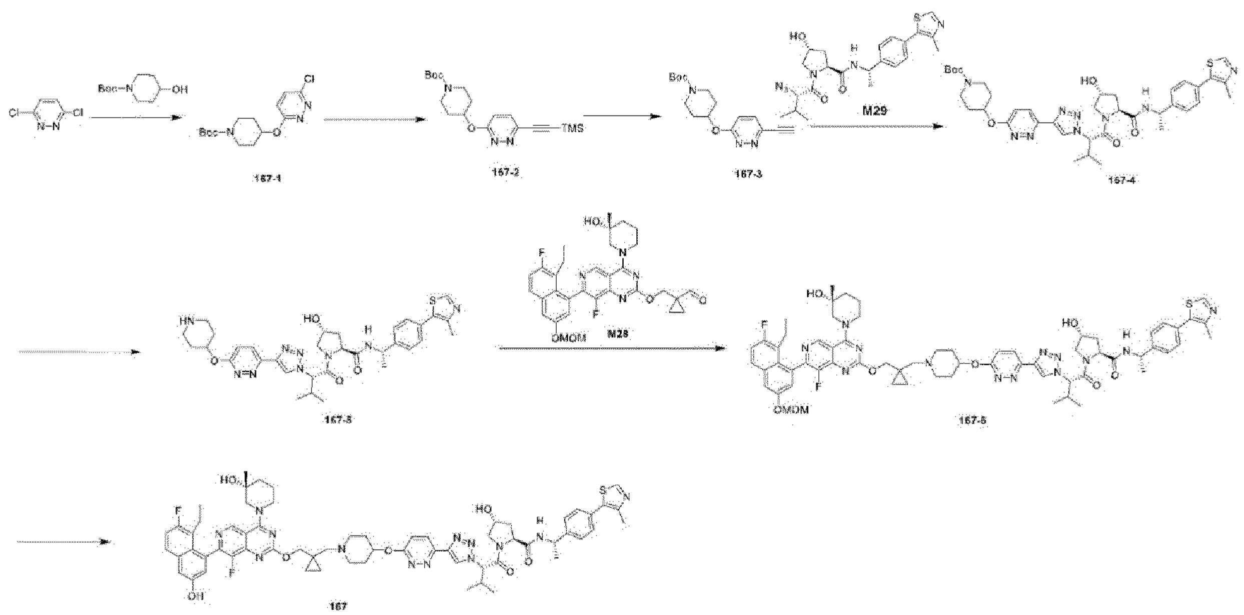


Step 1: Synthesis of compound 167-1

Tert-butyl 4-hydroxypiperidine-1-carboxylate (2.50 g) was dissolved in THF (20 mL), NaH (745 mg) was slowly added in an ice-bath, and the reaction was elevated to room temperature for 0.5 hours. Then, 3,6-dichloropyridazine (1.85 g) was added, and the reaction was slowly heated to 50°C for 3 hours. The reaction was monitored by LCMS until completion. The reaction was quenched by adding saturated ammonium chloride solution, and EA (50 mL) was added to the reaction solution. The organic phase was collected and concentrated. The resultant was separated and purified by column chromatography to obtain 3.80 g of white powder, i.e., compound 167-1. ESI-MS m/z: 314.1 $[M+H]^+$.

Step 2: Synthesis of compound 167-2

Compound 167-1 (1.0 g) was added to a 25 mL single-necked flask, and dissolved in THF (10 mL). Then, trimethylethynylsilane (907 mL), CuI (121 mg), $PdCl_2(PPh_3)_2$ (224 mg) and DIPEA (1.67 mL) were added. After the protection of nitrogen, the reaction was heated to 50°C for 3 hours. Saturated brine (5 mL) was added to the reaction solution. The mixture was extracted with EA, and the organic phase was dried with anhydrous sodium sulfate. The resultant was separated and purified by column chromatography to obtain 560 mg of a light yellow solid, i.e., compound

167-2. ESI-MS m/z: 376.2 $[M+H]^+$.

Step 3: Synthesis of compound 167-3

Compound 167-2 (560 mg) was added to a 25 mL single-necked flask, and dissolved in MeOH (10 mL). Then, potassium carbonate (763 mg) was added. The mixture was reacted at room temperature for 1 hour. The insoluble substance was removed by filtering with diatomaceous earth, and the filtrate was directly evaporated to dryness. The resultant was separated and purified by column chromatography to obtain 200 mg of a light yellow solid, i.e., compound 167-3. ESI-MS m/z: 304.2 $[M+H]^+$.

Step 4: Synthesis of compound 167-4

Compound M29 (271 mg) was added to a 25 mL single-necked flask, and dissolved in THF (5 mL). Then, compound 167-3 (180 mg), copper sulfate (85 mg), water (5 mL), tert-butanol (5 mL), and sodium ascorbate (306 mg) were added. The mixture was reacted at room temperature for 0.5 hours. The reaction solution was directly evaporated to dryness. The resultant was separated and purified by column chromatography to obtain 199 mg of a light yellow solid, i.e., compound 167-4. ESI-MS m/z: 760.4 $[M+H]^+$.

Step 5: Synthesis of compound 167-5

Compound 167-4 (199 mg) was added to a 20 mL single-necked flask, and dissolved in 4M hydrogen chloride in dioxane (5 mL). Then, DCM (3 mL) and MeOH were added. The mixture was reacted at room temperature for 1 hour. The reaction solution was directly evaporated to dryness. After 5 mL of saturated sodium bicarbonate solution was added to the reaction solution to adjust the pH of the solution until pH=7 to 8, the reaction solution was extracted with a solution of dichloromethane/isopropanol. The organic phase was dried with anhydrous sodium sulfate to obtain 175 mg of a yellow solid, i.e., compound 167-5. ESI-MS m/z: 660.3 $[M+H]^+$.

Step 6: Synthesis of compound 167-6

Compound 167-5 (175 mg) was added to a 25 mL single-necked flask, and dissolved in MeOH (5 mL). Then, M28 (157 mg) and 1 M zinc chloride in tetrahydrofuran (0.53 mL) were added. After the reaction was heated to 40°C for 2 hours, $NaBH_3CN$ (50.0 mg) was added. The mixture was reacted overnight. The reaction solution was directly evaporated to dryness. The resultant was separated and purified by column chromatography to obtain 153 mg of a light yellow solid, i.e., compound 167-6. ESI-MS m/z: 618.3 1/2$[M+2H]^+$.

Step 7: Synthesis of compound 167

Compound 167-6 (153 mg) was added to a 25 mL single-necked flask, and dissolved in DCM (2 mL). Then, TFA (2 mL) was added. The mixture was reacted at room temperature for 1 hour. The reaction solution was directly evaporated to dryness. After 5 mL of saturated sodium bicarbonate solution was added to the reaction solution to adjust the pH of the solution until pH=7 to 8, the reaction solution was extracted with a mixed solution of 10% dichloromethane/isopropanol. The organic phase was dried with anhydrous sodium sulfate, and the resultant was separated by Pre-HPLC ($CH_3CN/H_2O$ = 30%-55%) to obtain 40.4 mg of a white solid, i.e., compound 167.

ESI-MS m/z: 597.3 1/2$[M+2H]^+$.

$^1$H NMR (500 MHz, DMSO-$d_6$) δ 9.94 (s, 1H), 9.23 (s, 1H), 8.98 (d, *J* = 12.4 Hz, 1H), 8.72 (d, *J* = 9.6 Hz, 1H), 8.55 (d, *J* = 7.6 Hz, 1H), 8.19 - 8.12 (m, 1H), 7.77 (dd, *J* = 9.0, 6.0 Hz, 1H), 7.48 - 7.43 (m, 2H), 7.36 (dt, *J* = 8.3, 7.3 Hz, 5H), 7.26 *(d, J* = 9.3 Hz, 1H), 7.05 (d, *J* = 2.4 Hz, 1H), 5.43 (d, *J* = 10.1 Hz, 1H), 5.18 (t, *J* = 9.4 Hz, 2H), 4.94 (dd, J = 14.2, 7.1 Hz, 1H), 4.76 (d, *J* = 7.2 Hz, 1H), 4.37 (ddd, *J* = 26.1, 16.0, 9.6 Hz, 5H), 4.05 (dd, *J* = 26.4, 13.3 Hz, 1H), 3.80 - 3.52 (m, 3H), 2.83 (s, 2H), 2.46 (s, 3H), 2.31 (d, *J* = 52.0 Hz, 5H), 2.09 - 1.97 (m, 4H), 1.67 (dd, *J* = 22.6, 13.1 Hz, 5H), 1.39 (d, *J* = 7.0 Hz, 3H), 1.19 - 1.14 (m, 4H), 1.10 (d, *J* = 6.4 Hz, 3H), 0.88 - 0.82 (m, 1H), 0.78 - 0.65 (m, 9H), 0.44 (s, 2H).

Example 168 Synthesis of compound (2S,4R)-1-((S)-2-(4-(5-(7-((7-(8-ethyl-7-fluoro-3-hydroxynaphthalen-1-yl)-8-fluoro-4-((R)-3-hy droxy-3-methylpiperidin-1-yl)pyrido[4,3-d]pyrimidin-2-yl)oxy)methyl)cyclopropyl)methyl)-2,7-azaspiro[3.5]nonan-2-yl)pyrazin-2-yl)-1H-1,2,3-triazol-1-yl)-3-methylbutanoyl)-4-hydroxy-N-(( S)-1-(4-methylthiazol-5-yl) phenyl)ethyl)pyrrolidine-2-carboxamide

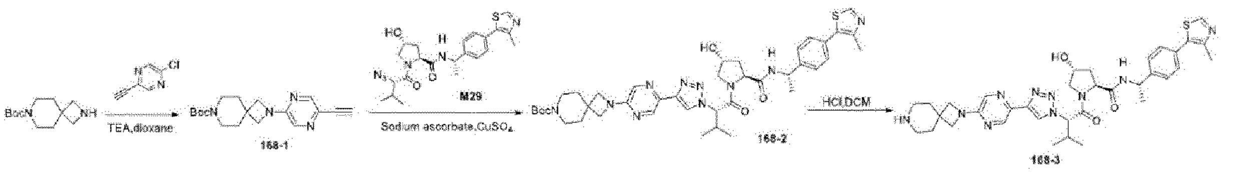


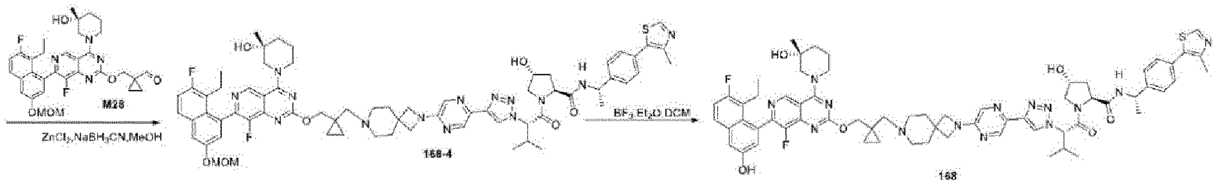


Step 1: Synthesis of compound 168-1

Tert-butyl 2,7-diazaspiro[3.5]nonan-7-carboxylate (212 mg) was dissolved by adding 3 mL of dioxane, then 2-chloro-5-ethynylpyrazine (100 mg) was added, followed by the addition of triethylamine (0.30 mL). The reaction was stirred at 60°C for 2 hours. The reaction was monitored by LCMS until completion. The reaction solution was concentrated under reduced pressure and the resultant was purified by column chromatography to obtain compound 168-1 (180 mg). ESI-MS m/z: 329.2 $[M+H]^+$.

Step 2: Synthesis of compound 168-2

Compound 168-1 (180 mg) was added to a 50 mL single-necked flask, 6 mL of tetrahydrofuran, 3 mL of tert-butanol and 3 mL of water were added, and then M29 (215 mg), copper sulfate (68 mg), sodium ascorbate (243 mg) were added. The mixture was stirred at 20°C for 12 hours. The reaction was monitored by LCMS until completion. The reaction solution was concentrated under reduced pressure and the resultant was purified by column chromatography to obtain compound 168-2 (173 mg). ESI-MS m/z: 785.4 $[M+H]^+$.

Step 3: Synthesis of compound 168-3

Compound 168-2 (173 mg) was added to a 10 mL single-necked flask, 3 mL of dichloromethane, and 4M hydrogen chloride in dioxane (1 mL) were added. The mixture was stirred at 20°C for 0.5 hours. The reaction solution was neutralized with saturated sodium bicarbonate solution, extracted twice with dichloromethane:isopropanol (10:1), and the organic phases were collected and combined, dried with anhydrous sodium sulfate and concentrated to obtain crude compound 168-3 (150 mg). ESI-MS m/z:685.3 $[M+H]^+$.

Step 4: Synthesis of compound 168-4

M28 (105 mg) was added to a 50 mL single-necked flask, 4 mL of methanol, compound 168-3 (146 mg) were added, and then 1M zinc chloride in tetrahydrofuran (0.27 mL) was added. The mixture was stirred at 40°C for 2 hours. Sodium cyanoborohydride (46 mg) was added, and the mixture was stirred at 50°C for 12 hours. The reaction was monitored by LCMS until completion. The reaction solution was concentrated under reduced pressure and the resultant was purified by column chromatography to obtain compound 168-4 (201 mg). ESI-MS m/z:631.3 $[M+H]^+$.

Step 5: Synthesis of compound 168

Compound 168-4 (201 mg) was added to a 10 mL single-necked flask, 2 mL of dichloromethane was added, and then 1 mL of trifluoroacetic acid was added at 0°C. The reaction was stirred at 20°C for 1 hour. The reaction was monitored by LCMS until completion. The reaction solution was neutralized with saturated sodium bicarbonate solution, extracted twice with dichloromethane:isopropanol (5:1), and the organic phases were collected and combined, dried with anhydrous sodium sulfate and concentrated under reduced pressure. The resultant was purified by pre-HPLC to obtain compound 168 (28.2 mg).

ESI-MS m/z:609.86 $[M+H]^+$.

$^1H$ NMR (500 MHz, Methanol-$d_4$) $\delta$ 9.21 (d, J = 4.6 Hz, 1H), 8.86 (d, J = 6.3 Hz, 1H), 8.61 (dd, J = 7.4, 1.5 Hz, 1H), 8.43 (s, 1H), 7.85 (dd, J = 4.5, 1.5 Hz, 1H), 7.67 (dd, J = 9.1, 5.8 Hz, 1H), 7.48 - 7.40 (m, 4H), 7.30 (d, J = 2.6 Hz, 1H), 7.24 (t, J = 9.4

Hz, 1H), 7.06 (t, J = 2.4 Hz, 1H), 5.36 (d, J = 10.2 Hz, 1H), 5.05 (t, J = 7.1 Hz, 1H), 4.58 (s, 1H), 4.53 (t, J = 8.3 Hz, 1H), 4.46 (dt, J = 7.2, 4.5 Hz, 2H), 4.26 (t, J = 14.1 Hz, 1H), 3.91 (td, J = 10.2, 9.6, 5.6 Hz, 1H), 3.85 (s, 4H), 3.63 (dd, J = 32.7, 13.4 Hz, 1H), 3.52 - 3.43 (m, 1H), 2.65 - 2.53 (m, 4H), 2.48 (s, 4H), 2.24 - 2.15 (m, 3H), 1.97 (ddd, J = 13.3, 9.0, 4.5 Hz, 1H), 1.87 (d, J = 9.0 Hz, 4H), 1.79 (t, J = 10.5 Hz, 2H), 1.66 (d, J = 7.0 Hz, 1H), 1.53 (d, J = 7.0 Hz, 3H), 1.15 (dd, J = 6.4, 2.1 Hz, 3H), 0.92 - 0.85 (m, 9H), 0.83 - 0.80 (m, 5H), 0.75 (s, 2H), 0.54 (s, 2H).

Example 169 Synthesis of compound (2S,4R)-1-((S)-2-(4-(5-(1-((1-((7-(8-ethyl-7-fluoro-3-hydroxynaphthalen-1-yl)-8-fluoro-4-((R)-3 -hydroxy-3-methylpiperidin-1-yl)pyrido[4,3-d]pyrimidin-2-yl)oxy)methyl)cyclopropyl)methyl)p iperidin-4-yl)oxy)pyrimidin-2-yl)-1H-1,2,3-triazol-1-yl)-3-methylbutanoyl)-4-hydroxy-N-((S)-1-(4-hydroxyl-N-(4-(4-methylthiazol-5-yl)phenyl)ethyl)pyrrolidine-2-carboxamide

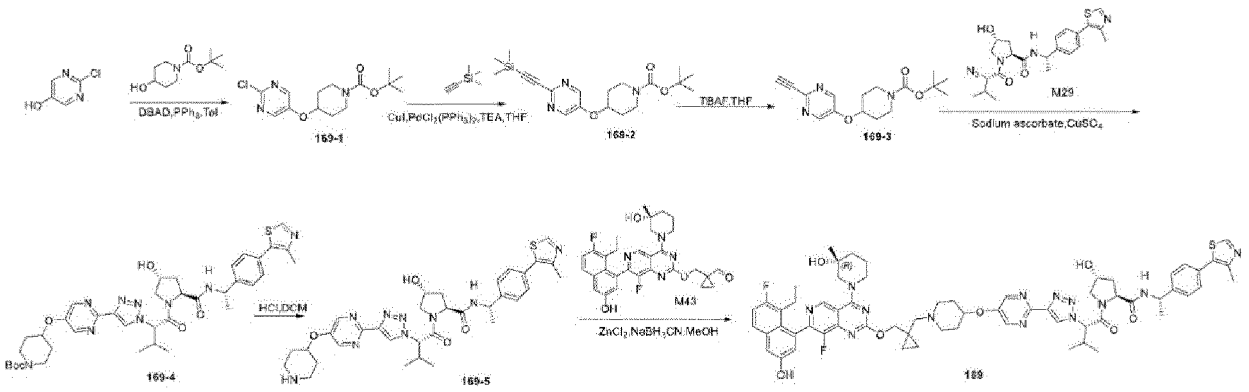


Step 1: Synthesis of compound 169-1

2-Chloro-5-hydroxypyrimidine (500 mg) was dissolved by adding 10 mL of toluene, and 1-tert-butoxycarbonyl-4-hydroxypyridine (771 mg) was added, followed by the addition of triphenylphosphine (2009 mg) and di-tert-butyl azodicarboxylate (1.66 mL). The reaction system was substituted three times with nitrogen, and under the protection of nitrogen atmosphere. The reaction was stirred at 85°C for 1 hour. The reaction was monitored by LCMS until completion. The reaction solution was concentrated and the resultant was purified by column chromatography to obtain compound 169-1 (915 mg). ESI-MS m/z: 314.1 $[M+H]^+$.

Step 2: Synthesis of compound 169-2

Compound 169-1 (835 mg) was added to a 100 mL single-necked flask, and 10 mL of tetrahydrofuran was added. Then, 0.75 mL of trimethylethynylsilane, cuprous iodide (51 mg), bistriphenylphosphine palladium dichloride (187 mg) and 1.85 mL of triethylamine were added. The reaction system was substituted three times with nitrogen under the protection of nitrogen atmosphere. The reaction was stirred at 90°C for 12 hours. The reaction was monitored by LCMS until completion. The reaction solution was concentrated and the resultant was purified by column chromatography to obtain compound 169-2 (611 mg). ESI-MS m/z: 376.2 $[M+H]^+$.

Step 3: Synthesis of compound 169-3

Compound 169-2 (561 mg) was added to a 50 mL single-necked flask, and 6 mL of tetrahydrofuran was added. 1M tetrabutyl ammonium fluoride in tetrahydrofuran (2.24 mL) was added at 0°C, and the mixture was stirred at 20°C for 1 hour. The reaction was monitored by LCMS until completion. The reaction solution was concentrated and the resultant was purified by column chromatography to obtain compound 169-3 (73 mg). ESI-MS m/z: 304.2 $[M+H]^+$.

Step 4: Synthesis of compound 169-4

Compound 169-3 (76 mg) was added to a 50 mL single-necked flask, 3 mL of tetrahydrofuran, 1.5 mL of tert-butanol and 1.5 mL of water were added, and then M29 (95 mg), copper sulfate (30 mg), and sodium ascorbate (107 mg) were added. The mixture was stirred at 20°C for 12 hours. The reaction was monitored by LCMS until completion. The reaction solution was concentrated and the resultant was purified by column chromatography to obtain compound 169-4

(155 mg). ESI-MS m/z: 760.4 $[M+H]^+$.

Step 5: Synthesis of compound 169-5

Compound 169-4 (155 mg) was added to a 10 mL single-necked flask, 3 mL of dichloromethane, and 4M hydrogen chloride in dioxane (1 mL) were added. The mixture was stirred at 20°C for 0.5 hours. The reaction solution was neutralized with saturated sodium bicarbonate solution, extracted twice with dichloromethane:isopropanol (10:1), and the organic phases were collected and combined, dried with anhydrous sodium sulfate and concentrated to obtain crude compound 169-5 (79 mg). ESI-MS m/z:660.3 $[M+H]^+$.

Step 6: Synthesis of compound 169

M43 (65 mg) was added to a 50 mL single-necked flask, 3 mL of methanol was added, compound 169-5 (78 mg) was added, and then 1M zinc chloride in tetrahydrofuran (0.18 mL) was added. The mixture was stirred at 40°C for 2 hours. Sodium cyanoborohydride (30 mg) was added, and the mixture was stirred at 50°C for 12 hours. The reaction was monitored by LCMS until completion. The reaction was quenched with saturated ammonium chloride solution, and the reaction solution was neutralized with saturated sodium bicarbonate solution, extracted twice with dichloromethane:isopropanol (5:1), and the organic phases were collected and combined, dried with anhydrous sodium sulfate and concentrated under reduced pressure. The resultant was purified by pre-HPLC to obtain compound 169 (45 mg).

ESI-MS m/z:597.16 $[M+H]^+$.

$^1H$ NMR (500 MHz, Methanol-$d_4$) δ 9.20 (d, J = 5.2 Hz, IH), 8.86 (d, J = 8.5 Hz, 1H), 8.65 (d, J = 13.0 Hz, 1H), 8.53 (d, J = 3.2 Hz, 2H), 7.66 (dd, J = 9.1, 5.8 Hz, 1H), 7.43 (q, J = 8.4 Hz, 4H), 7.30 (d, J = 2.7 Hz, 1H), 7.24 (t, J = 9.3 Hz, 1H), 7.07 (t, J = 2.6 Hz, 1H), 5.42 (d, J = 10.0 Hz, 1H), 5.05 (q, J = 7.0 Hz, 1H), 4.60 (s, 2H), 4.56 - 4.44 (m, 5H), 4.26 (t, J = 13.5 Hz, 1H), 3.94 - 3.84 (m, 2H), 3.62 (dd, J = 32.1, 13.3 Hz, 1H), 3.45 (q, J = 11.5 Hz, 1H), 2.99 (s, 2H), 2.61 (ddt, J = 16.5, 13.3, 6.6 Hz, 3H), 2.48 (s, 3H), 2.45 (s, 1H), 2.26 - 2.03 (m, 6H), 1.97 (ddd, J = 13.3, 9.1, 4.4 Hz, 1H), 1.89 - 1.74 (m, 5H), 1.53 (d, J = 7.0 Hz, 3H), 1.28 (d, J = 13.1 Hz, 3H), 1.16 (d, J = 6.8 Hz, 3H), 0.82 (dd, J = 9.8, 7.0 Hz, 6H), 0.77 (d, J = 9.4 Hz, 2H), 0.57 (s, 2H).

Example 170 Synthesis of compound (2S,4R)-1-((S)-2-(4-(5-(7-((7-(8-ethyl-7-fluoro-3-hydroxynaphthalen-1-yl)-8-fluoro-4-((R)-3-hy droxy-3-methylpiperidin-1-yl)pyrido[4,3-d]pyrimidin-2-yl)oxy)methyl)cyclopropyl)methyl)-2,7-azaspiro[3.5]nonan-2-yl)pyrazin-2-yl)-1H-1,2,3-triazol-1-yl)-3-methylbutanoyl)-4-hydroxy-N-(( R)-2-hydroxy-1-(4-(4-methylthiazol-5-yl)phenyl)ethyl)pyrrolidine-2-carboxamide

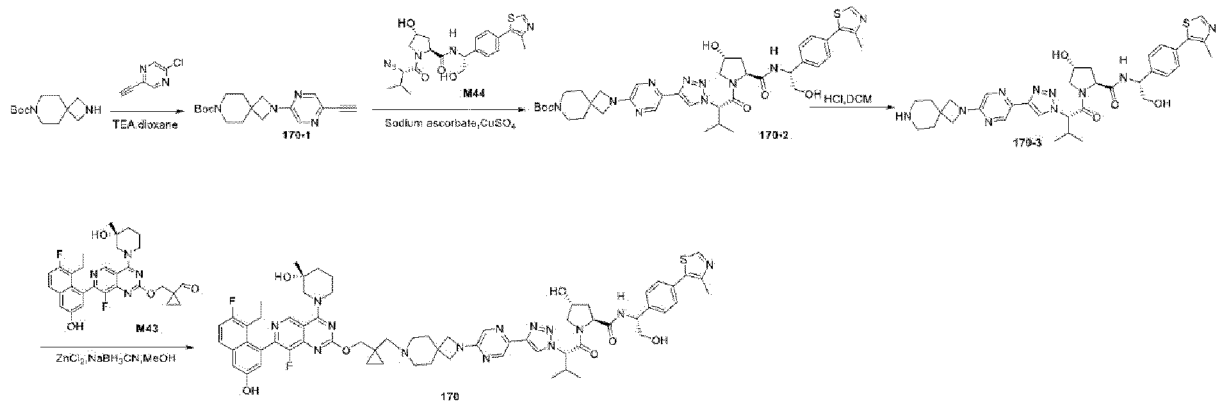


Step 1: Synthesis of compound 170-1

Tert-butyl 2,7-diazaspiro[3.5]nonan-7-carboxylate (212 mg) was dissolved by adding 3 mL of dioxane, then 2-chloro-5-ethynylpyrazine (100 mg) was added, followed by the addition of triethylamine (0.30 mL). The reaction was stirred at 60°C for 2 hours. The reaction was monitored by LCMS until completion. The reaction solution was concentrated and the resultant was purified by column chromatography to obtain compound 170-1 (193 mg). ESI-MS m/z: 329.2 $[M+H]^+$.

Step 2: Synthesis of compound 170-2

Compound 170-1 (193 mg) was added to a 50 mL single-necked flask, 6 mL of tetrahydrofuran, 3 mL of tert-butanol and 3 mL of water were added, and then M44 (223 mg), copper sulfate (68 mg), and sodium ascorbate (243 mg) were added. The mixture was stirred at 20°C for 12 hours. The reaction was monitored by LCMS until completion. The reaction solution was concentrated and the resultant was purified by column chromatography to obtain compound 170-2 (219 mg). ESI-MS m/z: 801.4 $[M+H]^+$.

Step 3: Synthesis of compound 170-3

Compound 170-2 (219 mg) was added to a 10 mL single-necked flask, 3 mL of dichloromethane, and 4M hydrogen chloride in dioxane (1 mL) were added. The mixture was stirred at 20°C for 0.5 hours. The reaction solution was neutralized with saturated sodium bicarbonate solution, extracted twice with dichloromethane:isopropanol (10:1), and the organic phases were collected and combined, dried with anhydrous sodium sulfate and concentrated to obtain crude compound 170-3 (112 mg). ESI-MS m/z:701.3 $[M+H]^+$.

Step 4: Synthesis of compound 170

M43 (75 mg) was added to a 50 mL single-necked flask, 3 mL of methanol, compound 170-3 (110 mg) were added, and then 1M zinc chloride in tetrahydrofuran (0.21 mL) was added. The mixture was stirred at 40°C for 2 hours. Sodium cyanoborohydride (34 mg) was added, and the mixture was stirred at 50°C for 12 hours. The reaction was monitored by LCMS until completion. The reaction was quenched with saturated ammonium chloride solution, and the reaction solution was neutralized with saturated sodium bicarbonate solution, extracted twice with dichloromethane:isopropanol (5:1), and the organic phases were collected and combined, dried with anhydrous sodium sulfate and concentrated under reduced pressure. The resultant was purified by pre-HPLC to obtain compound 170 (40.3 mg).

ESI-MS m/z: 617.93 $[M+H]^+$.

$^1$H NMR (500 MHz, Methanol-$d_4$) δ 9.20 (d, J = 4.1 Hz, 1H), 8.87 (d, J = 5.3 Hz, 1H), 8.60 (s, 1H), 8.44 (s, 1H), 7.85 (s, 1H), 7.67 (dd, J = 9.1, 5.8 Hz, 1H), 7.46 (s, 4H), 7.30 (d, J = 2.7 Hz, 1H), 7.24 (t, J = 9.3 Hz, 1H), 7.06 (t, J = 2.3 Hz, 1H), 5.38 (d, J = 10.2 Hz, 1H), 5.05 (t, J = 6.1 Hz, 1H), 4.59 (s, 1H), 4.52 - 4.47 (m, 2H), 4.45 (dd, J = 7.1, 5.2 Hz, 2H), 4.25 (t, J = 14.1 Hz, 1H), 3.93 (dd, J = 11.0, 3.8 Hz, 1H), 3.87 (d, J = 11.1 Hz, 1H), 3.85 - 3.82 (m, 5H), 3.63 (dd, J = 33.7, 13.3 Hz, 1H), 3.52 - 3.42 (m, 1H), 2.84 (q, J = 7.3 Hz, 1H), 2.65 - 2.59 (m, 1H), 2.47 (d, J = 6.9 Hz, 6H), 2.41 (d, J = 12.8 Hz. 1H), 2.20 (tdd, J = 11.6, 9.3, 6.0 Hz, 3H), 2.01 (ddd, J = 13.3, 9.1, 4.6 Hz, 1H), 1.85 (q, J = 9.2, 6.0 Hz, 5H), 1.79 (dp, J = 12.0, 4.3 Hz, 2H), 1.29 (d, J = 13.3 Hz, 4H), 1.21 (t, J = 7.2 Hz, 2H), 1.15 (d, J = 6.6 Hz, 3H), 0.81 (dd, J = 10.4, 6.9 Hz, 6H), 0.72 (s, 2H), 0.51 (s, 2H).

Example 171 Synthesis of compound (2S,4R)-1-((S)-2-(4-(6-(1-((1-((7-(8-ethyl-7-fluoro-3-hydroxynaphthalen-1-yl)-8-fluoro-4-((R)-3 -hydroxy-3-methylpiperidin-1-yl)pyrido[4,3-d]pyrimidin-2-yl)oxy)methyl)cyclopropyl)methyl)p iperidin-4-yl)oxy)pyridin-3-yl)-1H-1,2,3-triazol-1-yl)-3-methylbutanoyl)-4-hydroxy-N-((R)-2-hy droxyl-1-(4-methylthiazol-5-yl)phenyl)ethyl)pyrrolidine-2-carboxamide

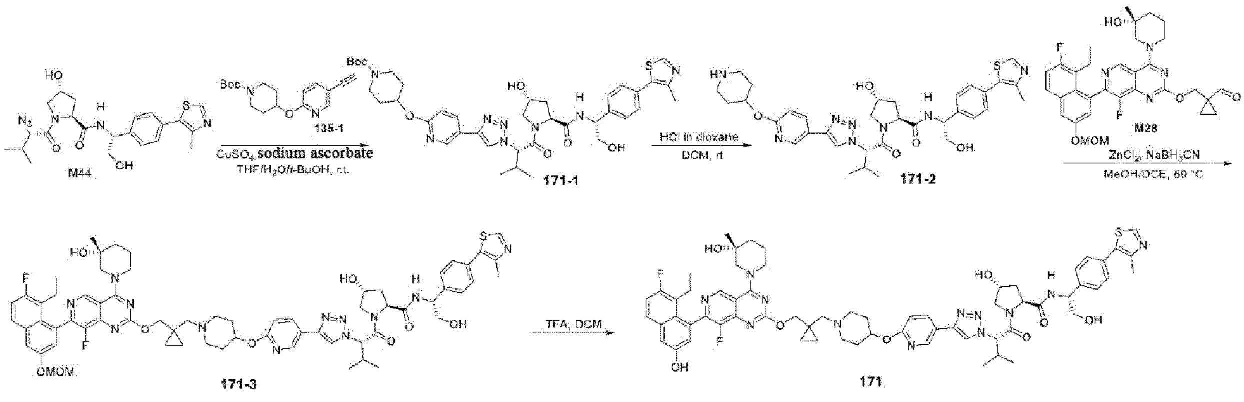


Step 1: Synthesis of compound 171-1

Intermediate M44 (100.00 mg), compound 135-1 (95.97 mg), copper sulfate (30.39 mg), sodium ascorbate (109.00 mg), tetrahydrofuran (1.20 mL), tert-butanol (1.20 mL), and water (1.20 mL) were added to a reaction flask. The mixture was reacted at room temperature for 1 hour under the protection of nitrogen, after the reaction system was substituted with nitrogen. The reaction solution was concentrated, diluted by adding water, and then extracted with DCM

and methanol, dried with anhydrous sodium sulfate, and concentrated. The resultant was separated by column chromatography (DCM/ammonia in methanol = 18:1) to obtain a white solid compound 171-1 (124 mg), ESI-MS m/z: 775.4 $[M+H]^+$.

Step 2: Synthesis of compound 171-2

Compound 171-1 (124.0 mg), dichloromethane (3.00 mL), and 4M hydrochloride in dioxane (1.00 mL) were added to a reaction flask, and the mixture was reacted at room temperature for 0.5 hours. After the reaction was completed, the supernatant was decanted, the insoluble substance was dissolved by adding methanol, and the pH of the solution was adjusted to pH=8 with aqueous sodium bicarbonate solution. The reaction solution was extracted with dichloromethane and methanol, dried with anhydrous sodium sulfate, and concentrated to obtain compound 171-2 (84 mg), ESI-MS m/z: 675.5 $[M+H]^+$.

Step 3: Synthesis of compound 171-3

Compound 171-2 (71.91 mg), intermediate M28 (50.00 mg), methanol (2.00 mL), 1,2-dichloroethane (2.00 mL) and 1 M zinc chloride in tetrahydrofuran (0.08 mL) were added to a reaction flask, and the mixture was reacted at 40°C for 1 hour. Then, sodium cyanoborohydride (53.04 mg, 0.84 mmol) was added, the mixture was reacted at 60°C for 2 hours, diluted by adding water, extracted with DCM, dried with anhydrous sodium sulfate, and concentrated. The resultant was prepared by Pre-TLC (DCM/ammonia in methanol = 18:1) to obtain a light yellow solid compound 171-3 (56 mg), ESI-MS m/z: 626.7 1/2$[M+2H]^+$.

Step 4: Synthesis of compound 171

Compound 171-3 (56.00 mg), dichloromethane (1.00 mL), and trifluoroacetic acid (1.00 mL) were added to a reaction flask, and the mixture was reacted at room temperature for 0.6 hours, concentrated and dissolved by adding DCM. The reaction solution was added to an ice-cold aqueous sodium bicarbonate solution to adjust pH=8, extracted with dichloromethane and methanol, dried with anhydrous sodium sulfate, and concentrated. The resultant was prepared by Pre-TLC (DCM/ammonia in methanol = 10:1) to obtain compound 171 (25.5 mg).

ESI-MS m/z: 604.8 1/2$[M+2H]^+$.

$^1$H NMR (500 MHz, Methanol-$d_4$) δ 9.19 (d, *J* = 7.4 Hz, 1H), 8.86 (d, *J*= 6.3 Hz, 1H), 8.56 (d, *J* = 2.1 Hz, 1H), 8.48 (d, *J* = 6.3 Hz, 1H), 8.06 (dd, *J* = 8.6, 2.3 Hz, 1H), 7.65 (dd, *J* = 8.8, 5.8 Hz, 1H), 7.43 (d, *J* = 11.2 Hz, 4H), 7.31 - 7.19 (m, 2H), 7.10 - 7.05 (m, 1H), 6.81 (d, *J* = 8.6 Hz, 1H), 5.37 (d, *J* = 10.2 Hz, 1H), 5.05 (t, *J* = 6.1 Hz, 2H), 4.60 (t, *J* = 8.3 Hz, 1H), 4.47 (m, 3H), 4.23 (t, *J* = 11.6 Hz, 1H), 3.89 (m, 3H), 3.65 - 3.42 (m, 2H), 3.31 (dt, *J* = 3.3, 1.6 Hz, 2H), 2.91 (s, 2H), 2.68 - 2.59 (m, 1H), 2.53 - 2.32 (m, 8H), 2.23 - 1.98 (m, 6H), 1.81 (m, 5H), 1.31 - 1.23 (m, 4H), 1.15 (d, *J* = 6.6 Hz, 3H), 0.85 - 0.78 (m, 6H), 0.72 (s, 2H), 0.51 (s, 2H).

Example 172 Synthesis of compound (2S,4R)-N-((R)-1-(4-(1-ethyl-1H-pyrazol-5-yl)phenyl)-2-hydroxyethyl)-1-((S)-2-(4-(1-((7-(8-eth yl-7-fluoro-3-hydroxynaphthalen-1-yl)-8-fluoro-4-((R)-3-hydroxy-3-methylpiperidin-1-yl)pyrido [4,3-d]pyrimidin-2-yl)oxy)methylcyclopropyl)methylpiperidin-4-yl)oxy)pyrazin-2-yl)-1H-1,2,3-t riazol-1-yl)-3-methylbutanoyl)-4-hydroxypyrrolidine-2-carboxamide

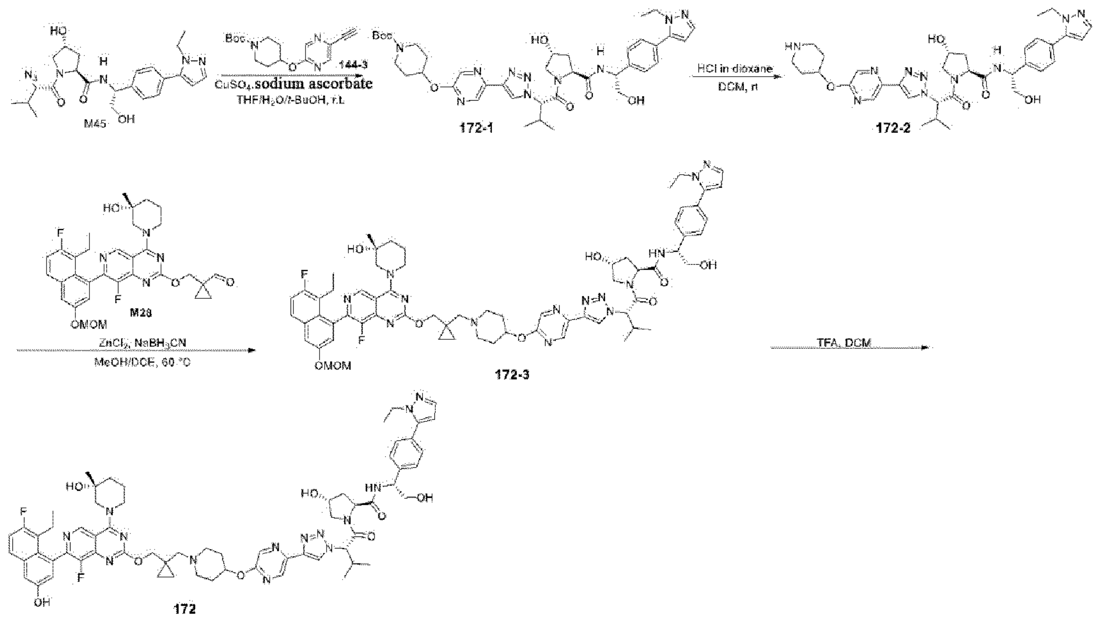

M45 172-1 172-2 M28 172-3 172

Step 1: Synthesis of compound 172-1

Intermediate M45 (105.00 mg), compound 144-3 (88.2 mg), copper sulfate (32.60 mg), sodium ascorbate (115.1 mg), tetrahydrofuran (1.50 mL), tert-butanol (1.50 mL), and water (1.50 mL) were added to a reaction flask. After the nitrogen substitution, the mixture was reacted at room temperature for 1 hour. The reaction solution was concentrated, diluted with water, extracted with DCM and methanol, dried with anhydrous sodium sulfate, and concentrated. The resultant was separated by column chromatography (DCM/ammonia in methanol = 18:1) to obtain a light yellow solid compound 172-1 (115 mg), ESI-MS m/z: 773.4 $[M+H]^+$.

Step 2: Synthesis of compound 172-2

Compound 172-1 (115.0 mg), dichloromethane (2.00 mL), and 4 M hydrochloride in dioxane (0.40 mL) were added to a reaction flask, and the mixture was reacted at room temperature for 0.5 hours. After the reaction was completed, the supernatant was decanted, the insoluble substance was dissolved by adding methanol, and the pH of the solution was adjusted to pH=8 with aqueous sodium bicarbonate solution. The reaction solution was extracted with DCM and methanol, dried with anhydrous sodium sulfate, and concentrated to obtain compound 172-2 (84 mg), ESI-MS m/z: 673.5 $[M+H]^+$.

Step 3: Synthesis of compound 172-3

Compound 172-2 (82.3 mg), intermediate M28 (65.00 mg), methanol (2.00 mL), 1,2-dichloroethane (2.00 mL) and 1M zinc chloride in tetrahydrofuran (0.1 mL) were added to a reaction flask, and the mixture was reacted at 40°C for 1 hour. Then, sodium cyanoborohydride (69.04 mg) was added, the mixture was reacted at 60°C for 3 hours, diluted by adding water, extracted with DCM, dried with anhydrous sodium sulfate, and concentrated. The resultant was prepared by Pre-TLC (DCM/ammonia in methanol = 18:1) to obtain a yellow solid compound 172-3 (66 mg), ESI-MS m/z: 625.8 1/2 $[M+2H]^+$.

Step 4: Synthesis of compound 172

Compound 172-3 (66.00 mg), dichloromethane (1.00 mL), and trifluoroacetic acid (1.00 mL) were added to a reaction flask, and the mixture was reacted at room temperature for 0.6 hours, concentrated and dissolved in DCM. The reaction solution was added to an ice-cold aqueous sodium bicarbonate solution to adjust pH=8, extracted with DCM and methanol, dried with anhydrous sodium sulfate, and concentrated. The resultant was separated by preparative liquid chromatography to obtain compound 172 (19.5 mg). ESI-MS m/z: 603.7 1/2$[M+2H]^+$.
$^1$H NMR (500 MHz, Methanol-$d_4$) δ 9.20 (d, *J* = 6.1 Hz, 1H), 8.72 (d, *J* = 1.4 Hz, 1H), 8.56 (d, *J* = 22.8 Hz, 1H), 8.17 (d, *J* = 1.3 Hz, 1H), 7.65 (dt, *J* = 14.7, 7.4 Hz, 1H), 7.51 (dd, *J* = 5.8, 1.9 Hz, 1H), 7.49 - 7.39 (m, 4H), 7.29 (d, *J* = 2.6 Hz, 1H), 7.23 (t, *J* = 9.2 Hz, 1H), 7.07 (t, *J* = 2.8 Hz, 1H), 6.29 (dd, *J* = 9.9, 1.9 Hz, 1H), 5.41 (d, *J* = 10.1 Hz, 1H), 5.07 (dd, *J* = 12.8, 6.8 Hz, 2H), 4.60 (t, *J* = 8.3 Hz, 1H), 4.50 - 4.44 (m, 3H), 4.24 (t, *J* = 12.9 Hz, 1H), 4.16 (q, *J* = 7.2 Hz, 2H), 3.89 (m, 3H), 3.62 (dd, *J* = 33.7,

13.3 Hz, 1H), 3.46 (dd, $J$ = 22.1, 10.7 Hz, 1H), 3.31 (dt, $J$ = 3.3, 1.6 Hz, 3H), 2.90 (s, 2H), 2.66 - 2.58 (m, 1H), 2.46 (t, $J$ = 9.3 Hz, 2H), 2.35 (s, 2H), 2.22 - 2.01 (m, 6H), 1.79 (m, 4H), 1.33 (m, 4H), 1.30 - 1.26 (m, 4H), 1.16 (d, $J$ = 6.6 Hz, 3H), 0.81 (dd, $J$ = 14.7, 7.1 Hz, 6H), 0.72 (s, 2H), 0.51 (s, 2H).

Example 173 Synthesis of compound (2S,4R)-1-((S)-2-(4-(5-(4-((1-((7-(8-ethyl-7-fluoro-3-hydroxynaphthalen-1-yl)-8-fluoro-4-((R)-3 -hydroxy-3-methylpiperidin-1-yl)pyrido[4,3-d]pyrimidin-2-yl)oxy)methyl)cyclopropyl)methyl)p iperazin-1-carbonyl)pyrazin-2-yl)-1H-1,2,3-triazol-1-yl)-3-methylbutanoyl)-4-hydroxy-N-((S)-1-(4-(4-methylthiazol-5-yl)phenyl) ethyl)pyrrolidine-2-carboxamide

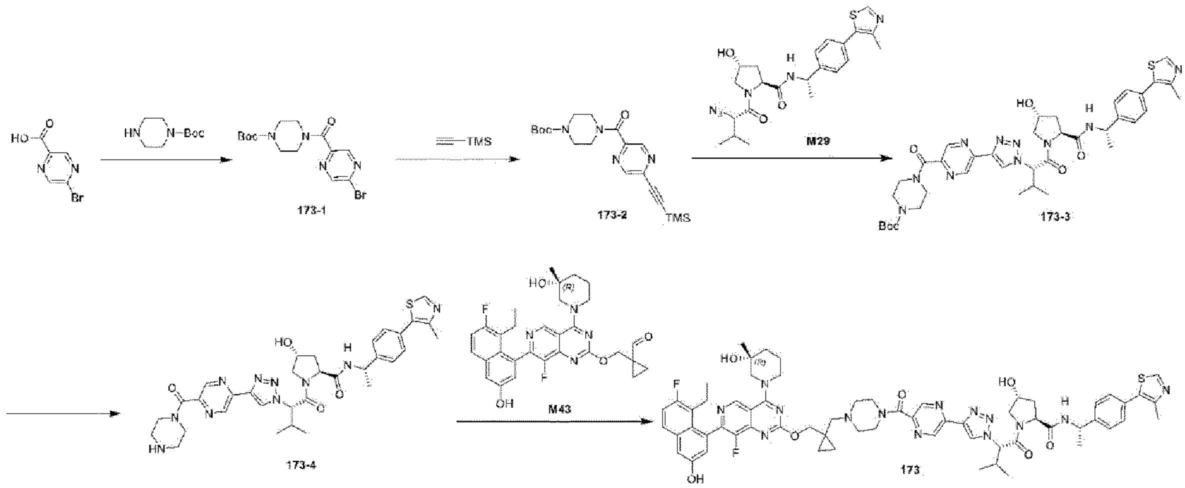


Step 1: Synthesis of compound 173-1

5-Bromo-2-pyrazine carboxylic acid (2.50 g), 1-tert-butoxycarbonylpiperazine (2.75 g), HATU (5.62 g), and DIPEA (6.44 mL) were weighed and dissolved in DMF (20 mL). The mixture was reacted at room temperature for 1 hour. The reaction was monitored by LC-MS until completion. The reaction was quenched by adding saturated ammonium chloride solution, and the reaction was extracted with EA, and washed with water. The organic phase was concentrated and the resultant was purified by column chromatography to obtain 3.62 g of a white solid, i.e., compound 173-1. ESI-MS m/z: 371.1 $[M+H]^+$.

Step 2: Synthesis of compound 173-2

Compound 173-1 (2.0 g) was weighed and dissolved in THF (30 mL). Then, trimethylethynylsilane (1.52 mL), CuI (204 mg), $PdCl_2(PPh_3)_2$ (378 mg) and DIPEA (2.82 mL) were added. After the protection of nitrogen, the reaction was heated to 60°C for 3 hours. Saturated brine (5 mL) was added to the reaction solution. The mixture was extracted with EA, and the organic phase was dried with anhydrous sodium sulfate. The resultant was separated and purified by column chromatography to obtain 1.03 g of a light yellow solid, i.e., compound 173-2. ESI-MS m/z: 389.2 $[M+H]^+$.

Step 3: Synthesis of compound 173-3

Compound 173-2 (200 mg) and compound M29 (235 mg) were weighed and dissolved in THF (5 mL), then copper sulfate (74 mg), water (5 mL), tert-butanol (5 mL), sodium ascorbate (265 mg) and potassium carbonate (569 mg) were added. The mixture was reacted at room temperature for 12 hours. The reaction was monitored by LC-MS until completion. The reaction solution was extracted with DCM, and the organic phase was concentrated. The resultant was separated and purified by column chromatography to obtain 316 mg of a light yellow solid, i.e., compound 173-3. ESI-MS m/z: 773.4 $[M+H]^+$.

Step 4: Synthesis of compound 173-4

Compound 173-3 (316 mg) was weighed and dissolved in 4 M hydrogen chloride in dioxane (8 mL). Then, DCM (6 mL) and MeOH (2 mL) were added. The mixture was reacted at room temperature for 1 hour. The reaction solution was

directly evaporated to dryness. After 5 mL of saturated sodium bicarbonate solution was added to the reaction solution to adjust the pH of the solution until pH=7 to 8, the reaction solution was extracted with a solution of dichloromethane/isopropanol (10:1). The organic phase was dried with anhydrous sodium sulfate to obtain 290 mg of a yellow solid, i.e., compound 173-4. ESI-MS m/z: 673.3 $[M+H]^+$.

Step 5: Synthesis of compound 173

Compound 173-4 (153 mg) was weighed and dissolved in MeOH (10 mL). Then, M43 (100 mg) and 1.0 M zinc chloride in tetrahydrofuran (0.37 mL) were added. After the reaction was heated to 40°C for 2 hours, $NaBH_3CN$ (34.3 mg) was added, and the mixture was reacted overnight. The reaction was monitored by LC-MS until completion. After the pH of the reaction solution was adjusted to pH=7 to 8, the reaction solution was extracted with a mixed solution of dichloromethane/isopropanol. The organic phase was dried with anhydrous sodium sulfate, and the resultant was separated by Pre-HPLC ($CH_3CN/H_2O$ = 55%:45%) to obtain 86.2 mg of a white solid, i.e., compound 173.

ESI-MS m/z: 603.6 1/2$[M+2H]^+$.

$^1$H NMR (500 MHz, DMSO-$d_6$) δ 9.96 (s, 1H), 9.24 (s, 2H), 8.99 (s, 1H), 8.87 (s, 2H), 8.54 (d, *J* = 7.4 Hz, 1H), 7.79 - 7.74 (m, 1H), 7.48 - 7.31 (m, 6H), 7.05 (s, 1H), 5.48 (d, *J* = 9.9 Hz, 1H), 5.20 (d, *J* = 3.2 Hz, 1H), 4.99 - 4.91 (m, 1H), 4.75 (d, *J* = 8.6 Hz, 1H), 4.44 (t, *J* = 7.9 Hz, 1H), 4.36 (d, *J* = 3.7 Hz, 5H), 4.06 (dd, *J* = 22.9, 13.2 Hz, 1H), 3.77 (dt, *J* = 21.1, 10.5 Hz, 3H), 3.64 (d, *J* = 21.9 Hz, 2H), 3.54 - 3.45 (m, 2H), 2.54 (d, *J* = 29.2 Hz, 4H), 2.39 (s, 3H), 2.20 - 1.96 (m, 3H), 1.82 (s, 1H), 1.76 - 1.62 (m, 3H), 1.40 (d, *J* = 6.8 Hz, 2H), 1.18 (d, *J* = 11.4 Hz, 2H), 1.11 *(d, J* = 6.1 Hz, 2H), 1.05 (d, *J* = 6.0 Hz, 8H), 0.75 (dd, *J* = 13.7, 6.8 Hz, 5H), 0.68 (s, 2H), 0.44 (s, 2H).

Example 174 Synthesis of compound (2S,4R)-1-((S)-2-(4-(5-(2-(4-((1-((7-(8-ethyl-7-fluoro-3-hydroxynaphthalen-1-yl)-8-fluoro-4-((R )-3-hydroxy-3-methylpiperidin-1-yl)pyrido[4,3-d]pyrimidin-2-yl)oxy)methyl)cyclopropyl)methy l)piperazin-1-yl)-2-oxyethyl)pyrazin-2-yl)-1H-1,2,3-triazol-1-yl)-3-methylbutanoyl)-4-hydroxy-N-((S)-1-(4-(4-methylthiazol-5-yl)phenyl)ethyl)pyrrolidine-2-carboxamide

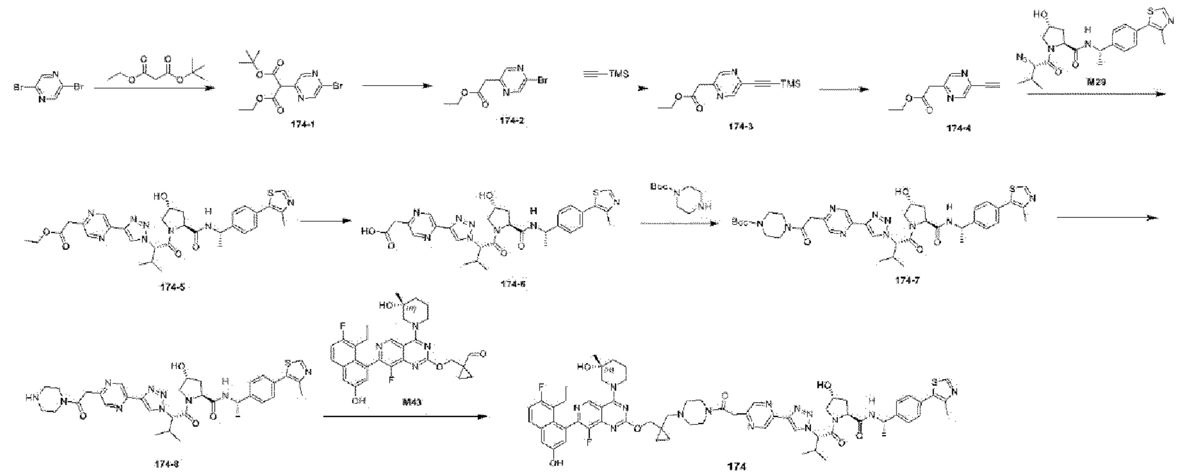


Step 1: Synthesis of compound 174-1

Tert-butyl ethyl malonate (4.35 g) was weighed and dissolved in THF (30 mL), and NaH (1261 mg) was slowly added under ice-bath. The reaction was elevated to room temperature for 0.5 hours. Then, 2,5-dibromopyrazine (5.00 g) was added, and the reaction was slowly heated to 80°C for 2 hours. The reaction was monitored by LC-MS until completion. The reaction was quenched by adding saturated ammonium chloride solution under ice bath, and EA (50 mL) was added to the reaction solution. The organic phase was collected and concentrated. The resultant was separated and purified by column chromatography to obtain 5.60 g of white powder, i.e., compound 174-1. ESI-MS m/z: 345.0 $[M+H]^+$.

Step 2: Synthesis of compound 174-2

Compound 174-1 (5.60 g) was weighed and dissolved in DCM (20 mL), and trifluoroacetic acid (20 mL) was added under ice bath. The reaction was elevated to room temperature for 2 hours. The reaction was monitored by LC-MS

until completion. The reaction system was concentrated, extracted with EA, and washed with water. The organic phase was collected, concentrated, and the resultant was purified by column chromatography to obtain 2.41 g of a light yellow oil, i.e., compound 174-2. ESI-MS m/z: 245.0 $[M+H]^+$.

Step 3: Synthesis of compound 174-3

Compound 174-2 (800 mg) was weighed and dissolved in THF (10 mL). Then, trimethylethynylsilane (0.92 mL), CuI (124 mg), $PdCl_2(PPh_3)_2$ (229 mg) and DIPEA (1.71 mL) were added. After the protection of nitrogen, the reaction was heated to 60°C for 3 hours. Saturated brine (5 mL) was added to the reaction solution. The mixture was extracted with EA, and the organic phase was dried with anhydrous sodium sulfate. The resultant was separated and purified by column chromatography to obtain 560 mg of a light yellow solid, i.e., compound 174-3. ESI-MS m/z: 263.1 $[M+H]^+$.

Step 4: Synthesis of compound 174-4

Compound 174-3 (415 mg) was weighed and dissolved in MeOH (10 mL), and then potassium carbonate (820 mg) was added. The mixture was reacted at room temperature for 1 hour. The insoluble substance was removed by filtering with diatomaceous earth, and the filtrate was directly evaporated to dryness. The resultant was separated and purified by column chromatography to obtain 340 mg of a light yellow solid, i.e., compound 174-4.

Step 5: Synthesis of compound 174-5

Compound 174-4 (340 mg) and compound M29 (642 mg) were weighed, and dissolved in THF (5 mL), copper sulfate (225 mg), water (5 mL), tert-butanol (5 mL), and sodium ascorbate (805 mg) were added. The mixture was reacted at room temperature for 1 hour. The reaction solution was directly evaporated to dryness. The resultant was separated and purified by column chromatography to obtain 646 mg of a light yellow solid, i.e., compound 174-5. ESI-MS m/z: 647.3 $[M+H]^+$.

Step 6: Synthesis of compound 174-6

Compound 174-5 (646 mg) and lithium hydroxide monohydrate (208 mg) were weighed and dissolved in MeOH (9 mL) and $H_2O$ (3 mL). The mixture was reacted at room temperature for 2 hours. The reaction was monitored by LC-MS until completion. The pH of the solution was adjusted to 3 with dilute hydrochloric acid, and the reaction solution was extracted with DCM, washed with water and concentrated to obtain 612 mg of a light yellow solid, i.e., compound 174-6. ESI-MS m/z: 619.3 $[M+H]^+$.

Step 7: Synthesis of compound 174-7

Compound 174-6 (511 mg), 1-tert-butoxycarbonylpiperazine (184 mg), HATU (471 mg), and DIPEA (0.430 mL) were weighed and dissolved in DMF (10 mL). The mixture was reacted at room temperature for 1 hour. The reaction was monitored by LC-MS until completion. The reaction was quenched by adding saturated ammonium chloride solution, and the reaction was extracted with EA, and washed with water. The organic phase was concentrated and the resultant was purified by column chromatography to obtain 333 mg of a light yellow solid, i.e., compound 174-7. ESI-MS m/z: 787.4 $[M+H]^+$.

Step 8: Synthesis of compound 174-8

Compound 174-7 (333 mg) was weighed and dissolved in 4 M hydrogen chloride in dioxane (8 mL). Then, DCM (6 mL) and MeOH (2 mL) were added. The mixture was reacted at room temperature for 1 hour. The reaction solution was directly evaporated to dryness. After 5 mL of saturated sodium bicarbonate solution was added to the reaction solution to adjust the pH of the solution until pH=7 to 8, the reaction solution was extracted with a solution of dichloromethane/isopropanol (10:1). The organic phase was dried with anhydrous sodium sulfate to obtain 258 mg of a yellow solid, i.e., compound 174-8. ESI-MS m/z: 687.3 $[M+H]^+$.

Step 9: Synthesis of compound 174

Compound 174-8 (125 mg) was weighed, and dissolved in MeOH (10 mL). Then, M43 (99 mg) and 1.0 M zinc chloride in tetrahydrofuran (0.37 mL) were added. After the reaction was heated to 40°C for 2 hours, $NaBH_3CN$ (34.3 mg) was added, and the mixture was reacted overnight. The reaction was monitored by LC-MS until completion. After the pH of

the reaction solution was adjusted to pH=7 to 8, the reaction solution was extracted with a mixed solution of 10% dichloromethane/isopropanol. The organic phase was dried with anhydrous sodium sulfate, and the resultant was separated by Pre-HPLC ($CH_3CN/H_2O$ = 55%:45%) to obtain 58.6 mg of a white solid, i.e., compound 174.

ESI-MS m/z: 610.8 1/2[M+2H]$^+$.

$^1$H NMR (500 MHz, DMSO-$d_6$) δ 9.93 (s, 1H), 9.22 (s, 1H), 9.14 (t, *J* = 3.1 Hz, 1H), 8.99 (s, 1H), 8.75 (s, 1H), 8.59 (d, *J* = 1.4 Hz, 1H), 8.53 (d, *J* = 7.6 Hz, 1H), 7.76 (dd, *J* = 9.1, 6.0 Hz, 1H), 7.47 - 7.32 (m, 6H), 7.04 (d, *J* = 2.6 Hz, 1H), 5.44 (d, *J* = 10.1 Hz, 1H), 5.19 (d, *J* = 3.8 Hz, 1H), 4.93 (t, *J* = 7.2 Hz, 1H), 4.74 *(d, J* = 8.6 Hz, 1H), 4.42 (t, *J* = 8.1 Hz, 1H), 4.38 - 4.27 (m, 4H), 4.09 - 3.96 (m, 3H), 3.82 - 3.70 (m, 2H), 3.63 (dd, *J* = 23.3, 10.2 Hz, 1H), 3.53 (d, *J* = 12.9 Hz, 2H), 3.42 (d, *J* = 24.6 Hz, 2H), 2.56 (ddd, *J* = 10.1, 9.3, 3.9 Hz, 1H), 2.46 (d, *J* = 4.1 Hz, 4H), 2.36 (dd, *J* = 18.9, 10.4 Hz, 4H), 2.20 - 1.98 (m, 3H), 1.83 - 1.76 (m, 1H), 1.74 - 1.62 (m, 3H), 1.46 (d, *J* = 20.7 Hz, 1H), 1.39 (d, *J* = 7.0 Hz, 3H), 1.18 (dd, *J* = 12.0, 7.3 Hz, 4H), 1.09 (t, *J* = 6.0 Hz, 3H), 0.85 (dd, *J* = 9.2, 4.6 Hz, 1H), 0.78 - 0.70 (m, 6H), 0.66 (d, *J* = 11.8 Hz, 2H), 0.43 (s, 2H).

Example 175 Synthesis of compound (2S,4R)-1-((R)-2-(3-(2-(4-((1-(((7-(8-ethyl-7-fluoro-3-hydroxynaphthalen-1-yl)-8-fluoro-4-((R)-3 -hydroxy-3-methylpiperidin-1-yl)pyrido[4,3-d]pyrimidin-2-yl)oxy)methyl)cyclopropyl)methyl)p iperazin-1-yl)ethoxy)isoxazol-5-yl)-3-methylbutanoyl)-4-hydroxy-N-((S)-1-(4-(4-methylthiazol-5-yl)phenyl)ethyl)pyrrolidine-2-carboxamide

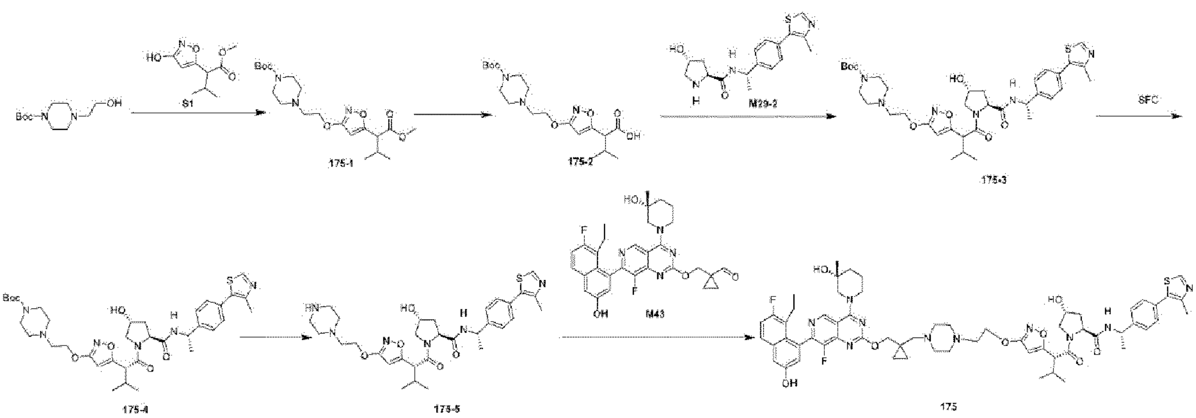


Step 1: Synthesis of compound 175-1

Tert-butyl-4-(2-hydroxyethyl)piperazin-1-carboxylate (1.16 g), compound methyl 2-(3-hydroxyisoxazol-5-yl)-3-methylbutyrate (1.00 g), triphenylphosphine (1.98 g) and DIAD (1.48 mL) were weighed and dissolved in THF (20 mL). The mixture was reacted at 60°C for 4 hours under the protection of nitrogen. The reaction was monitored by LC-MS until completion. The reaction was quenched by adding saturated ammonium chloride solution, and the reaction was extracted with EA, and washed with water. The organic phase was concentrated and the resultant was purified by column chromatography to obtain 1.50 g of a white solid, i.e., compound 175-1. ESI-MS m/z: 412.3 [M+H]$^+$.

Step 2: Synthesis of compound 175-2

Compound 175-1 (1.50 g) was dissolved in MeOH (20 mL) and water (5 mL), and then lithium hydroxide monohydrate (459 mg) was slowly added. The mixture was reacted at room temperature for 2 hours. The reaction was monitored by LC-MS until completion. The reaction was quenched by adding DCM (20 mL) to the reaction solution. The reaction solution was washed once with saturated brine, and the organic phase was dried with anhydrous sodium sulfate, filtered and concentrated to obtain 200 mg of a white solid, i.e., compound 175-2. ESI-MS m/z: 398.2 [M+H]$^+$.

Step 3: Synthesis of compound 175-3

Compound 175-2 (200 mg), compound M29-2 (175 mg) and HATU (229 mg) were weighed and dissolved in DMF (10 mL), and then DIPEA (0.31 mL) was slowly added in an ice bath. The reaction was slowly elevated to room temperature for 1 hour. The reaction was monitored by LC-MS until completion. The reaction was quenched by adding DCM (20 mL) to the reaction solution. The reaction solution was washed once with saturated brine, and the organic phase was dried with anhydrous sodium sulfate, filtered and concentrated. The resultant was separated and purified by column chromatography to obtain 186 mg of a white solid, i.e., compound 175-3. ESI-MS m/z: 711.4 $[M+H]^+$.

Step 4: Synthesis of compound 175-4

Compound 175-3 (186 mg) was resolved by SFC and concentrated to obtain 55 mg of a white solid, i.e., compound 175-4. ESI-MS m/z: 711.4 $[M+H]^+$.

Step 5: Synthesis of compound 175-5

Compound 175-4 (55 mg) was added to DCM (2 mL) and 4M hydrogen chloride in dioxane (2 mL), and the mixture was reacted at room temperature for 10 minutes. The reaction was monitored by LCMS until completion. The reaction solution was concentrated directly, and saturated sodium carbonate solution was added to the reaction solution until pH=8. DCM (20 mL) was added to the reaction solution. The organic phase was collected, washed once with saturated brine, dried with anhydrous sodium sulfate, filtered and concentrated to obtain 47 mg of a white solid, i.e., compound 175-5. ESI-MS m/z: 611.3 $[M+H]^+$.

Step 6: Synthesis of compound 175

Compound 175-5 (47 mg) was weighed, and dissolved in MeOH (10 mL). Then, M43 (47 mg) and 1.0 M zinc chloride in tetrahydrofuran (0.23 mL) were added. After the reaction was heated to 40°C for 2 hours, $NaBH_3CN$ (14.5 mg) was added, and the mixture was reacted overnight. The reaction was monitored by LC-MS until completion. After the pH of the reaction solution was adjusted to pH=7 to 8, the reaction solution was extracted with a mixed solution of 10% dichloromethane/isopropanol. The organic phase was dried with anhydrous sodium sulfate, and the resultant was separated by Pre-HPLC ($CH_3CN/H_2O$ = 55%:45%) to obtain 21.0 mg of a white solid, i.e., compound 175.

ESI-MS m/z: 572.5 1/2$[M+2H]^+$.
$^1H$ NMR (500 MHz, DMSO-$d_6$) δ 9.93 (s, 1H), 9.22 (s, 1H), 8.99 (s, 1H), 8.43 (d, *J* = 7.7 Hz, 1H), 7.77 (dd, *J* = 8.9, 6.1 Hz, 1H), 7.48 - 7.42 (m, 2H), 7.35 (dd, *J* = 16.2, 5.3 Hz, 4H), 7.03 (d, *J* = 2.5 Hz, 1H), 6.08 (s, 1H), 5.11 (d, *J* = 3.7 Hz, 1H), 4.91 (p, *J* = 7.1 Hz, 1H), 4.76 (d, *J* = 6.8 Hz, 1H), 4.40 - 4.25 (m, 5H), 4.21 (t, *J* = 5.4 Hz, 2H), 4.04 (dd, *J* = 26.7, 13.2 Hz, 1H), 3.82 - 3.41 (m, 4H), 2.60 (d, *J* = 17.5 Hz, 2H), 2.46 (s, 4H), 2.38 - 2.10 (m, 8H), 2.06 1.95 (m, 3H), 1.80 - 1.62 (m, 4H), 1.48 - 1.42 (m, 1H), 1.37 (d, *J* = 7.0 Hz, 2H), 1.17 (d, *J* = 11.8 Hz, 4H), 1.04 (d, *J* = 6.1 Hz, 2H), 0.95 (d, *J* = 6.6 Hz, 2H), 0.87 - 0.69 (m, 8H), 0.64 (s, 2H), 0.40 (s, 2H).

Example 176 Synthesis of compound (2S,4R)-1-((S)-2-(4-(6-((1-((1-(((7-(8-ethyl-7-fluoro-3-hydroxynaphthalen-1-yl)-8-fluoro-4-((*R*)-3-hydroxy-3-methylpiperidin-1-yl)pyrido[4,3-*d*]pyrimidin-2-yl)oxy)methyl)cyclopropyl)methyl) -4-methylpiperidin-4-yl)oxy)piperidin-3-yl)-1*H*-1,2,3-triazol-1-yl)-3-methylbutanoyl)-4-hydroxy -*N*-((*S*)-1-(4-(4-methylthiazol-5-yl) phenyl)ethyl)pyrrolidine-2-carboxamide

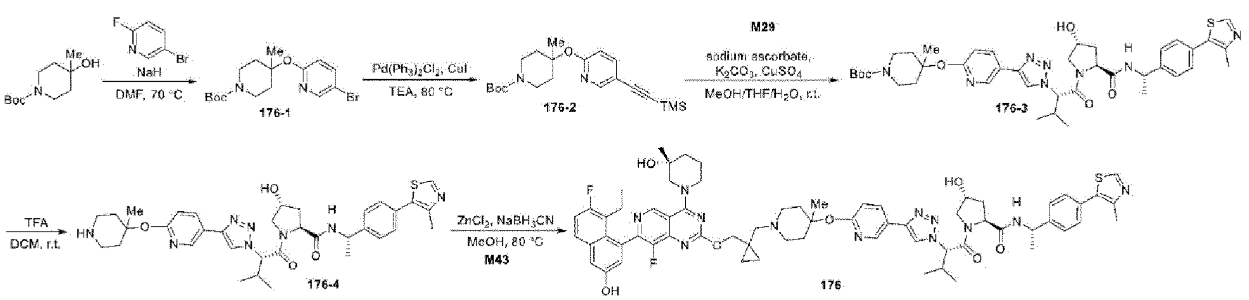


Step 1: Synthesis of compound 176-1

Tert-butyl 4-hydroxy-4-methylpiperidin-1-carboxylate (500.0 mg), 5-bromo-2-fluoropyridine (610.0 mg) and sodium hydride (170.0 mg) were dissolved in DMF (10 mL), the mixture was reacted at 70°C for 2 hours under the protection of nitrogen. The reaction was monitored by LCMS until completion. The reaction solution was directly concentrated, EA (10 mL) was added, and washed once with saturated brine. The organic phase was dried with anhydrous sodium sulfate, filtered and concentrated. The resultant was separated and purified by column chromatography to obtain 480.0 mg of a white solid, i.e., compound 176-1. ESI-MS m/z: 371 $[M+H]^+$.

Step 2: Synthesis of compound 176-2

Compound 176-1 (480.0 mg), bistriphenylphosphine palladium dichloride (90.7 mg) and cuprous iodide (49.4 mg) were dissolved in TEA (25 mL), under the protection of nitrogen, then, trimethylsilylacetylene (0.55 mL) was added, and the mixture was reacted at 50°C overnight. The reaction was monitored by LCMS until completion. The reaction solution was directly concentrated, EA (10 mL) was added, and washed once with saturated brine. The organic phase was dried with anhydrous sodium sulfate, filtered and concentrated. The resultant was separated and purified by column chromatography to obtain 478.0 mg of a brown solid, i.e., compound 176-2. ESI-MS m/z: 192 $[M+H]^+$.

Step 3: Synthesis of compound 176-3

Compound 176-2 (478.0 mg), M29 (2*S*,4*R*)-1-((S)-2-azido-3-methylbutanoyl)-4-hydroxy-*N*-((*S*)-1-(4-(4-methylthiazol-5-yl)phenyl)e thyl)pyrrolidine-2-carboxamide (560.9 mg), potassium carbonate (300.0 mg), copper sulfate (170.2 mg) and sodium ascorbate (640.2 g) were dissolved in a mixed solvent of THF (10 mL)/MeOH (10 mL)/$H_2O$ (10 mL). The mixture was reacted at room temperature for 3 hours under the protection of nitrogen. The reaction was monitored by LCMS until completion. DCM (10 mL) was added to the reaction solution, and the mixture was washed once with saturated brine. The organic phase was dried with anhydrous sodium sulfate, filtered and concentrated. The resultant was separated and purified by column chromatography to obtain 144.0 mg of a light yellow solid, i.e., compound 176-3. ESI-MS m/z: 773 $[M+H]^+$.

Step 4: Synthesis of compound 176-4

Compound 176-3 (100.2 mg) was added to DCM (10 mL) and TFA (1 mL), and the mixture was reacted at room temperature for 20 minutes. The reaction was monitored by LCMS until completion. The reaction solution was directly concentrated, and 1M NaOH solution was added to the reaction solution until pH=10. Then, the reaction solution was extracted by adding DCM (10 mL) and MeOH (1 mL), and washed once with saturated brine. The organic phase was dried with anhydrous sodium sulfate, filtered and concentrated to obtain 45.1 mg of white powder, i.e., compound 176-4. ESI-MS m/z: 673 $[M+H]^+$.

Step 5: Synthesis of compound 176

Compound 176-4 (45.1 mg) and M43 (*R*)-1-(((7-(8-ethyl-7-fluoro-3-hydroxynaphthalen-1-yl)-8-fluoro-4-(3-hydroxy-3-methylpiperidi n-1-yl)pyrido[4,3-d]pyrimidin-2-yl)oxy)methyl)cyclopropane-1-carboxamide (39.7 mg) were dissolved in MeOH (5 mL) and 1M zinc chloride in tetrahydrofuran (0.10 mL) was added. The mixture was reacted at 40°C for 1.5 hours. Sodium cyanoborohydride (13.0 mg) was added to the reaction solution, and the reaction was continued at 60°C overnight. The reaction was monitored by LCMS until completion. Saturated ammonium chloride solution (5 mL) was added to the reaction solution, and the organic phase was concentrated. Then, DCM (10 mL) was added to the reaction solution, and the mixture was washed once with saturated brine. The organic phase was dried with anhydrous sodium sulfate, filtered and concentrated. The resultant was separated by preparative liquid chromatography to obtain 20.1 mg of a white solid, i.e., compound 176.

ESI-MS m/z: 603.8 1/2$[M+2H]^+$.

$^1$H NMR (500 MHz, Methanol-$d_4$) δ 9.21 (d, *J* = 5.0 Hz, 1H), 8.85 (d, *J* = 7.0 Hz, 1H), 8.71 (s, 1H), 8.53 (d, *J* = 12.5 Hz, 1H), 8.18 (s, 1H), 7.70-7.63 (m, 1H), 7.49-7.36 (m, 4H), 7.28 (s, 1H), 7.24 (t, *J* = 9.0 Hz, 1H), 7.09 (s, 1H), 5.41 (d, *J* = 10.0 Hz, 1H), 5.11-5.01 (m, 3H), 4.54-4.40 (m, 5H), 4.25 (t, *J* = 13.5 Hz, 1H), 3.84 (dd, *J* = 27.5, 9.5 Hz, 2H), 3.60-3.55 (m, 2H), 2.91 (s, 2H), 2.63 (d, *J* = 6.5 Hz, 1H), 2.54-2.43 (m, 6H), 2.36 (s, 2H), 2.25-2.12 (m, 3H), 2.09-1.92 (m, 3H), 1.87-1.70 (m, 5H), 1.54 (d, *J* = 7.0 Hz, 3H), 1.45 (s, 3H), 1.38-1.25 (m, 4H), 1.15 (d, *J* = 6.5 Hz, 3H), 0.89-0.79 (m, 5H), 0.73 (s, 2H), 0.52 (s, 2H).

Example 177 Synthesis of compound (2*S*,4*R*)-1-((*S*)-2-(4-(5-((4-((1-(((7-(8-ethyl-7-fluoro-3-hydroxynaphthalen-1-yl)-8-fluoro-4-((*R*)-3-hydroxy-3-methylpiperidin-1-yl)pyrido[4,3-*d*]pyrimidin-2-yl)oxy)methyl)cyclopropyl)methyl) piperazin-1-yl)methyl)piperazin-3-yl)-1*H*-1,2,3-triazol-1-yl)-3-methylbutanoyl)-4-hydroxy-*N*-((*S* )-1-(4-(4-methylthiazol-5-yl)phenyl) ethyl)pyrrolidine-2-carboxamide

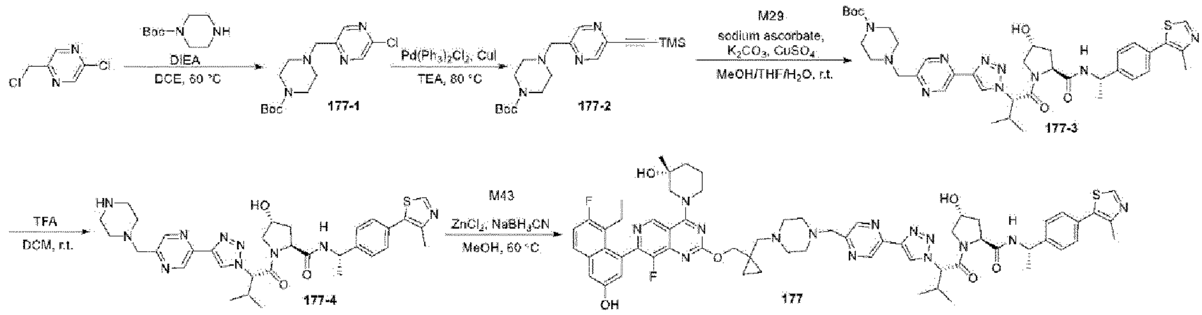


Step 1: Synthesis of compound 177-1

2-Chloro-5-2-chloromethylpyrazine (500.0 mg), N-tert-butoxycarbonylpiperazine (685.6 mg) and DIPEA(1.52 mL) were dissolved in DCM (15 mL). The mixture was reacted at 60°C for 24 hours under the protection of nitrogen. The reaction was monitored by LCMS until completion. The reaction solution was directly concentrated, EA (10 mL) was added, and washed once with saturated brine. The organic phase was dried with anhydrous sodium sulfate, filtered and concentrated. The resultant was separated and purified by column chromatography to obtain 841.3 mg of a white solid, i.e., compound 177-1. ESI-MS m/z: 313 $[M+H]^+$.

Step 2: Synthesis of compound 177-2

Compound 177-1 (841.3 mg), bis (triphenylphosphine)palladium dichloride (188.7 mg) and cuprous iodide (102.4 mg) were dissolved in TEA (25 mL), under the protection of nitrogen, and trimethylsilylacetylene (1.14 mL) was added. The mixture was reacted at 50°C overnight. The reaction was monitored by LCMS until completion. The reaction solution was directly concentrated, EA (10 mL) was added, and washed once with saturated brine. The organic phase was dried with anhydrous sodium sulfate, filtered and concentrated. The resultant was separated and purified by column chromatography to obtain 602 mg of a brown solid, i.e., compound 177-2. ESI-MS m/z: 375 $[M+H]^+$.

Step 3: Synthesis of compound 177-3

Compound 177-2 (60.0 mg), M29 (2*S*,4*R*)-1-((S)-2-azido-3-methylbutanoyl)-4-hydroxy-*N*-((*S*)-1-(4-(4-methylthiazol-5-yl)phenyl)e thyl)pyrrolidine-2-carboxamide (302.9 mg), potassium carbonate (400.6 mg), copper sulfate (95.2 mg) and sodium ascorbate (340.7 mg) were dissolved in a mixed solvent of THF (5 mL)/MeOH (5 mL)/$H_2O$ (5 mL). The mixture was reacted at room temperature for 3 hours under the protection of nitrogen. The reaction was monitored by LCMS until completion. DCM (10 mL) was added to the reaction solution, and the mixture was washed once with saturated brine. The organic phase was dried with anhydrous sodium sulfate, filtered and concentrated. The resultant was separated and purified by column chromatography to obtain 220.2 mg of a light yellow solid, i.e., compound 177-3. ESI-MS m/z: 380 $[M+H]^+$.

Step 4: Synthesis of compound 177-4

Compound 177-3 (100.2 mg) was added to DCM (10 mL) and TFA (1 mL), and the mixture was reacted at room temperature for 20 minutes. The reaction was monitored by LCMS until completion. The reaction solution was directly concentrated, and 1M NaOH solution was added to the reaction solution until pH=10. Then, the reaction solution was extracted by adding DCM (10 mL) and MeOH (1 mL), and washed once with saturated brine. The organic phase was dried with anhydrous sodium sulfate, filtered and concentrated to obtain 171.1 mg of white powder, i.e., compound 177-4. ESI-MS m/z: 330 $[M+H]^+$.

Step 5: Synthesis of compound 177

Compound 177-4 (171.1 mg) and M43 (*R*)-1-(((7-(8-ethyl-7-fluoro-3-hydroxynaphthalen-1-yl)-8-fluoro-4-(3-hydroxy-3-methylpiperidi n-1-yl)pyrido[4,3-d]pyrimidin-2-yl)oxy)methyl)cyclopropane-1-carboxamide (154.5 mg) were dissolved in MeOH (5 mL) and 1 M zinc chloride in tetrahydrofuran (0.30 mL) was added. The mixture was reacted at 40°C for 1.5 hours. Sodium cyanoborohydride (50.0 mg) was added to the reaction solution, and the reaction was continued at 60°C overnight. The reaction was monitored by LCMS until completion. Saturated ammonium chloride solution (5 mL) was added to the reaction solution, and the organic phase was concentrated. Then, DCM (10 mL) was added to the reaction solution, and the mixture was washed once with saturated brine. The organic phase was dried with anhydrous sodium

sulfate, filtered and concentrated. The resultant was separated by preparative liquid chromatography to obtain 42.1 mg of a white solid, i.e., compound 177.

ESI-MS m/z: 596.8 1/2[M+2H]$^+$.

$^1$H NMR (500 MHz, Methanol-$d_4$) δ 9.20 (s, 1H), 9.19 (t, *J* = 1.5 Hz, 1H), 8.87 (s, 1H), 8.71 (d, *J* = 2.0 Hz, 1H), 8.68 (d, *J* = 1.5 Hz, 1H), 7.68-7.65 (m, 1H), 7.59-7.58 (m, 1H), 7.45-7.38 (m, 4H), 7.29 (t, *J* = 2.0 Hz, 1H), 7.26-7.22 (m, 1H), 7.07 (t, *J* = 3.0 Hz, 1H), 5.44 (d, *J* = 10.0 Hz, 1H), 5.07-5.02 (m, 1H), 4.58-4.40 (m, 5H), 4.27-4.19 (m, 2H), 3.93-3.86 (m, 2H), 3.73 (s, 2H), 3.67-3.58 (m, 2H), 3.49-3.44 (m, 1H), 2.66-2.57 (m, 7H), 2.50-2.45 (m, 5H), 2.43-2.39 (m, 1H), 2.22-2.12 (m, 3H), 2.03-1.94 (m, 2H), 1.87-1.85 (m, 1H), 1.80-1.75 (m, 2H), 1.67 (d, *J* = 7.0 Hz, 1H), 1.59 (d, *J* = 7.5 Hz, 1H), 1.53-1.51 (m, 3H), 1.29 (s, 3H), 1.17 (d, *J* = 7.0 Hz, 1H), 0.84-0.80 (m, 6H), 0.70 (s, 2H), 0.49 (s, 2H).

Example 178 Synthesis of compound (2S,4R)-1-((S)-2-(4-(5-((1-((1-((7-(8-ethyl-7-fluoro-3-hydroxynaphthalen-1-yl)-8-fluoro-4-((R)-3-hydroxy-3-methylpiperidin-1-yl)pyrido[4,3-d]pyrimidin-2-yl)oxy)methyl)cyclopropyl)methyl) piperidin-4-yl)oxy)pyrazin-2-yl)-1H-1,2,3-triazol-1-yl)-3-methylbutanoyl)-4-hydroxy-N-((R)-2-hydroxy-1-(4-(4-methylthiazol-5-yl) phenyl)ethyl)pyrrolidine-2-carboxamide

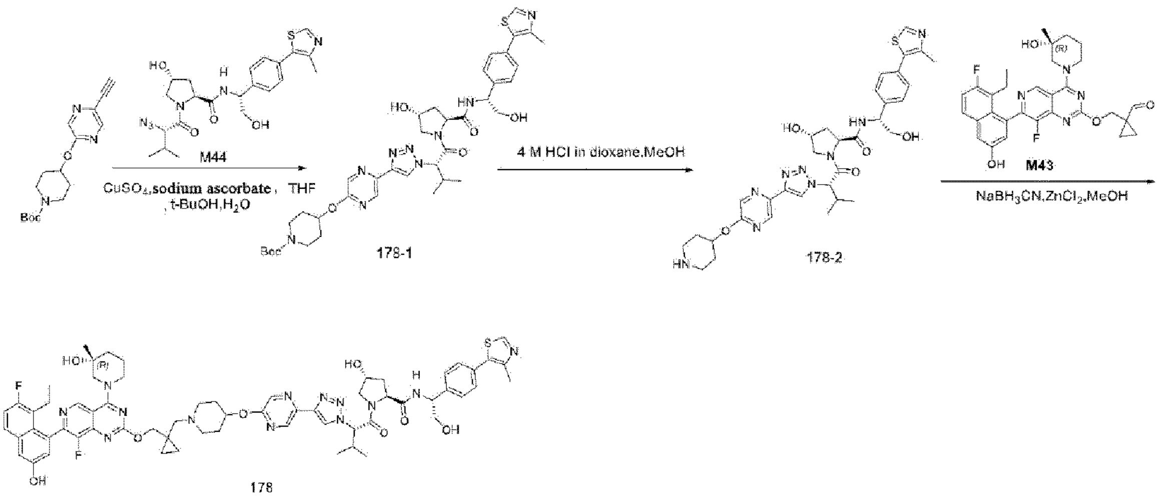


Step 1: Synthesis of compound 178-1

Compound M44 (100.0 mg) was added to a 25 mL single-necked flask and dissolved in THF (2 mL). Then, compound tert-butyl 4-((5-ethynylpyrazin-2-yl)oxy)piperidine-1-carboxylate (70.6 mg), copper sulfate (33.6 mg), water (2 mL), tert-butanol (2 mL), and sodium ascorbate (152.0 mg) were added. The mixture was reacted at room temperature for 1 hour. The reaction solution was directly evaporated to dryness. The resultant was separated and purified by column chromatography (DCM:MeOH = 12:1) to obtain 106.4 mg of a light yellow solid, i.e., compound 178-1.

Step 2: Synthesis of compound 178-2

Compound 178-1 (103.4 mg) was added to a 25 mL single-necked flask and dissolved in 4 M hydrogen chloride in dioxane (5 mL). Then, methanol (1 mL) was added. The mixture was reacted at room temperature for 1 hour. The reaction solution was directly evaporated to dryness. After 5 mL of saturated sodium bicarbonate solution was added to the reaction solution to adjust the pH of the solution until pH=7 to 8, the reaction solution was extracted with a solution of dichloromethane/isopropanol. The organic phase was dried with anhydrous sodium sulfate to obtain 79.3 mg of a yellow solid, i.e., compound 178-2.

Step 3: Synthesis of compound 178

Compound 178-2 (79.3 mg) was added to a 25 mL single-necked flask and dissolved in MeOH (5 mL). Then, M43 (71.8 mg) and 1M zinc chloride in tetrahydrofuran (0.1 mL) were added. After the reaction was heated to 40°C for 2 hours, $NaBH_3CN$ (32.7 mg) was added. The mixture was reacted overnight. The reaction solution was directly evaporated to dryness. The resultant was separated by Pre-HPLC ($CH_3CN/H_2O$ = 55%:45%) to obtain 30.1 mg of a white solid, i.e., compound 178 (purity 98.52%).

LCMS m/z: 605.3 1/2[M+2H]+.
$^1$H NMR (500 MHz, Methanol-$d_4$) δ 9.20 (d, J = 4.9 Hz, 1H), 8.87 (d, J = 6.0 Hz, 1H), 8.73 (s, 1H), 8.54 (s, 1H), 8.18 (s, 1H), 7.70 - 7.63 (m, 1H), 7.44 (d, J = 12.8 Hz, 4H), 7.30 (s, 1H), 7.24 (t, J = 9.3 Hz, 1H), 7.07 (s, 1H), 5.41 (d, J = 10.0 Hz, 1H), 5.06 (dd, J = 18.5, 12.6 Hz, 3H), 4.59 (t, J = 8.1 Hz, 2H), 4.54 - 4.40 (m, 5H), 4.25 (t, J = 14.1 Hz, 1H), 3.99 - 3.81 (m, 4H), 3.57 (ddd, J = 47.8, 27.2, 12.3 Hz, 3H), 2.95 (s, 2H), 2.68 - 2.35 (m, 8H), 2.27 - 1.98 (m, 6H), 1.91 - 1.74 (m, 5H), 1.35 - 1.23 (m, 4H), 1.16 (d, J = 6.4 Hz, 4H), 0.91 - 0.77 (m, 6H), 0.75 (s, 2H), 0.54 (s, 2H).

Example 179 Synthesis of compound (2*S*,4*R*)-1-((*S*)-2-(4-(6-((4-((1-(((7-(8-ethyl-7-fluoro-3-hydroxynaphthalen-1-yl)-8-fluoro-4-((*R*)-3-hydroxy-3-methylpiperidin-1-yl)pyrido[4,3-*d*]pyrimidin-2-yl)oxy)methyl)cyclopropyl)methyl) pyridin-1-yl) methyl)pyridin-3-yl)-1*H*-1,2,3-triazol-1-yl)-3-methylbutanoyl)-4-hydroxy-*N*-((*S*)-1-(4-(4-methylthiazol-5-yl)phenyl)ethyl) pyrrolidine-2-carboxamide

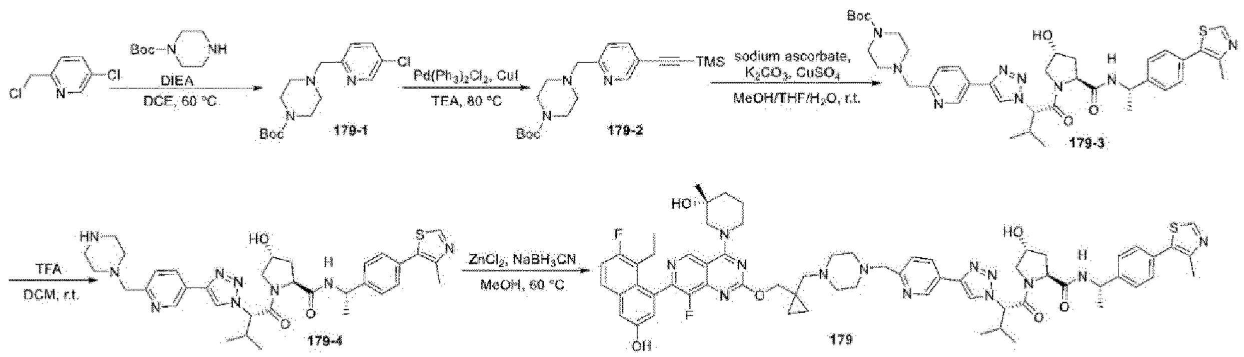


Step 1: Synthesis of compound 179-1

2-Chloro-5-2-chloromethylpyridine (500.0 mg), N-tert-butoxycarbonylpiperazine (685.6 mg) and DIPEA (1.52 mL) were dissolved in DCM (15mL). The mixture was reacted at 60°C for 24 hours under the protection of nitrogen. The reaction was monitored by LCMS until completion. The reaction solution was directly concentrated, EA (10 mL) was added, and washed once with saturated brine. The organic phase was dried with anhydrous sodium sulfate, filtered and concentrated. The resultant was separated and purified by column chromatography to obtain 840.0 mg of a white solid, i.e., compound 179-1. ESI-MS m/z: 312 [M+H]+.

Step 2: Synthesis of compound 179-2

Compound 179-1 (840.0 mg), bis (triphenylphosphine)palladium dichloride (188.7 mg) and cuprous iodide (102.4 mg) were dissolved in TEA (25 mL) under the protection of nitrogen, and trimethylsilylacetylene (1.14 mL) was added. The mixture was reacted at 50°C overnight. The reaction was monitored by LCMS until completion. The reaction solution was directly concentrated, EA (10 mL) was added, and washed once with saturated brine. The organic phase was dried with anhydrous sodium sulfate, filtered and concentrated. The resultant was separated and purified by column chromatography to obtain 600.0 mg of a brown solid, i.e., compound 179-2. ESI-MS m/z: 374 [M+H]+.

Step 3: Synthesis of compound 179-3

Compound 179-2 (60.0 mg), M29 (2*S*,4*R*)-1-((*S*)-2-azido-3-methylbutanoyl)-4-hydroxy-*N*-((*S*)-1-(4-(4-methylthiazol-5-yl)phenyl)e thyl)pyrrolidine-2-carboxamide (302.9 mg), potassium carbonate (400.6 mg), copper sulfate (95.2 mg) and sodium ascorbate (340.7 mg) were dissolved in a mixed solvent of THF (5 mL)/MeOH (5 mL)/$H_2O$ (5 mL). The mixture was reacted at room temperature for 3 hours under the protection of nitrogen. The reaction was monitored by LCMS until completion. DCM (10 mL) was added to the reaction solution, and the mixture was washed once with saturated brine. The organic phase was dried with anhydrous sodium sulfate, filtered and concentrated. The resultant was separated and purified by column chromatography to obtain 200.0 mg of a light yellow solid, i.e., compound 179-3. ESI-MS m/z: 379 [M+H]+.

Step 4: Synthesis of compound 179-4

Compound 179-3 (70.0 mg) was added to DCM (5 mL) and TFA (2 mL), and the mixture was reacted at room temperature for 10 minutes, the reaction was monitored by LCMS until completion. The reaction solution was concentrated directly, and 1 M NaOH was added to the reaction solution until pH=8. DCM (20 mL) was added to the reaction solution. The organic phase was collected, washed once with saturated brine, dried with anhydrous sodium sulfate, filtered and concentrated to obtain 60.0 mg of a white solid, i.e., compound 179-4. ESI-MS m/z: 658.3 $[M+H]^+$.

Step 5: Synthesis of compound 179

Compound 179-4 (60.0 mg) and M43 (*R*)-1-(((7-(8-ethyl-7-fluoro-3-hydroxynaphthalen-1-yl)-8-fluoro-4-(3-hydroxy-3-methylpiperidi n-1-yl)pyrido[4,3-d]pyrimidin-2-yl)oxy)methyl)cyclopropane-1-carboxamide (54.0 mg) were dissolved in MeOH (10 mL) and 1 M zinc chloride in tetrahydrofuran (0.14 mL) was added. The mixture was reacted at 40°C for 1 hour, and then sodium cyanoborohydride (17.2 mg) was added. The mixture was reacted at 60°C for 12 hours, and the reaction was monitored by LCMS until completion. The reaction was quenched by adding saturated sodium bicarbonate solution (5.0 mL), and DCM (20 mL) was added to the reaction solution. The organic phase was collected, washed once with saturated brine, dried with anhydrous sodium sulfate, filtered and concentrated. The resultant was separated by preparative liquid chromatography to obtain 20.7 mg of white powder, i.e., compound 179.

ESI-MS m/z: 595.8 1/2$[M+2H]^+$.

$^1$H NMR (500 MHz, Methanol-$d_4$) δ 9.19 (d, *J* = 1.5 Hz, 1H), 8.98 (t, *J* = 1.5 Hz, 1H), 8.87 (s, 1H), 8.64 (d, *J* = 1.5 Hz, 1H), 8.25-8.23 (m, 1H), 7.68-7.65 (m, 1H), 7.59-7.58 (m, 1H), 7.45-7.37 (m, 4H), 7.30-7.18 (m, 3H), 7.07 (t, *J*= 2.5 Hz, 1H), 5.40 (d, *J* = 10.0 Hz, 1H), 5.06-5.02 (m, 1H), 4.55-4.39 (m, 5H), 4.26-4.20 (m, 2H), 3.93-3.87 (m, 2H), 3.68-3.58 (m, 4H), 3.45-3.44 (m, 1H), 2.68-2.53 (m, 7H), 2.53-2.45 (m, 5H), 2.41-2.38 (m, 1H), 2.22-2.13 (m, 3H), 2.03-1.94 (m, 2H), 1.87-1.84 (m, 1H), 1.79-1.75 (m, 2H), 1.66 (d, *J* = 7.0 Hz, 1H), 1.59 *(d, J* = 7.5 Hz, 1H), 1.53-1.51 (m, 3H), 1.29 (s, 3H), 1.17 (d, *J* = 6.5 Hz, 1H), 0.84-0.80 (m, 6H), 0.70 (s, 2H), 0.49 (s, 2H).

Example 180 Synthesis of compound (2S,4R)-1-((S)-2-(4-(3-((1-((1-((7-(8-ethyl-7-fluoro-3-hydroxynaphthalen-1-yl)-8-fluoro-4-((R)-3-hydroxy-3-methylpiperidin-1-yl)pyrido[4,3-d]pyrimidin-2-yl)oxy)methyl)cyclopropyl)methyl) piperidin-4-yl)methyl)amino)bicyclo[1.1.1]pent-1-yl)-1H-1,3-triazol-1-yl)-3-methylbutanoyl)-4-hydroxy-N-((S)-1-(4-(4-methylthiazol-5-yl)phenyl)ethyl)pyrrolidine-2-carboxamide

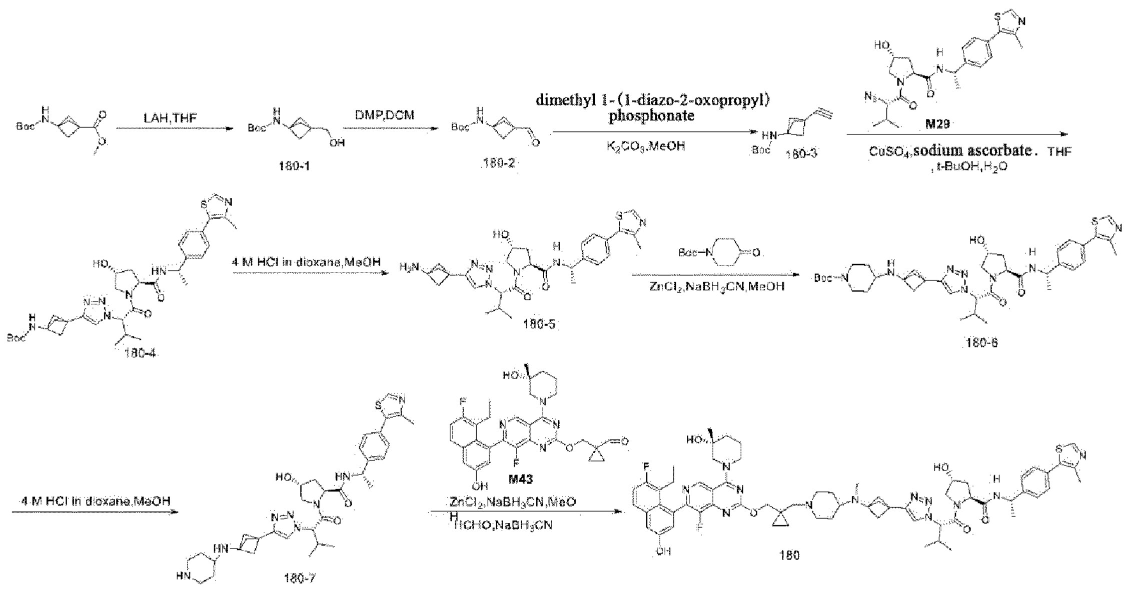


Step 1: Synthesis of compound 180-1

Compound methyl 3-((tert-butoxycarbonyl)amino)bicyclo[1.1.1]pentan-1-carboxylate (1.0g) was added to a 25 mL single-necked flask and dissolved in THF (10 mL). Then, LAH (236.0 mg) was added, and the mixture was reacted at room temperature for 3 hours. Water (0.5 mL), 15% NaOH solution (0.5 mL), and water (1.5 mL) were added to the reaction

solution in batches, and saturated brine (5 mL) was added. The mixture was extracted with EA, and the organic phase was dried with anhydrous sodium sulfate. The resultant was separated by column chromatography (PE: EA = 5:1) to obtain 786.8 mg of a light yellow solid, i.e., compound 180-1.

Step 2: Synthesis of compound 180-2

Compound 180-1 (786.8 mg) was added to a 25 mL single-necked flask and dissolved in DCM (10 mL). Then, DMP (1.88 g) was added, and the mixture was reacted at room temperature for 3 hours. Saturated brine (5 mL) was added to the reaction solution. The mixture was extracted with DCM, and the organic phase was dried with anhydrous sodium sulfate. The resultant was separated by column chromatography (PE:EA = 6:1) to obtain 538.2 mg of a light yellow solid, i.e., compound 180-2.

Step 3: Synthesis of compound 180-3

Compound 180-2 (538.2 mg) was added to a 25 mL single-necked flask, and dissolved in MeOH (10 mL). Then, $K_2CO_3$ (705.1 mg) and dimethyl (1-diazo-2-oxopropyl)phosphonate (734.6 mg) were added. The mixture was reacted at room temperature for 3 hours. The reaction solution was directly evaporated to dryness. The resultant was separated and purified by column chromatography (PE: EA = 47:1) to obtain 394.2 mg of a light yellow solid, i.e., compound 180-3.

Step 4: Synthesis of compound 180-4

Compound M29 (100.0 mg) was added to a 25 mL single-necked flask and dissolved in THF (2 mL). Then, compound 180-3 (54.5 mg), copper sulfate (35.2 mg), water (2 mL), tert-butanol (2 mL), and sodium ascorbate (160.0 mg) were added. The mixture was reacted at room temperature for 0.5 hours. The reaction solution was directly evaporated to dryness. The resultant was separated and purified by column chromatography (DCM:MeOH = 12:1) to obtain 114.6 mg of a light yellow solid, i.e., compound 180-4.

Step 5: Synthesis of compound 180-5

Compound 180-4 (114.6 mg) was added to a 25 mL single-necked flask and dissolved in 4 M hydrogen chloride in dioxane (5 mL). Then, methanol (1 mL) was added. The mixture was reacted at room temperature for 1 hour. The reaction solution was directly evaporated to dryness. After 5 mL of saturated sodium bicarbonate solution was added to the reaction solution to adjust the pH of the solution until pH=7 to 8, the reaction solution was extracted with a solution of dichloromethane/isopropanol. The organic phase was dried with anhydrous sodium sulfate to obtain 84.7 mg of a yellow solid, i.e., compound 180-5.

Step 6: Synthesis of compound 180-6

Compound 180-5 (84.7 mg) was added to a 25 mL single-necked flask and dissolved in MeOH (5 mL). Then, N-tert-butoxycarbonyl-4-piperidone (33.1 mg) and 1 M zinc chloride in tetrahydrofuran (0.1 mL) were added. After the reaction was heated to 40°C for 2 hours, $NaBH_3CN$ (24.1 mg) was added. The mixture was reacted overnight. The reaction solution was directly evaporated to dryness. The resultant was separated and purified by column chromatography (DCM:MeOH = 10:1) to obtain 98.4 mg of a light yellow solid, i.e., compound 180-6.

Step 7: Synthesis of compound 180-7

Compound 180-6 (98.4 mg) was added to a 25 mL single-necked flask, and dissolved in 4 M hydrogen chloride in dioxane (5 mL). Then, methanol (1 mL) was added. The mixture was reacted at room temperature for 1 hour. The reaction solution was directly evaporated to dryness. After 5 mL of saturated sodium bicarbonate solution was added to the reaction solution to adjust the pH of the solution until pH=7 to 8, the reaction solution was extracted with a solution of dichloromethane/isopropanol. The organic phase was dried with anhydrous sodium sulfate to obtain 69.5 mg of a yellow solid, i.e., compound 180-7.

Step 8: Synthesis of compound 180

Compound 180-7 (69.5 mg) was added to a 25 mL single-necked flask, and dissolved in MeOH (5 mL). Then, M43 (64.2 mg) and 1M zinc chloride in tetrahydrofuran (0.1 mL) were added. After the reaction was heated to 40°C for 2 hours, $NaBH_3CN$ (27.1 mg) was added. The mixture was reacted overnight. 37% Formaldehyde aqueous solution (0.2 mL) and

glacial acetic acid (0.1 mL) were added to the reaction solution. After the mixture was reacted at room temperature for 0.5 hours, $NaBH_3CN$ (27.1 mg) was added. The mixture was reacted at room temperature for 1 hour. The reaction solution was directly evaporated to dryness. The resultant was separated by Pre-HPLC ($CH_3CN/H_2O$ = 55%:45%) to obtain 23.7 mg of a white solid, i.e., compound 180, (purity 97.84%).

LCMS: $1/2[M+2H]^+$= 597.84

$^1$H NMR (500 MHz, Methanol-$d_4$) δ 9.21 (s, 1H), 8.87 (d, J = 2.4 Hz, 1H), 7.93 (s, 1H), 7.67 (dd, J = 8.9, 5.8 Hz, 1H), 7.47 - 7.36 (m, 4H), 7.30 (d, J = 2.6 Hz, 1H), 7.26 - 7.21 (m, 1H), 7.05 (t, J = 2.7 Hz, 1H), 5.27 (d, J = 10.3 Hz, 1H), 5.08 - 4.99 (m, 1H), 4.55 - 4.37 (m, 5H), 4.31 - 4.18 (m, 1H), 3.90 - 3.76 (m, 2H), 3.63 (dd, *J* = 33.3, 13.3 Hz, 1H), 3.51 - 3.41 (m, 1H), 3.34 (s, 4H), 3.17 (dd, J = 11.1,9.6 Hz, 3H), 2.71 (s, 1H), 2.63 - 2.36 (m, 6H), 2.24 (s, 3H), 2.17 - 2.10 (m, 5H), 2.07 - 1.93 (m, 3H), 1.89 - 1.75 (m, 3H), 1.71 - 1.60 (m, 4H), 1.51 (d, J = 7.0 Hz, 3H), 1.36 - 1.24 (m, 5H), 1.15 - 1.05 (m, 3H), 0.81 (dd, J = 16.7, 7.5 Hz, 3H), 0.76 - 0.67 (m, 5H), 0.51 (s, 2H).

Example 181: Synthesis of compound (2S,4R)-1-((S)-2-(4-(2-((1-((1-((7-(8-ethyl-7-fluoro-3-hydroxynaphthalen-1-yl)-8-fluoro-4-((R)-3-hydroxy-3-methylpiperidin-1-yl)pyrido[4,3-d]pyrimidin-2-yl)oxy)methyl)cyclopropyl)methyl) piperidin-4-yl)oxy)thiazol-5-yl)-1H-1,2,3-triazol-1-yl)-3-methylbutanoyl)-4-hydroxy-N-((S)-1-( 4-(4-methylthiazol-5-yl)phenyl)ethyl) pyrrolidine-2-carboxamide

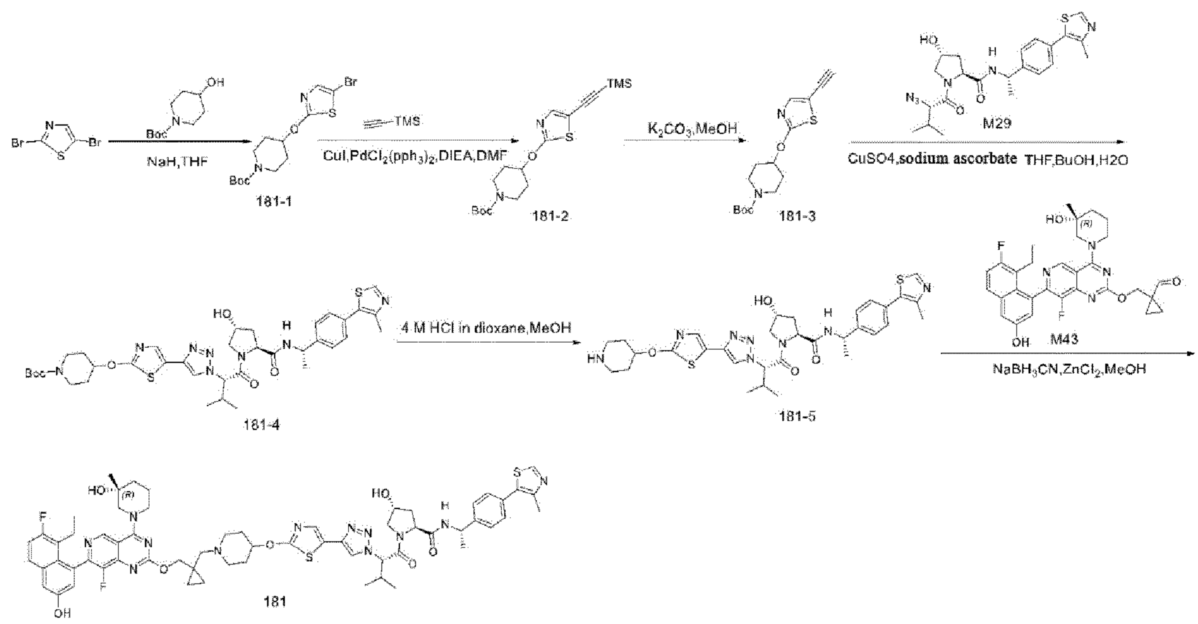


Step 1: Synthesis of compound 181-1

Compound 2,5-dibromothiazole (1.0 g) was added to a 25 mL single-necked flask and dissolved in THF (10 mL). Then, NaH (330.0 mg) and N-tert-butoxycarbonyl-4-hydroxypiperidine (0.91 g) were added. The mixture was heated to 50°C and reacted for 5 h, and the reaction solution was directly evaporated to dryness. The resultant was separated and purified by column chromatography (PE:EA = 8:1) to obtain 0.96 g of a pale yellow solid, i.e. compound 181-1.

Step 2: Synthesis of compound 181-2

Compound 181-1 (848.0 mg) was added to a 25 mL single-necked flask and dissolved in DMF (10 mL). Then, trimethylsilylacetylene (457.8 mg), CuI (82.3 mg), $PdCl_2(PPh_3)_2$ (148.2 mg), and DIPEA (1.1 mL) were added. After nitrogen protection, the mixture was heated to 50°C and reacted for 3 h. Saturated brine (5 mL) was added to the reaction solution and the resultant was extracted with EA. The organic phase was separated and dried with anhydrous sodium sulfate. The resultant was separated by column chromatography (PE:EA = 6:1) to obtain 594.7 mg of a pale yellow solid, i.e. compound 181-2.

Step 3: Synthesis of compound 181-3

Compound 181-2 (594.7 mg) was added to a 25 mL single-necked flask and dissolved in MeOH (10 mL). Then, $K_2CO_3$ (432.2 mg) was added. The mixture was reacted at room temperature for 2 h. The reaction solution was directly evaporated to dryness. The resultant was separated and purified by column chromatography (PE:EA = 4:1) to obtain 390.2 mg of a pale yellow solid, i.e. compound 181-3.

Step 4: Synthesis of compound 181-4

Compound M29 (100.0 mg) was added to a 25 mL single-necked flask and dissolved in THF (2 mL). Then, compound 181-3 (74.3 mg), copper sulfate (35.2 mg), water (2 mL), tert-butanol (2 mL), and sodium ascorbate (160.0 mg) were added. The mixture was reacted at room temperature for 1 h. The reaction solution was directly evaporated to dryness. The resultant was separated and purified by column chromatography (DCM:MeOH = 12:1) to obtain 103.4 mg of apale yellow solid, i.e. compound 181-4.

Step 5: Synthesis of compound 181-5

Compound 181-4 (103.4 mg) was added to a 25 mL single-necked flask and dissolved in 4M hydrogen chloride in dioxane solution (5 mL). Then, methanol (1 mL) was added. The mixture was reacted at room temperature for 1 h. The reaction solution was directly evaporated to dryness. Then, 5 mL of saturated sodium bicarbonate solution was added to the reaction solution. After adjusting the pH of the solution to 7-8, the resultant was extracted with dichloromethane/isopropanol. The organic phase was separated and dried with anhydrous sodium sulfate to obtain 74.6 mg of a yellow solid, i.e. compound 181-5.

Step 6: Synthesis of compound 181

Compound 181-5 (68.2 mg) was added to a 25 mL single-necked flask, and dissolved in MeOH (5 mL). M43 (61.9 mg) and 1 M zinc chloride in tetrahydrofuran solution (0.1 mL) were added. The mixture was heated to 40°C and reacted for 2 h, after that, $NaBH_3CN$ (28.3 mg) was added. The mixture was reacted overnight. The reaction solution was directly evaporated to dryness. The resultant was separated by Pre-HPLC ($CH_3CN/H_2O$ = 55%:45%) to obtain 17.4 mg of awhite solid, i.e. compound 181. (97.81% purity).

LCMS: 1/2[M+2H]$^+$ = 599.8

$^1$H NMR (500 MHz, Methanol-$d_4$) δ 9.20 (d, J = 4.8 Hz, 1H), 8.87 (s, 1H), 8.38 (s, 1H), 7.66 (d, J = 5.9 Hz, 1H), 7.48 - 7.36 (m, 5H), 7.29 (s, 1H), 7.24 (t, J = 9.2 Hz, 1H), 7.07 (s, 1H), 5.35 (d, J = 10.2 Hz, 1H), 5.04 (dd, J = 13.6, 6.7 Hz, 2H), 4.61 (s, 1H), 4.55 - 4.37 (m, 5H), 4.25 (t, J = 14.8 Hz, 1H), 3.87 (dd, J = 25.1, 9.4 Hz, 2H), 3.74 - 3.52 (m, 2H), 3.47 (s, 1H), 3.02 (dd, J = 14.5, 7.3 Hz, 1H), 2.87 (s, 2H), 2.67 - 2.38 (m, 4H), 2.25 - 2.01 (m, 4H), 2.00 -1.83 (m, 3H), 1.52 (d, J = 6.8 Hz, 3H), 1.43 (s, 1H), 1.37 - 1.23 (m, SH), 1.15 (d, J = 6.5 Hz, 3H), 0.87 - 0.76 (m, 6H), 0.73 (s, 2H), 0.52 (s, 2H).

Example 182: Synthesis of compound (2S,4R)-1-((S)-2-(4-(1-((1-((1-(((7-(8-ethyl-7-fluoro-3-hydroxynaphthalen-1-yl)-8-fluoro-4-((R)-3-hydroxy-3-methylpiperidin-1-yl)pyrido[4,3-d]pyrimidin-2-yl)oxy)methyl)cyclopropyl)methyl) piperidin-4-yl)methyl)-1H-pyrazol-4-yl)-1H-1,2,3-triazol-1-yl)-3-methylbutanoyl)-4-hydroxy-N-((S)-1-(4-(4-methylthiazol-5-yl) phenyl)ethyl)pyrrolidine-2-carboxamide

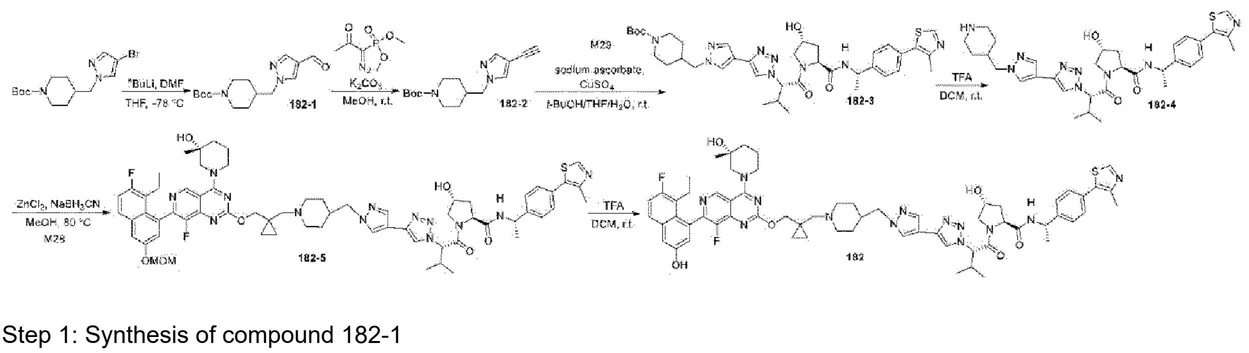


Step 1: Synthesis of compound 182-1

Tert-butyl 4-[(4-bromo-1H-pyrazol-1-yl)methyl]piperidine-1-carboxylate (1.0 g) was dissolved in THF (10 mL)

under nitrogen protection and placed in a low-temperature reactor at -78°C. 2.5 M n-butyllithium in hexane solution (1.74 mL) was added, and the mixture was stirred at -78°C for 1 hour. Then, DMF (0.34 mL) was added, and the resultant was stirred at -78°C for 1 hour. The reaction was monitored by LC-MS until it was completed. The reaction was quenched with saturated sodium bicarbonate solution (5.0 mL), DCM (20 mL) was added to the reaction solution. Then, the organic phase was collected, washed with saturated brine once, dried with anhydrous sodium sulfate, filtered, concentrated, and purified by column chromatography to obtain 450.0 mg of awhite solid, i.e. compound 182-1. ESI-MS m/z: 294.4 $[M+H]^+$.

Step 2: Synthesis of compound 182-2

Compound 182-1 (450.0 mg), dimethyl (1-diazo-2-oxopropyl)phosphonate (884.1 mg) and potassium carbonate (1.27 g) were added to MeOH (30 mL). The mixture was reacted at room temperature for 1 hour. The reaction was monitored by LCMS until it was completed. The reaction solution was filtered, and the organic phase was directly concentrated. The resultant was purified by column chromatography to obtain 373.0 mg of a white solid, i.e. compound 182-2. ESI-MS m/z: 290.4 $[M+H]^+$.

Step 3: Synthesis of compound 182-3

M29(2S,4R)-1-((S)-2-azido-3-methylbutanoyl)-4-hydroxy-N-((S)-1-(4-(4-methylthiazol-5-y l)phenyl)ethyl)pyrrolidine-2-carboxamide (131.5 mg), compound 182-2 (100.0 mg), anhydrous copper sulfate (49.7 mg), and sodium ascorbate (178.1 mg) were dissolved in a mixed solvent of THF/tert-butanol/water (3/3/3 mL). The mixture was reacted at room temperature for 3 hours. The reaction was monitored by LCMS until it was completed. DCM (10 mL) was added to the reaction solution, and the resultant was washed with saturated brine once. The organic phase was dried with anhydrous sodium sulfate, filtered, concentrated, and purified by column chromatography to obtain 250.0 mg of ayellow solid, i.e. compound 182-3. ESI-MS m/z: 746.9 $[M+H]^+$.

Step 4: Synthesis of compound 182-4

Compound 182-3 (250.0 mg) was added to DCM (5 mL) and TFA (2 mL), and the mixture was reacted at room temperature for 10 minutes. The reaction was monitored by LCMS until it was completed. The reaction solution was concentrated directly and 1 M NaOH solution was added until the reaction solution was pH = 8. DCM (20 mL) was added to the reaction solution. Then, the organic phase was collected, washed with saturated brine once, dried with anhydrous sodium sulfate, filtered, and concentrated to obtain 153.1 mg of a white solid, i.e. compound 182-4. ESI-MS m/z: 646.9 $[M+H]^+$.

Step 5: Synthesis of compound 182-5

Compound 182-4 (153.1 mg) and M28 (R)-1-(((7-(8-ethyl-7-fluoro-3-(methoxymethoxy)naphthalen-1-yl)-8-fluoro-4-(3-hydroxy-3-met hylpiperidin-1-yl)pyrido[4,3-d]pyrimidin-2-yl)oxy)methyl)cyclopropane-1-carbaldehyde (168.5 mg) were dissolved in methanol (8 mL). 1M zinc chloride in diethyl ether solution (0.36 mL) was added, and the mixture was reacted at 40°C for 1 hour. Then, sodium cyanoborohydride (44.7 mg) was added, and the mixture was reacted at 60°C for 12 hours. The reaction was monitored by LCMS until it was completed. The reaction was quenched with saturated sodium bicarbonate solution (5.0 mL). DCM (20 mL) was added to the reaction solution, and the organic phase was collected, washed with saturated brine once, dried with anhydrous sodium sulfate, filtered, and concentrated to obtain 171.0 mg of awhite solid, i.e. compound 182-5. ESI-MS m/z: 612.8 1/2$[M+2H]^+$.

Step 6: Synthesis of compound 182

Compound 182-5 (20.0 mg) was added to DCM (5 mL) and TFA (2 mL), and the mixture was reacted at room temperature for 10 minutes. The reaction was monitored by LCMS until it was completed. The reaction solution was directly concentrated, and was separated by the preparative liquid chromatography to obtain 7.6 mg of a white powder, i.e. compound 182. ESI-MS m/z: 590.2 1/2$[M+2H]^+$.
$^1$H NMR (500 MHz, Methanol-$d_4$) δ 9.20 (d, $J$ = 8.0 Hz, 1H), 8.87 (s, 1H), 8.03 (d, $J$ = 4.0 Hz, 1H), 7.95 (s, 1H), 7.92 (s, 1H), 7.67-7.64 (m, 1H), 7.44-7.39 (m, 4H), 7.29 (d, $J$ = 2.5 Hz, 1H), 7.23 (t, $J$ = 9.0 Hz, 1H), 7.06 (t, $J$ = 2.5 Hz, 1H), 5.27 (d, $J$ = 10.5 Hz, 1H), 5.03 (q, $J$ = 7.0 Hz, 1H), 4.52-4.39 (m, 5H), 4.24 (t, $J$ = 13.5 Hz, 1H), 3.90-3.87 (m, 1H), 3.66-3.56 (m, 1H), 3.49-3.41 (m, 1H), 3.26 (q, $J$ = 7.5 Hz, 2H), 3.10-3.03 (m, 2H), 2.57-2.53 (m, 1H), 2.47 (s, 1H), 2.41-2.37 (m, 3H), 2.21-2.16 (m, 2H), 1.98-1.91 (m, 2H), 1.86-1.84 (m, 1H), 1.80-1.74 (m, 2H), 1.69-1.66 (m, 2H), 1.51 (d, $J$ = 7.0 Hz, 2H), 1.29-1.27 (m, 6H), 1.12 (d, $J$ = 6.5 Hz, 3H), 0.91-0.86 (m, 2H), 0.83-0.79 (m, 3H), 0.75-0.74 (m, 3H), 0.70 (s, 2H), 0.49 (s, 1H).

Example 183: Synthesis of compound (2S,4R)-1-((S)-2-(4-(5-(3-(4-((1-((7-(8-ethyl-7-fluoro-3-hydroxynaphthalen-1-yl)-8-fluoro-4-((R )-3-hydroxy-3-methylpiperidin-1-yl)pyrido[4,3-d]pyrimidin-2-yl)oxy)methyl)cyclopropyl)methy l)piperazin-1-yl)azetidin-1-yl)pyrazin-2-yl)-1H-1,2,3-trizol-1-yl)-3-methylbutanoyl)-4-hydroxy -N-((S)-1-(4-(4-methylthiazol-5-yl) phenyl)ethyl)pyrrolidine-2-carboxamide

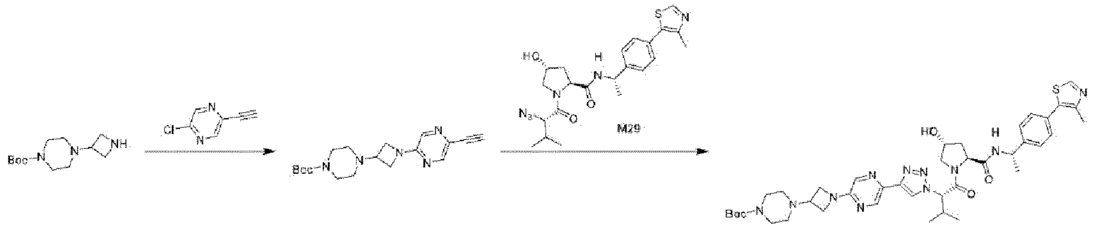


183-1 183-2

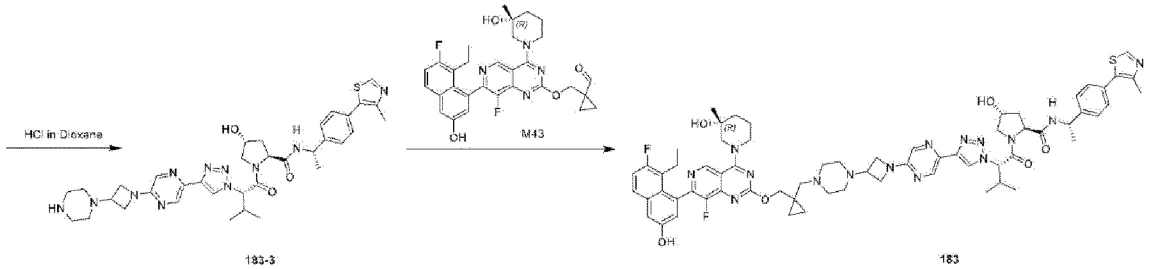


183-3 183

Step 1: Synthesis of compound 183-1

Tert-butyl 4-(azetidin-3-yl)piperazine-1-carboxylate (200 mg) was dissolved in 5.00 mL of 1,4-dioxane. TEA (0.576 mL) and 2-chloro-5-ethynylpyrazine (0.138 mg) were added successively. The mixture was reacted at 80°C for 3 h. The reaction solution was cooled to room temperature. The solvent was removed under reduced pressure and the resultant was purified by column chromatography to obtain 180 mg of a colorless oil, i.e. compound 183-1, ESI-MS m/z: 344 $[M+H]^+$;

Step 2: Synthesis of compound 183-2

Compound 183-1 (180 mg), M29 (230 mg), sodium ascorbate (216 mg), and anhydrous copper sulfate (60.2 mg) were dissolved in a mixed solvent of 2.00 mL of tert-butanol, 2.00 mL of tetrahydrofuran, and 2.00 mL of water at room temperature. The mixture was reacted at room temperature for 1 h. The reaction solution was quenched by adding 50 mL of water. The resultant was extracted with a mixed solvent of DCM/MeOH = 10/1 three times. The organic phases were combined, and dried with anhydrous sodium sulfate. The solvent was removed by under reduced pressure and the resultant was purified by column chromatography (DCM/MeOH = 10/1) to obtain 300 mg of a yellow solid product, i.e. compound 183-2. ESI-MS m/z: 800 $[M+H]^+$;

Step 3: Synthesis of compound 183-3

Compound 183-2 (300 mg) was dissolved in 5.00 mL of dichloromethane, and then 4 M hydrogen chloride in dioxane solution (2.00 mL) was added at room temperature. The mixture was reacted at room temperature for 0.5 h. The solvent was removed under reduced pressure. The resultant was redissolved in dichloromethane, and the solvent was removed again. The resultant was redissolved in 5.00 mL of tetrahydrofuran and then 1.00 mL of water was added. Solid sodium carbonate was added and the mixture was stirred for 20 min. Then anhydrous sodium sulfate was added, the mixture was dried and filtered. The solvent was removed under reduced pressure, and the resultant was purified by column chromatography (DCM/MeOH=10/1) to obtain 208 mg of ayellow solid product, i.e. compound 183-3. ESI-MS m/z: 700 $[M+H]^+$;

Step 4: Synthesis of compound 183

M43 (72.4 mg) and compound 183-3 (77.0) were dissolved in anhydrous methanol (3.00 mL) at room

temperature. 1 M zinc chloride in tetrahydrofuran solution (0.220 mL) was added. The mixture was reacted at 55°C for 1.0 h. Then, sodium cyanoborohydride (34.6 mg) was added and the mixture was reacted at 70°C for 3.0 h. The reaction solution was quenched by adding an appropriate amount of saturated ammonium chloride aqueous solution, and the mixture was extracted with DCM/MeOH = 10/1 three times, 20 mL each time. The organic phases were combined, and dried with anhydrous sodium sulfate. The solvent was removed under reduced pressure and then the resultant was purified by Pre-HPLC to obtain 52.5 mg of a white solid product, i.e. compound 183.

ESI-MS m/z: 617 1/2[M+2H]$^+$;

$^1$H NMR (500 MHz, Methanol-$d_4$) $\delta$ 9.21 (d, *J* = 6.3 Hz, 1H), 8.87 (d, *J* = 3.4 Hz, 1H), 8.64 (d, *J* = 6.6 Hz, 1H), 8.44 (s, 1H), 7.88 (s, 1H), 7.67 (dd, *J* = 8.8, 5.9 Hz, 1H), 7.47 - 7.37 (m, 4H), 7.30 (d, *J* = 2.6 Hz, 1H), 7.24 (t, *J* = 9.2 Hz, 1H), 7.06 (t, *J* = 2.4 Hz, 1H), 5.36 (d, *J* = 10.2 Hz, 1H), 5.04 (q, *J* = 7.1 Hz, 1H), 4.59 (s, 3H), 4.49 (ddd, *J* = 35.9, 16.3, 9.3 Hz, 4H), 4.24 (dd, *J* = 25.4, 11.2 Hz, 3H), 3.99 (s, 2H), 3.96 - 3.83 (m, 2H), 3.70 - 3.59 (m, 1H), 3.48 (s, 1H), 3.37 (s, 1H), 2.62 (dt, *J* = 10.1, 6.5 Hz, 3H), 2.51 (d, *J* = 13.1 Hz, 3H), 2.47 - 2.39 (m, 3H), 2.20 (dd, *J* = 14.8, 7.2 Hz, 4H), 1.98 (td, *J* = 8.9, 4.5 Hz, 1H), 1.91 - 1.74 (m, 3H), 1.53 (d, *J* = 7.0 Hz, 2H), 1.38 - 1.23 (m, 7H), 1.15 (d, *J* = 6.7 Hz, 3H), 0.90 (t, *J* = 6.8 Hz, 1H), 0.86 - 0.79 (m, 5H), 0.72 (s, 2H), 0.50 (s, 2H).

Example 184: Synthesis of compound (2S,4R)-1-((S)-2-(4-(5-((1-((1-(((7-(8-ethyl-7-fluoro-3-hydroxynaphthalen-1-yl)-8-fluoro-4-((R)-3-hydroxy-3-methylpiperidin-1-yl)pyrido[4,3-d]pyrimidin-2-yl)oxy)methyl)cyclopropyl)methyl) piperidin-4-yl)oxy)pyrazin-2-yl)-1H-1,2,3-triazol-1-yl)-3-methylbutanoyl)-4-hydroxy-N-((1R,2R )-2-hydroxy-3-methyl-1-(4-(4-methylthiazol-5-yl)phenyl)butyl)pyrrolidine-2-carboxamide

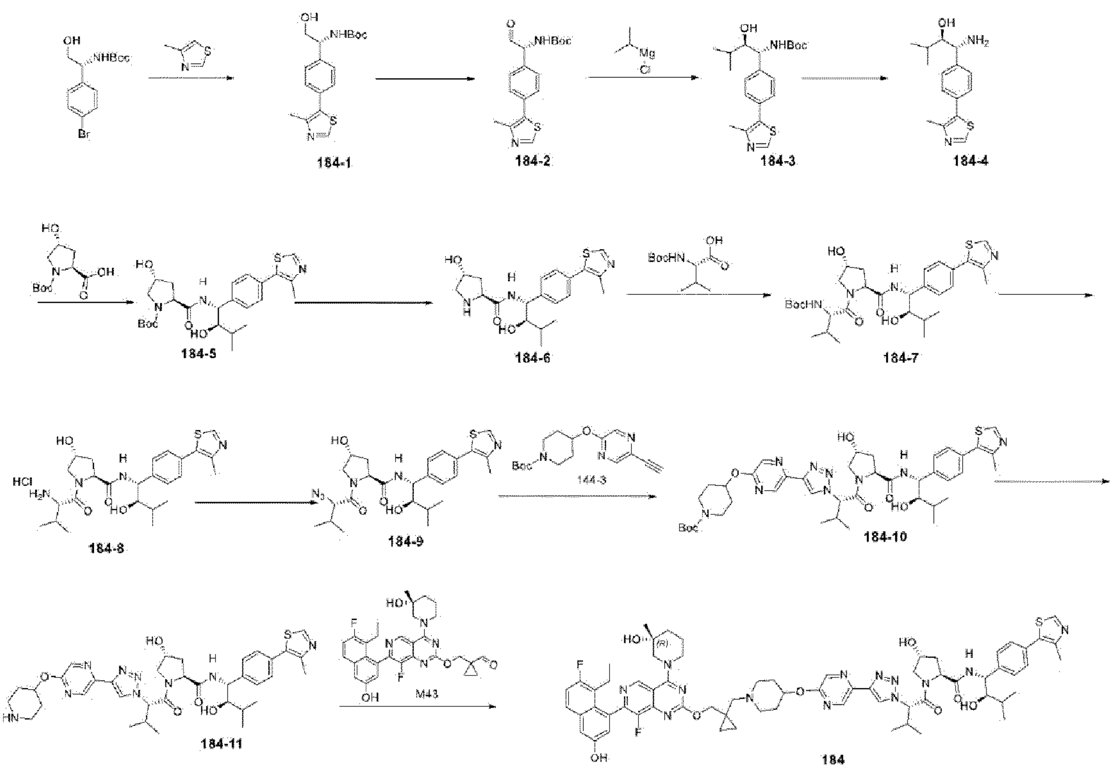


Step 1: Synthesis of compound 184-1

Under nitrogen protection, (R)-tert-butyl (1-(4-bromophenyl)-2-hydroxyethyl)carbamate (4.00 g, 12.65 mmol), 4-methylthiazole (2.51 g, 25.30 mmol), palladium acetate (0.28 g, 1.27 mmol), and potassium acetate (2.48 g, 25.30 mmol) were dissolved in N,N-dimethylformamide (60.00 mL). The mixture was heated to 100°C and reacted for 12 h. The reaction solution was added to 50 mL of water, the resultant was extracted with EA twice, 30 mL each time. The organic phases were combined, washed with saturated brine, dried, filtered, concentrated, and purified by column chromatography (EA:PE = 1% to 60%) to obtain 2.10 g of a colorless solid, i.e. compound 184-1. ESI-MS m/z: 335.1 [M+H]$^+$.

Step 2: Synthesis of compound 184-2

To a solution of compound 184-1 (1.00 g, 2.99 mmol) in tetrahydrofuran (30.00 mL), DMP (1.40 g, 3.29 mmol) was added. The mixture was reacted at 20°C for 1 h. The reaction was monitored by LCMS until it was completed. The resultant was filtered to obtain a solution of compound 184-2 in THF. The resultant was directly used for the next step. ESI-MS m/z: 351.1 $[M+H+18]^+$.

Step 3: Synthesis of compound 184-3

To a solution of compound 184-2 (1000.00 mg, 3.01 mmol) in tetrahydrofuran (33.00 mL) cooled down at 0°C under nitrogen protection, isopropylmagnesium chloride (9.02 mL, 2.00 mol/L, 18.05 mmol) was added. The mixture was reacted at 0°C for 2 h. The reaction solution was added to 100 mL of saturated ammonium chloride, and the resultant was extracted with EA. The organic phase was dried, filtered and concentrated under reduced pressure. The residue was purified by column chromatography to obtain 250.00 mg of a foamy solid, i.e., compound 184-3. ESI-MS m/z: 377.2 $[M+H]^+$.

Step 4: Synthesis of compound 184-4

To a solution of compound 184-3 (474.00 mg, 1.26 mmol) in tetrahydrofuran (4.80 mL), and 4M hydrogen chloride in dioxane solution (1.57 mL) was added. The mixture was reacted at 60°C for 2 h. The reaction was monitored by LCMS until the starting material was reacted completely. The reaction solution was added to 20 mL of saturated sodium bicarbonate solution, and the resultant was extracted with chloroform/isopropanol three times. The organic phases were dried, filtered and concentrated under reduced pressure to obtain 330.00 mg of a yellow liquid, i.e., compound 184-4. ESI-MS m/z: 277.2 $[M+H]^+$.

Step 5: Synthesis of compound 184-5

To a solution of compound 184-4 (330.00 mg, 1.19 mmol) and (2S,4R)-4-hydroxy-1-[(2-methylpropyl-2-yl) oxycarbonyl]pyrrolidine-2-carboxylic acid (303.69 mg, 1.31 mmol) in N,N-dimethylformamide (4.00 mL) cooled down to 0°C in an ice water bath, HATU (499.36 mg, 1.31 mmol) and DIPEA (0.83 mL,4.78 mmol) were added. The mixture was reacted at 0°C for 1 h, and then reacted at 20°C for 2 h. The reaction solution was added to 30 mL of water, and the resultant was extracted with EA three times. The organic phases were dried, filtered and concentrated under reduced pressure. The residue was purified by column chromatography (MeOH:DCM = 1% to 17%) to obtain 250.00 mg of a pale yellow foamy solid, i.e., compound 184-5. ESI-MS m/z: 490.2 $[M+H]^+$.

Step 6: Synthesis of compound 184-6

To a solution of compound 184-5 (250.00 mg, 0.51 mmol) in dichloromethane (1.50 mL) and methanol (1.50 mL) cooled down to 0°C, 4 M hydrogen chloride in dioxane solution (1.28 mL) was added. The mixture was reacted at 0°C for 1 h, and then reacted at 20°C for 1 h. The reaction solution was added to 20 mL of saturated sodium bicarbonate, and the resultant was extracted with chloroform/isopropanol for 8 times. The organic phases were dried, filtered and concentrated under reduced pressure to obtain 175.00 mg of a pale yellow foamy solid, i.e., compound 184-6. ESI-MS m/z: 390.1 $[M+H]^+$.

Step 7: Synthesis of compound 184-7

To a solution of compound 184-6 (180.00 mg, 0.46 mmol), (2S)-3-methyl-2-[(2-methylpropan-2-yl)oxycarbonylamino]butanoic acid (110.43 mg, 0.51 mmol), and HATU (193.27 mg, 0.51 mmol) in DMF (4.00 mL), DIPEA (0.32 mL, 1.85 mmol) was added. The mixture was reacted at 20°C for 2 h. After the starting material was reacted completely, the reaction solution was added to 30 mL of water, the resultant was extracted with EA twice, 15 mL each time. The organic phases were combined, washed with saturated brine, dried, filtered, and concentrated under reduced pressure. The residue was purified by column chromatography (MeOH:DCM = 1:13) to obtain 195.00 mg of a pale yellow foamy solid, i.e. compound 184-7. ESI-MS m/z: 589.2 $[M+H]^+$.

Step 8: Synthesis of compound 184-8

To a solution of compound 184-7 (190.00 mg, 0.32 mmol) in methanol (2.00 mL) and dichloromethane (2.00 mL), 4 M hydrogen chloride in dioxane solution (0.81 mL) was added at 0°C. The mixture was reacted at 20°C for 1 h. The

reaction solution was directly concentrated under reduced pressure without heating. 130.00 mg of a white solid was obtained, i.e. compound 184-8. The product was directly used for the next step. ESI-MS m/z: 489.2 $[M+H]^+$.

Step 9: Synthesis of compound 184-9

To a solution of compound 184-8 (150.00 mg, 0.31 mmol) and triethylamine (0.17 mL, 1.23 mmol) in a mixed solvent of tetrahydrofuran (1.50 mL) and acetonitrile (1.50 mL) in an ice water bath under nitrogen protection, a solution of 2-azido-1,3-dimethylimidazolinium hexafluorophosphate (175.07 mg, 0.61 mmol) in acetonitrile (0.75 mL) was added dropwise, and then the mixture was reacted at 0°C for 2 h. The reaction solution was added to 20 mL of water, and the resultant was extracted with DCM. The organic phase was dried, filtered and concentrated under reduced pressure. The residue was purified by column chromatography (MeOH:DCM = 1:16) to obtain 120.00 mg of a brown foamy solid, i.e., compound 184-9. ESI-MS m/z: 515.20 $[M+H]^+$.

Step 10: Synthesis of compound 184-10

To a solution of compound 184-9 (80.00 mg, 0.16 mmol), and compound 144-3 (51.84 mg, 0.17 mmol) in tert-butanol (2.50 mL) and tetrahydrofuran (2.50 mL), copper sulfate (24.80 mg, 0.16 mmol) and water (1.25 mL) containing sodium ascorbate (92.36 mg, 0.47 mmol) were added. The mixture was reacted at 20°C for 1 h. The reaction solution was added to 20 mL of water, and the resultant was extracted with DCM. The organic phase was dried, filtered, concentrated under reduced pressure, and the residue was purified by column chromatography (MeOH: DCM= 1:16) to obtain 80.00 mg of a brown solid, i.e., compound 184-10. ESI-MS m/z: 818.10 $[M+H]^+$.

Step 11: Synthesis of compound 184-11

To a solution of compound 184-10 (70.00 mg, 0.09 mmol) in dichloromethane (2.50 mL), trifluoroacetic acid (0.48 mL, 6.42 mmol) was added at 0°C. The mixture was reacted at 0°C for 1 h. The reaction solution was added to 20 mL of saturated sodium bicarbonate, and the resultant was extracted with chloroform/isopropanol. The organic phase was dried, filtered and concentrated under reduced pressure to obtain 55.00 mg of a colorless oil, i.e., compound 184-11. The product was directly used for the next step. ESI-MS m/z: 718.30 $[M+H]^+$.

Step 12: Synthesis of compound 184

To a solution of compound 184-11 (70.00 mg, 0.10 mmol) and M43 (58.86 mg, 0.11 mmol) in methanol (2.50 mL), 1 M zinc chloride in tetrahydrofuran solution (0.20 mL) was added. The mixture was reacted at 40°C for 1 h. And then sodium cyanoborohydride (24.51 mg, 0.39 mmol) was added and the mixture was reacted at 40°C for 16 h. The reaction solution was added to 20 mL of water, and the resultant was extracted with chloroform/isopropanol. The organic phases were combined, dried, filtered, concentrated under reduced pressure, and the residue was purified by Pre-HPLC to obtain 20.00 mg of a white powder, i.e., compound 184.

ESI-MS m/z: 625.8 1/2$[M+2H]^+$.
$^1$H NMR (500 MHz, Methanol-$d_4$) $\delta$ 9.20 (s, 1H), 8.87 (s, 1H), 8.74 (s, 1H), 8.58 (s, 1H), 8.19 (s, 1H), 7.68-7.65 (m, 1H), 7.47-7.41 (m, 4H), 7.30 (s, 1H), 7.26-7.22 (m, 1H), 7.07 (s, 1H),5.41-5.39 (d, $J$= 10.0 Hz, 1H), 5.10-5.14 (m, 2H), 4.63-4.58 (m, 5H), 4.47 (s, 4H), 4.29-4.22 (m, 1H), 3.95-3.85 (m, 2H), 3.69-3.52 (m, 4H), 2.66-2.63 (m, 1H), 2.48 (s, 6H), 2.22-2.19 (m, 3H), 2.06-1.99 (m, 3H), 1.81-1.77 (m, 6H), 1.30 (m, 3H), 1.28 (m, 3H), 1.18-1.15 (m, 3H), 1.04-1.02 (m, 6H), 0.84-0.79 (m, 6H).

Example 185: Synthesis of compound (2S,4R)-1-((S)-2-(4-(5-((1-((1-((7-(8-ethyl-7-fluoro-3-hydroxynaphthalen-1-yl)-8-fluoro-4-((R)-3-hydroxy-3-methylpiperidin-1-yl)pyrido[4,3-d]pyrimidin-2-yl)oxy)methyl)cyclopropyl)methyl) piperidin-4-yl)oxy)pyrazin-2-yl)-1H-1,2,3-triazol-1-yl)-3-methylbutanoyl)-4-hydroxy-N-((S)-1-( 2-methyl-4-(4-methylthiazol-5-yl) phenyl)ethyl)pyrrolidine-2-carboxamide

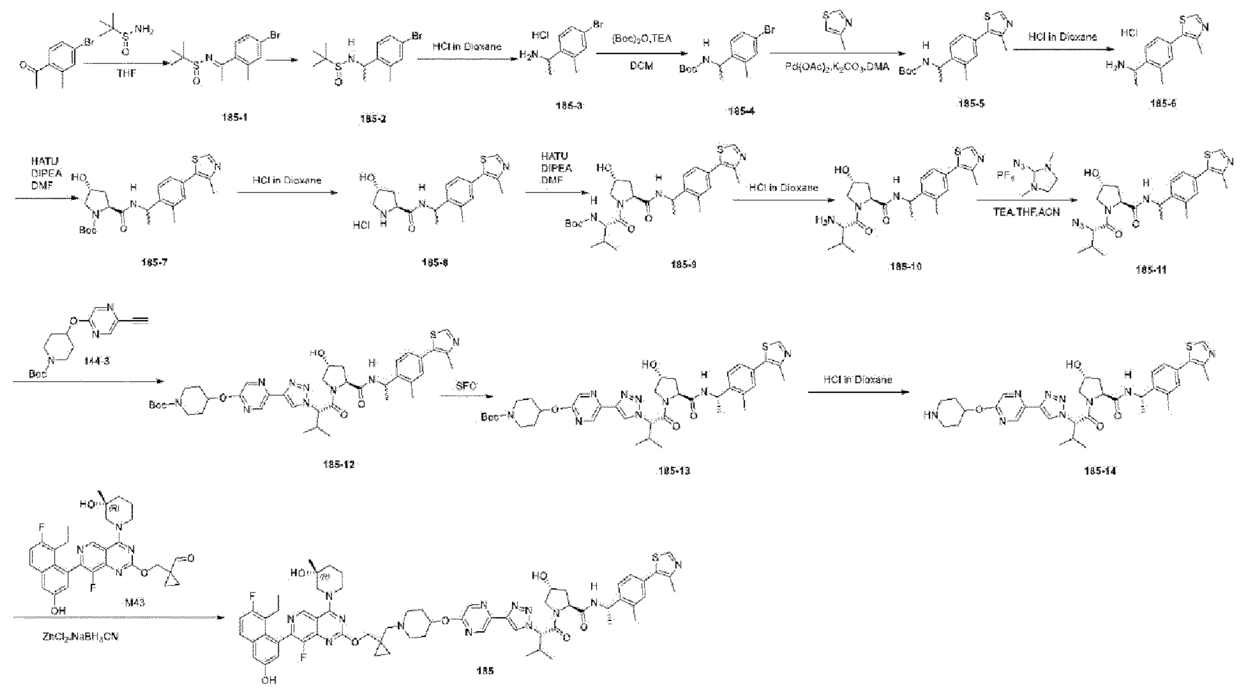


Step 1: Synthesis of compound 185-1

4-Bromo-2-methylacetophenone (3.0 g) and tert-butylsulfenamide (6.9 g) were dissolved in 30.00 mL of anhydrous tetrahydrofuran, and then titanium ethoxide (31.0 mL) was added. The mixture was reacted at 80°C for 3.0 h. The reaction solution was cooled down to room temperature, and then quenched by dropping ice water. The resultant was extracted with ethyl acetate three times. The organic phases were combined, and dried with anhydrous sodium sulfate. The solvent was removed under reduced pressure, and then the resultant was purified by column chromatography (PE/EA = 5:1) to obtain 3.0 of a crude product, i.e. compound 185-1. ESI-MS m/z: 316 [M+H] $^+$;

Step 2: Synthesis of compound 185-2

Compound 185-1 (3.0 g) was dissolved in 30.00 mL of anhydrous tetrahydrofuran, and then lithium triisobutylhydroborate (28.46 mL) was added. The mixture was reacted at room temperature for 2.0 h. The reaction solution was quenched by dropping ice water. The resultant was extracted with ethyl acetate three times. The organic phases were combined, and dried with anhydrous sodium sulfate. The solvent was removed under reduced pressure to obtain 3.0 g of a crude product, i.e. compound 185-2, which was directly used for the next step. ESI-MS m/z: 318 [M+H] $^+$;

Step 3: Synthesis of compound 185-3

Compound 185-2 (3.0 g) was dissolved in 4 M hydrogen chloride in dioxane solution (35.00 mL). The mixture was reacted at room temperature for 2.0 h. The solvent of the reaction solution was removed under reduced pressure. The resultant was slurried with EA and filtered to obtain 2.0 g of a white product, i.e. compound 185-3. ESI-MS m/z: 214 [M+H] $^+$;

Step 4: Synthesis of compound 185-4

Compound 185-3 (2.0 g) was dissolved in 20.0 mL of anhydrous dichloromethane, and then triethylamine (5.57 mL) and di-tert-butyl dicarbonate (2.76 mL) were added successively. The mixture was reacted at room temperature for 1.0 h. The solvent of the reaction solution was removed under reduced pressure. The resultant was purified by column chromatography to obtain 2.0 g of a white-like product, i.e. compound 185-4. ESI-MS m/z: 314 [M+H] $^+$;

Step 5: Synthesis of compound 185-5

Compound 185-4 (2.0 g), 4-methylthiazole (0.95 g), palladium acetate (0.29 g), and potassium carbonate (2.64 g) were dissolved in 20.0 mL of DMA. The mixture was reacted at 120°C for 2.0 h. The reaction solution was cooled down to room temperature, and then was quenched by dropping water. The resultant was extracted with ethyl acetate three times.

The organic phases were combined, and dried with anhydrous sodium sulfate. The solvent was removed under reduced pressure, and then the resultant was purified by column chromatography to obtain 2.0 g of a product, i.e. compound 185-5.

ESI-MS m/z: 333 [M+H] $^+$;

Step 6: Synthesis of compound 185-6

Compound 185-5 (2.0 g) was dissolved in 4 M hydrogen chloride in dioxane solution (10.00 mL). The mixture was reacted at room temperature for 1.0 h. The solvent of the reaction solution was removed under reduced pressure. The resultant was redissolved with methanol and the solvent was removed under reduced pressure again to obtain 1.1 g of a yellow product, i.e., compound 185-6, which was directly used for the next step. ESI-MS m/z: 233 [M+H] $^+$;

Step 7: Synthesis of compound 185-7

Compound 185-6 (1.1 g), (2S,4R)-1-tert-butoxycarbonyl-2-carbamoyl-4-hydroxypyrrolidine (1.31 g), and DIPEA (2.47 mL) were dissolved in 10.00 mL of DMF, and then HATU (1.98 g) was added. The mixture was reacted at room temperature for 1.0 h. Water was added to the reaction solution for quenching, and the resultant was extracted with ethyl acetate three times. The organic phases were combined, and dried with anhydrous sodium sulfate. The solvent was removed under reduced pressure, and then the resultant was purified by column chromatography to obtain 2.0 of a product, i.e. compound 185-7. ESI-MS m/z: 446 [M+H] $^+$;

Step 8: Synthesis of compound 185-8

Compound 185-7 (2.0 g) was dissolved in 4 M hydrogen chloride in dioxane solution (15.00 mL) and 3.0 mL of methanol. The mixture was reacted at room temperature for 1.0 h. 50 mL of EA was added to the reaction solution. A yellow solid was precipitated in the system. After filtration, 1.1 g of a yellow product was obtained, i.e. compound 185-8, which was directly used for the next step. ESI-MS m/z: 346 [M+H] $^+$;

Step 9: Synthesis of compound 185-9

Compound 185-8 (1.1 g), N-tert-butoxycarbonyl-L-valine (0.76 g) and DIPEA (2.77 mL) were dissolved in 15.00 mL of DMF. And then HATU (1.98 g) were added. The mixture was reacted at room temperature for 1.0 h. Water was added dropwise to the reaction solution for quenching, and the resultant was extracted with ethyl acetate three times. The organic phases were combined, and dried with anhydrous sodium sulfate. The solvent was removed under reduced pressure, and then the resultant was purified by column chromatography to obtain 1.4 g of a product, i.e. compound 185-9.

ESI-MS m/z: 545 [M+H] $^+$;

Step 10: Synthesis of compound 185-10

Compound 185-9 (1.4 g) was dissolved in 4 M hydrogen chloride in dioxane solution (15.00 mL). The mixture was reacted at room temperature for 1.0 h. 4 M NaOH solution was added dropwise to the reaction solution in an ice bath to adjust the pH to 12. The resultant was extracted with a mixed solvent of DCM/MeOH = 10/1 three times. The organic phases were combined, dried with anhydrous sodium sulfate, and filtered. The solvent was removed under reduce pressure to obtain 1.1 g of a product, i.e. compound 185-10. ESI-MS m/z: 445 [M+H]$^+$;

Step 11: Synthesis of compound 185-11

Compound 185-10 (1.1 g) was dissolved in a mixed solvent of anhydrous acetonitrile (15.00 mL) and tetrahydrofuran (8.0 mL), and then triethylamine (2.06 mL) was added. 2-azido-1,3-dimethylimidazolinium hexafluorophosphate (1.41 g) was added in an ice bath, and the mixture was reacted at room temperature for 1.5 h. Water was added to the reaction solution for quenching, and the resultant was extracted with ethyl acetate three times. The organic phases were combined, and dried with anhydrous sodium sulfate. The solvent was removed under reduced pressure, and then the resultant was purified by column chromatography to obtain 1.1 g of a product, i.e. compound 185-11. ESI-MS m/z: 471 [M+H] $^+$;

Step 12: Synthesis of compound 185-12

Compound 185-11 (500 mg), compound 144-3 (482 mg), sodium ascorbate (546 mg), and anhydrous copper sulfate (152 mg) were dissolved in a mixed solvent of 2.00 mL of tert-butanol, 2.00 mL of tetrahydrofuran, and 2.00 mL of

water at room temperature. The mixture was reacted at room temperature for 1 h. The reaction solution was quenched by adding 50 mL of water, and the resultant was extracted with a mixed solvent of DCM/MeOH = 10/1 three times. The organic phases were combined, and dried with anhydrous sodium sulfate. The solvent was removed by under reduced pressure and the resultant was purified by column chromatography (DCM/MeOH = 10/1) to obtain 800 mg of a yellow solid product, i.e. compound 185-12. ESI-MS m/z: 774 $[M+H]^+$;

Step 13: Synthesis of compound 185-13

The sample of compound 185-12 (800 mg) was purified by preparative chromatographic column under conditions: Chromatographic Column: CHIRAL ART Cellulose-SB (20×250mm, 5μm); Mobile Phase: 0.1% DEA in HEX:0.1% DEA in EtOH = 85:15; Flow Rate: 20 mL/min; Sample Retention Time: target compound A: 16.33 min, target compound B: 21.85 min. Compound B was collected to obtain 320 mg of a product, i.e. compound 185-13. ESI-MS m/z: 774 $[M+H]^+$;

Step 14: Synthesis of compound 185-14

Compound 185-13 (320 mg) was dissolved in 3.0 mL of methanol. Then, 4 M hydrogen chloride in dioxane solution (4.00 mL) was added. The mixture was reacted at room temperature for 1.0 h. 4 M NaOH solution was added dropwise to the reaction solution in an ice bath to adjust the pH to 12. The resultant was extracted with a mixed solvent of DCM/MeOH = 10/1 three times. The organic phases were combined, dried with anhydrous sodium sulfate, and filtered. The solvent was removed under reduce pressure to obtain 180 mg of a product, i.e. compound 185-14. ESI-MS m/z: 674 $[M+H]^+$;

Step 15: Synthesis of compound 185

M43 (48.8 mg) and compound 185-14 (50.0 mg) were dissolved in anhydrous methanol (2.00 mL) at room temperature. Then, 1M zinc chloride in tetrahydrofuran solution (0.15 mL) was added. The mixture was reacted at 55°C for 1.0 h. Then, sodium cyanoborohydride (23.3 mg) was added and the mixture was reacted at 70°C for 3.0 h. The reaction solution was quenched with an appropriate amount of saturated ammonium chloride aqueous solution, and the mixture was extracted with DCM/MeOH = 10/1 three times, 20 mL each time. The organic phases were combined, and dried with anhydrous sodium sulfate. The solvent was removed under reduced pressure and then the resultant was purified by Pre-HPLC to obtain 23.2 mg of a white solid product, i.e. compound 185.

ESI-MS m/z: 604.2 1/2$[M+2H]^+$;
$^1$H NMR (500 MHz, Methanol-$d_4$) δ 9.21 (d, *J* = 4.3 Hz, 1H), 8.85 (d, *J* = 7.3 Hz, 1H), 8.74 (dd, J = 4.3, 1.4 Hz, 1H), 8.54 (d, *J* = 4.7 Hz, 1H), 8.19 (d, *J* = 1.5 Hz, 1H), 7.67 (dd, *J* = 9.1, 5.7 Hz, 1H), 7.39 (d, *J* = 8.1 Hz, 1H), 7.32 - 7.21 (m, 4H), 7.06 (t, *J* = 2.6 Hz, 1H), 5.39 (d, *J* = 10.1 Hz, 1H), 5.24 (q, *J* = 7.1 Hz, 1H), 5.16 (d, *J* = 10.0 Hz, 1H), 4.58 (s, 1H), 4.55 - 4.49 (m, 2H), 4.49 - 4.46 (m, 2H), 4.27 (t, *J* = 14.5 Hz, 1H), 3.95 - 3.82 (m, 2H), 3.63 (dd, *J* = 33.1, 13.3 Hz, 2H), 3.47 (d, *J* = 11.3 Hz, 2H), 3.02 (s, 2H), 2.62 (dt, *J*= 10.1, 6.5 Hz, 3H), 2.45 (d, *J* = 16.8 Hz, 6H), 2.26 - 2.07 (m, 6H), 2.00 - 1.92 (m, 2H), 1.80 (dd, *J* = 27.5, 17.6 Hz, 5H), 1.61 (d, *J* = 6.9 Hz, 1H), 1.49 (d, *J* = 7.0 Hz, 3H), 1.32 - 1.26 (m, 5H), 1.15 (d, *J* = 6.5 Hz, 3H), 0.85 - 0.74 (m, 7H), 0.58 (s, 2H).

Example 186: Synthesis of compound (2S,4R)-1-((S)-2-(4-(5-(7-(1-((7-(8-ethyl-7-fluoro-3-hydroxynaphthalen-1-yl)-8-fluoro-4-(((R)-3 -hydroxy-3-methylpiperidin-1-yl)quinazolin-2-yl)oxy)methyl)cyclopropyl)methyl)-2,7-diazaspir o[3.5] nonan-2-yl)pyrazin-2-yl)-1H-1,2,3-triazol-1-yl)-3-methylbutanoyl)-4-hydroxy-N-((S)-1-(4 -(4-methylthiazol-5-yl)phenyl) ethyl)pyrrolidine-2-carboxamide

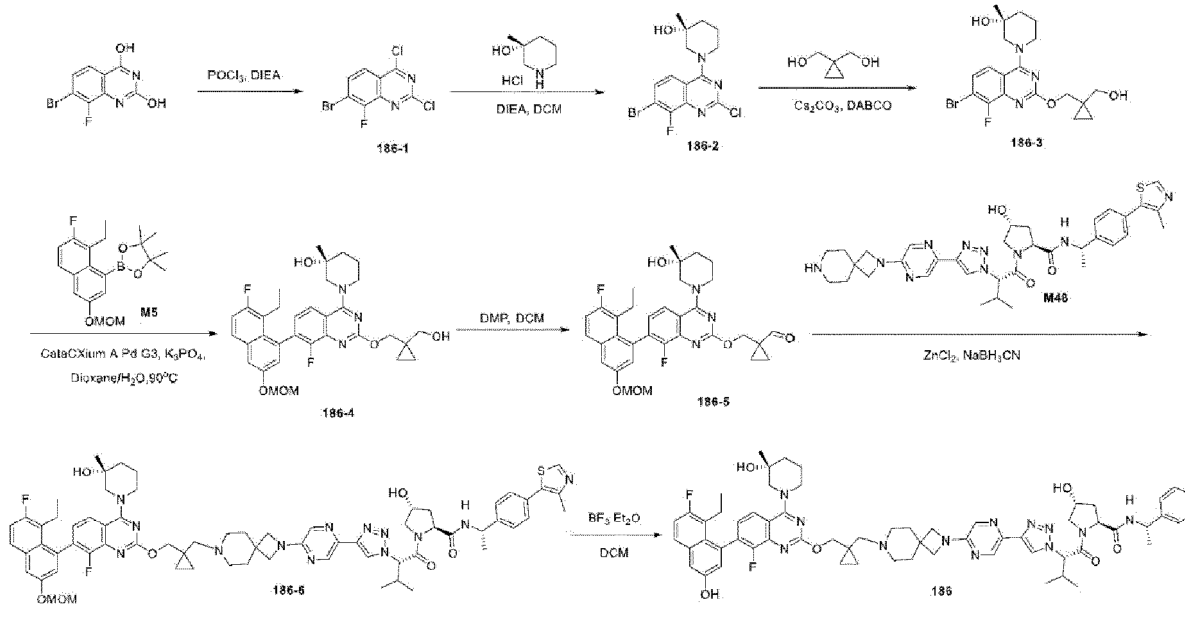


Step 1: Synthesis of compound 186-1

7-Bromo-8-fluoroquinazolin-2,4-diol (7.2 g) was dissolved in 60 mL of phosphorus oxychloride, and N,N-diisopropylethylamine (13.8 mL) was added under $N_2$ atmosphere. The mixture was heated to 100°C and reacted for 1 h. Phosphorus oxychloride was removed by rotary evaporator under reduced pressure. And the crude product was directly purified by column chromatography (PE/EA = 3:2) to obtain compound 186-1 (7.5 g), ESI-MS m/z: 294.8 $[M+H]^+$.

Step 2: Synthesis of compound 186-2

Compound 186-1 (0.5 g) was dissolved in 15.0 mL of dichloromethane. And then N,N-diisopropylethylamine (0.84 mL) and (3R)-3-methyl-piperidin-3-ol hydrochloride (0.27 g) were added at -40°C. The reaction was maintained at -40°C and reacted for 1 h. The reaction solution was added to saturated ammonium chloride solution. EA was added and the resultant was extracted. The organic phase was collected, dried with anhydrous sodium sulfate, filtered, dried by rotary evaporation and purified by column chromatography (PE/ EA = 1:1) to obtain compound 186-2 (0.55 g), ESI-MS m/z: 374.0 $[M+H]^+$.

Step 3: Synthesis of compound 186-3

Compound 186-2 (300 mg), and 1,1-bis(hydroxymethyl)cyclopropane (164 mg) were dissolved in 8.0 mL of DMF. And then cesium carbonate (522 mg) and triethylenediamine (18 mg) were added. The mixture was heated to 40°C and reacted for 3 h. The reaction solution was added to water. EA was added and the resultant was extracted. The organic phase was collected, dried with anhydrous sodium sulfate, filtered, concentrated and purified by column chromatography (DCM/ EA = 3:2) to obtain compound 186-3 (340 mg), ESI-MS m/z: 440.1 $[M+H]^+$.

Step 4: Synthesis of compound 186-4

Compound 186-3 (200 mg) was added to a 50 mL single-necked flask. And then 8 mL of 1,4-dioxane, 2 mL of water, intermediate M5 (327 mg), CataCXium A Pd G3 (66 mg), and potassium phosphate (289 mg) were added. The reaction was degassed with $N_2$ three times, and the mixture was heated to 90°C and reacted for 1 h. After the reaction was completed, EA and water were added to the reaction solution, and the resultant was extracted. The aqueous phase was further extracted with EA twice. The organic phases were collected and combined, dried with anhydrous sodium sulfate, and concentrated. The concentrate was purified by column chromatography (DCM/ EA = 3:2) to obtain compound 186-4 (205 mg), ESI-MS m/z: 594.2 $[M+H]^+$.

Step 5: Synthesis of compound 186-5

Compound 186-4 (200 mg) was dissolved in 8.0 mL of dichloromethane, and then Dess-Martin periodinane (214

mg) was added. The mixture was reacted at room temperature for 0.5 h. The insoluble impurities were removed by filtration. DCM and saturated sodium bicarbonate solution were added to the filtrate, and the resultant was extracted. The organic phases were collected, dried with anhydrous sodium sulfate, filtered, concentrated and purified by column chromatography (DCM/ EA = 3:2) to obtain compound 186-5 (142 mg), ESI-MS m/z: 592.2 [M+H] +.

Step 6: Synthesis of compound 186-6

Compound 186-5 (142 mg) and compound M48 (145 mg) were dissolved in methanol (5 mL). Then 1 M zinc chloride in tetrahydrofuran solution (0.29 mL) was added. The mixture was reacted at 40°C for 1 hour, and then sodium cyanoborohydride (45 mg) was added. The mixture was heated to 60°C and reacted for 6 hours. The resultant was quenched by adding saturated ammonium chloride solution. DCM was added to the reaction solution for extraction. The organic phase was collected, washed with saturated brine once, dried with anhydrous sodium sulfate, filtered. The filtrate was collected, concentrated and purified by column chromatography (DCM/MeOH = 8:1) to obtain compound 186-6 (138 mg), ESI-MS m/z: 630.7 1/2[M+2H]+.

Step 7: Synthesis of compound 186

Compound 186-6 was dissolved in 5 mL of dichloromethane and 5 mL of acetonitrile. 2.0 mL of boron trifluoride in diethyl ether solution (1 M) was added in an ice bath and maintain the ice bath reaction for 15 min. The reaction solution was added to icy saturated sodium bicarbonate solution, and DCM/MeOH (10:1) was added for extraction. The organic phase was collected, dried with anhydrous sodium sulfate, filtered, dried by rotary evaporation, and further purified by HPLC to obtain compound 186 (8.8 mg), ESI-MS m/z: 608.7 1/2[M+2H]+.

Example 187: Synthesis of compound (2S,4R)-1-((S)-2-(4-(5-((4-((1-((7-(8-ethyl-7-fluoro-3-hydroxynaphthalen-1-yl)-8-fluoro-4-((R)-3-hydroxy-3-methylpiperidin-1-yl)pyrido[4,3-d]pyrimidin-2-yl)oxy)methyl)cyclopropyl)methyl) piperazin-1-yl)methyl)thiazol-2-yl)-1H-1,2,3-triazol-1-yl)-3-methylbutanoyl)-4-hydroxy-N-((S)-1-(4-(4-methylthiazol-5-yl)phenyl) ethyl)pyrrolidine-2-carboxamide

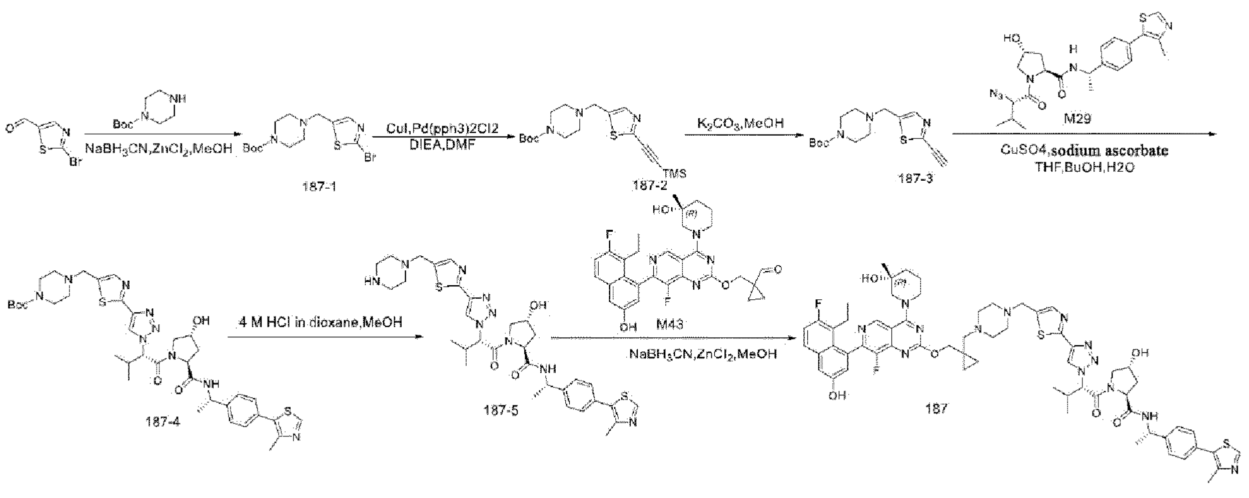


Step 1: Synthesis of compound 187-1

2-Bromo-5-fomylthiazole (1.0 g) was added to a 25 mL single-necked flask and dissolved in MeOH (10 mL). N-Boc-4-hydroxypiperidine (1.1 g) and 1 M zinc chloride in tetrahydrofuran solution (1.0 mL) were added. The mixture was heated to 40°C, and after the reaction for 2 h, $NaBH_3CN$ (0.62 g) was added. The mixture was reacted overnight. The reaction solution was directly evaporated to dryness. The resultant was separated and purified by column chromatography (PE:EA = 4:1) to obtain 1.2 g of a pale yellow solid, i.e. compound 187-1.

Step 2: Synthesis of compound 187-2

Compound 187-1 (1.2 g) was added to a 25 mL single-necked flask and dissolved in DMF (10 mL). Then,

trimethylsilylacetylene (487.8 mg), CuI (96.7 mg), $PdCl_2(PPh_3)_2$ (178.2 mg), and DIPEA (2.1 mL) were added. Under nitrogen protection, the mixture was heated to 50°C and reacted for 3 h. Saturated brine (5 mL) was added to the reaction solution and the resultant was extracted with EA. The organic phase was separated and dried with anhydrous sodium sulfate. The resultant was purified by column chromatography (PE:EA = 4:1) to obtain 475.2 mg of a pale yellow solid, i.e. compound 187-2.

Step 3: Synthesis of compound 187-3

Compound 187-2 (475.2 mg) was added to a 25 mL single-necked flask and dissolved in MeOH (10 mL). Then, $K_2CO_3$ (412.5 mg) was added. The mixture was reacted at room temperature for 2 h. The reaction solution was directly evaporated to dryness. The resultant was separated and purified by column chromatography (PE:EA = 4:1) to obtain 239.65 mg of a pale yellow solid, i.e. compound 187-3.

Step 4: Synthesis of compound 187-4

Compound M29 (100.0 mg) was added to a 25 mL single-necked flask and dissolved in THF (2 mL). Then compound 187-3 (74.0 mg), copper sulfate (35.2 mg), water (2 mL), tert-butanol (2 mL), and sodium ascorbate (188.0 mg) were added. The mixture was reacted at room temperature for 1 h. The reaction solution was directly evaporated to dryness. The resultant was separated and purified by column chromatography (DCM:MeOH = 12:1) to obtain 94.3 mg of a pale yellow solid, i.e. compound 187-4.

Step 5: Synthesis of compound 187-5

Compound 187-4 (94.3 mg) was added in a 25 mL single-necked flask and dissolved in HCl in dioxane solution(5 mL). Then, methanol (1 mL) was added. The mixture was reacted at room temperature for 1 h. The reaction solution was directly evaporated to dryness. Then, 5 mL of saturated sodium bicarbonate solution was added to the reaction solution. After adjusting the pH of the solution to 7-8, the resultant was extracted with dichloromethane/isopropanol, and the organic phase was separated and dried with anhydrous sodium sulfate to obtain 63.1 mg of a yellow solid, i.e. compound 187-5.

Step 6: Synthesis of compound 187

Compound 187-5 (63.1 mg) was added to a 25 mL single-necked flask, and dissolved in MeOH (5 mL). M43 (52.2 mg) and 1 M zinc chloride in tetrahydrofuran solution (0.1 mL) were added. The mixture was heated to 40°C, and after the reaction for 2 h, $NaBH_3CN$ (24.8 mg) was added. The mixture was reacted overnight. The reaction solution was directly evaporated to dryness. The resultant was separated by Pre-HPLC ($CH_3CN/H_2O$ = 55%:45%) to obtain 12.4 mg of a white solid, i.e. compound 187 (96.63% purity).

LCMS: $1/2[M+2H]^+$ = 599.2

$^1H$ NMR (500 MHz, Methanol-$d_4$) $\delta$ 9.18 (d, J = 10.7 Hz, 1H), 8.87 (d, J = 7.1 Hz, 1H), 8.61 (dd, J = 17.1, 2.9 Hz, 1H), 7.67 (dd, J = 9.7, 6.5 Hz, 2H), 7.41 (dq, J = 16.2, 8.2 Hz, 4H), 7.29 (d, J = 1.6 Hz, 1H), 7.26 - 7.19 (m, 1H), 7.06 (dd, J = 5.1, 2.7 Hz, 1H), 5.43 (dd, J = 10.0, 2.6 Hz, 1H), 5.08 - 4.99 (m, 1H), 4.60 (s, 1H), 4.55 - 4.36 (m, 6H), 4.22 (dd, J = 23.3, 13.2 Hz, 1H), 3.94 - 3.78 (m, 4H), 3.69-3.51 (m, 1H), 3.44 (s, 1H), 2.63 - 2.52 (m, 4H), 2.46 - 2.40 (m, 7H), 2.21- 2.16 (m, 3H), 1.96 (dd, J = 17.5, 8.6 Hz, 1H), 1.83 (s, 1H), 1.77 (d, J = 11.0 Hz, 2H), 1.64 (d, J = 7.2 Hz, 1H), 1.51 (dd, J = 7.0, 2.0 Hz, 3H), 1.28 - 1.21 (m, 4H), 1.15 (d, J = 6.5 Hz, 3H), 0.86 - 0.76 (m, 6H), 0.70 (s, 2H), 0.49 (s, 2H).

Example 188: Synthesis of compound 188. (2S,4R)-1-((S)-2-(4-(5-((((3R,7aR)-7a-(((7-(8-ethyl-7-fluoro-3-hydroxynaphthalen-1-yl)-8-fluor o-4-((R)-3-hydroxy-3-methylpiperidin-l-yl)pyrido[4,3-d]pyrimidin-2-yl)oxy)methyl)hexahydro-1H-pyrrolizin-3-yl)methoxy)pyrazin-2-yl)-1H-1,2,3-triazol-1-yl)-3-methylbutanoyl)-4-hydroxy-N-((S)-1-(4-(4-methylthiazol-5-yl)phenyl)ethyl)pyrrolidine-2-carboxamide

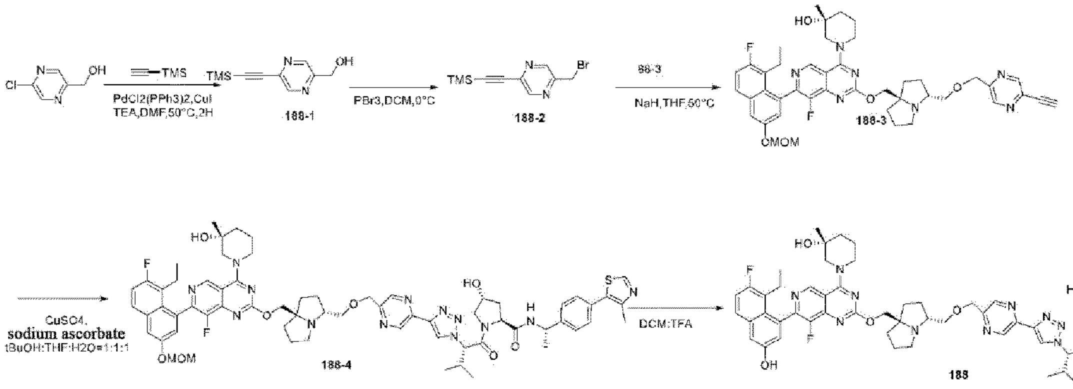


Step 1: Synthesis of compound 188-1

(5-Chloropyrazin-2-yl)methanol (1 g), trimethylsilylacetylene (1.16 mL), bis(triphenylphosphine)palladium(II) chloride (0.48 g) and cuprous iodide (0.13 g) were dissolved in 10 mL of DMF. And then triethylamine (2.88 mL) was added. The mixture was degassed with nitrogen three times, and reacted at 50°C for 2 h. The reaction was monitored by LCMS until it was completed. 30 mL of water was added to the reaction solution, the resultant was extracted with EA three times, 50 mL each time. The organic phases were combined, washed with saturated brine, dried with sodium sulfate, filtered, concentrated, and separated and purified by column chromatography to obtain 1 g of a white solid, i.e. compound 188-1. ESI-MS m/z: 207 $[M+H]^+$.

Step 2: Synthesis of compound 188-2

Compound 188-1 (1 g) was dissolved in 10 mL of DCM, and then $PBr_3$ (0.68 mL) was added dropwise at 0°C. The temperature of the mixture was maintained and the mixture was reacted for 1 hour. The reaction was monitored by LCMS until it was completed. 20.0 mL of water was added to the reaction solution, the resultant was extracted with DCM:MeOH = 10:1 three times, 20 mL each time. The organic phases were combined, dried with sodium sulfate, filtered, concentrated, and separated and purified by column chromatography to obtain 0.61 g of a white solid, i.e. compound 188-2. ESI-MS m/z: 269 $[M+H]^+$.

Step 3: Synthesis of compound 188-3

Compound 88-3 (R)-1-(7-(8-ethyl-7-fluoro-3-(methoxy-methoxy)naphthalen-1-yl)-8-fluoro-2-(((3R,7aR)-3-(hydr oxymethyl)tetrahydro-1H-pyrrolizin-7a(5H)-yl)methoxy)pyrido[4,3-d]pyrimidin-4-yl)-3-methyl piperidin-3-ol (200 mg) was dissolved in 4 mL of THF. And then NaH (32 mg) was added, and the mixture was stirred at room temperature for 30 minutes. Then, compound 188-2 (106 mg) was added, and the reaction vessel was equipped with a nitrogen balloon. The mixture was stirred at 50°C for 1 h. The reaction was monitored by LCMS until it was completed. 10mL of saturated ammonium chloride solution was slowly added to the reaction solution for quenching, and the resultant was extracted with EA three times. The organic phases were combined, and dried with anhydrous sodium sulfate. The solvent was removed under reduced pressure, and then the resultant was separated and purified by column chromatography to obtain 70 mg of a pale yellow solid, i.e. compound 188-3. ESI-MS m/z: 780 $[M+H]^+$;

Step 4: Synthesis of compound 188-4

Compound 188-3 (70 mg) and M29 (2S,4R)-1-((S)-2-azido-3-methylbutanoyl)-4-hydroxy-N-((S)-1-(4-(4-methylthiazol-5-yl)phenyl) ethyl)pyrrolidine-2-carboxamide (41 mg) were dissolved in 1.5 mL of THF, 1.5 mL of water, and 1.5 mL of tert-butanol. Then sodium ascorbate (39 mg) and anhydrous copper sulfate (11 mg) were added successively. The mixture was reacted at room temperature for 0.5 hours. The reaction was monitored by LCMS until it was completed. 20.0 mL of water was added to the reaction solution, the resultant was extracted with DCM:isopropanol = 4:1 three times, 20 mL each time. The organic phases were combined, dried with sodium sulfate, filtered, concentrated, and separated and purified by column chromatography to obtain 40 mg of a pale yellow solid, i.e. compound 188-4. ESI-MS m/z: 619 1/2$[M+2H]^+$.

Step 5: Synthesis of compound 188

Compound 188-4 (40 mg) was dissolved in 2 mL of DCM, and then 1 mL of TFA was added. The mixture was reacted at room temperature for 30 min. The reaction was monitored by LCMS until it was completed. The reaction solution was directly concentrated, and was separated by the preparative liquid chromatography to obtain 11 mg of a white powder, i.e. compound 188.

ESI-MS m/z: 597 1/2[M+2H]+.

Example 189: Synthesis of compound (2S,4R)-1-((S)-2-(4-(5-(7-(1-(7-(8-ethyl-7-fluoro-3-hydroxynaphthalen-1-yl)-8-fluoro-4-((R)-3-h ydroxy-3-methylpiperidin-1-yl)pyrido[4,3-d]pyrimidin-2-yl)oxy)methyl)cyclopropyl)methyl)-2, 7-diazaspiro[3.5]nonan-2-yl)methyl)pyrazin-2-yl)-1H-1,2,3-triazol-1-yl)-3-methylbutanoyl)-4-hy droxy-N-((S)-1-(4-(4-methylthiazol-5-yl)phenyl)ethyl)pyrrolidine-2-carboxamide

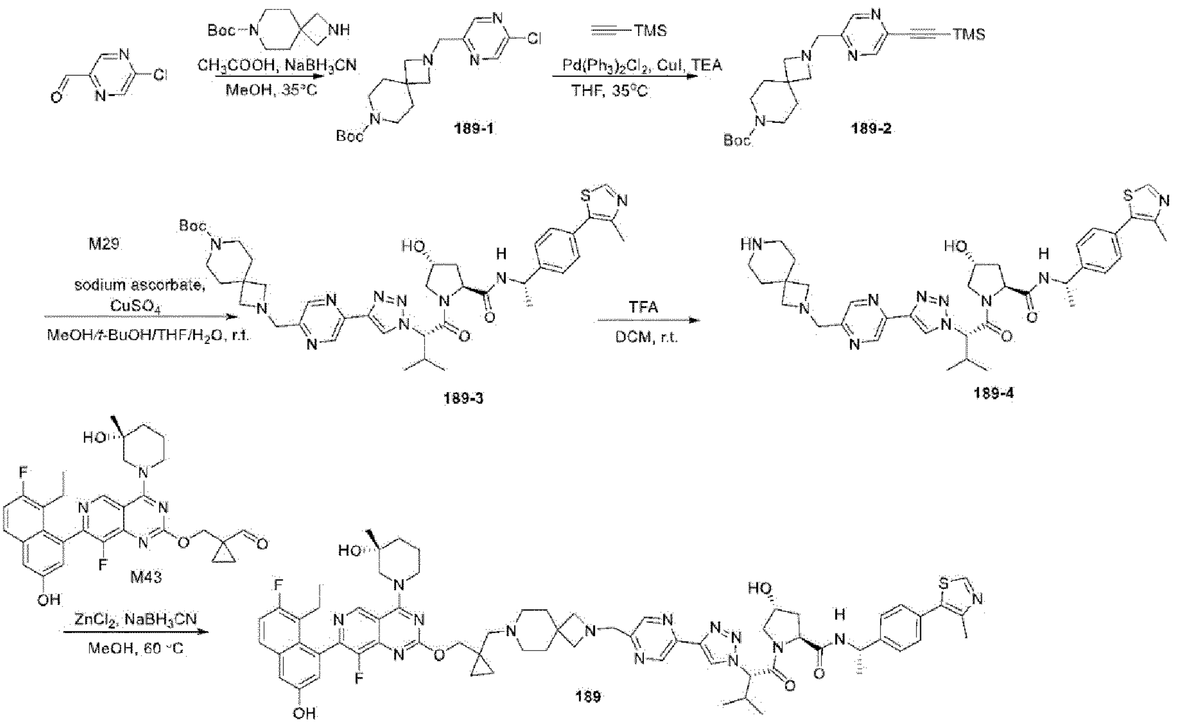


Step 1: Synthesis of compound 189-1

5-Chloropyrazine-2-carbaldehyde (1.0 g), and tert-butyl 2,7-diazaspiro[3.5]nonane-7-carboxylate (1.59 g) were dissolved in methanol (10 mL). After reacting for 1 hour, sodium cyanoborohydride was added in batches. After the reaction was completed, ethyl acetate and water were added and the resultant was extracted three times, dried with anhydrous sodium sulfate, filtered and concentrated to obtain a crude product. The crude product was purified by column chromatography to obtain compound 189-1 (480 mg). ESI-MS m/z: 353.2 [M+H]+.

Step 2: Synthesis of compound 189-2

Compound 189-1 (480 mg), bis(triphenylphosphine)palladium(II) chloride (85 mg), and cuprous iodide (46 mg) were dissolved in TEA (0.84 mL) under nitrogen protection. Then trimethylsilylacetylene (0.33 mL) was added and the mixture was reacted at 60°C overnight. The reaction was monitored by LCMS until it was completed. The reaction solution was directly concentrated. EA (10 mL) was added to the reaction solution, and the resultant was washed with saturated brine once. The organic phase was dried with anhydrous sodium sulfate, filtered, concentrated, separated and purified by column chromatography to obtain compound 189-2 (210 mg). ESI-MS m/z: 415.1 [M+H]+.

Step 3: Synthesis of compound 189-3

Compound 189-2 (200.0 mg), M29 (2S,4R)-1-((S)-2-azido-3-methylbutanoyl)-4-hydroxy-N-((S)-1-(4-(4-methylthiazol-5-yl)phenyl) ethyl)pyrrolidine-2-carboxamide (220 mg), potassium carbonate (333 mg), copper sulfate (70 mg), and sodium ascorbate (249 mg) were dissolved in a mixed solvent of THF (2 mL)/tBuOH (1 mL)/MeOH (1 mL)/$H_2O$ (2 mL) under nitrogen protection. The mixture was reacted at room temperature for 3 hours, and the reaction was monitored by LCMS until it was completed. DCM (10 mL) was added to the reaction solution, and the resultant was washed with saturated brine once. The organic phase was dried with anhydrous sodium sulfate, filtered, concentrated, separated and purified by column chromatography to obtain compound 189-3 (274 mg). ESI-MS m/z: 799.1 $[M+H]^+$.

Step 4: Synthesis of compound 189-4

Compound 189-3 (274 mg) was dissolved in dichloromethane (5 mL), and trifluoroacetic acid (1 mL) was added. The mixture was reacted at 25°C for 20 minutes. After the reaction was completed, the resultant was neutralized with sodium bicarbonate solution. Dichloromethane/isopropanol (10:1) was added and the resultant was extracted, dried and concentrated to obtain compound 189-4 (164 mg). ESI-MS m/z: 699.2 $[M+H]^+$.

Step 5: Synthesis of compound 189

Compound 189-4 (164 mg), M43 (208 mg), and 1 M zinc chloride in tetrahydrofuran solution (0.60 mL) were dissolved in methanol (5 mL), and the mixture was reacted at 40°C for 2 hour. Then sodium cyanoborohydride (95 mg) was added, and the mixture was reacted at 40°C for 8 hours. After the reaction was completed, the reaction was quenched with ammonium chloride solution. The resultant was concentrated and purified by column chromatography (dichloromethane/methanol) to obtain compound 189 (82 mg).

ESI-MS m/z: 616.6 $1/2[M+2H]^+$.

Example 190: Synthesis of compound (2S,4R)-1-((S)-2-(4-(5-(1-((((7-(8-ethyl-7-fluoro-3-hydroxynaphthalen-1-yl)-8-fluoro-4-(((R)-3-hydroxy-3-methylpiperidin-1-yl)pyrido[4,3-d]pyrimidin-2-yl)oxy)methyl)cyclopropyl)methyl)pi peridin-4-yl)oxy)-2-ylpyrazin-1H-1,2,3-triazol-1-yl)-3-methylbutanoyl)-4-hydroxy-N-((S)-3-(me thylamino)-1-(4-(4-methylthiazol-5-yl)phenyl)-3-oxopropyl)pyrrolidine-2-carboxamide

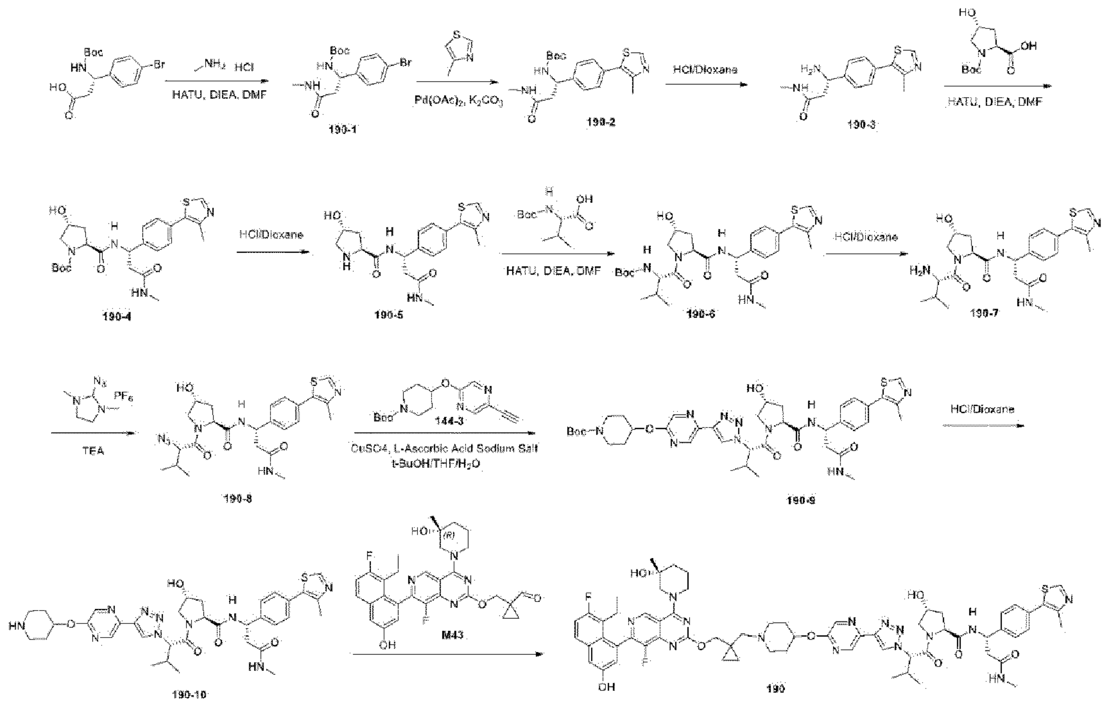

Step 1: Synthesis of compound 190-1

(3S)-3-(4-bromophenyl)-3-((tert-butoxycarbonyl)amino)propanoic acid (2.5 g) and methylamine hydrochloride (0.58 g) were dissolved in 15.0 mL of DMF. And then DIPEA (6.0 mL) and HATU (3.3 g) were added. The mixture was reacted at room temperature for 0.5 h. The reaction solution was added to water, and the resultant was extracted with EA. The organic phase was collected, dried with anhydrous sodium sulfate, filtered, dried by rotary evaporation and purified by column chromatography (DCM/MeOH = 10:1) to obtain compound 190-1 (2.4 g). ESI-MS m/z: 357.0 $[M+H]^+$.

Step 2: Synthesis of compound 190-2

Compound 190-1 (2.4 g) and 4-methylthiazole (1.0 g) were dissolved in 15.0 mL of DMA. $Pd(OAc)_2$ (0.3 g) and $K_2CO_3$ (2.8 g) were added. The mixture was heated to 120°C under $N_2$ atmosphere and reacted for 1.5 h. The reaction solution was added to water. EA was added and the resultant was extracted. The organic phase was collected, dried with anhydrous sodium sulfate, filtered, concentrated and purified by column chromatography (DCM/MeOH = 10:1) to obtain compound 190-2 (1.75 g). ESI-MS m/z: 376.1 $[M+H]^+$.

Step 3: Synthesis of compound 190-3

Compound 190-2 (1.75 g) was added to 10 mL of DCM. 4 M hydrogen chloride in dioxane solution (10 mL) was added to the mixture in an ice water bath. The mixture was reacted at room temperature for 1 h. The reaction solution was concentrated directly and 1 M NaOH solution was added until the reaction solution was pH = 8. DCM was added to the reaction solution, and the resultant was extracted. The organic phase was collected and washed with saturated brine once, dried with anhydrous sodium sulfate, filtered, and concentrated to obtain compound 190-3 (1.33 g). ESI-MS m/z: 276.1 $[M+H]^+$.

Step 4: Synthesis of compound 190-4

Compound 190-3 (1.33 g) and tert-butoxycarbonyl-L-hydroxyproline (1.12 g) were dissolved in 12.0 mL of DMF. And then DIPEA (4.0 mL) and HATU (2.2 g) were added. The mixture was reacted at room temperature for 0.5 h. The reaction solution was added to water, and the resultant was extracted with DCM/isopropanol (3:1). The organic phase was collected, dried with anhydrous sodium sulfate, filtered, concentrated and purified by column chromatography (DCM/MeOH = 10:1) to obtain compound 190-4 (2.28 g). ESI-MS m/z: 489.2 $[M+H]^+$.

Step 5: Synthesis of compound 190-5

Compound 190-4 (2.28 g) was added to 10 mL of DCM. 4 M hydrogen chloride in dioxane solution (10 mL) was added to the mixture in an ice water bath. The mixture was reacted at room temperature for 1 h. The reaction solution was concentrated directly and 1 M NaOH solution was added until the reaction solution was pH = 8. DCM/isopropanol (3:1) was added to the reaction solution, and the resultant was extracted. The organic phase was collected and washed with saturated brine once, dried with anhydrous sodium sulfate, filtered, and concentrated to obtain compound 190-5 (2.0 g). ESI-MS m/z: 389.1 $[M+H]^+$.

Step 6: Synthesis of compound 190-6

Compound 190-5 (2.0 g) and N-tert-butoxycarbonyl-L-valine (1.12 g) were dissolved in 15.0 mL of DMF. And then DIPEA (8.5 mL) and HATU (2.35 g) were added. The mixture was reacted at room temperature for 0.5 h. The reaction solution was added to water, and the resultant was extracted with DCM/MeOH (10: 1). The organic phase was collected, dried with anhydrous sodium sulfate, filtered, concentrated and purified by column chromatography (DCM/MeOH = 10:1) to obtain compound 190-6 (2.05 g). ESI-MS m/z: 588.2 $[M+H]^+$.

Step 7: Synthesis of compound 190-7

Compound 190-6 (2.05 g) was added to 10 mL of DCM. 4 M hydrogen chloride in dioxane solution (10 mL) was added to the mixture in an ice water bath. The mixture was reacted at room temperature for 1 h. The reaction solution was concentrated directly and 1 M NaOH solution was added until the reaction solution was pH = 8. DCM/isopropanol (3:1) was added to the reaction solution, and the resultant was extracted. The organic phase was collected and washed with saturated brine once, dried with anhydrous sodium sulfate, filtered, and concentrated to obtain compound 190-7 (1.6 g). ESI-MS m/z: 488.2 $[M+H]^+$.

Step 8: Synthesis of compound 190-8

Compound 190-7 (200 mg) and TEA (0.35 mL) were dissolved in ACN (5 mL) and THF (5 mL). 2-azido-1,3-dimethylimidazolium hexafluorophosphate (234 mg) was added in an ice water bath, and the mixture was heated naturally to room temperature and reacted for 3 h. The reaction solution was added to water, and the resultant was extracted with DCM/MeOH (10:1). The organic phase was collected, dried with anhydrous sodium sulfate, filtered. The organic phase was collected, concentrated and purified by column chromatography (DCM/MeOH = 10:1) to obtain compound 190-8 (150 mg). ESI-MS m/z: 514.2 $[M+H]^+$.

Step 9: Synthesis of compound 190-9

Compound 190-8 (100 mg) and compound 144-3 (66 mg) were dissolved in tert-butanol (4 mL), tetrahydrofuran (4 mL), and water (4 mL). Anhydrous copper sulfate (28 mg) and sodium ascorbate (100 mg) were added. The mixture was reacted at room temperature for 2 h. The reaction solution was dried by rotary evaporation. DCM/MeOH (10:1) and water were added, and the resultant was extracted. The organic phase was collected, dried with anhydrous sodium sulfate, filtered. The filtrate was collected, concentrated and purified by column chromatography (DCM/MeOH = 8:1) to obtain compound 190-9 (132 mg). ESI-MS m/z: 817.3 $[M+H]^+$.

Step 10: Synthesis of compound 190-10

Compound 190-9 (132 mg) was added to 4 mL of DCM. 4 M Hydrogen chloride in dioxane solution (4 mL) was added in an ice water bath. The mixture was reacted at room temperature for 1 h. The reaction solution was concentrated directly and 1 M NaOH solution was added until the reaction solution was pH = 8. DCM/isopropyl alcohol (3:1) was added to the reaction solution, and the resultant was extracted. The organic phase was collected and washed with saturated brine once, dried with anhydrous sodium sulfate, filtered, and concentrated to obtain compound 190-10 (105 mg). ESI-MS m/z: 717.3 $[M+H]^+$.

Step 11: Synthesis of compound 190

Compound 190-10 (105 mg) and compound M43 (96 mg) were dissolved in methanol (8 mL). Then 1 M zinc chloride in tetrahydrofuran solution (0.22 mL) was added. The mixture was reacted at 40°C for 1 hour, then sodium cyanoborohydride (28 mg) was added. The mixture was heated to 60°C and reacted for 6 hours. The reaction was quenched by adding saturated ammonium chloride solution, and DCM/MeOH (10:1) was added to the reaction solution, and the resultant was extracted. The organic phase was collected, washed with saturated brine once, dried with anhydrous sodium sulfate, and filtered. The filtrate was collected, and dried by rotary evaporation to obtain a crude product, and further purified by Pre-HPLC to obtain compound 190 (33.4 mg).

ESI-MS m/z: 625.2 $1/2[M+2H]^+$.

Example 191: Synthesis of compound (2S,4R)-1-((S)-2-(4-(5-(2-(4-((1-(((7-(8-ethyl-7-fluoro-3-hydroxynaphthalen-1-yl)-8-fluoro-4-(( R)-3-hydroxy-3-methylpiperidin-1-yl)pyrido[4,3-d]pyrimidin-2-yl)oxy)methyl)cyclopropyl)meth yl)piperazin-1-yl)ethyl)pyrazin-2-yl)-1H-1,2,3-triazol-1-yl)-3-methylbutanoyl)-4-hydroxy-N-((S )-1-(4-(4-methylthiazol-5-yl)phenyl)ethyl)pyrrolidine-2-carboxamide

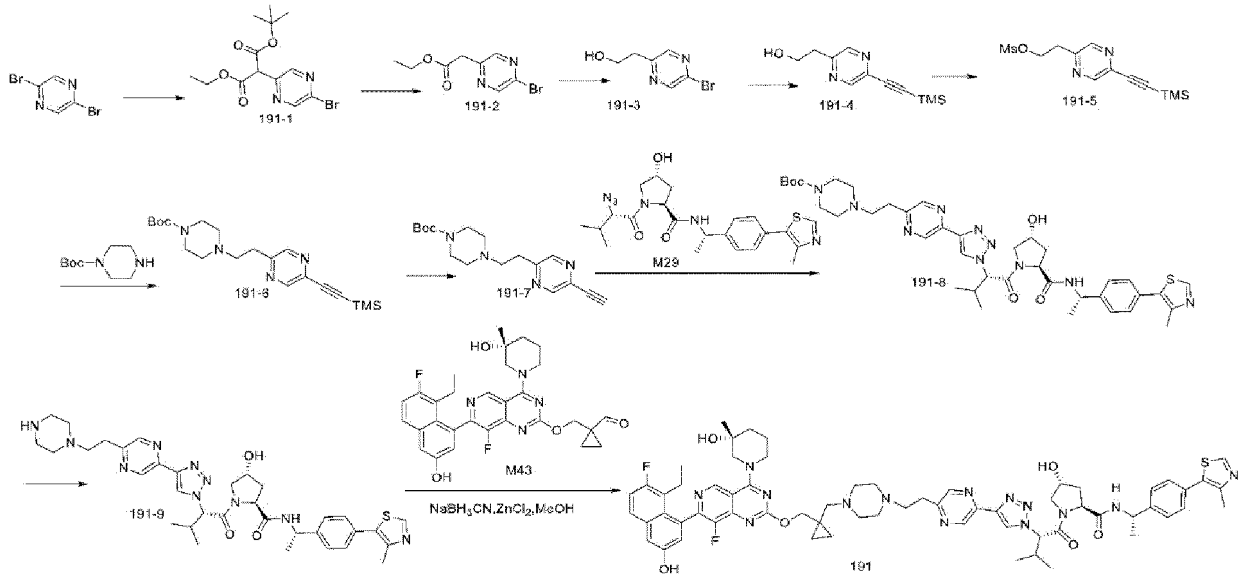


Step 1: Synthesis of compound 191-1

Under $N_2$ atmosphere, NaH (1.0 g) was added in batches to a solution of tert-butyl ethyl malonate (3.5 g) in dry DMF (30 mL) at 0°C. After the mixture was continued to be stirred at 0°C for 30 min, 2,5-dibromopyrazine (3.0 g) was added. And then the mixture was heated to 80°C and stirred for 3.0 h. After the reaction was completed, the resultant was quenched with water, extracted with EA three times. The organic phases were combined and washed with saturated brine, dried with anhydrous sodium sulfate, filtered and concentrated to obtain compound 191-1 (3.0 g). ESI-MS m/z: 345.2 $[M+H]^+$.

Step 2: Synthesis of compound 191-2

Compound 191-1 (3.0 g) was added to a 50 mL single-necked flask, and dissolved in dichloromethane (10 mL). The mixture was cooled down to about 0°C. TFA (10 mL) was added, and the mixture was stirred at room temperature for 2.5 h. After the reaction was completed, the reaction solution was concentrated. The resultant was diluted with water, and extracted with ethyl acetate three times. The organic phases were collected, combined and washed with saturated brine, dried with anhydrous sodium sulfate, filtered, concentrated and purified by column chromatography (PE:EA = 5:1) to obtain compound 191-2 (1.3 g). ESI-MS m/z: 245.1 $[M+H]^+$.

Step 3: Synthesis of compound 191-3

Compound 191-2 (500 mg) was dissolved in MeOH (20 mL), and sodium borohydride (1.0 g) was added in batches. The reaction was stirred at room temperature for 5.0 h. After the reaction was completed, the mixture was quenched with saturated ammonium chloride solution, extracted with dichloromethane/methanol (10:1) three times. The organic phases were combined, washed with saturated brine, dried with anhydrous sodium sulfate, filtered, concentrated, and purified by column chromatography (DCM:MeOH = 50:1) to obtain compound 191-3 (130 mg). ESI-MS m/z: 203.4 $[M+H]^+$.

Step 4: Synthesis of compound 191-4

Compound 191-3 (130 mg), trimethylsilylacetylene (188 mg), $Pd(PPh_3)_2Cl_2$ (45 mg), CuI (25 mg), and TEA (648 mg) were dissolved in THF (10 mL), and the mixture was heated to 80°C and stirred for 2.0 h. After the reaction was completed, the mixture was filtered with diatomaceous earth, dried by rotary evaporation, and purified by column chromatography (DCM:MeOH = 50:1) to obtain compound 191-4 (75 mg). ESI-MS m/z: 221.4 $[M+H]^+$.

Step 5: Synthesis of compound 191-5

Compound 191-4 (75 mg) was dissolved in dichloromethane (5 mL), and TEA (102 mg) was added. The mixture was cooled down to below 0°C. Then methylsulfonyl chloride (78 mg) was added and the mixture was stirred at room

temperature and reacted for 20 minutes. After the reaction was completed, the mixture was quenched with water, extracted with ethyl acetate three times. The organic phases were combined and washed with saturated brine, dried with anhydrous sodium sulfate, filtered and concentrated to obtain a crude compound 191-5 (130 mg). ESI-MS m/z: 299.2 [M+H] +.

Step 6: Synthesis of compound 191-6

Compound 191-5 (130 mg) was dissolved in acetonitrile (10 mL), and N-tert-butoxycarbonylpiperazine (200 mg) and $K_2CO_3$ (500 mg) were added. Then the mixture was heated to 80°C, stirred and reacted for 4.0 h. After the reaction was completed, the mixture was diluted with water, extracted with ethyl acetate three times. The organic phases were combined and washed with saturated brine, dried with anhydrous sodium sulfate, filtered, concentrated and purified by column chromatography (PE:EA = 1:1) to obtain compound 191-6 (55 mg). ESI-MS m/z: 389.2 [M+H] +.

Step 7: Synthesis of compound 191-7

Compound 191-6 (55 mg) was dissolved in acetonitrile (10 mL), and $K_2CO_3$ (500 mg) were added. Then the mixture was heated to 80°C, stirred and reacted for 3.0 h. After the reaction was completed, the mixture was diluted with water, extracted with ethyl acetate three times. The organic phases were combined and washed with saturated brine, dried with anhydrous sodium sulfate, filtered, concentrated and purified by column chromatography (PE:EA = 1:1) to obtain compound 191-7 (30 mg). ESI-MS m/z: 317.2 [M+H]+.

Step 8: Synthesis of compound 191-8

Compound 191-7 (30 mg), sodium ascorbate (40 mg), copper sulfate (15 mg) and M29 (45 mg) were added successively to a 25 mL single-necked flask. 3 mL of methanol, 3 mL of tetrahydrofuran and 3 ml of water were added, and the mixture was reacted at room temperature for 30 min. The resultant was dried by rotary evaporation and purified by column chromatography (DCM:MeOH = 10:1) to obtain compound 191-8 (40 mg). ESI-MS m/z: 773.4 [M+H] +.

Step 9: Synthesis of compound 191-9

Compound 191-8 (40 mg) was added to a 25 mL single-necked flask and dissolved in 10 mL of dichloromethane. TFA (5 mL) was added, and the reaction was stirred at room temperature for 1.0 h. After the reaction was completed, the reaction solution was dried by rotary evaporation. The pH of the resultant was adjusted to 10.0 with saturated sodium bicarbonate solution. The resultant was extracted with dichloromethane/isopropanol (10:1) three times. The organic phases were combined, washed with saturated brine, dried with anhydrous sodium sulfate, filtered, and concentrated to obtain a crude compound 191-9 (30 mg). ESI-MS m/z: 673.4 [M+H] +.

Step 10: Synthesis of compound 191

Compound 191-9 (30 mg), M43 (25 mg), and 1 M zinc chloride in tetrahydrofuran solution (0.5 mL) were dissolved in methanol (10 mL). The mixture was reacted at 40°C for 1 hour. Then sodium cyanoborohydride (10 mg) was added, and the mixture was stirred and reacted at 70°C for 5 hours. After the reaction was completed, the mixture was cooled down to room temperature, and quenched by adding ammonium chloride solution. The resultant was extracted with dichloromethane and methanol (10:1), dried with anhydrous sodium sulfate, filtered, concentrated, purified by column chromatography (DCM:MeOH = 85:15). And a crude product was purified by Pre-HPLC (C18 chromatographic column, A: 0.05% TFA aqueous solution, B: acetonitrile, concentration gradient: 20% B to 55% B, 6.45 min to6.90 min, flow rate: 20 mL/min, 254 nm) to obtain compound 191 (10.6 mg).

ESI-MS m/z: 603.7 1/2[M+2H]+.

$^1$H NMR (500 MHz, DMSO-$d_6$) δ 9.21 (s, 1H), 9.12 (s, 1H), 8.98 (s, 1H), 8.71 (s, 1H), 8.60 (s, 1H), 8.53 (d, J = 7.3 Hz, 1H), 7.75 (s, 1H), 7.44 (d, J = 7.1 Hz, 2H), 7.34 (dd, J = 20.2, 8.8 Hz, 4H), 7.03 (s, 1H), 5.43 (d, J = 10.4 Hz, 1H), 4.93 (d, J = 6.8 Hz, 1H), 4.74 (s, 1H), 4.41 (t, J = 7.8 Hz, 1H), 4.31 (d, J = 12.1 Hz, 4H), 4.04 (dd, J = 27.4, 13.5 Hz, 1H), 3.84 - 3.65 (m, 2H), 3.58 (dd, J = 51.2, 14.2 Hz, 1H), 3.17 (s, 1H), 2.95 (s, 3H), 2.65 (s, 3H), 2.46 (s, 5H), 2.29 (s, 2H), 2.04 (d, J = 31.8 Hz, 4H), 1.68 (s, 3H), 1.38 (d, J = 6.8 Hz, 3H), 1.23 (s, 4H), 1.17 (d, J = 12.8 Hz, 3H), 1.09 (d, J = 6.1 Hz, 4H), 0.86 (s, 2H), 0.73 (d, J = 6.7 Hz, 7H), 0.64 (s, 2H), 0.40 (s, 2H).

Example 192: Synthesis of compound (2S,4R)-1-((S)-2-(4-(5-(3-(4-((1-((7-(8-ethyl-7-fluoro-3-hydroxynaphthalen-1-yl)-8-fluoro-4-((R )-3-hydroxy-3-methylpiperidin-1-yl)pyrido[4,3-d]pyrimidin-2-yl)oxy)methyl)cyclopropyl)methy l)piperazin-1-yl)azetidin-1-yl)pyrazin-2-yl)-1H-1,2,3-triazol-1-yl)-3-methylbutanoyl)-4-hydroxy -N-((R)-2-hydroxy-1-(4-(4-methylthiazol-5-yl)phenyl)ethyl)pyrrolidine-2-carboxamide

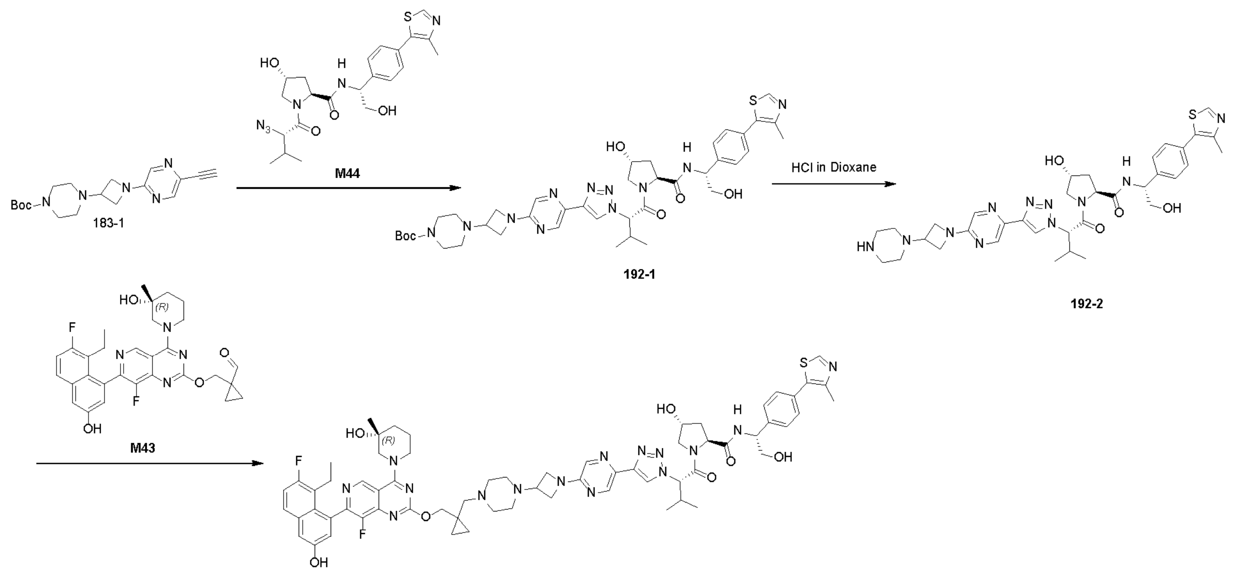


192

Step 1: Synthesis of compound 192-1

Compound 183-1 (190 mg), M44 (230 mg), sodium ascorbate (228 mg), and anhydrous copper sulfate (64.0 mg) were dissolved in a mixed solvent of 2.00 mL of tert-butanol, 2.00 mL of tetrahydrofuran, and 2.00 mL of water at room temperature. The mixture was reacted at room temperature for 1 h. The reaction solution was quenched with 50 mL of water, and extracted with a mixed solvent of DCM/MeOH = 10/1 three times. The organic phases were combined, and dried with anhydrous sodium sulfate. The solvent was removed under reduced pressure and the resultant was purified by column chromatography (DCM/MeOH = 10/1) to obtain 298 mg of a yellow solid product, i.e. compound 192-1. ESI-MS m/z: 816 $[M+H]^+$;

Step 2: Synthesis of compound 192-2

Compound 192-1 (298 mg) was dissolved in 5.00 mL of anhydrous methanol, and then 4M hydrogen chloride in dioxane solution (2.00 mL) was added at room temperature. The mixture was reacted at room temperature for 0.5 h. The resultant was concentrated under reduced pressure, and then purified by column chromatography to obtain 224 mg of a yellow solid, i.e. compound 192-2. ESI-MS m/z: 716 $[M+H]^+$;

Step 3: Synthesis of compound 192

M43 (64.4 mg) and compound 192-2 (70.0 mg) were dissolved in anhydrous methanol (3.00 mL) at room temperature. 1 M zinc chloride in tetrahydrofuran solution (0.196 mL) was added. The mixture was reacted at 55°C for 1.0 h. Then, sodium cyanoborohydride (30.7 mg) was added and the mixture was reacted at 70°C for 3.0 h. The reaction solution was quenched with an appropriate amount of saturated ammonium chloride aqueous solution, and the mixture was extracted with DCM/MeOH = 10/1 three times, 20 mL each time. The organic phases were combined, and dried with anhydrous sodium sulfate. The solvent was removed under reduced pressure and then the resultant was purified by Pre-HPLC to obtain 20.0 mg of a white solid product, i.e. compound 192.

ESI-MS m/z: 625.35 1/2$[M+2H]^+$;

$^1$H NMR (500 MHz, Methanol-$d_4$) $\delta$ 9.21 (d, $J$ = 6.1 Hz, 1H), 8.87 (d, $J$ = 2.4 Hz, 1H), 8.63 (d, $J$ = 1.5 Hz, 1H), 8.45 (s, 1H), 7.88 (d, $J$ = 1.5 Hz, 1H), 7.67 (dd, $J$ = 9.0, 5.8 Hz, 1H), 7.49 - 7.41 (m, 3H), 7.29 (d, $J$ = 2.6 Hz, 1H), 7.24 (t, $J$ = 9.3 Hz, 1H), 7.06 (t, $J$ = 2.5 Hz, 1H), 5.38 (d, $J$ = 10.1 Hz, 1H), 5.05 (t, $J$ = 6.1 Hz, 1H), 4.59 (t, $J$ = 8.3 Hz, 2H), 4.55 - 4.41 (m, 5H), 4.31 - 4.18 (m, 4H), 4.05 - 3.97 (m, 2H), 3.93 (dd, $J$ = 11.0, 3.9 Hz, 2H), 3.87 - 3.81 (m, 2H), 3.63 (dd, $J$ = 33.1, 13.3 Hz, 2H), 3.47 (s,

2H), 3.37 (s, 1H), 2.61 (dq, *J* = 12.5, 6.4 Hz, 4H), 2.52 (s, 2H), 2.41 (dd, *J* = 12.7, 4.2 Hz, 2H), 2.20 (dd, *J* = 15.0, 7.8 Hz, 4H), 2.08 - 2.00 (m, 2H), 1.85 (s, 1H), 1.79 (d, *J* = 11.1 Hz, 2H), 1.36 - 1.25 (m, 8H), 1.15 (d, *J* = 6.6 Hz, 3H), 0.90 (d, *J* = 6.5 Hz, 1H), 0.81 (dd, *J*= 9.4, 6.9 Hz, 5H), 0.72 (s, 2H), 0.50 (s, 2H).

Example 193: Synthesis of compound (2S,4R)-1-((S)-2-(4-(5-((1-((1-(((7-(8-ethyl-7-fluoro-3-hydroxynaphthalen-1-yl)-8-fluoro-4-((R)-3-hydroxy-3-methylpiperidin-1-yl)pyrido[4,3-d]pyrimidin-2-yl)oxy)methyl)cyclopropyl)methyl) piperidin-4-yl)methyl)piperazin-2-yl)-1H-1,2,3-triazol-1-yl)-3-methylbutanoyl)-4-hydroxy-N-((S )-1-(4-(4-methylthiazol-5-yl) phenyl)ethyl)pyrrolidine-2-carboxamide

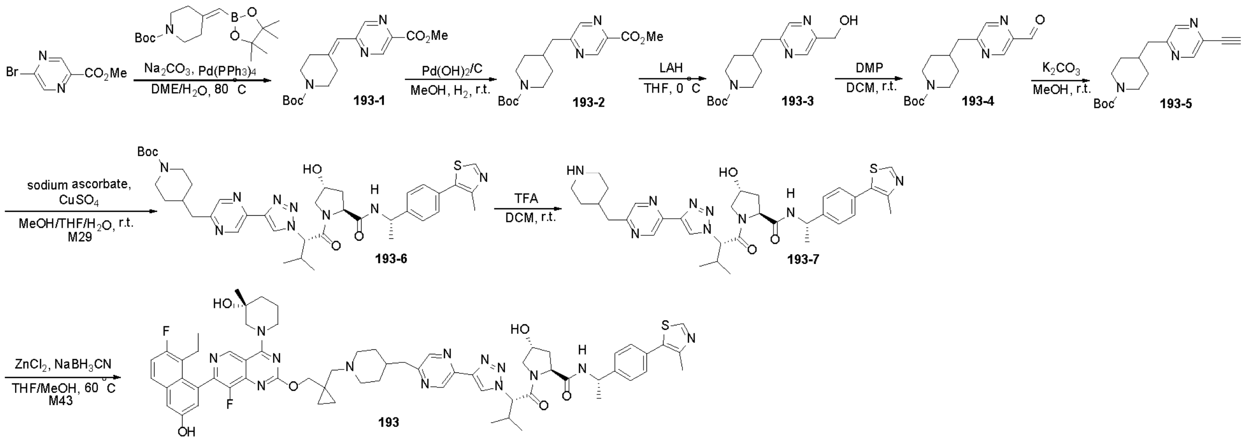


Step 1: Synthesis of compound 193-1

Methyl 5-bromopyrazine-2-carboxylate (4.0 g), tert-butyl 4-[(4,4,5,5-tetramethyl-1,3,2-dioxaborolan-2-yl) methylene)piperidine-1-carboxylate (7.2 g), tetrakis(triphenylphosphine)palladium (2.1 g), and sodium carbonate (3.9 g) were dissolved in a mixed solvent of DME (40 mL) and water (10 mL). The mixture was reacted under nitrogen protection at 80°C for 6 h. The reaction was monitored by LCMS until it was completed. The reaction solution was directly concentrated. DCM (30 mL) was added to the reaction solution, and the mixture was washed with saturated brine once. The organic phase was dried with anhydrous sodium sulfate, filtered, concentrated, separated and purified by column chromatography to obtain 1.9 g of a yellow solid, i.e. compound 193-1. ESI-MS m/z: 234 $[M+H]^+$.

Step 2: Synthesis of compound 193-2

Compound 193-1 (1.9 g) and palladium hydroxide/carbon (240.0 mg) were dissolved in MeOH (30 mL). The mixture was reacted under hydrogen atmosphere at room temperature for 12 h. The reaction was monitored by LCMS until it was completed. The reaction solution was directly filtered by suction. The resultant was concentrated, separated and purified by column chromatography to obtain 760.0 mg of a yellow solid, i.e. compound 193-2. ESI-MS m/z: 236 $[M+H]^+$.

Step 3: Synthesis of compound 193-3

Compound 193-2 (760.0 mg) was dissolved in THF (40 mL) under nitrogen protection. And lithium aluminum hydride (430.0 mg) was added in an ice water bath. The temperature of the reaction was maintained for 10 min, and the reaction was monitored by LCMS until it was completed. Water/sodium hydroxide solution/water (1/1/3 mL) was added to the reaction solution, and the resultant was filtered by suction. DCM (30 mL) was added, and the resultant was washed with saturated brine once. The organic phase was dried with anhydrous sodium sulfate, filtered, concentrated, separated and purified by column chromatography to obtain 126.0 mg of a yellow solid, i.e. compound 193-3. ESI-MS m/z: 208 $[M+H]^+$.

Step 4: Synthesis of compound 193-4

Compound 193-3 (126.0 mg) and DMP (173.8 mg) were dissolved in DCM (10 mL), and the mixture was reacted at room temperature for 1 h. The reaction was monitored by LCMS until it was completed. Saturated sodium bicarbonate solution (10 mL) was added to the reaction solution, followed by suction filtration. DCM (10 mL) was added, and the mixture was washed with saturated brine once. The organic phase was dried with anhydrous sodium sulfate, filtered, concentrated, separated and purified by column chromatography to obtain 75.0 mg of a yellow solid, i.e. compound 193-4. ESI-MS m/z: 206 $[M+H]^+$.

Step 5: Synthesis of compound 193-5

Compound 193-4 (70.0 mg), dimethyl (1-diazo-2-oxopropyl)phosphonate (0.11 mL), and potassium carbonate (203.7 mg) were dissolved in MeOH (5 mL). The mixture was reacted at room temperature for 1 h. The reaction was monitored by LCMS until it was completed. The reaction solution was directly concentrated. DCM (10 mL) was added to the reaction solution, and the resultant was washed with saturated brine once. The organic phase was dried with anhydrous sodium sulfate, filtered, concentrated, separated and purified by column chromatography to obtain 50.0 mg of a white solid, i.e. compound 193-5. ESI-MS m/z: 202 $[M+H]^+$.

Step 6: Synthesis of compound 193-6

Compound 193-5 (50.0 mg), M29 (2S,4R)-1-((S)-2-azido-3-methylbutanoyl)-4-hydroxy-N-((S)-1-(4-(4-methylthiazol-5-yl)phenyl) ethyl)pyrrolidine-2-carboxamide (75.7 mg), copper sulfate (23.8 mg), and sodium ascorbate (85.5 mg) were dissolved in a mixed solvent of THF (2 mL)/t-BuOH (2 mL)/$H_2O$ (2 mL) under nitrogen protection. The mixture was reacted at room temperature for 1 h, and the reaction was monitored by LCMS until it was completed. DCM (10 mL) was added to the reaction solution, and the resultant was washed with saturated brine once. The organic phase was dried with anhydrous sodium sulfate, filtered, concentrated, separated and purified by column chromatography to obtain 66.0 mg of a pale yellow solid, i.e. compound 193-6. ESI-MS m/z: 758 $[M+H]^+$.

Step 7: Synthesis of compound 193-7

Compound 193-6 (66.0 mg) was added to DCM (3 mL) and TFA (1 mL), and the mixture was reacted at room temperature for 20 minutes. The reaction was monitored by LCMS until it was completed. The reaction solution was directly concentrated, and 1 M NaOH solution was added until pH = 10. The resultant was extracted with DCM (10 mL) and MeOH (1 mL), and wash with saturated brine once. The organic phase was dried with anhydrous sodium sulfate, filtered, and concentrated to obtain 55.0 mg of a white powder, i.e. compound 193-7. ESI-MS m/z: 658 $[M+H]^+$.

Step 8: Synthesis of compound 193

Compound 193-7 (55.0 mg) and M43 (R)-1-(((7-(8-ethyl-7-fluoro-3-hydroxynaphthalen-1-yl)-8-fluoro-4-(3-hydroxy-3-methylpiperidi n-1-yl)pyrido[4,3-d]pyrimidin-2-yl)oxy)methyl)cyclopropane-1-carboxamide (55.1 mg) were dissolved in MeOH (5 mL). 1 M zinc chloride in tetrahydrofuran solution (0.13 mL) was added, and the mixture was reacted at 40°C for 1.5 hours. Sodium cyanoborohydride (15.8 mg) was added to the reaction solution, and the reaction was reacted at 60°C overnight. The reaction was monitored by LCMS until it was completed. Saturated ammonium chloride solution (5 mL) was added to the reaction solution, and the organic phase was concentrated. DCM (10 mL) was added, and the resultant was washed with saturated brine once. The organic phase was dried with anhydrous sodium sulfate, filtered, concentrated, separated and purified by preparative liquid chromatography to obtain 16.5 mg of a white solid, i.e. compound 193.

ESI-MS m/z: 596 1/2$[M+2H]^+$.

$^1H$ NMR (500 MHz, Methanol-$d_4$) $\delta$ 9.21 (d, $J$ = 2.5 Hz, 1H), 9.14 (d, $J$ = 1.5 Hz, 1H), 8.87 (s, 1H), 8.68 (d, $J$ = 1.5 Hz, 1H), 8.49 (d, $J$ = 1.5 Hz, 1H), 7.68-7.65 (m, 1H), 7.45-7.37 (m, 5H), 7.32-7.29 (m, 2H), 7.27-7.22 (m, 2H), 7.06 (t, $J$ = 2.5 Hz, 1H), 5.43 (d, $J$ = 10.0 Hz, 1H), 5.07-5.02 (m, 1H), 4.58-4.40 (m, 5H), 4.28-4.21 (m, 2H), 3.94-3.85 (m, 2H), 3.68-3.59 (m, 2H), 3.50-3.44 (m, 2H), 3.09 (s, 2H), 2.77 (d, $J$ = 7.0 Hz, 2H), 2.67-2.61 (m, 2H), 2.48-2.41 (m, 4H), 2.21-2.13 (m, 3H), 2.03-1.93 (m, 4H), 1.89-1.76 (m, 4H), 1.66 (d, $J$ = 7.0 Hz, 1H), 1.60 (d, $J$ = 7.5 Hz, 1H), 1.53-1.50 (m, 3H), 1.29 (s, 3H), 1.18 (d, $J$ = 7.0 Hz, 1H), 0.85-0.81 (m, 6H), 0.71 (s, 2H), 0.48 (s, 2H).

Example 194: Synthesis of compound (2S,4R)-1-((2S)-2-(4-(5-(1-(4-((1-(((7-(8-ethyl-7-fluoro-3-hydroxynaphthalen-1-yl)-8-fluoro-4-(( R)-3-hydroxy-3-methylpiperidin-1-yl)pyrido[4,3-d]pyrimidin-2-yl)oxy)methyl)cyclopropyl)meth yl)piperidin-1-yl)ethyl)piperidin-2-yl)-1H-1,2,3-triazol-1-yl)-3-methylbutanoyl)-4-hydroxy-N-(( S)-1-(4-(4-methylthiazol-5-yl)phenyl)ethyl)pyrrolidine-2-carboxamide

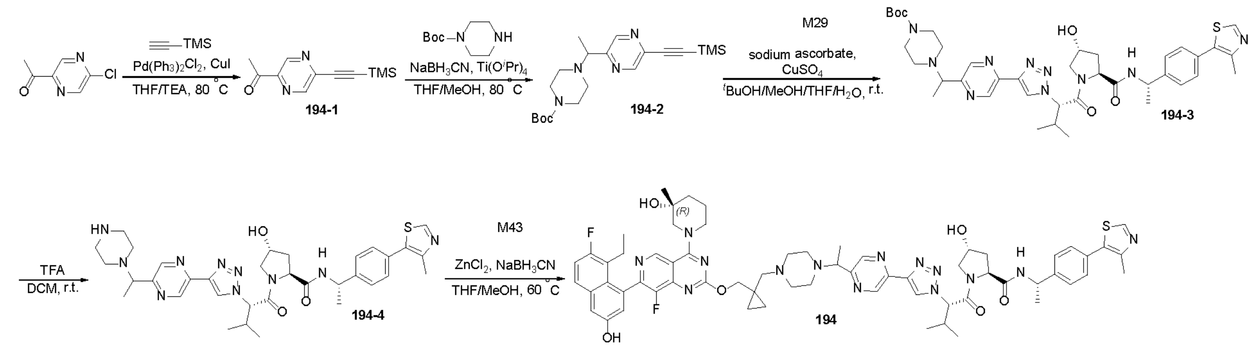


Step 1: Synthesis of compound 194-1

1-(5-Chloropyrazin-2-yl)ethanone (5.0 g), bis(triphenylphosphine)palladium(II) chloride (2.2 g), and cuprous iodide (1.2 g) were dissolved in TEA (44 mL) and THF (150 mL) under nitrogen protection. Trimethylsilylacetylene (13.5 mL) was added and the mixture was reacted at 80°C overnight. The reaction was monitored by LCMS until it was completed. The reaction solution was directly concentrated. EA (10 mL) was added to the reaction solution, and the resultant was washed with saturated brine once. The organic phase was dried with anhydrous sodium sulfate, filtered, concentrated, separated and purified by column chromatography to obtain 6.4 g of a brown solid, i.e. compound 194-1. ESI-MS m/z: 219 $[M+H]^+$.

Step 2: Synthesis of compound 194-2

Compound 194-1 (2.0 g), N-tert-butoxycarbonylpiperazine (1.9 g), and titanium tetraisopropanolate (4.1 mL) were dissolved in MeOH (50 mL) and THF (50 mL) under nitrogen protection. The mixture was reacted at 80°C for 2 h. Sodium cyanoborohydride (1.7 g) was added. The mixture was reacted at 80°C for 1 h, and the reaction was monitored by LCMS until it was completed. The reaction solution was directly concentrated. DCM (100 mL) was added to the reaction solution, and the resultant was washed with saturated brine once. The organic phase was dried with anhydrous sodium sulfate, filtered, concentrated, separated and purified by column chromatography to obtain 2.1 g of a brown solid, i.e. compound 194-2. ESI-MS m/z: 333 $[M+H]^+$.

Step 3: Synthesis of compound 194-3

Compound 194-2 (300.0 mg), M29 (2S,4R)-1-((S)-2-azido-3-methylbutanoyl)-4-hydroxy-N-((S)-1-(4-(4-methylthiazol-5-yl)phenyl) ethyl)pyrrolidine-2-carboxamide (235.0 mg), potassium carbonate (426.8 mg), copper sulfate (73.9 mg), and sodium ascorbate (265.1 mg) were dissolved in a mixed solvent of THF (5 mL)/iPrOH (5 mL)/MeOH (10 mL)/$H_2O$ (10 mL) under nitrogen protection. The mixture was reacted at room temperature for 3 hours. The reaction was monitored by LCMS until it was completed. DCM (10 mL) was added to the reaction solution, and the resultant was washed with saturated brine once. The organic phase was dried with anhydrous sodium sulfate, filtered, concentrated, separated and purified by column chromatography to obtain 201.0 mg of a pale yellow solid, i.e. compound 194-3. ESI-MS m/z: 773 $[M+H]^+$.

Step 4: Synthesis of compound 194-4

Compound 194-3 (100.0 mg) was added to DCM (10 mL) and TFA (3 mL), and the mixture was reacted at room temperature for 20 minutes. The reaction was monitored by LCMS until it was completed. The reaction solution was directly concentrated, and 1 M NaOH solution was added until pH = 10. The resultant was extracted with DCM (10 mL) and MeOH (1 mL), and washed with saturated brine once. The organic phase was dried with anhydrous sodium sulfate, filtered, and concentrated to obtain 70.1 mg of a white powder, i.e. compound 194-4. ESI-MS m/z: 673 $[M+H]^+$.

Step 5: Synthesis of compound 194

Compound 194-4 (70.1 mg) and M43 (R)-1-(((7-(8-ethyl-7-fluoro-3-hydroxynaphthalen-1-yl)-8-fluoro-4-(3-hydroxy-3-methylpiperidi n-1-yl)pyrido[4,3-d]pyrimidin-2-yl)oxy)methyl)cyclopropane-1-carboxamide (68.5 mg) were dissolved in MeOH (5 mL). 1 M zinc chloride in tetrahydrofuran solution (0.16 mL) was added, and the mixture was reacted at 40°C for 1.5 hours. Sodium cyanoborohydride (19.6 mg) was added to the reaction solution, and the reaction was reacted at 60°C overnight. The reaction was monitored by LCMS until it was completed. Saturated ammonium chloride solution (5

mL) was added to the reaction solution. The organic phase was concentrated, and DCM (10 mL) was added. The resultant was washed with saturated brine once, dried with anhydrous sodium sulfate, filtered, and concentrated. The resultant was separated and purified by preparative liquid chromatography to obtain 22.7 mg of a white solid, i.e. compound 194.

ESI-MS m/z: 603.8 1/2[M+2H]$^+$.

$^1$H NMR (500 MHz, Methanol-$d_4$) δ 9.20-9.17 (m, 2H), 8.87 (s, 1H), 8.72-8.70 (m, 1H), 8.65 (t, *J* = 1.5 Hz, 1H), 7.68-7.65 (m, 1H), 7.45-7.32 (m, 4H), 7.30-7.29 (m, 1H), 7.26-7.21 (m, 1H), 7.08-7.06 (m, 1H), 5.45-5.42 (m, 1H), 5.06-5.01 (m, 1H), 4.58-4.52 (m, 2H), 4.47-4.34 (m, 4H), 4.23-4.14 (m, 2H), 3.93-3.86 (m, 2H), 3.78-3.75 (m, 1H), 3.62-3.55 (m, 1H), 3.45-3.40 (m, 1H), 2.66-2.56 (m, 4H), 2.42-2.33 (m, 2H), 2.20-2.15 (m, 3H), 2.07-1.93 (m, 2H), 1.89-1.59 (m, 8H), 1.52-1.50 (m, 3H), 1.46 (d, *J* = 7.0 Hz, 3H), 1.33-1.32 (m, 5H), 1.23 (d, *J* = 1.5 Hz, 2H), 1.17-1.16 (m, 3H), 0.91-0.77 (m, 13H), 0.69 (s, 2H), 0.48 (s, 2H).

Example 195: Synthesis of compound (2S,4R)-1-((S)-2-(4-(5-(1'-(1-(7-(8-ethyl-7-fluoro)-3-hydroxynaphthalen-1-yl)-8-fluoro-4-((R)-3-hydroxy-3-methylpiperidin-1-yl)pyrido[4,3-d]pyrimidin-2-yl)oxy)methyl)cyclopropyl)methyl)-[ 1,4'-bipiperidin]-4-yl)pyrazin-2-yl)-1H-1,2,3-triazol-1-yl)-3-methylbutanoyl)-4-hydroxy-N-((S)-1-(4-(4-methylthiazol-5-yl)phenyl) ethyl)pyrrolidine-2-carboxamide

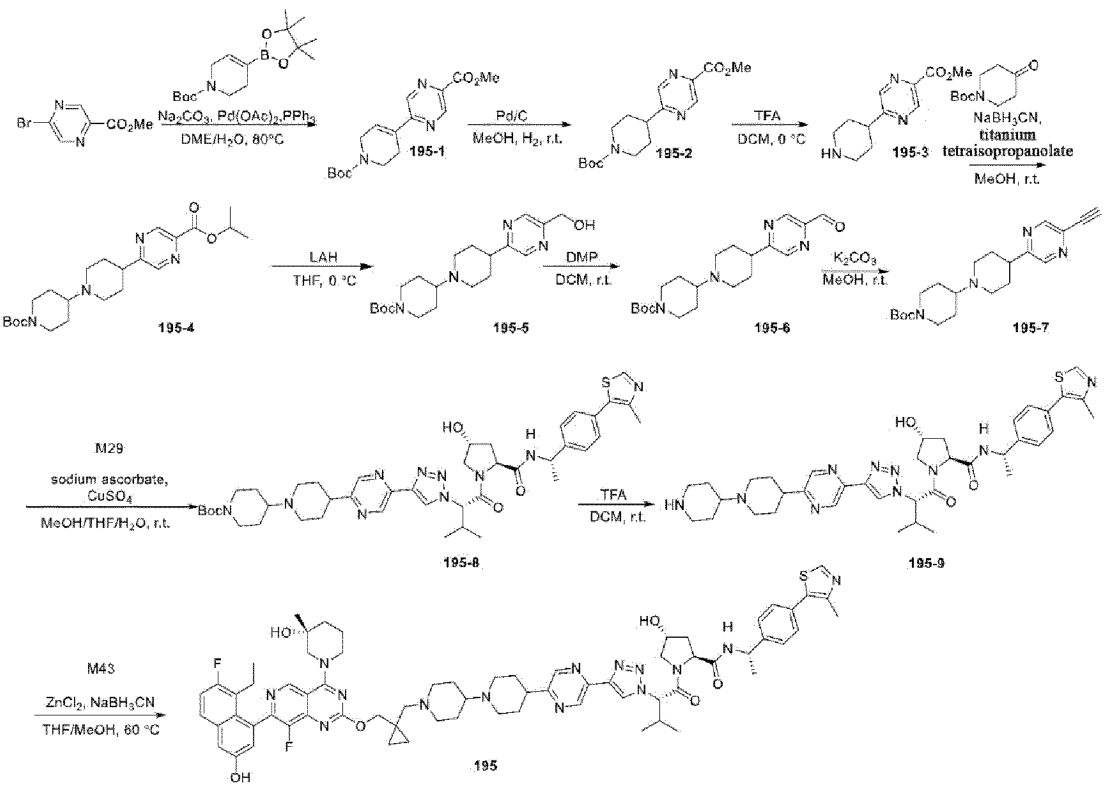


Step 1: Synthesis of compound 195-1

Methyl 5-bromopyrazine-2-carboxylate (5.0 g), N-tert-butoxycarbonyl-1,2,5,6-tetrahydropyridine-4-boronic acid pinacol ester (8.6 g), triphenylphosphine (1.2 g), palladium acetate (1.0 g), and sodium carbonate (4.9 g) were dissolved in a mixed solvent of DME (100 mL) and water (20 mL) under nitrogen protection. The mixture was reacted at 80°C for 3 h, and the reaction was monitored by LCMS until it was completed. The reaction solution was directly concentrated. DCM (30 mL) was added to the reaction solution, and the mixture was washed with saturated brine once. The organic phase was dried with anhydrous sodium sulfate, filtered, concentrated, separated and purified by column chromatography to obtain compound 195-1 (3.4 g). ESI-MS m/z: 320.3 [M+H] $^+$.

Step 2: Synthesis of compound 195-2

Compound 195-1 (3.4 g) and palladium/carbon (680.0 mg) were dissolved in MeOH (30 mL). The mixture was reacted under hydrogen atmosphere at room temperature for 12 h. The reaction was monitored by LCMS until it was

completed. The reaction solution was directly filtered by suction. The resultant was concentrated, separated and purified by column chromatography to obtain compound 195-2 (1.6 g). ESI-MS m/z: 322.2 [M+H] $^+$.

Step 3: Synthesis of compound 195-3

Compound 195-2 (1.6g) was dissolved in dichloromethane (10 mL), and trifluoroacetic acid (10 mL) was added. The mixture was reacted at 25°C for 1 hour. After the reaction was completed, the solvent was poured out, washed with dichloromethane twice then poured out. The resultant was dissolved in water, neutralized with sodium bicarbonate solution, extracted with dichloromethane/isopropanol (10:1), dried, and concentrated to obtain compound 195-3 (0.9 g). ESI-MS m/z: 222.2 [M+H]$^+$.

Step 4: Synthesis of compound 195-4

Compound 195-3 (0.9 g), N-tert-butoxycarbonyl-4-piperidone (0.7 g), and titanium tetraisopropanolate (1.9 mL) were dissolved in methanol (10 mL). After the reaction was reacted at 60°C for 1 hour, sodium cyanoborohydride (1 g) was added in batches. After the reaction was completed, ethyl acetate and water were added. The resultant was extracted three times, dried with anhydrous sodium sulfate, filtered and concentrated to obtain a crude product. The crude product was purified by column chromatography to obtain compound 195-4 (670 mg). ESI-MS m/z: 433.2 [M+H]

Step 5: Synthesis of compound 195-5

Compound 195-4 (630.0 mg) was dissolved in THF (10 mL) under nitrogen protection. And lithium aluminum hydride (120.0 mg) was added in an ice water bath. The temperature of the reaction was maintained for 10 min, and the reaction was monitored by LCMS until it was completed. Water/sodium hydroxide solution/water (0.12/0.12/0.36 mL) was added to the reaction solution, and the resultant was filtered by suction. DCM (30 mL) was added, and the resultant was washed with saturated brine once. The organic phase was dried with anhydrous sodium sulfate, filtered, concentrated, separated and purified by column chromatography to obtain compound 195-5 (380.0 mg). ESI-MS m/z: 377.3 [M+H] $^+$.

Step 6: Synthesis of compound 195-6

Compound 195-5 (355 mg) and DMP (355 mg) were dissolved in DCM (10 mL), and the mixture was reacted at room temperature for 1 h. The reaction was monitored by LCMS until it was completed. Saturated sodium bicarbonate solution (10 mL) was added to the reaction solution, followed by suction filtration. DCM (10 mL) was added, and mixture was washed with saturated brine once. The organic phase was dried with anhydrous sodium sulfate, filtered, concentrated, separated and purified by column chromatography to obtain compound 195-6 (121 mg). ESI-MS m/z: 375.1 [M+H] $^+$.

Step 7: Synthesis of compound 195-7

Compound 195-6 (121.0 mg), dimethyl (1-diazo-2-oxopropyl)phosphonate (93 mg), and potassium carbonate (134 mg) were dissolved in MeOH (5 mL). The mixture was reacted at room temperature for 1 h. The reaction was monitored by LCMS until it was completed. The reaction solution was directly concentrated. DCM (10 mL) was added to the reaction solution, and the resultant was washed with saturated brine once. The organic phase was dried with anhydrous sodium sulfate, filtered, concentrated, separated and purified by column chromatography to obtain compound 195-7 (63 mg). ESI-MS m/z: 371.1 [M+H] $^+$.

Step 8: Synthesis of compound 195-8

Compound 195-7 (46 mg), M29 (2S,4R)-1-((S)-2-azido-3-methylbutanoyl)-4-hydroxy-N-((S)-1-(4-(4-methylthiazol-5-yl)phenyl) ethyl)pyrrolidine-2-carboxamide (57 mg), copper sulfate (18 mg), and sodium ascorbate (64 mg) were dissolved in a mixed solvent of THF (1 mL)/t-BuOH (1 mL)/$H_2O$ (1 mL) under nitrogen protection. The mixture was reacted at room temperature for 1 h, and the reaction was monitored by LCMS until it was completed. DCM (10 mL) was added to the reaction solution, and the resultant was washed with saturated brine once. The organic phase was dried with anhydrous sodium sulfate, filtered, concentrated, separated and purified by column chromatography to obtain compound 195-8 (53 mg). ESI-MS m/z: 827.2 [M+H] $^+$.

Step 9: Synthesis of compound 195-9

Compound 195-8 (53 mg) was added to DCM (3 mL) and TFA (1 mL), and the mixture was reacted at room temperature for 20 minutes. The reaction was monitored by LCMS until it was completed. The reaction solution was directly concentrated, and 1 M NaOH solution was added until pH = 10. The resultant was extracted with DCM (10 mL) and MeOH (1 mL), and washed with saturated brine once. The organic phase was dried with anhydrous sodium sulfate, filtered, and concentrated to obtain compound 195-9 (35 mg). ESI-MS m/z: 727.1 $[M+H]^+$.

Step 10: Synthesis of compound 195

Compound 195-9 (35.0 mg) and M43 (R)-1-(((7-(8-ethyl-7-fluoro-3-hydroxynaphthalen-1-yl)-8-fluoro-4-(3-hydroxy-3-methylpiperidi n-1-yl)pyrido[4,3-d]pyrimidin-2-yl)oxy)methyl)cyclopropane-1-carboxamide (26 mg) were dissolved in MeOH (5 mL). 1 M zinc chloride in tetrahydrofuran solution (0.13 mL) was added, and the mixture was reacted at 40°C for 1.5 hours. Sodium cyanoborohydride (15.8 mg) was added to the reaction solution, and the mixture was continued to react at 60°C for 8 h. The reaction was monitored by LCMS until it was completed. Saturated ammonium chloride solution (5 mL) was added to the reaction solution, and the organic phase was concentrated. DCM (10 mL) was added, and the resultant was washed with saturated brine once. The organic phase was dried with anhydrous sodium sulfate, filtered, and concentrated. The resultant was separated and purified by preparative liquid chromatography to obtain 4.1 mg of a white solid, i.e. compound 195.

ESI-MS m/z: 630.6 1/2$[M+2H]^+$.

Example 196: Synthesis of compound (2S,4R)-1-((S)-2-(4-(5-(4-(1-(((7-(8-ethyl-7-fluoro-3-hydroxynaphthalen-1-yl)-8-fluoro-4-(((R)-3-hydroxy-3-methylpiperidin-1-yl)pyrido[4,3-d]pyrimidin-2-yl)oxy)methyl)cyclopropyl)methyl) piperidin-4-yl)piperazin-1-yl)pyrazin-2-yl)-1H-1,2,3-triazol-1-yl)-3-methylbutanoyl)-4-hydroxy-N-(((S)-1-(4-(4-methylthiazol-5-yl) phenyl)ethyl)pyrrolidine-2-carboxamide

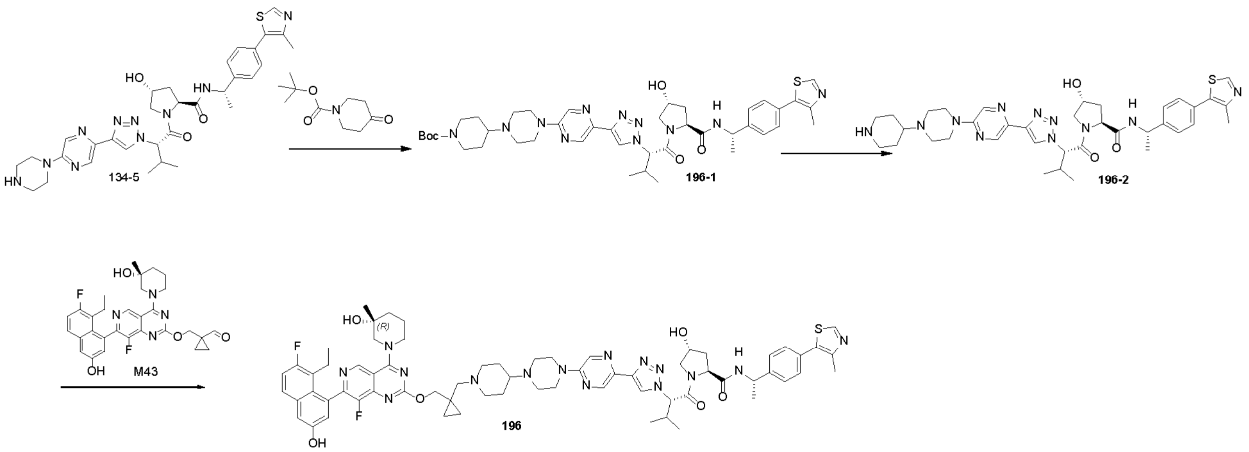


Step 1: Synthesis of compound 196-1

To a solution of compound 134-5 (75.00 mg, 0.12 mmol) and tert-butyl 4-oxopiperidine-1-carboxylate (34.77 mg, 0.17 mmol) in methanol (2.50 mL), 1 M zinc chloride in tetrahydrofuran solution (0.23 mL) was added. The mixture was reacted at 40°C for 0.5 h. Then sodium cyanoborohydride (29.23mg, 0.47 mmol) was added, and the mixture was reacted at 40°C for 6 h. The reaction solution was added to 20mL of water, extracted with chloroform/isopropanol. The organic phase was dried, filtered, concentrated under reduced pressure, and the residue was purified by column chromatography (MeOH: DCM= 1:11) to obtain 70.00 mg of a brown solid, i.e., compound 196-1. ESI-MS m/z: 828.30 $[M+H]^+$.

Step 2: Synthesis of compound 196-2

To a solution of compound 196-1 (75.00 mg, 0.09 mmol) in dichloromethane (1.50 mL), trifluoroacetic acid (0.34 mL, 4.53 mmol) was added. The mixture was reacted at 20°C for 0.5 h. The reaction solution was added to 20 mL of saturated sodium bicarbonate, extracted with chloroform/isopropanol. The organic phase was dried, filtered and concentrated under reduced pressure to obtain 63.00 mg of a colorless oil, i.e. compound 196-2. ESI-MS m/z: 728.20 $[M+H]^+$.

Step 3: Synthesis of compound 196

To a solution of compound 196-2 (65.00 mg, 0.09 mmol) and M43 (53.87 mg, 0.10 mmol) in methanol (2.50 mL), 1 M zinc chloride in tetrahydrofuran solution (0.18 mL) was added. The mixture was reacted at 40°C for 1 h. And then sodium cyanoborohydride (22.45 mg, 0.36 mmol) was added and the mixture was reacted at 40°C for 16 h. The reaction solution was added to 20 mL of water, and the resultant was extracted with chloroform/isopropanol. The organic phases were combined, dried, filtered, and concentrated under reduced pressure. The resultant was purified by Pre-HPLC to obtain 34.00 mg of a white solid, i.e. compound 196.

ESI-MS m/z: 630.7 1/2$[M+2H]^+$.

$^1$H NMR (500 MHz, Methanol-$d_4$) $\delta$ 9.09 (s, 1H), 8.77 (s, 1H), 8.58 (s, 1H), 8.35 (s, 1H), 8.11 (s, 1H), 7.51-7.49 (m, 1H), 7.33-7.28 (m, 4H), 7.12 (s, 1H), 7.10-7.08 (m, 1H), 6.94 (s, 1H), 5.28-5.26 (d, *J* = 10.0 Hz, 1H), 4.95-4.94 (m, 1H), 4.44-4.30 (m, 5H), 4.16-4.11 (m, 1H), 3.82-3.74 (m, 2H), 3.56 (s, 2H), 3.47-3.35 (m, 2H), 3.06-3.02 (m, 2H), 2.57 (s, 6H), 2.17-2.08 (m, 6H), 1.83-1.67 (m, 8H), 1.42 (s, 6H), 1.23-1.17 (m, 5H), 1.05 (s, 3H), 0.73-0.72 (m, 3H).

Example 197: Synthesis of compound (2S,4R)-1-((S)-2-(4-(5-(4-(4-(1-((7-(8-ethyl-7-fluoro-3-hydroxynaphthalen-1-yl)-8-fluoro-4-((R) -3-hydroxy-3-methylpiperidin-1-yl)pyrido[4,3-d]pyrimidin-2-yl)oxy)methyl)cyclopropyl)methyl )piperazin-1-yl)piperidin-1-yl)pyrazin-2-yl)-1H-1,2,3-triazol-1-yl)-3-methylbutanoyl)-4-hydroxy -N-((S)-1-(4-(4-methylthiazol-5-yl)phenyl)ethyl)pyrrolidine-2-carboxamide

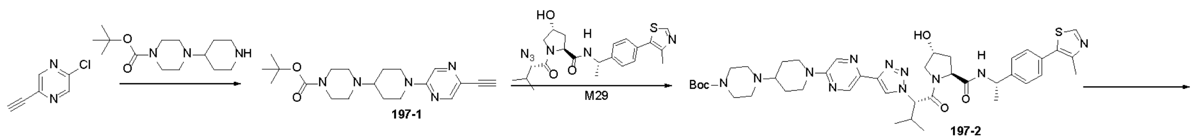


197-1

197-2

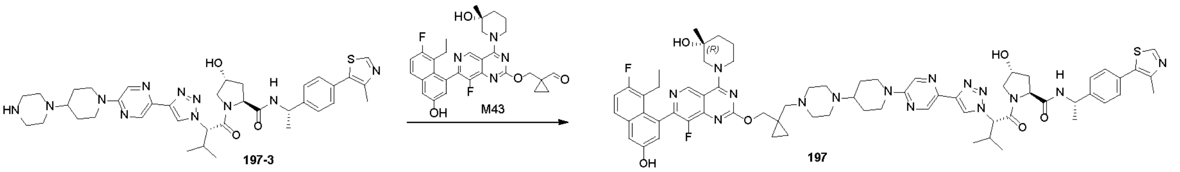


197-3

197

Step 1: Synthesis of compound 197-1

To a solution of 2-chloro-5-ethylpyrazine (200.00 mg, 1.44 mmol) and tert-butyl 4-(piperazin-1-yl)piperidine-1-carboxylate (466.63 mg, 1.73 mmol) in N,N-dimethylacetamide (5.00 mL), triethylamine (1.00 mL, 7.22 mmol) was added. The mixture was reacted at 100°C for 2 h. The reaction solution was cooled down to room temperature, then added to 30 mL of water. The resultant was extracted with EA. The organic phase was dried, filtered, and concentrated under reduced pressure. The resultant was purified by column chromatography to obtain 100.00 mg of a brown solid, i.e. compound 197-1. ESI-MS m/z: 372.3 $[M+H]^+$.

Step 2: Synthesis of compound 197-2

To a solution of compound 197-1 (100.00 mg, 0.27 mmol) and M29 (135.19 mg, 0.30 mmol) in tert-butanol (3.00 mL) and tetrahydrofuran (3.00 mL), copper sulfate (42.97 mg, 0.27 mmol) and water (1.50 mL) containing sodium ascorbate (159.99 mg, 0.81 mmol) were added. The mixture was reacted at 20°C for 1 h. The reaction solution was added to 30 mL of water. The resultant was extracted with DCM. The organic phase was combined, dried, filtered, and concentrated under reduced pressure. The resultant was purified by column chromatography to obtain 110.00 mg of a brown solid, i.e. compound 197-2. ESI-MS m/z: 828.3 $[M+H]^+$.

Step 3: Synthesis of compound 197-3

To a solution of compound 197-2 (105.00 mg, 0.13 mmol) in dichloromethane (2.50 mL), trifluoroacetic acid (0.47 mL, 6.34 mmol) was added. The mixture was reacted at 20°C for 0.5 h. The reaction solution was added to 30 mL of

saturated sodium bicarbonate, and extracted with DCM. The organic phase was combined, dried, filtered and concentrated under reduced pressure to obtain 80.00 mg of a brown solid, i.e. compound 197-3, which was directly used for the next step. ESI-MS m/z: 728.1 [M+H] $^+$.

Step 4: Synthesis of compound 197

To a solution of compound 197-3 (90.00 mg, 0.12 mmol) and M43(74.61 mg, 0.14 mmol) in methanol (3.00 mL), 1 M zinc chloride in tetrahydrofuran solution (0.25 mL) was added. The mixture was reacted at 40°C for 1 h. And then sodium cyanoborohydride (31.07 mg, 0.49 mmol) was added and the mixture was reacted at 40°C for 16 h. The reaction solution was added to 20 mL of water, and the resultant was extracted with chloroform/isopropanol. The organic phases were combined, dried, filtered, concentrated under reduced pressure, and purified by Pre-HPLC to obtain 60.00 mg of a white solid, i.e., compound 197.

ESI-MS m/z: 630.7 1/2[M+2H]$^+$.
$^1$H NMR (500 MHz, Methanol-$d_4$) δ 9.21 (s, 1H), 8.87 (s, 1H), 8.66 (s, 1H), 8.44 (s, 1H), 8.22 (s, 1H), 7.67-7.64 (m, 1H), 7.45-7.38 (m, 4H), 7.29 (s, 1H), 7.25-7.21 (m, 1H), 7.06 (s, 1H), 5.38-5.36 (d, *J* = 10.0 Hz, 1H), 4.95-4.94 (m, 1H), 4.44-4.30 (m, 5H), 4.16-4.11 (m, 1H), 3.82-3.74 (m, 2H), 3.56 (s, 2H), 3.47-3.35 (m, 2H), 3.06-3.02 (m, 2H), 2.57 (s, 6H), 2.17-2.08 (m, 6H), 1.83-1.67 (m, 8H),1.42 (s, 611),1.23-1.17 (m, 5H),1.05 (s, 3H), 0.73-0.72 (m, 3H).

Example 198: Synthesis of compound (2S,4R)-1-((S)-2-(4-(5-(((1-((1-((7-(8-ethyl-7-fluoro-3-hydroxynaphthalen-1-yl)-8-fluoro-4-((R)-3-hydroxy-3-methylpiperidin-1-yl)pyrido[4,3-d]pyrimidin-2-yl)oxy)methyl)cyclopropyl)methyl) piperidin-4-yl)oxy)methyl)pyrazin-2-yl)-1H-1,2,3-triazol-1-yl)-3-methylbutanoyl)-4-hydroxy-N-((S)-1-(4-(4-methylthiazol-5-yl) phenyl)ethyl)pyrrolidine-2-carboxamide

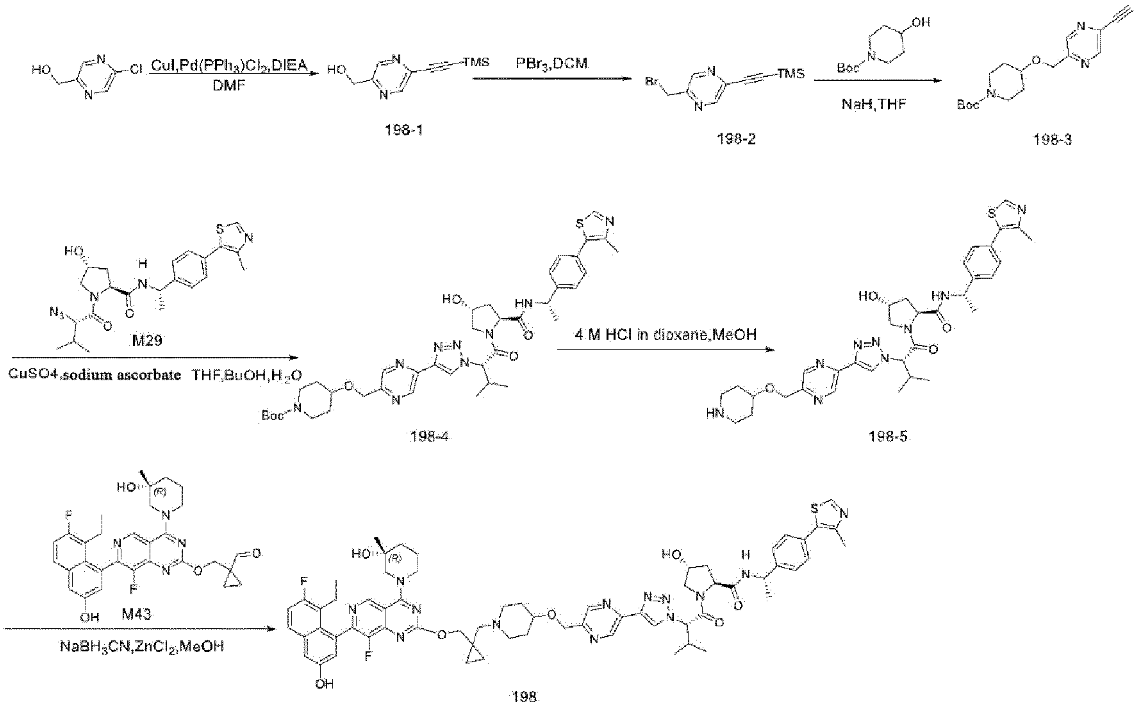


Step 1: Synthesis of compound 198-1

Compound (5-chloropyrazin-2-yl)methanol (2.0 g) was added to a 25 mL single-necked flask and dissolved in DMF (10 mL). Then, trimethylsilylacetylene (2.1 g), CuI (383.8 mg), $PdCl_2(PPh_3)_2$ (708.1 mg), and DIPEA (4.5 mL) were added. The mixture was heated to 50°C under nitrogen protection for 3 h. Saturated brine (5 mL) was added to the reaction solution and the mixture was extracted with EA. The organic phase was separated and dried with anhydrous sodium sulfate. The resultant was separated and purified by column chromatography (PE:EA = 3:1) to obtain 1.49 g of a pale yellow solid, i.e. compound 198-1.

Step 2: Synthesis of compound 198-2

Compound 198-1 (1.0 g) was added to a 25 mL single-necked flask and dissolved in DCM (10 mL). Then, $PBr_3$ (0.68 mL) was added. The mixture was reacted at room temperature for 2 h. The reaction solution was directly evaporated to dryness. The resultant was separated and purified by column chromatography (PE:EA = 4:1) to obtain 565.4 mg of a pale yellow solid, i.e. compound 198-2.

Step 3: Synthesis of compound 198-3

Compound 198-2 (565.4 mg) was added to a 25 mL single-necked flask and dissolved in THF (10 mL). Then, NaH (252.2 mg) and N-tert-butoxycarbonyl-4-hydroxypiperidine (464.7 mg) were added. The mixture was heated to 50°C and reacted for 5 h. The reaction solution was directly evaporated to dryness. The resultant was separated and purified by column chromatography (PE:EA = 5:1) to obtain 351.8 mg of a pale yellow solid, i.e. compound 198-3.

Step 4: Synthesis of compound 198-4

Compound M29 (100.0 mg) was added to a 25 mL single-necked flask and dissolved in THF (2 mL). Then compound 198-3 (76.7 mg), copper sulfate (35.2 mg), water (2 mL), tert-butanol (2 mL), and sodium ascorbate (188.0 mg) were added. The mixture was reacted at room temperature for 1 h. The reaction solution was directly evaporated to dryness. The resultant was separated and purified by column chromatography (DCM:MeOH = 12:1) to obtain 105.6 mg of a pale yellow solid, i.e. compound 198-4.

Step 5: Synthesis of compound 198-5

Compound 198-4 (105.6 mg) was added to a 25 mL single-necked flask and dissolved in 4 M hydrogen chloride in dioxane solution (5 mL). Then, nethanol (1 mL) was added. The mixture was reacted at room temperature for 1 h. The reaction solution was directly evaporated to dryness. Then, 5 mL of saturated sodium bicarbonate solution was added to the reaction solution. After adjusting the pH of the solution to 7-8, the resultant was extracted with dichloromethane/isopropanol, and the organic phase was separated and dried with anhydrous sodium sulfate to obtain 60.1 mg of a yellow solid, i.e. compound 198-5.

Step 6: Synthesis of compound 198

Compound 198-5 (60.1 mg) was added to a 25 mL single-necked flask, and dissolved in MeOH (5 mL). M43 (54.6 mg) and 1 M zinc chloride in tetrahydrofuran solution (0.1 mL) were added. The mixture was heated to 40°C, and after the reaction for 2 h, $NaBH_3CN$ (25.2 mg) was added. The reaction was carried out overnight. The reaction solution was directly evaporated to dryness. The product was separated by Pre-HPLC ($CH_3CN/H_2O$ = 55%:45%) to obtain 11.4 mg of a white solid, i.e. compound 198 (96.63% purity).

LCMS: $1/2[M+2H]^+$ = 604.09

$^1H$ NMR (500 MHz, Methanol-$d_4$) δ 9.20 (d, J = 5.0 Hz, 1H), 8.86 (d, J = 7.2 Hz, 1H), 8.72 (s, 1H), 8.54 (d, J = 12.7 Hz, 1H), 8.17 (s, 1H), 7.71-7.62 (m, 1H), 7.48-7.35 (m, 4H), 7.29 (s, 1H), 7.23 (t, J = 9.2 Hz, 1H), 7.07 (s, 1H), 5.39 (d, J = 10.0 Hz, 1H), 5.12-5.00 (m, 3H), 4.58-4.41 (m, 5H), 4.24 (t, J = 13.5 Hz, 1H), 4.11 - 3.96 (m, 2H), 3.88 (dd, J = 27.5, 9.3 Hz, 2H), 3.61-3.54 (m, 2H), 2.90 (s, 2H), 2.61 (d, J = 6.5 Hz, 1H), 2.53 - 2.42 (m, 6H), 2.35 (s, 2H), 2.24-2.11 (m, 3H), 2.08-1.94 (m, 3H), 1.89-1.71 (m, 5H), 1.52 (d, J = 6.9 Hz, 3H), 1.36-1.24 (m, 4H), 1.16 (d, J = 6.5 Hz, 3H), 0.88-0.77 (m, 5H), 0.72 (s, 2H), 0.51 (s, 2H).

Example 199: Synthesis of compound (2S,4R)-1-((S)-2-(4-(5-(((1-((1-((7-(8-ethyl-7-fluoro-3-hydroxynaphthalen-1-yl)-8-fluoro-4-((R)-3-hydroxy-3-methylpiperidin-1-yl)pyrido[4,3-d]pyrimidin-2-yl)oxy)methyl)cyclopropyl)methyl) piperidin-4-yl)methyl)amino)pyrazin-2-yl)-1H-1,2,3-triazol-1-yl)-3-methylbutanoyl)-4-hydroxy-N-((S)-1-(4-(4-methylthiazol-5-yl)phenyl)ethyl)pyrrolidine-2-carboxamide

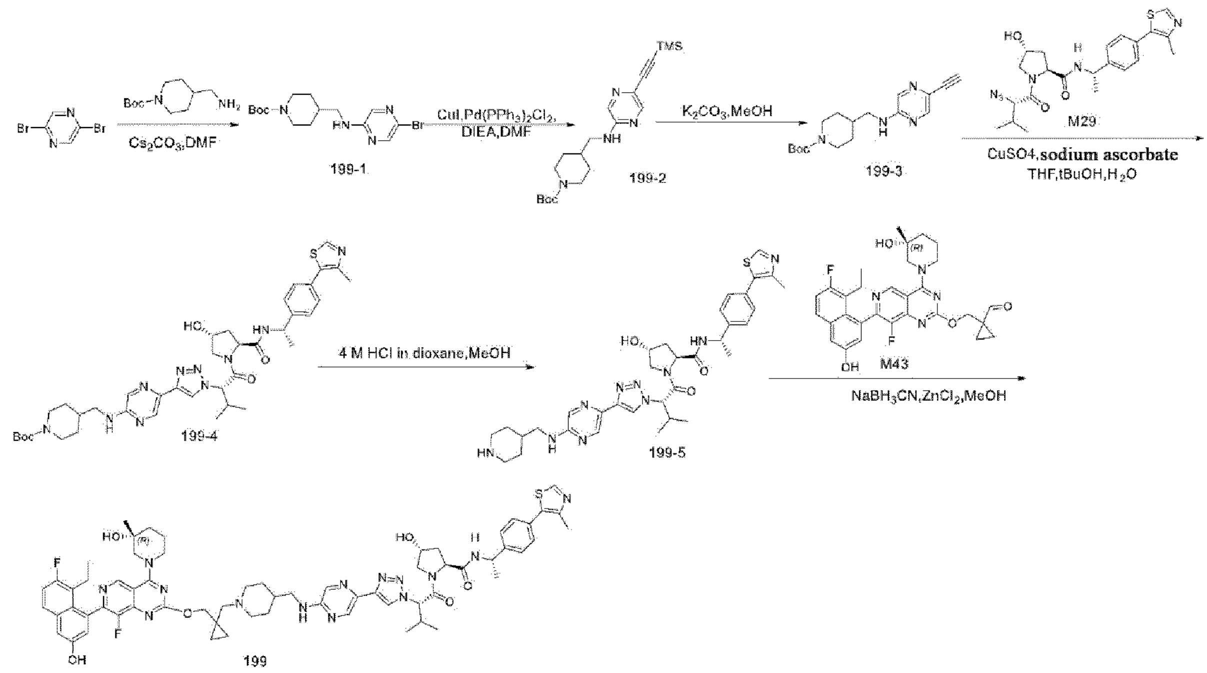


Step 1: Synthesis of compound 199-1

Compound 2,5-dibromopyrazine (1.0 g) was added to a 50 mL single-necked flask and dissolved in DMF (20 mL). Then, 1-tert-butoxycarbonyl-4-(aminomethyl)piperidine (0.99 g) and $Cs_2CO_3$ (3.4 g) were added. The mixture was heated to 90°C for 5 h. Saturated brine (5 mL) was added to the reaction solution and the resultant was extracted with EA. The organic phase was separated and dried with anhydrous sodium sulfate. The resultant was separated and purified by column chromatography (PE:EA = 4:1) to obtain 1.2 g of a pale yellow solid, i.e. compound 199-1.

Step 2: Synthesis of compound 199-2

Compound 199-1 (1.2 g) was added to a 25 mL single-necked flask and dissolved in DMF (10 mL). Then, trimethylsilylacetylene (633.4 mg), CuI (108.3 mg), $PdCl_2(PPh_3)_2$ (197.6 mg), and DIPEA (2.4 mL) were added under nitrogen protection. The mixture was heated to 50°C and reacted for 3 h. Saturated brine (5 mL) was added to the reaction solution and the mixture was extracted with EA. The organic phase was separated and dried with anhydrous sodium sulfate. The resultant was separated and purified by column chromatography (PE:EA = 4:1) to obtain 891.6 mg of a pale yellow solid, i.e. compound 199-2.

Step 3: Synthesis of compound 199-3

Compound 199-2 (891.6 mg) was added to a 25 mL single-necked flask and dissolved in MeOH (10 mL). Then, $K_2CO_3$ (517.2 mg) was added. The mixture was reacted at room temperature for 2 h. The reaction solution was directly evaporated to dryness. The resultant was separated and purified by column chromatography (PE:EA = 4:1) to obtain 563.4 mg of a pale yellow solid, i.e. compound 199-3.

Step 4: Synthesis of compound 199-4

Compound M29 (100.0 mg) was added to a 25 mL single-necked flask and dissolved in THF (2 mL). Then compound 199-3 (76.3 mg), copper sulfate (35.2 mg), water (2 mL), tert-butanol (2 mL), and sodium ascorbate (188.0 mg) were added. The mixture was reacted at room temperature for 1 h. The reaction solution was directly evaporated to dryness. The resultant was separated and purified by column chromatography (DCM:MeOH = 12:1) to obtain 103.7 mg of a pale yellow solid, i.e. compound 199-4.

Step 5: Synthesis of compound 199-5

Compound 199-4 (103.7 mg) was added to a 25 mL single-necked flask and dissolved in 4M hydrogen chloride in

dioxane solution (5 mL). Then, methanol (1 mL) was added. The mixture was reacted at room temperature for 1 h. The reaction solution was directly evaporated to dryness. Then, 5 mL of saturated sodium bicarbonate solution was added to the reaction solution. After adjusting the pH of the solution to 7-8, the resultant was extracted with dichloromethane/isopropanol, and the organic phase was separated and dried with anhydrous sodium sulfate to obtain 64.3 mg of a yellow solid, i.e. compound 199-5.

Step 6: Synthesis of compound 199

Compound 199-5 (64.3 mg) was added to a 25 mL single-necked flask, and dissolved in MeOH (5 mL). M43 (50.7 mg) and 1 M zinc chloride in tetrahydrofuran solution (0.1 mL) were added. The mixture was heated to 40°C, and after the reaction for 2 h, $NaBH_3CN$ (25.1 mg) was added. The reaction was carried out overnight. The reaction solution was directly evaporated to dryness. The product was separated by Pre-HPLC ($CH_3CN/H_2O$ = 55%:45%) to obtain 22.9 mg of a white solid, i.e. compound 199 (96.63% purity).

LCMS: 1/2[M+2H]$^+$ = 603.70

$^1$H NMR (500 MHz, Methanol-$d_4$) $\delta$ 9.20 (d, J = 5.0 Hz, 1H), 8.86 (d, J = 7.2 Hz, 1H), 8.72 (s, 1H), 8.54 (d, J = 12.7 Hz, 1H), 8.17 (s, 1H), 7.71-7.62 (m, 1H), 7.48-7.35 (m, 4H), 7.29 (s, 1H), 7.23 (t, J = 9.2 Hz, 1H), 7.07 (s, 1H), 5.39 (d, J = 10.0 Hz, 1H), 5.12-5.00 (m, 3H), 4.58-4.41 (m, 5H), 4.24 (t, J = 13.5 Hz, 1H), 3.88 (dd, J = 27.5, 9.3 Hz, 2H), 3.61-3.54 (m, 2H), 2.90 (s, 2H), 2.84 - 2.73 (m, 2H), 2.61 (d, J = 6.5 Hz, 1H), 2.53 - 2.42 (m, 6H), 2.35 (s, 2H), 2.24-2.11 (m, 3H), 2.08-1.94 (m, 3H), 1.89-1.71 (m, 5H), 1.52 (d, J = 6.9 Hz, 3H), 1.36-1.24 (m, 4H), 1.16 (d, J = 6.5 Hz, 3H), 0.88-0.77 (m, 5H), 0.72 (s, 2H), 0.51 (s, 2H).

Example 200: Synthesis of compound (2S,4R)-1-((S)-2-(4-(5-(3-(1-(((7-(8-ethyl-7-fluoro-3-hydroxynaphthalen-1-yl)-8-fluoro-4-((R)-3 -hydroxy-3-methylpiperidin-1-yl)pyrido[4,3-d]pyrimidin-2-yl)-3-methylbutanoyl)-4-hydroxy-N-((S)-1-(4-(4-methylthiazol-5-yl)phenyl)ethyl)pyrrolidine-2-carboxamide

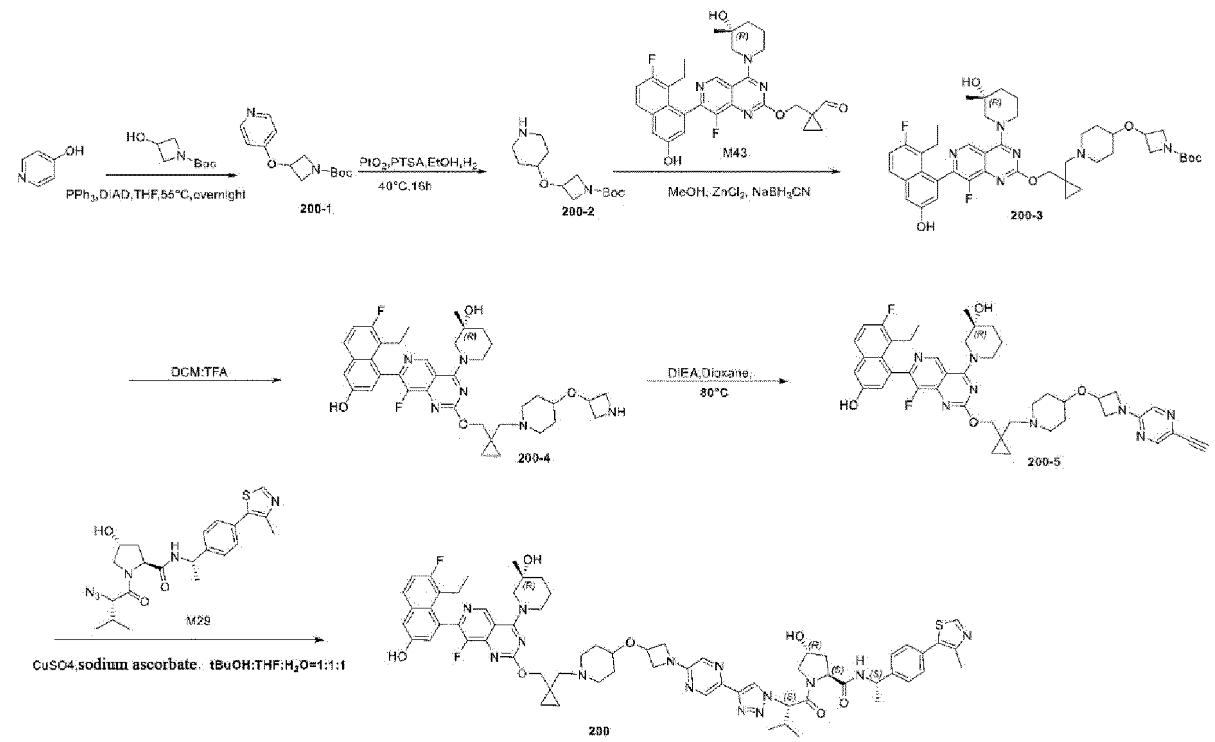


Step 1: Synthesis of compound 200-1

4-Hydroxypyridine (1 g) was dissolved in 10 mL of THF, then tert-butyl 3-hydroxy-azetidine-1-carboxylate (2.26 g) and triphenylphosphine (3.5 g) were added successively, and finally diisopropyl azodicarboxylate (2.6 g) was added dropwise. The mixture was stirred at 55°C for 15 h. The reaction was monitored by LCMS until it was completed. The reaction solution was concentrated and then separated and purified by column chromatography to obtain 2 g of a pale

yellow liquid, i.e. compound 200-1. ESI-MS m/z: 251 [M+H] +;

Step 2: Synthesis of compound 200-2

Compound 200-1 (1 g) was dissolved in ethanol (10 mL), and then p-toluenesulfonic acid (0.61 g) and platinum dioxide (0.27 g) were added successively. The mixture was degassed with $H_2$ three times, and stirred at 40°C for 16 h. The reaction was monitored by LCMS until it was completed. Icy sodium hydroxide aqueous solution (3 M) was added, and the resultant was filtered with diatomaceous earth. Ethanol was dried by rotary evaporation. The remaining aqueous phase was extracted with DCM:$NH_3$-MeOH = 4:1. The organic phase was combined, dried with sodium sulfate, and concentrated. The resultant was separated and purified by column chromatography to obtain 0.35 g of a pale yellow solid, i.e. compound 200-2. ESI-MS m/z: 257 [M+H] +.

Step 3: Synthesis of compound 200-3

Compound 200-2 (84 mg) and M43 (1-[[9-(8-ethyl-7-fluoro-3-hydroxy-naphthalen-1-yl)-10-fluoro-5-(3-hydroxy-3-methyl-1-piperidi nyl)-2,4,8-triazabicyclo[4.4.0]deca-2,4,6,8,10-pentaen-3-yl]oxymethyl]cyclopropane-1-carboxa mide (180.00 mg) were dissolved in methanol (2 mL). 1 M zinc chloride in n-hexane solution (0.49 mL) was added, and the mixture was heated to 40°C and reacted for 1 h. Then, sodium cyanoborohydride (62 mg) was added, and the mixture was reacted at 40°C for 12 h. The reaction was monitored by LCMS until it was completed. 5 mL of water was slowly added to the reaction solution for quenching, and the resultant was extracted with a mixture of DCM:isopropanol = 3:1 three times. The organic phases were combined, and dried with anhydrous sodium sulfate. The solvent was removed under reduced pressure, and then the resultant was separated and purified by column chromatography to obtain 180 mg of a white solid, i.e. compound 200-3. ESI-MS m/z: 789 [M+H]+.

Step 4: Synthesis of compound 200-4

Compound 200-3 (180 mg) was dissolved in 3 mL of DCM, and then 1 mL of TFA was added. The mixture was reacted at room temperature for 30 min. The reaction was monitored by LCMS until it was completed. The reaction solution was concentrated, dissolved by water, adjusted to alkaline with sodium carbonate aqueous solution, and extracted with DCM:isopropanol. The organic phase was combined, dried with sodium sulfate, filtered, concentrated, separated and purified by column chromatography to obtain 170 mg of a pale yellow solid, i.e. compound 200-4. ESI-MS m/z: 689 [M+H] +.

Step 5: Synthesis of compound 200-5

Compound 200-4 (150 mg) and 2-chloro-5-ethynylpyrazine (30 mg) were dissolved in 3 mL of 1,4-dioxane, and then TFA (0.09 mL) was added. The mixture was reacted at 80°C for 3h. The reaction was monitored by LCMS until it was completed. 10.0 mL of water was added to the reaction solution, and the resultant was extracted with EA three times, 10 mL each time. The organic phases were combined, dried with sodium sulfate, filtered, concentrated, separated and purified by column chromatography to obtain 84 mg of a pale yellow solid, i.e. compound 200-5. ESI-MS m/z: 791 [M+H] +.

Step 6: Synthesis of compound 200

Compound 200-5 (54 mg) and M29 (2S,4R)-1-((S)-2-azido-3-methylbutanoyl)-4-hydroxy-N-((S)-1-(4-(4-methylthiazol-5-yl)phenyl) ethyl)pyrrolidine-2-carboxamide (37 mg) were dissolved in 1.5 mL of THF, 1.5 mL of $H_2O$, and 1.5 mL of tert-butanol. Then sodium ascorbate (35 mg) and anhydrous copper sulfate (9.8 mg) were added successively. The mixture was reacted at room temperature for 0.5 h. The reaction was monitored by LCMS until it was completed. 20.0 mL of water was added to the reaction solution, and the resultant was extracted with DCM:isopropanol = 4:1 three times, 20 mL each time. The organic phases were combined, dried with sodium sulfate, filtered, concentrated, separated and purified by column chromatography to obtain 14 mg of a white solid, i.e. compound 200. ESI-MS m/z: 624 1/2[M+2H]+.

Example 201: Synthesis of compound (2S,4R)-1-((S)-2-(4-(5-(1-(1-((7-(8-ethyl-7-fluoronaphthalen-1-yl)-8-fluoro-4-((R)-((R)-3-hydro xy-3-methylpiperidin-1-yl)pyrido[4,3-d]pyrimidin-2-yl)oxy)methyl)cyclopropyl)methyl)piperidi n-4-yl)oxy)pyrazin-2-yl)-1H-1,2,3-triazol-1-yl)-3-methylbutanoyl)-4-hydroxy-N-((S)-1-(4-(4-me thylthiazol-5-yl)phenyl)ethyl) pyrrolidine-2-carboxamide

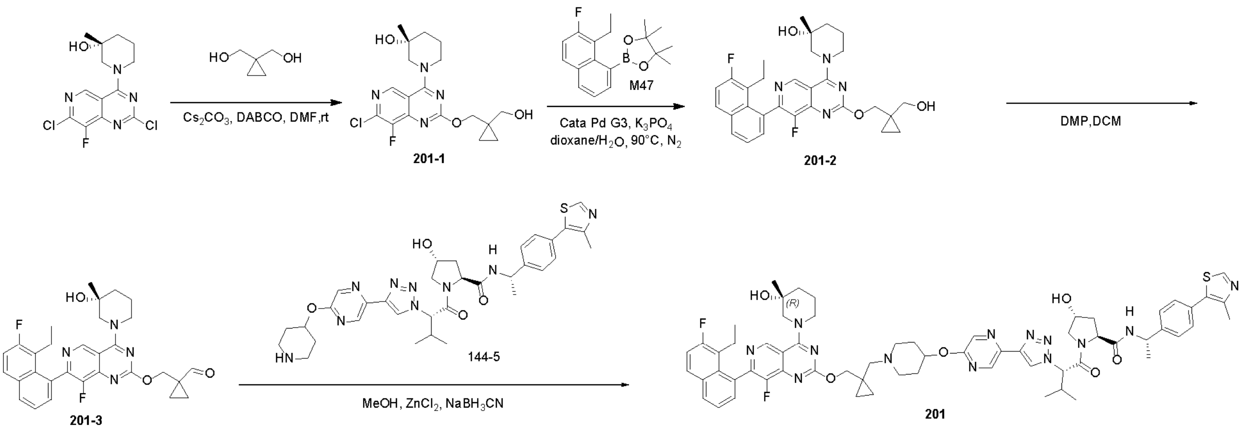


Step 1: Synthesis of compound 201-1

(R)-1-(2,7-dichloro-8-fluoropyrido[4,3-d]pyrimidin-4-yl)-3-methylpiperidin-3-ol (0.5 g), 1,1-cyclopropanedimethanol (0.31 g), cesium carbonate (0.98 g), and DABCO (0.03 g) were dissolved in 5 mL of DMF. The mixture was degassed with $N_2$ three times and reacted at 25°C for 12 h. The reaction was monitored by LCMS until it was completed. 20.0 mL of water was added to the reaction solution, and the resultant was extracted with EA three times, 20 mL each time. The organic phases were combined, dried with sodium sulfate, filtered, concentrated, and separated and purified by column chromatography to obtain 0.39 g of a pale yellow solid, i.e. compound 201-1. ESI-MS m/z: 397 $[M+H]^+$.

Step 2: Synthesis of compound 201-2

Compound 201-1 (0.39 g), M47 2-(8-ethyl-7-fluoronaphthalen-1-yl)-4,4,5,5-tetramethyl-1,3,2-dioxaborolane (0.32 g), potassium phosphate (0.62 g), and CataCXium A Pd G3 (71 mg) were dissolved in 4 mL of dioxane and 0.4 mL of water. The mixture was reacted at 90°C for 3 h. The reaction was monitored by LCMS until it was completed. 20.0 mL of water was added to the reaction solution, and the resultant was extracted with EA three times, 30 mL each time. The organic phases were combined, washed with saturated brine, dried with sodium sulfate, filtered, concentrated, and separated and purified by column chromatography to obtain 0.39 g of a pale yellow solid, i.e. compound 201-2. ESI-MS m/z: 535 $[M+H]^+$.

Step 3: Synthesis of compound 201-3

Compound 201-2 (0.39 g) was dissolved in 5 mL of DCM, and then Dess-Martin periodinane (0.37 g) was added. The mixture was reacted at room temperature for 1 h, and the reaction was monitored by LC-MS until it was completed. The reaction mixture was filtered with diatomaceous earth, and the filter cake was washed with 10 mL of DCM three times. The filtrate was collected, and the solvent was removed under reduced pressure. The resultant was separated and purified by column chromatography to obtain 0.27 g of a white solid, i.e. compound 201-3.

ESI-MS m/z: 533 $[M+H]^+$;

Step 4: Synthesis of compound 201

Compound 201-3 (57 mg) and compound 144-5 (2S,4R)-4-hydroxy-1-((S)-3-methyl-2-(4-(5-(piperidin-4-yloxy)pyrazin-2-yl)-1H-1,2,3-triazol-1-yl)butanoyl)-N-((S)-1-(4-(4-methylthiazol-5-yl)phenyl)ethyl)pyrrolidine-2-carboxamide (70 mg) were dissolved in methanol (2 mL). 1 M zinc chloride in n-hexane solution (0.16 mL) was added, and the mixture was heated to 40°C and reacted for 1 h. Then, sodium cyanoborohydride (20 mg) was added, and the mixture was reacted at 40°C for 12 h. The reaction was monitored by LCMS until it was completed. 5 mL of water was slowly added to the reaction solution for quenching, and the resultant was extracted with DCM: isopropanol = 3:1 three times. The organic phases were combined, and dried with anhydrous sodium sulfate. The solvent was removed under reduced pressure, and then the resultant was separated and purified by column chromatography to obtain 41.3mg of a white solid, i.e. compound 201.

ESI-MS m/z: 589 1/2$[M+2H]^+$.

Example 202: Synthesis of compound (2S,4R)-1-((2S)-2-(4-(3-((4-((1-((7-(8-ethyl-7-fluoro-3-hydroxynaphthalen-1-yl)-8-fluoro-4-(3-h ydroxy-3-methylpiperidin-1-yl)pyrido[4,3-d]pyrimidin-2-yl)oxy)methyl)cyclopropyl)methyl)pip erazin-1-yl)methyl)-1-methyl-1H-pyrazol-5-yl)-1H-1,2,3-triazol-1-yl)-3-methylbutanoyl)-4-hydr oxy-N-((S)-1-(4-(4-methylthiazol-5-yl)phenyl)ethyl)pyrrolidine-2-carboxamide

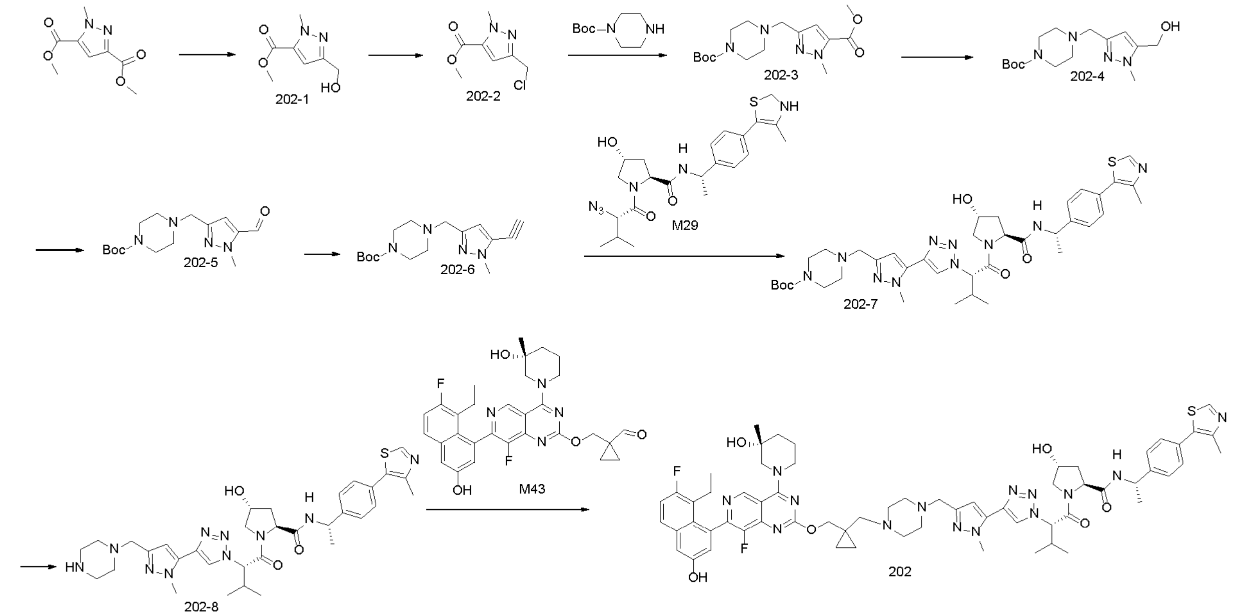

202-1, 202-2, 202-3, 202-4, 202-5, 202-6, M29, 202-7, 202-8, M43, 202

Step 1: Synthesis of compound 202-1

Compound dimethyl 1-methyl-1H-pyrazole-3,5-dicarboxylate (5.0 g) was dissolved in MeOH (100 mL). The mixture was cooled down to a temperature below 0°C in an ice bath. And then sodium borohydride (1.9 g) was added in batches. The reaction was stirred and reacted below 0°C for 2.0 h. After the reaction was completed, the resultant was quenched with saturated ammonium chloride solution, and extracted with ethyl acetate three times. The organic phases were combined and washed with saturated brine, dried with anhydrous sodium sulfate, filtered, concentrated and purified by column chromatography to obtain compound 202-1 (300 mg). ESI-MS m/z: 171.4 $[M+H]^+$.

Step 2: Synthesis of compound 202-2

Compound 202-1 (300 mg) was added to a 50 mL three-necked flask and dissolved in dichloromethane (10 mL). The mixture was cooled down to about 0°C in an ice bath. Thionyl chloride (10 mL) was added dropwise slowly, and the mixture was stirred at room temperature for 2.0 h. After the reaction was completed, the reaction solution was concentrated. The resultant was diluted with water, and extracted with ethyl acetate three times. The organic phases were collected and combined, washed with saturated brine, dried with anhydrous sodium sulfate, filtered, concentrated and purified by column chromatography (PE:EA = 5:1) to obtain compound 202-2 (260 mg). ESI-MS m/z: 189.1 $[M+H]^+$.

Step 3: Synthesis of compound 202-3

Compound 202-2 (260 mg), N-tert-butoxycarbonylpiperazine (290 mg), and DIPEA (535 mg) were added successively to a 50 mL three-necked flask. Then, acetonitrile (10 mL) was added to dissolve the mixture. The mixture was heated to 60°C and stirred for 4.0 h. After the reaction was completed, the reaction solution was concentrated. The resultant was diluted with water, and extracted with ethyl acetate three times. The organic phase was collected and combined, washed with saturated brine, dried with anhydrous sodium sulfate, filtered, concentrated and purified by column chromatography (PE:EA = 2:1) to obtain compound 202-3 (400 mg). ESI-MS m/z: 339.1 $[M+H]^+$.

Step 4: Synthesis of compound 202-4

Compound 202-3 (400 mg) was added to a 100 mL single-necked flask, then tetrahydrofuran (20 mL) was added.

The mixture was cooled down to below 0°C, and lithium aluminum hydride (134 mg) was slowly added in batches. The reaction was slowly moved to room temperature, then stirred and reacted for 1.0 h. After the reaction was completed, the resultant was cooled down to below 0°C. 0.2 mL of water was added dropwise slowly to the reaction solution, then 0.2 mL of sodium hydroxide aqueous solution (15% by mass) was slowly added dropwise. The resultant was stirred for 10 min, then 0.6 mL of water was slowly added dropwise to the reaction solution, and then anhydrous sodium sulfate was added. The resultant was stirred for 1.0 h then filtered, and the filtrate was dried by rotary evaporation. The concentrate was purified by column chromatography (DCM:MeOH = 10:1) to obtain compound 202-4 (300 mg). ESI-MS m/z: 311.3 $[M+H]^+$.

Step 5: Synthesis of compound 202-5

Compound 202-4 (300 mg) was added to a 100 mL single-necked flask, and dissolved in 10 mL of dichloromethane. DMP (615 mg) was added in batches, and the mixture was stirred at room temperature for 1.0 h. The resultant was quenched with saturated sodium thiosulfate aqueous solution, filtered, extracted with DCM twice, dried with anhydrous sodium sulfate, filtered, dried by rotary evaporation of filtrate, and concentrated to obtain crude compound 202-5 (300 mg). ESI-MS m/z: 309.4 $[M+H]^+$.

Step 6: Synthesis of compound 202-6

Compound 202-5 (300 mg) and dimethyl (1-diazo-2-oxopropyl)phosphonate (374 mg) were added successively in a 100 mL single-necked flask. The mixture was dissolved by methanol (20 mL), and then potassium carbonate (673 mg) was slowly added. The mixture was stirred at room temperature overnight. The reaction solution was diluted with water, and the resultant was extracted with EA twice. The organic phase was collected and combined, dried with anhydrous sodium sulfate, and the concentration was separated and purified by column chromatography (PE: EA = 3:1) to obtain compound 202-6 (200 mg). ESI-MS m/z: 205.4 $[M+H]^+$.

Step 7: Synthesis of compound 202-7

Compound 202-6 (200 mg), sodium ascorbate (260 mg), copper sulfate (105 mg) and M1 (300 mg) were added successively to a 50 mL single-necked flask. 3 mL of methanol, 3 mL of tetrahydrofuran and 3 mL of water were added, and the mixture was reacted at room temperature for 30 min. The solvent was dried by rotary evaporation, and extracted with EA twice. The organic phase was combined, dried with anhydrous sodium sulfate, and the concentrate was separated and purified by column chromatography (DCM:MeOH = 10:1) to obtain compound 202-7 (230 mg). ESI-MS m/z: 761.3 $[M+H]^+$.

Step 8: Synthesis of compound 202-8

Compound 202-7 (230 mg) was added to a 25 mL single-necked flask, and dissolved in 10 mL of dichloromethane. TFA (5 mL) was added, and the reaction was stirred at room temperature for 1.0 h. After the reaction was completed, the reaction solution was dried by rotary evaporation. The pH of the resultant was adjusted to 10.0 with saturated sodium bicarbonate solution. The resultant was extracted with dichloromethane/isopropanol (10:1) three times. The organic phases were combined, washed with saturated brine, dried with anhydrous sodium sulfate, filtered, and concentrated to obtain a crude compound 202-8 (180 mg). ESI-MS m/z: 661.4 $[M+H]^+$.

Step 9: Synthesis of compound 202

Compound 202-8 (90 mg), M43 (74 mg), and 1 M zinc chloride in tetrahydrofuran solution (0.6 mL) were dissolved in methanol (10 mL), and the mixture was reacted at 40°C for 1 hour. Then sodium cyanoborohydride (26 mg) was added, and the mixture was stirred and reacted at 70°C for 5 hours. After the reaction was completed, the resultant was cooled down to room temperature, quenched with ammonium chloride solution, extracted with dichloromethane and methanol (10:1), dried with anhydrous sodium sulfate, filtered, concentrated, and purified by column chromatography (DCM:MeOH = 85:15). And the crude product was purified by Pre-HPLC (C18 chromatographic column, A: 0.05% diethylamine aqueous solution, B: acetonitrile, concentration gradient: 30% B to 50% B, 6.3 min to 7.5 min, flow rate: 20 mL/min, 254nm) to obtain compound 202 (36 mg).

ESI-MS m/z: 597.72 1/2$[M+2H]^+$.

$^1$H NMR (500 MHz, DMSO-$d_6$) δ 9.21 (s, 1H), 8.99 (s, 1H), 8.65 (s, 1H), 8.54 (d, J = 7.6 Hz, 1H), 7.76 (dd, J = 8.9, 6.1 Hz, 1H), 7.48 - 7.42 (m, 2H), 7.40 - 7.29 (m, 4H), 7.04 (d, J = 2.5 Hz, 1H), 6.52 (d, J = 3.9 Hz, 1H), 5.41 - 5.30 (m, 1H), 5.34 - 5.12 (m, 1H), 4.91 (t, J = 7.1 Hz, 1H), 4.77 (d, J = 6.8 Hz, 1H), 4.40 (t, J = 8.1 Hz, 1H), 4.36 - 4.23 (m, 4H), 4.09 - 3.97 (m, 4H), 3.79 - 3.74 (m, 1H), 3.72 (d, J = 11.0 Hz, 1H), 3.64 - 3.58 (m, 1H), 3.52 (d, J = 13.2 Hz, 1H), 2.46 (d, J = 3.2 Hz, 4H), 2.38 - 2.25 (m, 6H), 2.19 - 2.04 (m, 3H), 1.99 (dd, J = 12.6, 6.9 Hz, 2H), 1.82 - 1.75 (m, 1H), 1.74 - 1.61 (m, 3H), 1.57 - 1.42 (m, 1H), 1.37 (d, J

= 7.0 Hz, 3H), 1.24 (s, 3H), 1.17 (d, J = 12.4 Hz, 3H), 1.08 (d, J = 6.6 Hz, 3H), 0.99 (t, J = 7.1 Hz, 1H), 0.85 (t, J = 6.8 Hz, 1H), 0.74 (dd, J = 14.8, 7.1 Hz, 6H), 0.63 (s, 2H), 0.40 (s, 2H).

Example 203: Synthesis of compound (2S,4R)-1-((S)-2-(4-(6-(1-(1-(7-(8-ethyl-7-fluoro))-3-hydroxynaphthalen-1-yl)-8-fluoro-4-((R)-3 -hydroxy-3-methylpiperidin-1-yl)pyrido[4,3-d]pyrimidin-2-yl)oxy)methyl)cyclopropyl)methyl)p iperidin-4-yl)pyridin-3-yl)-1H-1,2,3-triazol-1-yl)-3-methylbutanoyl)-4-hydroxy-N-((S)-1-(4-(4-methylthiazol-5-yl)phenyl)ethyl)pyrrolidine-2-carboxamide

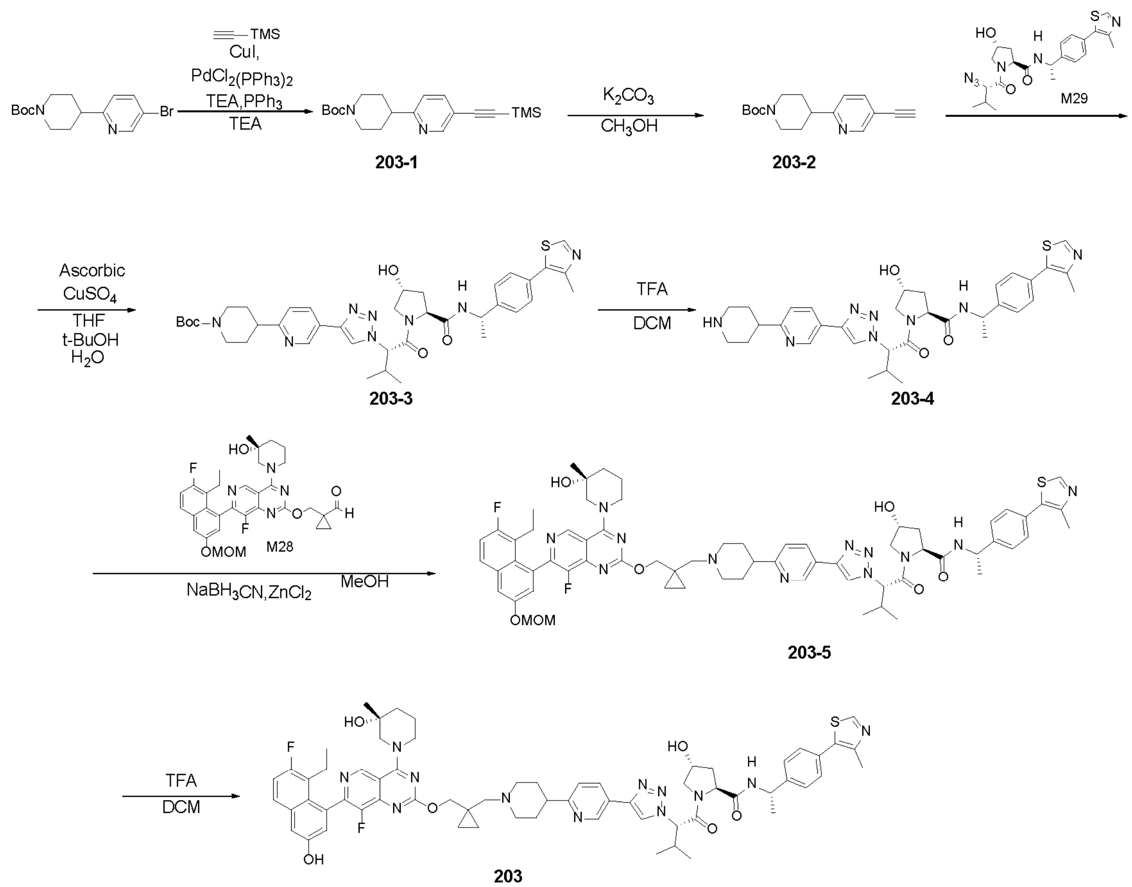


203-1 203-2 203-3 203-4 203-5 203

Step 1: Synthesis of compound 203-1

Tert-butyl 4-(5-bromopyridin-2-yl)piperidine-1-carboxylate (3.22 g, 9.44 mmol), TEA (60.00 mL), trimethylsilylacetylene (1.02 g, 10.38 mmol), $PdCl_2(PPh_3)_2$ (0.33 g, 0.47 mmol), $PPh_3$ (0.12 g, 0.47 mmol), and CuI (0.13 g, 0.66 mmol) were added successively in a 250 mL single-necked flask. The reaction was degassed with nitrogen and maintained under nitrogen atmosphere. The mxiture was stirred at room temperature and reacted overnight. The reaction was monitored by LCMS until it was completed. The reaction solution was concentrated, the concentrated residue was dissolved in 50 mL of ethyl acetate, then successively washed with 50 mL of saturated ammonium chloride aqueous solution and 50 mL of saturated brine. The resultant was separated, dried with anhydrous sodium sulfate, filtered, concentrated to obtain a crude product. And the crude product was separated and purified by column chromatography (PE to PE/EA = 97/3) to obtain a slightly yellow viscous product, i.e. compound 203-1 (3.3 g). ESI-MS m/z: 359.3 $[M+H]^+$.

Step 2: Synthesis of compound 203-2

$K_2CO_3$ (1.27 g, 9.20 mmol) and MeOH (70.00 mL) were added successively in a 250 mL three-necked flask. The mixture was cooled in a water bath, and then compound 203-1(3.30 g, 9.20 mmol) in MeOH (30.00 mL) was added. The reaction was stirred and reacted at room temperature. The reaction was monitored by LCMS until it was completed. The reaction solution was added to 280 g of an ice-water mixture and the resultant was stirred for 10 minutes. Then the resultant

was extracted with ethyl acetate three times, 100 mL each time. The organic phases were combined, washed with saturated brine and separated; dried with anhydrous sodium sulfate, filtered and concentrated to obtain a crude product. The crude product was separated and purified by column chromatography (PE to PE/EA = 95/5) to obtain a slightly yellow solid, i.e. compound 203-2 (860 mg). ESI-MS m/z: 287.2 [M+H] $^+$.

Step 3: Synthesis of compound 203-3

Compound 203-2 (410 mg, 1.43 mmol) and 98-Core (653.66 mg, 0.15 mmol), $CuSO_4$ (228.51 mg, 1.43 mmol), sodium ascorbate (737.45 mg, 3.72 mmol), THF/ tetrahydrofuran (10.00 mL), t-BuOH/ tert-butanol (10.00 mL), and $H_2O$ (10.00 mL) were added to a 100 mL single-necked flask. The mixture was stirred and reacted at room temperature for 1.0 hour. The reaction was monitored by LCMS until it was completed. The reaction solution was diluted with 100 mL water, then extracted and separated with a mixture of DCM/MeOH=4/1. The resultant was dried with anhydrous sodium sulfate, filtered and concentrated to obtain a crude product. The resultant was purified by column chromatography (PE to PE/EA = 1/1) to obtain a pale yellow foamy solid powder, which is compound 203-3 (840 mg). ESI-MS m/z: 743.6 [M+H] $^+$.

Step 4: Synthesis of compound 203-4

Compound 203-3 (840.00 mg, 1.13 mmol) and DCM (3.50 mL) were added successively in a 50 mL single-necked flask. The mixture was cooled in a water bath, and then TFA (1.50 mL) was added. The mixture was stirred at room temperature and reacted for half an hour. The reaction was monitored by LCMS until it was completed. The reaction solution was concentrated to dryness. A mixture of DCM/MeOH = 4/1 was added to dissolve concentrated residue. The resultant was neutralized with saturated sodium bicarbonate aqueous solution to alkaline, extracted and separated, dried with anhydrous sodium sulfate, filtered, and concentrated to obtain a crude product, and to obtain compound 203-4 (660 mg) as a pale yellow foam solid. ESI-MS m/z: 643.48, [M+H] $^+$.

Step 5: Synthesis of compound 203-5

M28 (109.99 mg, 0.19 mmol), compound 203-4 (108.44 mg, 0.17 mmol), MeOH (5.00 mL), and $ZnCl_2$ (0.25 mL, 1.00 mol/L, 0.25 mmol) were added successively in a 25 mL single-necked flask under nitrogen protection. The mixture was heated in an oil bath and maintained at 45°C for 1 hour. $NaBH_3CN$ (106.01 mg, 1.69 mmol) was added and the reaction was continued at a constant temperature. The reaction was monitored by LCMS until it was completed. The reaction solution was diluted with dichloromethane, and sodium bicarbonate aqueous solution was added. The mixture was quenched, extracted and the solution was separated. The aqueous phase was extracted with a mixture of DCM/MeOH = 4/1 three times, and the organic phase was combined, and dried with anhydrous sodium sulfate. The resultant was filtered, concentrated, and separated and purified by column chromatography (DCM to DCM:MeOH = 87:13) to obtain compound 203-5 (118 mg), ESI-MS m/z: 610.80 1/2[M+2H]$^+$.

Step 6: Synthesis of compound 203

Compound 203-5 (118.00 mg, 0.10 mmol) and DCM (3.00 mL) were added in a 10 mL single-necked flask successively. The mixture was cooled down in a water bath, and TFA (1.50 mL) was added dropwise. The reaction was stirred at room temperature. The reaction was monitored by LCMS until it was completed. The reaction soltion was combined with another batch (feeding: 98-5: 40 mg) and processed together. The resultant was concentrated under reduced pressure, the residue was dissolved in a mixture of DCM/MeOH = 4:1, and saturated aqueous sodium bicarbonate solution, extracted and phase separated. The organic phase was washed with water, dried with sodium sulfate, and concentrated to obtain a crude product. The crude product was separated and purified by column chromatography (DCM to DCM/MeOH = 80:20) to obtain compound 203 (64.6 mg).

ESI-MS m/z: 588.73 1/2[M+2H]$^+$.

$^1$H NMR (500 MHz, Methanol-d4) δ 9.22 (d, J = 4.9 Hz, 1H), 8.94 (s, 1H), 8.86 (d, J = 8.7 Hz, 1H), 8.60 (d, J = 6.4 Hz, 1H), 8.14 (s, 1H), 7.66 (t, J = 7.5 Hz, 1H), 7.41 (dq, J = 24.2, 9.5, 8.8 Hz, 6H), 7.30 (d, J = 7.0 Hz, 1H), 7.22 (td, J = 9.5, 3.3 Hz, 1H), 7.06 (d, J = 5.8 Hz, 1H), 5.49 (s, 1H), 5.41 (d, J = 10.2 Hz, 1H), 5.05 (q, J = 7.2 Hz, 1H), 4.50 (td, J = 22.6, 21.0, 10.0 Hz, 6H), 4.28 (t, J = 12.2 Hz, 1H), 4.00 - 3.84 (m, 2H), 3.69 - 3.50 (m, 3H), 3.42 (q, J = 11.7 Hz, 2H), 2.93 (s, 1H), 2.70 - 2.60 (m, 2H), 2.46 (d, J = 14.3 Hz, 4H), 2.24 - 2.17 (m, 2H), 2.11 - 2.04 (m, 3H), 1.98 (ddd, J = 12.9, 8.7, 4.4 Hz, 2H), 1.84 (d, J = 13.1 Hz, 1H), 1.76 (d, J = 13.6 Hz, 1H), 1.73 (s, 2H), 1.52 (d, J = 7.0 Hz, 3H), 1.27 (dd, J = 16.1, 8.2 Hz, 6H), 1.18 (d, J = 6.8 Hz, 3H), 0.83 (dp, J = 22.8, 8.1, 7.4 Hz, 9H), 0.72 (s, 1H).

**Biological Experiment**

Example 1 Cell Proliferation Experiment

Table 1

| Cell Line | KRAS Mutation Type | Culture Medium | Seeding Density |
|---|---|---|---|
| AGS | G12D | 1640+10%FBS | 1000 cells/well |
| PK59 | G12D | 1640+10%FBS | 3000 cells/well |
| H358 | G12C | 1640+10%FBS | 1000 cells/well |
| SW620 | G12V | 1640+10%FBS | 1000cells/well |
| HCT116 | G13D | 1640+10%FBS | 2000 cells/well |
| H1975 | WT | 1640+10%FBS | 1000 cells/well |

Cells with different mutations were seeded in low-attachment transparent 96-well plates at the seeding densities specified in Table 1, and incubated overnight in a cell culture incubator. After cell attachment, test compounds were added to the 96-well plates at final concentrations of 20000, 5000, 1250, 312.5, 78.13, 19.53, 4.88, 1.22, 0.31 and 0 nM (the final concentration of DMSO was all 0.25%), followed by incubation at 37°C for 96 h. Then, 50 μL of CellTiter-Glo working solution was added to each well. After vortex mixing, the mixture was incubated at room temperature for 10 min. The mixture was transferred to a white-bottom opaque 96-well plate, and luminescence values were read using a multifunctional microplate reader. The luminescence values were calculated and converted into percentage inhibition. The percentage inhibition of cell proliferation was calculated according to the following formula:

$$\text{Percentage Inhibition} = (1\text{-}(\text{Test Value-Blank}) / (\text{Max Value-Blank})) * 100\%$$

("Max Value" is from the 0.25% DMSO control well, "Blank" is from the blank medium control well, and "Test Value" is from the compound-treated well).

Curve fitting was performed using GraphPad Prism software to obtain $IC_{50}$ (nM) values, and the results are shown in Table 2.

Table 2

| Compound Name | $IC_{50}$(nM) | | | | | |
|---|---|---|---|---|---|---|
| | AGS | PK59 | HCT116 | SW620 | H358 | H1975 |
| **Compound 1** | 109 | | | | 1082 | |
| **Compound 2** | 44 | | | 501 | 143 | |
| **Compound 67** | 40 | | | 1721 | | |
| **Compound 69** | 14 | | | 34 | 42 | |
| **Compound 71** | 18 | | | 85 | 39 | |
| **Compound 73** | 22 | | | 81 | | |
| **Compound 75** | 11 | | | 122 | 40 | |
| **Compound 77** | 356 | | | | | |
| **Compound 79** | 44 | | | | | |
| **Compound 80** | 63 | | | | | |
| **Compound 88** | 33 | | | 199 | 90 | |
| **Compound 89** | 193 | | | 1420 | 635 | |
| **Compound 90** | 83 | | | | | |
| **Compound 93** | 6.8 | | | 86 | 20 | |
| **Compound 100** | 11 | | | 38 | | |

(continued)

| Compound Name | IC50(nM) | | | | | |
|---|---|---|---|---|---|---|
| | AGS | PK59 | HCT116 | SW620 | H358 | H1975 |
| **Compound 104** | 39 | | | | | |
| **Compound 105** | 12 | | | | | |
| **Compound 110** | 52 | | | | | |
| **Compound 111** | 20 | | | | | |
| **Compound 112** | 57 | | | | | |
| **Compound 113** | 8.3 | | | 49 | | |
| **Compound 114** | 13 | | | | | |
| **Compound 115** | 29 | | | | | |
| **Compound 116** | 27 | | | | | |
| **Compound 117** | 13 | 18.5 | | 47 | | |
| **Compound 118** | 13 | 12.2 | 37 | 26 | | >10000 |
| **Compound 119** | 37 | | | | | |
| **Compound 120** | 12 | | | 102 | | |
| **Compound 121** | 40 | | | | | |
| **Compound 122** | 28 | | | 87 | | |
| **Compound 123** | 82 | | | | | |
| **Compound 124** | 15 | | | 135 | | |
| **Compound 125** | 52 | | | | | |
| **Compound 126** | 32 | | | 246 | | |
| **Compound 127** | 47 | | | 223 | | |
| **Compound 128** | 33 | | | | | |
| **Compound 129** | 37 | | | | | |
| **Compound 130** | 17 | | | | | |
| **Compound 131** | 34 | | | | | |
| **Compound 132** | 25 | | | | | |
| **Compound 133** | 13 | | | 74 | 48 | |
| **Compound 134** | 10 | | | 58 | | |
| **Compound 135** | 5.2 | 14.7 | 33 | 39 | 7.3 | >10000 |
| **Compound 136** | 10 | | | 53 | 6.6 | |
| **Compound 137** | 37 | | | | | |
| **Compound 138** | 14 | | | 102 | | |
| **Compound 139** | 84 | | | 2035 | | |
| **Compound 140** | 28 | | | 167 | | |
| **Compound 141** | 31 | | | 250 | | |
| **Compound 142** | 10 | | | | | |
| **Compound 143** | 16 | | | 58 | 24 | |
| **Compound 144** | 3 | 4.4 | 13 | 8.4 | 5 | >10000 |
| **Compound 145** | 6.9 | | | 66 | | |

(continued)

| Compound Name | $IC_{50}$(nM) | | | | | |
|---|---|---|---|---|---|---|
| | AGS | PK59 | HCT116 | SW620 | H358 | H1975 |
| **Compound 146** | 6.9 | | | 37 | | |
| **Compound 147** | 16 | | | | | |
| **Compound 148** | 15 | | | | | |
| **Compound 149** | 15 | 57 | | | | |
| **Compound 150** | 11 | | | | | |
| **Compound 151** | 43 | | | | | |
| **Compound 152** | 30 | | | | | |
| **Compound 154** | 13 | | | | | |
| **Compound 155** | 23 | | | | | |
| **Compound 156** | 9.6 | | | | | |
| **Compound 157** | 19 | | | | | |
| **Compound 158** | 18 | | | | | |
| **Compound 159** | 6.2 | | | | | |
| **Compound 160** | 14 | 19.5 | | 88 | 17 | |
| **Compound 164** | 38 | | | | | |
| **Compound 165** | 9.2 | | | | | |
| **Compound 166** | 4.9 | | | 33 | 21 | |
| **Compound 167** | 8.8 | | | | | |
| **Compound 168** | 5.6 | | | | | |
| **Compound 169** | 20 | | | | | |
| **Compound 170** | 3.6 | | | 19 | 8.5 | |
| **Compound 171** | 13 | | | | | |
| **Compound 172** | 3.6 | | | | | |
| **Compound 173** | 6 | | | 35 | | |
| **Compound 174** | 8 | | | 59 | | |
| **Compound 175** | 26 | | | 70 | | |
| **Compound 176** | 33 | | | | | |
| **Compound 177** | 4.2 | | | | | |
| **Compound 178** | 2.5 | | | 9.4 | 5.7 | |
| **Compound 179** | 27 | | | | | |
| **Compound 180** | 20 | | | | | |
| **Compound 181** | 11 | | | | | |
| **Compound 182** | 24 | | | | | |
| **Compound 183** | 3 | | | 11 | 12 | |
| **Compound 184** | 2.7 | | | 11 | 15 | |
| **Compound 185** | 3.4 | | | 12 | 10 | |
| **Compound 186** | 20 | | | | | |
| **Compound 187** | 7.5 | | | | | |

(continued)

| Compound Name | $IC_{50}$(nM) | | | | | |
|---|---|---|---|---|---|---|
| | AGS | PK59 | HCT116 | SW620 | H358 | H1975 |
| **Compound 188** | 12 | | | | | |
| **Compound 189** | 2.9 | | | 13 | 15 | |
| **Compound 190** | 11 | | | 38 | | |
| **Compound 191** | 3.1 | | | 12 | | |
| **Compound 192** | 3.2 | | | 17 | | |
| **Compound 193** | 7.3 | | | 24 | | |
| **Compound 194** | 5.5 | | | 26 | | |
| **Compound 195** | 4.4 | | | 18 | | |
| **Compound 196** | 4 | | | 15 | | |
| **Compound 197** | 6.1 | | | 32 | | |
| **Compound 198** | 6.9 | | | 21 | | |
| **Compound 200** | 4.6 | | | 20 | | |
| **Compound 201** | 13 | | | 46 | | |
| **Compound 202** | 15 | | | 54 | | |
| **Compound 203** | 11 | | | 47 | | |
| **Compound 204** | 15 | | | 115 | | |
| **Compound 205** | 5 | | | 44 | | |
| **Compound 206** | 8.2 | | | 37 | | |
| **Compound 207** | 4.6 | | | 10 | | |

"Blank" indicates not determined.

Example 2: In-Cell Western Blotting Experiment

Reagent and Instrument

Table 3

| Instrument Name | Manufacturer | Model |
|---|---|---|
| Biosafety Cabinet | ESCO | AC2-4S1 |
| Microscope | Olympus | CKX-41 |
| $CO_2$ Incubator | Thermo | 3111 |
| Pipette | Eppendorf | |
| Cell Counter | Thermo | AMQAF1000 |
| Benchtop Centrifuge | Thermo | Heraeus Megafuge 8 |
| Microplate Reader | PerkinElmer | Envision |
| Nunc 96-well plate | In vitro scientific | 060096 |
| RPMI 1640 | Gibco | Cat.NO.A10491-01 #2192387 |
| FBS | Gibco | Cat.NO.10099-141C #2158737CP |
| Trypsin | Gibco | Cat.NO.25200-072 #2185855 |
| P/S | Solarbio | Cat.NO.P1400 #20200828 |
| PBS | Solarbio | Cat.NO.p1070-500 #20200821 |
| DMSO | Sigma | Cat.NO.D8370 #821D036 |

(continued)

| | | |
|---|---|---|
| Cell Counter | Thermo | AMQAF1000 |
| rabbit anti $KRAS^{G12D}$ | CST | # 14429 |
| mouse anti GAPDH | Proteintech | # 60004-1-Ig |
| IRDye 800CW marked Secondary Antibody 1 | LI-COR | 5151 |
| IRDye 680CW marked Secondary Antibody 2 | LI-COR | 5470 |
| Blocking Buffer | LI-COR | 927-40000 |
| 4% Formaldehyde | Beyotime | P0099 |
| Triton X-100 | Beyotime | P0096-100ml |
| Cell Culture Seeding | | |

Table 4

| Cell Strain | Source | Culture Medium |
|---|---|---|
| AGS | ATCC | RPMI1640 + 10% FBS+1% PS |
| 1) Under microscopic observation, the cells were in good condition, in the logarithmic growth phase, with a cell density of 80% to 90%.<br>2) Cells were digested with trypsin for 1 min to 2 min. Digestion was termintated by adding 5 mL of fresh culture medium. Cells were gently pipetted to generate a single-cell suspension and centrifuged at 1000 rpm/3 min.<br>3) Cells were resuspended in fresh culture medium. 10 μL of the cell suspension was aspirated, stained with Trypan blue, and cell density was counted using a cell counter.<br>4) A fresh cell suspension was prepared using fresh culture medium, adjusted to a cell density of $2.78\times10^5$ cells/mL ($5\times10^4$ cells/180 μL). The cell suspension was added to a black clear-bottom 96-well plate at 180 μL/well and incubated overnight in a 37°C, 5% $CO_2$ incubator. | | |

Compound for Administraion Preparation: 20 μL of a 10 mM compound stock solution was mixed with 30 μL of DMSO to yield a 4 mM solution. This was used as the highest concentration and serially diluted four-fold to obtain 9 concentrations of the compound. Then, 5 μL of each diluted compound solution was transferred to 195 μL of culture medium, mixed thoroughly to obtain a compound working solution. 20 μL of the working solution was transferred into the cells.

Experimental Preparation: Aliquoted frozen 4% paraformaldehyde was thawed at room temperature.

Cell Fixation: Culture medium was discarded. Prepared 4% paraformaldehyde was gently added along the well wall (150 μL/well) and incubated at room temperature for 20 min.

During fixation, 0.5% Triton X-100 permeabilization solution and 0.5% Tween-20 washing solution were prepared using PBS. After discarding the fixation solution, wells were washed with PBS containing 0.5% Tween-20 on a shaker for 5 min / 4 times.

Permeabilization: The prepared 0.5% Triton X-100 solution was added at 150 μL/well and incubated for 20 min.

After discarding the permeabilization solution, wells were washed with PBS containing 0.5% Tween-20 on a shaker for 5 min/4 times.

Blocking: 150 μL/well of blocking buffer was added and incubated at room temperature for 1.5 h.

Primary Antibody: 50 μL/well, incubated at 4°C overnight or at room temperature for 2 hr (The last column received blocking buffer as background; $KRAS^{G12D}$-1:200, GAPDH-1:400).

Primary antibody was discarded. Wells were washed with PBS containing 0.5% Tween-20 on a shaker for 5 min/4 times.

Secondary Antibody: 50 μL/well, incubated at room temperature for 1.5 h (anti-Rb 800-1:500; anti-Ms 680-1:500).

Secondary antibody was discarded. Wells were washed with PBS containing 0.5% Tween-20 on a shaker for 5 min/4 times. Plates underwent inverted centrifugation at 1000 rpm/1 min and were air-dried at room temperature for 3 hours before measurement.

The measured Relative ICW800 signal values were imported into Prism software for fitting, and $DC_{50}$ values were calculated. The results are shown in Table 5.

Table 5 Degradation Activity of Compounds in AGS Cell Line by In Cell Western Blotting

| Compound Name | $DC_{50}$ (nM) | Dmax (%) |
|---|---|---|
| **Compound 2** | 316 | 95 |
| **Compound 67** | 22 | 78 |
| **Compound 69** | 25 | 95 |
| **Compound 71** | 20 | 95 |
| **Compound 73** | 17 | 95 |
| **Compound 75** | 24 | 95 |
| **Compound 79** | 44 | 95 |
| **Compound 80** | 40 | 95 |
| **Compound 93** | 11 | 95 |
| **Compound 100** | 9.5 | 95 |
| **Compound 104** | 27 | 95 |
| **Compound 105** | 8.9 | 95 |
| **Compound 111** | 21 | 95 |
| **Compound 112** | 5 | 95 |
| **Compound 113** | 9.3 | 95 |
| **Compound 114** | 18 | 95 |
| **Compound 115** | 21 | 95 |
| **Compound 116** | 30 | 95 |
| **Compound 117** | 7.5 | 95 |
| **Compound 118** | 11 | 95 |
| **Compound 120** | 11 | 95 |
| **Compound 121** | 30 | 95 |
| **Compound 122** | 22 | 95 |
| **Compound 123** | 122 | |
| **Compound 124** | 21 | 95 |
| **Compound 126** | 23 | 95 |
| **Compound 128** | 84 | 95 |
| **Compound 131** | 18 | 95 |
| **Compound 132** | 19 | 95 |
| **Compound 133** | 17 | 95 |
| **Compound 134** | 5.9 | 95 |
| **Compound 135** | 14 | 95 |
| **Compound 136** | 14 | 95 |
| **Compound 137** | 37 | 95 |
| **Compound 138** | 13 | 95 |
| **Compound 139** | 31 | 95 |
| **Compound 142** | 6.3 | 95 |
| **Compound 143** | 12 | 95 |
| **Compound 144** | 1.1 | 95 |

(continued)

| Compound Name | $DC_{50}$ (nM) | Dmax (%) |
|---|---|---|
| **Compound 145** | 59 | 95 |
| **Compound 146** | 27 | 95 |
| **Compound 147** | 24 | 95 |
| **Compound 148** | 13 | 95 |
| **Compound 149** | 7.6 | 95 |
| **Compound 150** | 14 | 95 |
| **Compound 151** | 30 | 95 |
| **Compound 152** | 27 | 95 |
| **Compound 154** | 5.5 | 95 |
| **Compound 155** | 8.7 | 95 |
| **Compound 156** | 9.6 | 95 |
| **Compound 157** | 7.7 | 95 |
| **Compound 158** | 4.1 | 95 |
| **Compound 159** | 2.3 | 95 |
| **Compound 160** | 2 | 95 |
| **Compound 203** | 14 | 95 |

Example 3 Degradation Effect of Compound 2 on Different KRAS

AGS cells in good condition and logarithmic growth phase were plated in a 12-well plate at a density of $3\times10^5$ cells per well and cultured overnight at 37°C, 5% $CO_2$. The next day, Compound 2 was diluted to a corresponding concentration and administered to achieve final concentrations of 0.2, 1, and 5 μM. After incubation at 37°C, 5% $CO_2$ for 24 h, the culture medium was discarded. Cells were washed with PBS once, lysed with 50 μl/well of lysis buffer on ice for 10 min, centrifuged, and total protein was collected. After BCA protein quantification, samples were uniformly loaded onto an SDS-PAGE gel at 20 μg/well. The electrophoresis program was set to 80 V for 0.5 h, followed by 120 V for 1 h. After the electrophoresis was completed, the PAGE gel was transferred to a 0.22 μm PVDF membrane. The membrane was blocked with 5% BSA for 1 h. The PVDF membrane was cut at the 35 kD marker and incubated with primary antibodies against KRAS (Sigma #SAB1404011-100UG) and β-actin (CST #5057S) overnight at 4°C. The next day, the PVDF membrane was removed from the primary antibody solution, washed with TBST 4 times, 5 min each time. Then it was incubated with the corresponding secondary antibody at room temperature for 1.5 hr. After washing 4 times with TBST, exposure was performed on iBright FL1000. The results are shown in Figure 1.

Example 4 Western Blotting Experiment

Cell Seeding

AGS cells in logarithmic growth phase and good condition were selected. The culture medium was discarded. Cells were washed with PBS once, and digested with 0.25% Trypsin.

Culture medium was added to termintate digestion. Cells were collected into a 15 mL centrifuge tube, centrifuged at 1000 rpm for 3 min, and the supernatant was discarded. Cells were resuspended in 2 mL culture medium, counted, and diluted with culture medium to $3.33\times10^5$ cells/mL ($3\times10^5$ cells/0.9 mL/well). Cells were seeded in a 12-well plate at 0.9 mL per well and cultured overnight at 37°C, 5% $CO_2$.

Administration Treatment

Compound Working Solution Preparation: A certain volume of the stock solution was aspirated and diluted with DMSO to obtain a 1000x intermediate solution. This intermediate solution was then serially diluted with DMSO to obtain intermediate solutions at various concentrations. 3 μL of each diluted intermediate solution was aspirated into 300 μL of

culture medium, pipetted to mix thoroughly, and prepared into a 10x working solution.

Administration: 100 μL of the corresponding working solution was aspirated into each well. After mixing, the 12-well plate was incubated at 37°C, 5% $CO_2$ for 24 hr.

Protein Collection and Quantification

Cell Lysis Buffer Preparation: Prepared 10 x RIPA lysis buffer, 10x phosphatase inhibitor, and 25x protease inhibitor were taken out from -20°C in advance and thawed at room temperature. Cell lysis buffer was prepared by mixing 760 μL of $ddH_2O$, 100 μL of 10x RIPA lysis buffer, 100 μL of 10x phosphatase inhibitor, and 40 μL of 25x protease inhibitor per mL, mixed thoroughly, and set aside for later use.

Cell Lysis: After the drug treatment period ended, the culture medium in the wells was carefully aspirated. 500 μL of PBS was gently added to each well for washing, and the PBS was aspirated. 50 μL of cell lysis buffer was slowly added to the center of each well. After lysis on ice for 10 min to 15 min, the lysate was collected into 1.5 mL centrifuge tubes and centrifuged at 4°C, 15000 rpm for 15 min. The supernatant was aspirated into another clean centrifuge tube.

Protein Quantification: A clean transparent 96-well plate was taken. 20 μL of PBS was added to each well, followed by the addition of 5 μL of the collected protein supernatant per well for single-well testing. BCA solution A and B were premixed at a ratio of 50:1. 200 μL of the mixed BCA developing solution was added to each well. The 96-well plate was wrapped in aluminum foil and incubated at 37°C for 30 min. OD value (562 nm) was measured using an Evisin instrument. The protein concentration was calculated by reference to a standard curve.

Performing WB Experiment

1) Adjusting protein concentration: According to the calculation formula, the protein loading amount per well was adjusted to 30 μg using PBS, with a final volume of 10 μL. 2 μL of 6x SDS loading buffer was added to each sample, heated at 100°C for 5 min, centrifuged, and cooled to room temperature.
2) Assembling the electrophoresis tank: The electrophoresis tank was assembled, electrophoresis buffer was added, and 12 μL was loaded per well. 2.5 μL and 1.5 μL of marker were added to the left and right sides of the sample wells, respectively. Both the marker well and other wells were filled to 12 μL with 1x loading buffer. Electrophoresis was performed at 90 V for approximately 30 min. After the samples were compressed into a band within the gel, the voltage was increased to 120 V. Electrophoresis continued until the blue loading buffer band reached the bottom of the gel.
3) Preparing transfer buffer in advance according to the ratio 7:2:1 ($ddH_2O$: methanol : 10x Transfer Buffer), 2 L to 3 L total, and cooled at 4°C.
4) Disassembling carefully the gel cassette and removing the gel after electrophoresis ended. Unnecessary parts were trimmed off. The gel was placed on a transfer cassette pre-layered with filter paper. Pre-cut PVDF membrane (PVDF membrane was pre-activated by soaking in methanol and equilibrated in transfer buffer) was gently laid over the gel. A roller was used to carefully roll back and forth to remove bubbles between the gel and the membrane. Filter paper was then placed over the membrane. The transfer cassette was carefully clamped shut, inserted into the transfer tank, and transfer buffer was added. The transfer tank was placed within a larger container filled with crushed ice around it. Transfer was performed at 400 mA for 120 min to 150 min (duration determined based on protein molecular weight).
5) Preparing 5% BSA blocking buffer: 2.5 g BSA was weighed, dissolved in TBS-T, and the volume was adjusted to 50 mL.
6) Blocking the membrane: After transfer ended, the PVDF membrane was carefully removed and immersed in 5% BSA solution. Blocking was performed on a shaker at room temperature for 1 hr.
7) Preparing primary antibodies: 5% BSA was diluted to 2% for antibody dilution. Primary antibodies were diluted according to the dilution ratio recommended in the antibody datasheet.
8) Incubating with primary antibodies: The blocked PVDF membrane was transferred to the primary antibody solution and incubated overnight at 4°C.

Incubating with Secondary Antibody

1) The PVDF membrane was removed from the primary antibody solution and washed with TBS-T three times on a shaker, 10 min each time. Used primary antibody solution can be stored at -20°C for reuse.
2) According to the primary antibody datasheet, the corresponding rabbit or mouse secondary antibody was diluted

using 2% BSA at a dilution ratio of 4000:1. The PVDF membrane was placed in the secondary antibody solution and incubated at room temperature for 1 hr to 2 hr.

3) After secondary antibody incubation ended, the membrane was washed with TBS-T three times, 10 min each time.

Luminescence Procedure

1) A luminometer was turned on, and BioSense mode was selected.

2) Luminescence Reagent A and Reagent B were mixed in equal proportions in a container. The PVDF membrane was dried with lint-free paper, placed in the container, and the luminescence reagent mixture was repeatedly pipetted over the PVDF membrane. After the PVDF membrane was fully saturated with the luminescence reagent mixture, the membrane was placed in the instrument for exposure. Results for the other two cell lines, H358 and SW620, were obtained by referring to the above test method and are shown in Figure 2.

Example 5 Study on Metabolic Stability of Compounds in Human, Rat, Mouse, and Dog Liver Microsomes

Experimental Method: A diluted solution containing 0.5 mg/mL liver microsomes with K-buffer and $MgCl_2$ was prepared:

Table 6

| Reagent | Concentration of Stock Solution | Added Volume | Final Concentration |
|---|---|---|---|
| $MgCl_2$ solution | 50 mM | 40 μL | 5 mM |
| K-buffer | 100 mM | 302 μL | 75.5 mM |
| Liver Microsome | 20 mg/mL | 10 μL | 0.5 mg/mL |

352 μL of the diluted liver microsome solution prepared in Table 6 above was added into the pre-set wells of the incubation plate, labeled as the incubation plate. 88 μL of the above incubation solution was added to 400 μL precipitant containing internal standard. After vortex mixing, the mixture was placed in a refrigerator for at least 10 min. Then, 2 μL of the test compound working solution (50 μM) (final DMSO concentration ≤ 0.5%) and 10 μL of NADPH were added. The mixture was vortex mixed at 600 rpm at room temperature for 5 min as a 0 min sample. The incubation plate was placed in a 37°C constant temperature shaker and pre-incubated for 5-10 min. Then, 6 μL of the test compound working solution (50 μM) and pre-warmed 30 μL of NADPH were added to the incubation plate to initiate the reaction, and timing started. At each predetermined time point, 50 μL of the samples were taken from the incubation plate and added to the precipitation plate containing 200 μL of internal standard. Subsequent steps were the same as the treatment for the 0 min sample. The samples were mixed thoroughly, centrifuged at 4°C and 5000 rpm for 10 minutes. The supernatant after centrifugation was added to pre-added ultrapure water at an appropriate ratio, mixed thoroughly on a shaker at room temperature, and subjected to LC-MS/MS analysis.

Data Processing: The peak areas of the analyte and the internal standard in the samples were measured at different time points. The peak area ratio of the analyte to the internal standard at non-zero time points compared to the peak area ratio of the analyte to the internal standard in the 0 min sample was used to calculate the remaining percentage of the parent drug. The natural logarithm of the remaining percentage was plotted against the incubation time as the ordinate and abscissa, respectively, for linear fitting.

Calculation formulas: Elimination Rate Constant $k$ = -slope; Half-life $T_{1/2} = 0.693 / k$; In vitro intrinsic clearance in liver microsomes $CL_{int,\ in\ vitro} = k / C_{protein}$. Note: $C_{protein}$ represents the concentration of liver microsomal protein in the final incubation system. The results of in vitro liver microsomes for each compound are shown in Table 7 below.

Table 7

| **Compound No.** | **The in vitro intrinsic clearance in liver microsomes ($CL_{int,\ in\ vitro}$, μL/min/mg protein)** | | | |
|---|---|---|---|---|
| | **Human** | **Rat** | **Mouse** | **Dog** |
| **Compound 2** | 31.0 | 11.6 | 16.6 | 0.0 |
| **Compound 69** | 9.8 | 4.6 | 7.9 | 16.4 |
| **Compound 71** | 5.9 | 1.1 | 9.8 | 6.5 |
| **Compound 73** | 13.8 | 9.8 | 7.1 | 18.9 |

(continued)

| Compound No. | The in vitro intrinsic clearance in liver microsomes ($CL_{int, in vitro}$, μL/min/mg protein) | | | |
|---|---|---|---|---|
| | Human | Rat | Mouse | Dog |
| **Compound 75** | 1.0 | 20.7 | 8.6 | 22.1 |
| **Compound 88** | 13.4 | 10.1 | 24.5 | 13.3 |
| **Compound 93** | 23.5 | 10.9 | 8.3 | 28.5 |
| **Compound 100** | 47.0 | 7.9 | 3.2 | 57.5 |
| **Compound 105** | 9.9 | 5.9 | 13.3 | 28.5 |
| **Compound 111** | 21.7 | 15.7 | 10.6 | 8.4 |
| **Compound 114** | 46.5 | 25.2 | 18.9 | 50.5 |
| **Compound 115** | 32.1 | 13.8 | 15.9 | 18.3 |
| **Compound 117** | 8.8 | 11.3 | 10.1 | 6.6 |
| **Compound 118** | 21.3 | 3.4 | 11.4 | 17.2 |
| **Compound 119** | 6.1 | 9.1 | 10.4 | 14.9 |
| **Compound 120** | 128 | 9.3 | 48.5 | 113 |
| **Compound 122** | 46.4 | 8.5 | 24.8 | 53 |
| **Compound 124** | 49.7 | 9.1 | 18.6 | 88 |
| **Compound 127** | 52.8 | 15.6 | 42.4 | 31.5 |
| **Compound 133** | 7.4 | 7.2 | 8.0 | 9.3 |
| **Compound 134** | 28.4 | 3.0 | 6.4 | 5.5 |
| **Compound 135** | 11.3 | 1.5 | 13.2 | 26.9 |
| **Compound 136** | 19.6 | 8.0 | 22.1 | 20.8 |
| **Compound 138** | 11.2 | 5.7 | 13.6 | 25.5 |
| **Compound 141** | 21.2 | 3.9 | 12.1 | 12.9 |
| **Compound 142** | 11.1 | 0.0 | 17.7 | 14.2 |
| **Compound 144** | 18.8 | 6.2 | 10.9 | 7.4 |
| Note: When $CL_{int, in vitro}$ in human liver microsomes is < 8.6, it is classified as slow metabolism; when $CL_{int, in vitro}$ in human liver microsomes is > 47, it is classified as fast metabolism. | | | | |

The results indicate that Compound 71, Compound 75, Compound 119, and Compound 133 all belong to slow metabolism in human liver microsomes. For druggability, this is favorable for reducing drug dosing frequency.

Example 6 Study on Phase II Metabolic Stability of Compounds in Human, Rat, Mouse, and Dog Liver Microsomes

Experimental Method: A diluted solution containing 0.5 mg/mL liver microsomes with K-buffer and $MgCl_2$ was prepared:

Table 8

| Reagent | Concentration of Stock Solution | Added Volume | Final Concentration |
|---|---|---|---|
| $MgCl_2$ solution | 100 mM | 40 μL | 10 mM |
| K-buffer | 100 mM | 306 μL | 76.5 mM |
| Alamethicin | 5 mg/mL | 2 μL | 25 μg/mL |
| Liver Microsome | 20 mg/mL | 10 μL | 0.5 mg/mL |

358 μL of the diluted liver microsome solution prepared in Table 8 above was added to the pre-set wells of the incubation plate, labeled as the incubation plate. 2 μL of compound working solution (400 μM) (final concentration of

DMSO ≤ 0.5%) was added. The incubation plate was placed in a 37°C constant temperature shaker and pre-incubated for 5-10 min. Then, 40 μL of UDPGA (20 mM) was added to the incubation plate to initiate the reaction, and timing started. At each predetermined time point (0, 5, 15, 60 min), 50 μL of samples were taken from the incubation plate and added to the precipitation plate containing 200 μL of internal standard. Samples were mixed thoroughly, centrifuged at 4°C and 5000 rpm for 10 minutes. The supernatant after centrifugation was added to pre-added ultrapure water at an appropriate ratio, mixed thoroughly on a shaker at room temperature, and subjected to LC-MS/MS analysis.

Data processing: The peak areas of the analyte and the internal standard in samples were measured at different time points. The peak area ratio of the analyte to the internal standard at non-zero time points compared to the peak area ratio of the analyte to the internal standard in the 0 min sample was used to calculate the remaining percentage of the parent drug. The results of the remaining percentage of parent drug after 60 min incubation for each compound are shown in Table 9 below.

Table 9

| Compound No. | 60 minutes remaining percentage % | | | |
|---|---|---|---|---|
| | Human | Rat | Mouse | Dog |
| Compound 69 | 98.4 | 99 | 92 | 90.6 |
| Compound 71 | 94.6 | 75.4 | 93 | 108 |
| Compound 122 | 76.9 | 96.5 | 105 | 99.4 |
| Note: When the remaining percentage at 60 min % > 80%, the compound was considered to have very slow metabolism, which was favorable for further development. | | | | |

The results indicate: Compound 69 and Compound 71 were not metabolized or are minimally metabolized by UGT in human liver microsomes.

Example 7 PK Study of Compound 2 in Balbc Mice

Experimental Method and Results:

Compound 2 was mixed with the vehicle (10% DMSO / 5% Solutol HS 15 / 85% saline), vortexed, and sonicated to prepare a clear solution at 0.2 mg/mL. Female Balbc mice aged 6 to 7 weeks (non-fasted) were intravenously administered Compound 2. Whole blood was collected at specified time points. Drug concentration was analyzed by LC-MS/MS, and pharmacokinetic parameters were calculated using Phoenix WinNonlin software (Pharsight Corporation, USA). The results are shown in Table 10.

Table 10 Mouse PK Data for Intravenous Compound 2 Monotherapy

| Compound | 2 |
|---|---|
| Route | IV |
| Dosing Level (mg/kg) | 1 |
| Formulation | 10%DMSO/5%Solutol/85% saline |
| Sex | Female |
| Cl_obs (mL/min/kg) | 3.77 |
| t1/2 (h) | 6.49 |
| $C_0$ (ng/mL) | 8791 |
| $AUC_{last}$ (h*ng/mL) | 4379 |
| Vss_obs (L/kg) | 0.586 |

Example 8 PK Study of Compound in ICR Mice (whole blood)

Experimental Method and Results:

The compound was mixed with the vehicle (10% DMSO / 5% Solutol HS 15 / 85% saline), vortexed, and sonicated to prepare a clear solution at 0.2 mg/mL. Female ICR mice aged 6 to 7 weeks (non-fasted) were intravenously administered the compound. Whole blood was collected at specified time points. Drug concentration was analyzed by LC-MS/MS, and pharmacokinetic parameters were calculated using Phoenix WinNonlin software (Pharsight Corporation, USA). The results are shown in Table 11.

Table 11 Mouse PK Data for Intravenous Compound Monotherapy

| Compound | 71 | 73 | 88 | 133 |
|---|---|---|---|---|
| Route | IV | | | |
| Dosing Level (mg/kg) | 1 | | | |
| Formulation | 10%DMSO/5%Solutol/85% saline | | | |
| Sex | Female | | | |
| Cl_obs (mL/min/kg) | 16.3 | 7.97 | 4.03 | 14.2 |
| $t_{1/2}$ (h) | 6.08 | 5.07 | 5.61 | 5.77 |
| $C_0$ (ng/mL) | 5562 | 3008 | 5003 | 2838 |
| $AUC_{last}$ (h*ng/mL) | 1032 | 2051 | 4109 | 1203 |
| Vss_obs (L/kg) | 1.45 | 1.91 | 0.780 | 2.49 |

Example 9 PK Study of Compound in ICR Mice (plasma)

Experimental Method and Results:

The compound was mixed with the vehicle (10% DMSO / 5% Solutol HS 15 / 85% saline), vortexed, and sonicated to prepare a clear solution at 0.2 mg/mL. Female ICR mice aged 6 to 7 weeks (non-fasted) were intravenously administered the compound. Plasma was collected at specified time points. Drug concentration was analyzed by LC-MS/MS, and pharmacokinetic parameters were calculated using Phoenix WinNonlin software (Pharsight Corporation, USA). The results are shown in Table 12.

Table 12 Mouse PK Data for Intravenous Compound Monotherapy

| Compound | 135 | 142 | 144 |
|---|---|---|---|
| Route | IV | | |
| Dosing Level (mg/kg) | 1 | | |
| Formulation | 10%DMSO/5%Solutol/85% saline | | |
| Sex | Female | | |
| Cl_obs (mL/min/kg) | 5.80 | 7.32 | 5.81 |
| $t_{1/2}$ (h) | 6.92 | 4.10 | 9.34 |
| $C_0$ (ng/mL) | 5844 | 9464 | 5530 |
| $AUC_{last}$ (h*ng/mL) | 2747 | 2295 | 2728 |
| Vss_obs (L/kg) | 1.61 | 0.854 | 1.54 |

Example 10 PK Study of Compound 135 in Beagle Dogs

Experimental Method and Results:

Compound 135 was mixed with the vehicle (10% DMSO / 5% Solutol HS 15 / 85% saline), vortexed, and sonicated to prepare a clear solution at 0.5 mg/mL. Beagle dogs weighing approximately 10 kg (equal number of males and females, non-fasted) were intravenously administered the compound. Plasma was collected at specified time points. Drug

concentration was analyzed by LC-MS/MS, and pharmacokinetic parameters were calculated using Phoenix WinNonlin software (Pharsight Corporation, USA). The results are shown in Table 13.

Table 13 Beagle Dog PK Data for Intravenous Compound Monotherapy

| Compound | 135 | 144 |
|---|---|---|
| Route | IV | |
| Dosing Level (mg/kg) | 1 | |
| Formulation | 10%DMSO/5%Solutol/85% saline | |
| Sex | Female | |
| Cl_obs (mL/min/kg) | 5.74 | 6.19 |
| $t_{1/2}$ (h) | 21.4 | 20.5 |
| $C_0$ (ng/mL) | 10967 | 9889 |
| $AUC_{last}$ (h*ng/mL) | 2779 | 2522 |
| Vss_obs (L/kg) | 2.23 | 3.46 |

Example 11 Evaluation of Anti-Tumor Effects of Compounds in a Human Pancreatic Cancer Model Carrying $KRAS^{G12D}$ Mutation

PK-59 cells (Nanjing Cobioer Biosciences Co., Ltd., CBP61184) were resuscitated and cultured in RPMI1640 + 10% FBS + 1% penicillin-streptomycin dual antibiotic medium. Cells were passaged every 3 days. After sufficient growth, cells were resuspended in PBS and mixed with Matrigel at a 1:1 ratio. A suspension of $2 \times 10^6$ cells/100 μL was subcutaneously inoculated into the dorsal region of 4- to 6-week-old male immunodeficient BALB/c Nude Crlj mice (Beijing Vital River Laboratory Animal Technology Co., Ltd.). When tumor volumes reached 200 to 300 $mm^3$, mice were divided into 11 groups, each group for 6, and administered corresponding compounds/doses as shown in Table 14. Test compounds were dissolved in 80% saline / 10% (50% SBECD) / 10% RH40 and administered via tail vein. Tumor volumes were measured on Days 3, 7, 10, 14, 17, 21, 24, and 28 post-first administrations. Tumor growth inhibition (TGI) was calculated;

$$\text{Tumor Volume (mm}^3) = 0.5 * \text{Major Axis D} * \text{Minor Axis d}^2;$$

$$\text{TGI (\%)} = (1 - RTV_{treatment} / RTV_{control}) * 100,$$

wherein RTV is a relative tumor volume, and RVT = $V_t$ -$V_0$.

Table 14

| Group | Compound No. | Number of Each Group | Dosage (mg/kg) | Administration Frequency |
|---|---|---|---|---|
| G1 | Control | 6 | / | QW |
| G2 | Compound 135 | 6 | 1 | QW |
| G3 | | 6 | 3 | QW |
| G4 | | 6 | 10 | QW |
| G5 | | 6 | 20 | Q2W |
| G6 | Compound 144 | 6 | 1 | QW |
| G7 | | 6 | 3 | QW |
| G8 | | 6 | 10 | QW |
| Note: QW = once weekly; Q2W = once every two weeks. | | | | |

Table 15

| Compound No. | Dosage (mg/kg), Administration Frequency | TGI After 4 Weeks of Administration (%) |
|---|---|---|
| Compound 135 | 1, QW | 70 |
| | 3, QW | 92 |
| | 10, QW | 106 |
| | 20, Q2W | 101 |
| Compound 144 | 1, QW | 82 |
| | 3, QW | 93 |
| | 10, QW | 113 |

After 4 weeks of administration, results were shown in Tables 14-15 and Figs. 3-4. The results indicate that: for the test compounds administered at 1 mg/kg once weekly, TGI ranged from 70% to 87%, all achieving tumor suppression effects. When the dosage was increased to 10 mg/kg once weekly, all compounds induced tumor regression with TGI ranging from 106% to 118%. Wherein, Compound 135 achieved comparable TGI at an increased dosage of 20 mg/kg and extended administration frequency of once every two weeks versus 10 mg/kg once weekly. No body weight loss was observed in the mice of any administered groups, indicating that all the compounds exhibit good safety profiles.

Example 12 Study on KRAS Degradation by Compound 144 in Cells with Different Mutations

AGS, SW620, and HCT116 cells in logarithmic growth phase with good condition were seeded in 12-well plates at $3 \times 10^5$ cells/well and cultured overnight at 37°C, 5% $CO_2$. The next day, 10 mM compound stock solution was serially diluted five-fold using DMSO starting from 0.5 mM, with the last concentration being DMSO, to prepare working solutions. The working solutions were added to cell culture medium at 1:1000 (1 μL working solution per 1 mL medium/well). After 3 h incubation at 37°C, 5% $CO_2$, medium was discarded. The cells were washed with PBS and lysed with 50 μL/well lysis buffer on ice for 10 min. Total protein was collected by centrifugation. After BCA quantification, 20 μg protein/well was loaded onto SDS-PAGE gel. Electrophoresis: 80 V for 0.5 h, then 120 V for 1 h. Proteins were transferred to 0.22 μm PVDF membranes, blocked with 5% non-fat dry milk for 1 h, and cut at the 35 kD marker. Membranes were incubated with primary antibodies against KRAS (Sigma #SAB1404011-100UG) and β-actin (CST #5057S) at 4°C overnight. The next day, membranes were washed with TBST 4 times, each time 5 min, incubated with corresponding secondary antibodies for 1.5 hr at RT, washed with TBST 4 times, and imaged on an iBright™ FL1000.

Result was shown in Fig. 5: Compound 144 effectively degraded KRAS G12D/G12V and G13D proteins within 3 h.

Although the invention has been fully described through its implementation methods, it should be noted that various changes and modifications are obvious to those skilled in the field. Such changes and modifications should all be included within the scope of the claims attached to the invention.

## Claims

1. A compound having an F-L-M structure, or a tautomer, racemate, enantiomer, diastereomer thereof, or a mixture thereof, or a pharmaceutically acceptable salt thereof, wherein:

   F is a KRAS protein binding fragment represented by general formula (A);

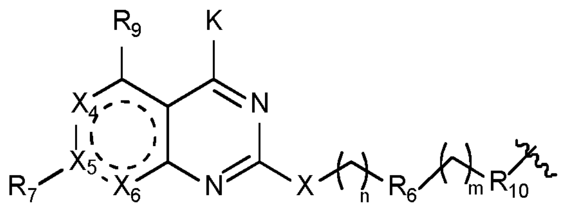

(A)

X is selected from the group consisting of -O- and

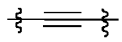;

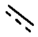 represents a bond independently selected from the group consisting of a single bond and a double bond;

$X_4$ is $CR_8$, $C(R_8)_2$, O, N or $NR_8$; $R_8$ is each independently selected from the group consisting of H, hydroxyl, oxo, halogen, cyano, $C_{1-6}$ alkyl, $C_{1-6}$ haloalkyl, -S-$C_{1-6}$ alkyl, -$C_{0-3}$ alkylene-$C_{2-4}$ alkenyl, $C_{1-6}$ alkoxy, $C_{1-6}$ haloalkoxy and $C_{3-14}$ cycloalkyl, wherein $C_{3-14}$ cycloalkyl or $C_{1-6}$ alkyl is optionally further substituted by one or more $R_a$;

$X_5$ is selected from the group consisting of C, CH and N;

$X_6$ is selected from the group consisting of $CR_{14}$, $C(R_{14})_2$, O, N and $NR_{14}$; $R_{14}$ is each independently selected from the group consisting of H, hydroxyl, oxo, halogen, cyano, $C_{1-6}$ alkyl, $C_{1-6}$ haloalkyl, -S-$C_{1-6}$ alkyl, -$C_{0-3}$ alkylene-$C_{2-4}$ alkenyl, $C_{1-6}$ alkoxy, $C_{1-6}$ haloalkoxy and $C_{3-14}$ cycloalkyl, wherein $C_{3-14}$ cycloalkyl or $C_{1-6}$ alkyl is optionally further substituted by one or more $R_a$;

$R_6$ is absent or is selected from the group consisting of $C_{3-14}$ cycloalkyl and 3- to 14-membered heterocyclyl, wherein $C_{3-14}$ cycloalkyl or 3- to 14-membered heterocyclyl is optionally further substituted by one or more $R_a$;

$R_9$ is selected from the group consisting of H, amino, substituted amino, cyano, $C_{1-6}$ alkyl, $C_{1-6}$ alkoxy, halogen, $C_{2-6}$ alkenyl and $C_{3-6}$ cycloalkyl, wherein $C_{1-6}$ alkyl, $C_{2-6}$ alkenyl or $C_{3-6}$ cycloalkyl is further substituted by one or more $R_a$;

$R_{10}$ is absent or is selected from the group consisting of -O- and -$NR_{11}$-; $R_{11}$ is selected from the group consisting of H, $C_{1-6}$ alkyl, $C_{1-6}$ haloalkyl, $C_{1-6}$ hydroxylalkyl and $C_{1-6}$ aminoalkyl;

$R_7$ is selected from the group consisting of $C_{3-14}$ cycloalkyl, 3- to 14-membered heterocyclyl, $C_{6-18}$ aryl and 5- to 18-membered heteroaryl, wherein $C_{3-14}$ cycloalkyl, 3- to 14-membered heterocyclyl, $C_{6-18}$ aryl or 5- to 18-membered heteroaryl is optionally further substituted by one or more $R_a$;

K is selected from the group consisting of $C_{3-14}$ cycloalkyl and 3- to 14-membered heterocyclyl, wherein $C_{3-14}$ cycloalkyl or 3- to 14-membered heterocyclyl is a monocyclic ring, a fused ring, a spiro ring or a bridged ring, optionally further substituted by one or more $R_a$, wherein 3- to 14-membered heterocyclyl is not

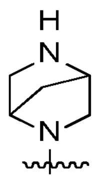, 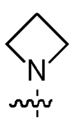, 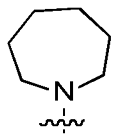, 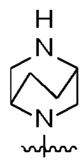, 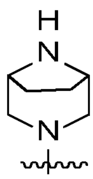,

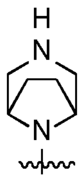 or 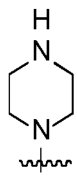;

m and n are each independently selected from the group consisting of 0, 1, 2, 3, 4 and 5;

L is a connection unit connecting F and M;

L is selected from the group consisting of

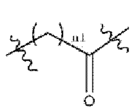, 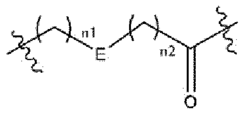, 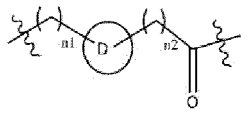, 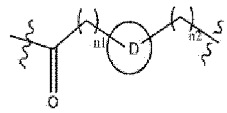,

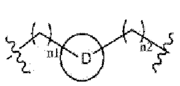, 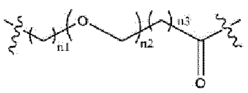, 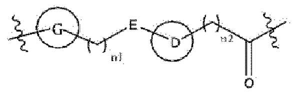, 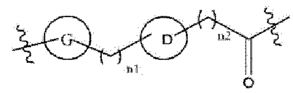,

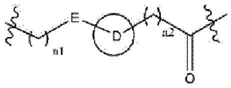, 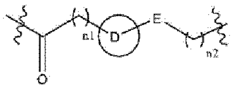, 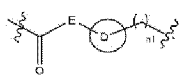, 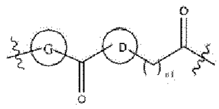, 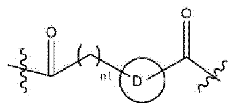,

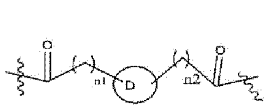, 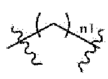, 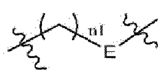, 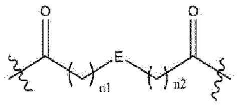, 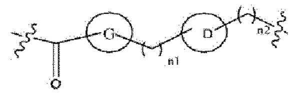,

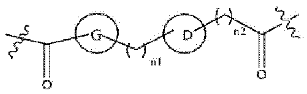, 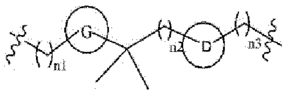, 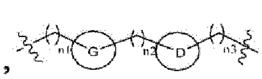, 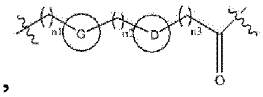,

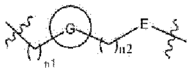, 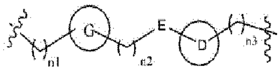, 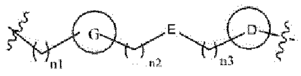, 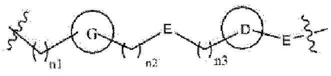,

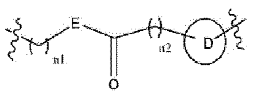, 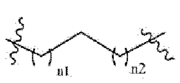, 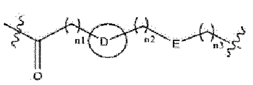, 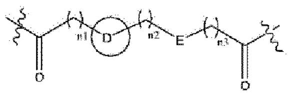,

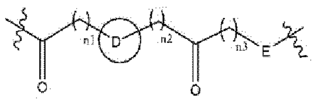, 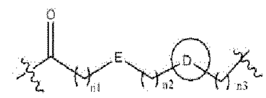, 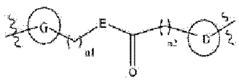 and 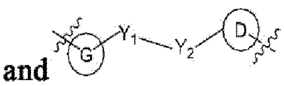;

wherein,
ring G or ring D is each independently selected from the group consisting of $C_{6-14}$ aryl, 5- to 14-membered heteroaryl, $C_{3-14}$ cycloalkyl and 3- to 14-membered heterocyclyl, wherein $C_{6-14}$ aryl, 5- to 14-membered heteroaryl or 3- to 14-membered heterocyclyl is optionally further substituted by one or more $R_a$;
E is each independently selected from the group consisting of -O-, -NH- and $-NCH_3-$;
$Y_1$ and $Y_2$ are each independently absent or selected from the group consisting of -O-, -NH-, 3- to 6-membered N-containing heterocyclyl, $C_{1-6}$ alkylene, carbonyl, acetylenyl and $C_{3-6}$ cycloalkyl;
n1, n2 and n3 are each independently selected from integers ranging from 0 to 10, and preferably n1, n2 and n3 are each independently 0, 1, 2, 3 or 4;
M is a VHL binding fragment represented by general formula (III) or (IV);

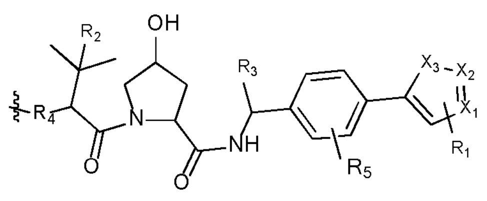
(III)
or
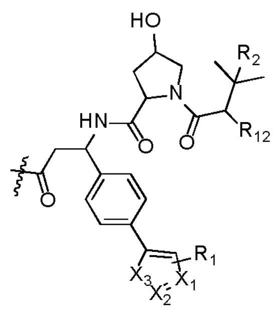
(IV)

$X_1$ is selected from the group consisting of C, CH and N;
$X_2$ is selected from the group consisting of C, CH and N;
$X_3$ is selected from the group consisting of N, NH, O and S;
$R_1$ is selected from the group consisting of H, $C_{1-6}$ alkyl, halogen and cyano, and is preferably methyl, ethyl, F or cyano;
$R_2$ is selected from the group consisting of H and $C_{1-6}$ alkyl, and is preferably H or methyl;
$R_3$ is selected from the group consisting of H, $C_{1-6}$ alkyl, $C_{1-6}$ hydroxylalkyl and $-(CH_2)_n-CO-NH-(CH_2)_n-CH_3$,

wherein n is selected from the group consisting of 0, 1, 2 and 3, and $R_3$ is preferably H, methyl, hydroxylmethyl,

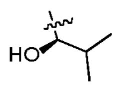

or $-CH_2-CO-NH-CH_3$;

$R_4$ is selected from the group consisting of

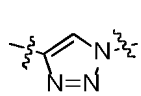 , 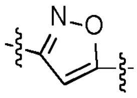

and -NH-;

$R_5$ is selected from the group consisting of H, $C_{1-6}$ alkyl and $C_{1-6}$ haloalkyl;

$R_{12}$ is selected from the group consisting of

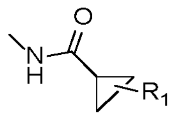 and 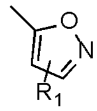 ;

$R_a$ is each independently selected from the group consisting of H, hydroxyl, amino, oxo, cyano, halogen, $C_{1-6}$ alkyl, $C_{1-6}$ haloalkyl, $C_{1-6}$ alkoxy, $-C_{0-3}$ alkylene-$OR_b$, $-OC(=O)C_{1-6}$ alkyl, $-C_{0-3}$ alkylene-$SR_b$, $-C_{0-3}$ alkylene-$N(R_b)_2$, $-C_{0-3}$ alkylene-$S(=O)R_b$, $-C_{0-3}$ alkylene-$S(=O)_2R_b$, $-C_{0-3}$ alkylene-$SR_b$, $-C_{0-3}$ alkylene-$S(R_b)_5$, $-C_{0-3}$ alkylene-$C(=O)R_b$, $-C_{0-3}$ alkylene-$C(=O)OR_b$, $-C_{0-3}$ alkylene-$C(=O)N(R_b)_2$, $C_{2-6}$ alkenyl,

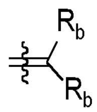 ,

$C_{2-6}$ alkynyl, $-C_{0-3}$ alkylene-$C_{3-14}$ cycloalkyl, $-C_{0-3}$ alkylene-(3- to 14-membered heterocyclyl), $-C_{0-3}$ alkylene-$C_{6-18}$ aryl and $-C_{0-3}$ alkylene-(5- to 18-membered heteroaryl), wherein $C_{1-6}$ alkyl, $C_{1-6}$ haloalkyl, $C_{1-6}$ alkoxy, $C_{2-6}$ alkenyl, $C_{2-6}$ alkynyl, $-C_{0-3}$ alkylene-$C_{3-14}$ cycloalkyl, $-C_{0-3}$ alkylene-(3- to 14-membered heterocyclyl), $-C_{0-3}$ alkylene-$C_{6-18}$ aryl or $-C_{0-3}$ alkylene-(5- to 18-membered heteroaryl) is optionally further substituted by one or more $R_b$; and

each $R_b$ is each independently H, halogen, hydroxyl, cyano, $C_{1-6}$ alkyl, $C_{3-6}$ cycloalkyl, $C_{1-6}$ haloalkyl, $C_{3-14}$ cycloalkyl, 3- to 14-membered heterocyclyl, $C_{6-18}$ aryl or 5- to 18-membered heteroaryl, wherein $C_{1-6}$ alkyl, $C_{3-6}$ cycloalkyl, $C_{1-6}$ haloalkyl, $C_{3-14}$ cycloalkyl, 3- to 14-membered heterocyclyl, $C_{6-18}$ aryl or 5- to 18-membered heteroaryl is optionally further substituted by one or more halogen, $C_{1-6}$ alkyl or $C_{1-6}$ haloalkyl; or two $R_b$ bound to one atom together with the atom to which they are bound form $C_{3-14}$ cycloalkyl, 3- to 14-membered heterocyclyl, $C_{6-18}$ aryl or 5- to 18-membered heteroaryl, wherein $C_{3-14}$ cycloalkyl, 3- to 14-membered heterocyclyl, $C_{6-18}$ aryl or 5- to 18-membered heteroaryl is optionally further substituted by one or more halogen, amino, hydroxy, cyano, $C_{1-6}$ alkyl, $C_{3-6}$ cycloalkyl or $C_{1-6}$ haloalkyl.

**2.** The compound, or the tautomer, racemate, enantiomer, diastereomer thereof, or the mixture thereof, or the pharmaceutically acceptable salt thereof according to claim 1, wherein L is selected from the group consisting of

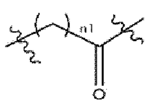 , 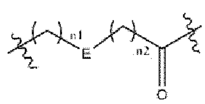 ,

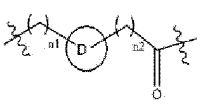 , 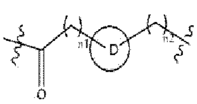 , 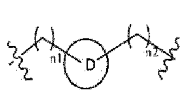 , 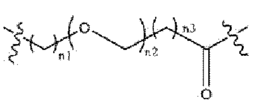 , 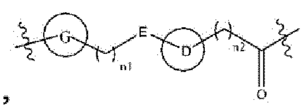 ,

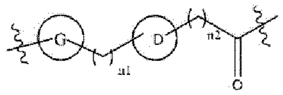, 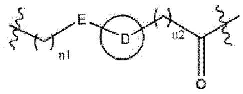, 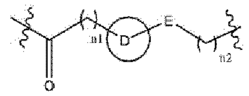, 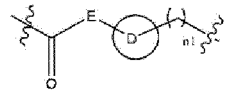,

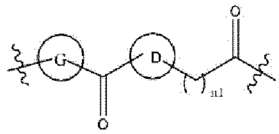, 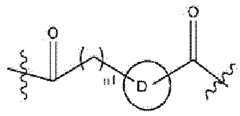, 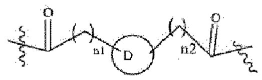, 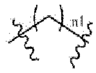, 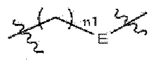,

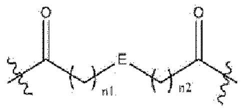, 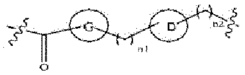, 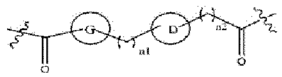, 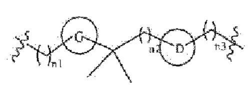,

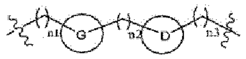, 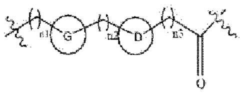, 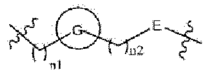, 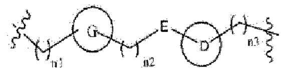,

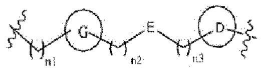, 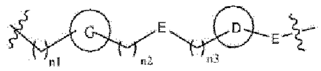, 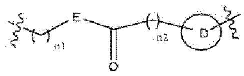, 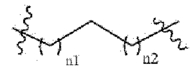,

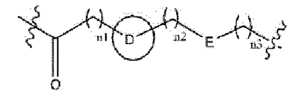, 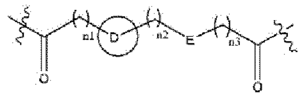, 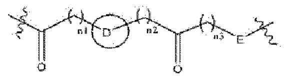, 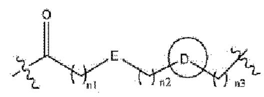

and

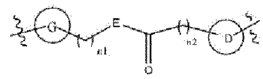;

wherein,

ring G or ring D is each independently selected from the group consisting of $C_{6-14}$ aryl, 5- to 14-membered heteroaryl, $C_{3-14}$ cycloalkyl and 3- to 14-membered heterocyclyl, wherein $C_{6-14}$ aryl, 5- to 14-membered heteroaryl or 3- to 14-membered heterocyclyl is optionally further substituted by one or more $R_a$;
E is each independently selected from the group consisting of -O-, -NH- and $-NCH_3-$;
n1, n2 and n3 are each independently selected from integers ranging from 0 to 10, and preferably n1, n2 and n3 are each independently 0, 1, 2, 3 or 4;
M is a VHL binding fragment represented by general formula (V) or (IV);

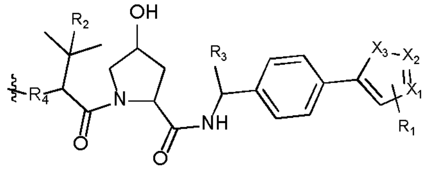

(V)

or

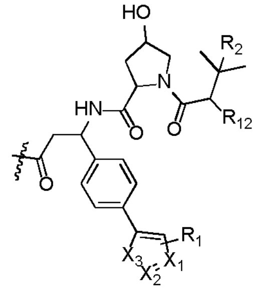

(IV)

$X_1$ is selected from the group consisting of C, CH and N;

$X_2$ is selected from the group consisting of C, CH and N;
$X_3$ is selected from the group consisting of N, NH, O and S;
$R_1$ is selected from the group consisting of H, $C_{1-6}$ alkyl, halogen and cyano, and is preferably methyl, ethyl, F or cyano;
$R_2$ is selected from the group consisting of H and $C_{1-6}$ alkyl, and is preferably H or methyl;
$R_3$ is selected from the group consisting of H, $C_{1-6}$ alkyl and $C_{1-6}$ hydroxylalkyl, and is preferably H, methyl or hydroxylmethyl;
$R_4$ is selected from the group consisting of

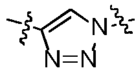 , 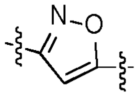

and -NH-; and
$R_{12}$ is selected from the group consisting of

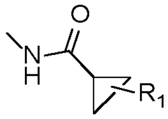 and 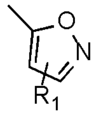 .

**3.** The compound, or the tautomer, racemate, enantiomer, diastereomer thereof, or the mixture thereof, or the pharmaceutically acceptable salt thereof according to claim 2, wherein:

L is selected from the group consisting of

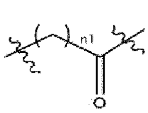 , 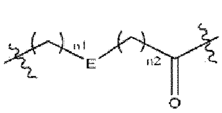 , 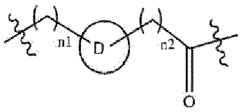 , 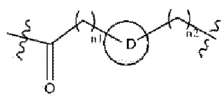 ,

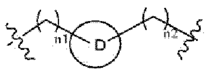 , 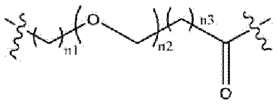 , 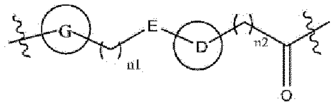 , 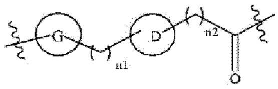 ,

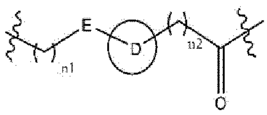 , 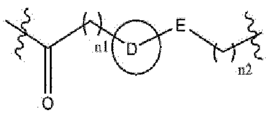 , 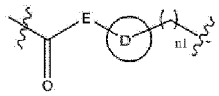 , 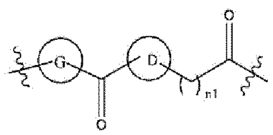 ,

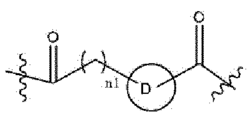 , 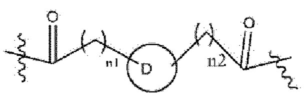 , 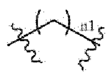 , 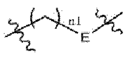 , 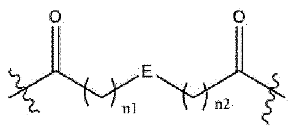 ,

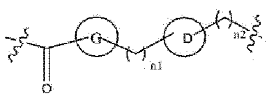 , 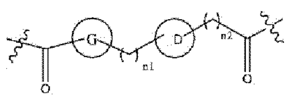 , 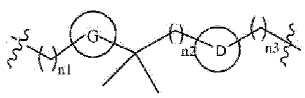 ,

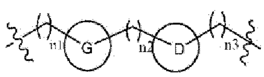 , 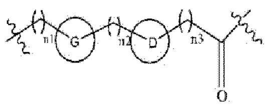 , 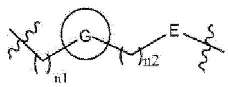 , 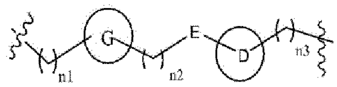 ,

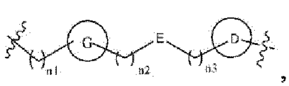, 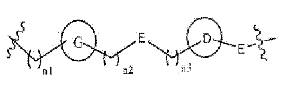, 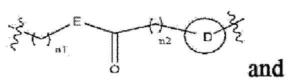 and 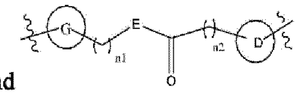;

ring G or ring D is each independently selected from the group consisting of $C_{6-14}$ aryl, 5- to 14-membered heteroaryl and 3- to 14-membered heterocyclyl, wherein $C_{6-14}$ aryl, 5- to 14-membered heteroaryl or 3- to 14-membered heterocyclyl is optionally further substituted by one or more $R_a$;
E is each independently selected from the group consisting of -O-, -NH- and $-NCH_3-$;
n1, n2 and n3 are each independently selected from integers ranging from 0 to 10, and preferably n1, n2 and n3 are each independently 0, 1, 2, 3 or 4;
M is a VHL binding fragment represented by general formula (V) or (IV);

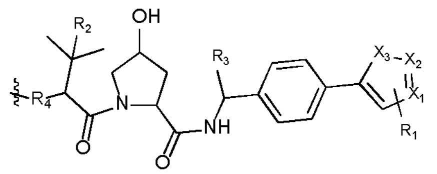

(V)

or

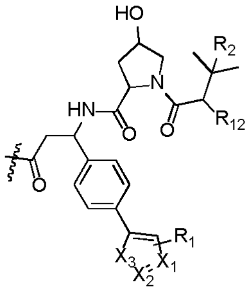

(IV)

$X_1$ is selected from the group consisting of C, CH and N;
$X_2$ is selected from the group consisting of C, CH and N;
$X_3$ is selected from the group consisting of N, NH, O and S;
$R_1$ is selected from the group consisting of H, $C_{1-6}$ alkyl, halogen and cyano, and is preferably methyl, ethyl, F or cyano;
$R_2$ is selected from the group consisting of H and $C_{1-6}$ alkyl, and is preferably H or methyl;
$R_3$ is selected from the group consisting of H, $C_{1-6}$ alkyl and $C_{1-6}$ hydroxylalkyl, and is preferably H, methyl or hydroxylmethyl;
$R_4$ is selected from the group consisting of

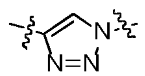, 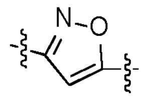

and -NH-;
$R_{12}$ is selected from the group consisting of

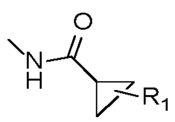 and 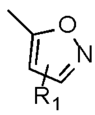;

$R_a$ is independently selected from the group consisting of H, hydroxyl, amino, oxo, cyano, halogen, $C_{1-6}$ alkyl, $C_{1-6}$ haloalkyl, $C_{1-6}$ alkoxy, $-C_{0-3}$ alkylene-$OR_b$, $-OC(=O)C_{1-6}$ alkyl, $-C_{0-3}$ alkylene-$SR_b$, $-C_{0-3}$ alkylene-$N(R_b)_2$, $-C_{0-3}$ alkylene-$S(=O)R_b$, $-C_{0-3}$ alkylene-$S(=O)_2R_b$, $-C_{0-3}$ alkylene-$SR_b$, $-C_{0-3}$ alkylene-$S(R_b)_5$, $-C_{0-3}$ alkylene-$C(=O)R_b$, $-C_{0-3}$ alkylene-$C(=O)OR_b$, $-C_{0-3}$ alkylene-$C(=O)N(R_b)_2$, $C_{2-6}$ alkenyl, $C_{2-6}$ alkynyl, $-C_{0-3}$ alkylene-$C_{3-14}$ cycloalkyl, $-C_{0-3}$ alkylene-(3- to 14-membered heterocyclyl), $-C_{0-3}$ alkylene-$C_{6-18}$ aryl and $-C_{0-3}$ alkylene-(5- to 18-membered heteroaryl), wherein $C_{1-6}$ alkyl, $C_{1-6}$ haloalkyl, $C_{1-6}$ alkoxy, $C_{2-6}$ alkenyl, $C_{2-6}$ alkynyl, $-C_{0-3}$ alkylene-$C_{3-14}$ cycloalkyl, $-C_{0-3}$ alkylene-(3- to 14-membered heterocyclyl), $-C_{0-3}$ alkylene-$C_{6-18}$ aryl or $-C_{0-3}$ alkylene-(5- to 18-membered heteroaryl) is optionally further substituted by one or more $R_b$; and
each $R_b$ is independently H, halogen, hydroxyl, cyano, $C_{1-6}$ alkyl, $C_{3-6}$ cycloalkyl, $C_{1-6}$ haloalkyl, $C_{3-14}$ cycloalkyl, 3- to 14-membered heterocyclyl, $C_{6-18}$ aryl or 5- to 18-membered heteroaryl, wherein $C_{1-6}$ alkyl, $C_{3-6}$ cycloalkyl, $C_{1-6}$ haloalkyl, $C_{3-14}$ cycloalkyl, 3- to 14-membered heterocyclyl, $C_{6-18}$ aryl or 5- to 18-membered heteroaryl is

optionally further substituted by one or more halogen, $C_{1-6}$ alkyl or $C_{1-6}$ haloalkyl; or two $R_b$ bound to one atom together with the atom to which they are bound form $C_{3-14}$ cycloalkyl, 3- to 14-membered heterocyclyl, $C_{6-18}$ aryl or 5- to 18-membered heteroaryl, wherein $C_{3-14}$ cycloalkyl, 3- to 14-membered heterocyclyl, $C_{6-18}$ aryl or 5- to 18-membered heteroaryl is optionally further substituted by one or more halogen, amino, hydroxy, cyano, $C_{1-6}$ alkyl, $C_{3-6}$ cycloalkyl or $C_{1-6}$ haloalkyl.

**4.** The compound, or the tautomer, racemate, enantiomer, diastereomer thereof, or the mixture thereof, or the pharmaceutically acceptable salt thereof according to any one of claims 1 to 3, wherein:

F is a KRAS protein binding fragment represented by general formula (I) or (II);

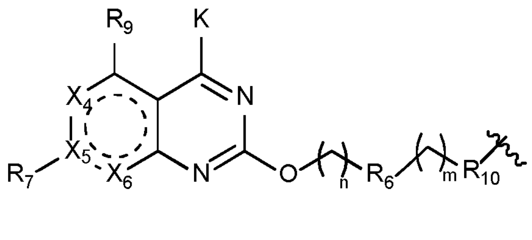

(I)

or

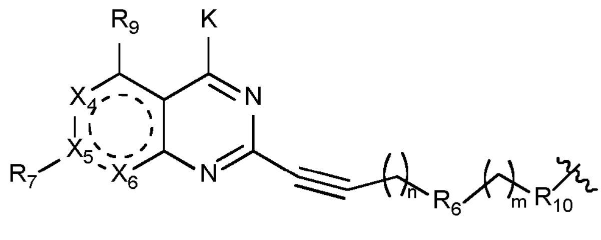

(II)

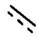 represents a bond independently selected from the group consisting of a single bond and a double bond;
$X_4$ is $CR_8$, $C(R_8)_2$, O, N or $NR_8$; $R_8$ is each independently selected from the group consisting of H, hydroxyl, oxo, halogen, cyano, $C_{1-6}$ alkyl, $C_{1-6}$ haloalkyl, -S-$C_{1-6}$ alkyl, -$C_{0-3}$ alkylene-$C_{2-4}$ alkenyl, $C_{1-6}$ alkoxy, $C_{1-6}$ haloalkoxy and $C_{3-14}$ cycloalkyl, wherein $C_{3-14}$ cycloalkyl or $C_{1-6}$ alkyl is optionally further substituted by one or more $R_a$;
$X_5$ is selected from the group consisting of C, CH and N;
$X_6$ is selected from the group consisting of $CR_{14}$, $C(R_{14})_2$, O, N and $NR_{14}$; $R_{14}$ is each independently selected from the group consisting of H, hydroxyl, oxo, halogen, cyano, $C_{1-6}$ alkyl, $C_{1-6}$ haloalkyl, -S-$C_{1-6}$ alkyl, -$C_{0-3}$ alkylene-$C_{2-4}$ alkenyl, $C_{1-6}$ alkoxy, $C_{1-6}$ haloalkoxy and $C_{3-14}$ cycloalkyl, wherein $C_{3-14}$ cycloalkyl or $C_{1-6}$ alkyl is optionally further substituted by one or more $R_a$;
$R_6$ is absent or is selected from the group consisting of $C_{3-14}$ cycloalkyl and 3- to 14-membered heterocyclyl, wherein $C_{3-14}$ cycloalkyl or 3- to 14-membered heterocyclyl is optionally further substituted by one or more $R_a$;
$R_9$ is selected from the group consisting of H, amino, substituted amino, cyano, $C_{1-6}$ alkyl, $C_{1-6}$ alkoxy, halogen, $C_{2-6}$ alkenyl and $C_{3-6}$ cycloalkyl, wherein $C_{1-6}$ alkyl, $C_{2-6}$ alkenyl or $C_{3-6}$ cycloalkyl is further substituted by one or more $R_a$;
$R_{10}$ is absent or is selected from the group consisting of -O- and -$NR_{11}$-; $R_{11}$ is selected from the group consisting of H, $C_{1-6}$ alkyl, $C_{1-6}$ haloalkyl, $C_{1-6}$ hydroxylalkyl and $C_{1-6}$ aminoalkyl;
$R_7$ is selected from the group consisting of $C_{3-14}$ cycloalkyl, 3- to 14-membered heterocyclyl, $C_{6-18}$ aryl and 5- to 18-membered heteroaryl, wherein $C_{3-14}$ cycloalkyl, 3- to 14-membered heterocyclyl, $C_{6-18}$ aryl or 5- to 18-membered heteroaryl is optionally further substituted by one or more $R_a$;
K is selected from the group consisting of $C_{3-14}$ cycloalkyl and 3- to 14-membered heterocyclyl, wherein $C_{3-14}$ cycloalkyl or 3- to 14-membered heterocyclyl is a monocyclic ring, a fused ring, a spiro ring or a bridged ring, optionally further substituted by one or more $R_a$, wherein 3- to 14-membered heterocyclyl is not

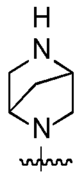, 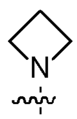, 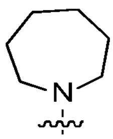, 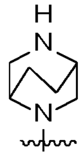, 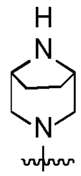, 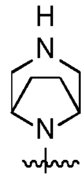

or

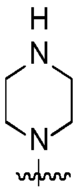;

and

m and n are each independently selected from the group consisting of 0, 1, 2, 3, 4 and 5.

5. The compound, or the tautomer, racemate, enantiomer, diastereomer thereof, or the mixture thereof, or the pharmaceutically acceptable salt thereof according to any one of claims 1 to 4, wherein $R_4$ is selected from the group consisting of

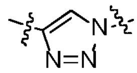

and -NH-.

6. The compound, or the tautomer, racemate, enantiomer, diastereomer thereof, or the mixture thereof, or the pharmaceutically acceptable salt thereof according to any one of claims 1 to 4, wherein $R_4$ is

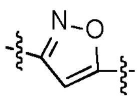.

7. The compound, or the tautomer, racemate, enantiomer, diastereomer thereof, or the mixture thereof, or the pharmaceutically acceptable salt thereof according to any one of claims 1 to 6, wherein K is selected from the group consisting of $C_{3-14}$ cycloalkyl and 3- to 14-membered heterocyclyl, wherein $C_{3-14}$ cycloalkyl or 3- to 14-membered heterocyclyl is a monocyclic ring and is optionally further substituted by one or more $R_a$.

8. The compound, or the tautomer, racemate, enantiomer, diastereomer thereof, or the mixture thereof, or the pharmaceutically acceptable salt thereof according to claim 7, wherein K is selected from the group consisting of

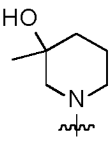, 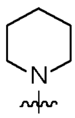, 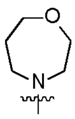, 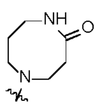, 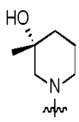, 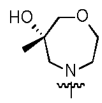,

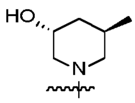, 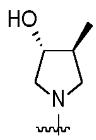, 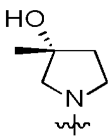, 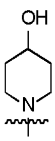, 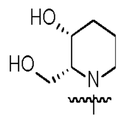, 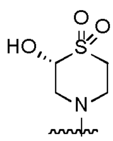, 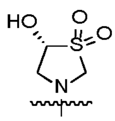, 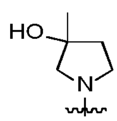 and 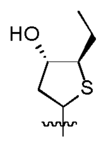.

9. The compound, or the tautomer, racemate, enantiomer, diastereomer thereof, or the mixture thereof, or the pharmaceutically acceptable salt thereof according to any one of claims 1 to 6, wherein K is $C_{3-14}$ cycloalkyl or 3- to 14-membered heterocyclyl, wherein $C_{3-14}$ cycloalkyl or 3- to 14-membered heterocyclyl is a spiro ring and is optionally further substituted by one or more $R_a$.

10. The compound, or the tautomer, racemate, enantiomer, diastereomer thereof, or the mixture thereof, or the pharmaceutically acceptable salt thereof according to claim 9, wherein K is selected from the group consisting of

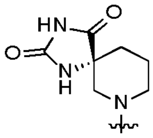, 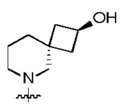, 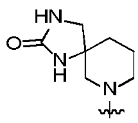, 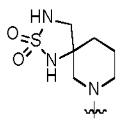, 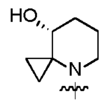,

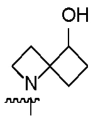, 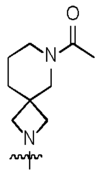, 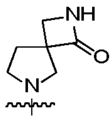, 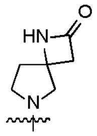, 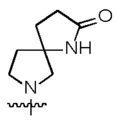, 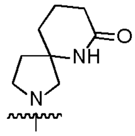, 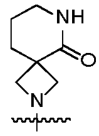, 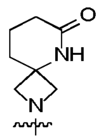, and 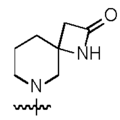.

**11.** The compound, or the tautomer, racemate, enantiomer, diastereomer thereof, or the mixture thereof, or the pharmaceutically acceptable salt thereof according to any one of claims 1 to 6, wherein K is $C_{3-14}$ cycloalkyl or 3- to 14-membered heterocyclyl, wherein $C_{3-14}$ cycloalkyl or 3- to 14-membered heterocyclyl is a fused ring and is optionally further substituted by one or more $R_a$.

**12.** The compound, or the tautomer, racemate, enantiomer, diastereomer thereof, or the mixture thereof, or the pharmaceutically acceptable salt thereof according to claim 11, wherein K is selected from the group consisting of

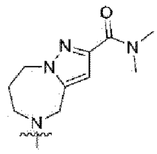, 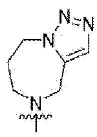, 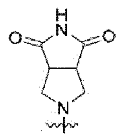,

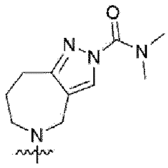, 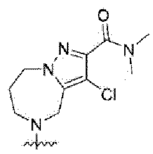, 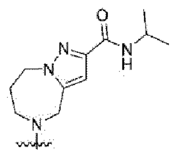, 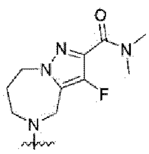, 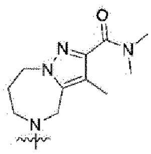, 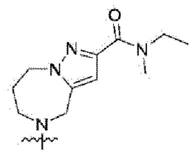,

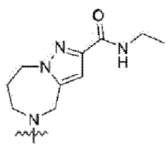, 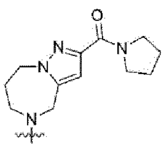, 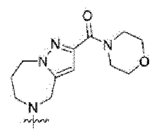, 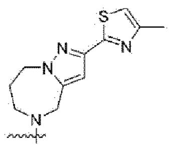, 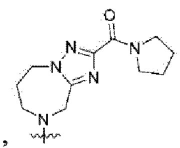, 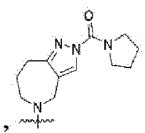, 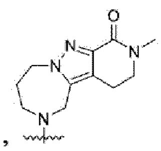,

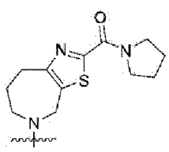, 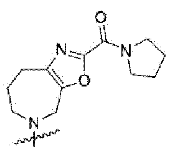, 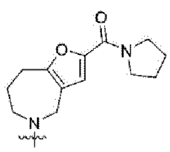, 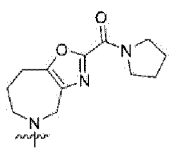, 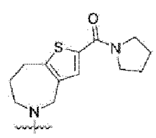, 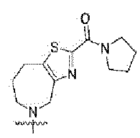, 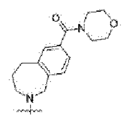,

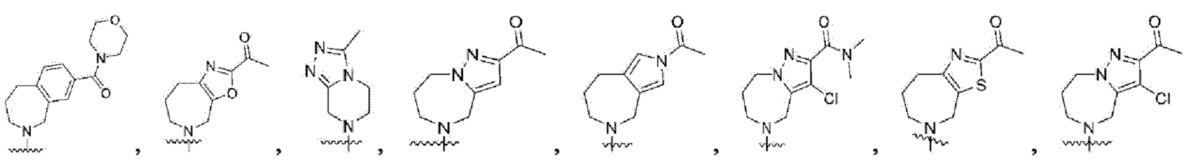, , , , , , , ,

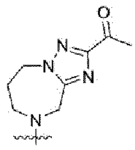, 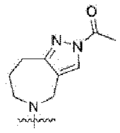, 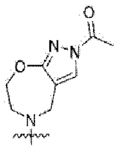, 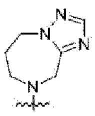, 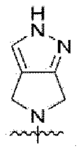 and 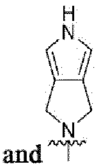.

**13.** The compound, or the tautomer, racemate, enantiomer, diastereomer thereof, or the mixture thereof, or the pharmaceutically acceptable salt thereof according to any one of claims 1 to 6, wherein K is $C_{3-14}$ cycloalkyl or 3- to 14-membered heterocyclyl, wherein $C_{3-14}$ cycloalkyl or 3- to 14-membered heterocyclyl is a bridged ring and is optionally further substituted by one or more $R_a$.

**14.** The compound, or the tautomer, racemate, enantiomer, diastereomer thereof, or the mixture thereof, or the pharmaceutically acceptable salt thereof according to claim 13, wherein K is selected from the group consisting of

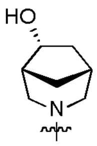, 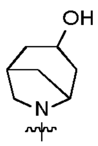, 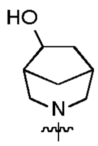, 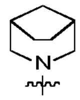, 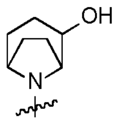, 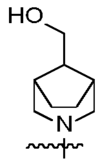,

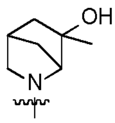, 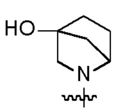, 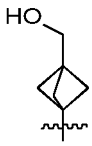, 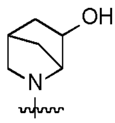, 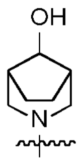, 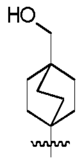, 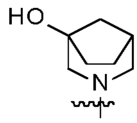, 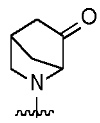, 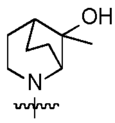

and

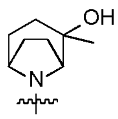.

**15.** The compound, or the tautomer, racemate, enantiomer, diastereomer thereof, or the mixture thereof, or the pharmaceutically acceptable salt thereof according to any one of claims 1 to 14, wherein K is selected from the group consisting of

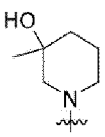, 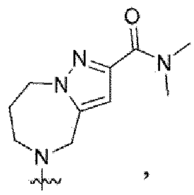, 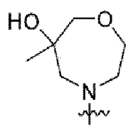, 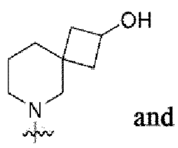 and 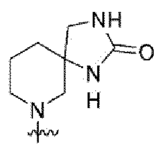.

**16.** The compound, or the tautomer, racemate, enantiomer, diastereomer thereof, or the mixture thereof, or the pharmaceutically acceptable salt thereof according to any one of claims 1 to 15, wherein $R_7$ is selected from the group consisting of $C_{6-18}$ aryl and 5- to 18-membered heteroaryl, wherein $C_{6-18}$ aryl or 5- to 18-membered heteroaryl is optionally further substituted by one or more $R_a$, wherein $R_a$ is each independently selected from the group consisting of H, halogen, hydroxyl, amino, cyano, $C_{2-6}$ alkynyl, $C_{1-6}$ alkyl, $C_{1-6}$ haloalkyl and $C_{2-6}$ alkenyl.

**17.** The compound, or the tautomer, racemate, enantiomer, diastereomer thereof, or the mixture thereof, or the pharmaceutically acceptable salt thereof according to any one of claims 1 to 16, wherein $R_7$ is selected from the group consisting of

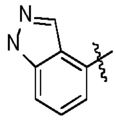, 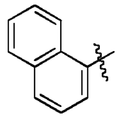, 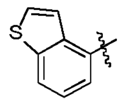,

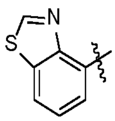, 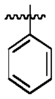, 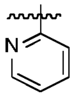, 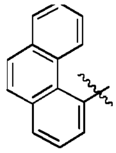, 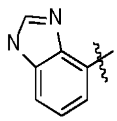, 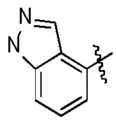, 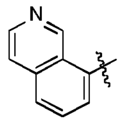 and 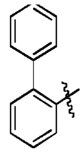,

wherein

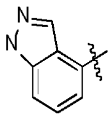, 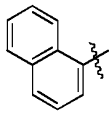, 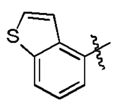, 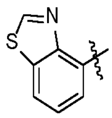, 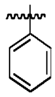, 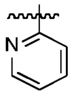, 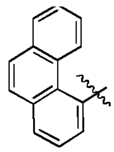, 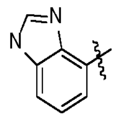, 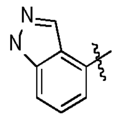,

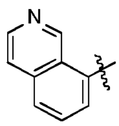 or 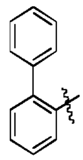

is optionally further substituted by one or more $R_a$, wherein $R_a$ is each independently selected from the group consisting of H, halogen, hydroxyl, amino, cyano, $C_{2-6}$ alkynyl, $C_{1-6}$ alkyl, $C_{1-6}$ haloalkyl and $C_{2-6}$ alkenyl.

**18.** The compound, or the tautomer, racemate, enantiomer, diastereomer thereof, or the mixture thereof, or the pharmaceutically acceptable salt thereof according to any one of claims 1 to 17, wherein $R_7$ is selected from the group consisting of

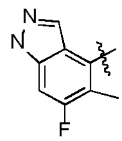, 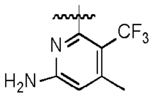, 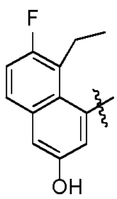, 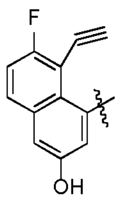, 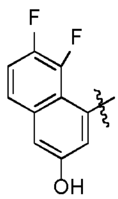, 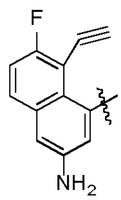, 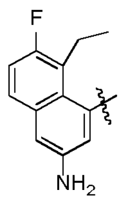,

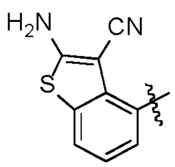, 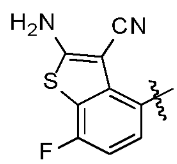, 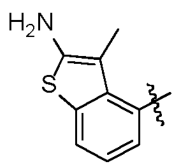 and 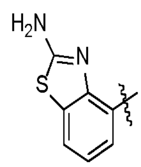.

**19.** The compound, or the tautomer, racemate, enantiomer, diastereomer thereof, or the mixture thereof, or the pharmaceutically acceptable salt thereof according to any one of claims 1 to 18, wherein F is selected from the group consisting of

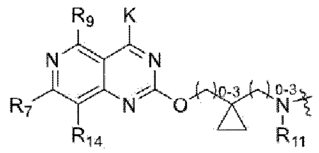 ,

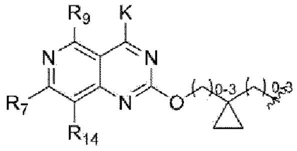 ,

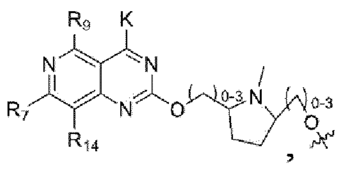 ,

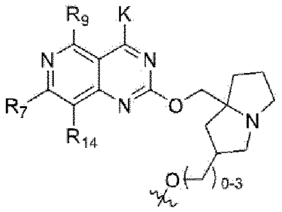 ,

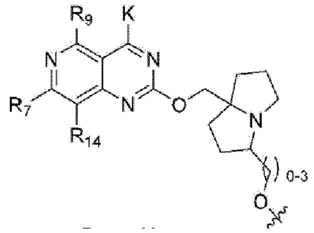 ,

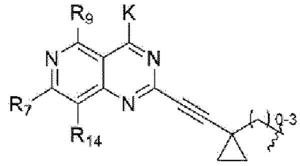 ,

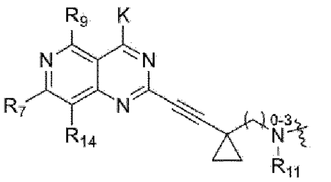 ,

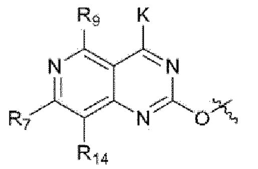 ,

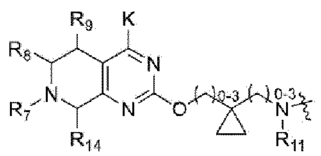 ,

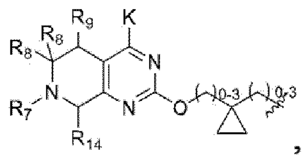 ,

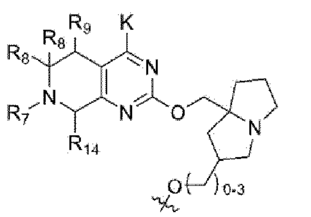 ,

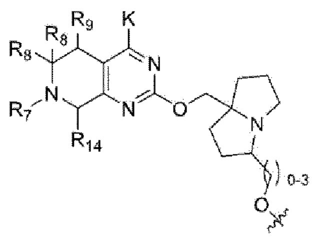 ,

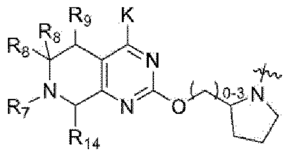 ,

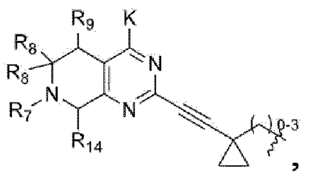 ,

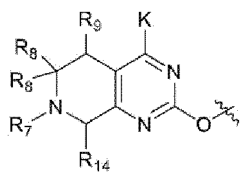 ,

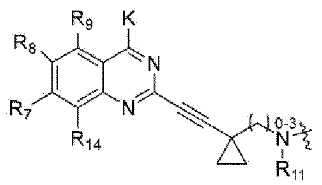 ,

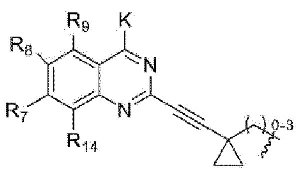 ,

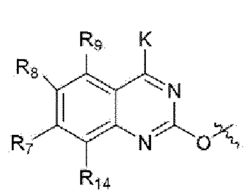 ,

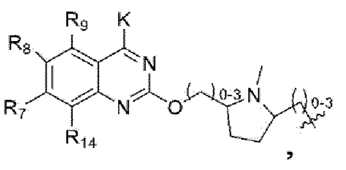 ,

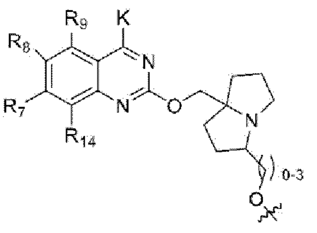 ,

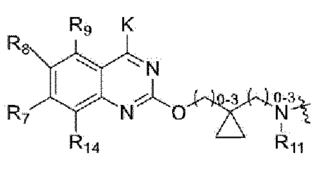 ,

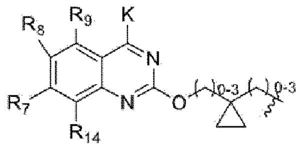,
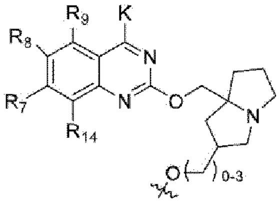 and 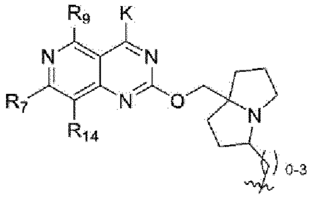,

wherein, the definitions of $R_7$, $R_9$, $R_8$, $R_{11}$ and $R_{14}$ are as defined in claim 1; wherein K is selected from the group consisting of

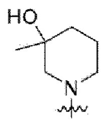, 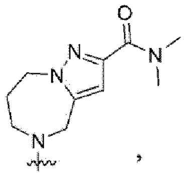, 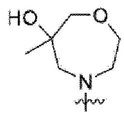, 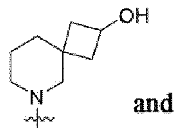 and 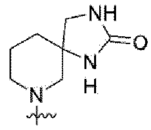.

20. The compound, or the tautomer, racemate, enantiomer, diastereomer thereof, or the mixture thereof, or the pharmaceutically acceptable salt thereof according to any one of claims 1 to 19, wherein K is selected from the group consisting of

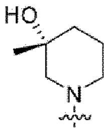, 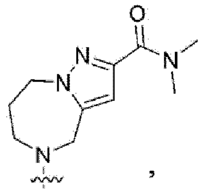, 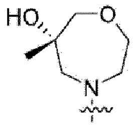, 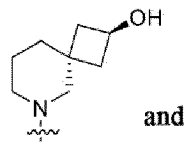 and 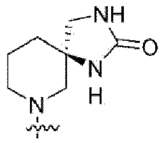.

21. The compound, or the tautomer, racemate, enantiomer, diastereomer thereof, or the mixture thereof, or the pharmaceutically acceptable salt thereof according to any one of claims 1 to 20, wherein F is selected from the group consisting of

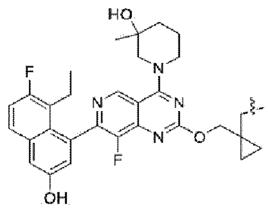, 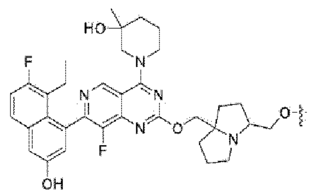, 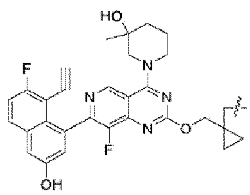, 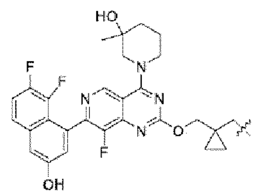,

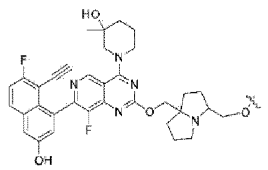, 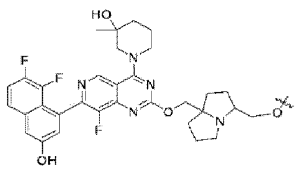, 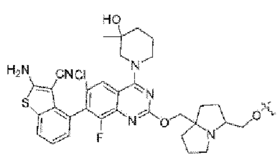, 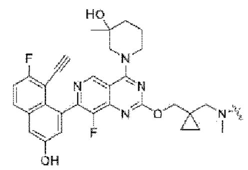,

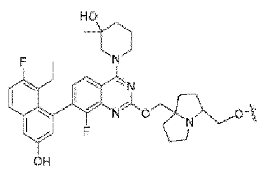, 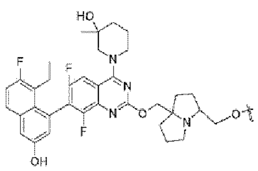, 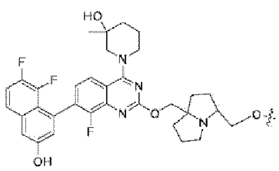, 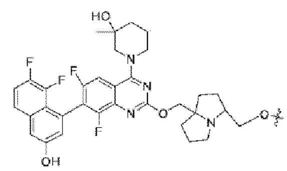,

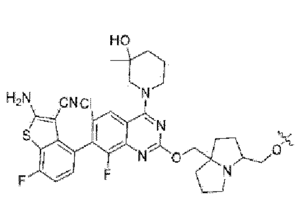 , 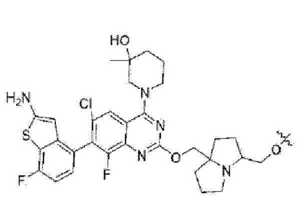 , 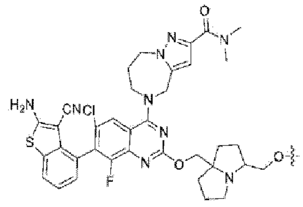 , 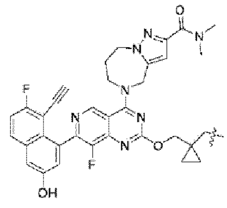 ,

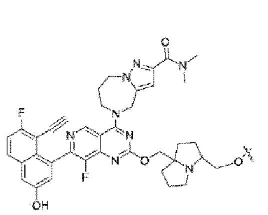 , 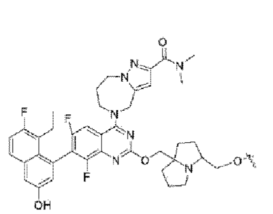 , 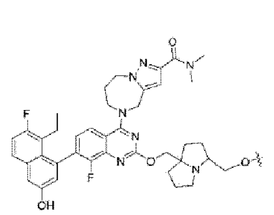 , 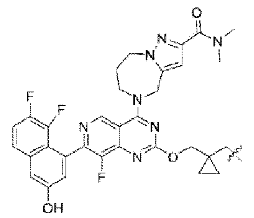 ,

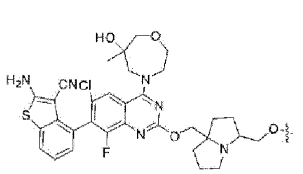 , 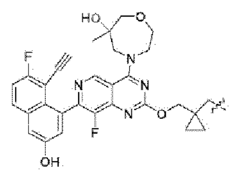 , 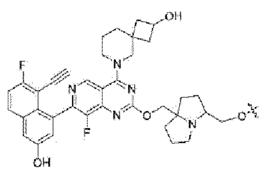 , 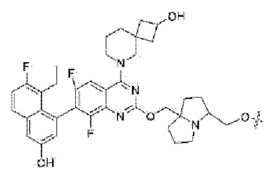 ,

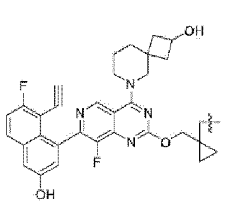 , 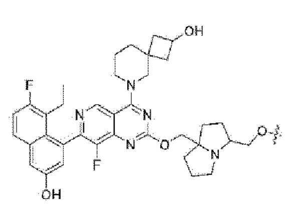 , 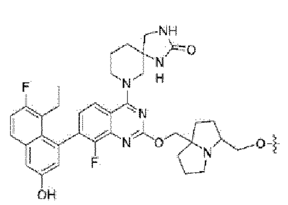 , 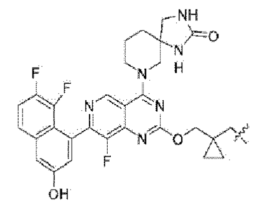 ,

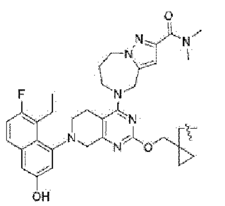 , 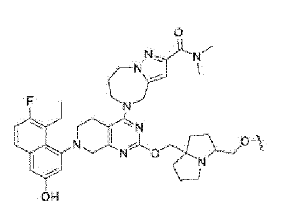 , 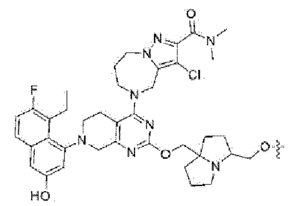 , 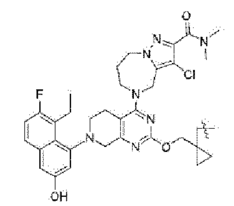 ,

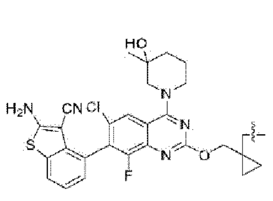 , 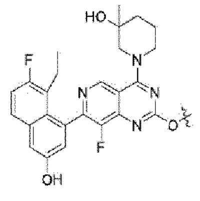 , 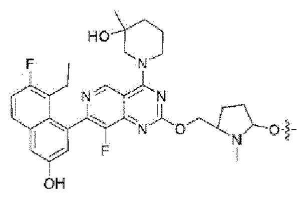 , 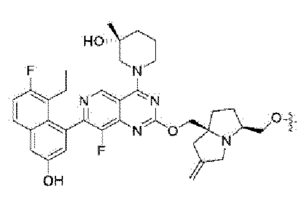 ,

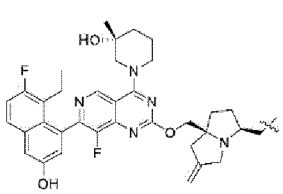 , 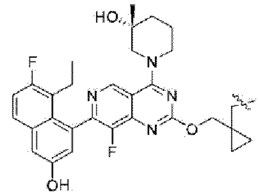 , 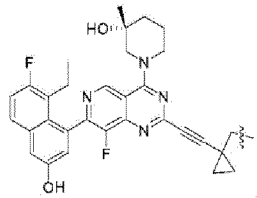 and 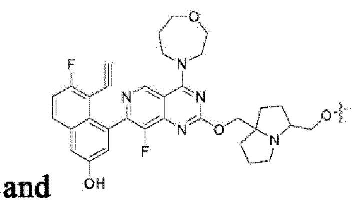 .

**22.** The compound, or the tautomer, racemate, enantiomer, diastereomer thereof, or the mixture thereof, or the pharmaceutically acceptable salt thereof according to any one of claims 1 to 21, wherein F is selected from the group consisting of

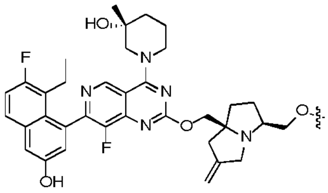

,

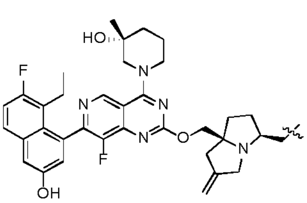, 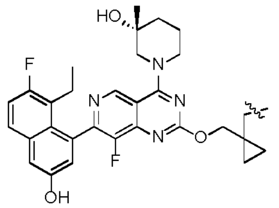 and 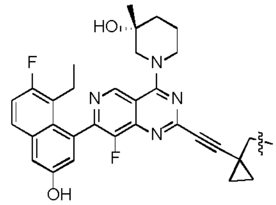.

23. The compound, or the tautomer, racemate, enantiomer, diastereomer thereof, or the mixture thereof, or the pharmaceutically acceptable salt thereof according to any one of claims 1 to 22, wherein, ring G or ring D in L is each independently selected from the group consisting of $C_{6-14}$ aryl, 5- to 14-membered heteroaryl, $C_{3-14}$ cycloalkyl and 3- to 14-membered heterocyclyl, wherein $C_{6-14}$ aryl, 5- to 14-membered heteroaryl, $C_{3-14}$ cycloalkyl or 3- to 14-membered heterocyclyl is optionally further substituted by halogen or $C_{1-3}$ alkyl;

$C_{6-14}$ aryl is preferably

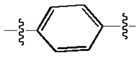;

5- to 14-membered heteroaryl is preferably selected from the group consisting of

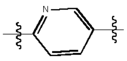, 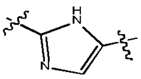, 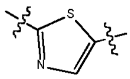, 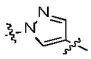, 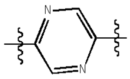, 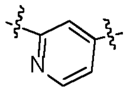, 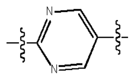, 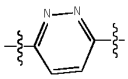

, 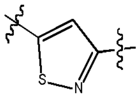, 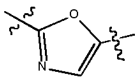 and 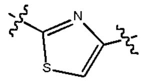;

3- to 14-membered heterocyclyl is preferably selected from the group consisting of

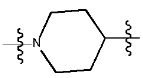, 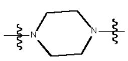, 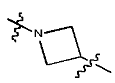, 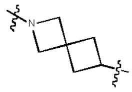, 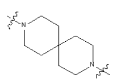, 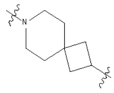, 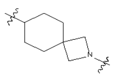, 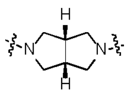,

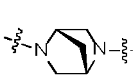, 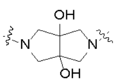, 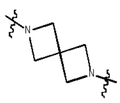 and 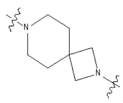;

and
$C_{3-14}$ cycloalkyl is preferably selected from the group consisting of

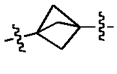

and

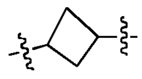.

**24.** The compound, or the tautomer, racemate, enantiomer, diastereomer thereof, or the mixture thereof, or the pharmaceutically acceptable salt thereof according to any one of claims 1 to 23, wherein, ring G or ring D in L is each independently selected from the group consisting of $C_{6-14}$ aryl, 5- to 14-membered heteroaryl, $C_{3-14}$ cycloalkyl and 3- to 14-membered heterocyclyl, wherein $C_{6-14}$ aryl, 5- to 14-membered heteroaryl, $C_{3-14}$ cycloalkyl or 3- to 14-membered heterocyclyl is optionally further substituted by halogen or $C_{1-3}$ alkyl;

$C_{6-14}$ aryl is preferably

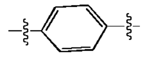;

5- to 14-membered heteroaryl is preferably selected from the group consisting of

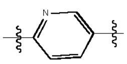, 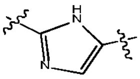, 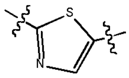, 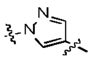, 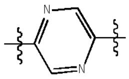, 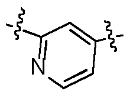, 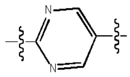, 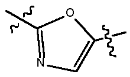

and

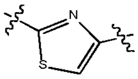;

3- to 14-membered heterocyclyl is preferably selected from the group consisting of

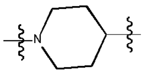, 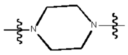, 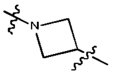, 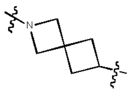, 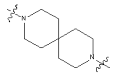, 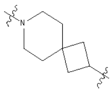, 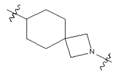, 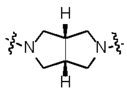,

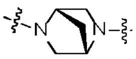, 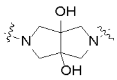, 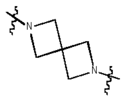 and 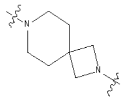;

and

$C_{3-14}$ cycloalkyl is preferably selected from the group consisting of

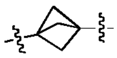

and

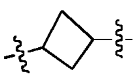.

**25.** The compound, or the tautomer, racemate, enantiomer, diastereomer thereof, or the mixture thereof, or the pharmaceutically acceptable salt thereof according to any one of claims 1 to 24, wherein, ring G or ring D in L is each independently selected from the group consisting of $C_{6-14}$ aryl, 5- to 14-membered heteroaryl and 3- to 14-membered heterocyclyl, wherein $C_{6-14}$ aryl, 5- to 14-membered heteroaryl or 3- to 14-membered heterocyclyl is optionally further substituted by halogen or $C_{1-3}$ alkyl;

$C_{6-14}$ aryl is preferably

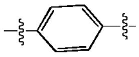;

5- to 14-membered heteroaryl is preferably selected from the group consisting of

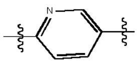, 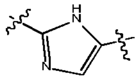, 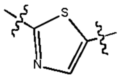, 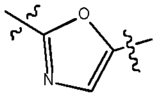 and 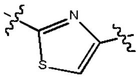;

and

3- to 14-membered heterocyclyl is preferably selected from the group consisting of

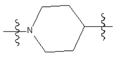, 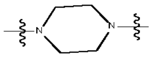, 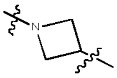, 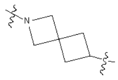, 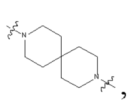, 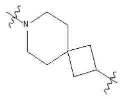, 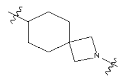, 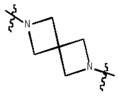

and

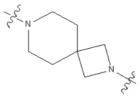.

**26.** The compound, or the tautomer, racemate, enantiomer, diastereomer thereof, or the mixture thereof, or the pharmaceutically acceptable salt thereof according to any one of claims 1 to 25, wherein L is selected from the group consisting of

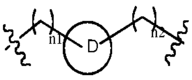,

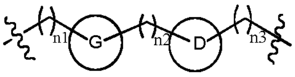, 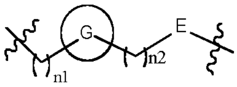, 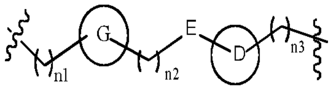,

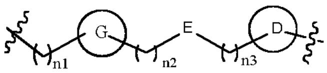, 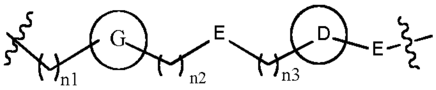 and 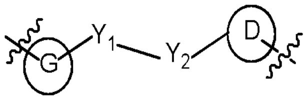;

wherein,

ring G or ring D is each independently selected from the group consisting of $C_{6-14}$ aryl, 5- to 14-membered heteroaryl, $C_{3-14}$ cycloalkyl and 3- to 14-membered heterocyclyl, wherein $C_{6-14}$ aryl, 5- to 14-membered heteroaryl or 3- to 14-membered heterocyclyl is optionally further substituted by one or more $R_a$, wherein $R_a$ is independently selected from the group consisting of H, hydroxyl, amino, oxo, cyano, halogen, $C_{1-6}$ alkyl, $C_{1-6}$ haloalkyl and $C_{1-6}$ alkoxy;

$Y_1$ and $Y_2$ are each independently absent or selected from the group consisting of -O-, -NH-, 3- to 6-membered N-containing heterocyclyl, $C_{1-6}$ alkylene, carbonyl, acetylenyl and $C_{3-6}$ cycloalkyl;

E is each independently selected from the group consisting of -O-, -NH- and -$NCH_3$-; and

n1, n2 and n3 are each independently selected from integers ranging from 0 to 10, and preferably n1, n2 and n3 are each independently 0, 1, 2, 3 or 4.

**27.** The compound, or the tautomer, racemate, enantiomer, diastereomer thereof, or the mixture thereof, or the pharmaceutically acceptable salt thereof according to any one of claims 1 to 26, wherein L is selected from the group consisting of the following structures:

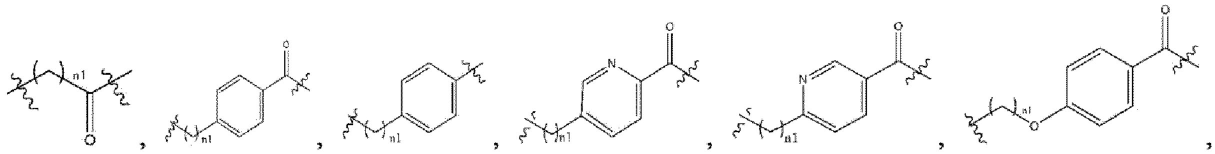

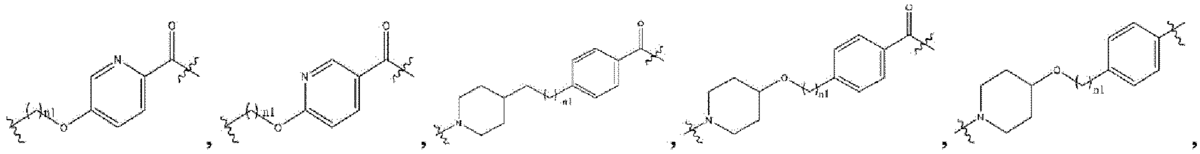

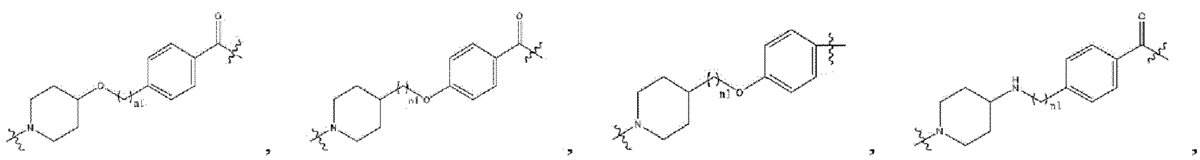

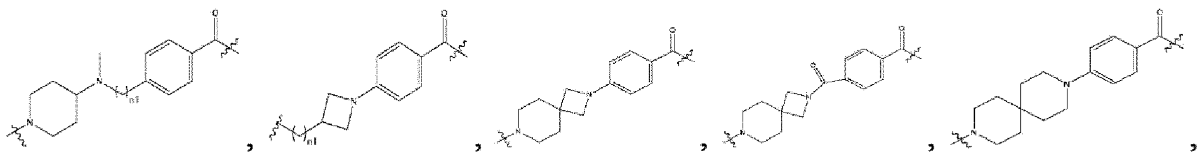

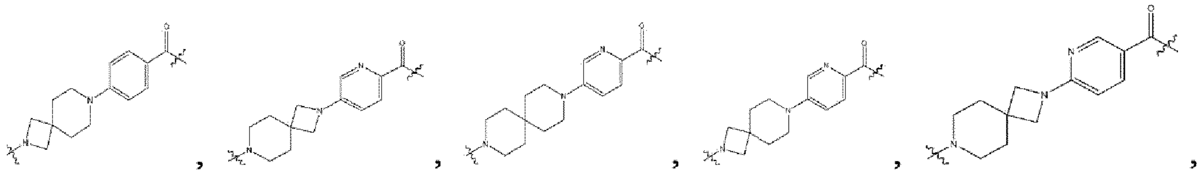

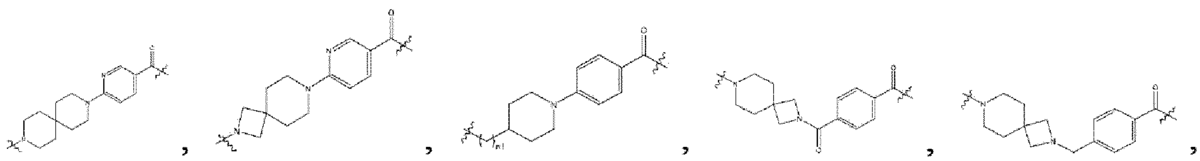

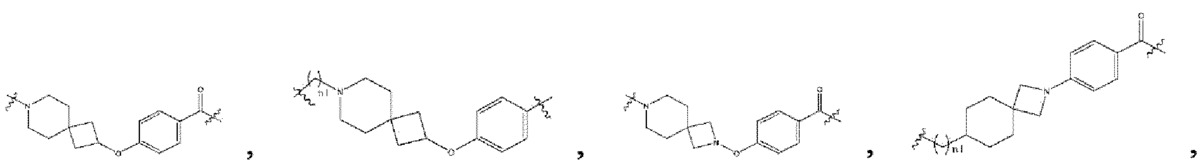

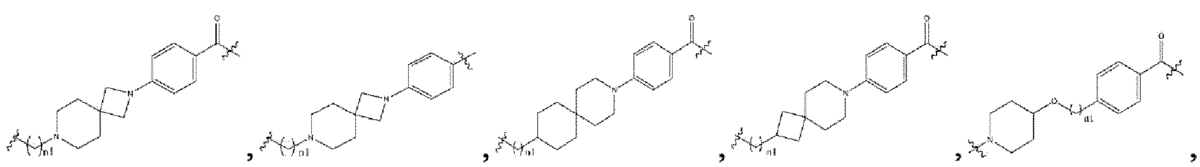

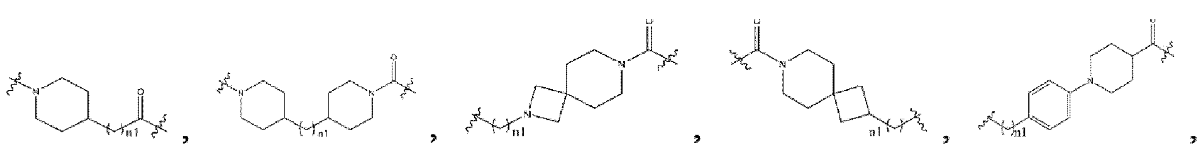

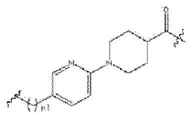, 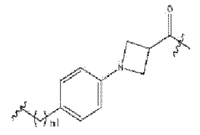, 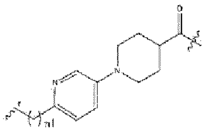, 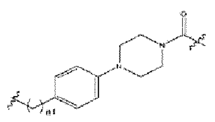, 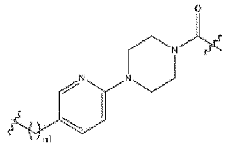,

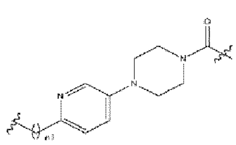, 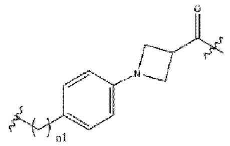, 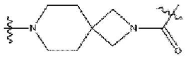, 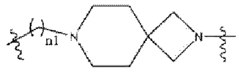, 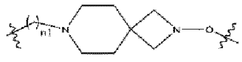,

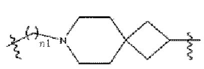, 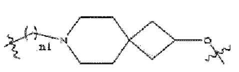, 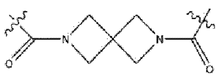, 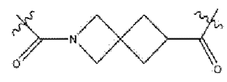, 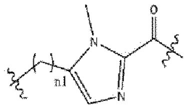,

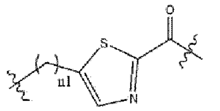, 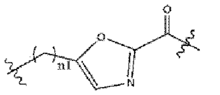, 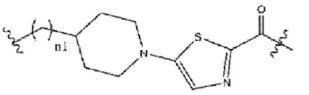, 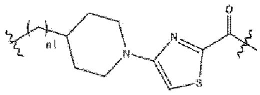,

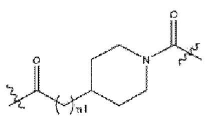, 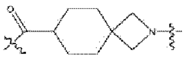, 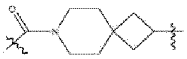, 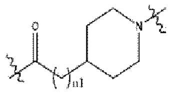, 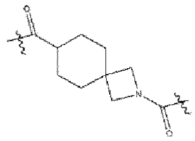, 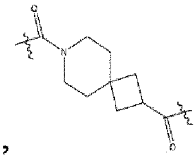,

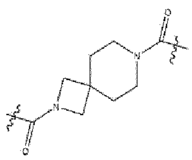, 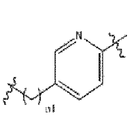, 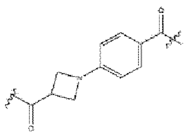, 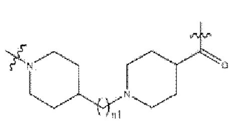, 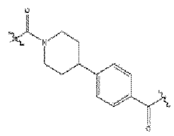, 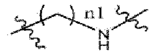,

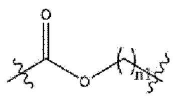, 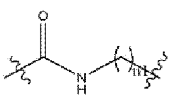, 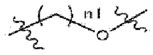, 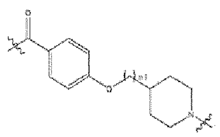, 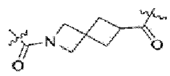, 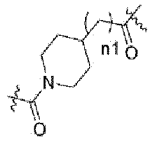,

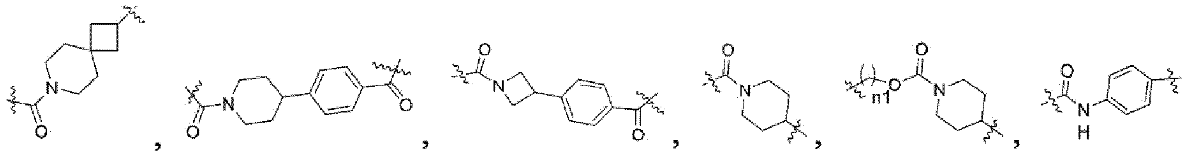, , , , , ,

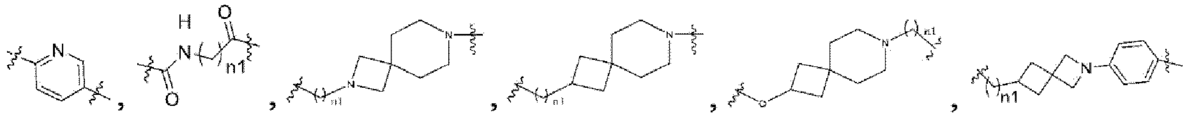, , , , , ,

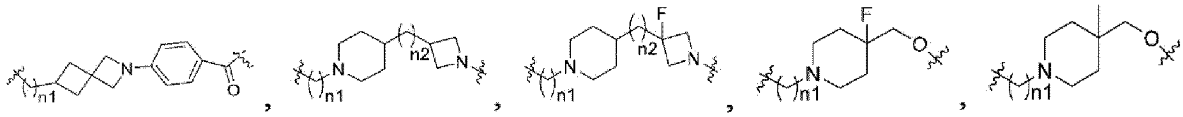, , , , ,

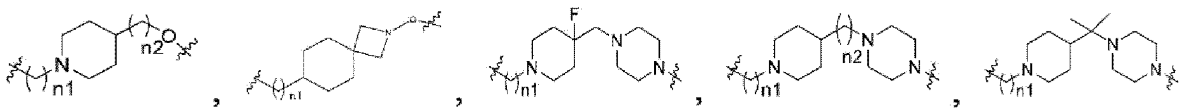 , , , , ,

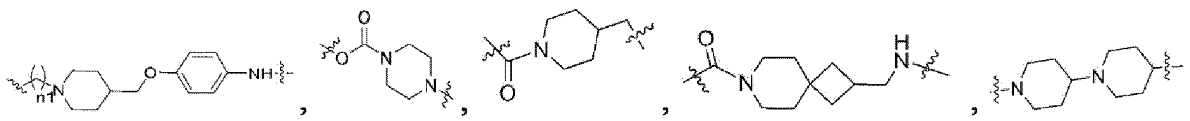 , , , , ,

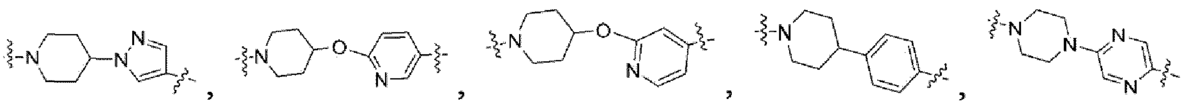 , , , , ,

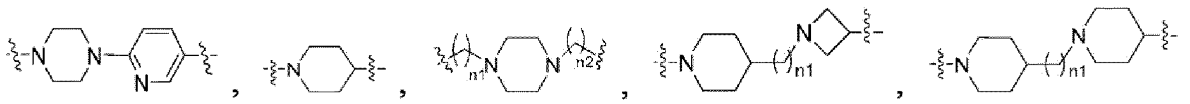 , , , , ,

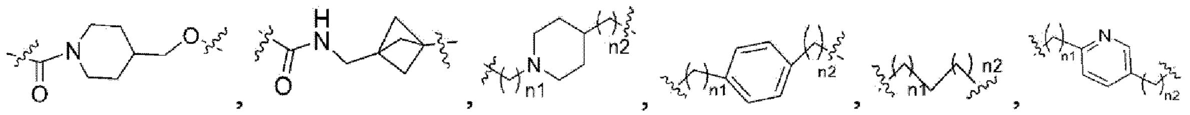 , , , , , ,

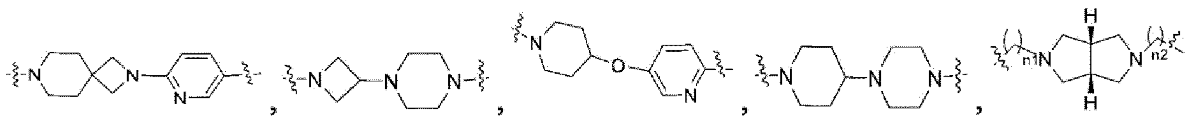 , , , , ,

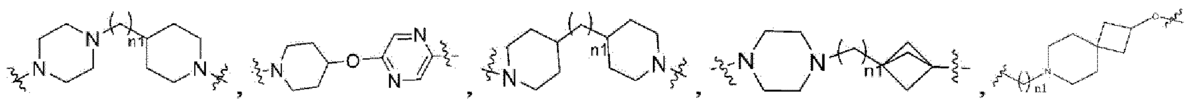 , , , , ,

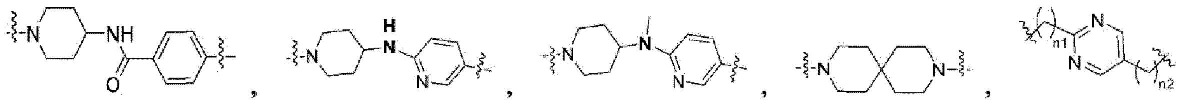 , , , , ,

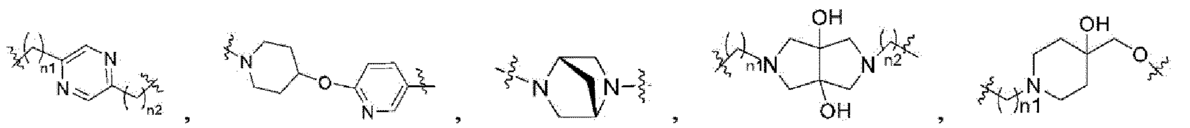 , , , , ,

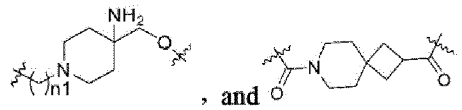 , and ,

wherein, n1 and n2 are each independently selected from integers ranging from 0 to 10, and preferably n1 and n2 are each independently 0, 1, 2 or 3.

**28.** The compound, or the tautomer, racemate, enantiomer, diastereomer thereof, or the mixture thereof, or the pharmaceutically acceptable salt thereof according to any one of claims 1 to 29, wherein L is selected from the following structures:

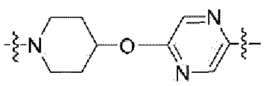, 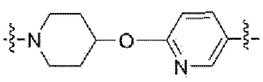, 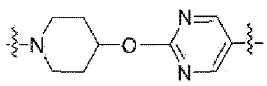, 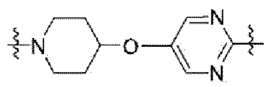,

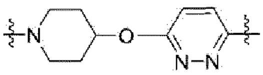, 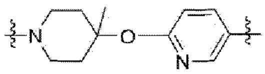, 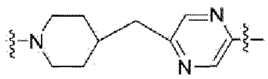, 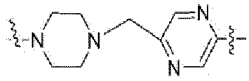,

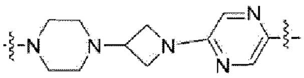, 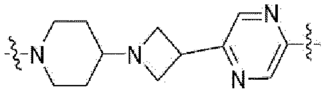, 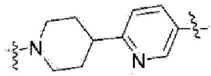, 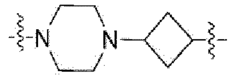,

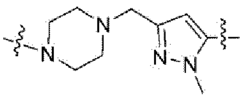, 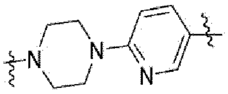, 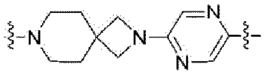, 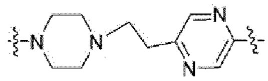,

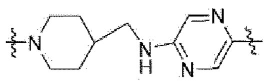, 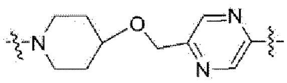, 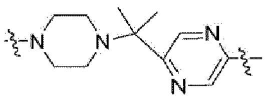, 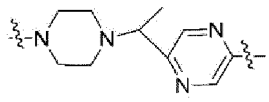,

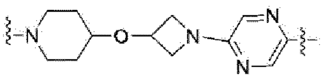, 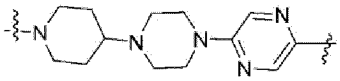, 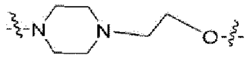,

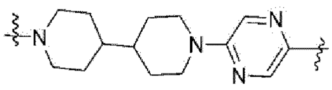, 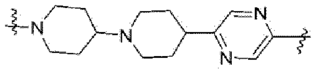, 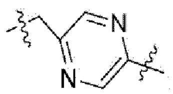, 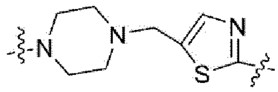,

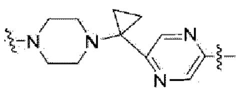, 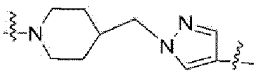, 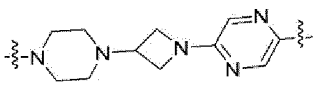, 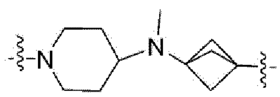,

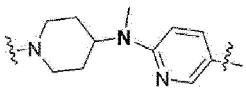, 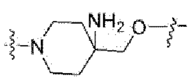, 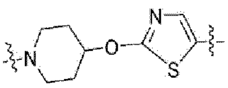, 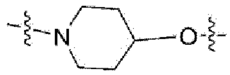,

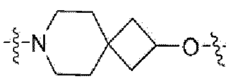, 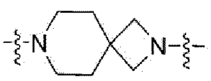, 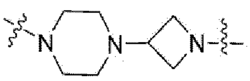, 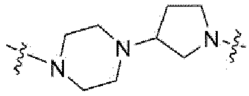,

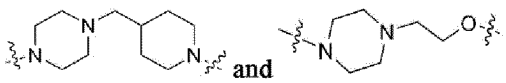 and .

**29.** The compound, or the tautomer, racemate, enantiomer, diastereomer thereof, or the mixture thereof, or the pharmaceutically acceptable salt thereof according to any one of claims 1 to 28, wherein M is selected from the group consisting of

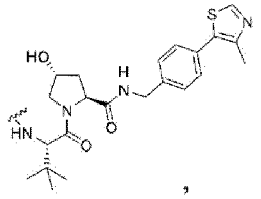, 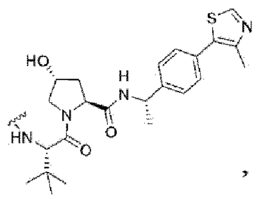, 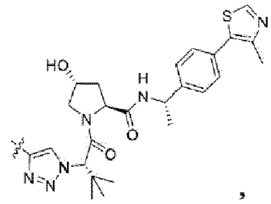, 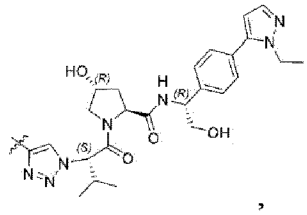,

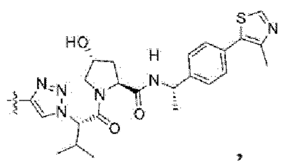, 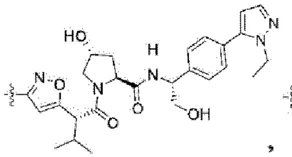, 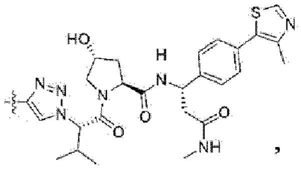, 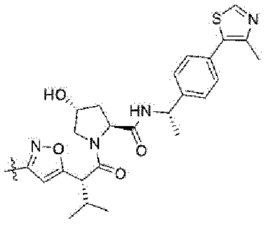

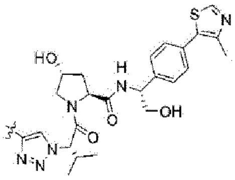, 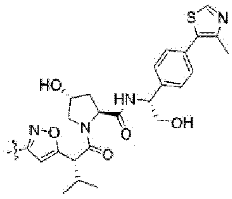, 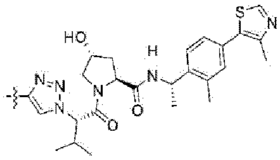 and 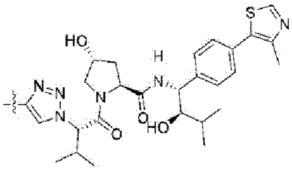.

**30.** The compound, or the tautomer, racemate, enantiomer, diastereomer thereof, or the mixture thereof, or the pharmaceutically acceptable salt thereof according to any one of claims 1 to 29, wherein L is selected from the group consisting of

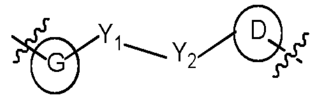 and 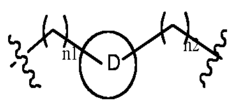;

wherein ring G is selected from the group consisting of $C_{6-14}$ aryl, 5- to 14-membered heteroaryl, $C_{3-14}$ cycloalkyl and 3- to 14-membered heterocyclyl, wherein $C_{6-14}$ aryl, 5- to 14-membered heteroaryl or 3- to 14-membered heterocyclyl is optionally further substituted by one or more $R_a$, wherein $R_a$ is each independently selected from the group consisting of H, hydroxyl, amino, oxo, cyano, halogen, $C_{1-6}$ alkyl, $C_{1-6}$ haloalkyl and $C_{1-6}$ alkoxy;
ring D is 5- to 6-membered N-containing heteroaryl, and is preferably

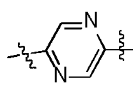

or

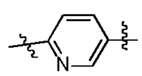;

$Y_1$ and $Y_2$ are each independently absent or selected from the group consisting of -O-, -NH-, 3- to 6-membered N-containing heterocyclyl, $C_{1-6}$ alkylene, carbonyl, acetylenyl and $C_{3-6}$ cycloalkyl; and
n1 and n2 are each independently 0, 1, 2, 3, or 4.

**31.** The compound, or the tautomer, racemate, enantiomer, diastereomer thereof, or the mixture thereof, or the pharmaceutically acceptable salt thereof according to claim 30, wherein L is selected from the group consisting of

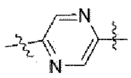, 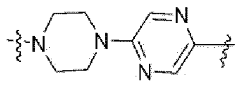,

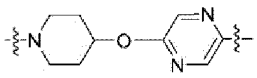, 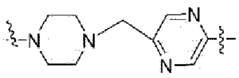, 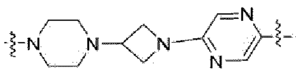, 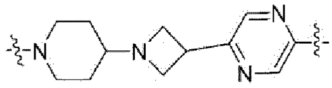,

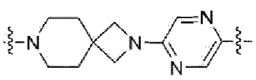, 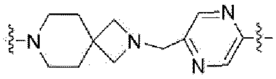, 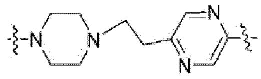, 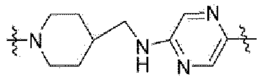,

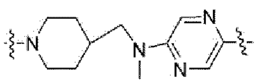 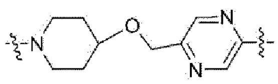, 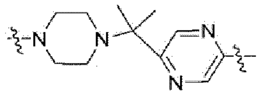, 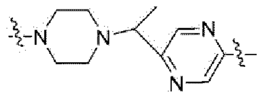,

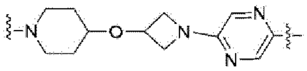, 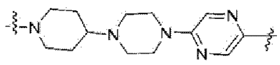, 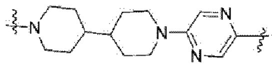, 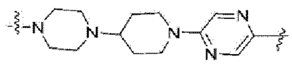,

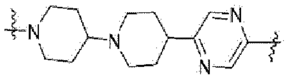, 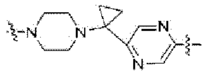, 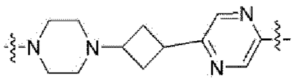, 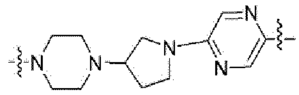,

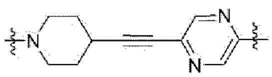, 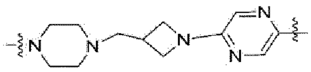, 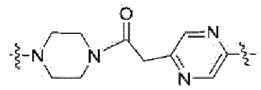, 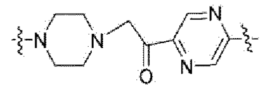,

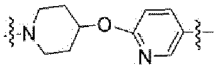, 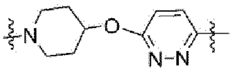, 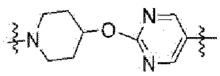, 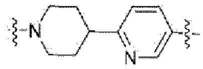 and 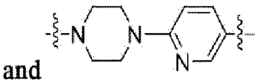.

**32.** The compound, or the tautomer, racemate, enantiomer, diastereomer thereof, or the mixture thereof, or the pharmaceutically acceptable salt thereof according to any one of claims 1 to 31, wherein:

F is

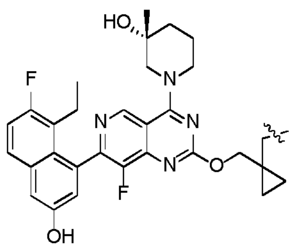 or 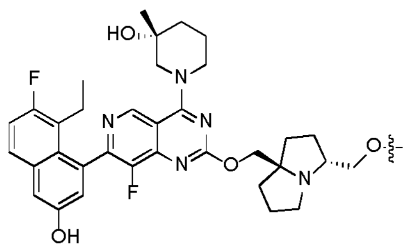,

L is selected from the group consisting of the following structures:

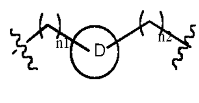,

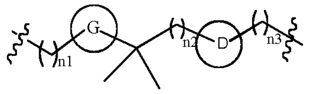,
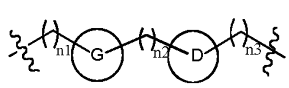,
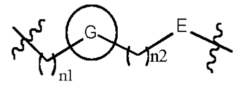,

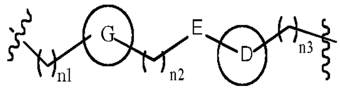,
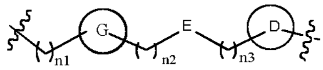,
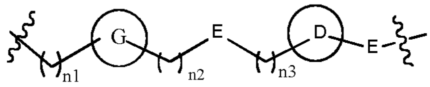

and

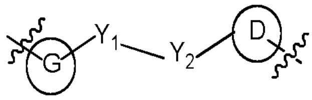;

wherein,
ring G or ring D is each independently selected from the group consisting of $C_{6-14}$ aryl, 5- to 14-membered heteroaryl, $C_{3-14}$ cycloalkyl and 3- to 14-membered heterocyclyl, wherein $C_{6-14}$ aryl, 5- to 14-membered heteroaryl, $C_{3-14}$ cycloalkyl or 3- to 14-membered heterocyclyl is optionally further substituted by one or more $R_a$;
$Y_1$ and $Y_2$ are each independently absent or selected from the group consisting of -O-, -NH-, 3- to 6-membered N-containing heterocyclyl, $C_{1-6}$ alkylene, carbonyl, acetylenyl and $C_{3-6}$ cycloalkyl;
E is each independently selected from the group consisting of -O-, -NH- and $-NCH_3-$;
n1, n2 and n3 are each independently selected from integers ranging from 0 to 10, and preferably n1, n2 and n3 are each independently 0, 1, 2, 3 or 4;
M is a VHL binding fragment represented by general formula (III);

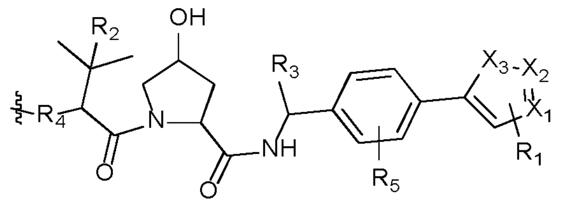

(III)

wherein $X_1$ is selected from the group consisting of C, CH and N;
$X_2$ is selected from the group consisting of C, CH and N;
$X_3$ is selected from the group consisting of N, NH, O and S;
$R_1$ is selected from the group consisting of H, $C_{1-6}$ alkyl, halogen and cyano, and is preferably methyl, ethyl, F or cyano;
$R_2$ is selected from the group consisting of H and $C_{1-6}$ alkyl, and is preferably H or methyl;
$R_3$ is selected from the group consisting of H, $C_{1-6}$ alkyl, $C_{1-6}$ hydroxylalkyl and $-(CH_2)_n-CO-NH-(CH_2)_n-CH_3$, wherein n is selected from the group consisting of 0, 1, 2 and 3, and $R_3$ is preferably H, methyl, hydroxylmethyl,

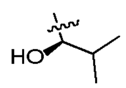

or $-CH_2-CO-NH-CH_3$;
$R_4$ is selected from the group consisting of

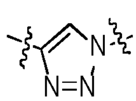, 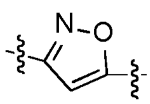

and -NH-;
$R_5$ is selected from the group consisting of H, $C_{1-6}$ alkyl and $C_{1-6}$ haloalkyl; and

$R_a$ is each independently selected from the group consisting of H, hydroxyl, amino, oxo, cyano, halogen, $C_{1-6}$ alkyl, $C_{1-6}$ haloalkyl and $C_{1-6}$ alkoxy.

**33.** The compound, or the tautomer, racemate, enantiomer, diastereomer thereof, or the mixture thereof, or the pharmaceutically acceptable salt thereof according to any one of claims 1 to 32, wherein the compound having an F-L-M structure is selected from the group consisting of the following compounds:

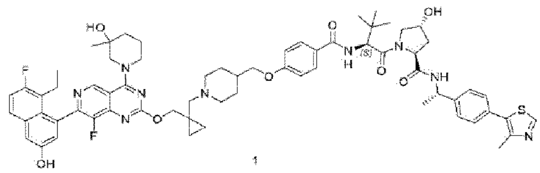

1

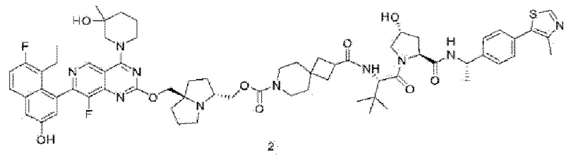

2

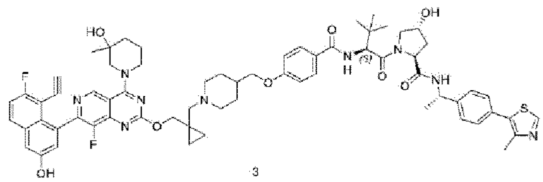

3

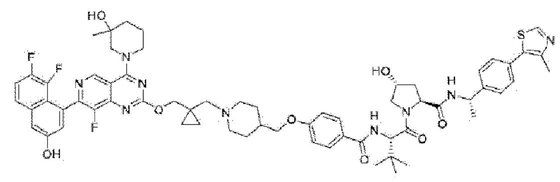

4

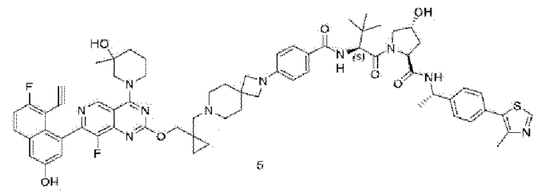

5

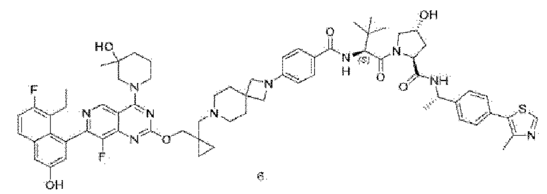

6

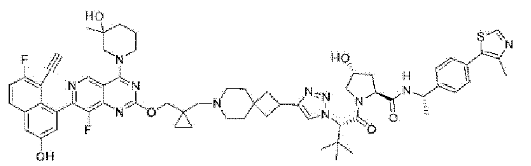

7

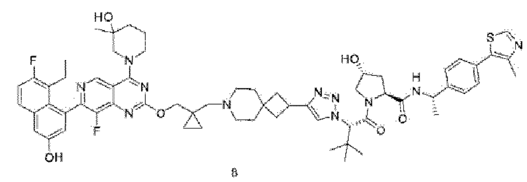

8

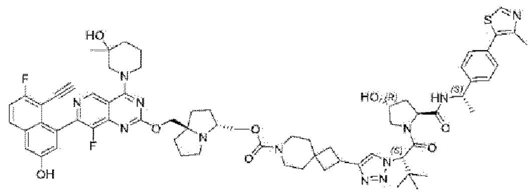

9

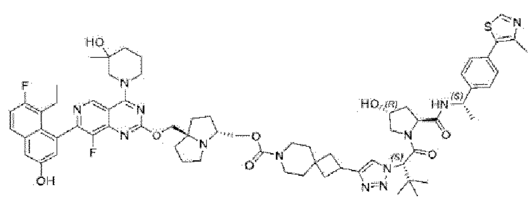

10

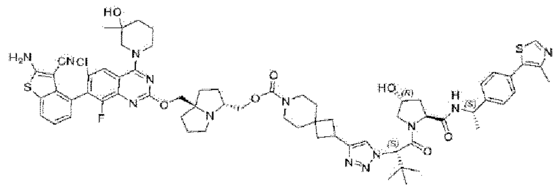

11

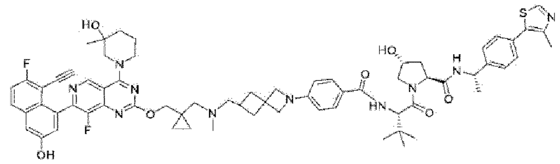

12

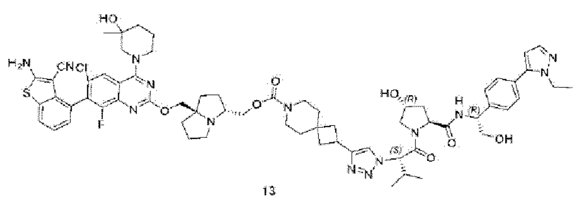

13

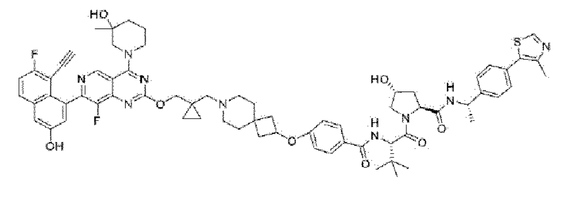

14

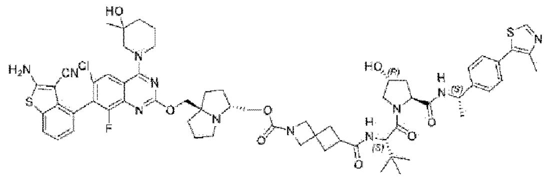

15

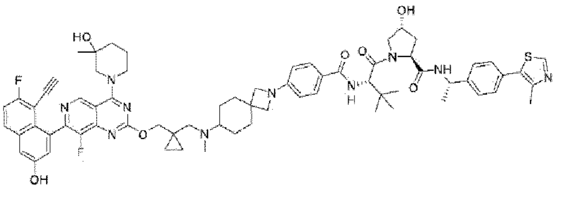

16

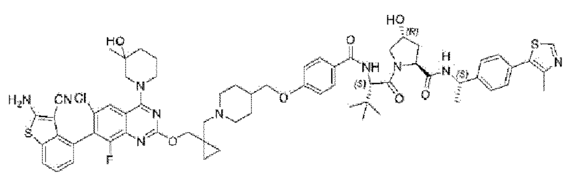

17

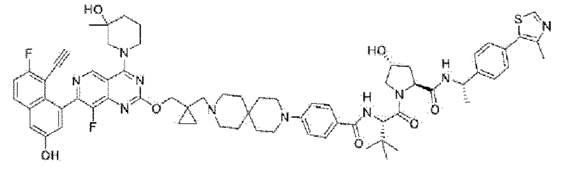

18

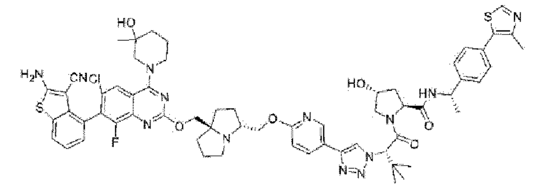

19

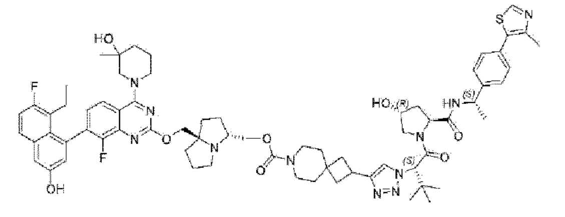

20

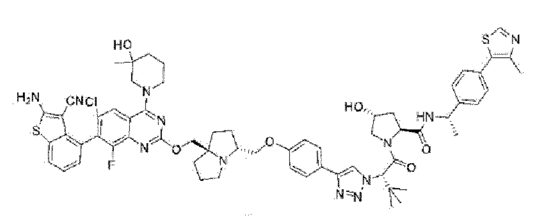

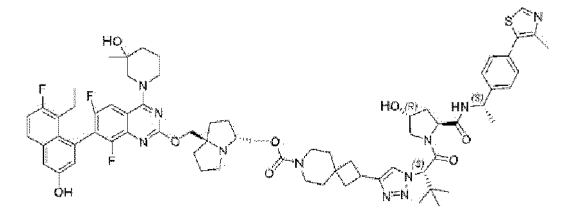

22

21

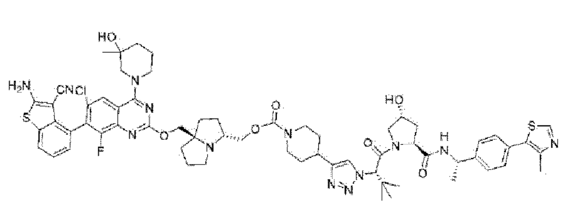

23

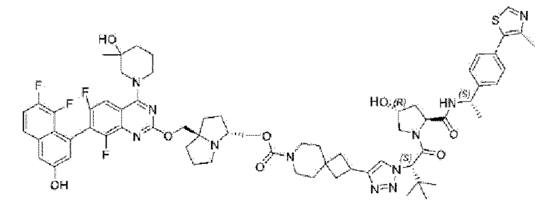

24

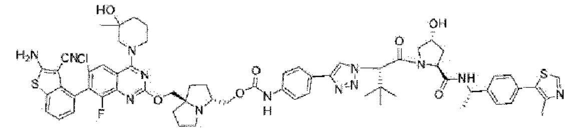

25

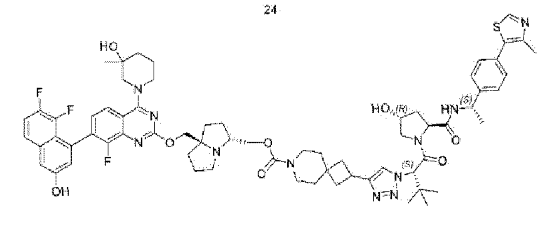

26

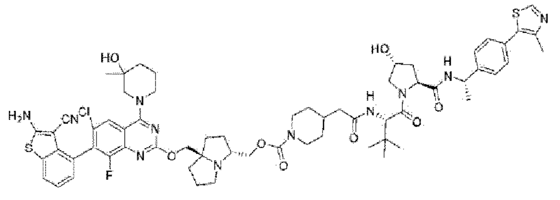

27

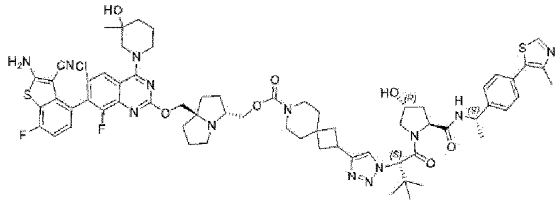

28

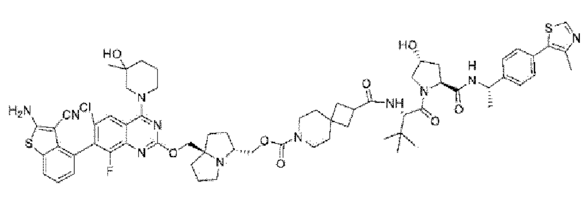

29

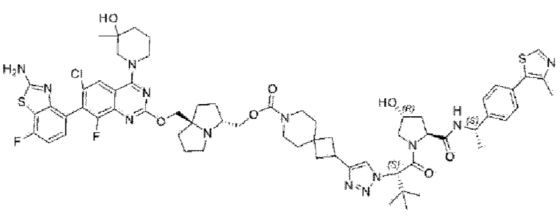

30

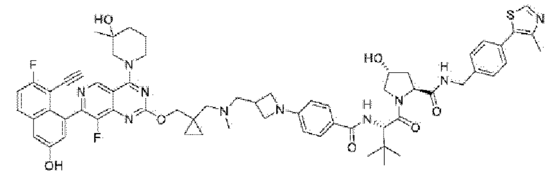

31

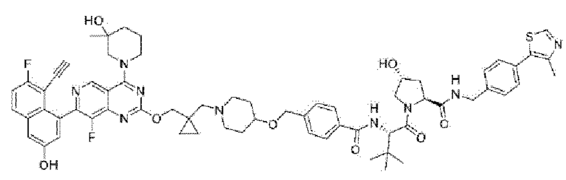

32

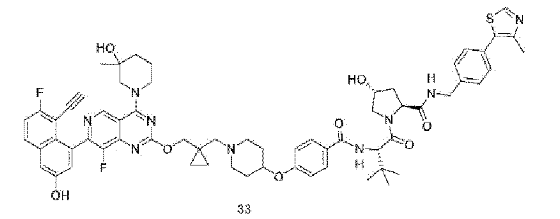

33

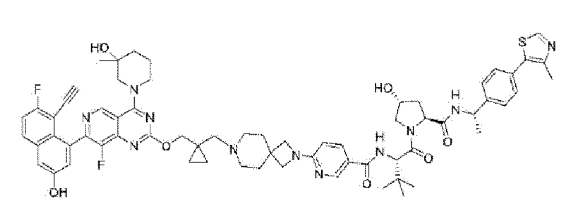

34

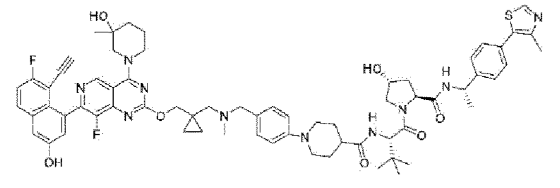

35

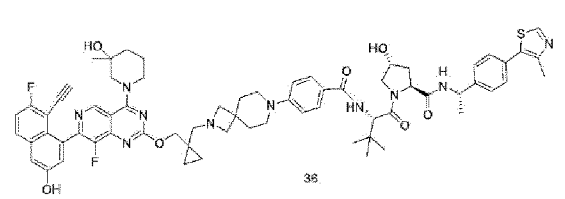

36

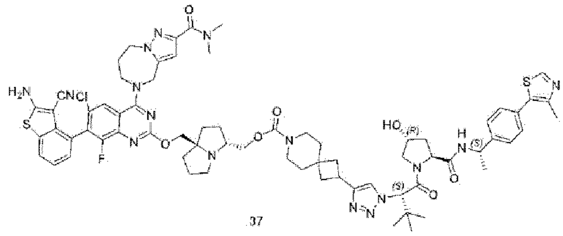

37

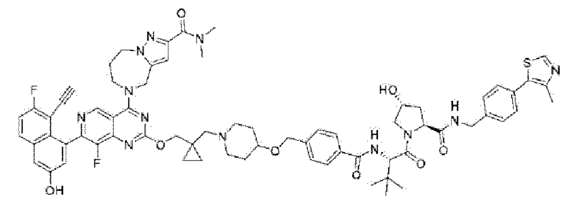

38

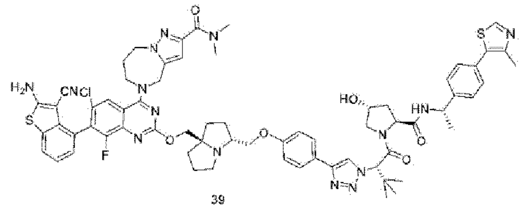

39

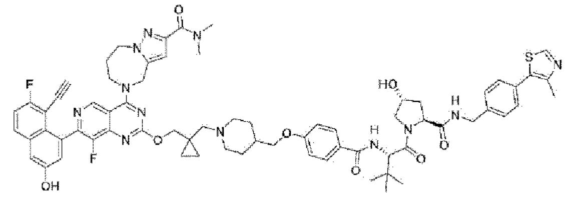

40

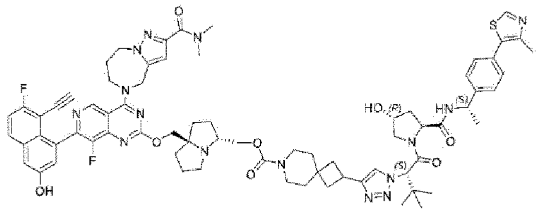

41

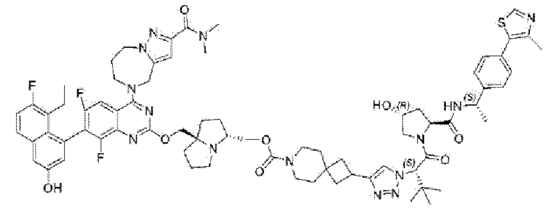

42

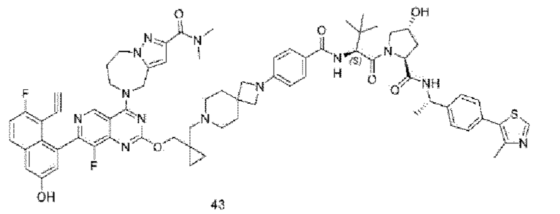

43

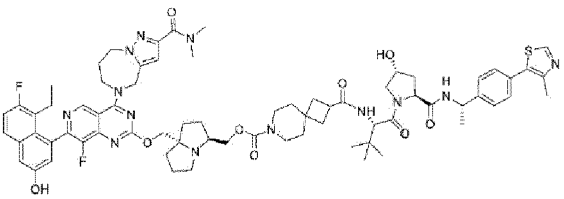

44

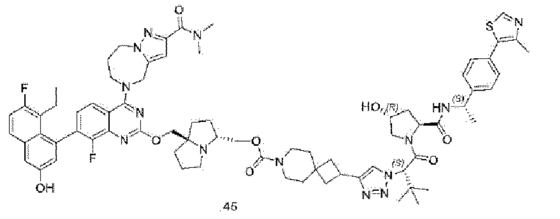

45

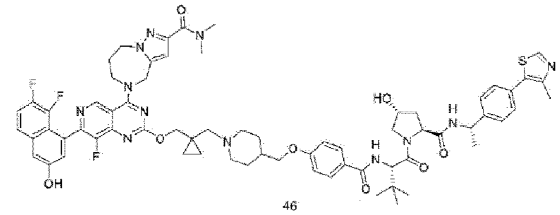

46

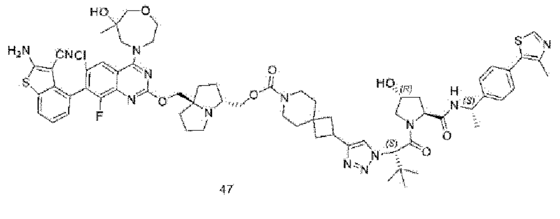

47

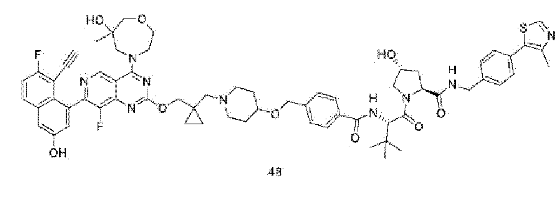

48

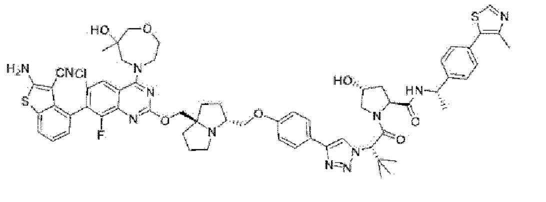

49

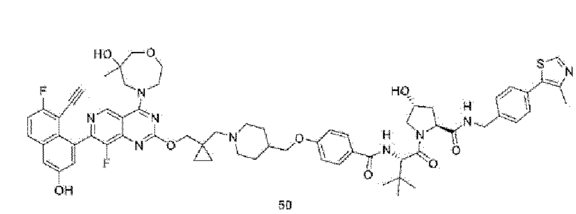

50

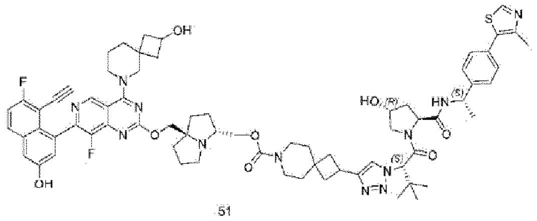

51

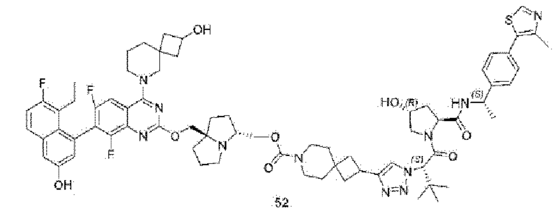

52

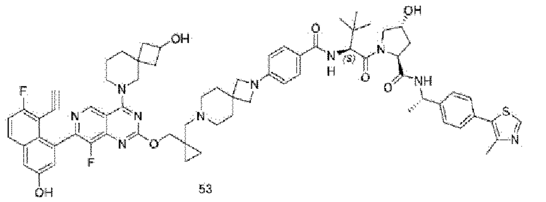

53

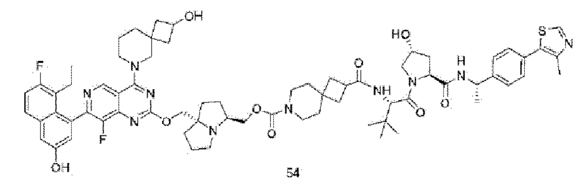

54

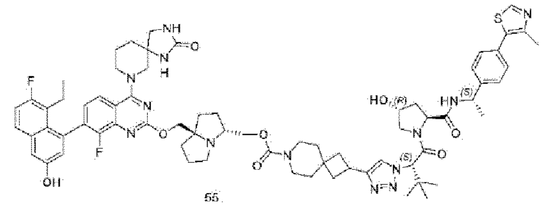

55

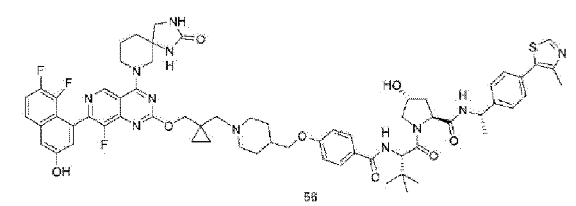

56

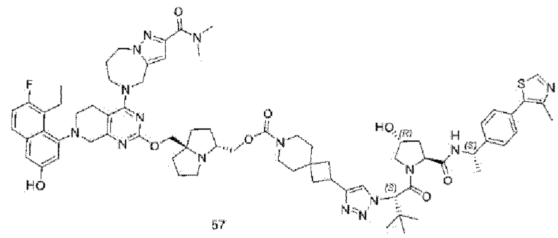

57

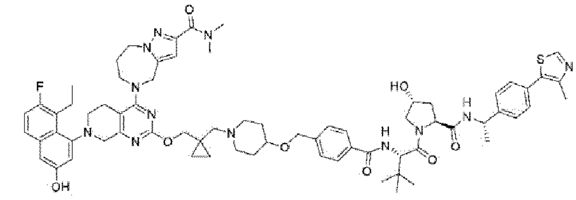

58

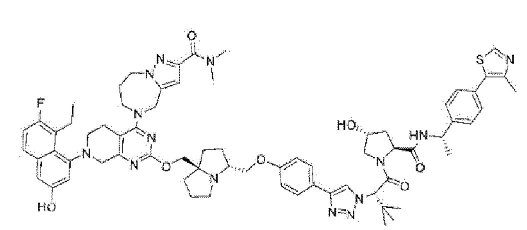

59

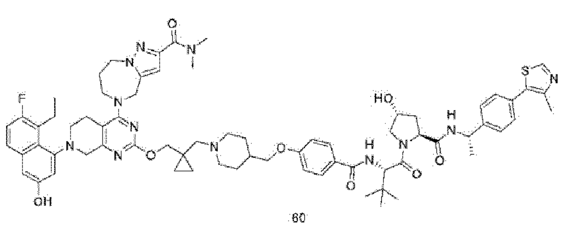

60

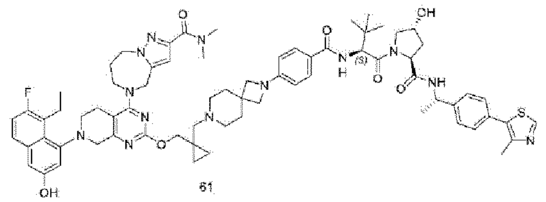
61

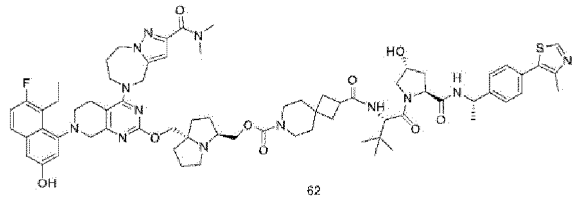
62

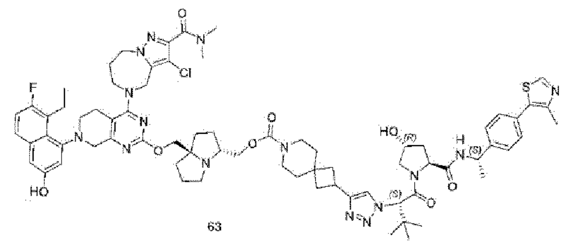
63

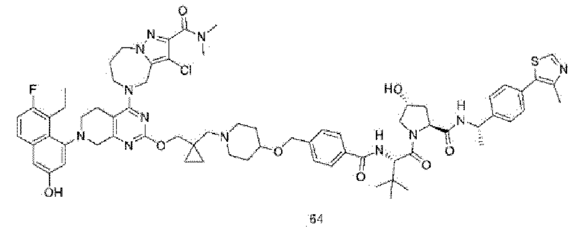
64

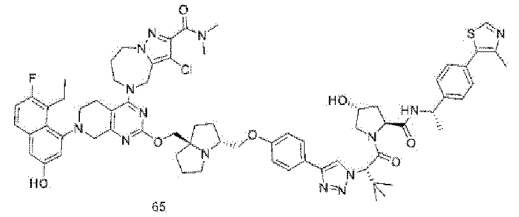
65

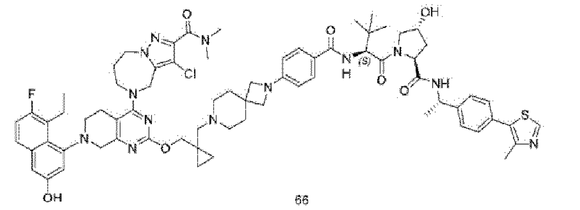
66

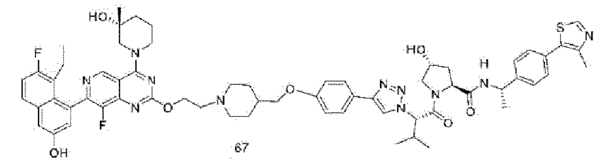
67

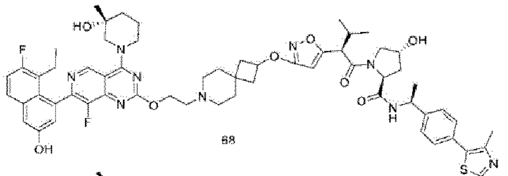
68

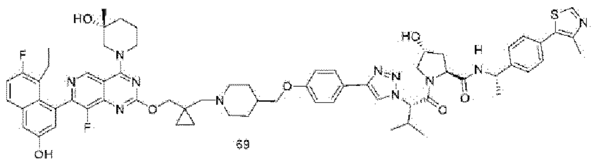
69

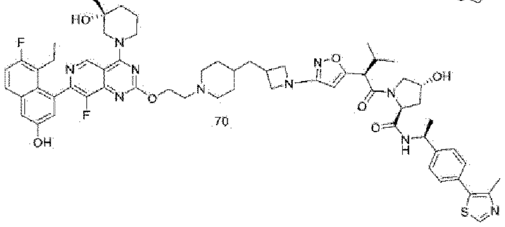
70

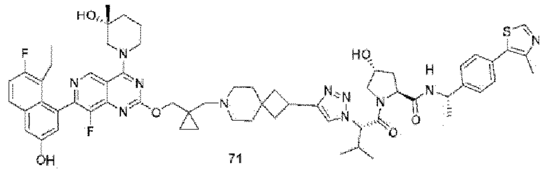
71

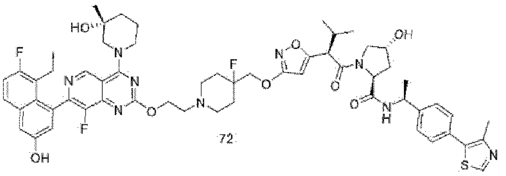
72

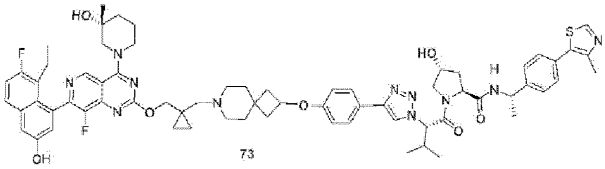
73

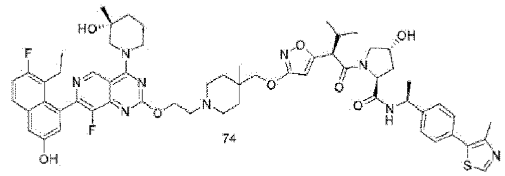
74

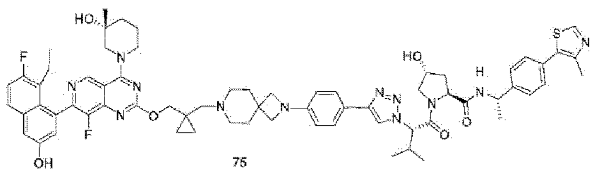
75

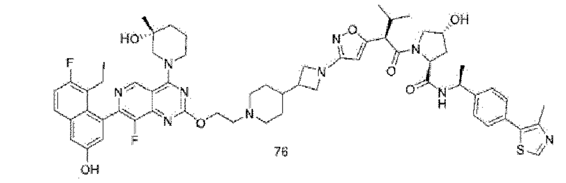
76

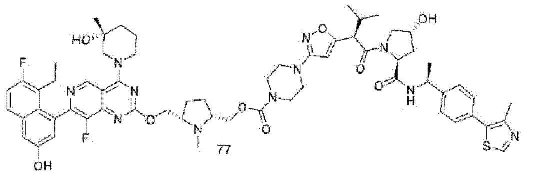
77

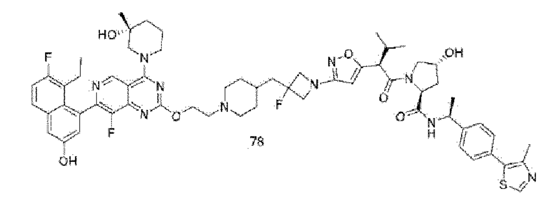
78

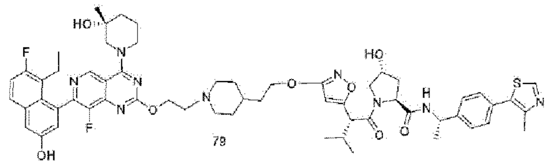
79

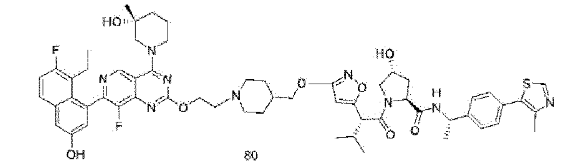
80

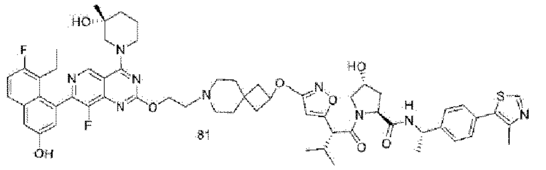
81

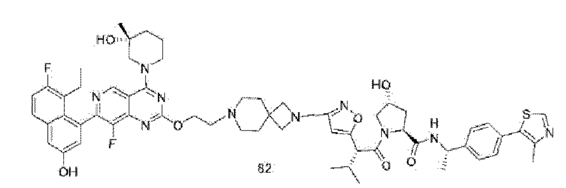
82

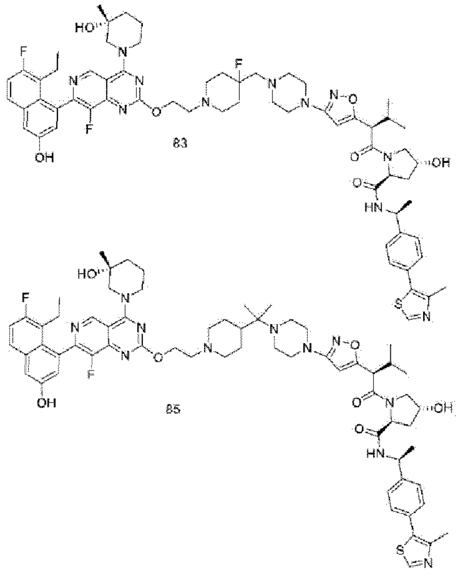
83

85

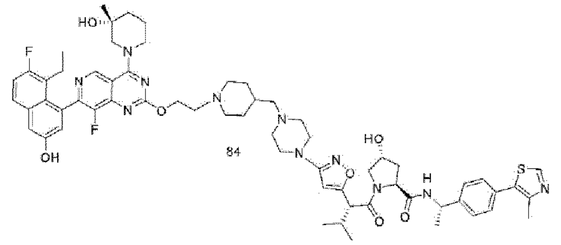
84

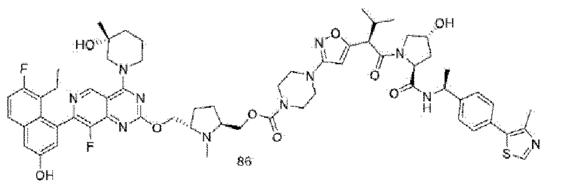
86

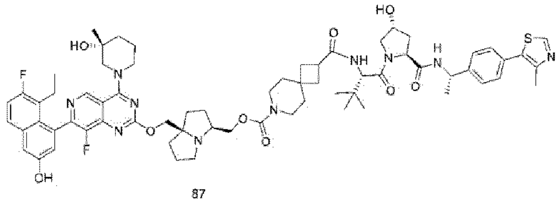
87

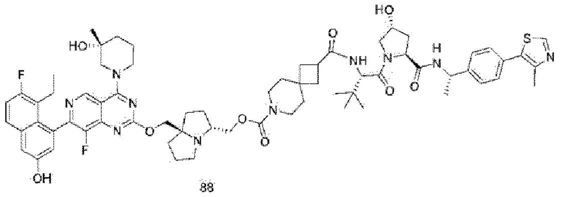
88

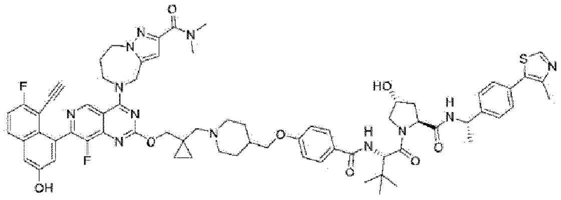

89

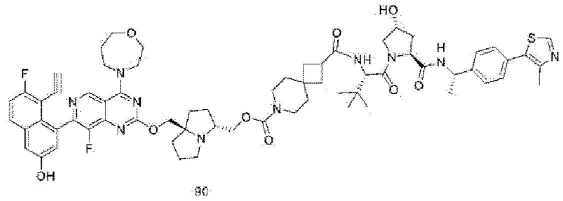

90

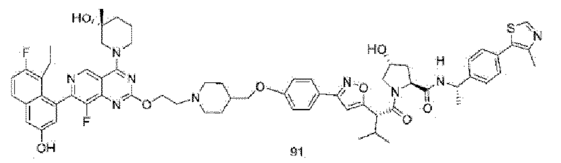

91

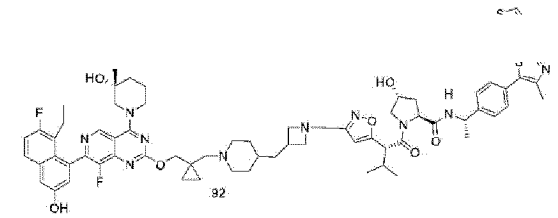

92

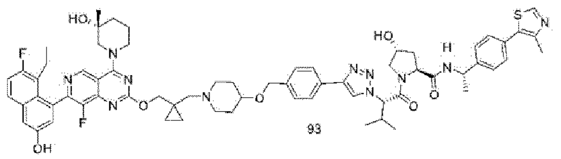

93

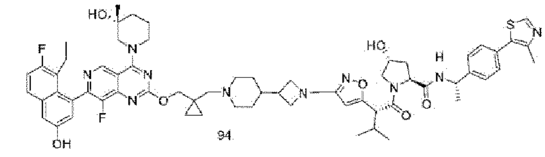

94

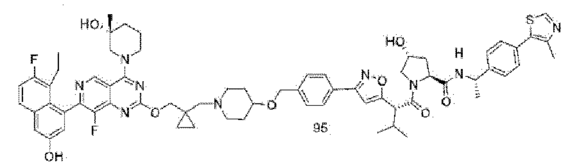

95

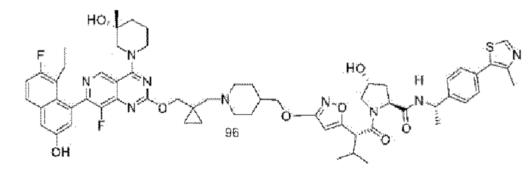

96

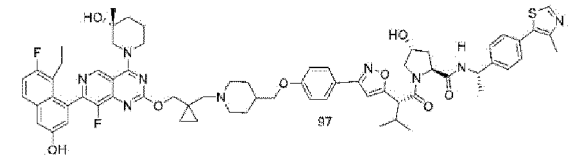

97

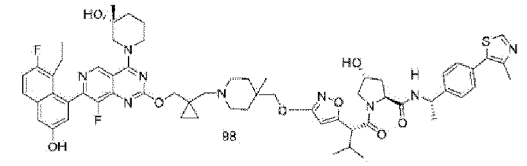

98

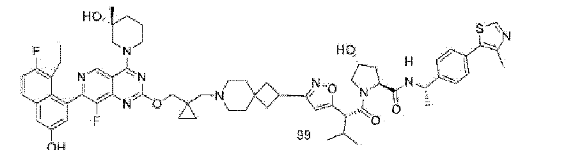

99

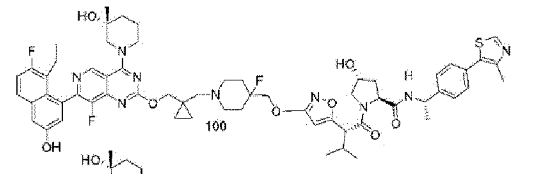

100

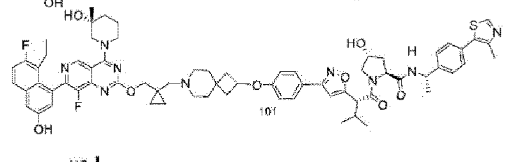

101

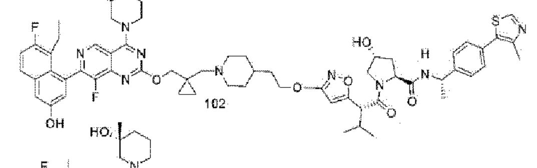

102

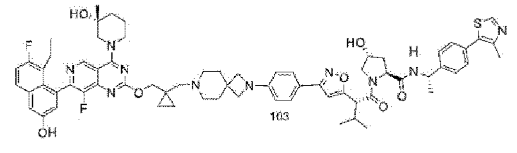

103

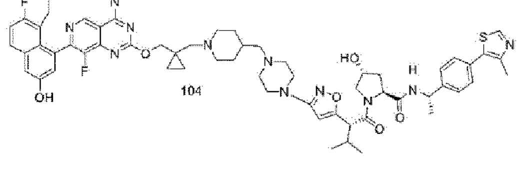

104

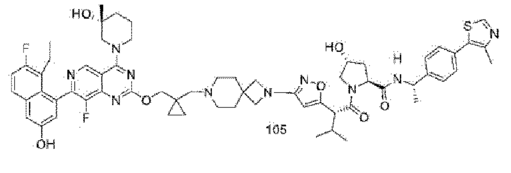

105

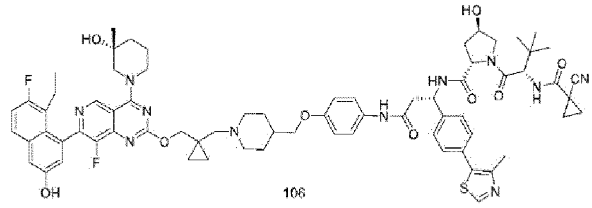

106

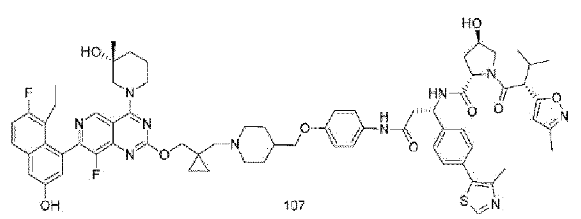

107

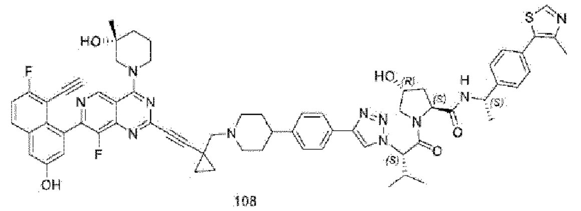

108

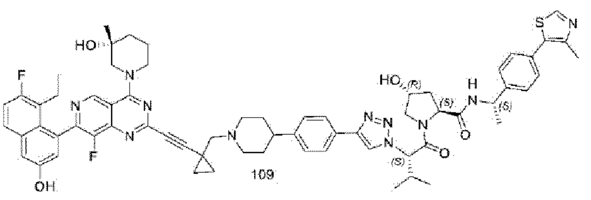

109

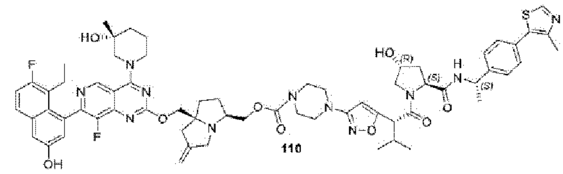

110

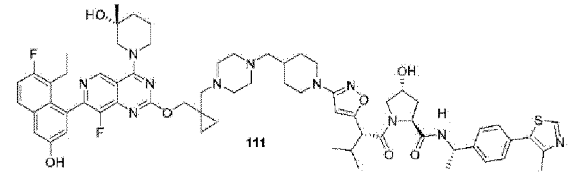

111

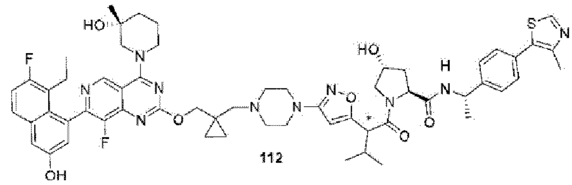

112

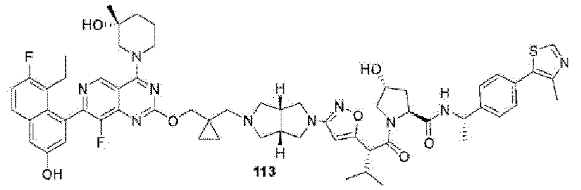

113

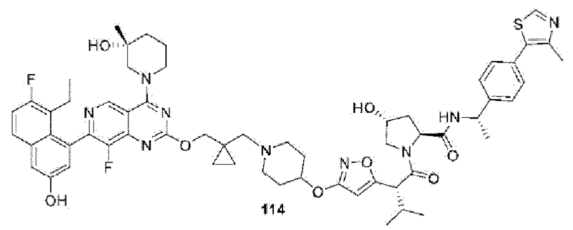

114

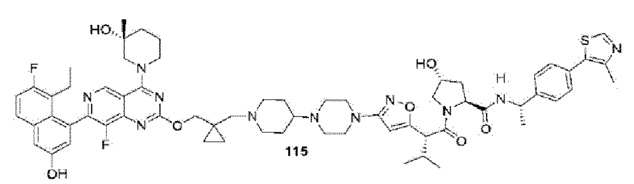

115

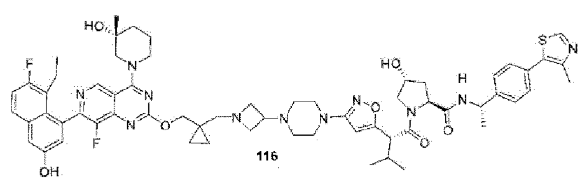

116

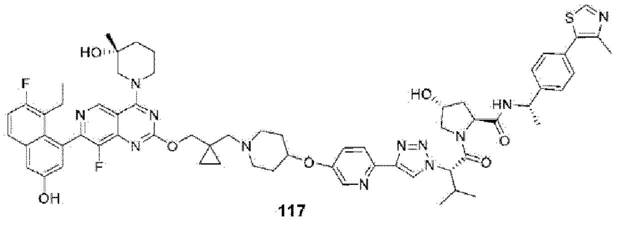

117

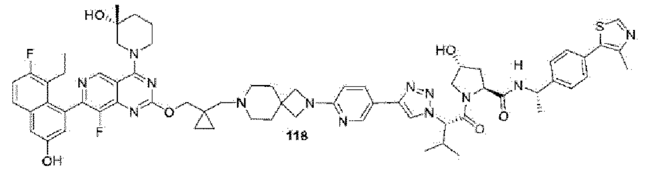

118

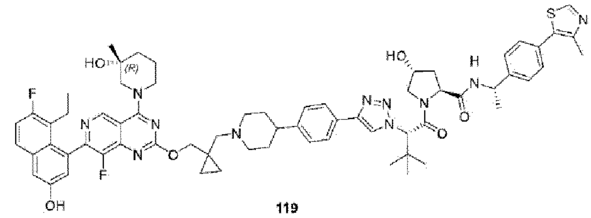

119

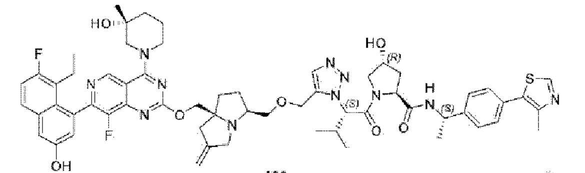

120

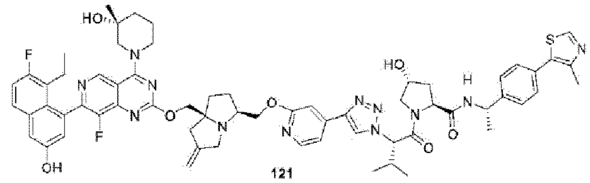

121

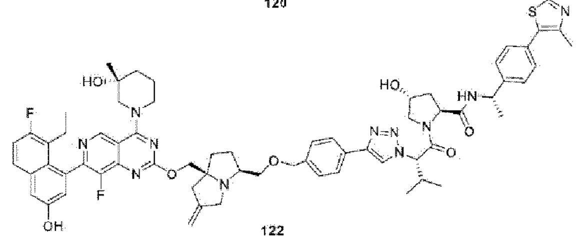

122

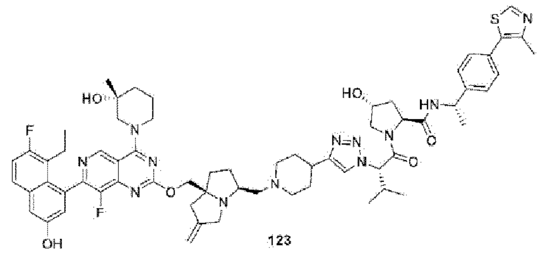

123

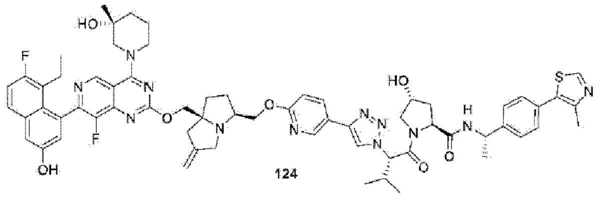

124

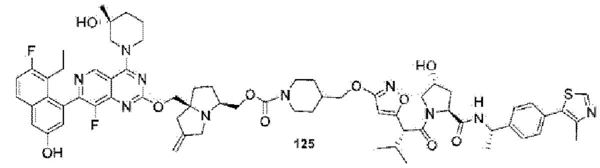

125

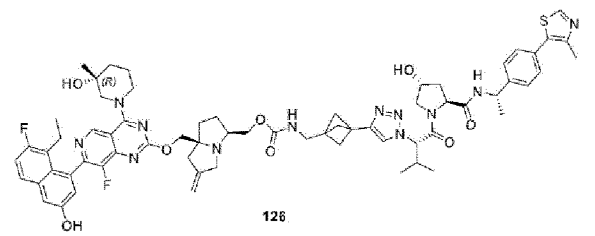

126

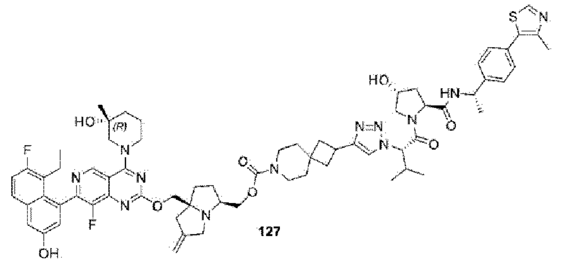

127

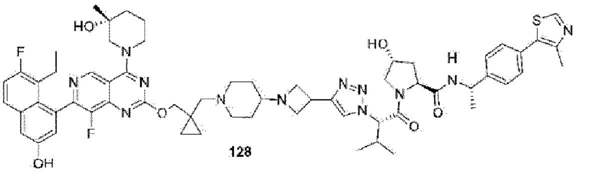

128

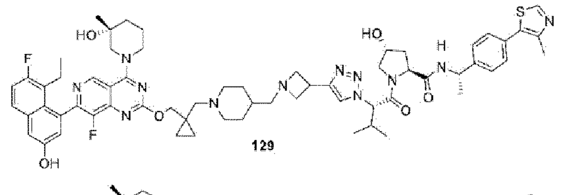

129

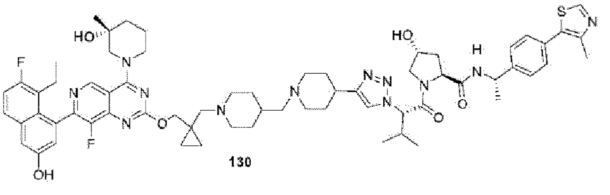

130

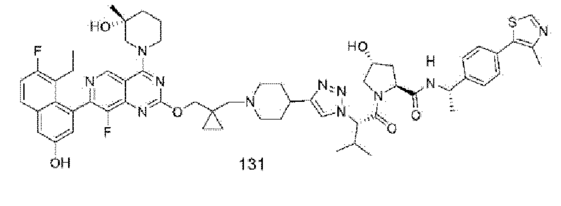

131

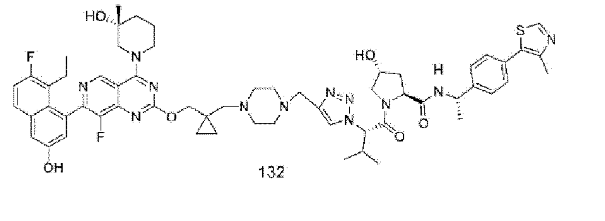

132

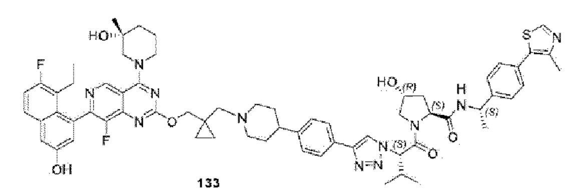

133

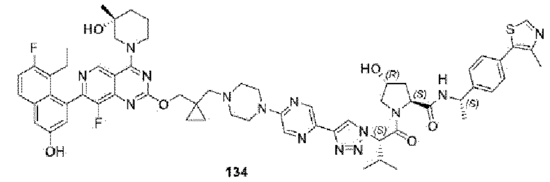

134

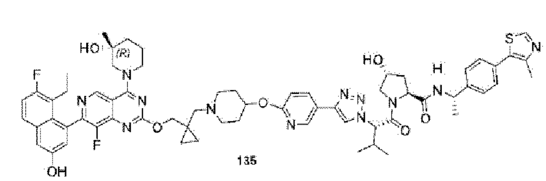

135

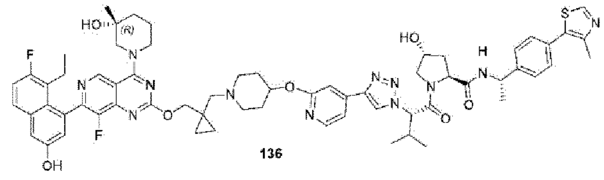

136

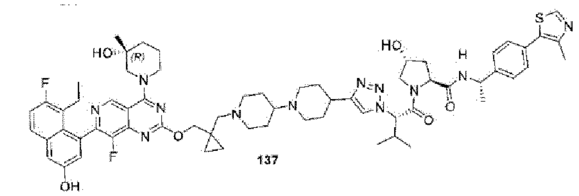

137

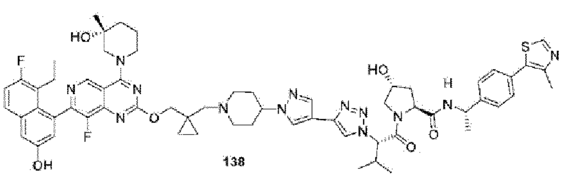

138

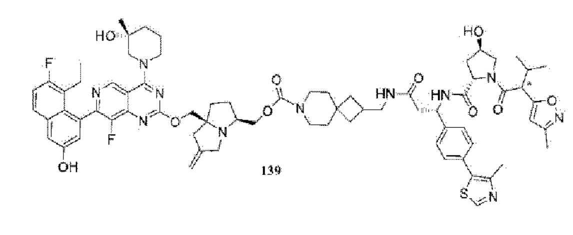

139

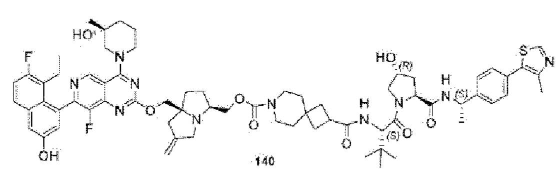

140

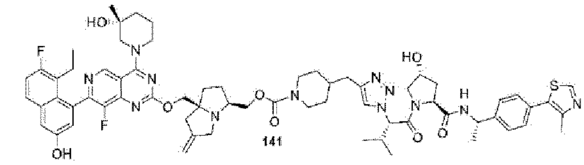

141

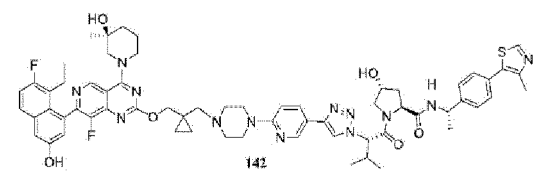

142

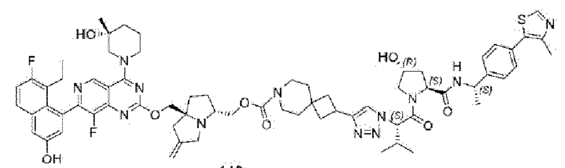

143

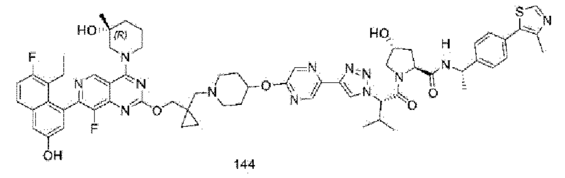

144

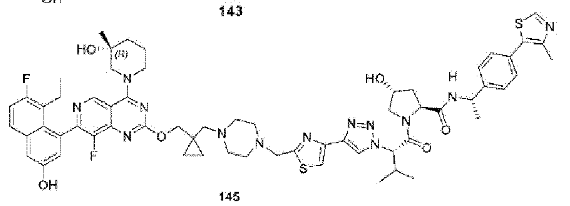

145

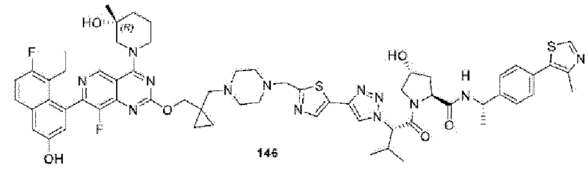

146

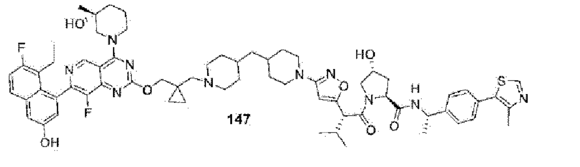

147

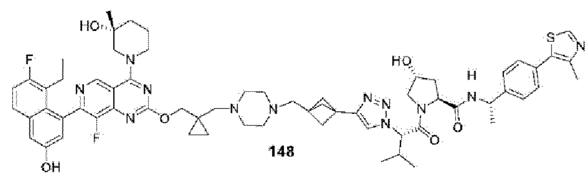

148

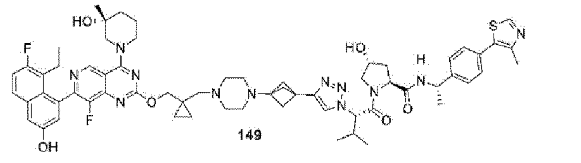

149

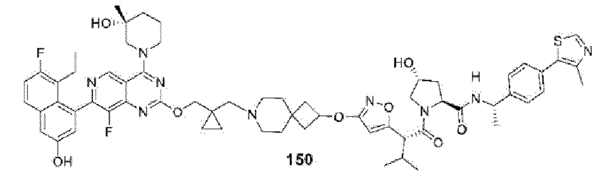

150

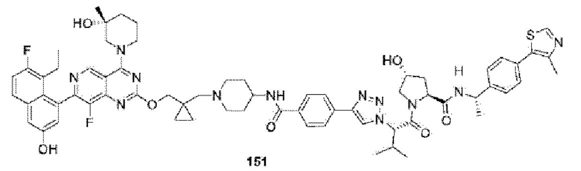

151

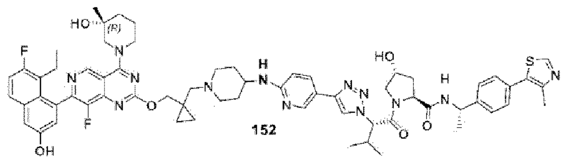

152

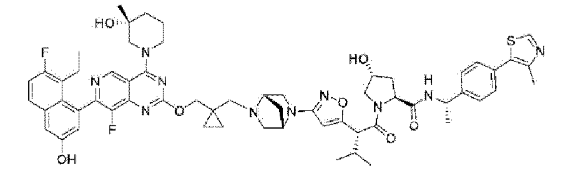

153

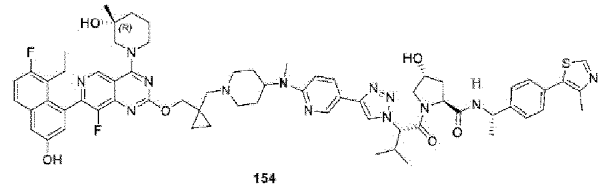

154

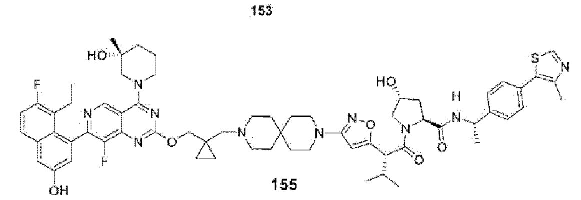

155

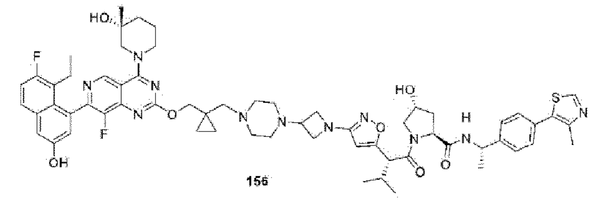

156

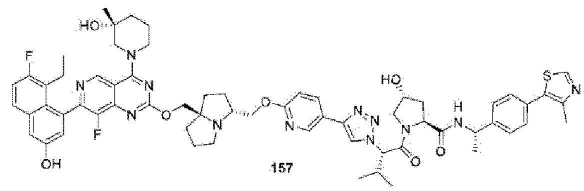

157

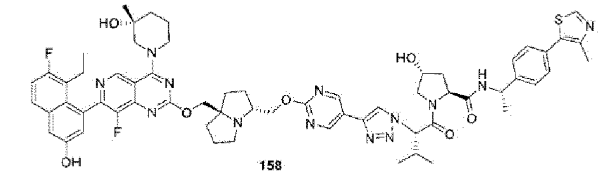

158

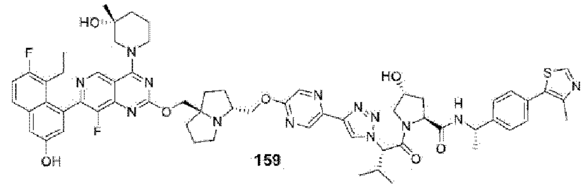

159

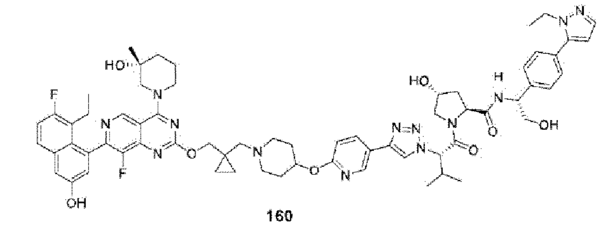

160

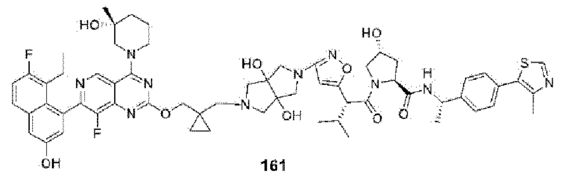

161

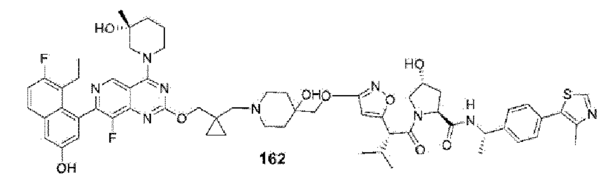

162

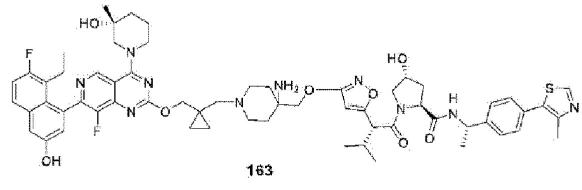

163

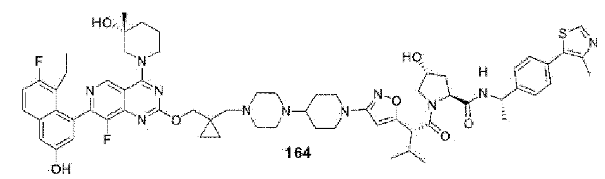

164

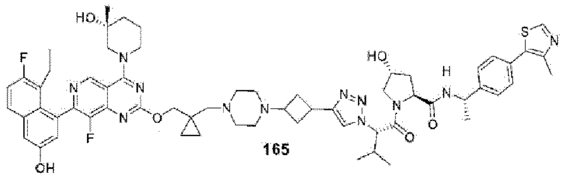

165

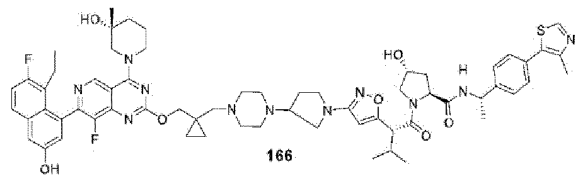

166

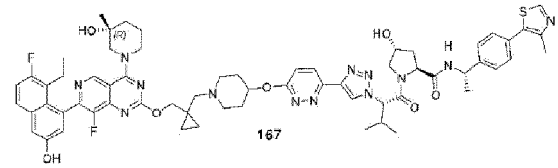

167

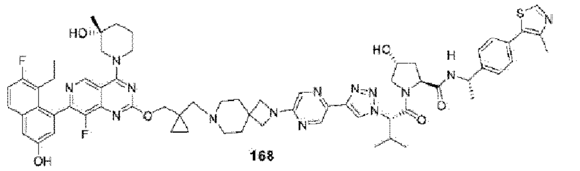

168

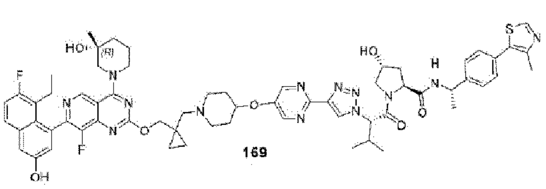

169

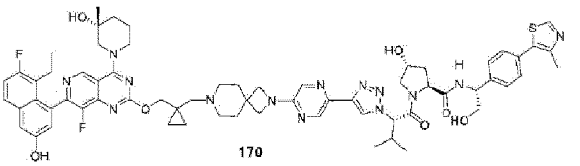

170

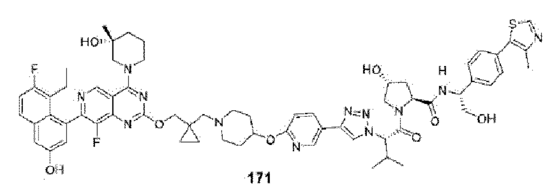

171

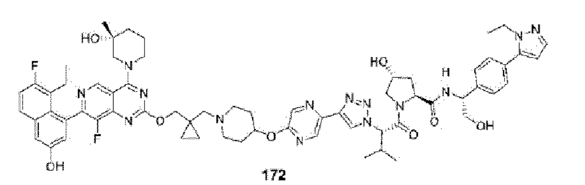

172

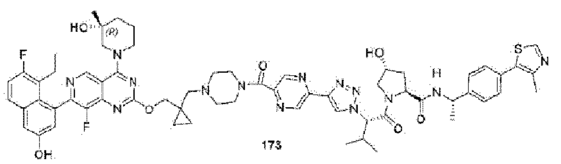

173

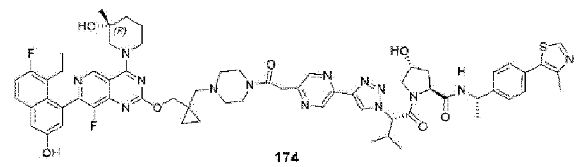

174

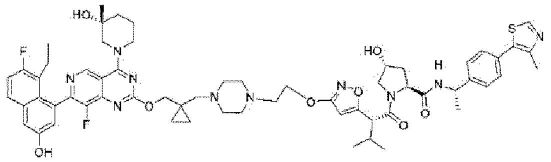

175

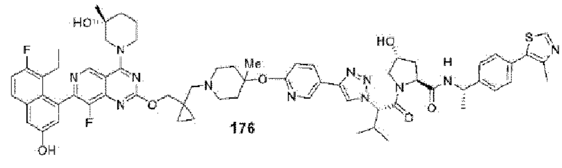

176

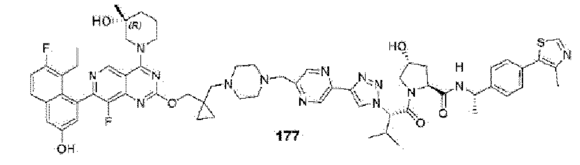

177

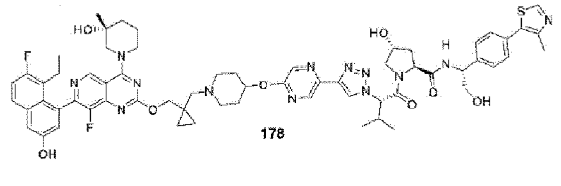

178

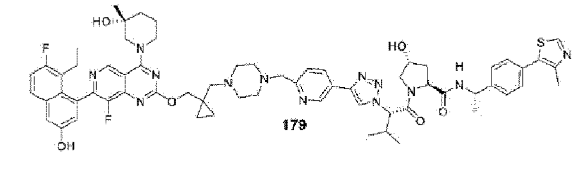

179

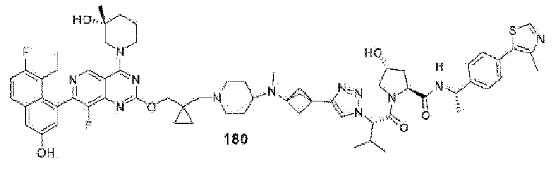

180

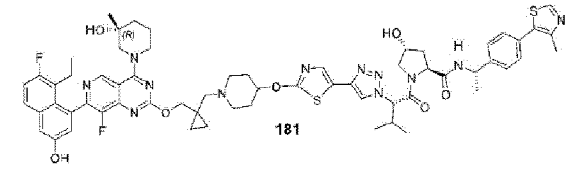

181

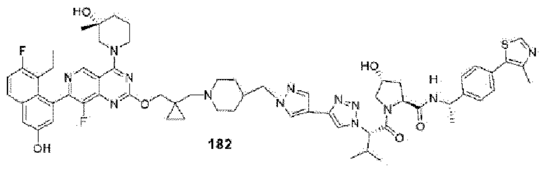

182

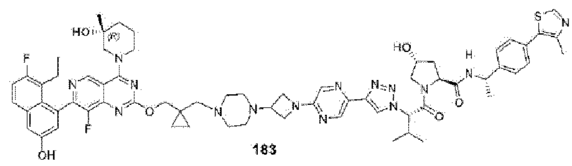

183

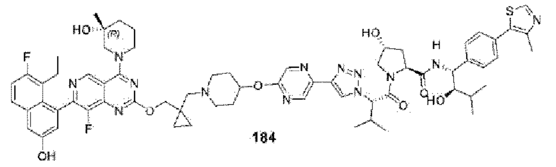

184

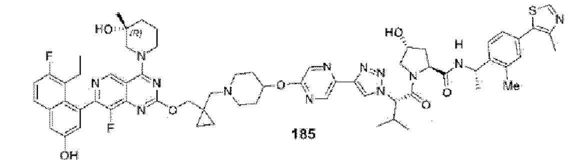

185

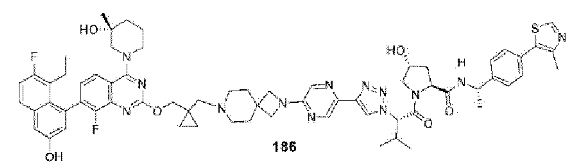

186

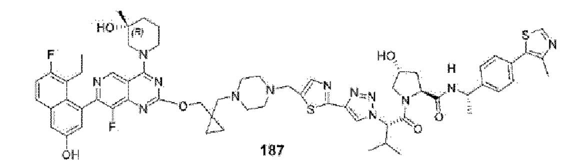

187

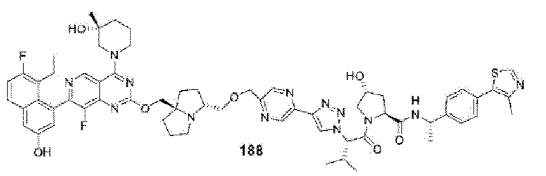

188

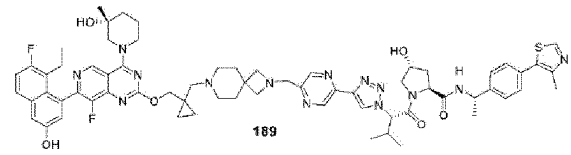

189

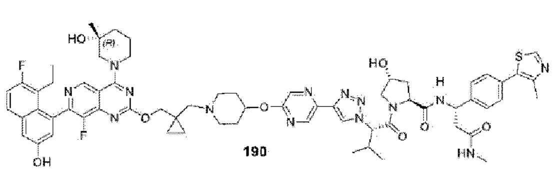

190

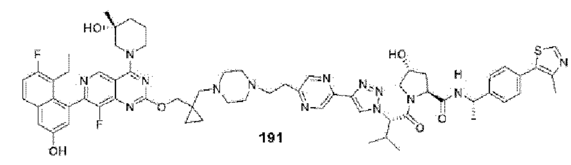

191

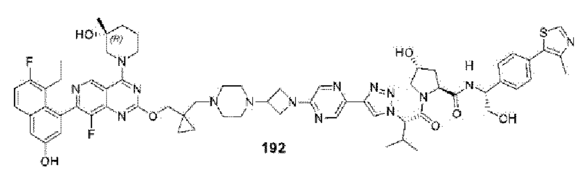

192

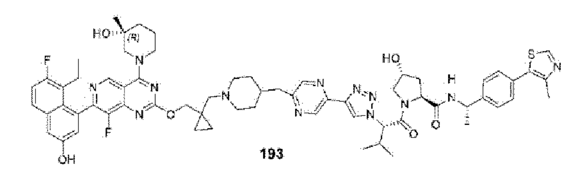

193

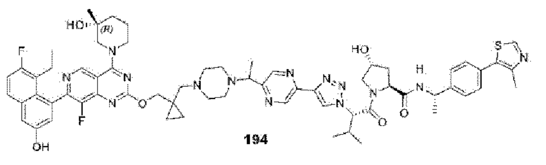

194

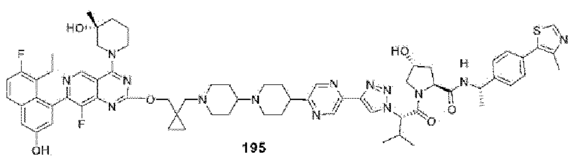

195

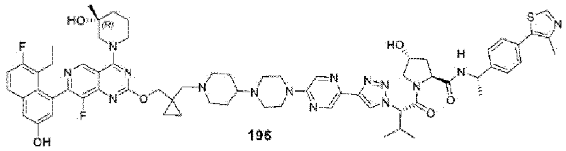

196

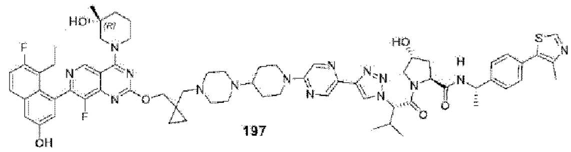

197

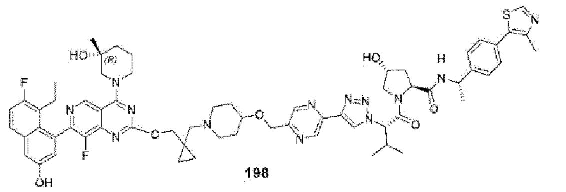

198

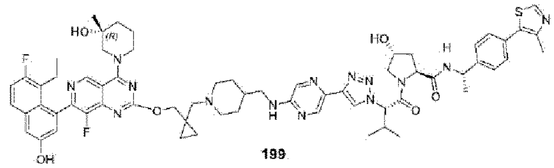

199

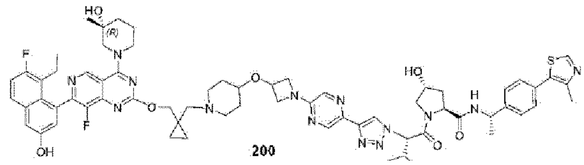

200

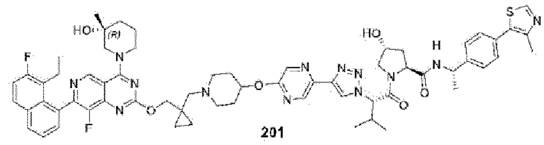

201

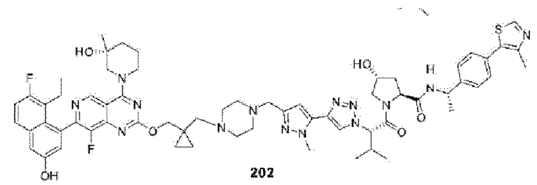

202

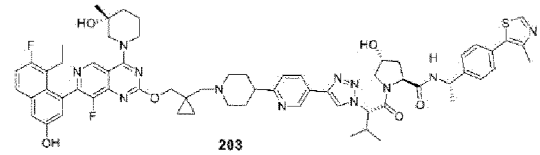

203

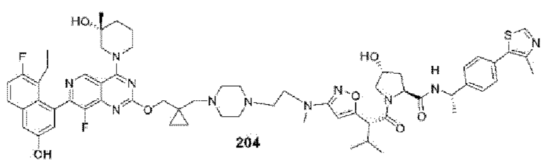

204

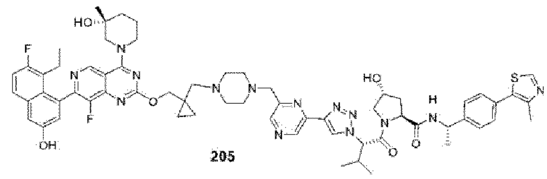

205

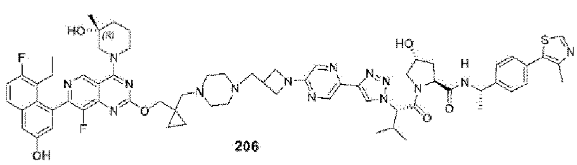

206

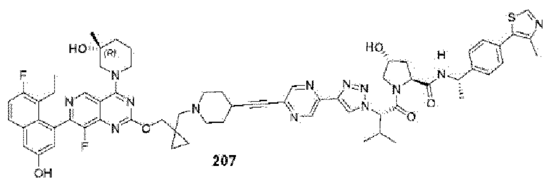

207

.

**34.** A pharmaceutical composition, wherein the pharmaceutical composition comprises the compound, or the tautomer, racemate, enantiomer, diastereomer thereof, or the mixture thereof, or the pharmaceutically acceptable salt thereof according to any one of claims 1 to 33.

**35.** Use of the compound, or the tautomer, racemate, enantiomer, diastereomer thereof, or the mixture thereof, or the pharmaceutically acceptable salt thereof according to any one of claims 1 to 33, or the pharmaceutical composition according to claim 33 in the manufacture of a medicament for regulating ubiquitination and degradation of KRAS protein in a subject.

**36.** Use of the compound, or the tautomer, racemate, enantiomer, diastereomer thereof, or the mixture thereof, or the pharmaceutically acceptable salt thereof according to any one of claims 1 to 33, or the pharmaceutical composition according to claim 33 in the manufacture of a medicament for treating and/or preventing a KRAS protein-mediated or dependent disease, wherein the KRAS protein-mediated disease is preferably a tumor.

**37.** The use according to claim 36, wherein the disease is selected from the group consisting of breast cancer, multiple myeloma, bladder cancer, endometrial cancer, gastric cancer, cervical cancer, rhabdomyosarcoma, non-small cell

lung cancer, small cell lung cancer, pleomorphic lung cancer, ovarian cancer, esophageal cancer, melanoma, colorectal cancer, hepatocellular carcinoma, head and neck tumor, intrahepatic cholangiocarcinoma, myelodysplastic syndrome, malignant glioma, prostate cancer, thyroid cancer, Schwann cell tumor, pulmonary squamous cell carcinoma, lichenoid keratosis, synovial sarcoma, skin cancer, pancreatic cancer, testicular cancer and liposarcoma.

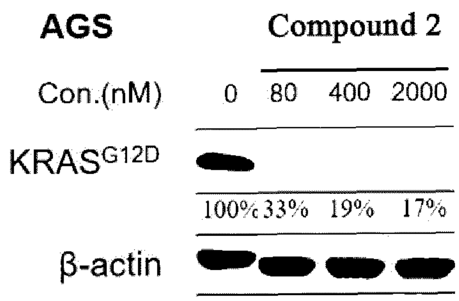


FIG. 1

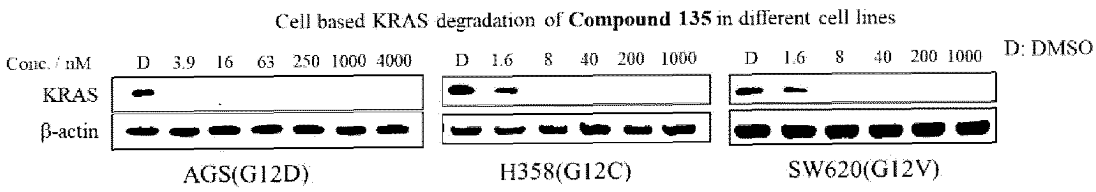


FIG. 2

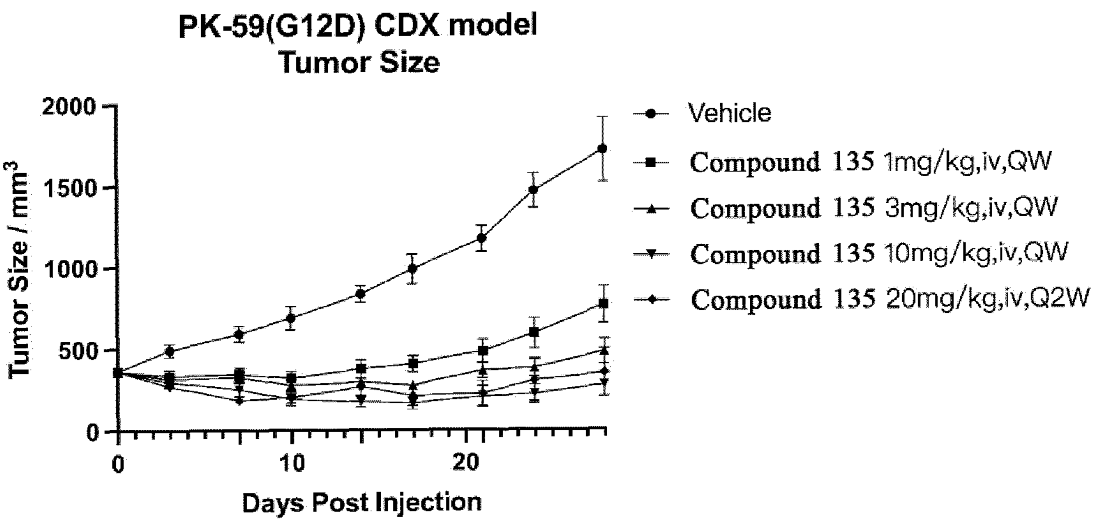


FIG. 3

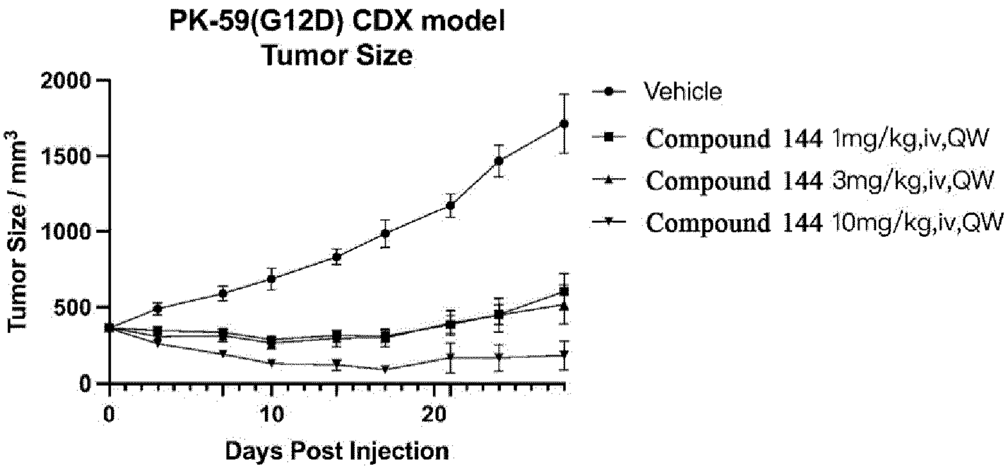
PK-59(G12D) CDX model
Tumor Size
Tumor Size / mm3
2000
1500
1000
500
0
0
10
20
Days Post Injection
Vehicle
Compound 144 1mg/kg,iv,QW
Compound 144 3mg/kg,iv,QW
Compound 144 10mg/kg,iv,QW

FIG. 4

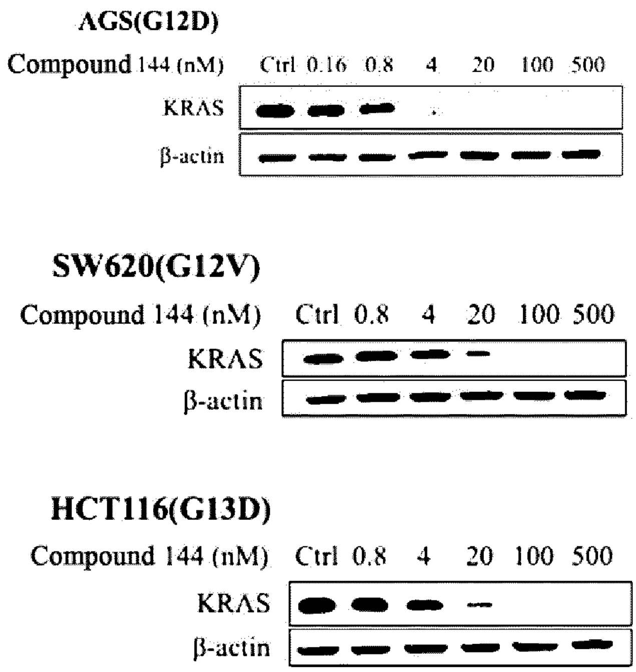
AGS(G12D)
Compound 144 (nM) Ctrl 0.16 0.8 4 20 100 500
KRAS
β-actin
SW620(G12V)
Compound 144 (nM) Ctrl 0.8 4 20 100 500
KRAS
β-actin
HCT116(G13D)
Compound 144 (nM) Ctrl 0.8 4 20 100 500
KRAS
β-actin

FIG. 5

**INTERNATIONAL SEARCH REPORT**

International application No.
**PCT/CN2023/136686**

**A. CLASSIFICATION OF SUBJECT MATTER**

C07D401/14(2006.01)i； C07D487/08(2006.01)i； C07D471/08(2006.01)i； C07D403/12(2006.01)i； A61K31/517(2006.01)i； A61P35/00(2006.01)i

According to International Patent Classification (IPC) or to both national classification and IPC

**B. FIELDS SEARCHED**

Minimum documentation searched (classification system followed by classification symbols)

IPC： C07D, A61K, A61P

Documentation searched other than minimum documentation to the extent that such documents are included in the fields searched

Electronic data base consulted during the international search (name of data base and, where practicable, search terms used)

CNABS, DWPI, CNTXT, EPTXT, USTXT, WOTXT, CNKI, ENTXT, BING, STNext: 蛋白降解, 结构检索, protein degradation, Proteolysis 1w targeting chimera?, PROTAC?, ubiquitin, e3 ligase, cereblon, CRBN, von Hippel Lindau, VHL, kras, g12d

**C. DOCUMENTS CONSIDERED TO BE RELEVANT**

| Category* | Citation of document, with indication, where appropriate, of the relevant passages | Relevant to claim No. |
|---|---|---|
| PY | WO 2022266206 A1 (ERASCA, INC.) 22 December 2022 (2022-12-22) claims 1-61 | 1-37 |
| PY | WO 2023081476 A1 (RANOK THERAPEUTICS (HANGZHOU) CO., LTD.) 11 May 2023 (2023-05-11) claims 1-20 | 1-37 |
| PY | WO 2023141570 A2 (ARVINAS OPERATIONS, INC.) 27 July 2023 (2023-07-27) claims 1-25 | 1-37 |
| Y | WO 2022228576 A1 (SHANGHAI PHARMACEUTICALS HOLDING CO., LTD.) 03 November 2022 (2022-11-03) claims 1-15, description, paragraph 0011, and abstract | 1-37 |
| Y | WO 2022132200 A1 (MIRATI THERAPEUTICS, INC.) 23 June 2022 (2022-06-23) claims 1-2, 10-12, 61, and 63 | 1-37 |
| Y | WO 2021207172 A1 (ARVINAS OPERATIONS, INC. et al.) 14 October 2021 (2021-10-14) claims 1-27 | 1-37 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **12 March 2024** | **22 March 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2023/136686**

**C. DOCUMENTS CONSIDERED TO BE RELEVANT**

| Category* | Citation of document, with indication, where appropriate, of the relevant passages | Relevant to claim No. |
|---|---|---|
| Y | CN 112218859 A (ARVINAS OPERATIONS INC. et al.) 12 January 2021 (2021-01-12) claims 1-8 and 20-37, and table 8 and table 10 | 1-37 |

Form PCT/ISA/210 (second sheet) (July 2022)

**INTERNATIONAL SEARCH REPORT**
**Information on patent family members**

International application No.
**PCT/CN2023/136686**

| Patent document cited in search report | | | Publication date (day/month/year) | Patent family member(s) | | | Publication date (day/month/year) |
|---|---|---|---|---|---|---|---|
| WO | 2022266206 | A1 | 22 December 2022 | TW | 202317198 | A | 01 May 2023 |
| WO | 2023081476 | A1 | 11 May 2023 | WO | 2023077441 | A1 | 11 May 2023 |
| WO | 2023141570 | A2 | 27 July 2023 | WO | 2023141570 | A3 | 14 September 2023 |
| WO | 2022228576 | A1 | 03 November 2022 | | None | | |
| WO | 2022132200 | A1 | 23 June 2022 | CA | 3198885 | A1 | 23 June 2022 |
| | | | | EP | 4262807 | A1 | 25 October 2023 |
| | | | | CO | 2023009083 | A2 | 30 November 2023 |
| | | | | US | 2023072276 | A1 | 09 March 2023 |
| | | | | JP | 2023553492 | A | 21 December 2023 |
| | | | | AU | 2021401232 | A1 | 22 June 2023 |
| | | | | KR | 20230142465 | A | 11 October 2023 |
| | | | | IL | 303446 | A | 01 August 2023 |
| WO | 2021207172 | A1 | 14 October 2021 | US | 2022370416 | A1 | 24 November 2022 |
| | | | | JP | 2023521698 | A | 25 May 2023 |
| | | | | EP | 4132655 | A1 | 15 February 2023 |
| CN | 112218859 | A | 12 January 2021 | AU | 2019249231 | A1 | 15 October 2020 |
| | | | | AU | 2019249231 | B2 | 21 April 2022 |
| | | | | AU | 2022206739 | A1 | 11 August 2022 |
| | | | | US | 2019315732 | A1 | 17 October 2019 |
| | | | | US | 11161841 | B2 | 02 November 2021 |
| | | | | JP | 2021521112 | A | 26 August 2021 |
| | | | | WO | 2019195609 | A2 | 10 October 2019 |
| | | | | WO | 2019195609 | A3 | 28 November 2019 |
| | | | | IL | 277729 | A | 30 November 2020 |
| | | | | MX | 2020010420 | A | 11 December 2020 |
| | | | | BR | 112020020307 | A2 | 12 January 2021 |
| | | | | KR | 20230130752 | A | 12 September 2023 |
| | | | | US | 2022402907 | A1 | 22 December 2022 |
| | | | | KR | 20210006356 | A | 18 January 2021 |
| | | | | RU | 2020136178 | A3 | 06 May 2022 |
| | | | | CA | 3095494 | A1 | 10 October 2019 |
| | | | | CA | 3095494 | C | 07 November 2023 |
| | | | | CO | 2020013864 | A2 | 10 December 2020 |
| | | | | EP | 3774777 | A2 | 17 February 2021 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2023138524 A1